# EUROPEAN PATENT SPECIFICATION

(11) **EP 1 060 163 B1**
(45) Date of publication and mention of the grant of the patent: **12.10.2005**
(21) Application number: 98965275.5
(22) Date of filing: 16.12.1998
(51) Int. Cl.: C07D 213/56, A61K 31/495, C07F 9/6509, C07D 213/66, C07D 401/12, C07D 213/70, C07D 213/64, C07D 213/61, C07D 487/08, C07D 495/04, C07D 405/12, C07D 409/12, C07D 491/04, C07D 417/12, C07D 513/04

(54) **NEW PIPERAZINYL-SUBSTITUTED PYRIDYLALKANE, ALKENE AND ALKINE CARBOXAMIDES**
NEUE PIPERAZINYL-SUBSTITUIERTE PYRIDYLALKAN, ALKEN AND ALKIN CARBOXAMIDE
NOUVEAUX CARBOXAMIDES DE PIPERAZINYLE SUBSTITUEEPYRIDYLALCANE, ALCENE ET ALCYNE

(30) Priority: 17.12.1997 DE 19756236
(43) Date of publication of application: 20.12.2000
(73) Proprietor: Klinge Pharma GmbH, D-81673 München (DE)
(72) Inventor: BIEDERMANN, Elfi, D-85591 Vaterstetten (DE); HASMANN, Max, D-82061 Neuried (DE); LÖSER, Roland, D-82340 Feldafing (DE); RATTEL, Benno, D-81249 Munich (DE); REITER, Friedemann, D-85640 Putzbrunn (DE); SCHEIN, Barbara, D-85375 Neufahrn (DE); SEIBEL, Klaus, D-82166 Gräfelfing (DE); VOGT, Klaus, D-81669 Munich (DE); WOSIKOWSKI, Katja, D-85586 Poing (DE); SCHEMAINDA, Isabel, D-80804 München (DE)
(74) Representative: HOFFMANN EITLE
(86) International application number: PCT/EP1998/008268
(87) International publication number: WO 1999/031063

(56) References cited:
- EP-A- 0 048 045
- EP-A- 0 210 782
- WO-A-95/24894
- WO-A-97/48696
- NISHIKAWA, YOSHINORI ET AL: "Acrylamide derivatives as antiallergic agents. 2. Synthesis and structure activity relationships of N-[4-[4-(diphenylmethyl)-1- piperazinyl]butyl]-3-(3-pyridyl)acrylamide s" J. MED. CHEM. (1989), 32(3), 583-93 CODEN: JMCMAR;ISSN: 0022-2623, XP002101517 cited in the application
- DATABASE WPI Section Ch, Week 8239 Derwent Publications Ltd., London, GB; Class B03, AN 82-82639E XP002101518 & JP 57 136518 A (EISAI CO LTD) , 23 August 1982

## Description

The invention relates to new piperazinyl-substituted pyridylalkane, alkene and alkine carboxamides with a saturated, one or several-fold unsaturated hydrocarbon residue in the carboxylic acid portion, methods for the synthesis of these compounds, medicaments containing these and their production as well as their therapeutic use especially as cytostatic agents and immunosuppresive agents, for example, in the treatment or prevention of various types of tumors and control of immune reactions, for example of autoimmune diseases.

A pressing need exists for new pharmaceuticals and/or medicaments for cytostatic therapy which not only possess a strong activity, but also exert diminished side effects in comparison to many classical cancerostatic agents, whereby treatment of a broad as possible spectrum of tumors should be made accessible. Furthermore, effective cytostatic agents for an efficient therapy should be made available. Active ingredients of this type should also be exceptionally suitable in the mentioned indications for a combination therapy, be it in connection with other cytostatic agents or with radiation (for example X-rays, radioactive elements, such as cobalt, or linear accelerator, etc.), with operative procedures, heat treatment, etc.

Additionally, from another point of view, there exists a strong need in the field of tumor therapy for new compounds, for example for overcoming or avoiding resistances, which enrich the pallet of cancerostatics based on new modes of action in the ideal case.

This object was successfully solved by the creation of the piperazinyl-substituted pyridylalkane, alkene and alkine carboxamide derivatives as defined in detail in the claims and medicaments containing these as well as the use of these compounds, optionally in combination with other suitable active ingredients and adjuvants, for cytostatic and immunosuppressive therapy or prevention.

It is known that various pyridine compounds or piperazine derivatives substituted in a specific manner have pharmacologically useful properties - however, in contrast to the actions of the compounds according to the invention, these lie in completely different fields of indication.

Thus, ω-pyridyl alkane and/or alkene amides with anti-allergic activity are described in EP 0 210 782 which are referred to as having a 5-lipoxygenase-inhibiting and antihistamine action, wherein the amide components of these compounds contain a piperazine or homopiperazine ring and the pyridine ring can be linked together in the 2-, 3- or 4-position. However, corresponding overlapping compound groups are excluded from the present claimed scope of protection according to the invention.

JP 63,179,869 describes further pyridyl amides, ω-pyridyl alkane and alkene amides as anti-allergic effective substances containing a substituted piperidine ring in the amine component. Similarly structured compounds with the same properties are mentioned in Chem. Pharm. Bull 37, 100-105 (1989) as well as in J. Med. Chem. 1989, 583-593.

The synthesis and pharmacological evaluation of heterocyclic carboxamides which can be substituted at an end of the molecule by completely different heterocycles such as thiophene, guinoline, indole, benzimidazole or indazole as well as pyridine are described in J. Med. Chem., 1996, pages 4692-4706. As opposed to the compounds according to the invention, those published carboxamides possess an activity directed against psychoses. A few particularly named piperazine-substituted pyridyl carboxamides and/or their formula group therein are not encompassed by the present protective scope of the compounds of the invention according to the meanings given in the present claims because they have a direct bond instead of the structural element A. Based on the completely different therapeutic possibility of the known compounds in the field of psychiatry as compared to the indications according to the invention, it could not be expected that the compounds of the present invention would have the named cancerostatic and immunosuppressive effects.

Pyridyl ureas, pyridyl thioureas and pyridyl carbonamides, wherein the amide portion is bound over an aryl-substituted alkyl chain with a piperidine ring or piperidine ring or piperazine ring, are described for example in EP-A-0 428 434 or in EP-A-0 512 902 as antagonists of the neurokinin receptor and substance P. Furthermore, pyridyl(alkyl)carbonamides, pyridyl(alkyl)sulfonamides and analogous ureas, wherein the amide portion is bound to piperidine ring over an alkyl chain, are disclosed in EP-A-0 479 601 as active ingredients with anti-arrhythmic properties.

Further structurally closely related compounds are represented by the piperidine compounds described in EP-A-0 330 026. However, no 3-pyridyl derivatives were concretely described and no concrete examples were disclosed in this publication, aside from a single compound which is described below. These known compounds are distinguished by an anticholinesterase activity, an anti-amnesia activity as well as activities directed against hyperkinesia, senile dementia, mania and Alzheimer's disease.

In WO 91/15 485, the production of pyridine-3,5-dicarboxylic acid esters and amides as well as their use for the treatment of tumor conditions is described. These compounds differ from the compounds according to the invention described below in very important structural features, for example by the dicarboxyl grouping on the pyridine ring or the absence of the hydrocarbon chain between the pyridine ring and the amide grouping. The compounds disclosed in WO 89/07 443 in the form of optically pure R(-)-niguldipin and further analogous dihydropyridines with cytotoxic activity have larger structural differences. However, as compared to these known compounds, the compounds according to the invention unexpectedly possess a better activity and a wider spectrum of action despite the large structural difference.

In the international PCT patent applications WO 95/10516, WO 96/31477, WO 96/31478 or for example in WO 95/10515, tricyclic amide compounds are described which possess an anti-proliferative activity. All of these compounds described therein are distinguished in that they must imperatively possess a tricyclic anellated ring system with at least one nitrogen atom, for example 6, 11-dihydro-5H-benzo[5,6]cyclohepta[1,2-b]pyridinyl ring system as a pharmaphoric group, whereby the molecule portion opposite this tricyclic anellated system is uncommonly variable and represents one of many variation possibilities among numerous substitution possibilities of the given pyridyl substitution. A further meaningful difference in the substitution of these molecules in comparison to the compounds according to the invention is to be seen in the lack of the present structural element D, i.e. both heterocycles found in opposition are directly bound over the carboxy group without being bound over an alkaline chain found there.

A further essential difference of the compounds according to the invention in comparison to these tricyclic compounds is to be recognized in the presence of the terminal 3-pyridyl-substitution which must be present. The presence of this heterocyclic ring required according to the invention as well as the particular bond in the 3-position according to the substitution of the invention in comparison to the above mentioned anti-proliferative compounds of the state of the art indicates that this 3-pyridyl group is an important factor for the anti-tumor action.

In fact, the compounds according to the invention cover a different tumor spectrum from those named in the PCT/WO publications with this necessarily present tricyclic anellated ring system. In the mentioned PCT/WO publications of the state of the art, a treatment possibility in tumors is merely mentioned which is made in connection with a potential inhibition of the farnesyl protein transferase, whereby this mechanism relates to the expression of the activated ras-oncogene. In contrast to this, the compounds according to the invention with the 3-pyridyl-substitution required according to the invention are not limited to the therapy of tumor cells of this type with abnormal production of the ras-oncogene; rather, the therapy possibilities with the new compounds according to the invention extend to the combat of numerous other types of tumors with different causal mechanisms as well as immunosuppressive treatment possibilities such as autoimmune diseases.

In view of this art, the finding that the compounds according to the general formula (I) with the particular substitutions defined below have superior pharmacological activities which make them particularly suitable in an excellent manner for the therapy of abnormal cell growth such as tumor illnesses over a broad anti-proliferative spectrum, was completely unexpected. The pharmacological finding that, aside from the cytostatic effectiveness, especially with different tumor spectra, the compounds according to the invention also possess immunosuppressive properties and additionally favorable abortive properties without harmful mutagenic effects is to be considered as equally surprising.

Structurally close pyridyl compounds wherein instead of the piperazine ring, a cyclic non-aromatic heterocyclic ring residue with merely one ring nitrogen atom and optionally an additional ring oxygen atom, preferably a piperidinyl residue, is incorporated as well as their use especially as cytostatic agents are subject matter of the older patent applications P 196 24 704.7-44, P 196 24 668.7-41 as well as P 196 24 659.8-44 which have not yet been published.

The most important differentiating feature of the new compounds according to the invention with respect to these older, non-prepublished compounds is therefore the structural feature E which in the present application always represents especially piperazine or hexahydro-1,4-diazepine.

These new piperazinyl-substituted pyridyl carboxamides correspond to the following general formula: 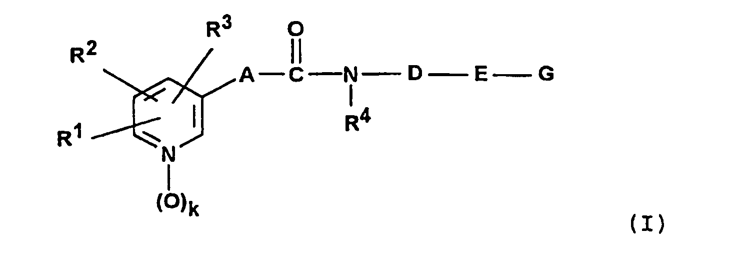 wherein the structural element **E** has the following meaning: 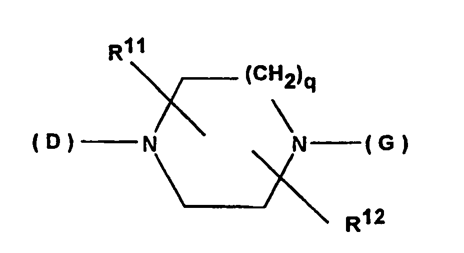 whereby
- **q**: is 1, 2 or 3 and therewith can represent the N-heterocyclic ring piperazine, hexahydro-1,4-diazepine or octahydro-1,4-azocine.

The meaning of the remaining substituents and the preferred embodiments of the compound groups according to the invention falling under the general formula as well as particularly preferred end products are defined in claims 1 to 7 in detail.

The compounds of Formula (I), which represent the end products can optionally exist as cis- and trans-isomers, E- and Z-isomers, for example when A is a cyclopropane ring or D contains one or more double bonds. Subject matter of the invention is the pure isomers as well as their mixtures. Furthermore, the compounds of Formula (I) can contain one or more asymmetric carbon atoms and, as a result, exist in the form of different optical isomers (enantiomers, diastereomers). The invention includes all optical isomers and their racemic or non-racemic mixtures. Finally, compounds of Formula (I) can exist as endo/exo-isomers in case the ring system E is bicyclic. The pure endo- and exo- isomers as well as their mixtures are also comprised by the invention.

Compounds of Formula (I), in which G is a heterocyclic aromatic ring or contains such in an anellated ring system can optionally be present as tautomers when this heterocyclic ring is substituted by free hydroxy-, mercapto- or amino groups. In this case, the invention includes all tautomeric forms.

Subject matter of the invention are further pharmacologically acceptable acid addition salts of the compounds of Formula (I) with inorganic or organic acids. Preferable examples for addition salts with suitable inorganic acids are hydrochlorides, hydrobromides, hydroiodides, sulfates and phosphates. Addition salts of organic acids are preferably acetates, benzoates, citrates, fumarates, gluconates, malates, maleates, methanesulfonates, lactates, oxalates, succinates, tartrates and tosylates.

Compounds of Formula (I) as well as their acid addition salts can also be optionally present as hydrates or other solvates. The invention includes such hydrates and solvates.

In the compounds of Formula (I), the definitions for the atoms or atomic groups preferably have the following meanings:
Halogen means fluorine, chlorine, bromine or iodine;
Alkyl can be straight chained or branched and preferably signifies a C₁-C₆-alkyl residue, especially a methyl-, ethyl- , propyl-, isopropyl-, butyl-, isobutyl-, sec-butyl-, tert-butyl-, cyclopropylmethyl-, pentyl-, isopentyl-, tert-pentyl-, neopentyl-, cyclopropylethyl-, cyclobutylmethyl- or hexyl group.

Alkylene signifies for example methylene, ethylene, propylene, tetramethylene, pentamethylene, hexamethylene, heptamethylene, octamethylene, nonamethylene or decamethylene.

Alkenyl preferably signifies C₃-C₆-alkenyl and can be straight chained or branched and preferably signifies an allyl-, 2-butenyl-, 3-butenyl-, 2-methyl-2-propenyl-, 2-pentenyl-, 4-pentenyl-, 2-methyl-2-butenyl-, 3-methyl-2-butenyl-, 2-hexenyl-, 5-hexenyl-, 4-methyl-3-pentenyl- or 2,2-dimethyl-3-butenyl-group.

Alkenylene signifies for example ethenylene, propenylene, butenylene, pentenylene, hexenylene, hexadienylene, heptenylene, octenylene, nonenylene or decenylene.

Alkinyl preferably signifies C₂-C₆-alkinyl which can be straight chained or branched and can preferably signify an ethinyl-, propargyl-, 2-butinyl-, 3-butinyl-, 4-pentinyl-, 5-hexinyl- or 4-methyl-2-pentinyl group.

Alkinylene signifies for example propinylene, butinylene, pentinylene, hexinylene, hexeninylene, heptinylene, octinylene, noninylene or decinylene.

Cycloalkyl is preferably a C₃-C₈-cycloalkyl residue, especially a cyclopropyl-, cyclobutyl-, cyclopentyl-, cyclohexyl-, cycloheptyl- or cyclooctyl group.

Hydroxyalkyl contains a hydroxyl group in one of the above mentioned alkyl residues, especially in a C₁-C₆-alkyl residue, whereby among the C₁-C₆-hydroxyalkyl residues, the hydroxymethyl- and the hydroxyethyl residue are preferred.

Aside from the oxygen atom, alkoxy, alkenyloxy, alkinyloxy contain one of the above mentioned preferred C₁-C₆-alkyl-, C₃-C₆-alkenyl- and/or C₃-C₆-Alkinyl groups. Particularly preferred groups for this are the methoxy-, ethoxy-, isopropoxy-, tert-butoxy-, allyloxy- and propargyloxy groups.

Alkoxy, especially C₁-C₆-alkoxy, entirely or partially replaced by fluorine is for example difluoromethoxy, trifluoromethoxy or 2,2,2-trifluoroethoxy.

Aside from the sulphur atom, alkylthio, alkenylthio, alkinylthio contain one of the above mentioned preferred C₁-C₆-alkyl-, C₃-C₆-alkenyl- or C₃-C₆-alkinyl groups. Preferred groups among these are the methylthio-, ethylthio-, isopropylthio- and tert-butylthio groups.

Cyclopentyloxy- and cyclopentylthio- and/or cyclohexyloxy- and cyclohexylthio residues represent preferred C₃-C₈-cycloalkyloxy and C₃-C₈-cycloalkylthio.

Aside from the oxygen atom, alkanoyloxy groups preferably contain an aliphatic acyl group with 1 to 7 carbon atoms.

Among preferred alkanoyloxy groups are the acetoxy-, propionyloxy- and pivaloyloxy groups.

Alkoxycarbonyl groups, preferably C₂-C₇-alkoxycarbonyl groups contain, aside from the carbonyl group, one of the above mentioned alkoxy groups, especially C₁-C₆-alkoxy groups. Preferred alkoxycarbonyl groups are the methoxycarbonyl-, ethoxycarbonyl-, isopropoxycarbonyl-, isobutoxycarbonyl- and tert-butoxycarbonyl groups.

Aside from the oxygen atom, alkoxycarbonyloxy groups preferably contain one of the above mentioned C₂-C₇-alkoxycarbonyl residues. Among preferred alkoxycarbonyl groups are the methoxycarbonyloxy-, ethoxycarbonyloxy-, isopropoxycarbonyloxy-, isobutoxycarbonyloxy- and tert-butoxycarbonyl groups as well as allyloxycarbonyloxy groups.

Aside from the carbonyl group, alkylaminocarbonyl, especially C₂-C₇-alkylaminocarbonyl and dialkylaminocarbonyl groups, preferably C₃-C₁₃-dialkylaminocarbonyl groups, contain an alkylamino- and/or dialkylamino residue whose alkyl groups correspond especially to the C₁-C₆-alkyl groups of the above description. Preferred groups are the dimethylaminocarbonyl-, diethylaminocarbonyl- and diisopropylamino-carbonyl groups.

Aside from the unsubstituted amino group, the amino group of the Formula NR⁵R⁶ is one of the below mentioned alkylamino groups, especially C₁-C₆-alkylamino groups and/or dialkylamino groups, especially di-(C₁-C₆-alkyl)amino groups.

Alkylamino especially contains one of the above mentioned C₁-C₆-alkyl groups. Preferred groups are the methylamino-, ethylamino-, propylamino-, isopropylamino-, butylamino-, and the tert-butylamino groups.

The preferred di-(C₁-C₆-alkyl)amino residue carries two of the same or different of the above mentioned C₁-C₆-alkyl groups on the nitrogen atom. Preferred groups are the dimethylamino-, diethylamino-, dipropylamino-, diisopropylamino-, isopropyl-, methylamino-, dibutylamino- or tert-butylmethylamino groups.

Acyl, especially C₁-C₆-acyl, signifies the residue of an aliphatic saturated or unsaturated, straight chained, branched or cyclic carboxylic acid. Preferred acyl residues are formyl-, acetyl-, propionyl-, acryloyl-, butyryl-, isobutyryl-, methacryloyl-, cyclopropylcarbonyl-, pentanoyl-, pivaloyl-, cyclobutylcarbonyl-, hexanoyl- and dimethylacryloyl groups.

Alkanesulfonyl, especially C₁-C₆-alkanesulfonyl is preferably the methanesulfonyl-, ethanesulfonyl-, propanesulfonyl-, butanesulfonyl-, pentanesulfonyl- or hexanesulfonyl groups.

Saturated or unsaturated, preferably four- to eight-membered heterocycles with one or two hetero-atoms, are for example azetidine, tetrahydrofuran, tetrahydrothiophene, pyrrolidine, tetrahydropyran, tetrahydropyridine, piperidine, tetrahydroazepine, hexahydroazepine, octahydroazocine, pyrazolidine, piperazine, morpholine, thiomorpholine, thiomorpholin-1,1-dioxide, hexahydrodiazepine or hexahydrooxazepine.

Preferred monocyclic aromatic five- or six-membered heterocycles with one to three hetero-atoms are for example furyl, thienyl, pyrrolyl, oxazolyl, isoxazolyl, thiazolyl, isothiazolyl, imidazolyl, pyrazolyl, oxadiazolyl, thiadiazolyl, triazolyl, pyridyl, pyrazinyl, pyridazinyl, pyrimidinyl or triazinyl.

Anellated bi- and tricyclic aromatic or partially hydrated carbocyclic ring systems with 8 to 16 ring atoms and at least one aromatic ring are preferably benzocyclobutyl, indanyl, indenyl, naphthyl, dihydronaphthyl, tetrahydronaphthyl, biphenylenyl, fluoroenyl, anthryl, dihydroanthryl, phenanthryl, dihydrophenanthryl, dibenzocycloheptenyl, dihydrodibenzocycloheptenyl, dihydrodibenzocyclooctenyl or tetrahydrodibenzocyclooctenyl. Their mono- or dioxo-derivates, i.e. for example the residues of indanone, tetralone, anthrone, anthraquinone, fluoroenone, phenanthrone, dibenzocycloheptenone, dihydrodibenzocycloheptenone or tetrahydrodibenzocyclooctenone are also to be understood as partially hydrated carbocyclic ring systems.

Anellated bi- and tricyclic aromatic or partially hydrated heterocyclic ring systems with 8 to 16 ring atoms and at least one aromatic ring are, for example, imidazothiazolyl, benzofuryl, dihydrobenzofuryl, benzothienyl, dihydrobenzothienyl, indolyl, indolinyl, isoindolinyl, benzimidazolyl, indazolyl, benzooxazolyl, benzoisoxazolyl, benzothiazolyl, benzoisothiazolyl, benzofurazanyl, benzothiadiazolyl, benzotriazolyl, oxazolopyridyl, thiazolopyridyl, isothiazolopyridyl, imidazopyridyl, pyrazolopyridyl, thienopyrimidinyl, chromanyl, benzopyranyl, quinolyl, isoquinolyl, dihydroquinolyl, tetrahydroquinolyl, benzodioxanyl, quinoxalinyl, quinazolinyl, naphthyridinyl, carbazolyl, tetrahydrocarbazolyl, pyridoindolyl, acridinyl, phenanthridinyl, dihydrophenanthridinyl, dibenzoisoquinolinyl, dihydrodibenzoisoquinolinyl, phenothiazinyl, dihydrodibenzooxepinyl, benzocycloheptathienyl, dihydrothienobenzothiepinyl, dihydrodibenzothiepinyl, octahydrodibenzothiepinyl, dibenzoazepinyl, dihydrodibenzoazepinyl, octahydrodibenzoazepinyl, benzocycloheptapyridyl, dihydrobenzocycloheptapyridyl, pyridobenzoazepinyl, dihydropyridobenzoazepinyl, dihydropyridobenzodiazepinyl, dihydrodibenzooxazepinyl, dihydropyridobenzooxepinyl, dihydropyridobenzooxazepinyl, dihydrodibenzothiazepinyl or dihydropyridobenzothiazepinyl.

Furthermore, their mono- or dioxo-derivates and/or optionally their possible tautomers are also to be understood as partially hydrated heterocyclic ring systems, i.e.for example the residues of indolinone, isatin, of benzooxazolone and/or its tautomer hydroxybenzooxazole, of benzisoxazolone, benzothiazolone, benzoisothiazolone and benzimidazolone and/or their corresponding tautomers, hydroxybenzoisoxazole, hydroxybenzothiazole, hydroxybenzoisothiazole and hydroxybenzimidazole, as well as indazolinone, of oxazolopyridinones, thiazolopyridinones, pyrazolopyridinones and imidazopyridinones and/or their corresponding tautomers hydroxyoxazolopyridine, hydroxythiazolopyridine, hydroxypyrazolopyridine and hydroxyimidazopyridine, the residues from the series chromanone, chromone, quinolinone, dihydroquinolinone, tetrahydrocarbazolone, acridone, phenanthridone, benzoisoquinolinone, dihydrodibenzoxepinones, benzocycloheptathiophenones, dihydrothienobenzothiepinones, dihydrodibenzothiepinones, dihydrodibenzoazepinones, benzocycloheptapyridinones, dihydropyridobenzoazepinones, dihydropyridobenzodiazepinones, dihydropyridobenzooxazepinones, dihydrodibenzothiazepinones and of dihydropyridobenzothiazepinones.

Saturated and unsaturated monocyclic, four- to eight-membered heterocycles (as the group ―NR¹³R¹⁵) which, aside from the essential nitrogen atom, can optionally contain one or two further hetero-atoms selected from N and/or S and/or O,are for example azetidine, pyrrolidine, piperidine, (1H)-tetrahydropyridine, hexahydroazepine, (1H)-tetrahydroazepine, octahydroazocine, pyrazolidine, piperazine, hexahydrodiazepine, morpholine, hexahydroxazepine, thiomorpholine or thiomorpholin-1,1-dioxide.

Saturated or unsaturated bi- or tricyclic, anellated or bridged heterocycles with 8 to 16 ring atoms (as the group ―NR¹³R¹⁵) which, aside from the essential nitrogen atom, can optionally contain one or two further hetero-atoms, selected from N and/or S and/or O, are for example 5-aza-bicyclo[2.1.1]hexane, 2-aza-bicyclo[2.2.1]heptane, 7-aza-bicyclo[2.2.1]heptane, 2,5-diaza-bicyclo[2.2.1]heptane, 2-aza-bicyclo[2.2.2]octane, 8-aza-bicyclo[3.2.1]octane, 2,5-diaza-bicyclo[2.2.2]octane, 9-aza-bicyclo[3.3.1]nonane, indoline, isoindoline, (1H)-dihydroquinoline, (1H)-tetrahydroquinoline, (2H)-tetrahydroisoquinoline, (1H)-tetrahydroquinoxaline, (4H)-dihydrobenzoxazine, (4H)-dihydrobenothiazine, (1H)-tetrahydrobenzo[b]azepine, (1H)-tetrahydrobenzo[c]azepine, (1H)-tetrahydrobenzo[d]azepine, (5H)-tetrahydrobenzo[b]oxazepine, (5H)-tetrahydrobenzo[b]thiazepine, 1,2,3,4-tetrahydro-9H-pyrido[3,4-b]indole, (10H)-dihydroacridine, (10H)-dihydrophenanthridine, 1,2,3,4-tetrahydroacridanone, (10H)-phenoxazine, (10H)-phenothiazine, (5H)-dibenzazepine, (5H)-dihydrodibenzazepine, (5H)-octahydrodibenzazepine, dihydrobenzo[d,e]isoquinoline, (5H)-dihydrodibenzodiazepine, (5H)-benzo[b]pyrido[f]azepine, (5H)-dihydrobenzo[b]pyrido-[f]azepine (11H)-dihydrodibenzo[b,e]oxazepine, (11H)-dihydrodibenzo[b,e]thiazepine, (10H)-dihydrodibenzo[b,f]oxazepine, (10H)-dihydrodibenzo[b,f]thiazepine, (5H)-tetrahydrodibenzazocine, (11H)-dihydrobenzo[e]pyrido[b]-1,4-diazepin-6-one, (11H)-di-hydrobenzo[b]pyrido[e]-1,4-diazepin-5-one.

Concretely, the invention relates to a pyridylalkane, pyridylalkene or pyridylalkine carboxamide according to formula (I) 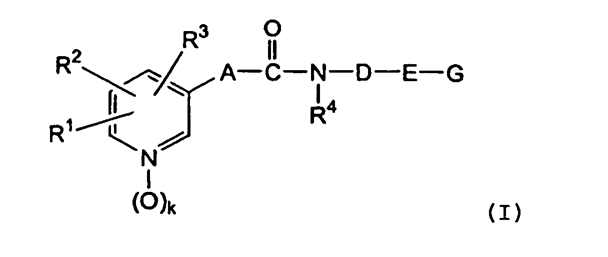 wherein:
- **R**^{**1**}: is selected from
hydrogen, halogen, cyano, C₁-C₆-alkyl, C₃-C₆-alkenyl, C₂-C₆-alkinyl, trifluoromethyl, C₃-C₈-cycloalkyl, C₁-C₆-hydroxyalkyl, hydroxy, C₁-C₆-alkoxy, C₃-C₆-alkenyloxy, C₃-C₆-alkinyloxy, benzyloxy, C₁-C₇-alkanoyloxy, C₂-C₇-alkoxycarbonyloxy, C₁-C₆-alkylthio, C₃-C₆-alkenylthio, C₃-C₆-alkinylthio, C₃-C₈-cycloalkyloxy, C₃-C₈-cycloalkylthio, C₂-C₇-alkoxycarbonyl, aminocarbonyl, C₂-C₇-alkylaminocarbonyl, C₃-C₁₃-dialkylaminocarbonyl, carboxy, phenyl, phenoxy, phenylthio, pyridyloxy, pyridylthio, and **NR**^{**5**}**R**^{**6**}**,** wherein
- **R**^{**5**} and **R**^{**6**}: are selected independently of each other from
hydrogen, C₁-C₆-alkyl, C₃-C₆-alkenyl, C₃-C₆-alkinyl, benzyl and phenyl;
- **R**^{**2**}: is selected from
hydrogen, halogen, cyano, C₁-C₆-alkyl, trifluoromethyl, hydroxy, C₁-C₆-alkoxy, benzyloxy and C₁-C₇-alkanoyloxy; or
- **R**^{**1**} and **R**^{**2**},: if adjacent, optionally form a bridge selected from -(CH₂)₄-, -(CH=CH)₂- and -CH₂O-C**R**^{**7**}**R**^{**8**}-O-, wherein
**R**^{**7**} and **R**^{**8**} are selected independently from each other from hydrogen and C₁-C₆-alkyl;
- **R**^{**3**}: is selected from
hydrogen, halogen, C₁-C₆-alkyl, trifluoromethyl and C₁-C₆-hydroxyalkyl;
- **R**^{**4**}: is selected from
hydrogen, C₁-C₆-alkyl, C₃-C₆-alkenyl, C₃-C₆-alkinyl, C₃-C₆-cycloalkyl, hydroxy, C₁-C₆-alkoxy and benzyloxy;
- **k**: is 0 or 1,
- **A**: is selected from
C₁-C₆-alkylene, optionally substituted once to threefold by C₁-C₃-alkyl, hydroxy, C₁-C₃-alkoxy, fluorine, or phenyl,
C₂-C₆-alkylene, in which a methylene unit is isosterically replaced by O, S, **NR**^{**9**}**,** CO, SO or SO₂, wherein, with the exception of CO, the isosteric substitution is not adjacent to the amide group and **R**^{**9**} is selected from hydrogen, C₁-C₆-alkyl, C₃-C₆-alkenyl, C₃-C₆-alkinyl, C₁-C₆-acyl and C₁-C₆-alkanesulfonyl,
1,2-cyclopropylene,
C₂-C₆-alkenylene, optionally substituted once to threefold by C₁-C₃-alkyl, hydroxy, C₁-C₃-alkoxy, fluorine, cyano or phenyl,
C₄-C₆-alkadienylene, optionally substituted once or twice by C₁-C₃-alkyl, fluorine, cyano or phenyl;
1,3,5-hexatrienylene, optionally substituted by C₁-C₃-alkyl, fluorine, cyano or phenyl, and
ethinylene
- **D**: is selected from
C₂-C₁₀-alkylene, optionally substituted once or twice by C₁-C₆-alkyl, hydroxy, or C₁-C₆-alkoxy;
C₄-C₁₀-alkenylene, optionally substituted once or twice by C₁-C₆-alkyl, hydroxy, or C₁-C₆-alkoxy;
C₄-C₁₀-alkinylene, optionally substituted once or twice by C₁-C₆-alkyl, hydroxy, or C₁-C₆-alkoxy; and
the group consisting of C₂-C₁₀-alkylene, C₄-C₁₀-alkenylene and C₄-C₁₀-alkinylene, wherein one to three methylene units are isosterically replaced by O, S, **NR**^{**10**}**,** CO, SO or SO₂, wherein
**R**^{**10**} has the same meaning as **R**^{**9**}**,** but is selected independently thereof;
- **E**: represents 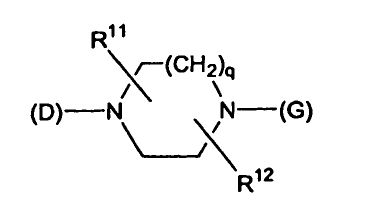 wherein
- **q**: is 1, 2 or 3;
- **R**^{**11**}: is selected from
hydrogen, C₁-C₆-alkyl, hydroxy, hydroxymethyl, carboxy, and C₂-C₇-alkoxycarbonyl, and
- **R**^{**12**}: is selected from
hydrogen, C₁-C₆-alkyl and an oxo group adjacent to a nitrogen atom,
or
**R**^{**11**} and **R**^{**12**} together form a C₁-C₃-alkylene bridge under formation of a bicyclic ring system;
- **G**: is selected from G1, G2, G3, G4 and G5, wherein
- **G1**: represents

―(CH₂)ᵣ―(CR¹⁴R¹⁵)ₛ―R¹³,
- **r**: is 0, 1, 2 or 3,
- **s**: is 0 or 1,
- **R**^{**13**}: is selected from
hydrogen, C₁-C₆-alkyl, C₃-C₆-alkenyl, C₃-C₆-alkinyl, C₃-C₈-cycloalkyl;
the group consisting of saturated and unsaturated, four to eight-membered heterocycles, which optionally contain one or two hetero-atoms selected from N, S and O;
benzyl, phenyl;
the group consisting monocyclic aromatic five to six-membered heterocycles, which optionally contain one to
three hetero-atoms selected from the N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms are selected from N, S and O and the linkage occurs either over an aromatic ring or a hydrogenated ring and either directly or over a methylene group,
- **R**^{**14**}: has the same meaning as **R**^{**13**}**,** but is selected independently thereof;
- **R**^{**15**}: is selected from
hydrogen, hydroxy, methyl, benzyl, phenyl,
the group consisting of monocyclic aromatic five to six-membered heterocycles, which contain one to three hetero-atoms from the group N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group,
- **G2**: is selected from 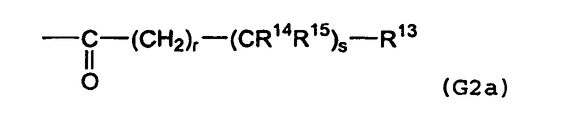 and 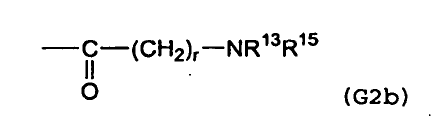 wherein **r, s** and the substituents **R**^{**13**} to **R**^{**15**} have the above meaning, or the group

―NR¹³R¹⁵

represents a nitrogen-containing heterocycle bound over the nitrogen atom which nitrogen-containing heterocycle is selected from
the group consisting of saturated and unsaturated monocyclic, four to eight-membered heterocycles, which, aside from the essential nitrogen atom, optionally contain one or two further hetero-atoms selected from N, S and O, and
the group consisting of saturated and unsaturated bi- or tricyclic, anellated or bridged heterocycles with 8 to 16 ring atoms, that aside from the essential nitrogen atom, optionally contain one or two further hetero-atoms selected from N, S and O;
- **G3**: represents

―SO₂―(CH₂)ᵣ―R¹³,

wherein **r** and **R**^{**13**} have the above meanings,
- **G4**: represents 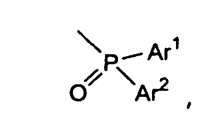 wherein
- **Ar**^{**1**} and **Ar**^{**2**}: are selected independently from each other from phenyl, pyridyl and naphthyl;
- **G5**: represents

―COR¹⁶ (G5)

wherein
- **R**^{**16**}: is selected from
trifluoromethyl, C₁-C₆-alkoxy, C₃-C₆-alkenyloxy, and benzyloxy,
wherein aromatic ring systems in the substituents **R**^{**1**}**, R**^{**2**}**, R**^{**4**}**, R**^{**5**}**, R**^{**6**}**, R**^{**13**}**, R**^{**14**}**, R**^{**15**}**, R**^{**16**}**, Ar**^{**1**} and **Ar**^{**2**} and/or in the ring system **―NR**^{**13**}**R**^{**15**} optionally are substituted independently from each other by one to three of the same or different groups selected from
halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide for two adjacent residues on the aromatic ring, and wherein
alkyl-, alkenyl- and cycloalkyl residues in the groups **G1, G2** and **G3** optionally are substituted by one or two of the same or different groups which are selected from hydroxy, carboxy, C₂-C₇-alkoxycarbonyl, benzyloxycarbonyl, amino, mono-C₁-C₆-alkylamino and di-(C₁-C₆-alkyl-amino);
their cis- and trans-isomers, E- and Z-isomers, enantiomers, diastereomers and other isomers as well as their racemic or non-racemic mixtures and the corresponding endo- and exo-isomers for the case that the ring system E is bicyclic;
their tautomeres; as well as
their acid addition salts including their hydrates and solvates,
wherein **G** does not represent

―(CH₂)ᵣ―(CR¹⁴R¹⁵)ₛ―R¹³,

in the case that simultaneously
- **R**^{**13**}: represents pyridyl or phenyl which is optionally substituted by halogen, alkyl, alkoxy or trifluoromethyl,
- **R**^{**14**}: represents hydrogen or phenyl, which is optionally substituted by halogen, alkyl, alkoxy or trifluoromethyl,
- **R**^{**15**}: represents hydrogen,
- **A**: represents alkylene, optionally substituted ethenylene or butadienylene,
- **D**: represents alkylene or alkenylene,
- **E**: represents piperazine or homopiperazine and
- **s**: is 1;
and wherein **G** does not represent
a member of the group consisting of phenyl, N-containing heteroaryl and 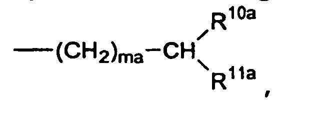 wherein: R^{10a} is hydrogen or phenyl, R^{11a} is a phenyl or a pyridyl, and ma is an integer of 0 to 2, provided that the phenyl group may optionally be substituted by one or two members selected from the group consisting of a halogen, a C₁-C₆ alkyl, trifluoromethyl and a C₁-C₆ alkoxy;
in the case that simultaneously
- **R**^{**1**}: is hydrogen, a halogen, a C₁-C₆-alkyl, a C₁-C₆-alkoxy, a C₁-C₆-alkylthio, a C₃-C₈-cycloalkyloxy, a C₃-C₈-cycloalkylthio, a C₂-C₇-alkoxycarbonyl, carboxy, a phenyl, a phenoxy, a phenylthio, 3-pyridyloxy or 3-pyridylthio;
- **R**^{**2**}: is hydrogen, hydroxy, a C₁-C₇-alkanoyloxy or a C₂-C₇-alkoxycarbonyloxy, or when R¹ and R₂ are adjacent to each other, they may combine to form tetramethylene or -CH₂OCR^{8a}R^{9a}O-, wherein R^{8a} and R^{9a} are the same or different and are each a C₁-C₆-alkyl;
- **R**^{**3**}: is hydrogen, a C₁-C₆-alkyl or a hydroxy-C₁-C₆-alkyl;
- **A**: is a C₁-C₆-alkylene or -(CR^{6a}=CR^{7a})ᵣₐ-, wherein R^{6a} is hydrogen, a C₁-C₃-alkyl or a phenyl, R^{7a} is hydrogen, a C₁-C₃-alkyl, cyano or a phenyl, and ra is 1 or 2;
- **R**^{**4**}: is hydrogen;
- **D**: represents a C₂-C₁₀-alkylene or a C₄-C₁₀-alkylene interrupted by one double bond; and
- **E**: is selected from piperazine, piperazine, which is substituted by C₁-C₆-alkyl, homopiperazine, and homopiperazine, which is substituted by C₁-C₆-alkyl.

According to a preferred embodiment, the invention relates to compounds of formula (I) 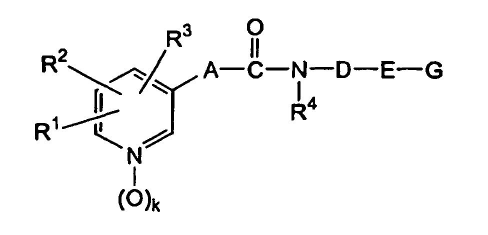 wherein
- **R**^{**1**}: is selected from
hydrogen, halogen, cyano, C₁-C₆-alkyl, trifluoromethyl, C₃-C₈-cycloalkyl, C₁-C₆-hydroxyalkyl, hydroxy, C₁-C₄-alkoxy, benzyloxy, C₁-C₄-alkylthio, C₁-C₅-alkanoyloxy, C₂-C₅-alkoxycarbonyl, aminocarbonyl, C₂-C₅-alkylaminocarbonyl, C₃-C₉-dialkylaminocarbonyl, carboxy, phenyl, phenoxy, phenylthio, pyridyloxy, and NR⁵R⁶, wherein
- **R**^{**5**} and **R**^{**6**}: are selected independently from each other from hydrogen and C₁-C₆-alkyl;
- **R**^{**2**}: is selected from
hydrogen, halogen, cyano, C₁-C₆-alkyl, trifluoromethyl, hydroxy, and C₁-C₄-alkoxy;
- **R**^{**3**}: is selected from
hydrogen, halogen and C₁-C₆-alkyl;
- **R**^{**4**}: is selected from
hydrogen, C₁-C₆-alkyl, C₃-C₆-alkenyl, C₃-C₆-cycloalkyl, hydroxy, C₁-C₆-alkoxy and benzyloxy;
- **k**: is 0 or 1,
- **A**: is selected from
C₁-C₆-alkylene, optionally substituted once to threefold by C₁-C₃-alkyl, hydroxy, fluorine, or phenyl,
C₂-C₆-alkylene, in which a methylene unit is isosterically replaced by O, S, **NR**^{**9**}, CO, SO or SO₂, wherein, with the exception of CO, the isosteric substitution is not adjacent to the amide group and the residue **R**^{**9**} is selected from hydrogen, C₁-C₆-alkyl, C₁-C₆-acyl and methane sulfonyl;
1,2-cyclopropylene,
C₂-C₆-alkenylene, optionally substituted once to threefold by C₁-C₃-alkyl, hydroxy, fluorine, cyano or phenyl,
C₄-C₆-alkadienylene, optionally substituted once or twice by C₁-C₃-alkyl, fluorine, cyano or phenyl;
1,3,5-hexatrienylene, optionally substituted by C₁-C₃-alkyl, fluorine, or cyano, and
ethinylene
- **D**: is selected from
C₂-C₁₀-alkylene, optionally substituted once or twice by C₁-C₃-alkyl or hydroxy;
C₄-C₁₀-alkenylene, optionally substituted once or twice by C₁-C₃-alkyl or hydroxy;
C₄-C₁₀-alkinylene, optionally substituted once or twice by C₁-C₃-alkyl or hydroxy; and
the group consisting of C₂-C₁₀-alkylene, C₄-C₁₀-alkenylene and C₄-C₁₀-alkinylene, wherein one to three methylene units are isosterically replaced by O, S, **NR**^{**10**}**,** CO, SO or SO₂, wherein
**R**^{**10**} has the same meaning as **R**^{**9**}**,** but is selected independently thereof;
- **E**: represents 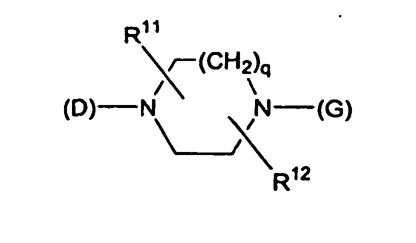 wherein
- **q**: is 1, 2 or 3;
- **R**^{**11**}: is selected from
hydrogen, C₁-C₃-alkyl, hydroxy, hydroxymethyl, carboxy, and C₂-C₇-alkoxycarbonyl, and
- **R**^{**12**}: is selected from hydrogen and an oxo group adjacent to a nitrogen atom or
- **R**^{**11**} and **R**^{**12**}**,**: together, form a C₁-C₃-alkylene bridge under formation of a bicyclic ring system;
- **G**: is selected from G1, G2, G3, G4 and G5, wherein
- **G1**: represents

―(CH₂)ᵣ―(CR¹⁴R¹⁵)ₛ―R¹³,
- **r**: is 0, 1 or 2,
- **s**: is 0 or 1,
- **R**^{**13**}: is selected from
hydrogen, C₁-C₆-alkyl, C₃-C₆-alkenyl, C₃-C₆-alkinyl, C₃-C₈-cycloalkyl; benzyl, phenyl;
the group consisting of monocyclic aromatic five to six-membered heterocycles, which contain one to three hetero-atoms selected from N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group,
- **R**^{**14**}: has the same meaning as **R**^{**13**}**,** but is selected independently thereof;
- **R**^{**15**}: is selected from
hydrogen, hydroxy, methyl, benzyl, phenyl;
the group consisting of monocyclic aromatic five to six-membered heterocycles, which contain one to three hetero-atoms selected from N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group, and
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group;
- **G2**: is selected from 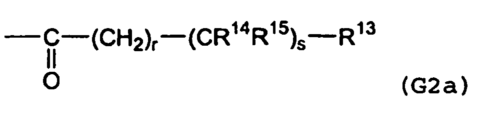 and 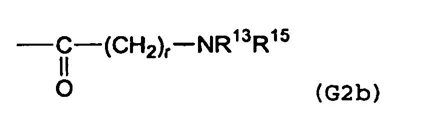 wherein **r, s** and the substituents **R**^{**13**} to **R**^{**15**} have the above meaning, or the group

―NR¹³R¹⁵

is a nitrogen-containing heterocycle bound over the nitrogen atom which nitrogen-containing heterocycle is selected from
the group consisting of saturated and unsaturated monocyclic, four to eight-membered heterocycles, which, aside from the essential nitrogen atom, optionally contain one or two further hetero-atoms selected from N, S and O, and
the group consisting of saturated and unsaturated bi- or tricyclic, anellated or bridged heterocycles with 8 to 16 ring atoms, that aside from the essential nitrogen atom, optionally contain one or two further hetero-atoms selected from N, S and O;
- **G3**: represents

―SO₂―(CH₂)ᵣ―R¹³,

wherein **r** and **R**^{**13**} have the above meaning,
- **G4**: represents 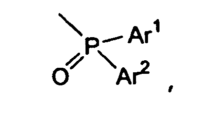 wherein
- **Ar**^{**1**} and **Ar**^{**2**}: are selected independently from each other from phenyl, pyridyl and naphthyl;
- **G5**: represents

―COR¹⁶,

wherein
**R**^{**16**} is selected from
trifluoromethyl, C₁-C₆-alkoxy, C₃-C₆-alkenyloxy, and benzyloxy,
wherein aromatic ring systems in the substituents **R**^{**1**}**, R**^{**2**}**, R**^{**4**}**, R**^{**5**}**, R**^{**6**}**, R**^{**13**}**, R**^{**14**}**, R**^{**15**}**, R**^{**16**}**, Ar**^{**1**} and **Ar**^{**2**} and/or in the ring system ―NR¹³R¹⁵ optionally are substituted independently from each other by one to three of the same or different groups selected from
halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide in the case of two adjacent residues on the aromatic ring,
wherein alkyl, alkenyl and cycloalkyl residues in the groups **G**^{**1**}**, G**^{**2**} and **G**^{**3**} optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl-amino),
wherein **G** does not represent
a member of the group consisting of phenyl, N-containing heteroaryl and 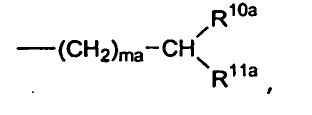 wherein: R^{10a} is hydrogen or phenyl, R^{11a} is a phenyl or a pyridyl, and ma is an integer of 0 to 2, provided that the phenyl group may optionally be substituted by one or two members selected from the group consisting of a halogen, a C₁-C₆ alkyl, trifluoromethyl and a C₁-C₆ alkoxy,
in the case that simultaneously
- **R**^{**1**}: is hydrogen, a halogen, a C₁-C₆-alkyl, a C₁-C₆-alkoxy, a C₁-C₆-alkylthio, a C₃-C₈-cycloalkyloxy, a C₃-C₈-cycloalkylthio, a C₂-C₇-alkoxycarbonyl, carboxy, a phenyl, a phenoxy, a phenylthio, 3-pyridyloxy or 3-pyridylthio;
- **R**^{**2**}: is hydrogen, hydroxy, a C₁-C₇-alkanoyloxy or a C₂-C₇-alkoxycarbonyloxy, or when R¹ and R₂ are adjacent to each other, they may combine to form tetramethylene or -CH₂OCR^{8a}R^{9a}O-, wherein R^{8a} and R^{9a} are the same or different and are each a C₁-C₆-alkyl;
- **R**^{**3**}: is hydrogen, a C₁-C₆-alkyl or a hydroxy-C₁-C₆-alkyl;
- **A**: is a C₁-C₆-alkylene or -(CR^{6a}=CR^{7a})ᵣₐ-, wherein R^{6a} is hydrogen, a C₁-C₃-alkyl or a phenyl, R^{7a} is hydrogen, a C₁-C₃-alkyl, cyano or a phenyl, and ra is 1 or 2;
- **R**^{**4**}: is hydrogen;
- **D**: represents a C₂-C₁₀-alkylene or a C₄-C₁₀-alkylene interrupted by one double bond; and
- **E**: is selected from piperazine, piperazine, which is substituted by C₁-C₆-alkyl, homopiperazine, and homopiperazine, which is substituted by C₁-C₆-alkyl.

According to a further embodiment, the invention especially relates to compounds of formula (I), wherein
- **R**^{**1**}: is selected from
hydrogen, halogen, cyano, methyl, ethyl, trifluoromethyl, hydroxy, C₁-C₄-alkoxy, benzyloxy, C₁-C₅-alkanoyloxy, methylthio, ethylthio, methoxycarbonyl, tert-butoxycarbonyl aminocarbonyl, carboxy, phenoxy, and phenylthio,
- **R**^{**2**}: is selected from
hydrogen, halogen, trifluoromethyl, and hydroxy,
- **R**^{**3**}: is selected from
hydrogen, and halogen,
- **R**^{**4**}: is selected from
hydrogen, C₁-C₃-alkyl, allyl, hydroxy, and C₁-C₃-alkoxy;
- **k**: is 0 or 1,
- **A**: is selected from
C₁-C₆-alkylene, optionally substituted once or twice by C₁-C₃-alkyl, hydroxy or fluorine;
C₂-C₆-alkylene, in which a methylene unit is isosterically replaced by O, S, **NR**^{**9**}**,** CO, SO or SO₂, wherein, with the exception of CO, the isosteric substitution is not adjacent to the amide group,
C₂-C₆-alkenylene, optionally substituted once or twice by C₁-C₃ alkyl, hydroxy and/or fluorine;
C₄-C₆-alkadienylene, optionally substituted by C₁-C₃-alkyl or one or two fluorine atoms; and
1,3,5-hexatrienylene, optionally substituted by fluorine;
- **D**: is selected from
C₂-C₈-alkylene, optionally substituted once or twice by methyl or hydroxy;
C₄-C₈-alkenylene, optionally substituted once or twice by methyl or hydroxy;
C₄-C_{B}-alkinylene, optionally substituted once or twice by methyl or hydroxy; and
the group consisting of C₂-C₈-alkylene, C₄-C₈-alkenylene and C₄-C₈-alkinylene, wherein one to three methylene units are each isosterically replaced by O, S, NH, N(CH₃) N(COCH₃), N(SO₂CH₃), CO, SO or SO₂,
- **E**: represents 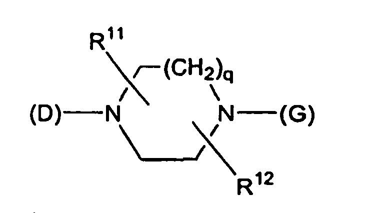 wherein
- **q**: is 1 or 2;
- **R**^{**11**}: is selected from
hydrogen, C₁-C₃-alkyl, hydroxymethyl, and carboxy, and
- **R**^{**12**}: is selected from
hydrogen and an oxo group adjacent to a nitrogen atom
- **G**: is selected from G1, G2, G3, G4 and G5, wherein
- **G1**: represents

―(CH₂)ᵣ―(CR¹⁴R¹⁵)ₛ―R¹³
- **r**: is 0, 1 or 2,
- **s**: is 0 or 1;
- **R**^{**13**}: is selected from
hydrogen, C₁-C₆-alkyl, C₃-C₈-cycloalkyl, benzyl, phenyl,
the group consisting of benzocyclobutyl, indanyl, indenyl, oxoindanyl, naphthyl, dihydronaphthyl, tetrahydronaphthyl, oxotetrahydronaphthyl, biphenylenyl, fluorenyl, oxofluorenyl, anthryl, dihydroanthryl, oxodihydroanthryl, dioxodihydroanthryl, phenanthryl, dihydrophenanthryl, oxodihydrophenanthryl, dibenzocycloheptenyl, oxodibenzocycloheptenyl, dihydrodibenzocycloheptenyl, oxodihydrodibenzocycloheptenyl, dihydrodibenzocyclooctenyl, tetrahydrodibenzocyclooctenyl and oxotetrahydrodibenzocyclooctenyl, bound directly or over a methylene group; and
the group consisting of furyl, thienyl, pyrrolyl, oxazolyl, isoxazolyl, thiazolyl, iso-thiazolyl, pyrazolyl, imidazolyl, oxadiazolyl, thiadiazolyl, triazolyl, pyridyl, pyrazinyl, pyridazinyl, pyrimidinyl, triazinyl, imidazothiazolyl, benzofuryl, dihydrobenzofuryl, benzothienyl, dihydrobenzothienyl, indolyl, indolinyl, isoindolinyl, oxoindolinyl, dioxoindolinyl, benzoxazolyl, oxobenzooxazolinyl, benzoisooxazolyl, oxobenzoisooxazolinyl, benzothiazolyl, oxobenzthiazolinyl, benzoisothiazolyl, oxobenzoisothiazolinyl, benzoimidazolyl, oxobenzoimidazolinyl, indazolyl, oxoindazolinyl, benzofurazanyl, benzothiadiazolyl, benzotriazolyl, oxazolopyridyl, oxodihydrooxazolopyridyl, thiazolopyridyl, oxodihydrothiazolopyridyl, isothiazolopyridyl, imidazopyridyl, oxodihydroimidazopyridyl, pyrazolopyridyl, oxodihydropyrazolopyridyl, thienopyrimidinyl, chromanyl, chromanonyl, benzopyranyl, chromonyl, quinoloyl, isoquinoloyl, dihydroquinolyl, oxodihydroquinolinyl, tetrahydroquinolyl, oxotetrahydroquinolinyl, benzodioxanyl, quinoxalinyl, quinazolinyl, naphthyridinyl, carbazolyl, tetrahydrocarbazolyl, oxotetrahydrocarbazolyl, pyridoindolyl, acridinyl, oxodihydroacridinyl, phenanthridinyl, dihydrophenanthridinyl, oxodihydrophenanthridinyl, dibenzoisoquinolinyl, dihydrodibenzoisoquinolinyl, oxodihydrodibenzoisoquinolinyl, phenothiazinyl, dihydrodibenzooxepinyl, oxodihydrodibenzooxepinyl, benzocycloheptathienyl, oxobenzocycloheptathienyl, dihydrothienobenzothiepinyl, oxodihydrothienobenzothiepinyl, dihydrodibenzothiepinyl, oxodihydrodibenzothiepinyl, octahydrodibenzothiepinyl, dibenzoazepinyl, dihydrodibenzoazepinyl, oxodihydrodibenzoazepinyl, octahydrodibenzoazepinyl, benzocycloheptapyridyl, oxobenzocycloheptapyridyl, pyridobenzoazepinyl, dihydropyridobenzoazepinyl, oxodihydropyridobenzoazepinyl, dihydropyridobenzodiazepinyl, dihydrodibenzooxazepinyl, dihydropyridobenzooxepinyl, dihydropyridobenzooxazepinyl, oxodihydropyridobenzooxazepinyl, dihydrodibenzothiazepinyl, oxodihydrodibenzothiazepinyl, dihydropyridobenzothiazepinyl and oxodihydropyridobenzothiazepinyl, bound directly or over a methylene group;
- **R**^{**14**}: has the same meaning as **R**^{**13**} but is selected independently thereof;
- **R**^{**15**}: is selected from
hydroxy, methyl, benzyl, phenyl, indanyl, indenyl, naphthyl, dihydronaphthyl, tetrahydronaphthyl, furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, triazinyl, benzofuryl, benzothienyl, indolyl, indolinyl, benzooxazolyl, benzothiazolyl, benzoimidazolyl, chromanyl, quinolyl and tetrahydroquinolyl, bound directly or over a methylene group;
- **G2**: is selected from 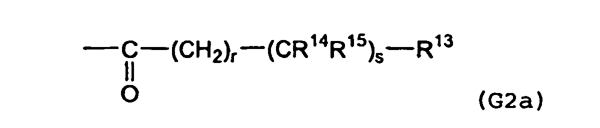 and 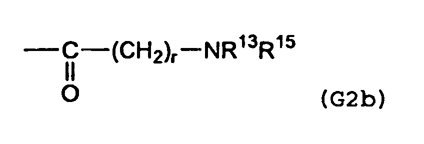 wherein **r, s** and the substituents **R**^{**13**} to **R**^{**15**} have the above meaning, or the group

―NR¹³R¹⁵

represents azetidine, pyrrolidine, piperidine, (1H)-tetrahydropyridine, hexahydroazepine, (1H)-tetrahydroazepine, octahydroazocine, pyrazolidine, piperazine, hexahydrodiazepine, morpholine, hexahydrooxazepine, thiomorpholine, thiomorpholin-1,1-dioxide, of 5-aza-bicyclo-[2.1.1]hexane, 2-aza-bicyclo[2.2.1]heptane, 7-aza-bicyclo-[2.2.1]heptane, 2,5-diaza-bicyclo[2.2.1]heptane, 2-aza-bicyclo[2.2.2]octane, 8-aza-bicyclo[3.2.1]octane, 2,5-diaza-bicyclo[2.2.2]octane, 9-aza-bicyclo[3.3.1]nonane, indoline, isoindoline, (1H)-dihydroquinoline, (1H)-tetrahydroquinolin, (2H)-tetrahydroisoquinoline, (1H)-tetrahydroquinoxaline, (4H)-dihydrobenzooxazine, (4H)-dihydrobenzothiazine, (1H)-tetrahydrobenzo[b]azepine, (1H)-tetrahydrobenzo[c]azepine, (1H)-tetrahydrobenzo[d]azepine, (5H)-tetrahydrobenzo[b]ox-azepine, (5H)-tetrahydrobenzo[b]thiazepine, 1,2,3,4-tetra-hydro-9H-pyrido[3,4-b]indole, (10H)-dihydroacridine, (10H)-dihydrophenanthridine, 1,2,3,4-tetrahydroacridanone, (10H)-phenoxazine, (10H)-phenothiazine, (5H)-dibenzoazepine, (5H)-dihydrodibenzoazepine, (5H)-octahydrodibenzoazepine, dihydrobenzo[d,e]isoquinoline, (5H)-dihydrodibenzodiazepine, (5H)-benzo[b]pyrido-[f]azepine, (5H)-Dihydrobenzo[b]pyri-do[f]azepine, (11H)-Dihydrodibenzo[b,e]oxazepine, (11H)-dihydrodibenzo[b,e]thiazepine, (10H)-dihydrodibenzo[b,f]-oxazepine, (10H)-dihydrodibenzo[b,f]thiazepine, (5H)-tetra-hydrodibenzoazocine, (11H)-dihydrobenzo[e]pyrido[b]-1,4-diazepin-6-one or (11H)-Dihydrobenzo[b]pyrido[e]-1,4-diazepin-5-one,
- **G3**: represents

―SO₂―(CH₂)ᵣ―R¹³,

wherein **r** and **R**^{**13**} have the above meaning,
- **G4**: represents 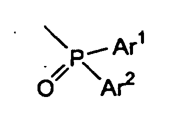 wherein
- **Ar**^{**1**} and **Ar**^{**2**}: are selected independently from each other from phenyl, pyridyl and naphthyl;
- **G5**: represents

―COR¹⁶,

wherein
- **R**^{**16**}: is selected from
trifluoromethyl, C₁-C₆-alkoxy, C₃-C₆-alkenyloxy, and benzyloxy,
wherein aromatic ring systems in the substituents optionally are substituted independently from each other by one to three of the same or different groups selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide in the case of two adjacent residues on the aromatic ring, and
wherein alkyl-, alkenyl- and cycloalkyl residues in the groups **G1, G2** and **G3** optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl-amino).

According to a further very preferred embodiment, the invention relates to compounds of formula (I), wherein
- **R**^{**1**}: is selected from
hydrogen, fluorine, chlorine, bromine, methyl, ethyl, trifluoromethyl, hydroxy, C₁-C₄-alkoxy, methylthio, ethylthio, carboxy and phenoxy;
- **R**^{**2**}: is selected from hydrogen, chlorine and methyl;
- **R**^{**3**}: is hydrogen;
- **R**^{**4**}: is selected from
hydrogen, C₁-C₃-alkyl and hydroxy,
- **k**: is 0
- **A**: is selected from
C₂-C₆-alkylene, which is optionally substituted once or twice by hydroxy or fluorine;
C₂-C₆-alkylene, wherein a methylene unit is isosterically replaced by O, S or CO, wherein, with the exception of CO, the isosteric substitution is not adjacent to the amide group;
C₂-C₆-alkenylene which is optionally substituted by C₁-C₃-alkyl and/or fluorine; and
C₄-C₆-alkadienylene;
- **D**: is selected from
C₂-C₈-alkylene which is optionally substituted by methyl or hydroxy;
C₄-C₈-alkenylene, which is optionally substituted by hydroxy;
C₄-C₈-alkinylene, which is optionally substituted by hydroxy; and
the group consisting of C₂-C₈-alkylene, C₄-C₈-alkenylene, and C₄-C₈-alkinylene wherein a methylene unit is isosterically replaced by O, NH, N(CH₃), CO or SO₂ or an ethylene group is isosterically replaced by a group NH-CO and/or CO-NH or a propylene group is isosterically replaced by a group NH-CO-O and/or O-CO-NH;
- **E**: is selected from piperazine and hexahydro-1,4-diazepine, wherein the ring optionally is substituted by one or two methylene groups and/or by an oxo group adjacent to a nitrogen atom;
- **G**: is selected from hydrogen, C₃-C₈-cycloalkyl, methoxycarbonyl, tert-butoxycarbonyl, benzyloxycarbonyl, trifluoroacetyl, diphenylphosphinoyl,

―(CH₂)ᵣ―(CR¹⁴R¹⁵)ₛ―R¹³,

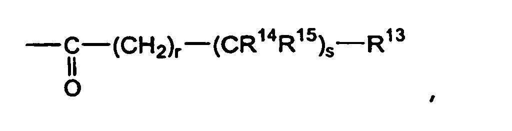

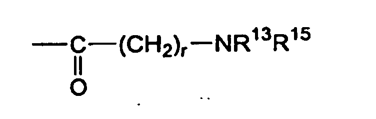

and

―SO₂―(CH₂)ᵣ―R¹³

wherein
- **r**: is 0 or 1
- **s**: is 0 or 1,
- **R**^{**13**}: is selected from hydrogen, methyl, benzyl, phenyl,
the group consisting of indanyl, indenyl, oxoindanyl, naphthyl, tetrahydronaphthyl, fluorenyl, oxofluorenyl, anthryl, dihydroanthryl, oxodihydroanthryl, dioxodihydroanthryl, phenanthryl, dihydrophenanthryl, oxydihydrophenanthryl, dibenzocycloheptenyl, dihydrodibenzocycloheptenyl, and oxodihydrodibenzocycloheptyl, bound directly or over a methylene group, and
the group consisting of furyl, thienyl, oxazolyl, isooxazolyl, thiazolyl, imidazolyl, oxadiazolyl, pyridyl, pyrazinyl, pyrimidinyl, imidazothiazolyl, benzofuryl, benzothienyl, indolyl, indolinyl, oxoindolinyl, dioxoindolinyl, benzoxazolyl, oxobenzooxazolinyl, benzothiazolyl, oxobenzthiazolinyl, benzimidazolyl, oxobenzimidazolinyl, indazolyl, benzofurazanyl, benzotriazolyl, oxazolopyridyl, oxodihydrooxazolopyridyl, thiazolopyridyl, oxodihydrothiazolopyridyl, imidazopyridyl, oxodihydroimidazopyridyl, chromanyl, chromanonyl, benzopyranyl, chromonyl, quinolyl, isoquinolyl, oxodihydroquinolinyl, tetrahydroquinolyl, oxotetrahydroquinolinyl, benzodioxanyl, quinazolinyl, carbazolyl, acridinyl, dihydroacridinyl, oxodihydroacridinyl, dihydrophenanthridinyl dihydrobenzoisoquinolinyl, phenothiazinyl, dihydrodibenzooxepinyl, benzocycloheptathienyl, dihydrothienobenzothiepinyl, dihydrodibenzothiepinyl, oxodihydrodibenzothiepinyl, dihydrodibenzoazepinyl, oxodihydrodibenzoazepinyl, octahydrodibenzoazepinyl, benzocycloheptapyridyl, dihydropyridobenzodiazepinyl, and dihydrodibenzothiazepinyl, bound directly or over a methylene group,
- **R**^{**14**}: is selected from hydrogen, methyl, benzyl, and phenyl,
- **R**^{**15**}: is selected from
hydrogen, hydroxy, methyl, benzyl, phenyl; and
the group consisting of naphthyl, tetrahydronaphthyl, furyl, thienyl, oxazolyl, thiazolyl, imidazolyl, pyridyl, benzofuryl, benzothienyl, indolyl, indolinyl, benzoxazolyl, benzothiazolyl, benzimidazolyl, chromanyl, quinolyl and tetrahydroquinolyl, bound directly or over a methylene group;
wherein the group

―NR¹³R¹⁵

represents a nitrogen-containing heterocycle bound over the nitrogen atom which nitrogen-containing heterocycle is selected from
pyrrolidine, piperidine, hexahydroazepine, piperazine, hexahydrodiazepine, morpholine, hexahydroxazepine, thiomorpholine, 7-aza-bicyclo[2,2.1]heptane, 2,5-diaza-bicyclo[2.2.1]heptane, indoline, isoindoline, (1H)-dihydroquinoline, (1H)-tetrahydroquinoline, (2H)-tetrahydroisoquinoline, (4H)-dihydrobenzoxazine, (4H)-dihydrobenzothiazine, (1H)-tetrahydrobenzo[b]azepine, (1H)-tetrahydrobenzo[d]azepine, (5H)-tetrahydrobenzo[b]oxazepine, (5H)-tetrahydrobenzo[b]thiazepine, (10H)-dihydroacridine, 1,2,3,4-tetrahydroacridanone, (10H)-dihydrophenanthridine, (1H)-dihydrobenzo-[d,e]isoquinoline, (10H)-phenothiazine, (5H)-dibenzo[b,f]azepine, (5H)-dihydrodibenzo[b,f]azepine, (5H)-dihydrodibenzo[c,e]azepine, (5H)-dihydrodibenzo-diazepine, (11H)-dihydrodibenzo[b,e]oxazepine (11H)-dihydrodibenzo[b,e]thiazepine, (5H)-dihydrobenzo[b]pyrido[3,2-f]azepine and (11H)-6-oxodihydrobenzo[e]pyrido[3,2-b][1,4]diazepine,
wherein aromatic ring systems in the substituents optionally are substituted, independently of each other, by one to three of the same or different groups selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carbocyalkenyl, C₂-C₇-alkoxycarbonyl, benzyloxycarbonyl, nitro, amino, mono-C₁-C₆-alkylamino, di-(C₁-C₆-alkyl)-amino, and in the case of two adjacent residues on the aromatic ring, methylenedioxy, and
wherein alkyl, alkenyl and cycloalkyl residues in the group G optionally are substituted by one or two of the same or different groups which are selected from hydroxy, carboxy, C₂-C₇-alkoxycarbonyl, benzyloxycarbonyl, amino, mono-C₁-C₆-alkylamino and di-(C₁-C₆-alkyl) amino.

Furthermore, according to a particularly preferred embodiment, the invention relates to compounds of formula (I), wherein
- **R**^{**1**}: is selected from
hydrogen, fluorine, methyl, trifluoromethyl and ethylthio;
- **R**^{**2**}, **R**^{**3**} and **R**^{**4**}: are each hydrogen;
- **k**: is 0,
- **A**: is selected from
the group consisting of ethylene, propylene and butylene, which are each optionally substituted by hydroxy or one or two fluorine atoms;
OCH₂, SCH_{2;}
ethenylene, and 1,3-butadienylene;
- **D**: is selected from
C₂-C₆-alkylene which is optionally substituted by hydroxy;
C₄-C₆ alkenylene;
C₄-C₆ alkinylene; and
the group consisting of C₂-C₆ alkylene, C₄-C₆ alkenylene and C₄-C₆ alkinylene, wherein one or two methylene units are isosterically replaced by O, NH, CO or SO₂;
- **E**: is piperazine or hexahydro-1,4-diazepine;
- **G**: is selected from
phenyl, benzyl, phenethyl, diphenylmethyl, naphthyl, tetrahydronaphtyl, naphthylmethyl, fluorenyl fluorenylmethyl, anthrylmethyl, dihydrodibenzocycloheptenyl, furylmethyl, thienylmethyl, thiazolylmethyl, pyridylmethyl, benzothienylmethyl, quinolylmethyl, phenylthienylmethyl, phenylpyridylmethyl, benzocycloheptapyridinyl, dihydrobenzocycloheptapyridinyl, dihydrodibenzooxepinyl, dihydrodibenzothiepinyl, dihydrodibenzoazepinyl, dihdrobenzopyridodiazepinyl, formyl, acetyl, pivaloyl, phenylacetyl, diphenylacetyl, diphenylpropionyl, naphthylacetyl, benzoyl, naphthoyl, oxofluorenylcarbonyl, oxodihydroanthrylcarbonyl, dioxodihydroanthrylcarbonyl, furoyl, pyridylacetyl, pyridylcarbonyl, chromonylcarbonyl, quinolylcarbonyl, phenylylaminocarbonyl, naphthylaminocarbonyl, tetrahydronaphthylaminocarbonyl, dibenzylaminocarbonyl, benzylphenylaminocarbonyl, diphenylaminocarbonyl, indolinyl-N-carbonyl, isoindolinyl-N-carbonyl, tetrahydroquinolinyl-N-carbonyl, carbazolyl-N-carbonyl, tetrahydrobenzoazepinyl-N-carbonyl, dihydrodibenzoazepinyl-N-carbonyl, dihydrobenzopyridoazepinyl-N-carbonyl, oxodihydrobenzopyridoazepinyl-N-carbonyl, methanesulfonyl, toluenesulfonyl, naphthylsulfonyl, quinolinsulfonyl and diphenylphosphinoyl,
wherein aromatic ring systems optionally are substituted independently of each other by one to three of the same or different groups which are selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and in the case of two adjacent residues in the aromatic ring methylenedioxy, and wherein
alkyl, alkenyl and cycloalkyl residues in the group **G** optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl)-amino.

The following compounds represent very particular preferred embodiments of the invention:
N-[4-(4-diphenylmethylpiperazin-1-yl)-3-hydroxybutyl]-3-pyridin-3-yl-acrylamide;
N-[3-(4-diphenylmethylpiperazin-1-yl)-propoxy]-3-pyridin-3-yl-acrylamide;
N-[4-(4-diphenylmethylpiperazin-1-yl)-4-oxo-butyl]-3-pyridin-3-yl-acrylamide;
N-[3-(4-diphenylmethylpiperazin-1-sulfonyl)-propyl]-3-pyridin-3-yl-acrylamide;
N-{2-[2-(4-diphenylmethylpiperazin-1-yl)-ethoxy]-ethyl}-3-pyridin-3-yl-acrylamide;
N-(4-{4-[bis-(4-fluorophenyl)-methyl]-piperazin-1-yl}-but-2-in-yl)-3-pyridin-3-yl-acrylamide;
N-{4-[4-(4-carboxyphenyl-phenylmethyl)-piperazin-1-yl]-butyl}-3-pyridin-3-yl-acrylamide;
N-(4-{4-[(4-aminophenyl)-phenylmethyl]-piperazin-1-yl}-butyl)-3-pyridin-3-yl-acrylamide;
N-{4-[4-(9H-fluoren-9-yl)-piperazin-1-yl]-butyl}-2-(pyridin-3-yloxy)-acetamide;
N-{5-[4-(9H-fluoren-9-yl)-piperazin-1-yl]-pentyl}-3-pyridin-3-yl-acrylamide;
N-{6-[4-(9H-fluoren-9-yl)-piperazin-1-yl]-hexyl}-3-pyridin-3-yl-acrylamide;
3-pyridin-3-yl-N-{4-[4-(1,2,3,4-tetrahydronaphthalen-1-yl)-piperazin-1-yl]-butyl}-acrylamide;
3-pyridin-3-yl-N-{4-[4-(5,6,7,8-tetrahydronaphthalen-1-yl)-piperazin-1-yl]-butyl}-acrylamide;
N-{4-[4-(naphthalen-1-yl)-piperazin-1-yl]-butyl}-3-pyridin-3-yl-acrylamide;
N-[4-(4-biphenyl-2-yl-piperazin-1-yl)-butyl]-3-pyridin-3-yl-propionamide;
N-[5-(4-biphenyl-2-yl-piperazin-1-yl)-pentyl]-3-pyridin-3-yl-acrylamide;
N-[6-(4-biphenyl-2-yl-piperazin-1-yl)-hexyl]-3-pyridin-3-yl-acrylamide;
N-[4-(4-biphenyl-2-yl-piperazin-1-yl)-butyl]-2-(pyridin-3-yloxy)-acetamide;
N-[4-(4-biphenyl-2-yl-piperazin-1-yl)-butyl]-5-(pyridin-3-yl)-penta-2,4-dienoic acid amide;
N-{4-[4-(10,11-dihydro-5H-dibenzo[a,d]cyclohepten-5-yl)-piperazin-1-yl]-butyl}-3-pyridin-3-yl-propionamide;
N-{5-[4-(10,11-dihydro-5H-dibenzo[a,d]cyclohepten-5-yl)-piperazin-1-yl]-pentyl}-3-pyridin-3-yl-acrylamide;
N-{6-[4-(10,11-dihydro-5H-dibenzo[a,d]cyclohepten-5-yl)-piperazin-1-yl]-hexyl}-3-pyridin-3-yl-acrylamide;
N-{4-[4-(10,11-dihydro-5H-dibenzo[a,d]cyclohepten-5-yl)-piperazin-1-yl]-butyl}-5-(pyridin-3-yl)-penta-2,4-dienoic acid amide;
N-{4-[4-(6,11-dihydro-dibenzo[b,e]oxepin-11-yl)-piperazin-1-yl]-butyl-3-pyridin-3-yl-propionamide;
N-{2-[4-(6,11-dihydrodibenzo[b,e]thiepin-11-yl)-piperazin-1-yl]-ethyl}-3-pyridin-3-yl-acrylamide;
N-[4-(4-diphenylacetyl-piperazin-1-yl)-butyl]-3-pyridin-3-yl-acrylamide;
N-[4-(4-benzoylpiperazin-1-yl)-butyl]-3-pyridin-3-yl-acrylamide;
N-{4-[4-(2-aminobenzoyl)-piperazin-1-yl]-butyl}-3-pyridin-3-yl-acrylamide;
N-{4-[4-(4-carboxybenzoyl)-piperazin-1-yl]-butyl}-3-pyridin-3-yl-acrylamide;
N-{4-[4-(biphenyl-2-carbonyl)-piperazin-1-yl]-butyl}-3-pyridin-3-yl-acrylamide;
N-{4-[4-(9-oxo-9H-fluoren-4-carbonyl)-piperazin-1-yl]-butyl}-3-pyridin-3-yl-acrylamide;
N-{4-[4-(furan-2-carbonyl)-piperazin-1-yl]-butyl}-3-pyridin-3-yl-acrylamide;
N-{4-[4-(naphthalen-1-yl-aminocarbonyl)-piperazin-1-yl]-butyl}-3-pyridin-3-yl-propionamide;
N-{4-[4-(diphenylaminocarbonyl)-piperazin-1-yl]-butyl}-3-pyridin-3-yl-acrylamide;
N-{4-[4-(naphthalen-2-sulfonyl)-piperazin-1-yl]-butyl}-3-pyridin-3-yl-acrylamide;
N-[4-(4-diphenylphosphinoyl-piperazin-1-yl)-butyl]-3-pyridin-3-yl-acrylamide;
N-[4-(4-biphenyl-2-yl-piperazin-1-yl)-butyl]-3-pyridin-3-yl-acrylamide;
N-{4-[4-(9H-fluoren-9-yl)-piperazin-1-yl]-butyl}-3-pyridin-3-yl-acrylamide; and
N-{4-[4-(10,11-dihydro-5H-dibenzo[a,d]cyclohepten-5-yl)-piperazin-1-yl]-butyl}-3-pyridin-3-yl-acrylamide.

Additionally the invention comprises any one of the above mentioned compounds of formula (I), which is present
as cis- and/or trans-isomer, E- and/or Z-isomer, pure isomer and/or mixture of isomers, as enantiomer and/or diastereomer, as racemic or non-racemic mixture, as endo/exo-isomer in case the ring system E is bicyclic,
as tautomer, when G is a heterocyclic aromatic ring or contains such in an anellated ring system, when this heterocyclic ring is substituted by free hydroxy-, mercapto- or amino groups,
as pharmacologically acceptable acid addition salt with inorganic or organic acids, such as hydrochlorides, hydrobromides, hydroiodides, sulfates, phosphates, acetates, benzoates, citrates, fumarates, gluconates, malates, maleates, methanesulfonates, lactates, oxalates, succinates, tartrates and/or tosylates, and/or
as hydrate or other solvate.

In the following a series of compounds with the respective specific substituent definitions are listed in Table 1 without any limitation for further illustration of the compounds of the invention.

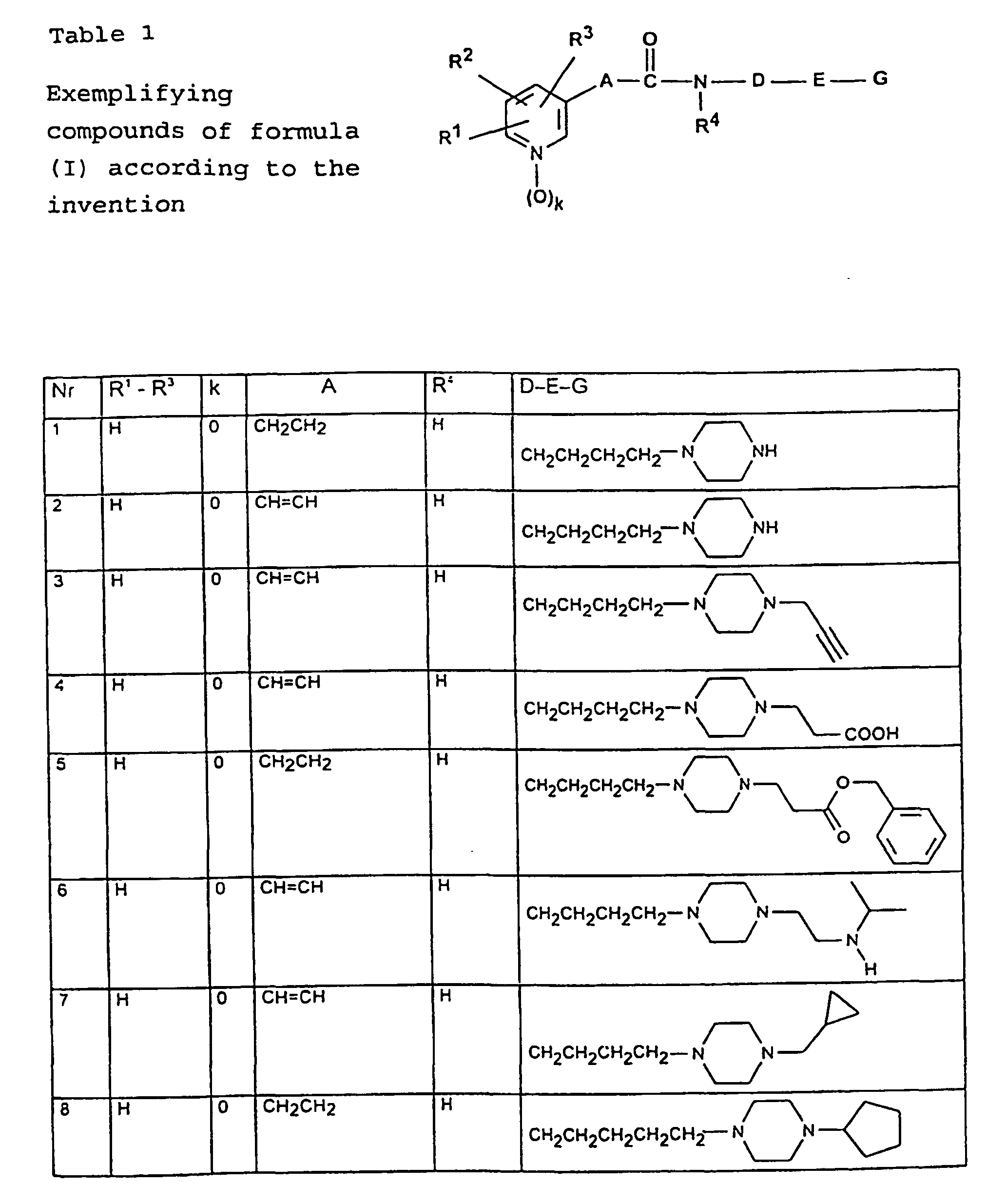

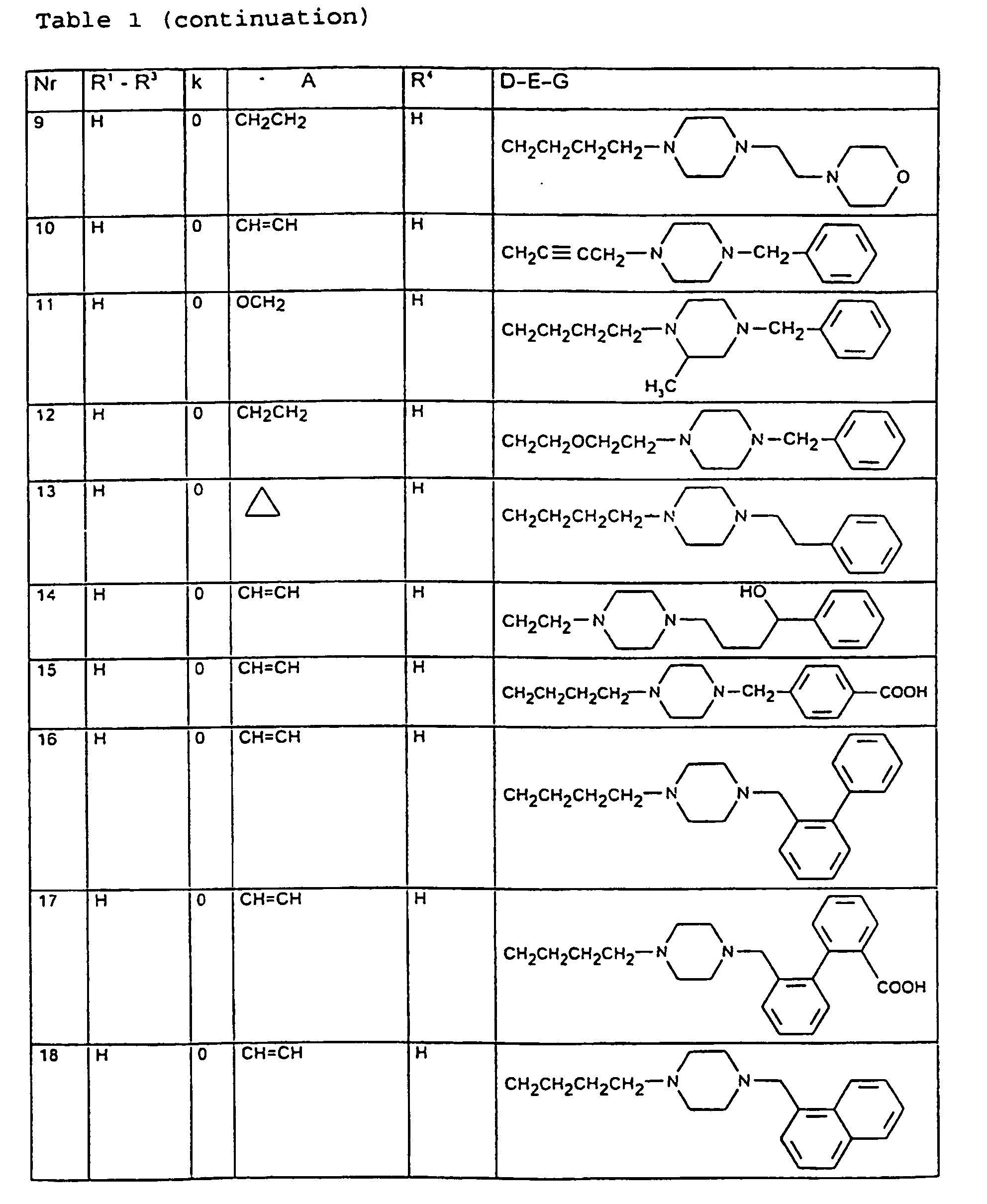

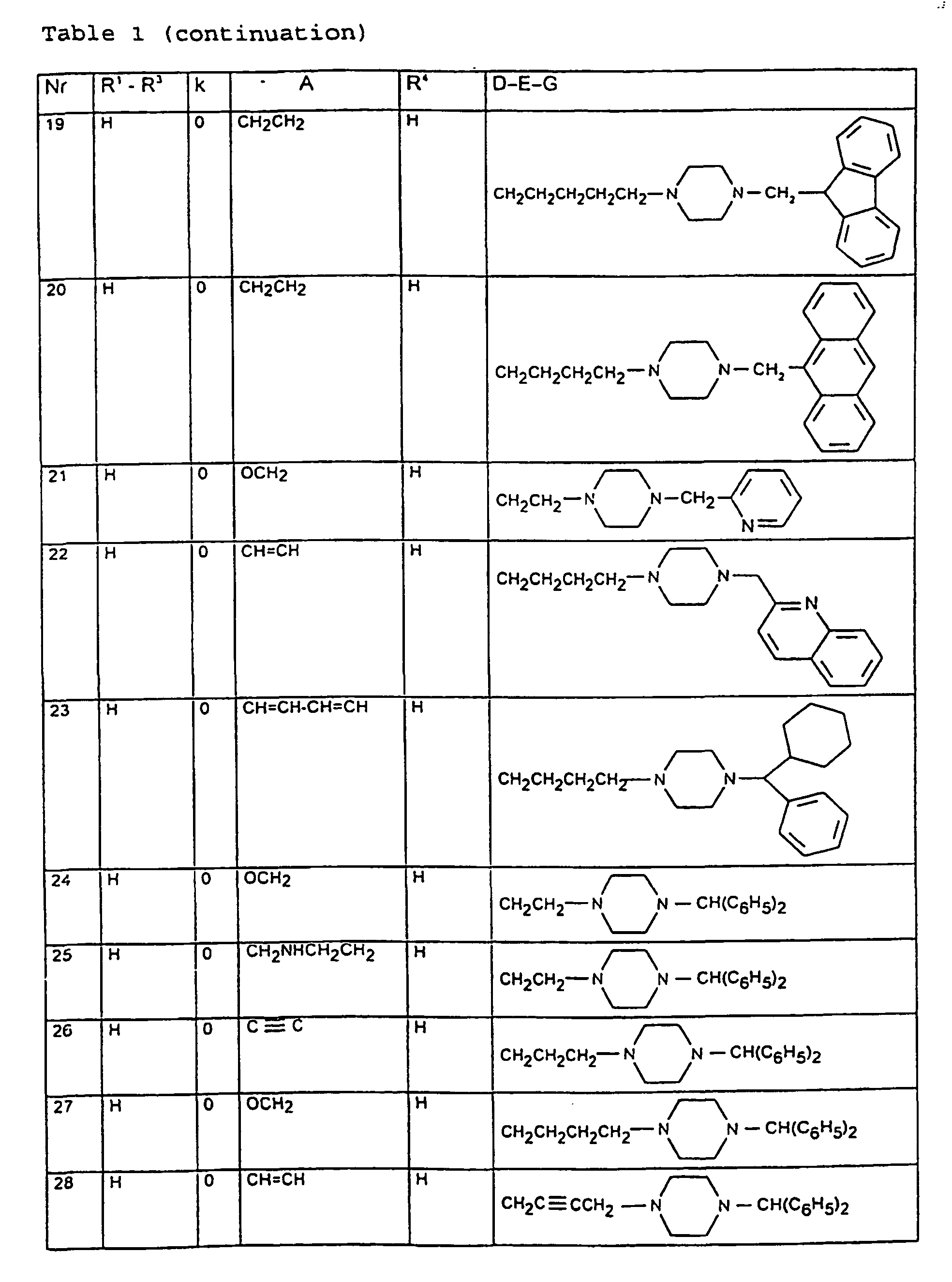

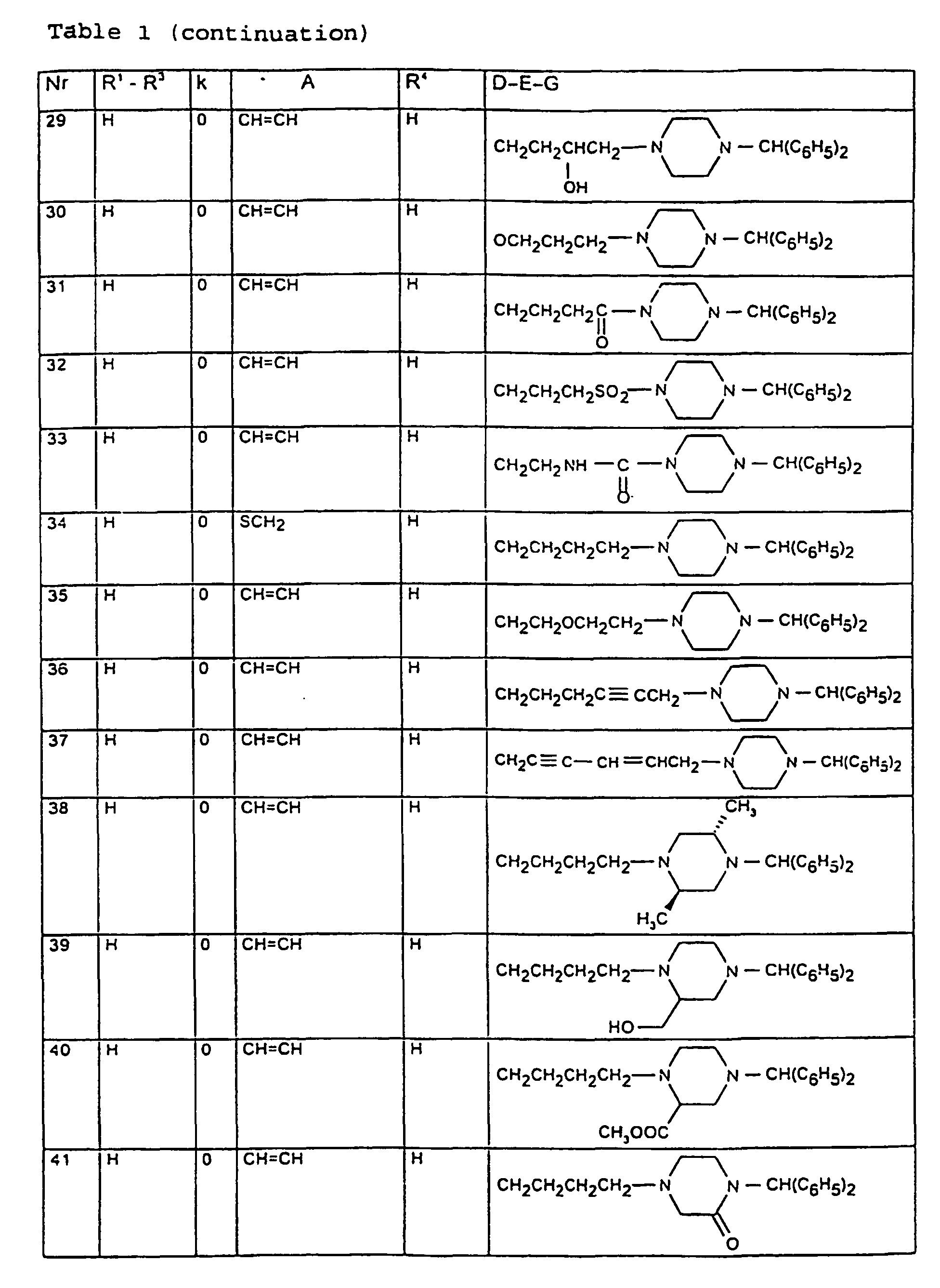

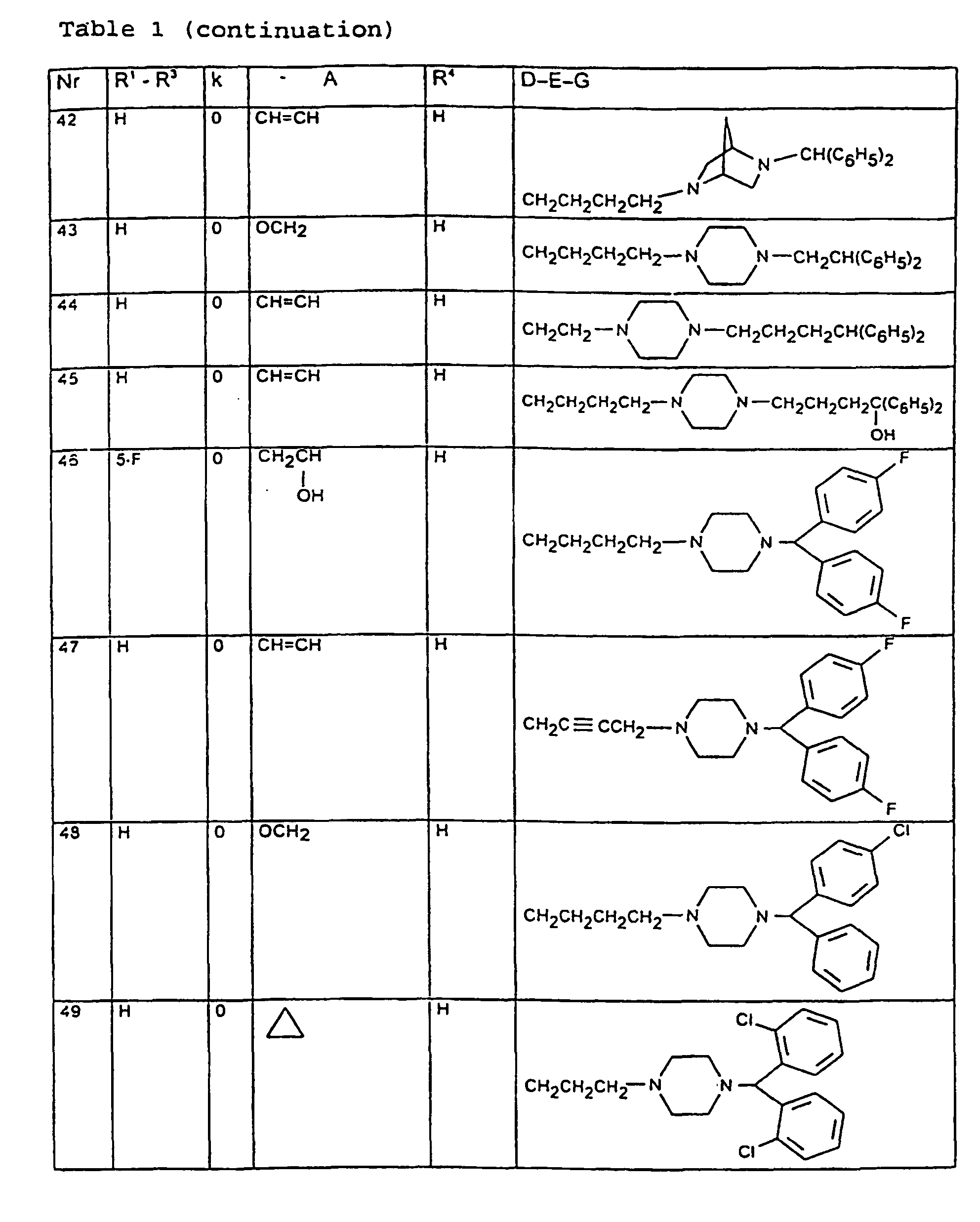

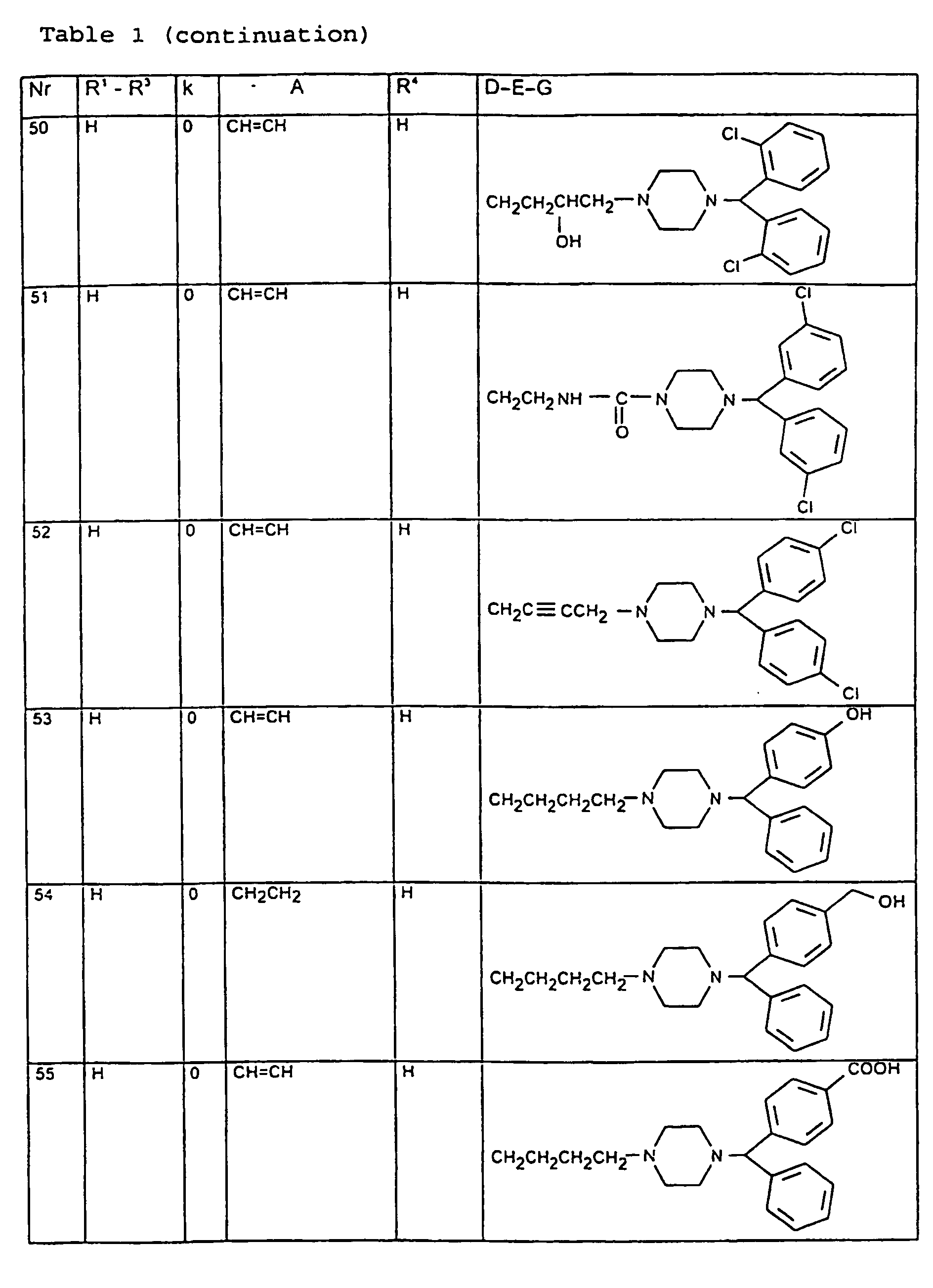

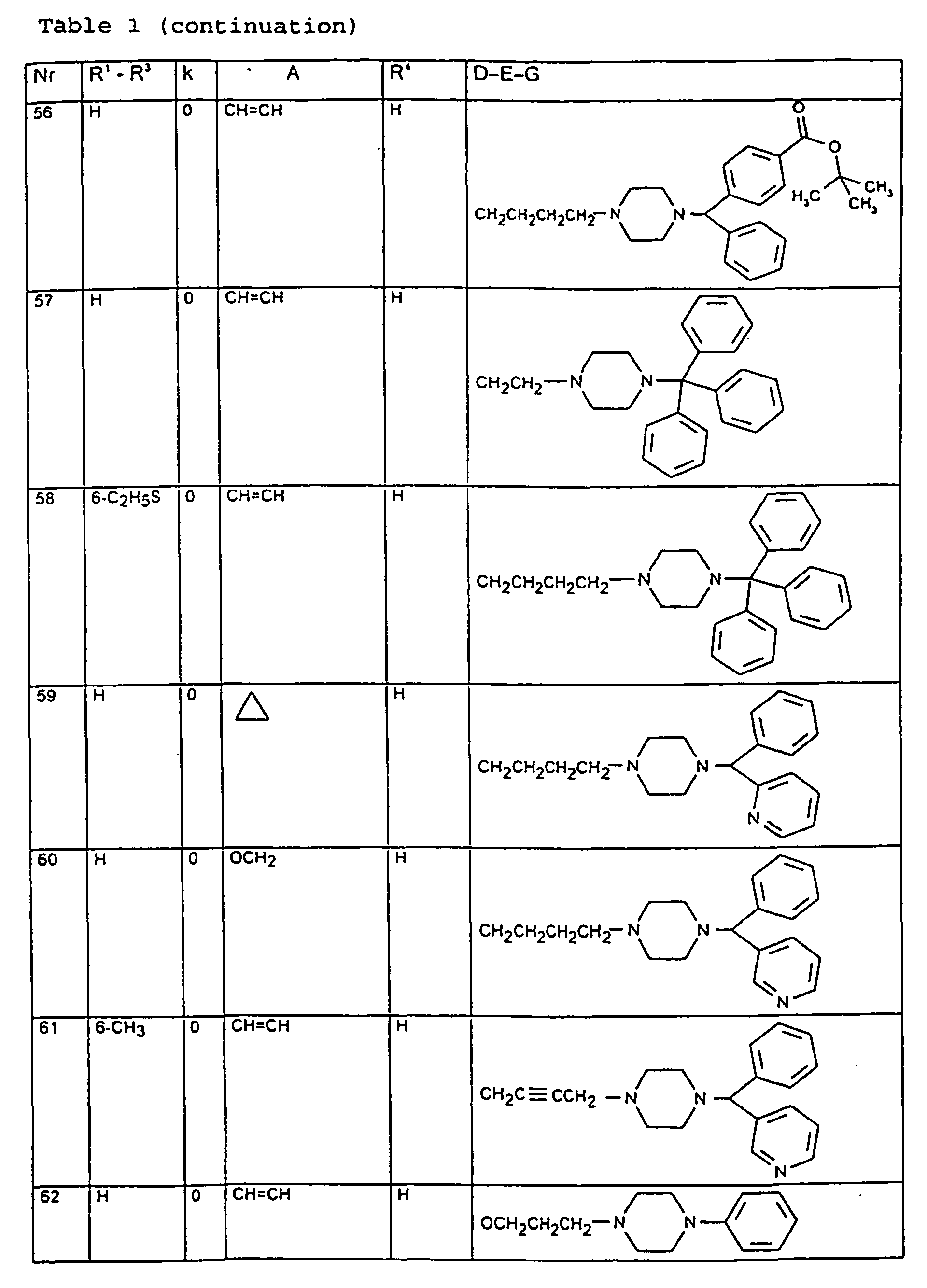

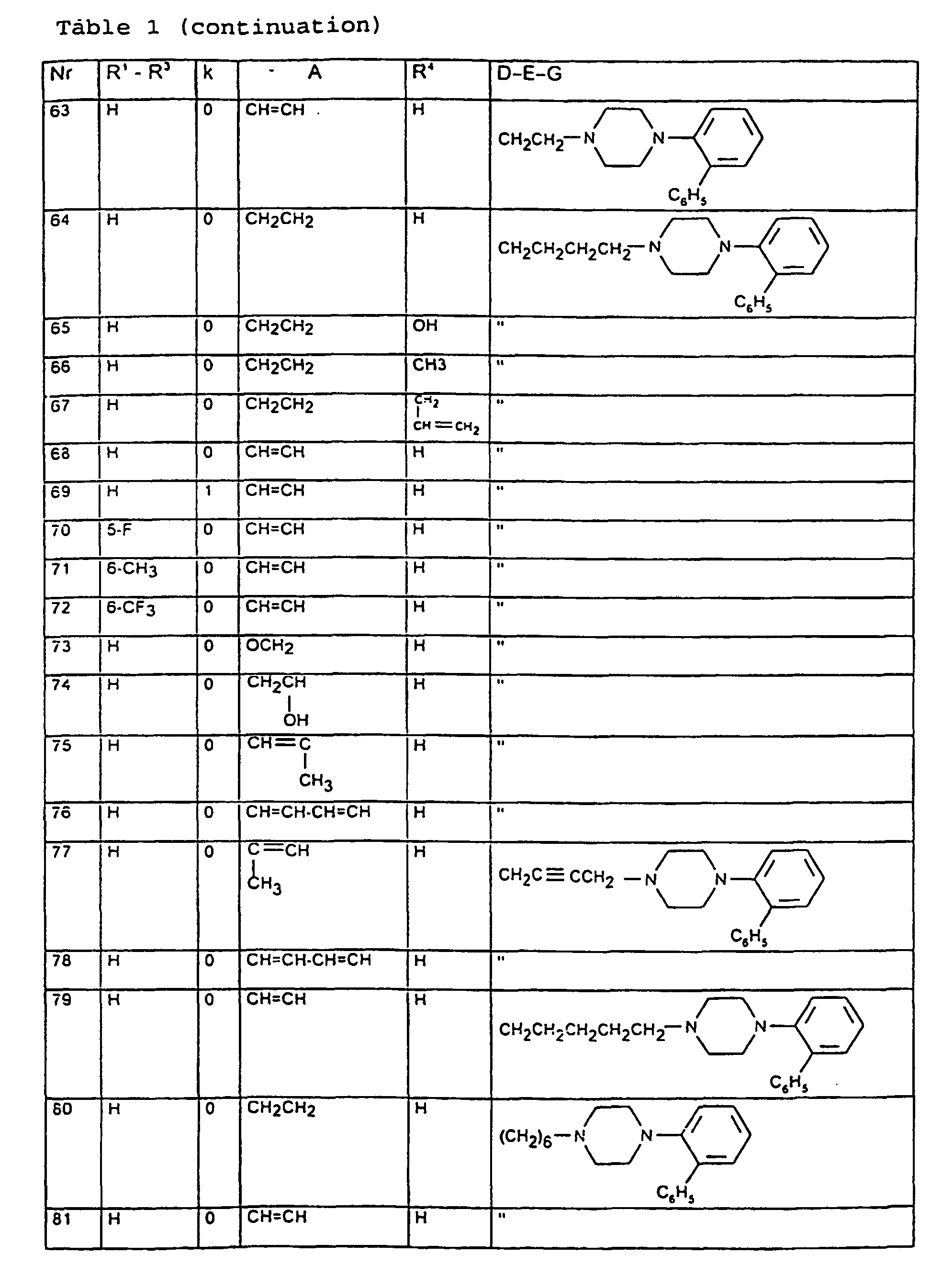

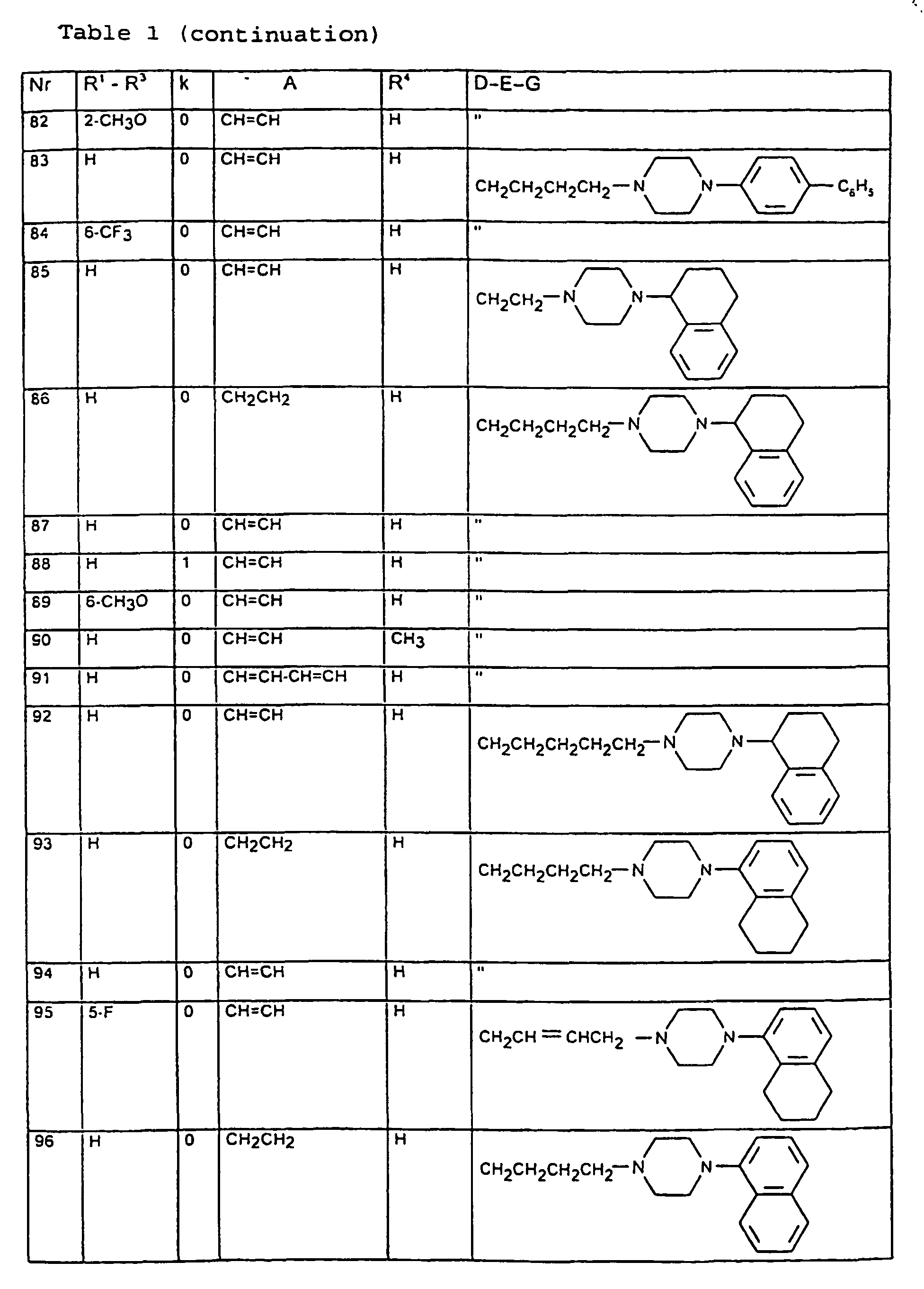

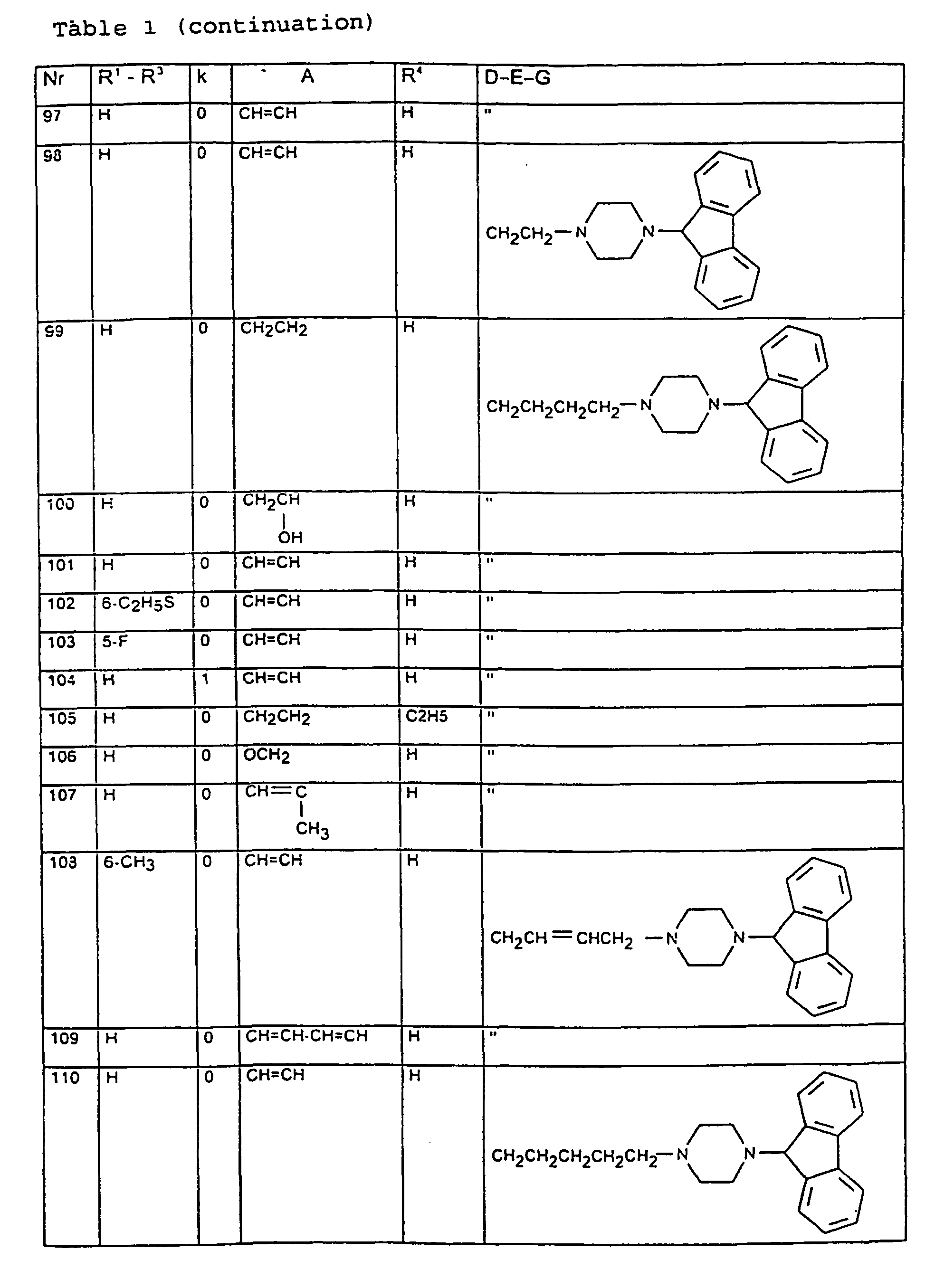

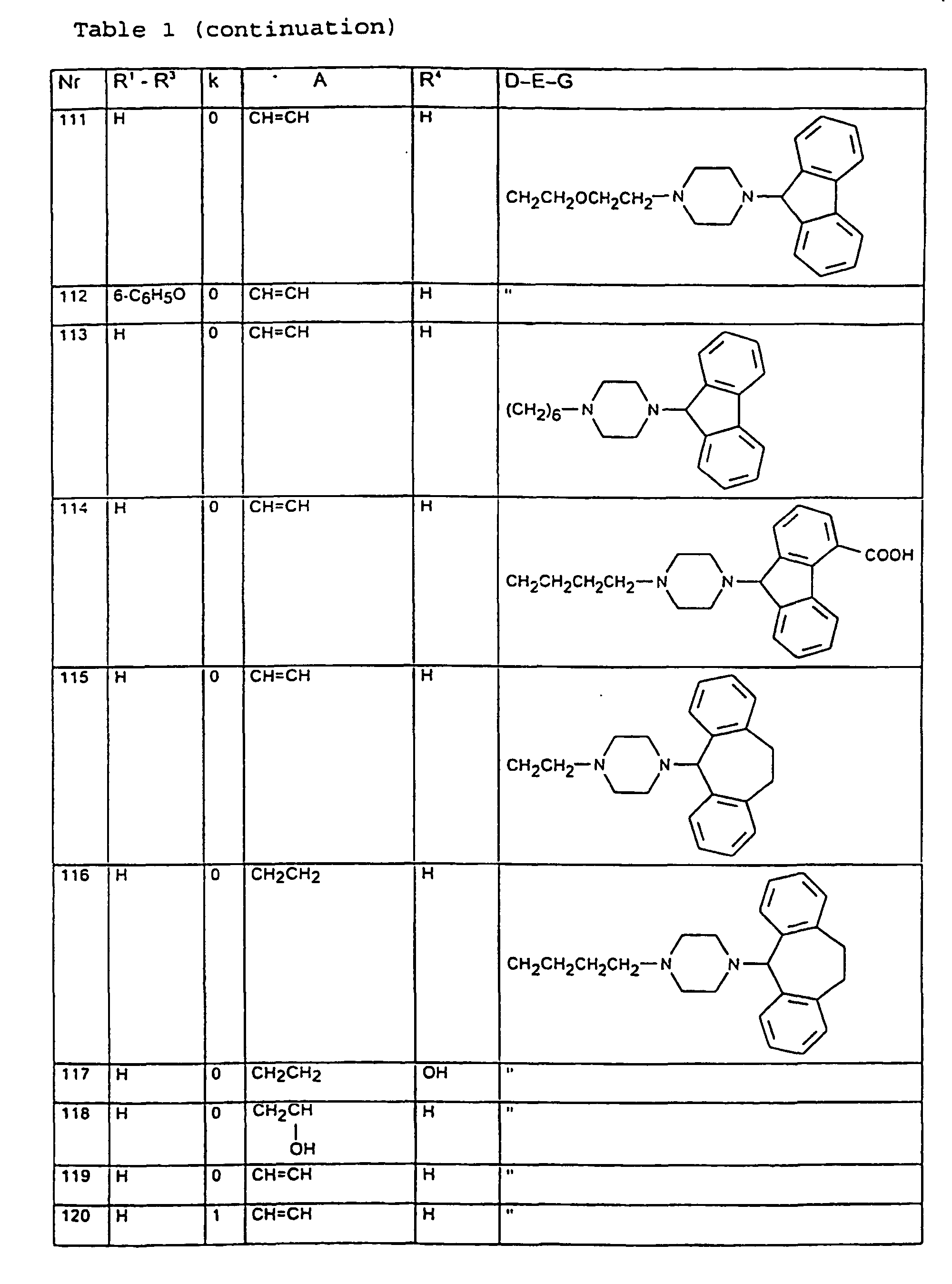

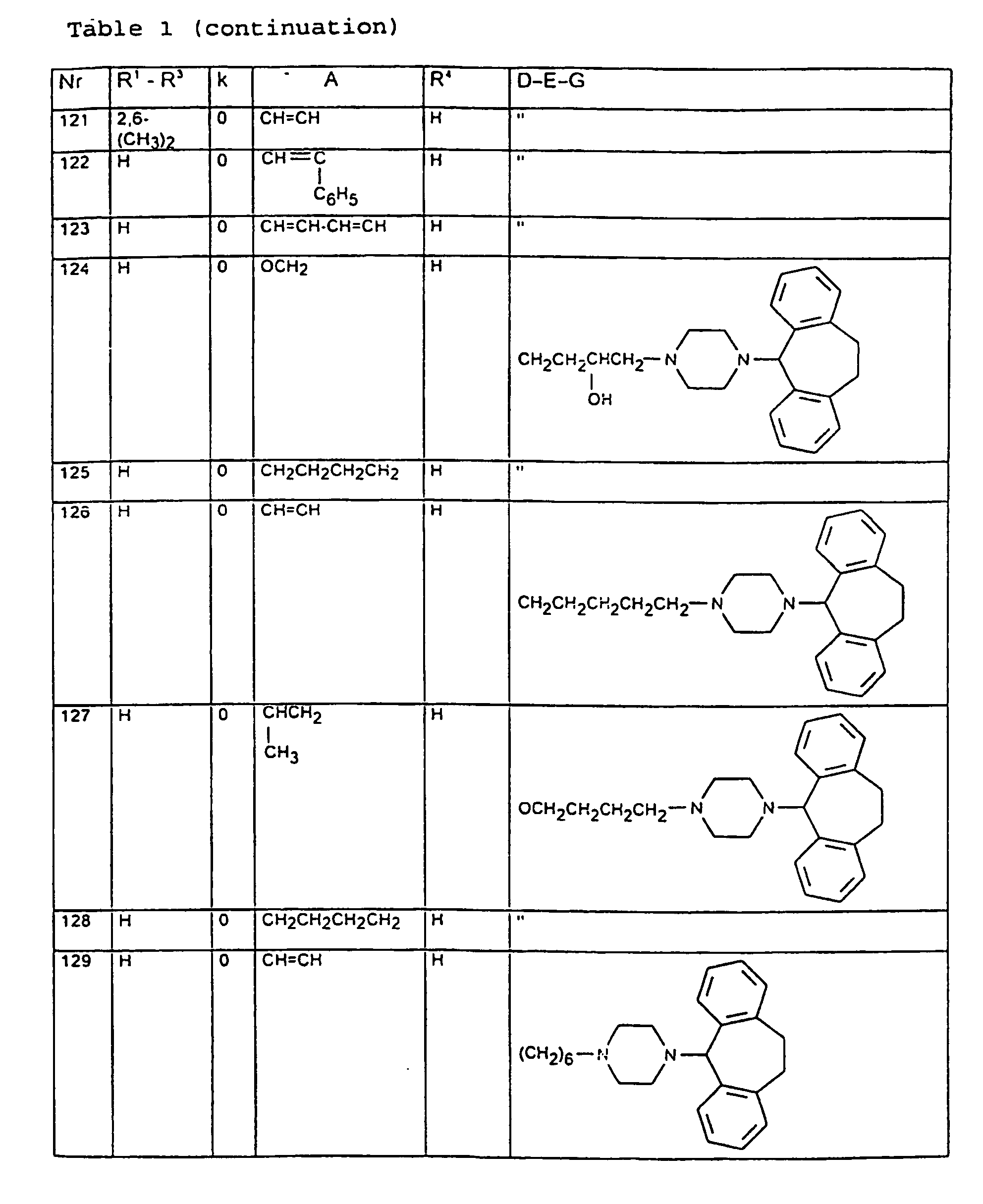

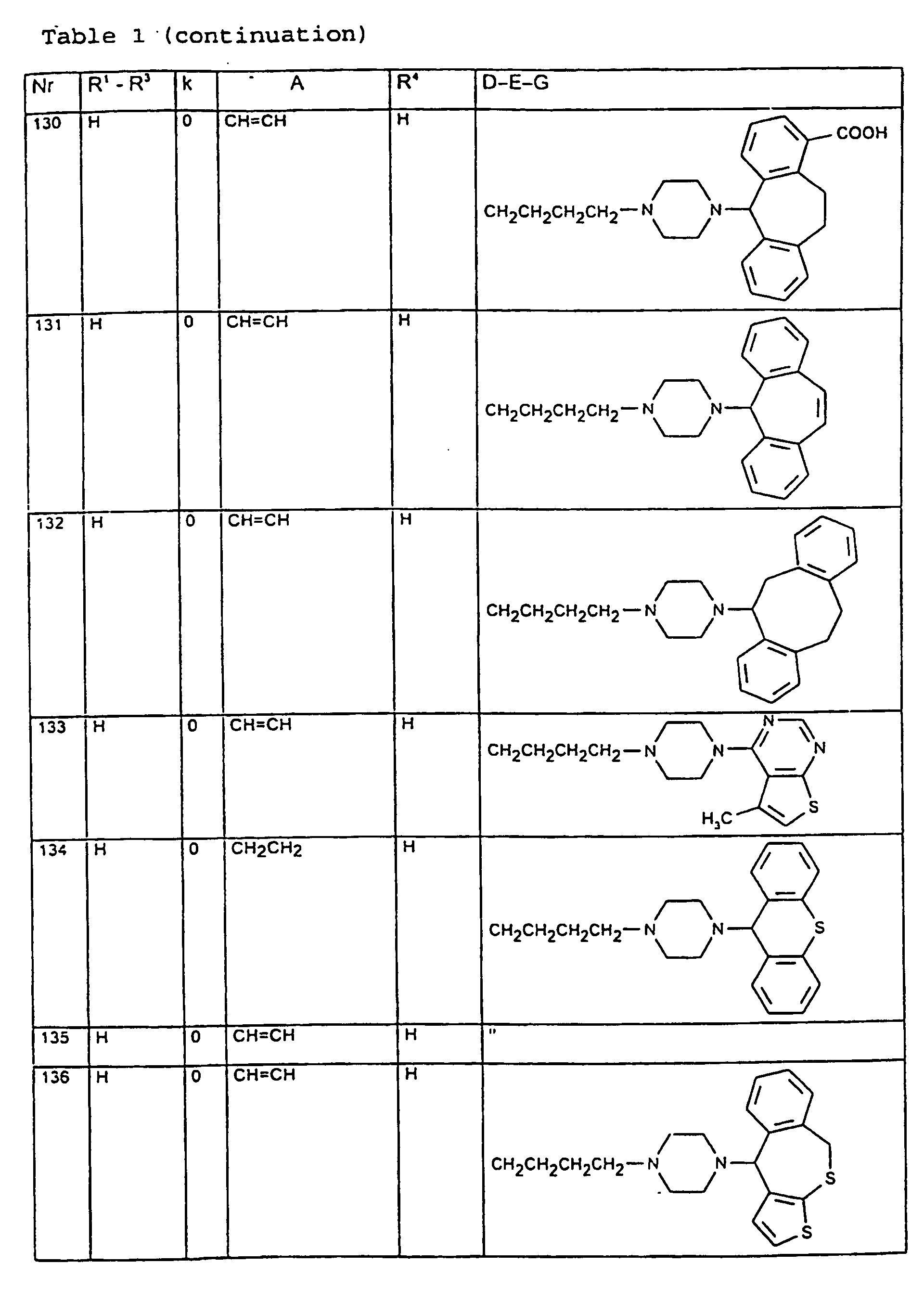

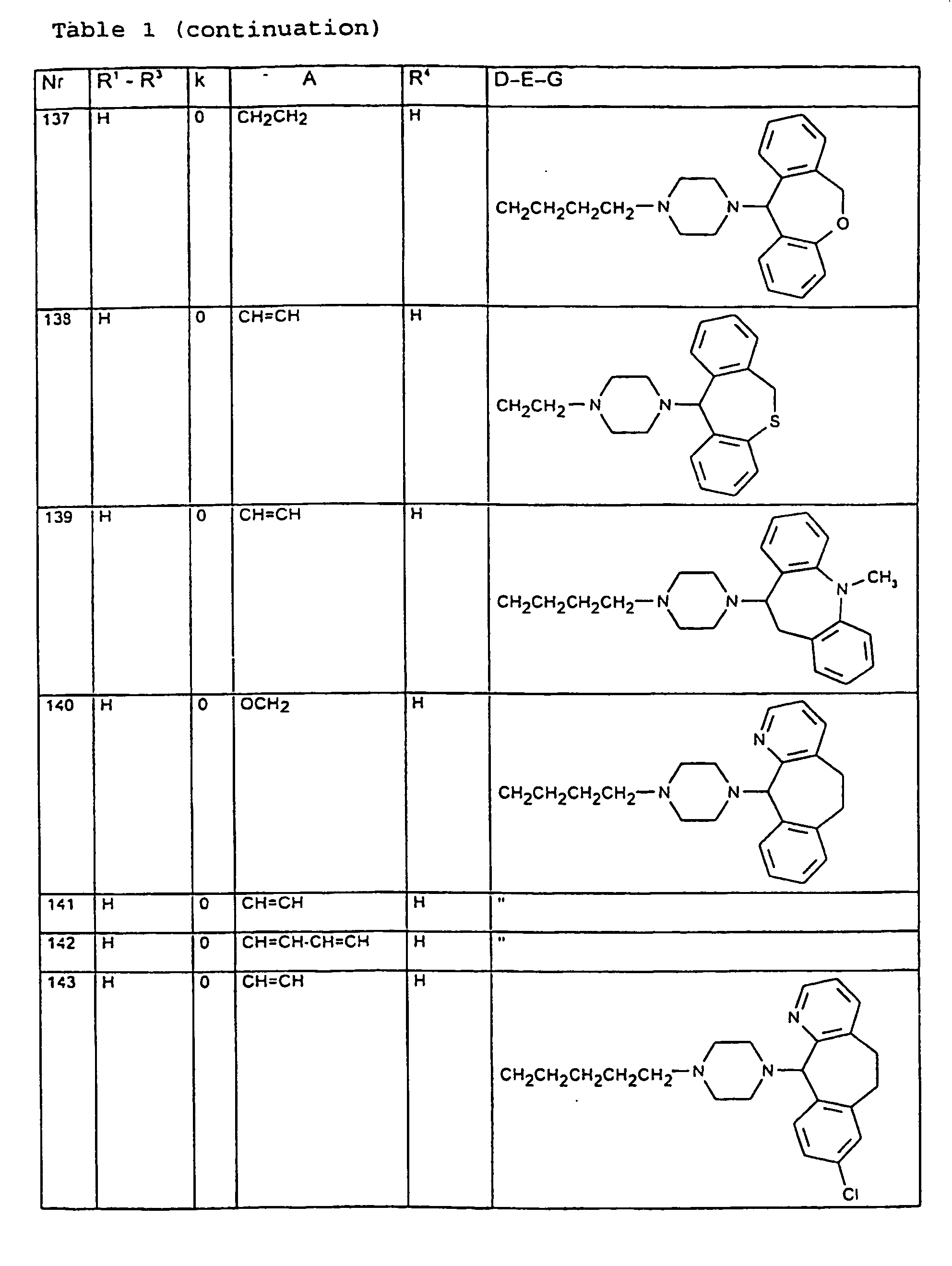

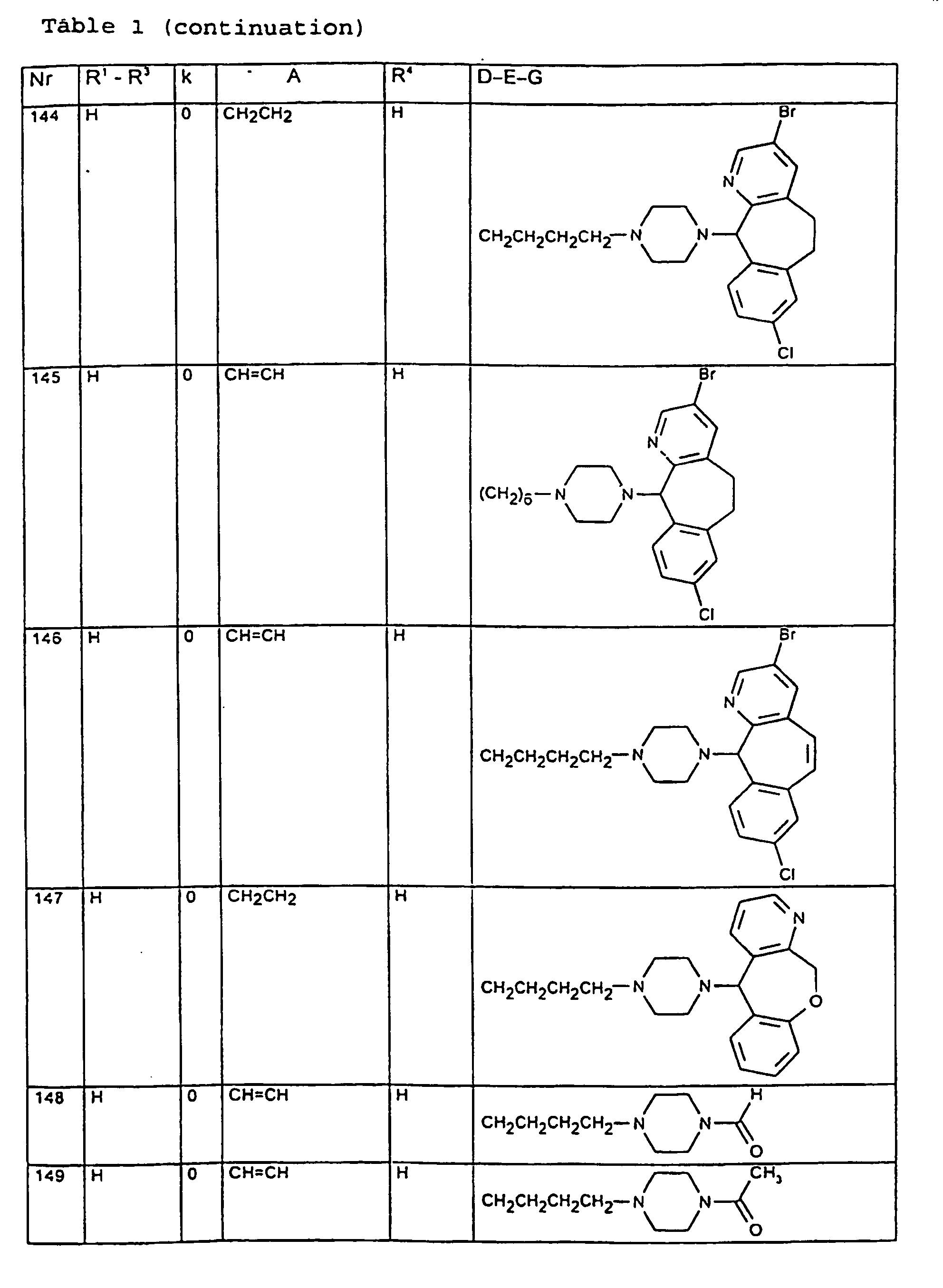

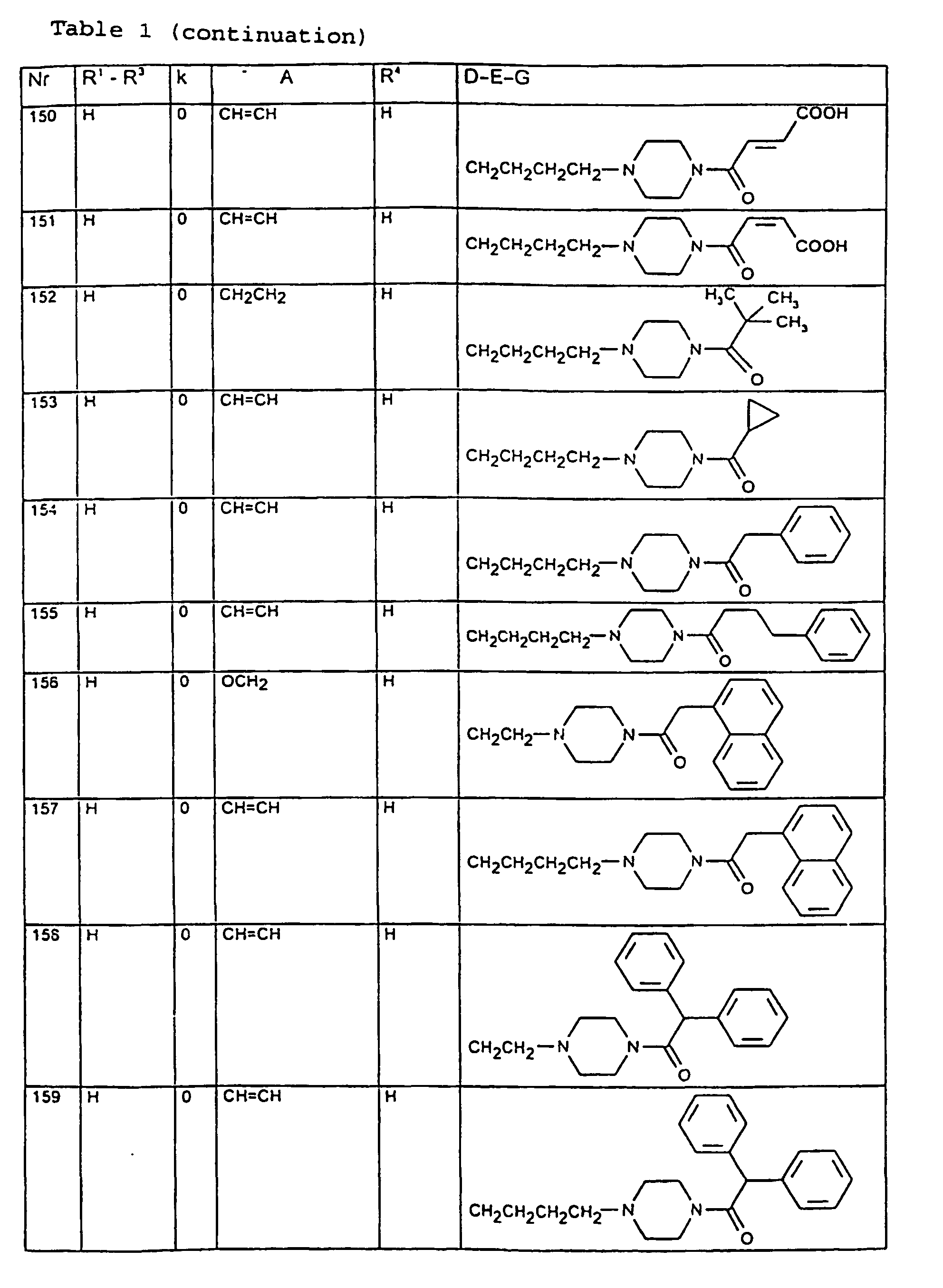

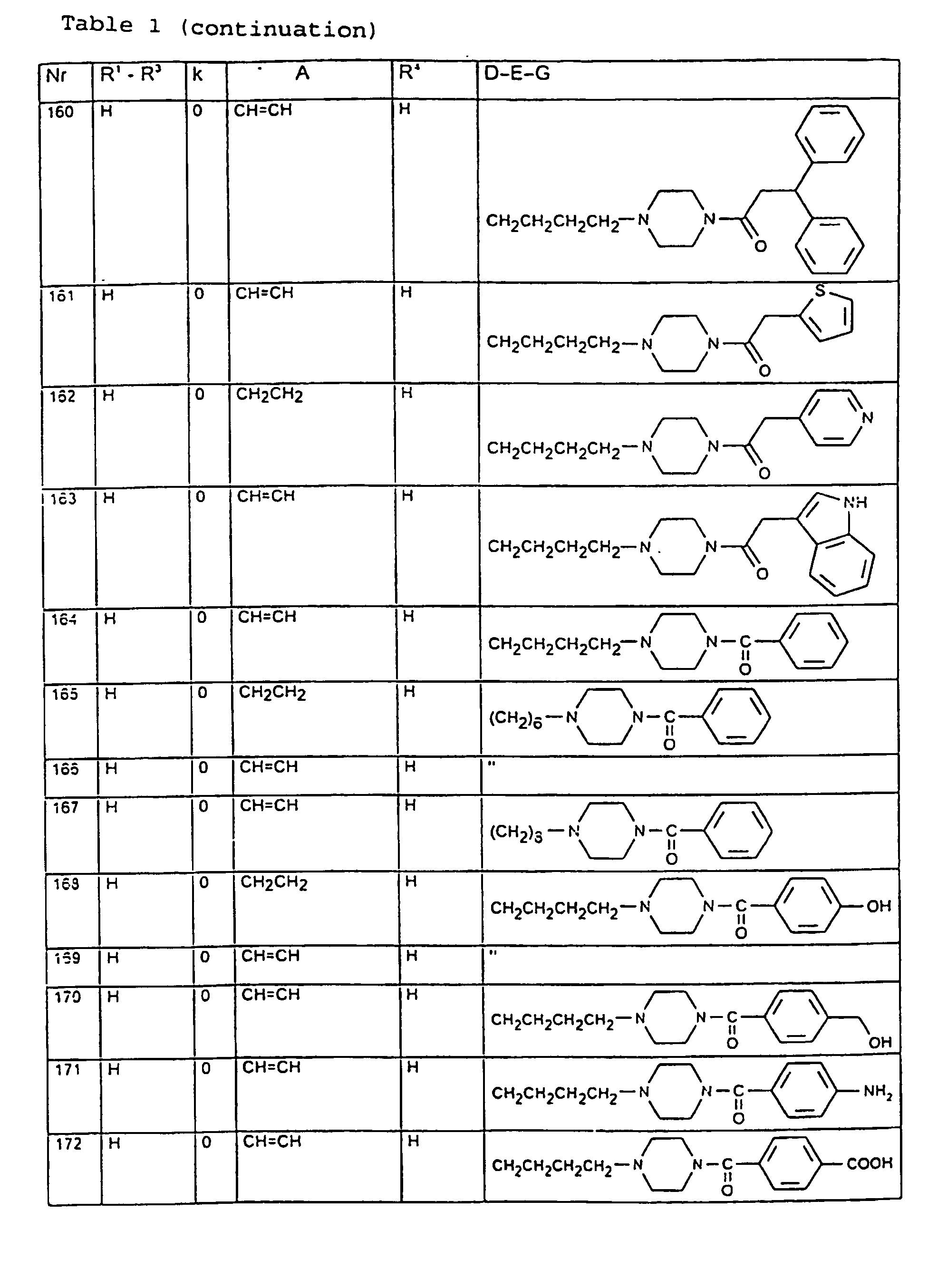

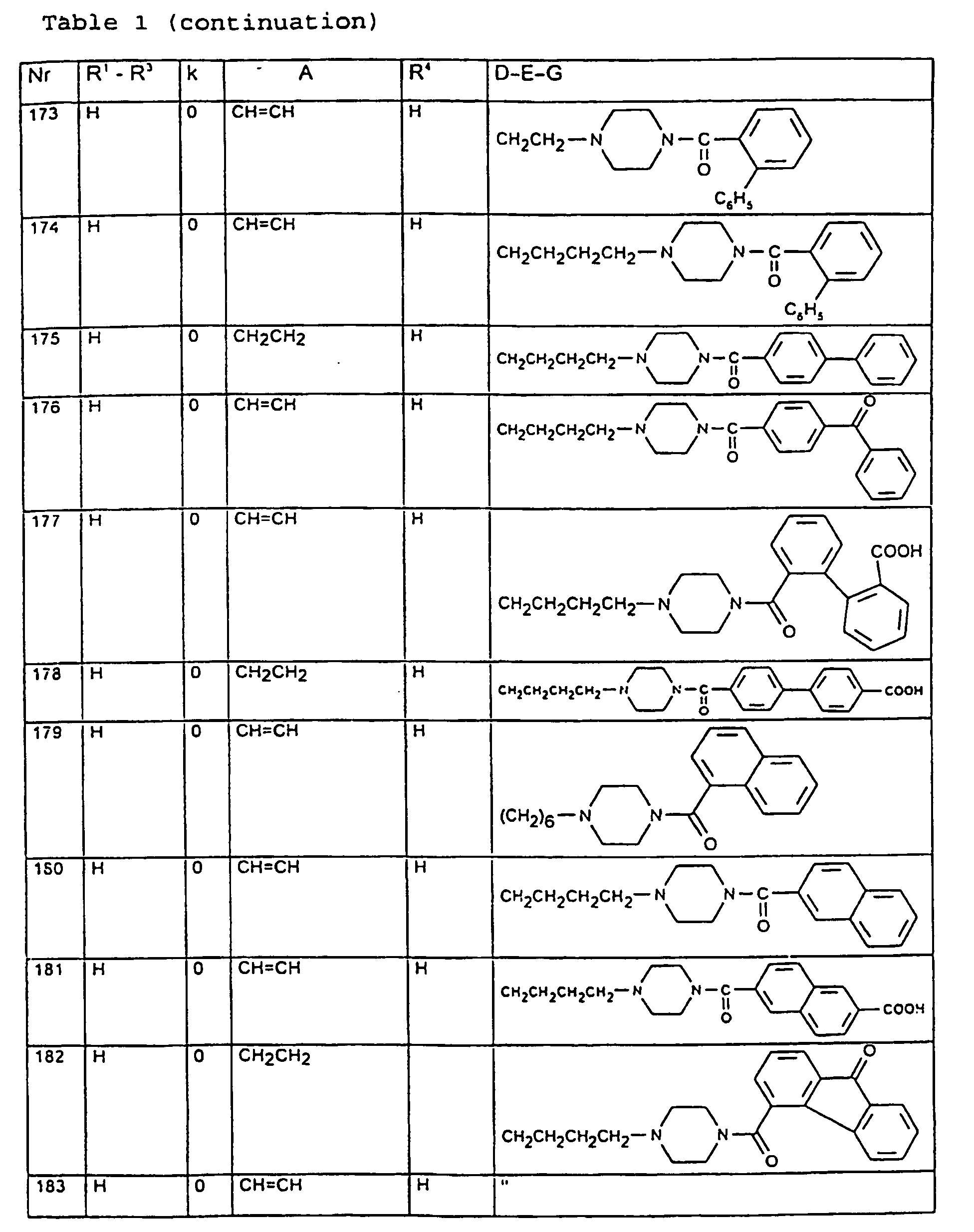

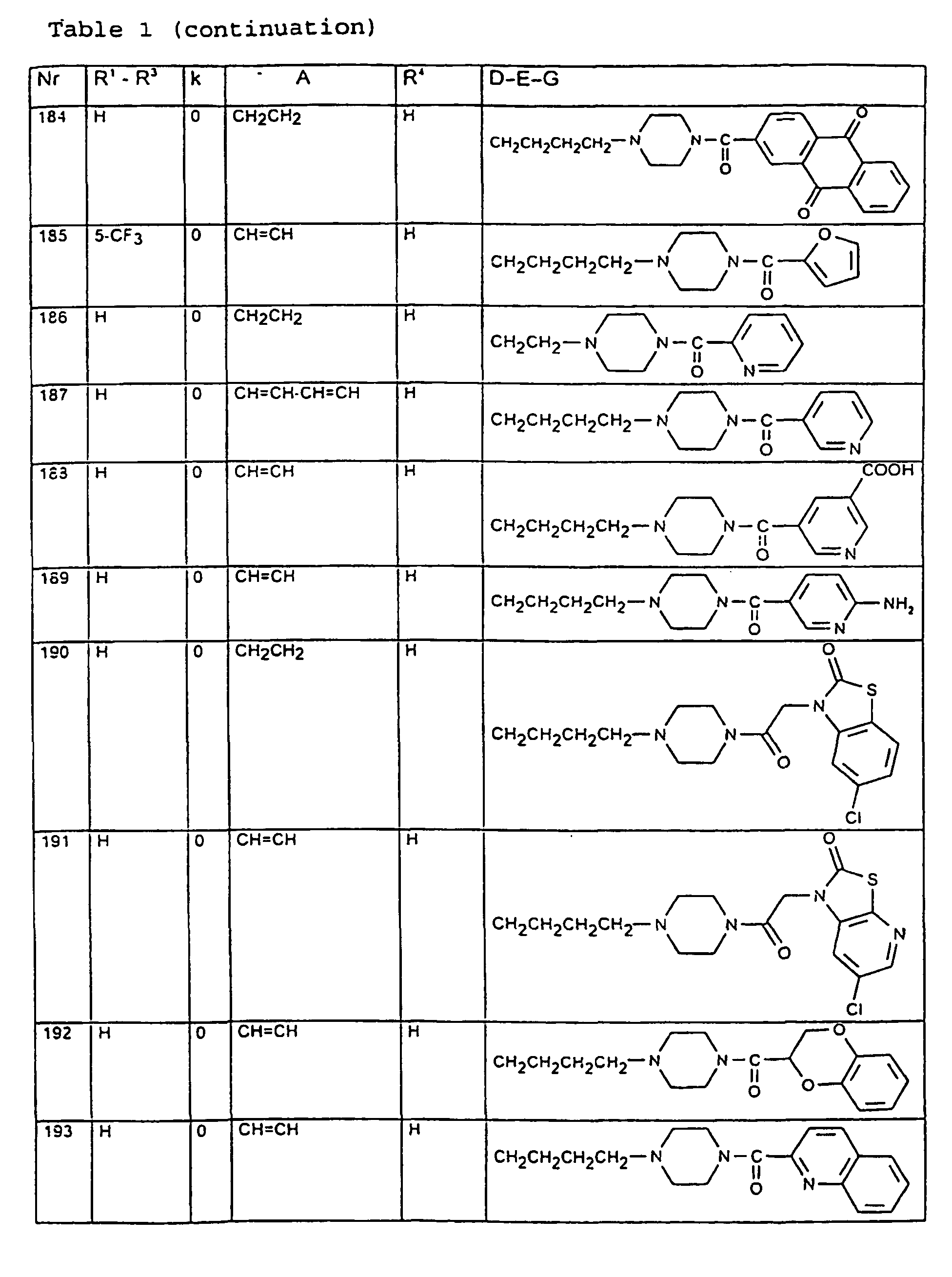

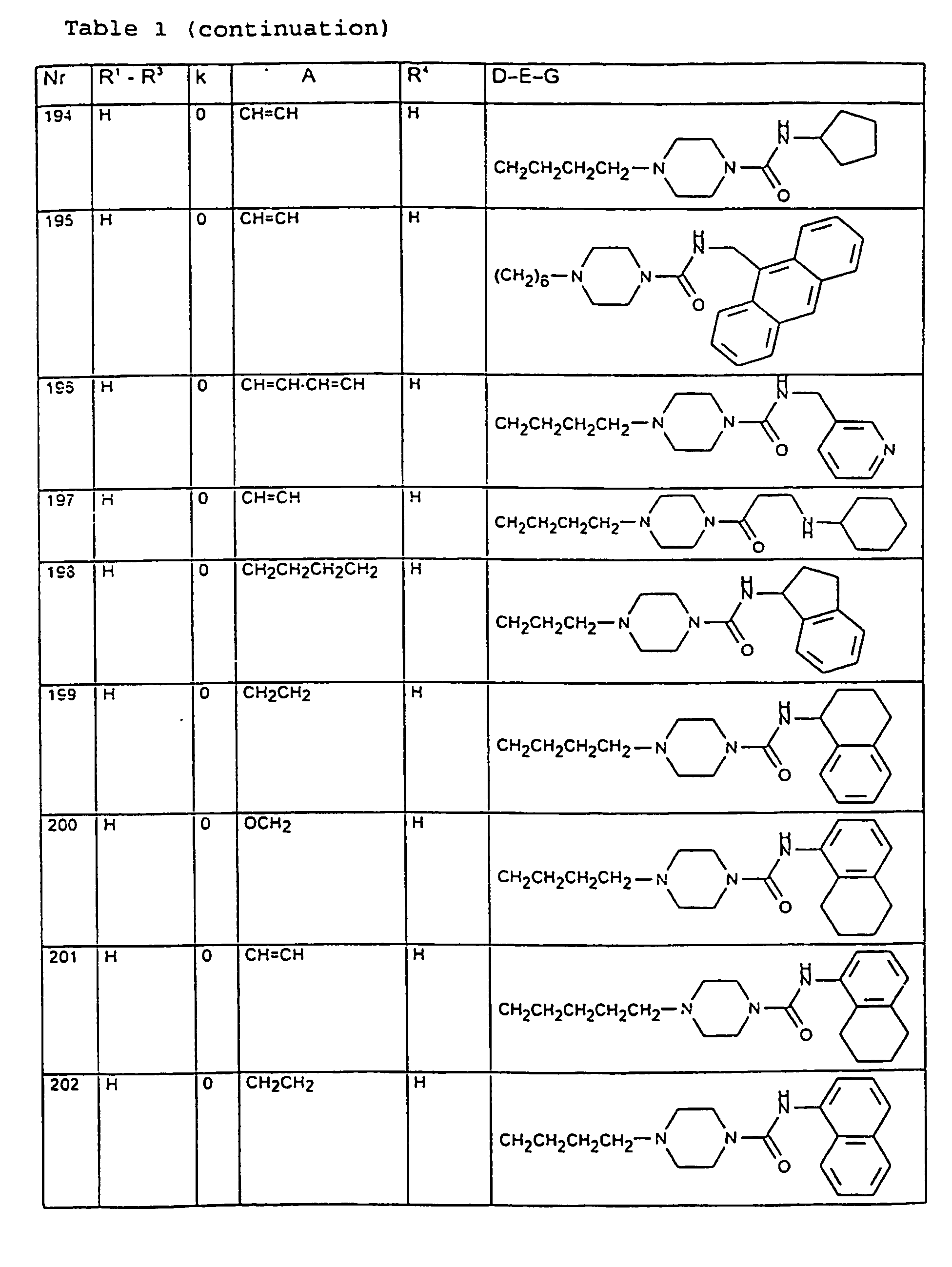

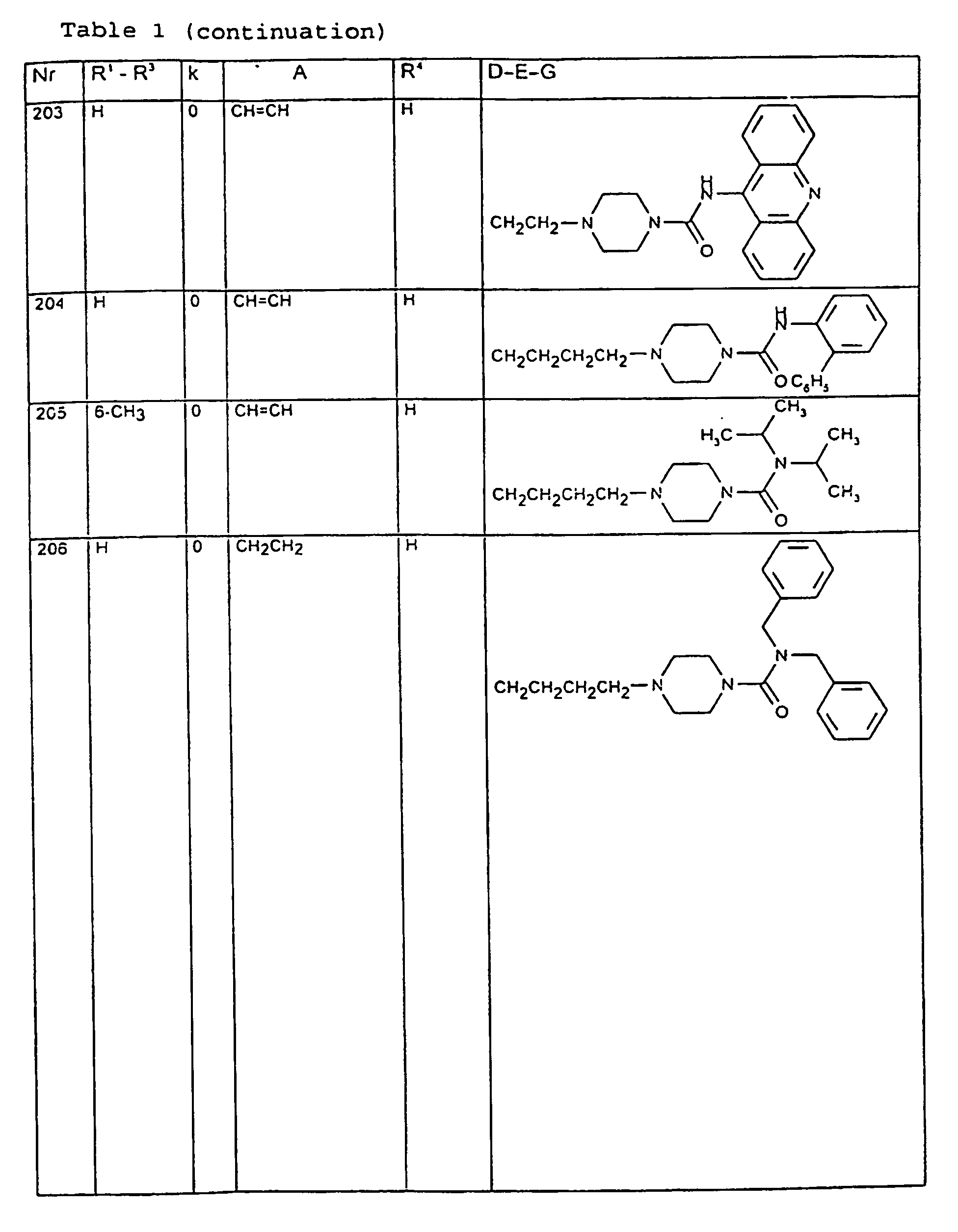

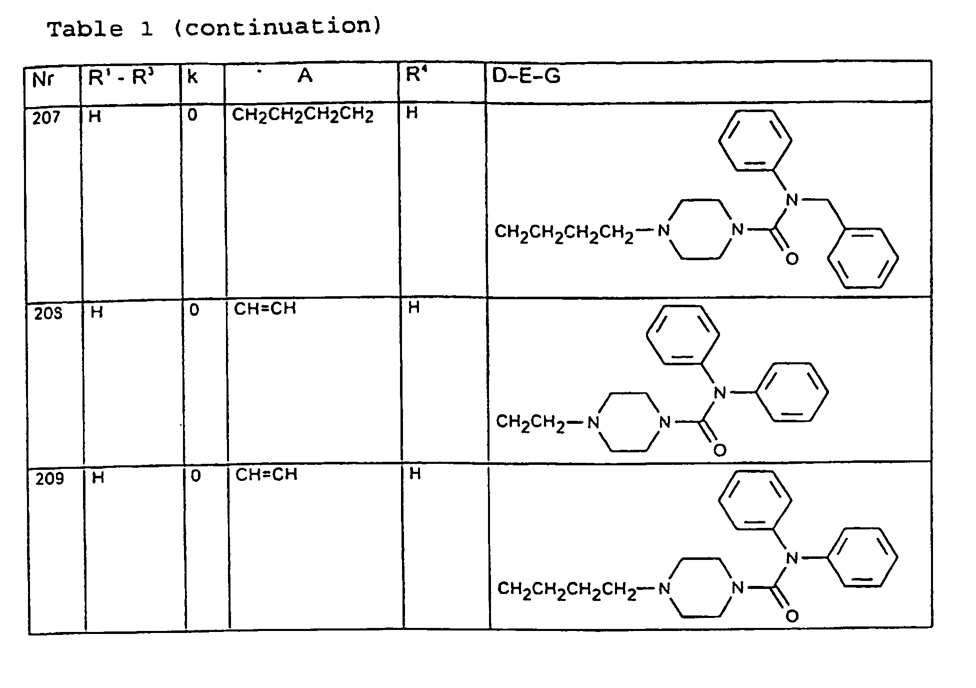

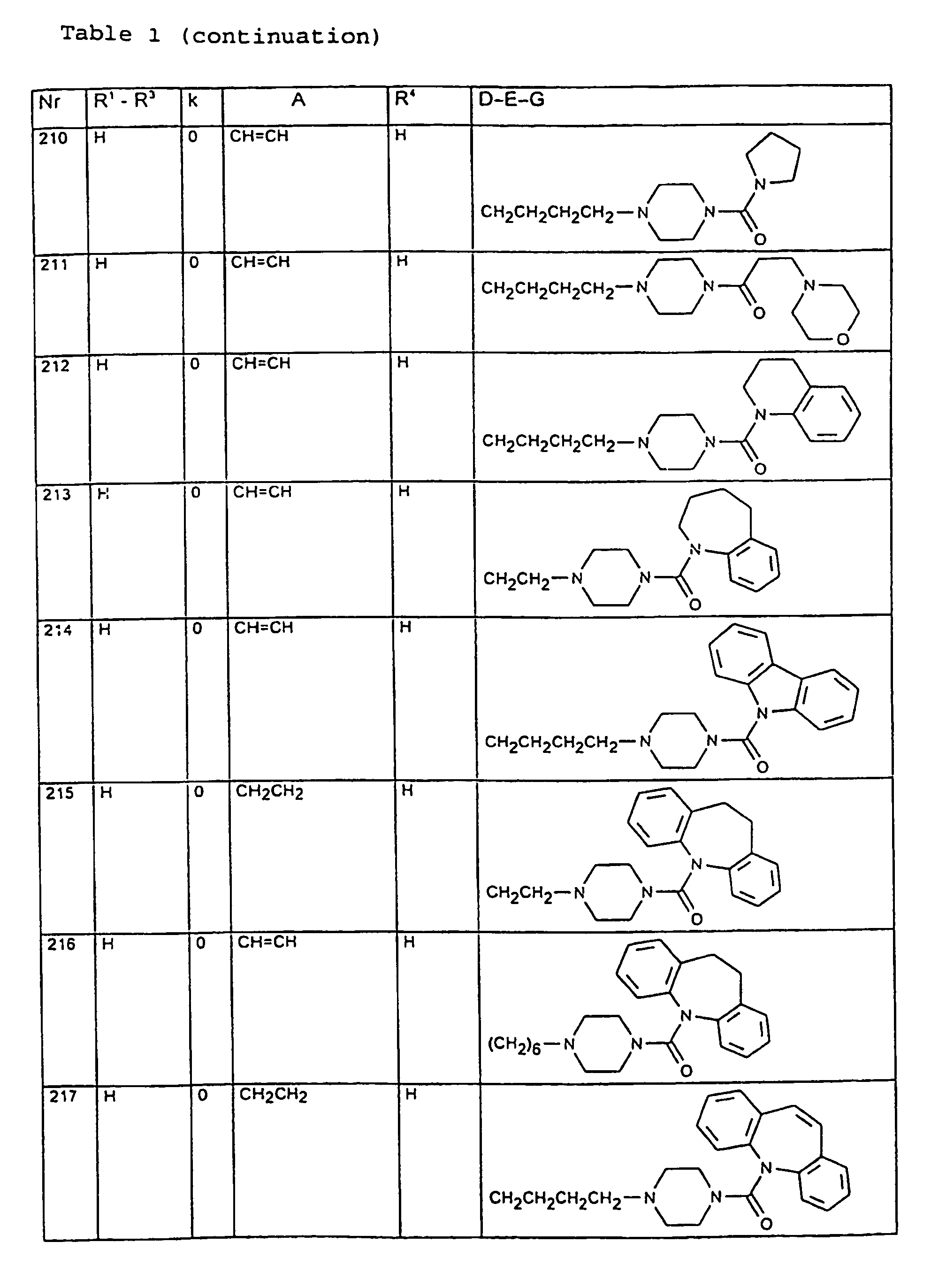

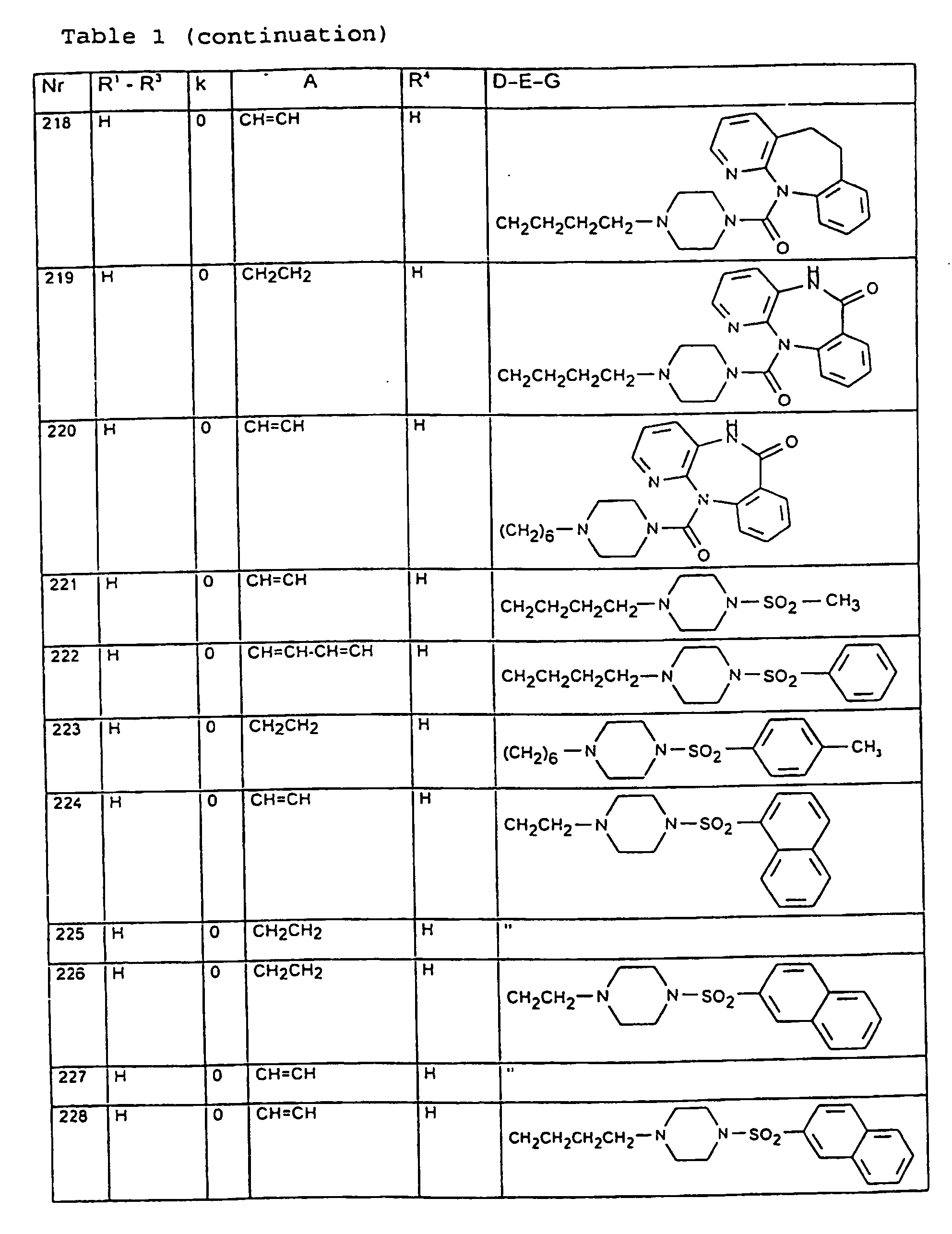

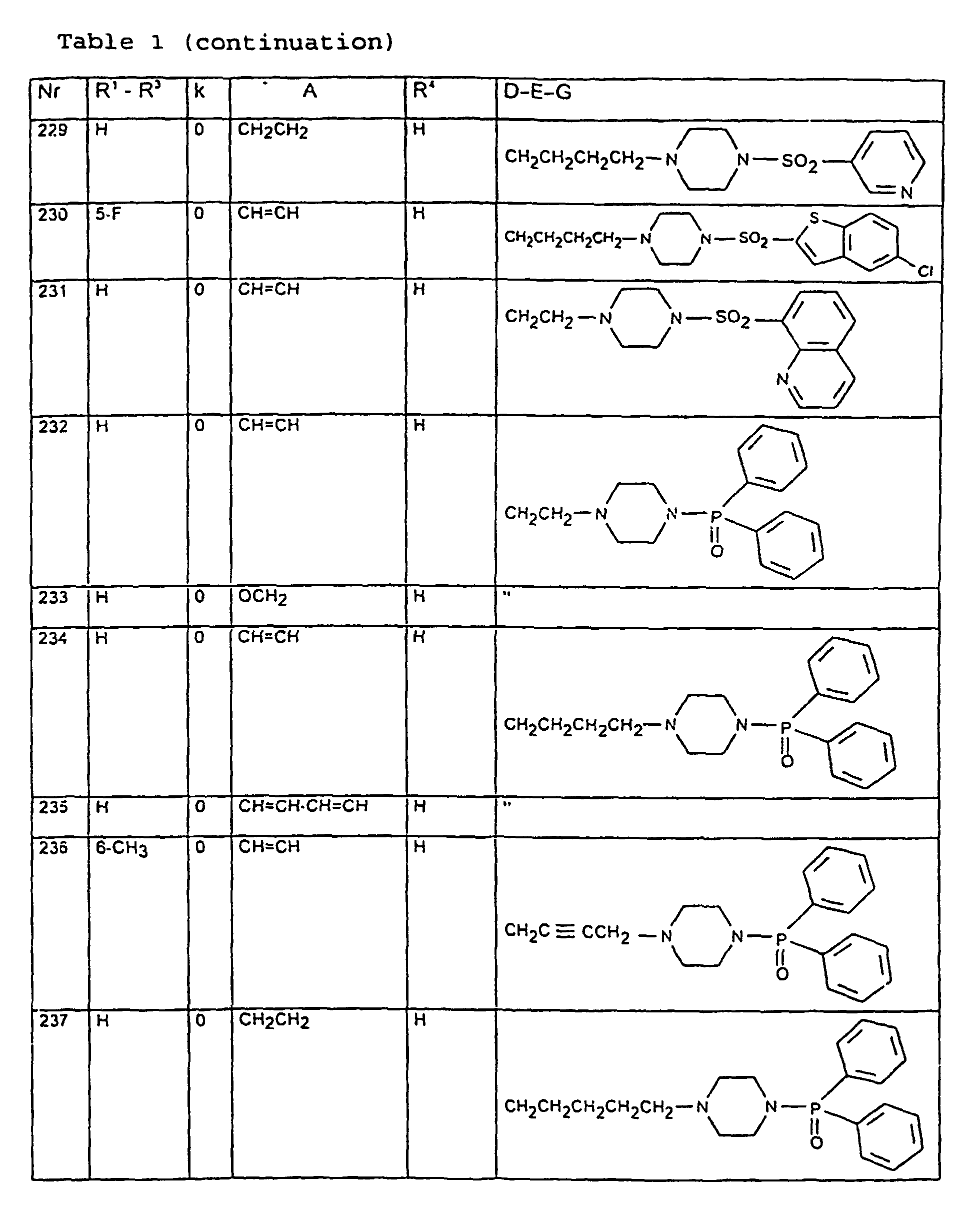

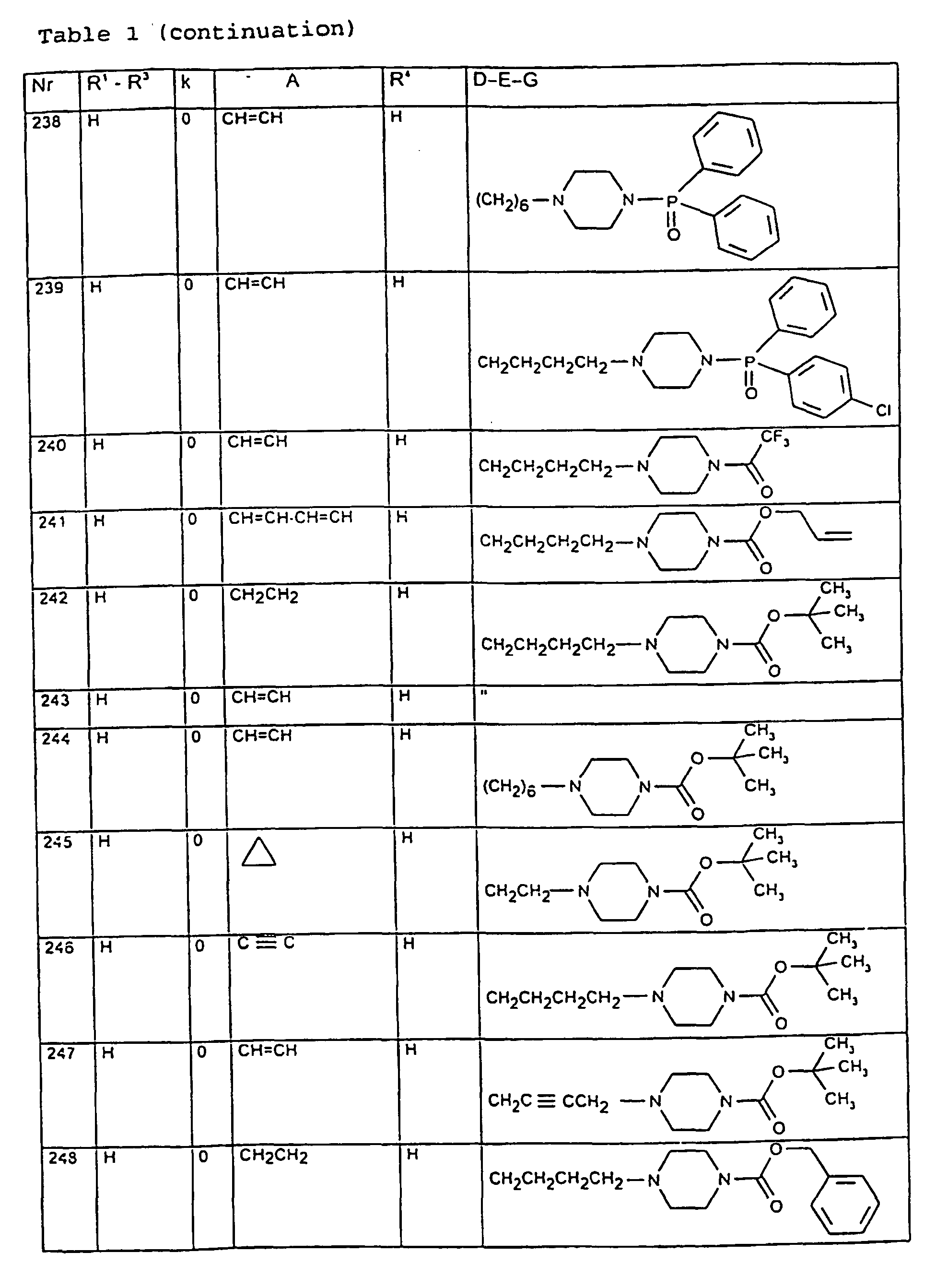

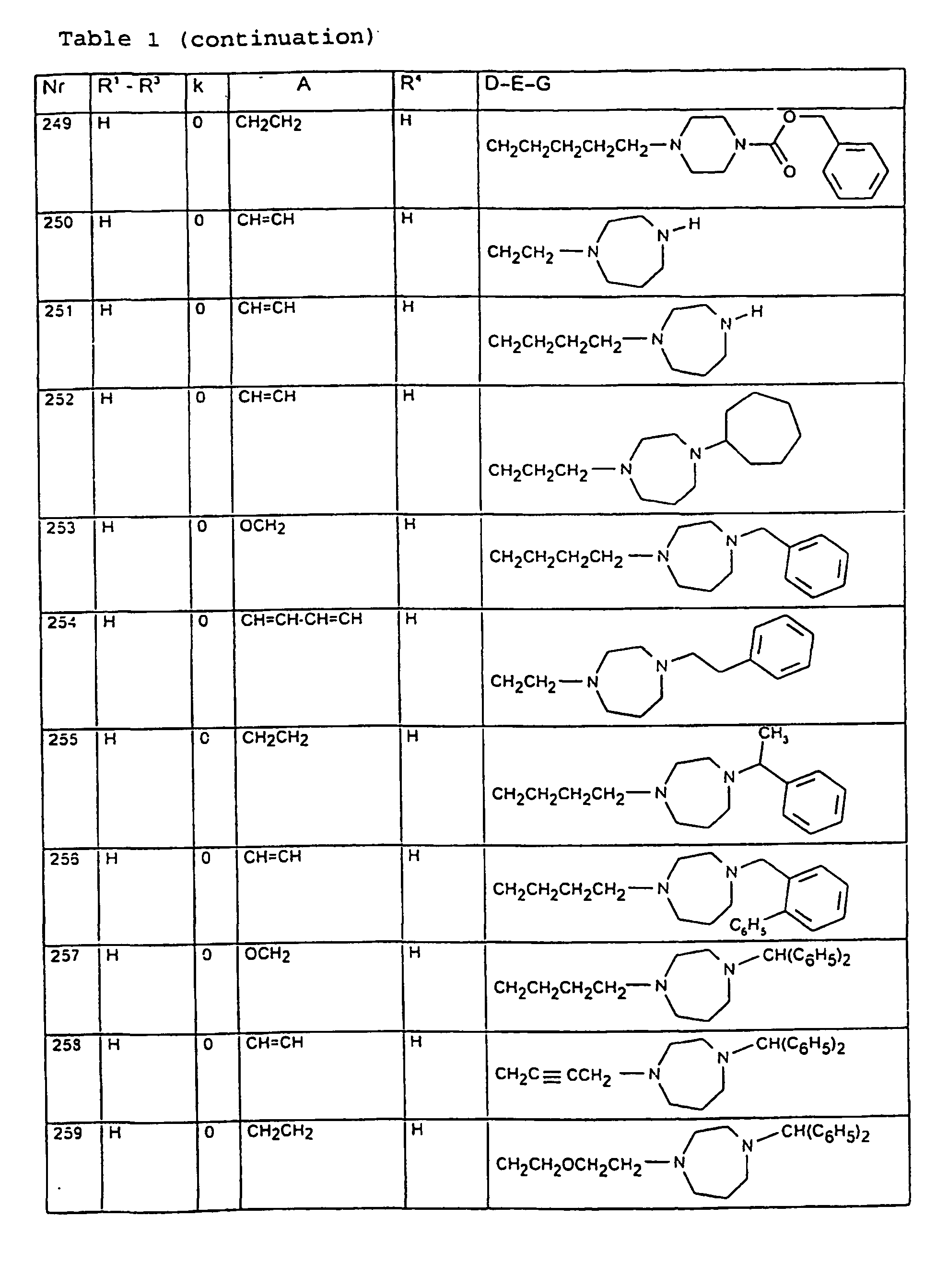

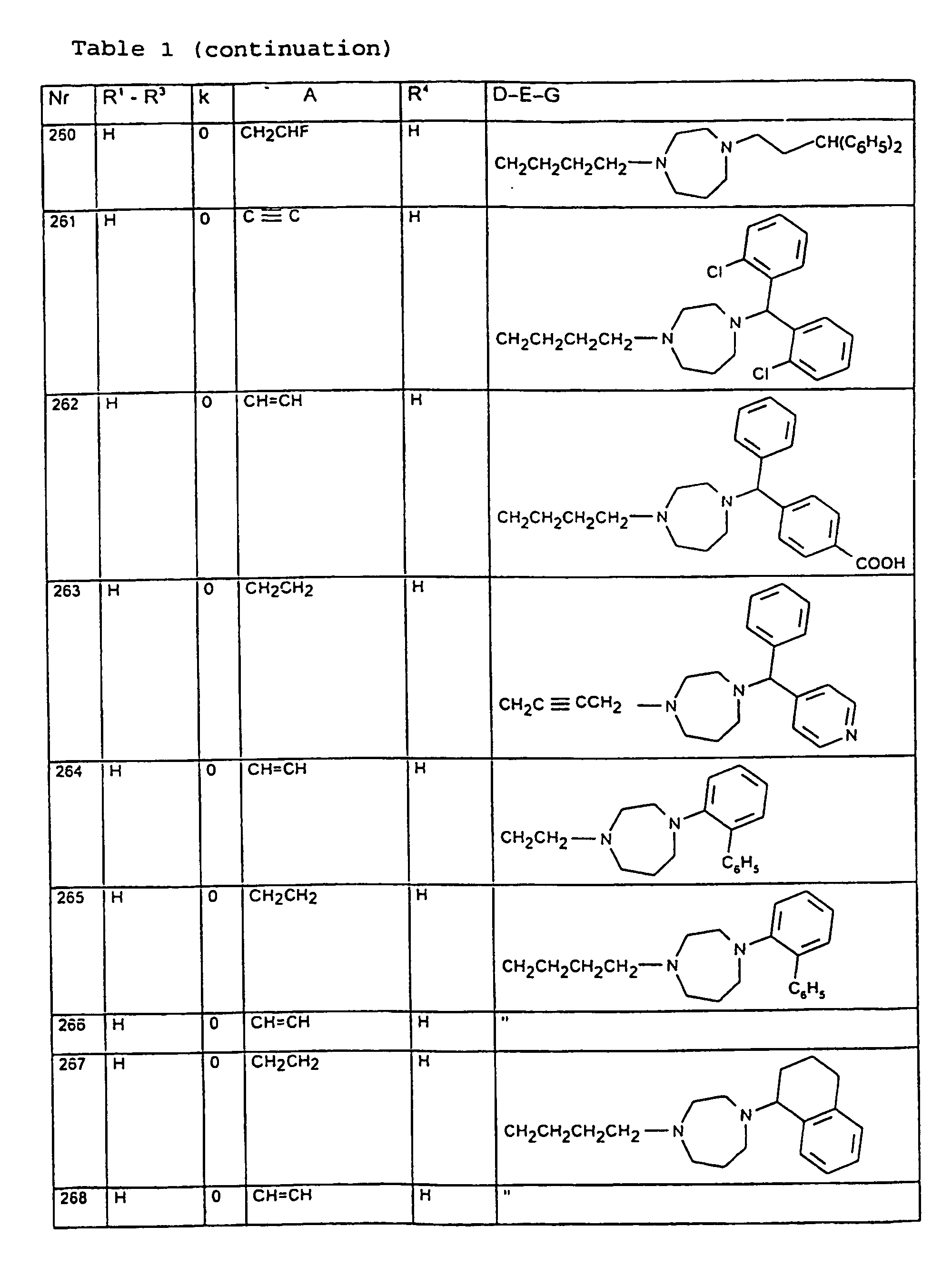

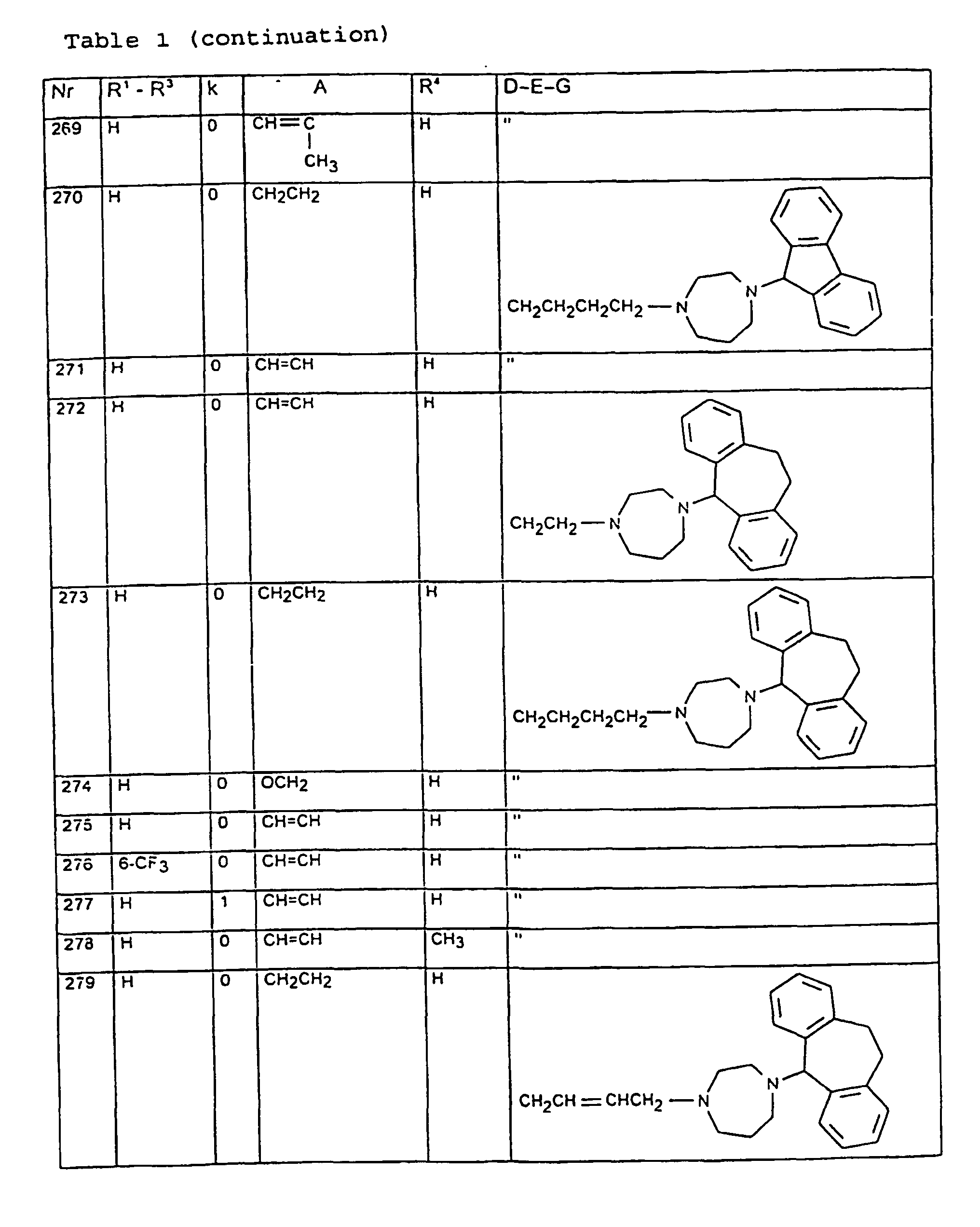

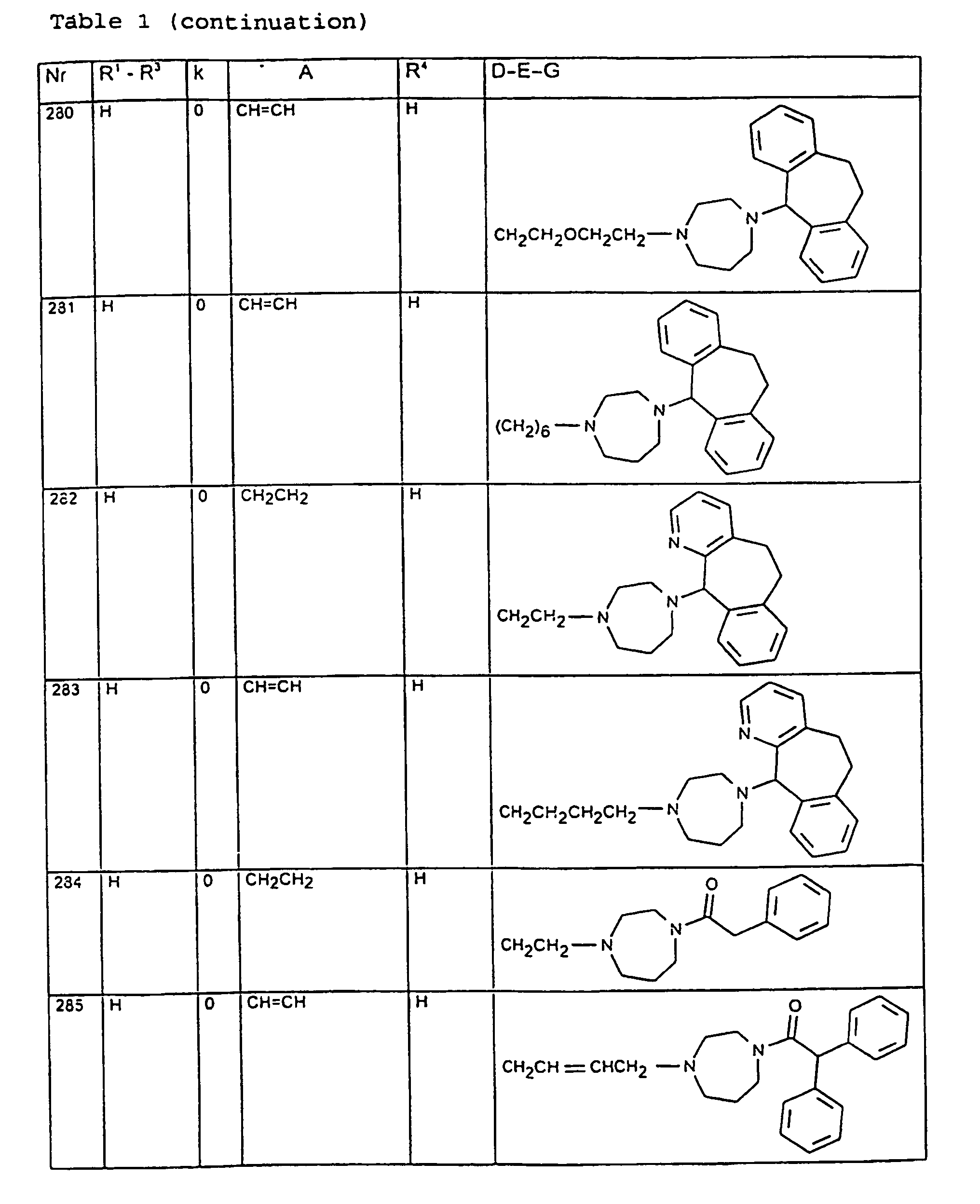

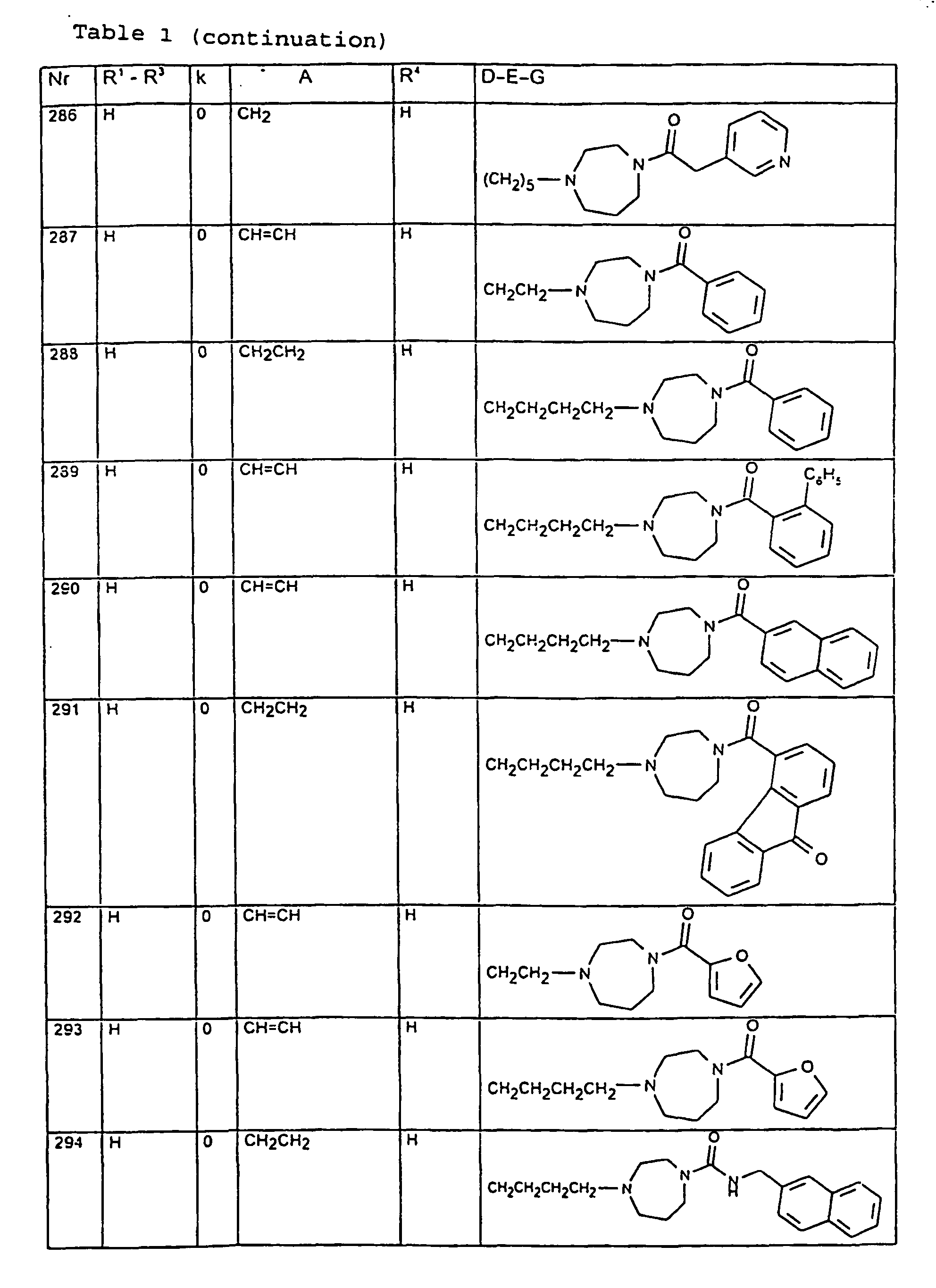

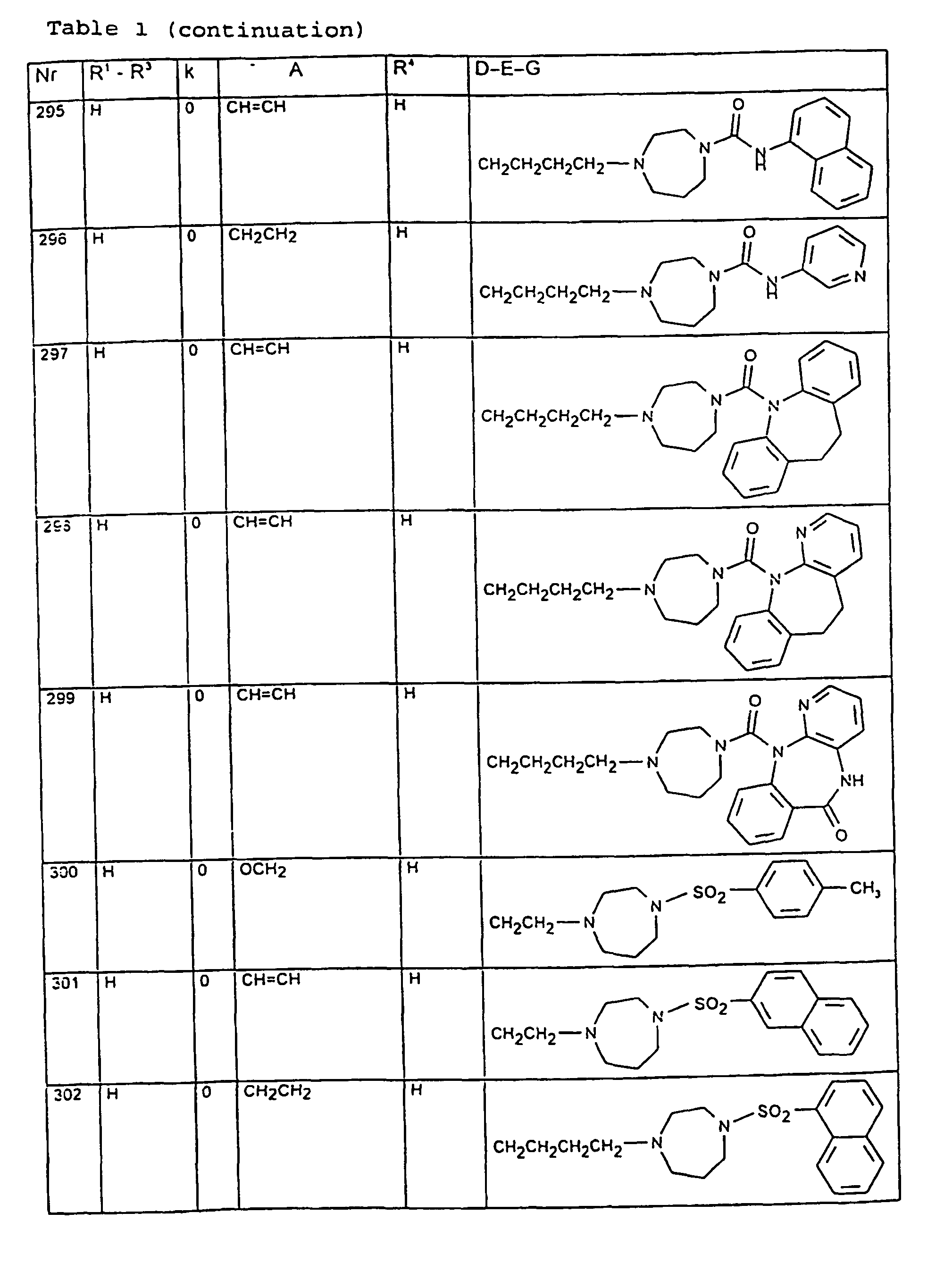

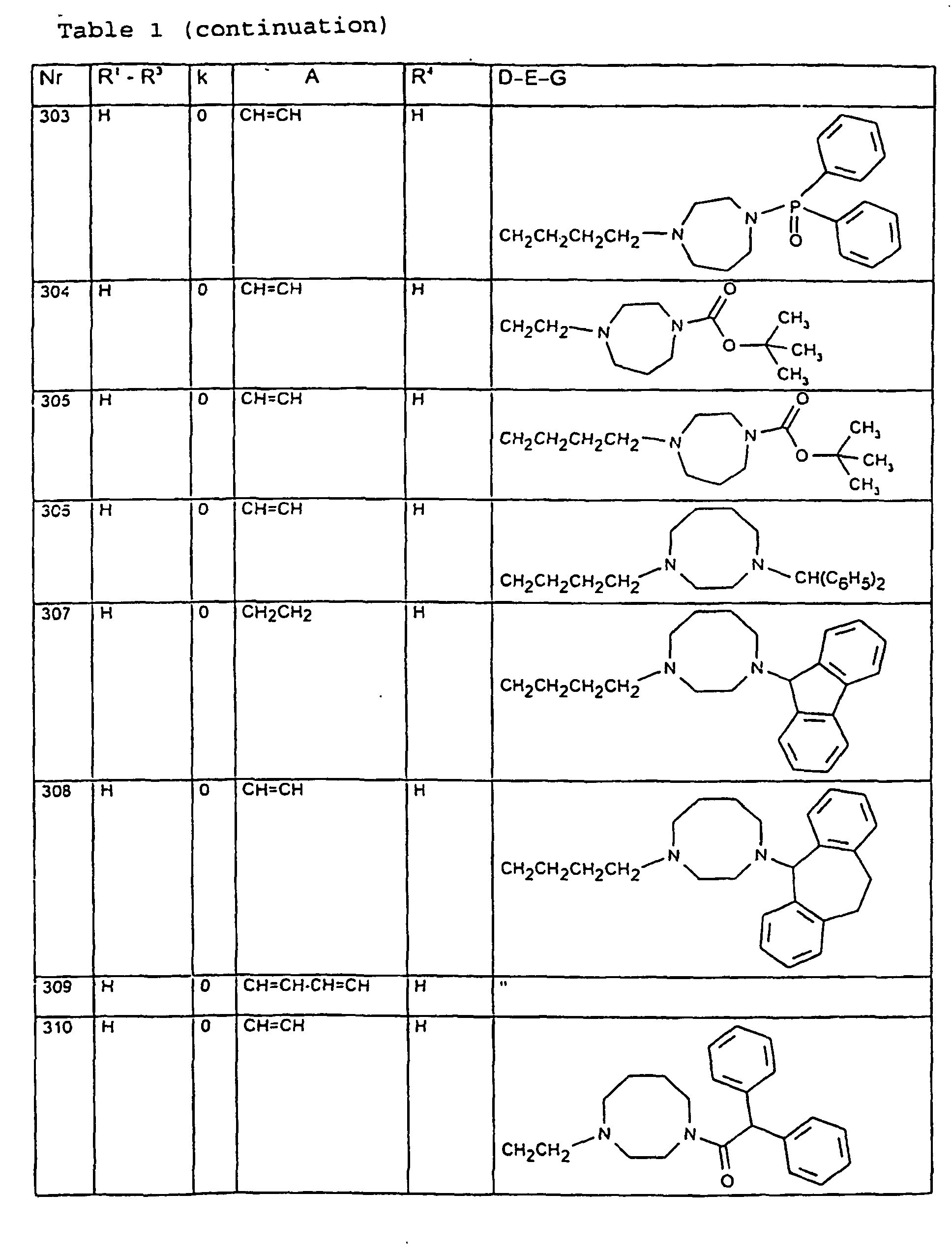

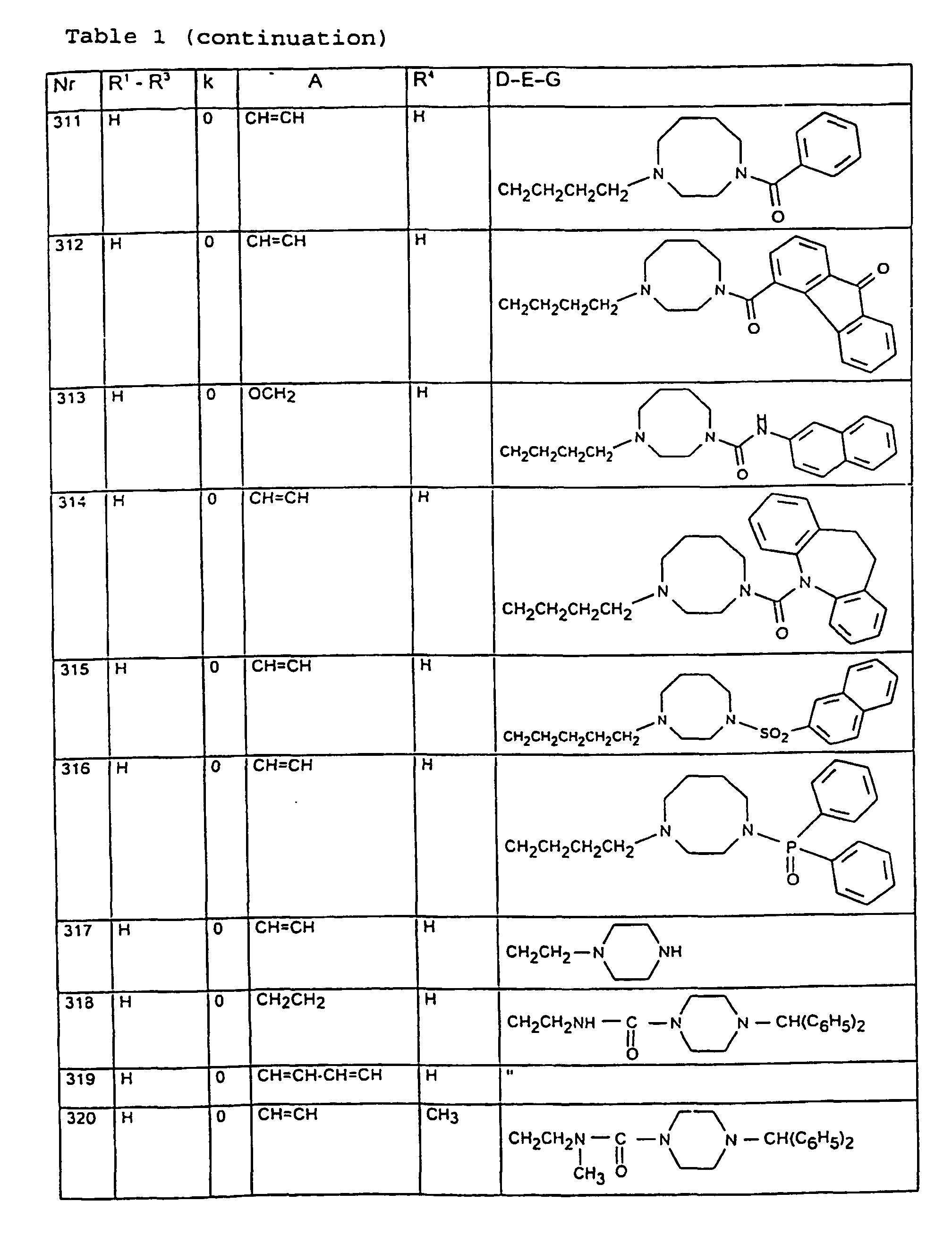

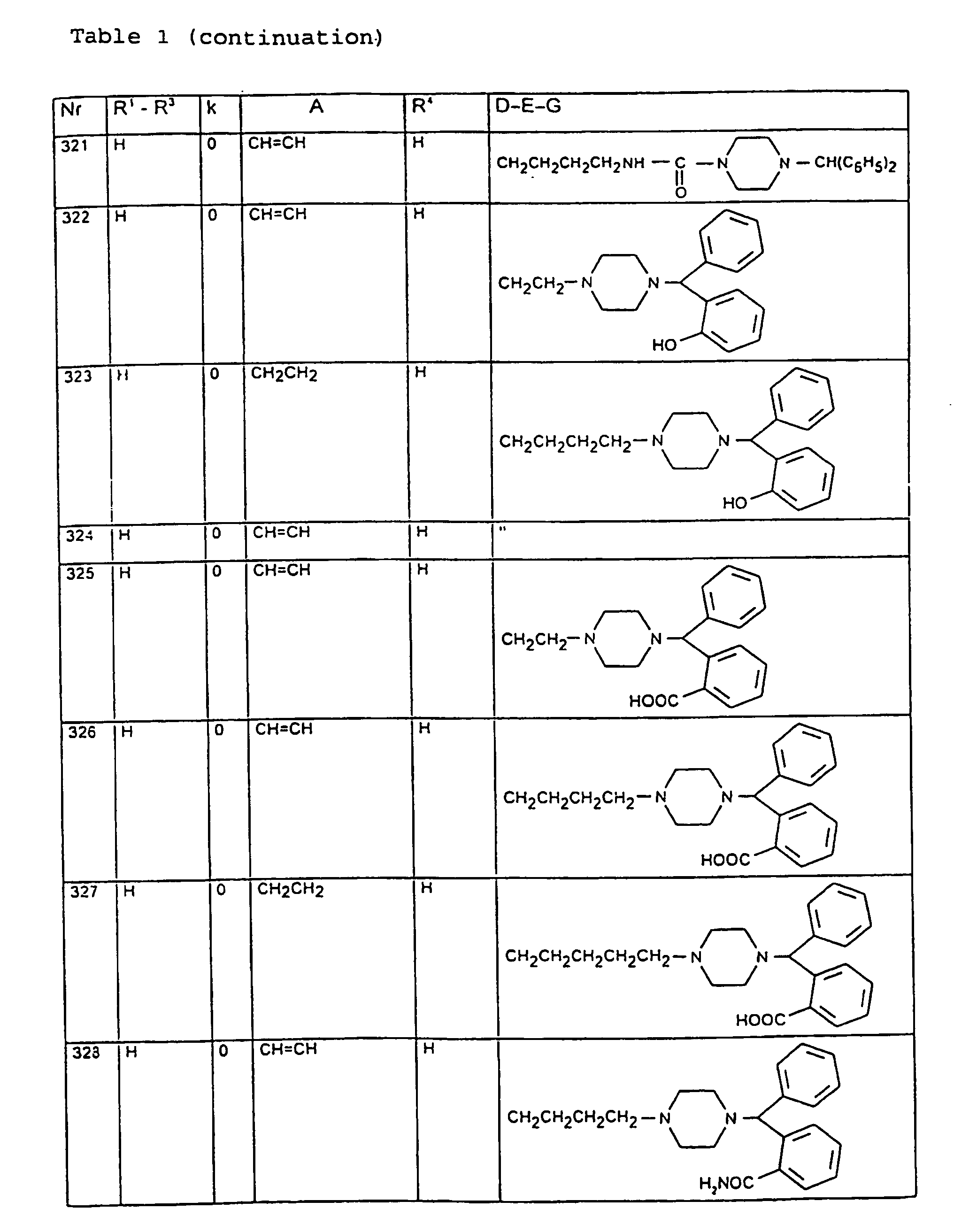

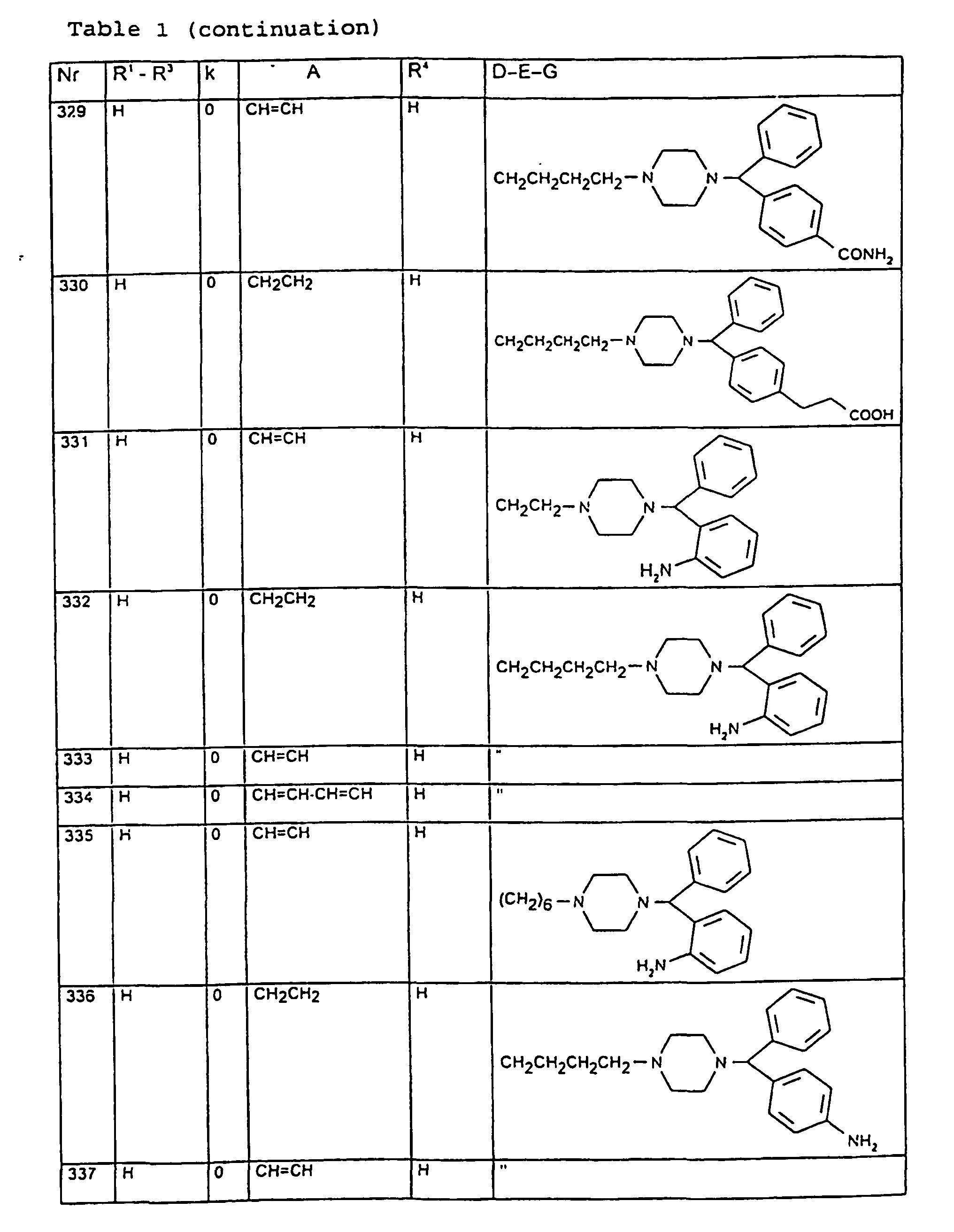

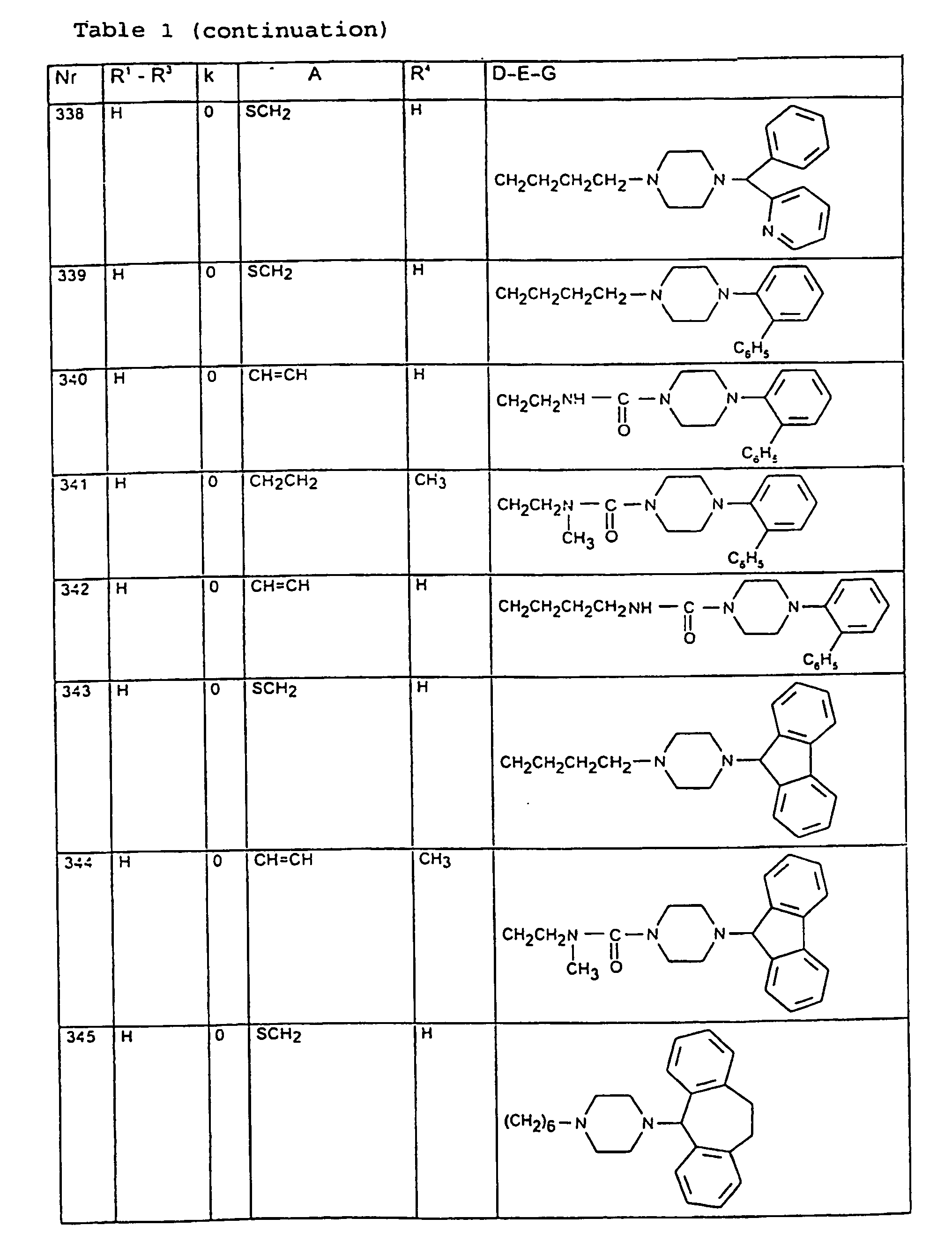

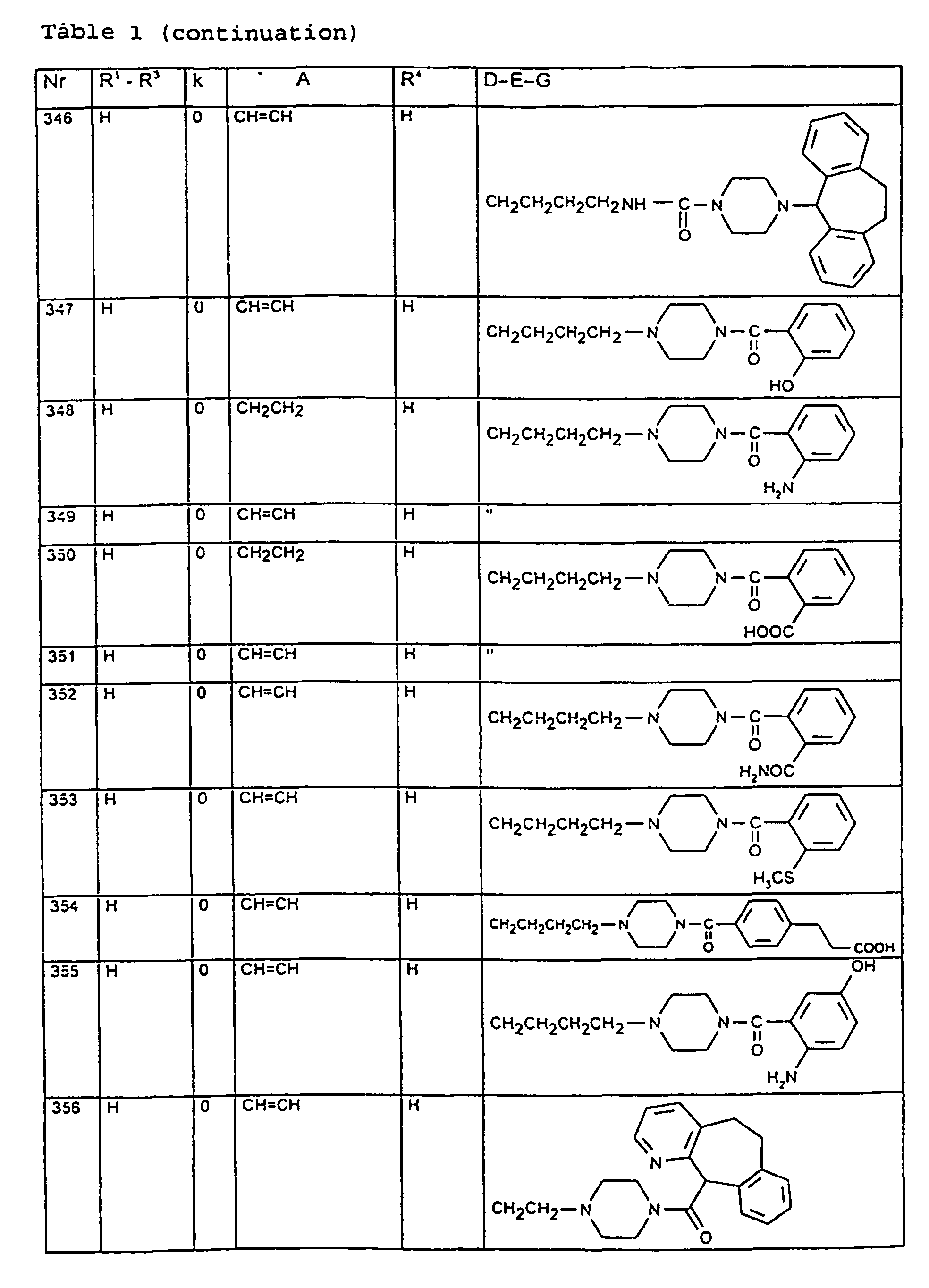

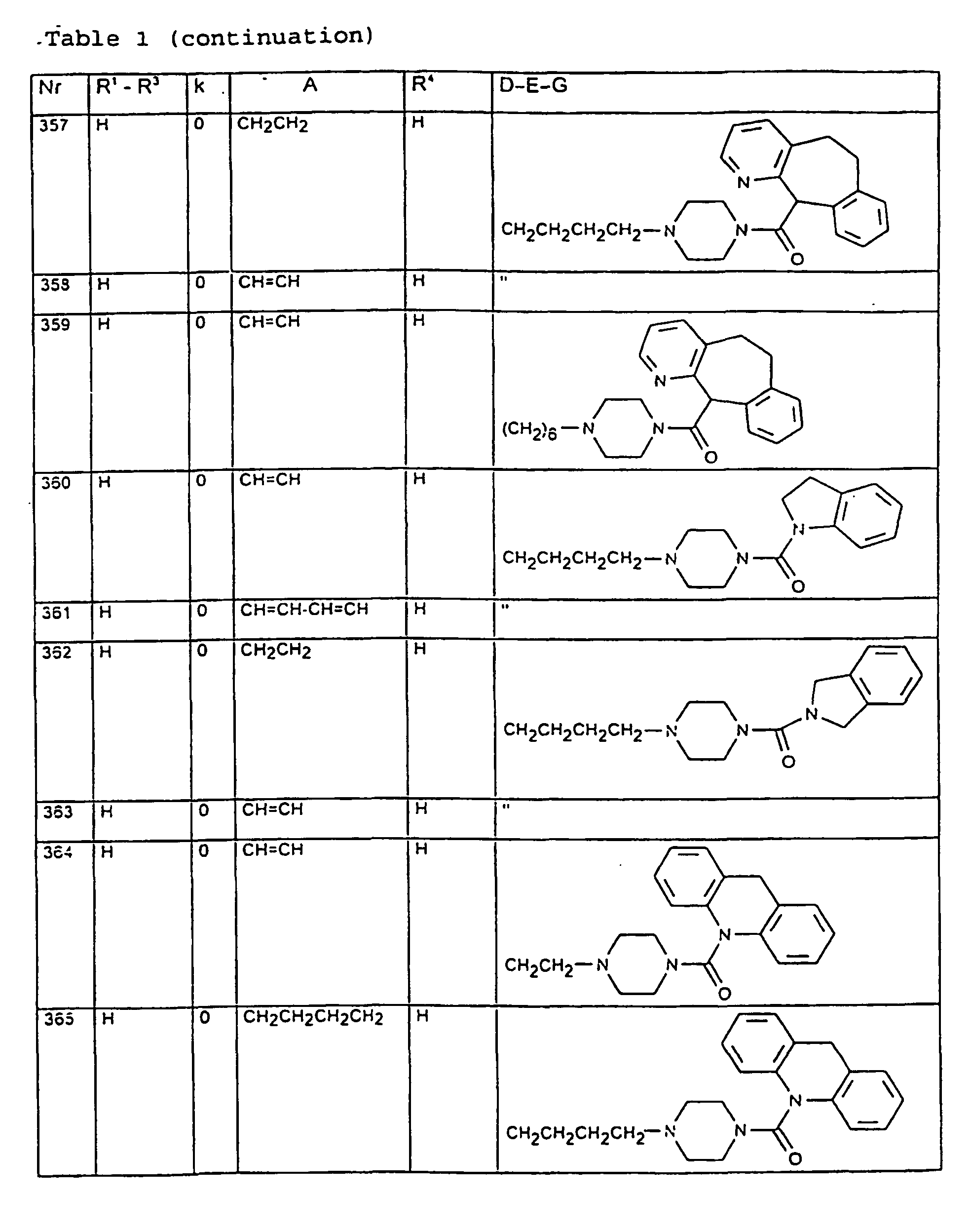

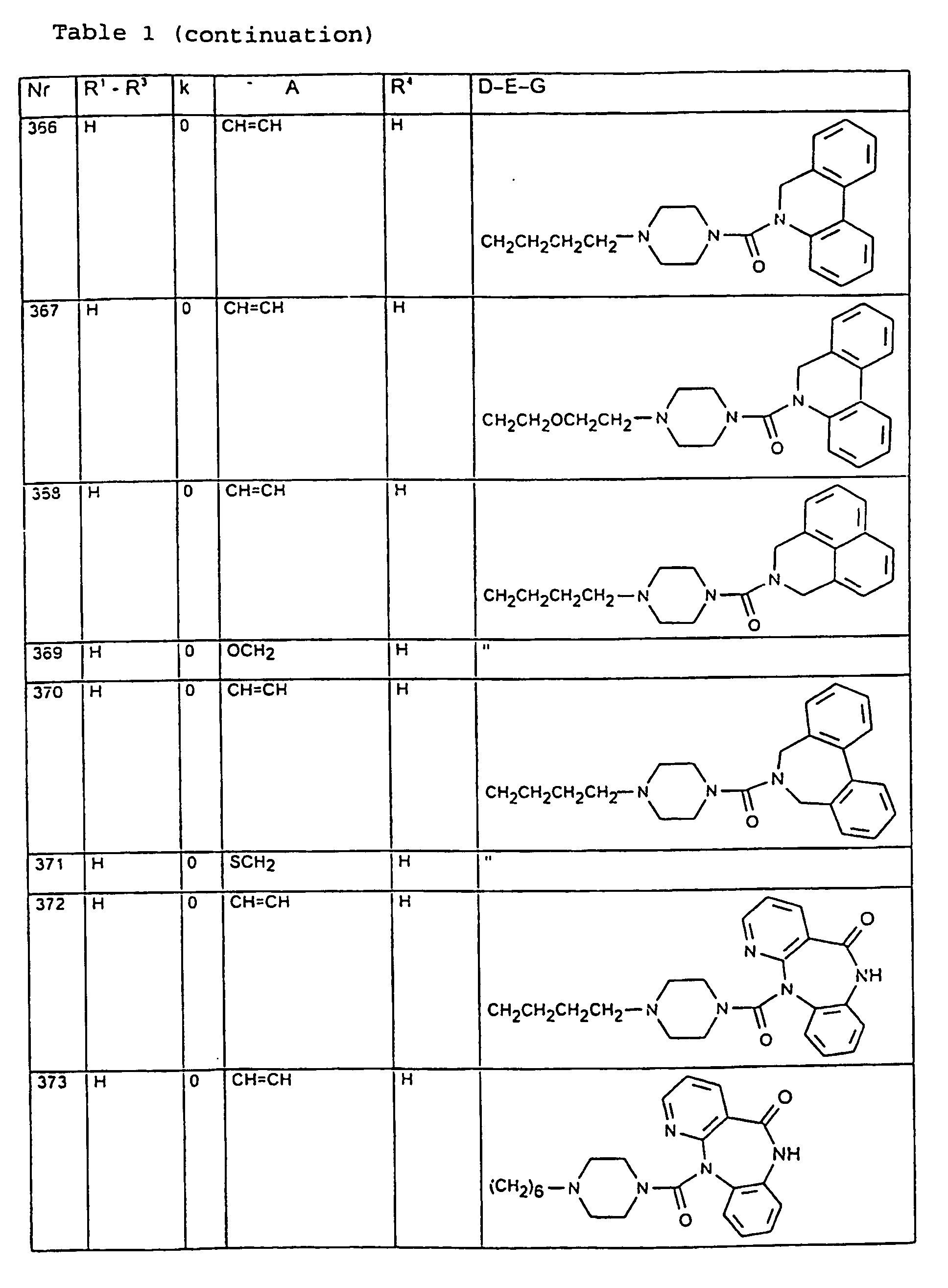

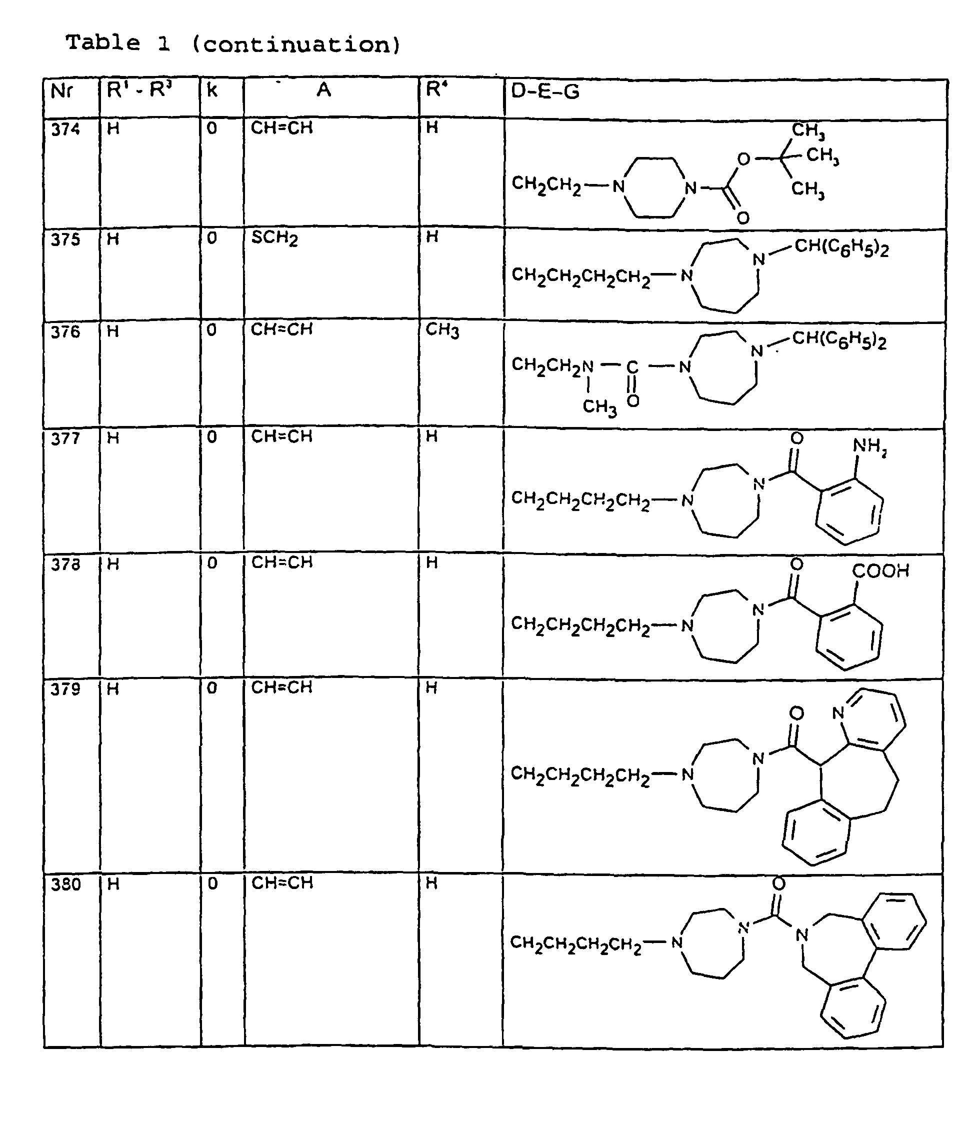

Further subject-matter of the claims are known analogous methods for the production of the compounds of formula (I) according to the invention.

According to **method variant (A),**compounds of formula (I) are
**(a)** obtained in the manner by reacting carboxylic acids of formula (II) 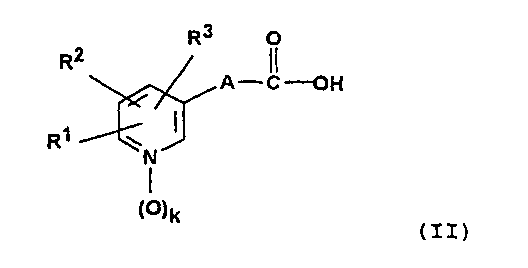 in which R¹, R², R³, A and k have the meaning given above or their reactive derivatives with compounds of formula (III) 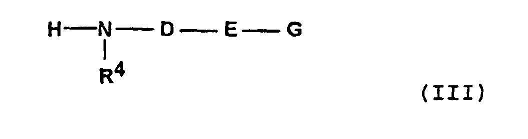
wherein D, E, G and R⁴ also have the above described meanings and the meanings given in the claims.

Reactive derivatives of compound (II) can be, for example, activated esters, anhydrides, acid halides (especially acid chlorides) or simple low alkyl esters. Suitable activated esters are, for example, p-nitrophenyl ester, 2,4,6-trichlorphenyl ester, pentachlorophenyl ester, cyanomethyl ester, esters of N-hydroxysuccinimide, N-hydroxyphthalimides, 1-hydroxybenzotriazole, N-hydroxypiperidine, 2-hydroxypyridine, 2-mercaptopyridine, etc. Anhydrides can be symmetric anhydrides or mixed, as they are obtained, for example, with pivaloyl chloride or with chloroformates. Aromatic (for example chloroformic phenyl ester), araliphatic (for example chloroformic benzyl ester) or aliphatic chloroformates (for example chloroformic methyl ester, -ethyl ester or -isobutyl ester) can be used for this.

Reaction of compounds (II) with compounds (III) can also be carried out in the presence of condensation agents such as dicyclohexylcarbodiimide, 1-ethyl-3-(3-dimethylaminopropyl)-carbodiimide hydrochloride, N,N'-carbonyldiimidazole, 1-ethoxycarbonyl-2-ethoxy-1,2-dihydroquinoline, etc. If carbodiimides are used as the condensation agent, reagents such as N-hydroxysuccinimide, N-hydroxyphthalimide, 1-hydroxybenzotriazole, N-hydroxypiperidine, etc. can be advantageously added.

Compounds of formula (III) can be used for reaction as free bases as well as in the form of their acid addition salts. For this, the salts of inorganic acids are to be preferred, i.e. hydrochlorides, hydrobromides or sulfates for example.

Reaction of compounds (II) or their reactive derivatives with compounds (III) are normally carried out in a suitable, preferably inert solvent. As examples, aromatic hydrocarbons such as benzene, toluene, xylene, halogenated hydrocarbons (for example dichloromethane, chloroform, 1,2-dichloroethane, trichloroethylene), ethers (for example diethyl ether, tetrahydrofuran, dioxane, glycol dimethyl ether), ethyl acetate, acetonitrile or polar aprotic solvents such as, for example, dimethylsulfoxide, dimethylformamide or N-methylpyrrolidone are to be named. Pure solvents, as well as mixtures of two or more of them, can be used.

The reaction is optionally carried out in the presence of an auxiliary base. Suitable examples for this are alkali metal carbonates (sodium carbonate, potassium carbonate), alkali metal hydrogen carbonates (sodium hydrogen carbonate, potassium hydrogen carbonate), or organic bases such as, for example, triethylamine, ethyl diisopropylamine, tributylamine, N-methylmorpholine or pyridine. A suitable excess of compound (III) can also be used as a base. If compounds (III) are used in form of their acid addition salts, then it is appropriate to consider the amount of auxiliary base used as equivalent.

The reaction temperatures can - depending on reactivity of the starting materials - vary in a wide range. Generally, the reaction is carried out at temperatures between -40°C and 180°C, preferably between -10°C and 130°C, especially at the boiling point of the solvent used.

The starting compounds and/or intermediates (II) and (III) are known and/or can be produced according to known methods in an analogous manner. Moreover, their production is further described below by means of representative examples.

Compounds of formula (I) can also be produced according to the variant pursuant to **Method B** by reaction of compounds of formula (I) wherein G is hydrogen, whereby the latter which themselves are anti-proliferative active ingredients according to the invention (as follows from the definitions for the general formula). Thereby, the reaction of compounds according to formula (I) occurs with a compound of formula (IV),

L―G (IV)

in which G has the meaning given above, with the exception of hydrogen, and L represents a suitable nucleofuge or reactive group. The type of nucleofuge or reactive group L and the conditions of the reaction are dependent of the nature of group **G**. According to a further variant pursuant to **method (B1)** compounds of formula (I), in which **G**, with the exception of hydrogen, has the meaning of G¹ according to the above definition can also be synthesized by reacting compounds of formula (I), in which **G** is hydrogen, with a suitable alkylation agent and/or arylation agent of formula (IV), wherein **G** is an alkyl-, alkenyl-, alkinyl-, cycloalkyl-, aryl-, aralkyl-, heteroaryl- or heteroaralkyl residue according to definition and the leaving group L can be a reactive derivative of an alcohol, for example, a halogen atom such as chlorine, bromine or iodine or a sulfonic acid ester, i.e. for example a methanesulfonyloxy group, trifluoromethanesulfonyloxy-, ethanesulfonyloxy-, benzenesulfonyloxy-, p-toluenesulfonyloxy-, p-bromobenzenesulfonyloxy-, m-nitrobenzenesulfonyloxy group, etc. A reactive group L can be a terminal epoxide group.

The reaction of compounds (I), in which G is a hydrogen, and (IV) is usually conducted in a suitably inert solvent. Such solvents can be for example, aromatic hydrocarbons (benzene, toluene, xylene), ethers (for example tetrahydrofuran, dioxane, glycol dimethyl ether), ethyl acetate, acetonitrile, ketones (acetone, ethyl methyl ketone), polar protic solvents such as alcohols (ethanol, isopropanol, butanol, glycol monomethyl ether) or polar aprotic solvents such as, for example, dimethylsulfoxide, dimethylformamide or N-methylpyrrolidone. Pure solvents as well as mixtures of two or more can also be used. Preferably, the reactions are carried out in the presence of bases, whereby the same bases as named in method (a) above can be used. If chlorides or bromides are used as compound (IV), the reaction can be accelerated by the addition of alkali metal iodides, for example sodium iodide, potassium iodide. The reaction temperatures can vary between 0°C and 180°C depending on the reactivity of the educts, but preferably lie between 20°C and 130°C. Finally, according to the variant pursuant to **method (B2)** compounds of formula (I), in which **G** represents an acyl residue, a carbamoyl residue, a sulfonyl residue or a phosphinoyl residue according to the above definition, can also be produced in a manner by reacting compounds of formula (I), wherein **G** is hydrogen, with a carboxylic acid, carbamic acid, sulfonic acid and/or phosphinic acid of formula (V),

HO―G (V)

wherein **G** is an acyl residue, carbamoyl residue, sulfonyl residue or phosphinoyl residue according to definition, or their derivatives capable of reaction. Preferred derivatives of carboxylic acids and/or sulfonic acids (V) which are capable of reaction are symmetric or unsymmetric carboxylic acid anhydrides and/or sulfonic acid anhydrides or acyl- and/or sulfonyl halides, especially acyl- and/or sulfonyl chlorides. Preferred derivatives of carbamates and/or phosphinic acids which are capable of reaction are the carbamoyl halides and/or phosphinyl halides, especially carbamyl- and/or phosphinyl chlorides. The reaction of the acids (V) and/or their reactive derivatives with compounds (I), in which G is hydrogen, preferably occurs in the presence of auxiliary bases in solvents and under conditions as they are described in **method (A).**

Compounds of formula (I), wherein **G** represents a carbamoyl residue according to the definition (G2b) with r = 0, i.e. is a group 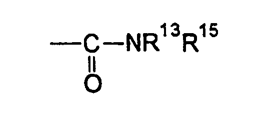 can also be produced pursuant to the variant according to **method (B3)** by reacting compounds of formula (I), in which **G** is hydrogen, with a carbonyl group transmitter to an intermediate product and subsequently reacting this directly with a primary or secondary amine with the formula (VI)

H―NR¹³R¹⁵ (VI)

wherein R¹³ and R¹⁵ and/or the grouping ―NR¹³R¹⁵ have the above meanings without purifying or isolating the intermediate product.

Bis-trichloromethyl carbonate (triphosgene) and carbonyldiimidazole have been proven as particularly reactive carbonyl group transmitters. The reaction of compounds of formula (I), wherein **G** is hydrogen, with triphosgene and/or carbonyldiimidazole are typically conducted in an absolute, inert solvent in the presence of a tertiary organic amine as an auxiliary base in such a manner that the solution of compounds (I) and the auxiliary base are slowly poured into a solution of an equivalent amount of carbonyl group transmitter. Thereby, the reaction requires molar ratios of 1 : 1 for the reaction of compound (I) and carbonyldiimidazol, and, in contrast, a ratio of 1 : 0.35 for the use of triphosgene. After complete reaction of the components to the intermediate product, compound (VI) is added in stochiometric amounts or in excess as a solution or a solid, whereby the reaction is typically completed at elevated temperature. Suitable inert solvents are, for example hydrocarbons such as hexane, heptane, benzene, toluene, xylene, chlorinated hydrocarbons (for example dichloromethane, chloroform, 1,2-dichloroethane, trichloroethylene), ethers (for example diethyl ether, tetrahydrofuran, dioxane), esters such as ethyl acetate, butyl acetate, acetonitrile or polar aprodic solvents such as formamide or dimethylformamide. Pure solvents as well as mixtures of various solvents can be used. Sometimes it is of advantage to carry out the first partial reaction at low temperature in a low-viscosity, highly-volatile solvent and to remove the solvent after formation of the intermediate and replace it by a higher boiling solvent. Amines such as for example triethylamine, ethyl diisopropylamine, tributylamine, N-methylmorpholine or pyridine are suitable as auxiliary bases.

If compounds (I) or (VI) are used as salts, the amount of the auxiliary base is increased accordingly. The reaction temperatures can lie between -40°C and 50°C for the first partial reaction, preferably 0°C to 30°C, and between 0°C and 150°C for the second partial reaction, preferably 20°C to 120°C. Finally, according to variant pursuant to **method (B4)** compounds of formula (I), wherein **G** represents a carbamoyl residue according to the definition (G2b) with r = 0 and R¹⁵ = hydrogen, i.e. the group represents 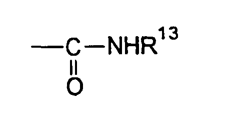 can also be produced by reacting the compounds of formula (I) in which G is hydrogen, with an isocyanate of formula (VII) in which R¹³ has the meaning according to definition

O=C=N-R¹³. (VII).

Reaction of the compounds of formula (I), in which **G** is hydrogen, with the isocyanates of formula (VII) are conducted thereby in an absolute, inert solvent which can be a hydrocarbon such as pentane, hexane, heptane, benzene, toluene, or xylene, chlorinated hydrocarbons (such as dichloromethane, chloroform, 1,2-dichloroethane, trichloroethylene), an ether (for example, diethyl ether, tetrahydrofuran, dioxane), esters such as ethyl acetate, butyl acetate, or polar aprotic solvents such as formamide or dimethylformamide. Mixtures of various solvents can also be used. Thereby, the reaction temperatures can vary in the region from -20°C to 150°C, but preferably lie at 20°C to 100°C.

As already mentioned, the compounds of formula (I), wherein **G** is hydrogen, are themselves active ingredients according to the invention with tumor growth inhibiting activity.
However, independent of their therapeutic applicability, they also represent useful intermediate compounds for the production of a multitude of other compounds according to the invention corresponding to the method variants (B1) to (B4).

In principle, the compounds of the formula (I), in which **G** represents hydrogen, can be produced according to method A by reacting a carboxylic acid of formula (II) with amines of formula (III) in which G is hydrogen as described above.
However, since the compounds of formula (III) with hydrogen as G represent α,ω-diamines, the formation of product mixtures is always to be expected in their reaction with carboxylic acids (II) or their reactive derivatives making a subsequent separation necessary.

In contrast, compounds of formula (I), in which **G** is hydrogen, are essentially more advantageously produced from other compounds of formula (I), in which G is a selectively cleavable group under mild conditions, i.e. corresponds to a nitrogen protective group.

In this connection, among the compounds according to formula (I) with tumor growth inhibiting properties, compounds in which G represents a benzyl group, a 4-methoxybenzyl group, a diphenylmethyl group, a triphenylmethyl group, a benzyloxycarbonyl group, a methoxy- and/or ethoxycarbonyl group, a tert-butoxycarbonyl group, an allyloxycarbonyl group or a trifluoroacetyl group are particularly suitable. Thus, compounds of formula (I) with benzyl, diphenylmethyl, triphenylmethyl or benzyloxy-carbonyl groups as G can already be catalytically transformed into compounds of formula (I) with hydrogen as G at room temperature under mild conditions with elementary hydrogen or by transfer hydration. Compounds of formula (I) with a 4-methoxybenzyl group are converted to compounds of formula (I) with hydrogen as G by selective oxidation with ammonium-cer(IV)-nitrate. The cleavage of simple alkoxycarbonyl groups such as the methoxy- or ethoxycarbonyl group as well as the trifluoroacetyl group as **G** in compounds of formula (I) succeed by alkali hydrolysis under mild conditions without cleaving the A and D linked amide function. This is suitably valid for the cleavage of the triphenylmethyl group and the tert-butoxycarbonyl group in the form of **G** in compounds of formula (I) which occurs in acidic medium under mild conditions. Finally, compounds of formula (I) with an allyloxycarbonyl group as the meaning for G can be converted into such with hydrogen as the meaning for G in neutral medium with palladium catalyst. All these methods are fully familiar to the person skilled in the art, and are furthermore also documented in various monographs, see for example Greene, Wuts: Protective Groups in Organic Synthesis, New York, 1991.

The compounds of formula (I) produced according to the methods (A) to (B) can be isolated and purified in a known manner, for example by subjecting the residue after distillation of the solvent to partition, extraction, reprecipitation or re-crystallization or another purification method. For this, column chromatography on a suitable support or preparative middle or high pressure liquid chromatography (HPLC) are preferred for this.

The compounds (I) are first normally obtained in form of their free bases or their hydrates or solvates, depending on the type of isolation and purification. Their addition salts with pharmaceutically suitable acids are obtained in a typical manner by converting the base with the desired acid in a suitable solvent. Depending on the number of basic centers of compounds (I), one or more equivalent acids per mole of base can be bound.

Suitable solvents are, for example, chlorinated hydrocarbons such as dichloromethane or chloroform; ethers such as diethyl ether, dioxane or tetrahydrofuran; acetonitrile; ketones such as acetone or ethyl methyl ketone; esters such as methyl acetate or ethyl acetate or low molecular alcohols such as methanol, ethanol or isopropanol; and water. Pure solvents as well as mixtures of two or three solvents can also be used. The salts can be isolated by crystallization, precipitation or the evaporation of the solvent. Thereby, they optionally accumulate as hydrates or solvates.

The bases can be recovered from the salts by alkalization, for example with aqueous ammonia solution, alkali carbonate or diluted sodium hydroxide solution.

The following synthetic examples for end products as well as for starting products and/or intermediate products are meant for illustrating the method variants given above and claimed compounds:

### SYNTHETIC EXAMPLES for the end products of the invention according to formula (I)

In the production examples for the end products, the abbreviations stand for the following terms:
- MP =: melting point,
- RT =: room temperature,
- MPLC =: intermediate pressure liquid chromatography
- THF =: tetrahydrofuran,
- DMF =: dimethylformamide,
- abs. =: absolute,
- CDI =: carbonyldiimidazole,
- EDC =: N-(3-dimethylaminopropyl)-N'-ethyl-carbodiimide hydrochloride,
- HOBT =: 1-hydroxybenzotriazole,
- TEA =: triethylamine.

¹H-NMR-Spectrum = proton resonance spectrum, taken at 100 MHz. The chemical shifts are given in ppm against TMS as a standard (δ = 0.0), whereby
- s =: singlet,
- d =: doublet,
- t =: triplet,
- dt =: doublet-triplet,
- m =: multiplet,
- ar =: aromatic,
- py =: pyridine.

### Example 1

### N-[3-(4-diphenylmethylpiperazin-1-yl)-propoxy]-3-pyridin-3-yl-acrylamide (substance 30)

3.8 g (22,9 mmol) of 3-(3-pyridyl)-acrylic acid are suspended in 40 ml absolute dichloromethane and after addition of two drops pyridine, are cooled to ca. 0°C in an ice bath under moisture exclusion. 5 ml (58,5 mmol) of oxalyl chloride are slowly added and the mixture is first stirred for 30 min under ice cooling and then stirred overnight at RT.
Subsequently, the solvent and excess oxalyl chloride are distilled off on a rotary evaporator. In order to completely remove the oxalyl chloride, the colorless residue is dried further for two hours under high-vacuum. The acid chloride obtained in this manner is suspended without further purification in 30 ml absolute dichloromethane and cooled to ca. 0°C in an ice bath under moisture exclusion. 7.45 g (22.9 mmol) 3-(4-diphenylmethyl-piperazinyl)-propyl-hydroxylamine are dissolved in 40 ml absolute dichloromethane and are added dropwise to this suspension. After complete addition, the ice bath is removed and the reaction is stirred for a further two hours at RT. The mixture is subsequently concentrated, taken up in 10% sodium hydroxide solution and extracted three times with acetic acid ethyl ester. The combined organic phases are washed with saturated NaCl solution, dried over sodium sulfate and the solvent is removed under vacuum. The residue is chromatographically purified over silica gel with CHCl₃/CH₃OH (99/1 to 85/15) and crystallized twice from acetic ethyl acid ester after evaporation of the solvent: Colorless crystals with a MP. of 115 - 117°C; yield 3.66 g (35%).
- C₂₈H₃₂N₄O₂: (456.6)
- IR-Spectrum (KBr):: n(NH) 3200 cm⁻¹
n(C=O) 1660 cm⁻¹
n(C=C) 1630 cm⁻¹
- ¹H-NMR-Spectrum: (DMSO-D6):
1.50 - 1.90 (2H, m, C-CH₂-C)
2.05 - 2.65 (10H, m, piperazine, N-CH₂)
3.86 (2H, t, OCH₂, J=6.1 Hz)
4.24 (1H, s, Ar₂CH)
6.54 (1H, d, CH=CHCO, J=16.0 Hz)
7.05 - 7.70 (13H, m, Ar, Py, NH, CH=CHCO)
7.90 - 8.15 (1H, m, Py)
8.50 - 8.70 (1H, m, Py)
8.70 - 8.90 (1H, m, Py)

### Example 2

### N-[4-(4-diphenylphosphinoyl-piperazin-1-yl)-butyl]-3-pyridin-3-yl-acrylamide (substance 234)

2.7 g (18 mmol) 3-(3-pyridyl)-acrylic acid and 3.1 g (19 mmol) CDI are heated under reflux in 100 ml absolute THF under moisture exclusion. After an hour, this is cooled to RT and 7.0 g (19 mmol) 4-(4-diphenyl-phosphinoyl-piperazin-1-yl)-bu-tylamine, dissolved in 30 ml absolute THF, is added dropwise. Following addition, this is further stirred for three hours at RT and left standing overnight. The mixture is poured into 150 ml water and extracted three times with acetic acid ethyl ester by shaking. The combined organic phases are washed with saturated NaCl solution, dried over sodium sulphate and the solution is removed under vacuum. The residue is chromatographically pre-purified over silica gel with CHCl₃/CH₃OH (85/15). After removal of the solvent, the colorless oily residue is further purified by preparative middle pressure chromatography with CH₂Cl₂/CH₃OH (93/7): yield 3.5 g (40%) as amorphous solid material.
- C₂₈H₃₃N₄O₂P: (488.5)
- ¹H-NMR-Spectrum (CDCl₃):: 1.40 - 1.80 (4H, m, C-CH₂-CH₂-C)
2.20 - 2.60 (6H, m, piperazine, N-CH₂)
2.90 - 3.25 (4H, m, piperazine)
3.25 - 3.55 (2H, m, CONHCH₂)
6.53 (1H, d, CH=CHCO, J=15.7 Hz)
6.70 - 6.95 (1H, m, NH)
7.00 - 8.00 (13H, m, Ar, Py, CH=CHCO)
8.54 (1H, dd, Py, J=1.4Hz, J=4.8Hz)
8.70 (1H, d, Py, J=1.8Hz)

### Example 3

### N-[4-(4-diphenylmethyl-piperazin-1-yl)-3-hydroxy-butyl]-3-pyridin-3-yl-acrylamide (substance 29)

### Production analogues to Example 2.

Batch size: 2.0 g (13.3 mmol) 3-(3-pyridyl)-acrylic acid, 2.4 g (14.6 mmol) CDI and 4.5 g (13.3 mmol) 4-(4-diphenyl-methyl-piperazin-1-yl)-3-hydroxy-butylamine. The addition of the amine occurs at -10°C. Subsequently, this is stirred further for an hour at 0°C.

In the purification, chromatography first occurs with CHCl₃/CH₃OH (95/5); subsequently, crystallization occurs once from 20 ml ethanol and twice each from 50 ml acetic acid ethyl ester: Colorless crystals of MP. 168 - 171°C; yield 0.4 g (6%).
- C₂₉H₃₄N₄O₂: (470.6)
- IR-Spectrum (KBr):: n(NH) 3270 cm⁻¹
n(C=O) 1660, 1565 cm⁻¹
n(C=C) 1615 cm⁻¹
- ¹H-NMR-Spectrum (CDCl₃):: 1.25 - 2.00 (2H, m, C-CH₂-C)
2.05 - 2.85 (10H, m, piperazine, N-CH₂)
3.00 - 3.60 (2H, m, CH-OH)
3.60 - 4.00 (2H, m, CONHCH₂)
4.22 (1H, s, Ar₂CH)
6.44 (1H, d, CH=CHCO, J=15.7 Hz)
6.60 - 8.85 (1H, m, NH)
6.95 - 7.60 (11H, m, Ar, Py)
7.58 (1H, d, CH=CHCO, J=15.7 Hz)
7.65 - 7.90 (1H, m, Py)
8.45 - 8.65 (1H, m, Py)
8.65 - 8.85 (1H, m, Py)

### Example 4

### N-[4-(4-diphenylmethyl-piperazin-1-yl)-4-oxo-butyl]-3-pyridin-3-yl-acrylamide (substance 31)

### Production analogous to Example 1.

Batch size: 2.3 g (15.4 mmol) 3-(3-pyridyl)-acrylic acid, 4 ml (46.8 mmol) oxalyl chloride and 5.2 g (15.4 mmol) 4-(4-diphenyl-methyl-piperazin-1-yl)-4-oxo-butylamine.

In the purification, chromatography first occurs with CHCl₃/CH₃OH (90/10); subsequently, crystallization occurs twice from 400 ml acetic acid ethyl ester and 300 ml ethyl methyl ketone: colorless crystals of MP. 179 - 180°C; yield 3.2 g (45%).
- C₂₉H₃₂N₄O₂: (468.6)
- IR-Spectrum (Kbr):: n(NH) 3240 cm⁻¹
n(C=O) 1665, 1550 cm⁻¹
n(C=C) 1630 cm⁻¹
- ¹H-NMR-Spectrum (CDCl₃):: 1.70 - 2.10 (2H, m, C-CH₂-C)
2.15 - 2.65 (6H, m, piperazine, CO-CH₂)
3.20 - 3.80 (6H, m, CONHCH₂, piperazine)
4.20 (1H, s, Ar₂CH)
6.45 (1H, d, CH=CHCO, J=15.7 Hz)
6.75 - 7.00 (1H, m, NH)
7.05 - 7.55 (11H, m, Ar, Py)
7.58 (1H, d, CH=CHCO, J=15.7 Hz)
7.70 - 7.90 (1H, m, Py)
8.45 - 8.65 (1H, m, Py)
8.65 - 8.85 (1H, m, Py)

### Example 5

### N-[3-(4-diphenylmethyl-piperazin-1-yl-sulfonyl)-propyl]-3-pyridin-3-yl-acrylamide (substance 32)

### Production analogous to Example 1.

Batch size: 0.5 g (3.3 mmol) 3-(3-pyridyl)-acrylic acid, 2 ml (23.4 mmol) oxalyl chloride and 1.24 g (3.3 mmol) 3-(4-diphenyl-methyl-piperazin-1-yl-sulfonyl)-propylamine. In the purification, chromatography first occurs CHCl₃/CH₃OH (95/5); subsequently, crystallization occurs from 75 ml acetic acid ethyl ester: colorless crystals of MP. 167 - 168°C; yield 0.7 g (84%).
- C₂₈H₃₂N₄O₃S: (504.6)
- IR-Spectrum (KBr):: n(NH) 3360 cm⁻¹
n(C=O) 1660, 1540 cm⁻¹
n(C=C) 1630 cm⁻¹
- ¹H-NMR-Spectrum (CDCl₃) :: 2.00 - 2.35 (2H, m, C-CH₂-C)
2.35 - 2.65 (4H, m, piperazine)
3.00 (2H, t, SO₂-CH₂, J=7.1 Hz)
3.20 - 3.40 (4H, m, piperazine)
3.57 (2H, dt, CONHCH₂, J=6.4 Hz, J=12.8 Hz)
4.27 (1H, s, Ar₂CH)
6.10 - 6.35 (1H, m, NH)
6.47 (1H, d, CH=CHCO, J=15.7 Hz)
7.05 - 7.55 (11H, m, Ar, Py)
7.63 (1H, d, CH=CHCO, J=15.7 Hz)
7.70 - 7.95 (1H, m, Py)
8.50 - 8.65 (1H, m, Py)
8.70 - 8.85 (1H, m, Py)

### Example 6

### N-{2-[2-(4-diphenylmethyl-piperazin-1-yl)-ethoxy]-ethyl}-3-pyridin-3-yl-acrylamide trihydrochloride (substance 35 as trihydrochloride)

### Production analogous to Example 1.

Batch size: 6.0 g (40 mmol) 3-(3-pyridyl)-acrylic acid, 9.4 ml (110 mmol) oxalyl chloride and 12.4 g (36.5 mmol) 2-[2-(4-diphenylmethyl-piperazin-1-yl)-ethoxy]-ethylamine. The crude product is chromatographically pre-purified over silica gel with CHCl₃/CH₃OH (98/2 to 95/5). After removal of the solvent, the residue is dissolved in isopropanol and mixed with isopropanolic HCl solution. The mixture is rotated in and the HCl salt is cyrstallized from 50 ml methanol/6 drops diisopropyl ether: Colorless crystals of MP. 157 - 159°C; yield 0.6 g (3%).
- C₂₉H₃₄N₄O₂ • 3HCl: (580.0)
- IR-Spectrum (Kbr) :: n(NH) 3240 cm⁻¹
n(C=O) 1670, 1550 cm⁻¹
n(C=C) 1630 cm⁻¹
- ¹H-NMR-Spectrum (CD₃OD) :: 3.40 - 4.00 (16H, m, piperazine, CH₂-CH₂-O-CH₂-CH₂)
5.58 (1H, s, Ar₂CH)
7.14 (1H, d, CH=CHCO, J=15.8 Hz)
7.30 - 7.95 (10H, m, Ar)
7.67 (1H, d, CH=CHCO, J=15.8 Hz)
8.10 - 8.25 (1H, m, Py)
8.80 - 9.00 (2H, m, Py)
9.15 - 9.20 (1H, m, Py)

### Example 7

### N-{4-[4-(bis-(4-fluorophenyl)-methyl)-piperazin-1-yl]-but-2-in-yl}-3-pyridin-3-yl-acrylamide•trihydrochloride (substance 47 as trihydrochloride)

### Production analogous to Example 1.

Batch size: 2.5 g (16.9 mmol) 3-(3-pyridyl)-acrylic acid, 2 ml (23 mmol) oxalyl chloride and 6.0 g (16.9 mmol) 4-{4-[bis-(4-fluorophenyl)-methyl]-piperazin-1-yl}-but-2-inylamine. The crude product is chromatographically pre-purified over silica gel with CHCl₃/CH₃OH (95/5). After removal of the solvent, the residue is dissolved in methanol and mixed with methanolic HCl solution. The drawn off HCl salt is first crystallized from isopropanol and subsequently from ethanol/diisopropylether: Colorless crystals of MP. 160 - 163°C; yield 3.5 g (35%).
- C₂₉H₂₈F₂N₄O • 3HCl: (595.9)
- IR-Spectrum (KBr):: n(NH) 3240 cm⁻¹
n(C=O) 1670, 1550 cm⁻¹
n(C=C) 1630 cm⁻¹
- ¹H-NMR-Spectrum (D₂O) :: 2.95 - 3.55 (8H, m, piperazine)
3.80 - 4.10 (4H, m, CH₂-CC-CH₂)
5.04 (1H, s, Ar₂CH)
6.72 (1H, d, CH=CHCO, J=15.9 Hz)
6.85 - 7.60 (9H, m, Ar, CH=CHCO)
7.80 - 8.00 (1H, m, Py)
8.50 - 8.70 (2H, m, Py)
8.70 - 8.85 (1H, m, Py)

### Example 8

### 3-pyridin-3-yl-N-{4-[4-(1,2,3,4-tetrahydronaphthalin-1-yl)-piperazin-1-yl]-butyl}-acrylamide (substance 87)

### Production analogous to Example 2.

Batch size: 2.7 g (18 mmol) 3-(3-pyridyl)-acrylic acid, 3.15 g (19 mmol) CDI and 5.0 g (17.4 mmol) 4-[4-(1,2,3,4-tetrahydro-naphthalin-1-yl)-piperazin-1-yl]-butylamine. In the purification, chromatography first occurs with CHCl₃/CH₃OH (95/5 to 90/10); subsequently, crystallization occurs twice each from 40 ml 1-chlorobutane: Colorless crystals of MP. 110 - 114°C; yield 3.7 g (50%).
- C₂₆H₃₄N₄O: (418,6)
- IR-Spectrum (Kbr):: n(NH) 3300 cm⁻¹
n(C=O) 1650, 1530 cm⁻¹
n(C=C) 1620 cm⁻¹
- ¹H-NMR-Spectrum (CDCl₃):: 1.40 - 2.10 (8H, m, C-CH₂-CH₂-C, cyclohexyl)
2.25 - 3.10 (12H, m, piperazine, N-CH₂, cyclohexyl)
3.30 - 3.60 (2H, m, CONHCH₂)
3.70 - 4.00 (1H, m, cyclohexyl)
6.50 (1H, d, CH=CHCO, J=15.7 Hz)
6.90 - 7.45 (5H, m, Ar, Py, NH)
7.62 (1H, d, CH=CHCO, J=15.7 Hz)
7.50 - 7.90 (2H, m, Py, Ar)
8.50 - 8.65 (1H, m, Py)
8.70 - 8.80 (1H, m, Py)

### Example 9

### 3-pyridin-3-yl-N-[4-(4-{5,6,7,8-tetrahydro-naphthalin-1-yl}-piperazin-1-yl)-butyl]-acrylamide (substance 94)

1.6 g (11.1 mmol) 3-(3-pyridyl)-acrylic acid and 6.2 ml (44.3 mmol) TEA are suspended in 80 ml absolute dichloromethane and cooled to ca. 0°C under moisture exclusion. 2.0 g (12.1 mmol) 81% HOBT and 2.3 g (12.1 mmol) EDC are added and the mixture is stirred for 30 min under ice cooling. 4.0 g (10.1 mmol) 4-[4-(5,6,7,8-tetrahydro-naphthalin-1-yl)-piperazin-1-yl]-butylamine are added and the mixture is stirred without cooling overnight at RT. Subsequently, the batch is washed twice with 25 ml 2M sodium hydroxide solution and 25 ml water. The organic phase is dried over sodium sulfate and the solvent is removed under vacuum. The residue is chromatographically pre-purified over silica gel with CHCl₃/CH₃OH (95/5) and crystallized twice from acetonitrile (25 ml and 15 ml): Colorless crystals of MP. 108-109°C; yield 2.7 g (64%).
- C₂₆H₃₄N₄O: (418.6)
- IR-Spectrum (Kbr):: n(NH) 3260 cm⁻¹
n(C=O) 1650, 1555 cm⁻¹
n(C=C) 1620 cm⁻¹
- ¹H-NMR-Spectrum (CDCl₃):: 1.40 - 2.10 (8H, m, C-CH₂-CH₂-C, cyclohexyl)
2.25 - 3.30 (14H, m, piperazine, N-CH₂, cyclohexyl)
3.30 - 3.70 (2H, m, CONHCH₂)
6.51 (1H, d, CH=CHCO, J=15.6 Hz)
6.70 - 7.45 (5H, m, Ar, Py, NH)
7.63 (1H, d, CH=CHCO, J=15.6 Hz)
7.70 - 7.95 (1H, m, Py)
8.45 - 8.65 (1H, m, Py)
8.65 - 8.85 (1H, m, Py)

### Example 10

### N-{4-[(naphthalin-1-yl)-piperazin-1-yl]-butyl}-3-pyridin-3-yl-acrylamide (substance 97)

### Production analogous to Example 1.

Batch size: 3.38 g (22.7 mmol) 3-(3-pyridyl)-acrylic acid, 7.85 g (61.8 mmol) oxalyl chloride and 8.55 g (20.6 mmol) 4-[(naphtha-lin-1-yl)-piperazin-1-yl]-butylamine. In the recovery, 40 ml 10% sodium hydroxide solution is added to the reaction solution. The aqueous phase is extracted with dichloromethane. The combined organic phases are washed with 15 ml water, dried over sodium sulfate and the solvent is removed under vacuum. The residue is chromatographically purified over silica gel with CHCl₃/CH₃OH (98/2 to 96/4). After removal of the solvent, this is crystallized three times each with 40 ml acetic acid ethyl ester under addition of 5 drops diisopropylether respectively: solid with MP. 124-125°C; yield 1.5 g (18%).
- C₂₆H₃₀N₄O: (414.6)
- IR-Spectrum (KBr) :: n(NH) 3280 cm⁻¹
n(C=O) 1650, 1545 cm⁻¹
n(C=C) 1620 cm⁻¹
- ¹H-NMR-Spectrum (CDCl₃):: 1.45 - 1.95 (4H, m, C-CH₂-CH₂-C)
2.30 - 3.40 (10H, m, piperazine, N-CH₂)
3.30 - 3.60 (2H, m, CONHCH₂)
6.50 (1H, d, CH=CHCO, J=15.7 Hz)
6.55 - 6.85 (1H, m, NH)
6.95 - 7.95 (8H, m, Ar, Py)
7.63 (1H, d, CH=CHCO, J=15.7 Hz)
8.05 - 8.35 (1H, m, Py)
8.50 - 8.70 (1H, m, Py)
8.70 - 8.85 (1H, m, Py)

### Example 11

### N-{2-[4-(6,11-dihydrodibenzo[b,e]thiepin-11-yl)-piperazin-1-yl]-ethyl}-3-pyridin-3-yl-acrylamide (substance 138)

5.0 g (14.1 mmol) N-[2-(piperazin-1-yl)-ethyl]-3-pyridin-3-yl-acrylamidetrihydrochloride (substance 317 as trihydrochloride) and 5.8 ml (42.3 mmol) TEA are suspended in 65 ml absolute dichloromethane. 4.7 g (15.5 mmol) 11-methanesulfonyloxy-6,11-dihydrodibenzo[b,e]thiepine dissolved in 60 ml absolute dichloromethane is added dropwise under moisture exclusion. The mixture is stirred overnight at RT. Subsequently, the batch is washed three times each with 50 ml water. The organic phase is dried over sodium sulfate and the solvent is removed under vacuum. The residue is chromatographically purified over silica gel with CHCl₃/CH₃OH (95/5). Subsequently, a further purification occurs by means of MPLC with CHCl₃/CH₃OH (95/5): yield 4.3 g (44%) as an amorphic solid.
- C₂₈H₃₀N₄OS: (470,6)
- IR-Spectrum (Kbr):: n(NH) 3270 cm⁻¹
n(C=O) 1655, 1535 cm⁻¹
n(C=C) 1620 cm⁻¹
- ¹H-NMR-Spectrum (CDCl₃) :: 2.00 - 2.90 (10H, m, Piperazine, Piperazin-CH₂)
3.20 - 3.70 (3H, m, CONHCH₂, SCH₂)
4.10 (1H, s, Ar₂CH)
5.90 - 6.50 (2H, m, NH, SCH₂)
6.49 (1H, d, CH=CHCO, J=15.7 Hz)
6.90 - 7.50 (9H, m, Ar, Py)
7.62 (1H, d, CH=CHCO, J=15.7 Hz)
7.75 - 7.95 (1H, m, Py)
8.50 - 8.70 (1H, m, Py)
8.70 - 8.90 (1H, m, Py)

### Example 12

### N-[4-(4-biphenyl-2-yl-piperazin-1-yl)-butyl]-3-pyridin-3-yl-acrylamide (substance 68)

### Production analogous to Example 1.

Batch size: 5.8 g (39.1 mmol) 3-(3-pyridyl)-acrylic acid, 9.1 ml (106 mmol) oxalyl chloride and 5.0 g (16.1 mmol) 4-(4-biphenyl-2-yl-piperazin-1-yl)-butylamine.
In the recovery, 60 ml 10% sodium hydroxide solution is added to the reaction solution. The aqueous phase is extracted twice each with 15 ml dichloromethane. The combined organic phases are washed twice each with 15 ml water, dried over sodium sulfate and the solvent is removed under vacuum. The residue is chromatographically purified over silica gel with CHCl₃/CH₃OH/NH₄OH (85/15/2). After removal of the solvent, this is crystallized twice with 1-chlorobutane: solid with MP. 115°C; yield 3.3 g (46%).
- C₂₈H₃₂N₄O: (440.6)
- IR-Spectrum (Kbr) :: n(NH) 3280 cm⁻¹
n(C=O) 1650, 1545 cm⁻¹
n(C=C) 1620 cm⁻¹
- ¹H-NMR-Spectrum (CDCl₃):: 1.35 - 1.85 (4H, m, C-CH₂-CH₂-C)
2.10 - 2.55 (6H, m, piperazine, N-CH₂)
2.75 - 3.00 (4H, m, piperazine)
3.20 - 3.50 (2H, m, CONHCH₂)
6.44 (1H, d, CH=CHCO, J=15.6 Hz)
6.45 - 6.70 (1H, m, NH)
6.95 - 7.85 (12H, m, Ar, CH=CHCO, Py)
8.45 - 8.65 (1H, m, Py)
8.65 - 8.80 (1H, m, Py)

### Example 13

### N-{4-[4-(9H-fluoren-9-yl)-piperazin-1-yl]-butyl}-3-pyridin-3-yl-acrylamide (substance 101)

### Production analogous to Example 1.

Batch size: 19.2 g (129 mmol) 3-(3-pyridyl)-acrylic acid, 15.1 ml (176 mmol) oxalyl chloride and 37.6 g (117 mmol) 4-(9-fluoroenyl-piperazin-1-yl)-butylamine. In the recovery, 150 ml 10% sodium hydroxide solution is added to the reaction solution. The organic phase is washed three times each with 60 ml water, dried over sodium sulfate and the solvent is removed under vacuum. The residue is chromatographically purified over silica gel with CHCl₃/CH₃OH (90/10). After removal of the solvent, this is crystallized with 1700 ml acetic acid ethyl ester: solid with MP. 145-147°C; yield 39.0 g (73%).
- C₂₉H₃₂N₄O: (452.6)
- IR-Spectrum (Kbr):: n(NH) 3300 cm⁻¹
n(C=O) 1655, 1530 cm⁻¹
n(C=C) 1620 cm⁻¹
- ¹H-NMR-Spectrum (CDCl₃):: 1.35 - 1.80 (4H, m, C-CH₂-CH₂-C)
2.10 - 2.80 (10H, m, piperazine, N-CH₂)
3.20 - 3.50 (2H, m, CONHCH₂)
4.81 (1H, s, Ar₂CH)
6.34 (1H, d, CH=CHCO, J=15.7 Hz)
6.75 - 7.05 (1H, m, NH)
7.00 - 7.80 (11H, m, Ar, CH=CHCO, Py)
8.40 - 8.60 (1H, m, Py)
8.60 - 8.70 (1H, m, Py)

### Example 14

### N-{4-[4-(10,11-dihydro-5H-dibenzo[a,d]cyclohepten-5-yl)-piperazin-1-yl]-butyl}-3-pyridin-3-yl-acrylamide (substance 119)

### Production analogous to Example 1.

Batch size: 4.16 g (27.9 mmol) 3-(3-pyridyl)-acrylic acid, 6.5 ml (76.2 mmol) oxalyl chloride and 12.2 g (25.4 mmol) 4-[4-(10, 11-dihydro-5H-dibenzo[a,d]cyclohepten-5-yl)-piperazin-1-yl]-butylamine.
In the recovery, 50 ml 10% sodium hydroxide solution is added to the reaction solution. The aqueous phase is extracted with dichloromethane. The combined organic phases are washed with 50 ml water, dried over sodium sulfate and the solvent is removed under vacuum. The residue is chromatographcially pre-purified over silica gel with CHCl₃/CH₃OH (98/2 to 90/10). After removal of the solvent, this is crystallized four-fold each with 50 ml acetic acid ethyl ester: Colorless solid with MP. 119-120°C; yield 4.9 g (40%).
- C₃₁H₃₆N₄O: (480.7)
- IR-Spectrum (Kbr):: n(NH) 3280 cm⁻¹
n(C=O) 1670, 1540 cm⁻¹
n(C=C) 1625 cm⁻¹
- ¹H-NMR-Spectrum (CDCl₃):: 1.40 - 1.80 (4H, m, C-(CH₂)₂-C)
2.10 - 2.55 (10H, m, piperazine, N-CH₂)
2.55 - 3.00 (2H, m, Ar-CH₂-CH₂-Ar)
3.20 - 3.50 (2H, m, CONHCH₂)
4.75 - 4.20 (3H, m, Ar₂CH, Ar-CH₂-CH₂-Ar)
6.50 (1H, d, CH=CHCO, J=15.7 Hz)
6.90 - 7.40 (10H, m, Ar, Py, NH)
7.61 (1H, d, CH=CHCO, J=15.7 Hz)
7.70 - 7.85 (1H, m, Py)
8.50 - 8.65 (1H, m, Py)
8.70 - 8.80 (1H, m, Py)

### Example 15

### N-[2-(4-diphenylacetyl-piperazin-1-yl)-ethyl]-3-pyridin-3-yl-acrylamide (substance 158)

8.0 g (22.6 mmol) N-[2-(piperazin-1-yl)-ethyl]-3-pyridin-3-yl-acrylic amide trihydrochloride (substance 317 as a trihydrochloride) and 13 ml (92.7 mmol) TEA are present in 100 ml absolute dichloromethane and cooled to ca. 0°C under moisture exclusion. 6.3 g (24.9 mmol) diphenylacetyl chloride (90%) are dissolved in 70 ml absolute dichloromethane and added dropwise. The mixture is stirred overnight at RT without further cooling. Subsequently, 200 ml dichloromethane are added and the batch is washed three times each with 100 ml water. The organic phase is dried over sodium sulfate and the solvent is removed under vacuum. The residue is crystallized twice from 40 ml and 30 ml acetonitrile. Beige coloured crystals of MP. 174°C. yield 4.6 g (44%).
- C₂₈H₃₀N₄O₂: (454.6)
- IR-Spectrum (Kbr):: n(NH) 3320 cm⁻¹
n(C=O) 1675, 1550 cm⁻¹
n(C=C) 1610 cm⁻¹
- ¹H-NMR-Spectrum (CDCl₃) :: 2.10 - 2.60 (6H, m, piperazine, N-CH₂)
3.30 - 3.85 (6H, m, piperazine, CONHCH₂)
5.20 (1H, s, Ar₂CH)
6.20 - 6.40 (1H, m, NH)
6.46 (1H, d, CH=CHCO, J=15.7 Hz)
7.10 - 7.40 (11H, m, Ar, Py)
7.61 (1H, d, CH=CHCO, J=15.7 Hz)
7.70 - 7.90 (1H, m, Py)
8.50 - 8.65 (1H, m, Py)
8.70 - 8.80 (1H, m, Py)

### Example 16

### N-{2-[4-(10,11-dihydro-dibenzo[b,f]azepin-5-yl-carbonyl)-piperazin-1-yl]-ethyl}-3-pyridin-3-yl-propionamide (substance 215)

### Production analogous to Example 15.

Batch size: 8.0 g (21.5 mmol) N-[2-(piperazin-1-yl)-ethyl]-3-pyridin-3-yl-propionamide·trihydrochloride, 12.3 ml (88.1 mmol) TEA and 6.1 g (23.6 mmol) 10,11-dihydro-dibenzo[b,f]azepin-5-carbonyl chloride in 170 ml absolute dichloromethane.
In the purification, this is first chromatographically pre-purified over silica gel with CHCl₃/CH₃OH (100/0 to 90/10) and, after removal of the solvent, crystallized from 10 ml acetonitrile. Colorless crystals of MP. 146 - 147°C. yield 0.7 g (6%).
- C₂₉H₃₃N₅O₂: (483.6)
- IR-Spectrum (KBr):: n(NH) 3330 cm⁻¹
n(C=O) 1660, 1535 cm⁻¹
n(C=C) 1630 cm⁻¹
- ¹H-NMR-Spectrum (CDCl₃) :: 2.10 - 2.60 (8H, m, piperazine, CO-CH₂, N-CH₂)
2.96 (2H, t, Py-CH₂, J=7.4 Hz)
3.10 - 3.45 (10H, m, CONHCH₂, piperazine, Ar-CH₂-CH₂-Ar)
5.80 - 6.00 (1H, m, NH)
7.00 - 7.60 (10H, m, Ar, Py)
8.35 - 8.55 (2H, m, Py)

### Example 17

### N-{2-[4-(naphthalin-2-yl-sulfonyl)-piperazin-1-yl]-ethyl}-3-pyridin-3-yl-acrylamide (substance 227)

### Production analogous to Example 15.

Batch size: 8.0 g (22.6 mmol) N-[2-(piperazin-1-yl)-ethyl]-3-pyridin-3-yl-acrylamide·trihydrochloride (substance 317 as a trihydrochloride), 13 ml (92.7 mmol) TEA and 5.6 g (24.9 mmol) naphthalin-2-sulfonic acid chloride in 180 ml absolute dichloromethane.
For purification, this is crystallized twice from 150 ml and 100 ml acetonitrile. Beige coloured crystals of MP. 183 - 184°C. yield 4.0 g (39%).
- C₂₄H₂₆N₄O₃S: (450.6)
- IR-Spectrum (Kbr):: n(NH) 3250 cm⁻¹
n(C=O) 1665, 1555 cm⁻¹
n(C=C) 1625 cm⁻¹
- ¹H-NMR-Spectrum (CDCl₃) :: 2.35 - 2.80 (6H, m, piperazine, N-CH₂)
3.00 - 3.35 (4H, m, piperazine)
3.44 (2H, dd, CONHCH₂, J=5.5 Hz, J=11.2 Hz)
5.90 - 6.15 (1H, m, NH)
6.35 (1H, d, CH=CHCO, J=15.6 Hz)
7.15 - 8.15 (9H, m, Ar, CH=CHCO, Py)
8.35 (1H, bs , Ar)
8.45 - 8.60 (1H, m, Py)
8.60 - 8.75 (1H, m, Py)

### Example 18

### N-{2-[4-(tert-butoxycarbonyl)-piperazin-1-yl]-ethyl]-3-pyridin-3-yl-acrylamide (substance 374)

### Production analogous to Example 1.

Batch size: 36.1 g (242 mmol) 3-(3-pyridyl)-acrylic acid, 23.1 ml (264 mmol) oxalyl chloride, 50 g (15.4 mmol) 2-[4-(tert-but-oxycarbonyl)-piperazin-1-yl]-ethylamine and 30.4 ml (220 mmol) TEA in 400 ml absolute dichloromethane.
In the purification, this is crystallized twice from 100 ml acetic acid ethyl ester and 150 ml 1-chlorbutane: Colorless crystals of MP. 92 - 93°C; yield 38.4 g (48%).
- C₁₉H₂₈N₄O₃: (360.5)
- IR-Spectrum (Kbr):: n(NH) 3320 cm⁻¹
n(C=O) 1670, 1530 cm⁻¹
n(C=C) 1620 cm⁻¹
- ¹H-NMR-Spectrum (CDCl₃):: 1.47 (9H, s, tert. Butyl )
2.30 - 2.80 (6H, m, piperazine, N-CH₂)
3.30 - 3.70 (6H, m, piperazine CONHCH₂)
6.30 - 6.55 (1H, m, NH)
6.52 (1H, d, CH=CHCO, J=15.7 Hz)
7.20 - 7.40 (1H, m, Py)
7.63 (1H, d, CH=CHCO, J=15.7 Hz)
7.70 - 7.90 (1H, m, Py)
8.45 - 8.65 (1H, m, Py)
8.65 - 8.85 (1H, m, Py)

### Example 19

### N-[2-(piperazin-1-yl)-ethyl]-3-pyridin-3-yl-acrylamide•trihydrochloride (substance 317 as a trihydrochloride)

38 g (105 mmol) N-{2-[4-(tert-butoxycarbonyl)-piperazin-1 yl]-ethyl]-3-pyridin-3-yl-acrylamide (substance 374) are dissolved in 380 ml methanol and 42 ml concentrated hydrochloric are added. The mixture is stirred for four hours under reflux. After cooling of the solution, the solvent is removed under vacuum. The residue is crystallized from 185 ml methanol. Colorless crystals of MP. 216 -226°C (under decomposition). yield 34.8 g (93%).
- C₁₄H₂₀N₄O·3HCl: (369,7)
- IR-Spectrum (Kbr):: n(NH) 3150 cm⁻¹
n(C=O) 1670, 1540 cm⁻¹
n(C=C) 1610 cm⁻¹
- ¹H-NMR-Spectrum (D₂O):: 3.20 - 3.75 (12H, m, piperazine, N-CH₂-CH₂)
6.74 (1H, d, CH=CHCO, J=15.9 Hz)
7.44 (1H, d, CH=CHCO, J=15.9 Hz)
7.80 - 8.00 (1H, m, Py)
8.50 - 8.70 (2H, m, Py)
8.80 - 8.90 (1H, m, Py)

In the following Table 2, further synthesized end products according to formula (I) are listed:

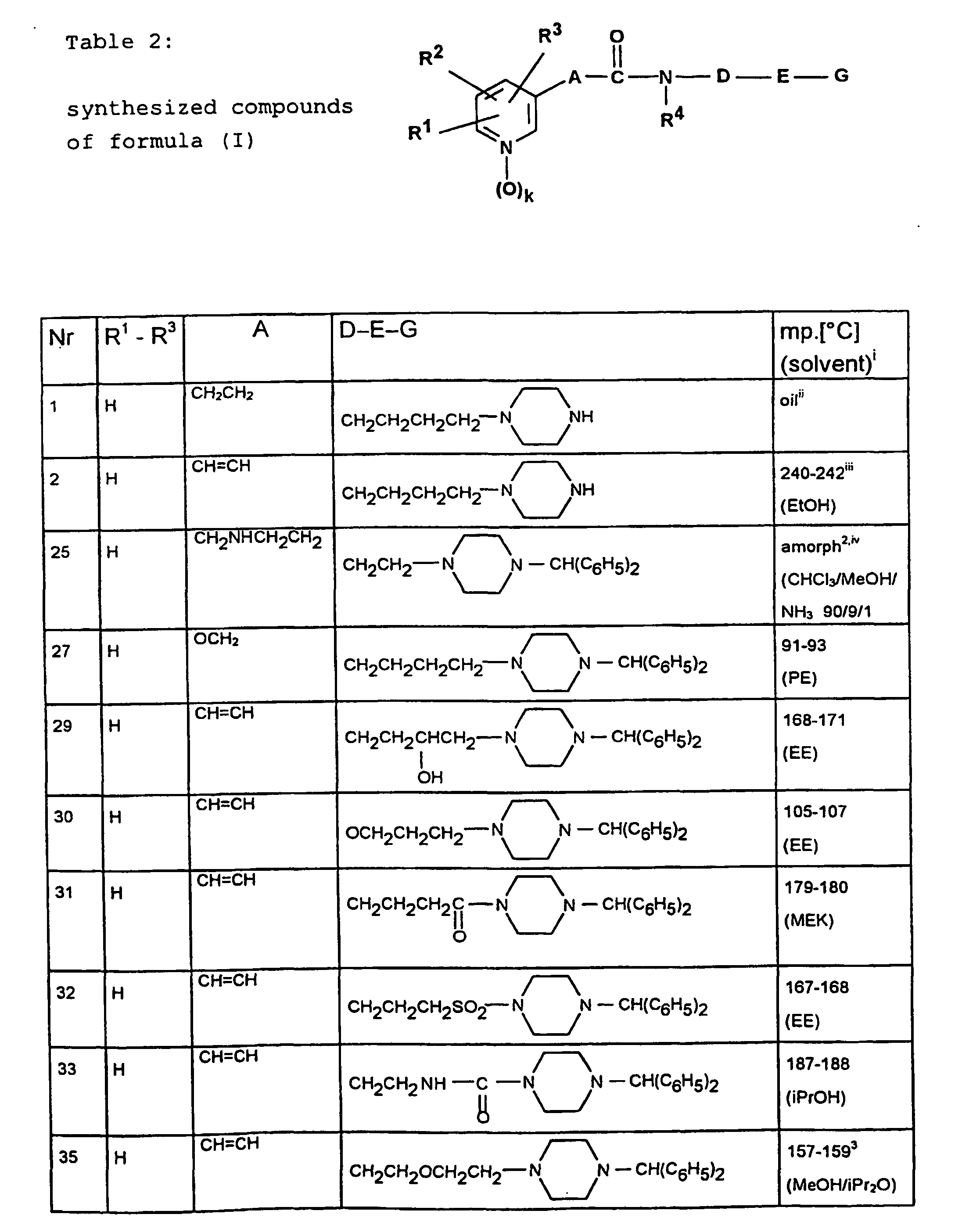

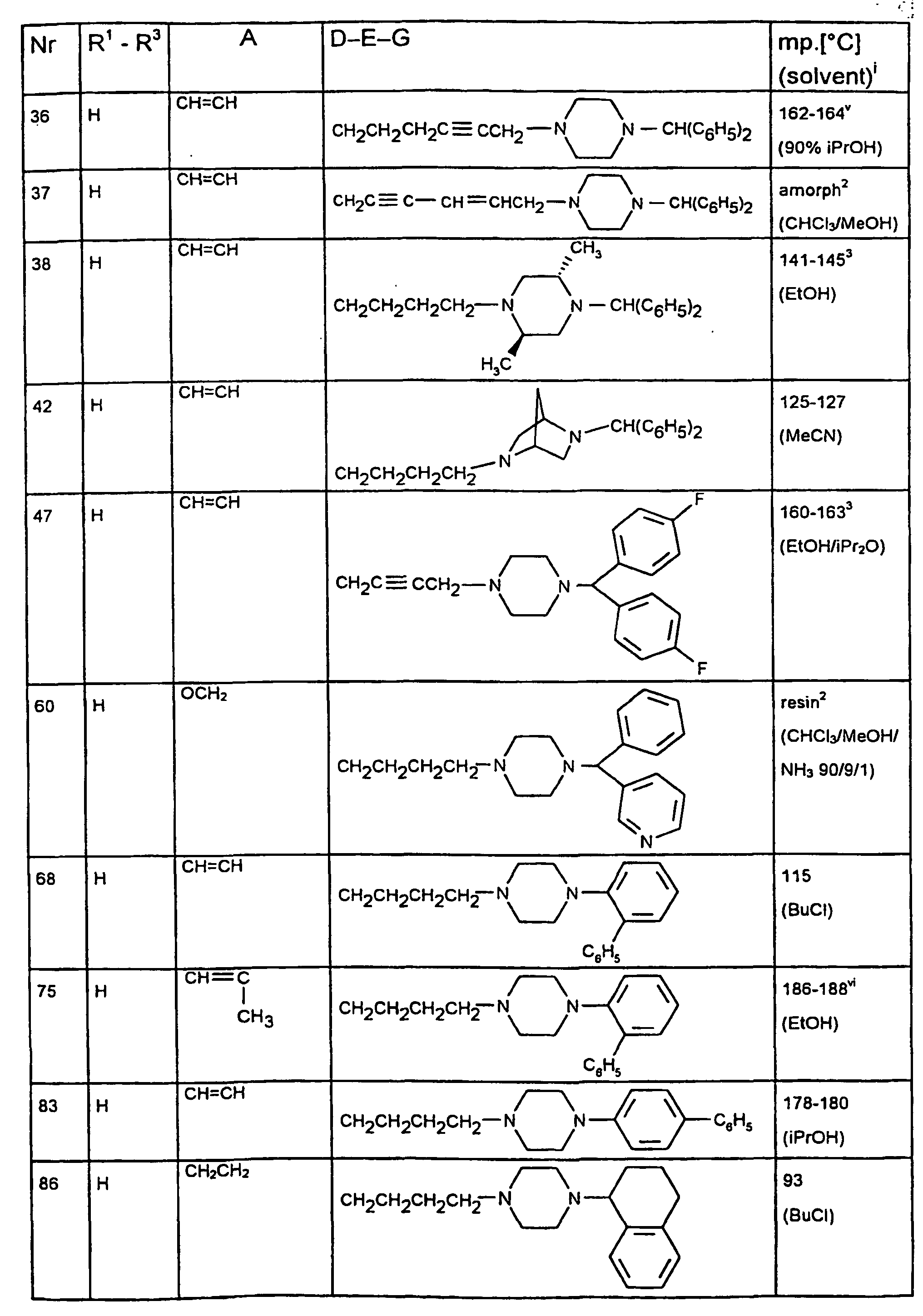

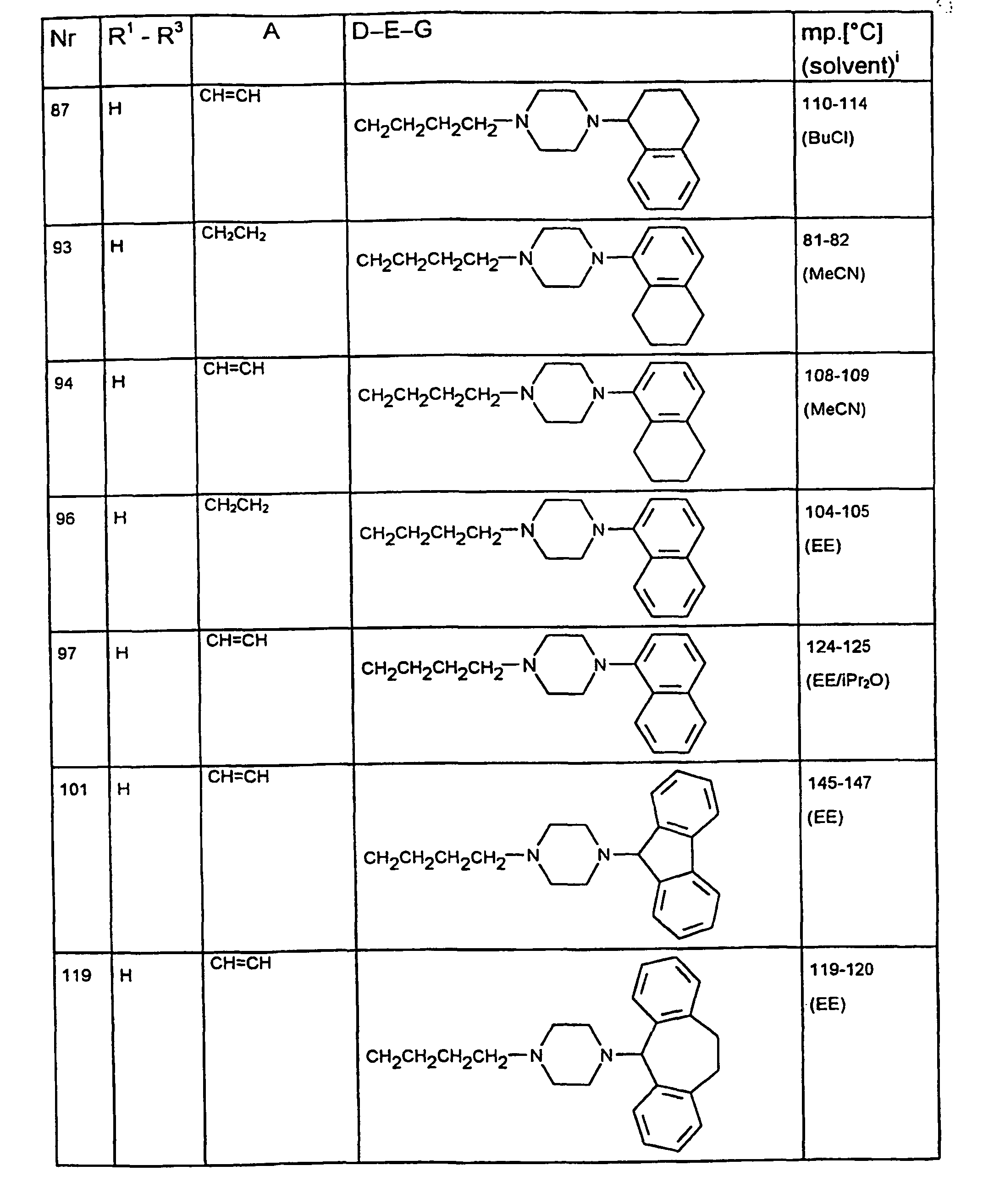

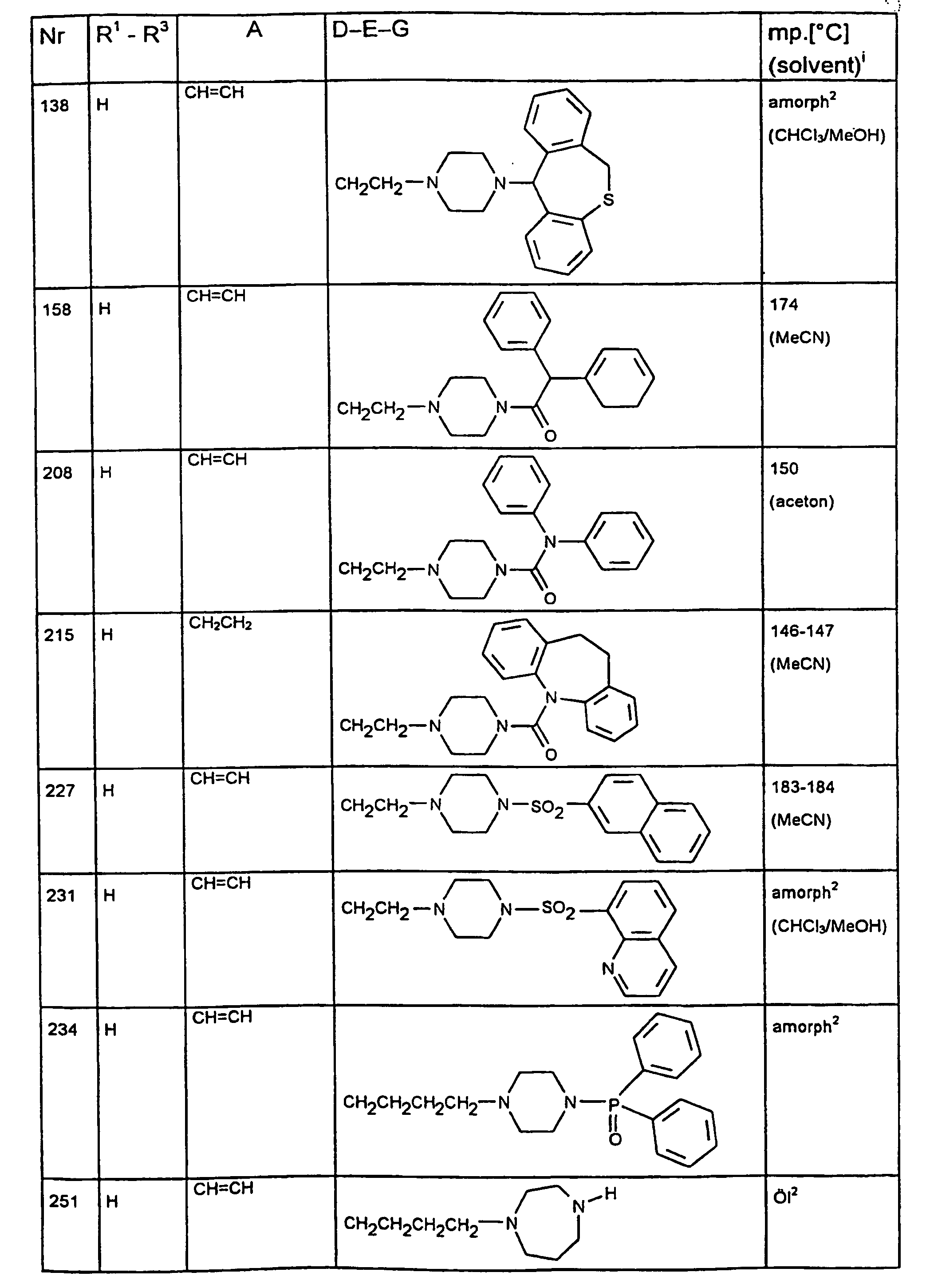

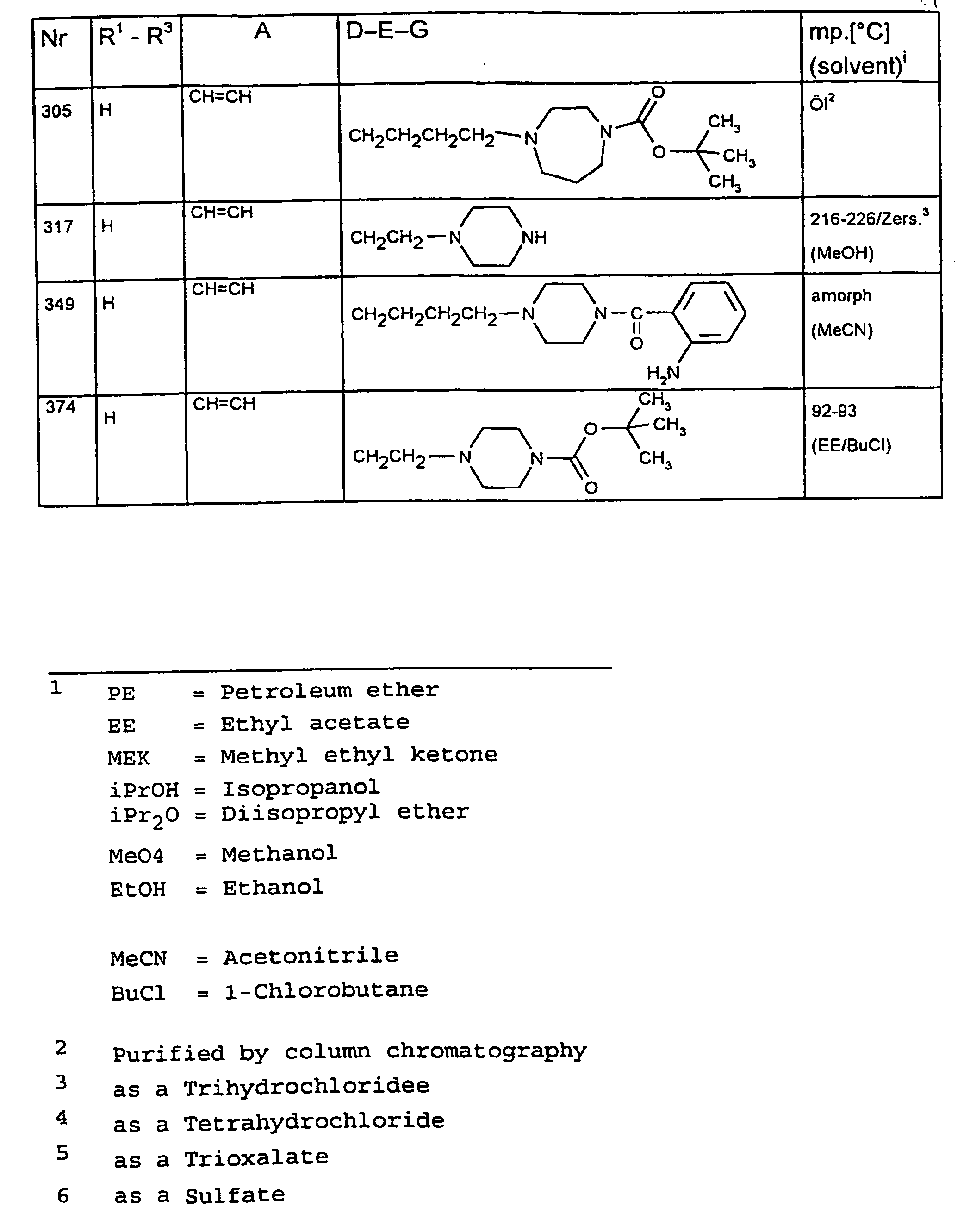

### Production of the Starting Substances

### Example A

### 4-(4-diphenylphosphinoyl-piperazin-1-yl)-butylamine

### a) N-diphenylphosphinoyl-piperazine:

20 g (84.5 mmol) diphenylphosphinate chloride dissolved in 30 ml DMF are added dropwise to a solution of 21.8 g (253 mmol) piperazine in DMF. After four hours, the solution is concentrated under vacuum, taken up in chloroform and extracted by shaking with 10% hydroxide solution. The organic phase is dried over sodium sulfate and solvent is removed under vacuum. The residue is chromatographically purified over silica gel with CHCl₃/CH₃OH/TEA (90/10/0 to 90/10/6): yield 12 g (49%).

### b) 2-[4-(4-diphenylphosphinoyl)-butyl]-isoindolin-1,3-dione:

12 g (41.9 mmol) N-diphenylphosphinoyl-piperazine, 11.8 g (42 mmol) N-(4-bromobutyl)-phthalimide, 5.8 g (42 mmol) potassium carbonate and 1.4 g (8 mmol) potassium iodide are heated in ethyl methyl ketone for 6 hours under reflux. After cooling, the reaction mixture is concentrated under vacuum. The residue is taken up in acetic acid ethyl ester and extracted by shaking with water. The organic phase is dried over sodium sulfate and the solvent is removed under vacuum: yield 20 g (100%).

### c) 4-(4-diphenylphosphinoyl-piperazin-1-yl)-butylamine:

20 g (40 mmol) 2-[4-(4-diphenylphosphinoyl)-butyl]-isoindolin-1,3-dione and 4 ml (80 mmol) hydrazine hydrate are heated in 400 ml ethanol for three hours under reflux. The cooling solution is concentrated under vacuum and the residue is taken up in acetic acid ethyl ester. The suspension is filtered and the residue is washed with toluene. The filtrate and washing fluid are concentrated under vacuum until dry. Subsequently, the residue is taken up in chloroform and shaken with 10% sodium sulfate. The organic phase is dried over sodium sulfate and the solvent is removed under vacuum. The accumulated crude product is further processed without further purification: yield 13.3 g (95%).

### Example B

### 4-(4-diphenylmethyl-piperazin-1-yl)-3-hydroxy-butylamine

### a) 2-(but-3-enyl)-isoindolin-1,3-dione:

50 g (370 mmol) 4-bromo-1-butene and 68.5 g (370 mmol) phthalimide potassium salt are suspended in 800 ml ethyl methyl ketone and heated under reflux for 14 hours. After cooling, the mixture is filtrated and the filtrate is concentrated under vacuum. The residue is taken up in acetic acid ethyl ester and washed with water. The organic phase is dried over sodium sulfate and the solvent is removed under vacuum: yield 50 g (67%).

### b) 2-(3,4-epoxybutyl)-isoindolin-1,3-dione:

70 g (350 mmol) 2-(but-3-enyl)-isoindolin-1,3-dione are dissolved in dichloromethane. The solution is cooled to ca. 0°C and a suspension of 120.8 g (350 mmol) 50% 3-chloro-peroxy-benzoic acid in dichloromethane is added under cooling. The mixture is left standing without further cooling at room temperature for two days. After addition of 250 ml saturated NaHCO₃ solution the organic phase is separated and washed three times each with 200 ml saturated NaHCO₃ solution and once with water. The organic phase is dried over sodium sulfate and the solvent is removed under vacuum: yield 80 g.

### c) 2-[4-(4-diphenylmethyl-piperazin-1-yl)-3-hydroxy-butyl]-isoindolin-1,3-dione:

5 g (~25 mmol) 2-(3,4-epoxybutyl)-isoindolin-1,3-dione, 7.5 g (30 mmol) benzhydrylpiperazine and 3.5 g (25 mmol) potassium carbonate are stirred in DMF for 6 hours 80°C. After cooling, the reaction mixture is filtered and concentrated under vacuum. The residue is taken up in 300 ml acetic acid ethyl ester and washed three times each with 20 ml water.
The organic phase is dried over sodium sulfate and the solvent is removed under vacuum. The residue is chromatographically purified over silica gel with CHCl₃/CH₃OH (98/2): yield 5.3 g (95%).

### d) 4-(4-diphenylmethyl-piperazin-1-yl)-3-hydroxy-butylamine:

15 g (30 mmol) 2-[4-(4-diphenylmethyl-piperazin-1-yl)-3-hydroxy-butyl]-isoindolin-1,3-dione and 3.9 ml (60 mmol) hydrazine·hydrate (80%) are heated under reflux in 100 ml ethanol for three hours. The cooled solution is concentrated under vacuum and the residue is taken up in acetic acid ethyl ester. The suspension is filtrated and the residue is distributed between acetic acid ethyl ester and 10% sodium hydroxide solution. The combined organic phases are dried over sodium sulfate and concentrated under vacuum until dry. The resin is further processed without further purification: yield 4.8 g (47%).

### Example C

### 1-(4-aminobutyryl)-4-diphenylmethyl-piperazine

### a) 1-(4-chlorobutyryl)-4-diphenylmethyl-piperazine:

25 g (99 mmol) benzhydrylpiperazine and 15.2 ml (109 mmol) TEA are present in 200 ml absolute THF and cooled to ca. 0°C under moisture exclusion. 14 g (99 mmol) 4-chlorobutyric chloride are dissolved in 40 ml absolute THF and added dropwise. The mixture is stirred an additional three hours at RT and subsequently filtered. The filtrate is concentrated under vacuum, the residue is taken up in acetic acid ethyl ester and washed with saturated NaCl solution. The organic phase is dried over sodium sulfate and concentrated under vacuum until dry. The resin is further processed without further purification: yield 35.1 g (99%).

### b) 1-(4-aminobutyryl)-4-diphenylmethyl-piperazine:

8.9 g (24.9 mmol) 1-(4-chlorobutyryl)-4-diphenylmethylpiperazine, 4.8 g (73.8 mmol) sodium azide, 1 g potassium iodide and 1 g molecular sieve 4A are stirred in 70 ml DMF for five hours at 70°C. After cooling, the reaction mixture is filtered and the filtrate is concentrated under vacuum. The accumulated crude product is dissolved in methanol and mixed with a spatula tip of palladium-carbon (10%). The mixture is stirred for two days at RT under hydrogen atmosphere. The mixture is filtered from the catalyst and the solvent is removed under vacuum. The residue is chromatographically purified over silica gel with CHCl₃/CH₃OH/TEA (90/10/5): yield 5.2 g (62%) as a colorless amorphous solid.

### Example D

### 3-(4-diphenylmethyl-piperazin-1-yl-sulfonyl)-propylamine

### a) 1-diphenylmethyl-4-(3-chloropropanesulfonyl)-piperazine:

39.2 g (155 mmol) benzhydrylpiperazine and 19.7 ml (141 mmol) TEA are present in 100 ml absolute dichloromethane and cooled to ca. 0°C under moisture exclusion. 25 g (141 mmol) 3-chloropropanesulfonyl chloride are dissolved in 70 ml absolute dichloromethane and added dropwise. The mixture is stirred for two hours under cooling and subsequently mixed with chloroform and washed with saturated NaCl solution. The organic phase is dried over sodium sulfate and concentrated under vacuum until dry. The solid is further processed without further purification: yield 59.2 g.

### b) 2-[3-(4-diphenylmethyl-piperazin-1-yl-sulfonyl)-propyl]-isoindolin-1,3-dione:

The reaction of the halogenide to phthalimide occurs analogously to Example B)a).
Batch size: 15 g (38 mmol) 1-diphenylmethyl-4-(3-chloropropane sulfonyl)-piperazine and 7.2 g (39 mmol) phthalimide·potassium salt in DMF at 80°C.
The purification occurs by chromatography on silica gel with petroleum ether/acetic acid ethyl ester (4/1). Yield 14 g (71%).

### c) 3-(4-diphenylmethyl-piperazin-1-yl-sulfonyl)-propylamine:

The reaction of the phthalimide to the amine occurs analogously to Example B)d).
Batch size: 14g (27.8 mmol) 2-[3-(4-diphenylmethyl-piperazin-1-yl-sulfonyl)-propyl]-isoindolin-1,3-dione and 2.8 ml (55.6 mmol) hydrazine·hydrate.
The resulting resin is further processed without further purification: Yield 1.5 g (15%).

### Example E

### 2-[2-(4-diphenylmethyl-piperazin-1-yl)-ethoxy]-ethylamine

### a) 2-[2-(4-diphenylmethyl-piperazin-1-yl)-ethoxy]-ethanol:

71.7 g (284 mmol) benzhydrylpiperazine, 45 g (361 mmol) 2-(2-chloroethoxy)-ethanol, 43.2 g (312 mmol) potassium carbonate and 9.4 g (57 mmol) potassium iodide are stirred in 400 ml absolute DMF for 8 hours at 75°C. After cooling, the solution is concentrated under vacuum. The residue is distributed between acetic acid ethyl ester and water. The aqueous phase is extracted twice with acetic acid ethyl ester and the combined organic phases are washed three times with saturated sodium chloride solution. The organic phase is dried over sodium sulfate and the solvent is removed under vacuum. The residue is chromatographically purified over silica gel with CHCl₃/CH₃OH (90/10): Yield 104g.

### b) 2-{2-[2-(4-diphenylmethyl-piperazin-1-yl)-ethoxy)-ethyl}-isoindolin-1,3-dione:

40 g (~118 mmol) 2-[2-(4-diphenylmethyl-piperazin-1-yl)-ethoxy]-ethanol, 31.1 g (119 mmol) triphenylphosphine and 17.3 g (118 mmol) phthalimide are suspended in 200 ml THF and 24.2 ml (119 mmol) are azodicarbonic acid diethyl ester are added dropwise under protective atmosphere and light cooling (to ca. 15°C). The mixture is stirred without further cooling for three hours and subsequently; the solvent is removed under vacuum. The residue is taken up in 1N HCl and washed twice each with 50 ml acetic acid ethyl ester respectively. The aqueous phase is neutralized with ca. 50 g sodium hydrogen carbonate and extracted four times each with 125 ml chloroform. The combined chloroform phases are dried over sodium sulfate and the solvent is removed under vacuum. The residue is chromatographically purified over silica gel with CHCl₃/CH₃OH (99/1 to 90/10): Yield 11 g (19%).

### c)2-[2-(4-diphenylmethyl-piperazin-1-yl)-ethoxy]-ethylamine:

The reaction of the phthalimide to the amine occurs analogously to Example B)d).

Batch size: 27g (55.6 mmol) 2-{2-[2-(4-diphenylmethylpiperazinyl)-ethoxy]-ethyl}-isoindol-1,3-dione and 5.4 ml (110 mmol) hydrazine·hydrate.
The purification occurs by chromatography on silica gel with CHCl₃/CH₃OH/TEA (9/1/0 to 9/1/1): Yield 12.4 g (66%).

### Example F

### 4-{4-[bis-(4-fluorophenyl)-methyl]-piperazin-1-yl}-but-2-inylamine

### a) 2-propinyl-isoindolin-1,3-dione:

32.3 g (271 mmol) 3-bromopropine are dissolved in 150 ml DMF and 50.3 g (271 mmol) phthalimide potassium salt are added under ice cooling. The suspension is warmed at 70°C for eight hours. The mixture is concentrated under a vacuum and the residue is distributed between acetic acid ethyl ester and water. The organic phase is dried over sodium sulfate and the solvent is removed under vacuum. The residue is crystallized from acetic acid ethyl ester: Yield 36.4 g (72%) colorless crystals.

### b) 2-(4-{4-[bis-(4-fluorophenyl)-methyl]-piperazin-1-yl}-but-2-inyl)-isoindolin-1,3-dione:

15 g (81 mmol) 2-propinyl-isoindolin-1,3-dione, 15 g (52 mmol) [bis-(4-fluorophenyl)-methyl]-piperazine, 2.5 g (81 mmol) paraformaldehyde and 0,2 g copper sulfate are stirred for three hours in 200 ml dioxane at 100°C. The cool solution is concentrated under vacuum and the residue is distributed between acetic acid ethyl ester and saturated NaCl solution. The organic phase is dried over sodium sulfate and concentrated under vacuum until dry. The residue is chromatographically purified over silica gel with acetic acid ethyl ester/petroleum ether (1/1): Yield 23.4 g (93%) yellow amorphous solid.

### c) 4-{4-[bis-(4-fluorophenyl)-methyl]-piperazin-1-yl}-but-2-inylamine:

The reaction of the phthalimide to the amine occurs analogously to Example 21)d): 23.3 g (48 mmol) 2-(4-{4-[bis-(4-fluorophenyl)-methyl]-piperazin-1-yl}-but-2-inyl)-isoindolin-1,3-dione and 4.7 ml (96 mmol) hydrazine•hydrate. The purification occurs by chromatography on silica gel with CHCl₃/CH₃OH (90/10 to 85/15): Yield 9.8 g (58%).

### Example G

### 4-[4-(1,2,3,4-tetrahydronaphthalin-1-yl)-piperazin-1-yl]-butylamine

### a) 2-{4-[4-(1,2,3,4-tetrahydronaphthalin-1-yl)-piperazin-1-yl]-butyl}-isoindolin-1,3-dione·dihydrochloride:

30 g (138,6 mmol) 1-(1,2,3,4-tetrahydronaphthyl)-1-piperazine, 40.3 g (140 mmol) N-(4-bromobutyl)-phthalimide and 27.6 g (200 mmol) potassium carbonate are stirred in 150 ml DMF for three hours at RT. The mixture is concentrated under a vacuum and the residue is distributed between 200 ml acetic acid ethyl ester and 150 ml water. The aqueous phase is extracted 50 ml acetic acid ethyl ester and the combined organic phases are washed four times with water. The organic phase is dried over sodium sulfate and concentrated under vacuum until dry. The residue is dissolved in 400 ml methanol and mixed with 70 ml 6.6M isopropanolic hydrochloric acid. The salt precipitated in the cold is drawn off and dried. Colorless crystals of MP.175-180°C: Yield 53.2 g (78%).

### b) 4-[4-(1,2,3,4-tetrahydronaphthalin-1-yl)-piperazin-1-yl]-butylamine:

The reaction of the phthalimide to the amine occurs analogously to Example B)d).
Batch size: 52 g (106 mmol) 2-{4-[4-(1,2,3,4-tetrahydronaphthalin-1-yl)-piperazin-1-yl]-butyl}-isoindolin-1,3-dione·dihydrochloride and 14.6 ml (300 mmol) hydrazine·hydrate in 500 ml ethanol.
The accumulated crude product is further processed without further purification: Yield 27.4 g (89%).

### Example H

### 4-[4-(5,6,7,8-tetrahydro-naphthalin-1-yl)-piperazin-1-yl]-butylamine•trihydrochloride

### a) 2-{4-[4-(5,6,7,8-tetrahydro-naphthalin-1-yl)-piperazin-1-yl]-butyl}-isoindolin-1,3-dione:

The reaction of the piperazine with the phthalimide occurs analogously to Example 26a.
Batch size: 24 g (110.9 mmol) 1-(5,6,7,8,-tetrahydronaphthalin-1-yl)-piperazine, 32.6 g (115.4 mmol) N-(4-bromobutyl)-phthalimide and 30.6 g (221.8 mmol) potassium carbonate in 240 ml DMF.
The purification occurs by chromatography over silica gel with CHCl₃/CH₃OH (100/0 to 98/2): Yield 41.6 g (89%).

### b) 4-[4-(5,6,7,8-tetrahydronaphthalin-1-yl)-piperazin-1-yl]-butylamine·Trihydrochloride:

The reaction of the phthalimide to the amine occurs analogously to Example 21d.
Batch size: 41.5 g (99.4 mmol) 2-{4-[4-(5,6,7,8-tetrahydronaphthalin-1-yl)-piperazin-1-yl]-butyl}-isoindol-in-1,3-dione and 9.5 ml (198.8 mmol) hydrazine·hydrate in 400 ml ethanol.
The purification occurs by a chromatography over silica gel with CHCl₃/CH₃OH/NH₄OH (90/10/0 to 90/9/1). After removing the solvent the residue is dissolved in 300 ml isopropanol and mixed with 47 ml 6M isopropanolic hydrochloric acid. The salts precipitating in the cold is filtered off and dried: Yield 23.5 g (59%).

### Example I

### 4-[4-(naphthalin-1-yl)-piperazin-1-yl)]-butylamine

### a) 2-[4-(naphthalin-1-yl-piperazin-1-yl)-butyl]-isoindolin-1,3-dione:

The reaction of the piperazine with the phthalimide occurs analogously to Example G)a).

Batch size: 21 g (100 mmol) 1-(1-naphthyl)-piperazine [Production according to Glennon et al., J.Med.Chem. **29**, 2375 (1986)], 28.3 g (100 mmol) N-(4-bromobutyl)-phthalimide and 20.8 g (150 mmol) potassium carbonate in 250 ml DMF.
The accumulated crude product is further processed without further purification: Yield 30 g (68%).

### b) 4-[4-(naphthalin-1-yl)-piperazin-1-yl)]-butylamine:

The reaction of phthalimide to the amine occurs analogously to Example B)d) .
Batch size: 30 g (68 mmol) 2-[4-(naphthalin-1-yl-piper-azin-1-yl)-butyl]-isoindolin-1,3-dione and 6.9g (137 mmol) hydrazine·hydrate in ethanol.

The accumulated crude product is further processed without further purification: Yield 14.6 g (75%).

### Example J

### 11-methanesulfonyloxy-6,11-dihydrodibeazo[b,e]-thiepine

### a) 6.11-dihydro-dibenzo[b,e]thiepin-11-ol:

48 g (212 mmol) 6.1-dihydro-dibenzo[b,e]thiepin-11-one are dissolved in 480 ml absolute methanol and cooled to ca. - 10°C. 19.2 g (507 mmol) sodium borohydride is added portion wise to this solution. The mixture is stirred for three hours at RT without further cooling. After the careful addition of 30 ml water, the suspension is concentrated under vacuum. The residue is taken up in 500 ml dichloromethane and washed twice each with 150 ml water. The organic phase is dried over sodium sulfate and the solvent is removed under vacuum. The residue is crystallized from 180 ml toluene. Colorless crystals of MP. 108°C: Yield 41.2 g (85%).

### b) 11-methanesulfonyloxy-6,11-dihydrodibenzo[b,e]-thiepine:

3.5 g (15.5 mmol) 6,11-dihydro-dibenzo[b,e]thiepin-11-ole and 2.4 ml (17 mmol) TEA are dissolved in 50 ml absolute dichloromethane. The mixture is cooled to ca. 5°C and a solution of 1.3 ml (16.3 mmol) methanesulfonyl chloride in 10 ml absolute dichloromethane is added dropwise. The mixture is additionally stirred for two at RT and directly employed in Example 11.

### Example K

### 2-(4-tert-butoxycarbonyl-piperazin-1-yl)-ethylamine

### a) 2-{2-[4-(tert-butoxycarbonyl)-piperazin-1-yl]-ethyl}-isoindolin-1,3-dione:

44.7 g (240 mmol) N-(tert-butoxycarbonyl)-piperazine, 60.9 g (240 mmol) N-(2-bromoethyl)-phthalimide, 49.8 g (360 mmol) potassium carbonate and 49.5 g (330 mmol) sodium iodide are heated in 1000 ml ethyl methyl ketone for five hours under reflux. After cooling the reaction mixture and concentrated under vacuum. The residue is taken up in 700 ml chloroform and extracted twice by shaking each with 50 ml water. The organic phase is dried over sodium sulfate and the solvent is distilled off. The residue is crystallized from 110 ml methanol. Colorless crystals of MP. 136 - 138°C: Yield 47.6 g (55%).

### b) 2-[4-(tert-butoxycarbonyl)-piperazin-1-yl]-ethylamine:

The reaction of the phthalimide to the amine occurs analogously to Example B)d).
Batch size: 42.2 g (120 mmol) 2-{2-[4-(tert-butoxycarbonyl)-piperazin-1-yl]-ethyl}-isoindolin-1,3-dione and 11.6 ml (240 mmol) hydrazine·hydrate in 450 ml ethanol.

The accumulated crude product is further processed without further purification: Yield 24.8 g (90%).

### Example L

### 4-(4-biphenyl-2-yl-piperazin-1-yl)-butylamine

### a) 2-[4-(4-biphenyl-2-yl-piperazin-1-yl)-butyl]-isoindolin-1,3-dione:

The reaction of piperazine with the phthalimide occurs analogously to Example G)a).

Batch size: 15.3 g (64.2 mmol) 1-(o-biphenylyl)-piperazine, 18.5 g (64.2 mmol) N-(4-bromobutyl)-phthalimide and 13.3 g (96 mmol) potassium carbonate in 270 ml ethyl methyl ketone. The purification occurs by chromatography over silica gel with CHCl₃/CH₃OH (98/2): Yield 29 g (99%).

### b) 4-(4-biphenyl-2-yl-piperazin-1-yl)-butylamine:

The reaction of the phthalimide to the amine occurs analogously to Example B)d).
Batch size: 20.8 g (47.3 mmol) 2-[4-(4-biphenyl-2-yl-piperazin-1-yl)-butyl]-isoindolin-1,3-dione and 4.6 ml (94.6 mmol) hydrazine·hydrate in 185 ml ethanol.

The accumulated crude product is further processed without further purification: Yield 11.6 g (79%).

### Example M

### 4-[4-(9H-fluoroen-9-yl)-piperazin-1-yl]-butylamine

### a) 2-{4-[4-(9H-fluoroen-9-yl)-piperazin-1-yl]-butyl}-isoindol-in-1,3-dione:

The reaction of the piperazine with the phthalimide occurs analogously to Example 26a.
Batch size: 25 g (77.3 mmol) 1-(9-fluoroenyl)-piperazine·dihydrochloride, 22.9 g (81 mmol) N-(4-bromobutyl)-phthalimide and 34 g (246 mmol) potassium carbonate in 80 ml DMF.
The purification occurs by chromatography over silica gel with CHCl₃/CH₃OH (99/1 to 90/10): Yield 30 g (86%).

### b) 4-[4-(9H-fluoroen-9-yl)-piperazin-1-yl]-butylamine:

The reaction to the phthalimide to the amine occurs analogously to Example 21d.
Batch size: 33 g (76.4 mmol) 2-{4-[4-(9H-fluoroen-9-yl)-piper-azin-1-yl]-butyl}-isoindolin-1,3-dione and 7.4 ml (153 mmol) hydrazine·hydrate in ethanol.
The accumulated crude product is further processed without further purification: Yield 11.5 g (46%).

### Example N

### 4-[4-(10,11-dihydro-5H-dibenzo[a,d]cyclohepten-5-yl)-1-piperazin-1-yl]-butylamine

### a) 1-(10,11-dihydro-5H-dibenzo[a,d]cyclohepten-5-yl)-piperazine:

48 g (210 mmol) 5-chloro-10,11-dihydro-5H-dibenzo[a,d]dicyclo-heptene are dissolved in 500 ml dioxane and after addition of 45 g (522 mmol) piperazine, the mixture is heated under reflux for 7 hours with stirring. After cooling, this is concentrated under vacuum and the residue is distributed between 500 ml chloroform and 300 ml water. The aqueous phase is additionally washed three times each with 200 ml chloroform. The combined organic phases are dried over sodium sulphate and the solvent is removed under vacuum. The residue is chromatographically purified over silica gel with CHCl₃/ CH₃OH (98/2 to 90/10) : Yield 34.5 g (58%).

### b) 2-{4-[4-(10,11-dihydro-5H-dibenzo[a,d]cyclohepten-5-yl)-piperazin-1-yl]-butyl}-isoindolin-1,3-dione:

The reaction of the piperazine with the phthalimide occurs analogously to Example G)a).
Batch size: 34.5 g (124 mmol) 1-(10,11-dihydro-5H-dibenzo-[a,d]cyclohepten-5-yl)-piperazine, 35 g (124 mmol) N-(4-bromobutyl)-phthalimide and 3.7 g (24.8 mmol) sodium iodide in 80 ml DMF.
The purification occurs by chromatography over silica gel with CHCl₃/CH₃OH (98/2): Yield 55.3 g (93%).

### c) 4-[4-(10,11-dihydro-5H-dibenzo[a,d]cyclohepten-5-yl)-1-piperazin-1-yl]-butylamine:

The reaction of the phthalimide to the amine occurs analogously to Example B)d).
Batch size: 30 g (62.5 mmol) 2-{4-[4-(10,11-dihydro-5H-dibenzo[a,d]cyclohepten-5-yl)-piperazin-1-yl]-butyl}-isoindolin-1,3-dione and 6.2 ml (125 mmol) hydrazine•hydrate in 250 ml ethanol.

The accumulated crude product is further processed without further purification: Yield 12.8 g (58%).

The active ingredients according to the invention can be processed to the desired medicaments in the form of their acid addition salts, hydrates or solvates individually or in combination with each other, optionally under addition of other active ingredients. In the case of the combination of active ingredients according to the invention with other medicinal products, these can also optionally be separately present next to each other in the medicine packaging, for example as tablets next to vials, depending on the requirements.

Further subject-matter of the invention is a pharmaceutical composition according to claim 9, comprising one or more of the compounds according to any one of claims 1 to 7 optionally together with (a) pharmaceutically acceptable carrier(s), (a) toxicologically safe adjuvant(s), and/or in combination with other active ingredients. Preferred embodiments of the pharmaceutical composition are described in claims 10 to 14.

Furthermore, the invention relates to a compound or compound mixture according to formula (I) for use in a therapeutic method in which the therapeutic use is carried out in connection with one or more medical indications with tumors or for immunosuppression, optionally in combination with further pharmaceuticals suitable for the named indications.

Further subject-matter of the invention is the use according to claim 15 of one or more compounds according to formula (I) for the manufacture of a pharmaceutical composition for the treatment of cancer and/or abnormal cell growth, and/or the prevention of proliferation and/or the formation of metastases, in the human or animal body, optionally in combination with a further cytostatic agent or immunosuppressive agent. Additional subject-matter of the invention is the use according to claim 21 of one or more compounds according to formula (I) for the manufacture of a pharmaceutical composition for the suppression of the rejection reaction after an organ transplantation in the human or animal body, optionally in combination with a further cytostatic agent or immunosuppressive agent and/or further pharmaceutical compositions suitable for these indications. Preferred embodiments of these uses are described in claims 16 to 20 and claims 22 to 26.

In addition, subject matter of the invention is the use according to claim 27 of one or more compounds of formula (I) for the manufacture of a pharmaceutical composition for the immunosuppression in the human or animal body, optionally in combination with a further cytostatic agent or immunosuppressive agent and/or further pharmaceutical compositions suitable for this indication.

The respective suitable tumor indications are illustrated in the last section of the description in the discussion of the pharmacological test results.

A method for the production of medicaments with an amount of one or more compounds according to formula (I) which are suitable for the processing of these active ingredients together with respective suitable pharmaceutically acceptable carriers and adjuvants for finished medicinal forms equally belongs to the scope of protection according to the invention.

The production methods of the respective suitable medicaments as well as a series of examples of medicinal forms are described in the following for better understanding of the invention.

### Therapeutic Administration Forms

The production of medicaments with an amount of one or more compounds according to the invention and/or their use in the application according to the invention occurs in the customary manner by means of common pharmaceutical technology methods. For this, the active ingredients as such or in the form of their salts are processed together with suitable, pharmaceutically acceptable adjuvants and carriers to medicinal forms suitable for the various indications and types of application. Thereby, the medicaments can be produced in such a manner that the respective desired release rate is obtained, for example a quick flooding and/or a sustained or depot effect.

Preparations for parenteral use, to which injections and infusions belong, are among the most important systemically employed medicaments for tumor treatment as well as for other indications.

Preferably, injections are administered for the treatment of tumors. These are prepared either in the form of vials or also as so-called ready-to-use injection preparations, for example as ready-to-use syringes or single use syringes in addition to perforation bottles for multiple withdrawals. Administration of the injection preparations can occur in the form of subcutaneous (s.c.), intramuscular (i.m.), intravenous (i.v.) or intracutaneous (i.c.) application. The respective suitable injection forms can especially be produced as solutions, crystal suspensions, nanoparticular or colloid-disperse systems, such as for example, hydrosols.

The injectable formulations can also be produced as concentrates which can be adjusted with aqueous isotonic dilution agents to the desired active ingredient dosage. Furthermore, they can also be produced as powders, such as for example lyophilisates, which are then preferably dissolved or dispersed immediately before application with suitable diluents. The infusions can also be formulated in the form of isotonic solutions, fat emulsions, liposome formulations, microemulsions and liquids based on mixed micells, for example, based on phospholipids. As with injection preparations, infusion formulations can also be prepared in the form of concentrates to dilute. The injectable formulations can also be applied in the form of continuous infusions as in stationary as well as in outpatient therapy, for example in the form of mini-pumps.

Albumin, plasma expanders, surface active compounds, organic solvents, pH influencing compounds, complex forming compounds or polymeric compounds can be added to the parenteral medicinal forms, especially as substances for influencing the adsorption of the active ingredients to protein or polymers or also with the aim of decreasing the adsorption of the active ingredient to materials such as injection instruments or packaging materials, for example plastic or glass.

The active ingredients can be bound to nanoparticles in the preparations for parenteral use, for example on finely dispersed particles based on poly(meth)acrylates, polyacetates, polyglycolates, polyamino acids or polyether urethanes. The parenteral formulations can also be constructively modified as depot preparations, for example on the multiple unit principle, where the active ingredients are incorporated in a most finely distributed and/or dispersed, suspended form or as crystal suspensions, or on the single unit principle, where the active ingredient is enclosed in a medicinal form, for example, a tablet or a seed which is subsequently implanted. Often, these implantations or depot medicaments in single unit and multiple unit medicinal forms consist of so-called biodegradable polymers, such as for example, polyether urethanes of lactic and glycolic acid, polyether urethanes, polyamino acids, poly(meth)acrylates or polysaccharides.

Sterilized water, pH value influencing substances, such as for example organic and inorganic acids or bases as well as their salts, buffer substances for setting the pH value, agents for isotonicity, such as for example sodium chloride, monosodium carbonate, glucose and fructose, tensides and/or surface active substances and emulsifiers, such as for example, partial fatty acid esters of polyoxyethylene sorbitan (Tween®) or for example fatty acid esters of polyoxethylene (Cremophor®), fatty oils such as for example peanut oil, soybean oil and castor oil, synthetic fatty acid esters, such as for example ethyl oleate, isopropyl myristate and neutral oil (Miglyol®) as well as polymer adjuvants such as for example gelatine, dextran, polyvinylpyrrolidone, organic solvent additives which increase solubility, such as for example propylene glycol, ethanol, N,N-dimethylacetamide, propylene glycol or complex forming compounds such as for example citrates and urea, preservatives, such as for example hydroxypropyl benzoate and hydroxymethyl benzoate, benzyl alcohol, anti-oxidants, such as for example sodium sulphite and stabilizers, such as for example EDTA, are suitable as adjuvants and carriers in the production of preparations for parenteral use.

In suspensions, addition of thickening agents to prevent the settling of the active ingredients from tensides and peptizers, to secure the ability of the sediment to be shaken, or complex formers, such as EDTA, ensues. This can also be achieved with the various polymeric agent complexes, for example with polyethylene glycols, polystyrol, carboxymethylcellulose, Pluronics® or polyethylene glycol sorbitan fatty acid esters. The active ingredient can also be incorporated in liquid formulations in the form of inclusion compounds, for example with cyclodextrins. As further adjuvants, dispersion agents are also suitable. For production of lyophilisates, builders are also used, such as for example mannite, dextran, saccharose, human albumin, lactose, PVP or gelatine varieties.

As long as the active ingredients are not incorporated in the liquid medicinal formulations in the form of a base, they are used in the form of their acid addition salts, hydrates or solvates in the preparations for parenteral use.

A further systemic application form of importance is peroral administration as tablets, hard or soft gelatine capsules, coated tablets, powders, pellets, microcapsules, oblong compressives, granules, chewable tablets, lozenges, gums or sachets. These solid peroral administration forms can also be prepared as sustained action and/or depot systems. Among these are medicaments with an amount of one or more micronized active ingredients, diffusions and erosion forms based on matrices, for example by using fats, wax-like and/or polymeric compounds, or so-called reservoir systems. As a retarding agent and/or agent for controlled release, film or matrix forming substances, such as for example ethylcellulose, hydroxypropylmethylcellulose, poly(meth)acrylate derivatives (for example Eudragit®), hydroxypropylmethylcellulose phthalate are suitable in organic solutions as well as in the form of aqueous dispersions. In this connection, so-called bio-adhesive preparations are also to be named in which the increased retention time in the body is achieved by intensive contact with the mucus membranes of the body. An example of a bio-adhesive polymer is the group of Carbomers®.

For sublingual application, compressives, such as for example non-disintegrating tablets in oblong form of a suitable size with a slow release of active ingredient, are especially suitable. For purposes of a targeted release of active ingredients in the various sections of the gastrointestinal tract, mixtures of pellets which release at the various places are employable, for example mixtures of gastric fluid soluble and small intestine soluble and/or gastric fluid resistant and large intestine soluble pellets. The same goal of releasing at various sections of the gastrointestinal tract can also be conceived by suitably produced laminated tablets with a core, whereby the coating of the agent is quickly released in gastric fluid and the core of the agent is slowly released in the small intestine milieu. The goal of controlled release at various sections of the gastrointestinal tract can also be attained by multilayer tablets. The pellet mixtures with differentially released agent can be filled into hard gelatine capsules.

Anti-stick and lubricant and separating agents, dispersion agents such as flame dispersed silicone dioxide, disintegrates, such as various starch types, PVC, cellulose esters as granulating or retarding agents, such as for example wax-like and/or polymeric compounds on the basis of Eudragit®, cellulose or Cremophor® are used as a further adjuvants for the production of compressives, such as for example tablets or hard and soft gelatine capsules as well as coated tablets and granulates.

Anti-oxidants, sweetening agents, such as for example saccharose, xylite or mannite, masking flavors, aromatics, preservatives, colorants, buffer substances, direct tableting agents, such as for example microcrystalline cellulose, starch and starch hydrolysates (for example Celutab®), lactose, polyethylene glycols, polyvinylpyrrolidone and dicalcium phosphate, lubricants, fillers, such as lactose or starch, binding agents in the form of lactose, starch varieties, such as for example wheat or corn and/or rice starch, cellulose derivatives, for example methylcellulose, hydroxypropylcellulose or silica, talcum powder, stearates, such as for example magnesium stearate, aluminium stearate, calcium stearate, talc, siliconized talc, stearic acid, acetyl alcohol or hydrated fats, etc. are also used.

In this connection, oral therapeutic systems constructed especially on osmotic principles, such as for example GIT (gastrointestinal therapeutic system) or OROS (oral osmotic system), are also to be mentioned.

Effervescent tablets or tabsolute both of which represent immediately drinkable instant medicinal forms which are quickly dissolved or suspended in water are among the perorally administrable compressives.

Among the perorally administrable forms are also solutions, for example drops, juices and suspensions, which can be produced according to the above given method, and can still contain preservatives for increasing stability and optionally aromatics for reasons of easier intake, and colorants for better differentiation as well as antioxidants and/or vitamins and sweeteners such as sugar or artificial sweetening agents. This is also true for inspissated juices which are formulated with water before ingestion. Ion exchange resins in combination with one or more active ingredients are also to be mentioned for the production of liquid injestable forms.

A special release form consists in the preparation of so-called floating medicinal forms, for example based on tablets or pellets which develop gas after contact with body fluids and therefore float on the surface of the gastric fluid. Furthermore, so-called electronically controlled release systems can also be formulated by which active ingredient release can be selectively adjusted to individual needs.

A further group of systemic administration and also optionally topically effective medicinal forms are represented by rectally applicable medicaments. Among these are suppositories and enema formulations. The enema formulations can be prepared based on tablets with aqueous solvents for producing this administration form. Rectal capsules can also be made available based on gelatine or other carriers.

Hardened fat, such as for example Witepsol®, Massa Estarinum®, Novata®, coconut fat, glycerol-gelatine masses, glycerol-soap-gels and polyethylene glycols are suitable as suppository bases.

For long-term application with a systematic active ingredient release up to several weeks, pressed implants are suitable which are preferably formulated on the basis of so-called biodegradable polymers.

As a further important group of systemically active medicaments, transdermal systems are also to be emphasized which distinguish themselves, as with the above-mentioned rectal forms, by circumventing the liver circulation system and/or liver metabolism. These plasters can be especially prepared as transdermal systems which are capable of releasing the active ingredient in a controlled manner over longer or shorter time periods based on different layers and/or mixtures of suitable adjuvants and carriers. Aside from suitable adjuvants and carriers such as solvents and polymeric components, for example based on Eudragit®, membrane infiltration increasing substances and/or permeation promoters, such as for example oleic acid, Azone®, adipinic acid derivatives, ethanol, urea, propylglycol are suitable in the production of transdermal systems of this type for the purpose of improved and/or accelerated penetration.

As topically, locally or regionally administration medicaments, the following are suitable as special formulations: vaginally or genitally applicable emulsions, creams, foam tablets, depot implants, ovular or transurethral administration instillation solutions. For opthalmological application, highly sterile eye ointments, solutions and/or drops or creams and emulsions are suitable.

In the same manner, corresponding otological drops, ointments or creams can be designated for application to the ear. For both of the above-mentioned applications, the administration of semi-solid formulations, such as for example gels based on Carbopols® or other polymer compounds such as for example polyvinylpyrolidone and cellulose derivatives is also possible.

For customary application to the skin or also to the mucus membrane, normal emulsions, gels, ointments, creams or mixed phase and/or amphiphilic emulsion systems (oil/water-water/oil mixed phase) as well as liposomes and transfersomes can be named. Sodium alginate as a gel builder for production of a suitable foundation or cellulose derivatives, such as for example guar or xanthene gum, inorganic gel builders, such as for example aluminium hydroxides or bentonites (so-called thixotropic gel builder), polyacrylic acid derivatives, such as for example Carbopol®, polyvinylpyrolidone, microcrystalline cellulose or carboxymethylcellulose are suitable as adjuvants and/or carriers. Furthermore, amphiphilic low and high molecular weight compounds as well as phospholipids are suitable. The gels can be present either as hydrogels based on water or as hydrophobic organogels, for example based on mixtures of low and high molecular paraffin hydrocarbons and Vaseline.

Anionic, cationic or neutral tensides can be employed as emulsifiers, for example alkalized soaps, methyl soaps, amine soaps, sulfonated compounds, cationic soaps, high fatty alcohols, partial fatty acid esters of sorbitan and polyoxyethylene sorbitan, for example lanette types, wool wax, lanolin, or other synthetic products for the production of oil/water and/or water/oil emulsions.

Hydrophilic organogels can be formulated, for example, on the basis of high molecular polyethylene glycols. These gel-like forms are washable. Vaseline, natural or synthetic waxes, fatty acids, fatty alcohols, fatty acid esters, for example as mono-, di-, or triglycerides, paraffin oil or vegetable oils, hardened castor oil or coconut oil, pig fat, synthetic fats, for example based on acrylic, caprinic, lauric and stearic acid, such as for example Softisan® or triglyceride mixtures such as Miglyol® are employed as lipids in the form of fat and/or oil and/or wax-like components for the production of ointments, creams or emulsions.

Osmotically effective acids and bases, such as for example hydrochloric acid, citric acid, sodium hydroxide solution, potassium hydroxide solution, monosodium carbonate, further buffer systems, such as for example citrate, phosphate, tries-buffer or triethanolamine are used for adjusting the pH value.

Preservatives, for example such as methyl- or propyl benzoate (parabenes) or sorbic acid can be added for increasing stability.

Pastes, powders or solutions are to be mentioned as further topically applicable forms. Pastes often contain lipophilic and hydrophilic auxiliary agents with very high amounts of fatty matter as a consistency-giving base.

Powders or topically applicable powders can contain for example starch varieties such as wheat or rice starch, flame dispersed silicon dioxide or silica, which also serve as diluents, for increasing flowability as well as lubricity as well as for preventing agglomerates.

Nose drops or nose sprays serve as nasal application forms. In this connection, nebulizers or nose creams or ointments can come to use.

Furthermore, nose spray or dry powder formulations as well as controlled dosage aerosols are also suitable for systemic administration of the active ingredients.

These pressure and/or controlled dosage aerosols and dry powder formulations can be inhaled and/or insufflated. Administration forms of this type also certainly have importance for direct, regional application in the lung or bronchi and larynx. Thereby, the dry powder compositions can be formulated for example as active ingredient-soft pellets, as an active ingredient-pellet mixture with suitable carriers, such as for example lactose and/or glucose. For inhalation or insufflation, common applicators are suitable which are suitable for the treatment of the nose, mouth and/or pharynx. The active ingredients can also be applied by means of an ultrasonic nebulizing device. As a propellant gas for aerosol spray formulations and/or controlled dosage aerosols, tetrafluoroethane or HFC 134a and /or heptafluoropropane or HFC 227 are suitable, wherein non-fluorinated hydrocarbons or other propellants which are gaseous at normal pressure and room temperature, such as for example propane, butane or dimethyl ether can be preferred. Instead of controlled dosage aerosols, propellant-free, manual pump systems can also be used.

The propellant gas aerosols can also suitably contain surface active adjuvants, such as for example isopropyl myristate, polyoxyethylene sorbitan fatty acid ester, sorbitan trioleate, lecithins or soya lecithin.

For regional application in situ, solutions for instillation, for example for transurethral administration in bladder tumors or genital tumors, or for profusion in liver tumors or other organ carcinomas are suitable.

The respective suitable medicinal forms can be produced in accordance with the prescription and procedures based on pharmaceutical-physical fundamentals as they are described for example in the following handbooks and are included in the present inventive subject-matter with respect to the production of the respective suitable medicaments:
Physical Pharmacy (A.N. Martin, J. Swarbrick, A. Cammarata), 2nd Ed., Philadelphia Pennsylvania, (1970), German version: Physikalische Pharmazie, (1987), 3rd edition, Stuttgart;
R. Voigt, M. Bornschein, Lehrbuch der pharmazeutischen Technologie, Verlag Chemie, Weinheim, (1984), 5th edition;
P.H. List, Arzneimformenlehre, Wissenschaftliche Verlagsgesellschaft mbH, Stuttgart, (1985), 4th edition;
H. Sucker, P. Fuchs, P. Speiser, Pharmazeutische Technologie, Georg Thieme Verlag, Stuttgart - New York, (1991), 2nd edition;
A.T. Florence, D. Attwood, Physicochemical Principles of Pharmacy, The Maximillan Press Ltd., Hong Kong, (1981);
L.A. Trissel, Handbook on Injectable Drugs, American Society of Hospital Pharmacists, (1994), 8th edition;
Y.W. Chien, Transdermal Controlled Systemic Medications, Marcel Dekker Inc., New York - Basel, (1987);
K.E. Avis, L. Lachmann, H.A. Liebermann, Pharmaceutical Dosage Forms: Parenteral Medications, volume 2, Marcel Dekker Inc., New York - Basel, (1986);
B.W. Müller, Controlled Drug Delivery, Paperback APV, volume 17, Wissenschaftliche Verlagsgesellschaft mbH, Stuttgart, (1987);
H. Asch, D. acetic , P.C. Schmidt, Technologie von Salben, Suspensionen and Emulsionen, Wissenschaftliche Verlagsgesellschaft mbH, Stuttgart, (1984);
H.A. Liebermann, L. Lachman, J.B. Schwartz, Pharmaceutical Desage forms: Tablets, Volume 1, Marcel Dekker Inc., New York, 2nd Edition (1989);
D. Chulin, M. Deleuil, Y. Pourcelot, Powder Technology and Pharmaceutical Processes, in J.C. Williams, T. Allen, Handbook of Powder Technology, Elsevier Amsterdam - London - New York - Tokyo, (1994);
J.T. Carstensen, Pharmaceutical Principles of Solid Dosage Forms, Technomic Publishing Co., Inc., Lancaster - Basel, (1993).

### PRODUCTION EXAMPLES

### 1. Injection therapeutics

### a) Parenteral Solution

| | |
|---|---|
| active ingredient used according to the invention | 5.000 g |
| acid sodium phosphate | 5.000 g |
| sodium tartrate | 12.000 g |
| benzyl alcohol | 7.500 g |
| water for injection purposes | to 1000.000 ml |

The solution is produced according to the customary method, sterilized and filled into 10 ml vials. One vial contains 50 mg of the compound according to the invention.

### b) Parenteral Solution

| | |
|---|---|
| active ingredient used according to the invention | 1.000 g |
| hydrochloric acid, dilute | 5.000 g |
| sodium chloride | 6.000 g |
| water for injection purposes | to 1000.000 ml |

The solution is produced according to a customary method by stirring; the medicinal form is adjusted to a suitable pH value by acid addition and subsequently filled into 100 ml vials and sterilized. A vial contains 100 mg of the compound according to the invention.

### c) Parenteral Dispersion

| | |
|---|---|
| active ingredient used according to the invention | 10.000 g |
| soya lecithin | 20.000 g |
| saturated triglycerides | 100.000 g |
| sodium hydroxide | 7.650 g |
| water for injection purposes | to 1000.000 ml |

The active ingredient(s) used according to the invention is dispersed in the saturated triglycerides. Then the soya lecithin is added under stirring, and subsequent to this, the aqueous solution of sodium hydroxide is added with subsequent homogenization. The dispersion is sterilized and filled into 10 ml vials. A vial contains 50 mg of the compound according to the invention.

### d) Biodegradable Parenteral Depot Medicinal Form

| | |
|---|---|
| active ingredient used according to the invention | 10.000 g |
| polylactic acid /polygylcolic acid polymer | 70.000 g |
| polyvinylpyrrolidone | 0.200 g |
| gelatine | 2.000 g |
| soya lecithin | 2.000 g |
| isotonic sodium chloride solution | to 1000.000 ml |

First, the active ingredient is incorporated into the biodegradable polymer comprising polylactic acid and polyglycolic acid by a suitable method (spray drying, solvent-evaporation or phase separation) and subsequently subjected to a sterilization process. The particles are introduced into a 2-chamber ready-made syringe in which the adjuvant solution, which is also produced in a sterile manner, is filled. The biodegradable microparticles are mixed with the dispersion agent shortly before application and dispersed. A ready-made syringe contains 200 mg of the active compound according to the invention.

### e) Parenteral Dispersion for Subcutaneous Instillation

| | |
|---|---|
| active ingredient used according to the invention | 25,000 g |
| soya lecithin | 25,000 g |
| arachis oil | 400,000 g |
| benzyl alcohol | 50,000 g |
| Miglyole® | to 1000,000 g |

The active ingredient is dispersed together with soya lecithin and arachis oil. The benzyl alcohol is dissolved in Miglyole® and added to the dispersion. The entire dispersion is sterilized and subsequently filled into vials with 2 ml content. A vial contains 50 mg active ingredient.

### f) Parenteral Perfusions Solution

The solution named under example b) can also be used for perfusion of liver for example.

According to need, instead of ampules with injection solution, so-called perforation bottles (vials), which can also be optionally preserved, and infusion solutions with an amount of one or more active ingredients according to the invention can also be made available in the customary manner under addition of buffer substances for adjustment of physiological pH value and/or the isotonicity and/or a best possible suitable pH value for the medicinal form (euhydria) and optional further required nutrients, vitamins, amino acids, stablizers and other necessary adjuvants, possibly in combination with further medicinal agents suitable for the mentioned indications.

### 2. Solid, Peroral Administrable Medicaments

### a) Tablets

| | |
|---|---|
| active ingredient used according to the invention | 10.000 g |
| lactose | 5.200 g |
| starch, soluble | 1.800 g |
| hydroxypropylmethylcellulose | 900 g |
| magnesium stearate | 100 g |

The above components are mixed with each other and compacted in a conventional manner, wherein a tablet weight of 180 mg is set. Each tablet contains 100 mg active ingredient. If desired, the tablets obtained in this manner are coated, provided with a film coat and/or enterically coated.

### b) Coated Tablet Core

| | |
|---|---|
| active ingredient used according to the invention | 10.000 g |
| flame dispersed silicon dioxide | 500 g |
| corn starch | 2.250 g |
| stearic acid | 350 g |
| ethanol | 3.0 1 |
| gelatine | 900 g |
| purified water | 10.0 1 |
| talcum | 300 g |
| magnesium stearate | 180 g |

From these components, a granulate is produced which is pressed to the desired coated tablet cores. Each core contains 50 mg of active ingredient. The core can be further processed in a customary manner to coated tablets. If desired, a gastric fluid resistant or retarding film coat can be applied in a known manner.

### c) Vials for Drinking

| | |
|---|---|
| active ingredient used according to the invention | 0.050 g |
| glycerine | 0.500 g |
| sorbite, 70% solution | 0.500 g |
| sodium saccharinate | 0.010 g |
| methyl-p-hydroxybenzoate | 0.040 g |
| aromatic agent q.s. | |
| sterile water q.s. | to 5 ml |

The above-mentioned components are mixed in a customary manner to a suspension and filled in a suitable drink vial having 5 ml content.

### d) Poorly Soluble Sublingual Tablets

| | |
|---|---|
| active ingredient used according to the invention | 0.030 g |
| lactose | 0.100 g |
| stearic acid | 0.004 g |
| talcum purum | 0.015 g |
| sweetener q.s. | |
| aromatic agent q.s. | |
| rice starch q.s. | to 0.500 g |

The active ingredient is compacted together with the adjuvants under high pressure to sublingual tablets, favourably in oblong form.

### e) Soft Gel Capsule

| | |
|---|---|
| active ingredient used according to the invention | 0.050 g |
| fatty acid glyceride mixture (Miglyole®) q.s. | to 0.500 g |

The active ingredient is impasted together with the fluid carrier mixture and mixed together with further adjuvants suitable for the encapsulation and filled into elastic soft gelatine capsules which are sealed.

### f) Hard Gelatine Capsules

| | |
|---|---|
| active ingredient used according to the invention | 0.150 g |
| microcrystalline cellulose | 0.100 g |
| hydroxypropylmethylcellulose | 0.030 g |
| mannite | 0.100 g |
| ethylcellulose | 0.050 g |
| triethyl citrate | 0.010 g |

The active ingredient is mixed together with the adjuvants, microcrystalline cellulose, hydroxypropylmethylcellulose and mannite, wet with granulation liquid and formed into pellets. These are subsequently coated with a solution of ethylcellulose and triethyl citrate in organic solvents in a fluidized-bed apparatus. A hard gelatine capsule contains 150 mg of active ingredient.

### 3. Topically Administrable Medicinal Forms

### a) Hydrophilic Ointment

| | |
|---|---|
| active ingredient used according to the invention | 0.500 g |
| Eucerinum® anhydricum | 60.000 g |
| microcrystalline wax | 15.000 g |
| Vaseline oil q.s. | to 100.000 g |

The above-mentioned adjuvants are melted and further processed together with the active ingredient to an ointment in a customary manner.

### b) Lipophilic Ointment

| | |
|---|---|
| active ingredient used according to the invention | 10.000 g |
| propylene glycol | 50.000 g |
| paraffin, liquid | 100.000 g |
| paraffin wax | 100.000 g |
| Vaseline | to 1000.000 ml |

The active ingredient(s) used according to the invention is dissolved in propylene glycol at ca. 60°C. At the same time, the lipophilic components are melted at 60-70°C and subsequently combined with the active ingredient solution. The ointment is emulsified at first at 60-70°C and subsequently cooled to 35-40°C under constant emulsification and then filled in 10 g tubes. A tube contains 100 mg of the compound according to the invention.

### 4. Inhalation Therapeutic Agent

Further subject-matter is a pharmaceutical formulation which is characterized in that it contains an active ingredient(s) used according to the invention as a base or a physiologically acceptable salt thereof together with carriers and/or diluents customary for this and suitable for administration by means of inhalation.

In connection with the production of the medicaments, particularly suitable physiologically acceptable salts of the active ingredients are, as already illustrated in the synthesis section, acid addition salts derived from inorganic or organic acids such as for example especially hydrochloride, hydrobromide, sulfate, phosphate, maleate, tartrate, citrate, benzoate, 4-methoxybenzoate, 2- or 4-hydroxybenzoate, 4-chlorobenzoate, p-tosylate, methane sulfonate, ascorbate, salicylate, acetate, formate, succinate, lactate, glutarate, gluconate or tricarballylate.

The administration of the active ingredient(s) used of the invention by means of inhalation occurs according to the invention in conventional ways customary for administrations of this form, for example in the form of a commercial controlled dosage aerosol or in combination with a spacer. In controlled dosage aerosols, a metering valve is delivered with whose help, a dosed amount of the composition is administered. For spraying, the present compositions can be formulated for example as aqueous solutions or suspensions and be administered by means of an atomizer. Aerosol spray formulations in which the active ingredient is either suspended with one or two stabilizers in a propellant as a carrier and/or diluent, for example tetrafluoroethane or HFC 134a and/or heptafluoropropane or HFC 227 can equally be used, whereby however, non-fluorinated hydrocarbons or other propellants which are gaseous at normal pressure and room temperature, such as propane, butane or dimethyl ether, can be preferred. Thereby, propellant-free manual pump systems or dry powder systems as described below can also be used.

Suitably, the propellant aerosols can also contain surface active adjuvants, such as for example isopropyl myristate, polyoxyethylene sorbitan fatty acid ester, sorbitan trioleate, lecithins, oleic acid.

For administration by means of inhalation and/or insufflation, the medicaments with an amount of compounds according to the invention can also be formulated in the form of dry powder compositions, for example as active ingredient-soft pellets or as an active ingredient-powder mixture with a suitable carrier, such as for example lactose and/or glucose. The powder compositions can be formulated and administered as single doses or as multiple doses.

The compounds according to the invention are preferably administered by means of a controlled dosage aerosol or in the form of a dry powder dosage formulation, wherein the latter preferably contains glucose and/or lactose as a carrier substance.

As applicators for inhalation of the pharmaceutical formulations containing one or more of the active ingredient(s) used according to the invention, all applicators are generally suitable which are suitable for controlled dosage aerosols and/or a dry powder dosage formulation, such as for example usual applicators for the nose, mouth and or pharynx, or also devices standing under propellant gas for the delivery of a spray (as controlled dosage aerosol or dry powder dosage formulation) as they are also used for inhalations in the nose, mouth and/or pharynx.

A further embodiment can also consist of an aqueous solution of the active ingredient(s) used according to the invention, which also optionally contains further active ingredients and/or additives, which are applied by means of an ultrasound atomizer.

### a) Controlled Dosage Aerosol

| | Intended dose per stroke | per aerosol % by weight |
|---|---|---|
| active ingredient used according to the invention | 0.500 mg | 0.66 |
| stabilizer | 0.075 mg | 0.10 |
| HFC 134a | 75.500 mg | 99.24 |

### b) Controlled Dosage Aerosol

| | Intended dose per stroke | per aerosol % by weight |
|---|---|---|
| active ingredient used according to the invention | 0.250 mg | 0.32 |
| Stabilizer | 0.038 mg | 0.05 |
| HFC 227 | 79.180 mg | 99.63 |

In the examples a) and b) the micronized active ingredient is, after previous dispersion in a small amount of the stabilizer, placed in a suspension vessel in which the bulk amount of propellant gas solution is found. The corresponding suspension is dispersed by means of a suitable stirring system (for example high performance mixer or ultrasound mixer) until an ultra-fine dispersion results. The suspension is then continuously held in flux in a filling apparatus suitable for cold propellants or pressure fillings.

Alternatively, the suspension can also be produced in a suitable cooled stabilizer solution in HFC 134a/227.

The examples c) to d) describe the composition and production of dosage dry powder formulations.

### c) Dosage-Dry Powder Formulation

| | mg/dose |
|---|---|
| active ingredient used according to the invention | 0.500 mg |

### d) Dosage-Dry Powder Formulation

| | mg/dose |
|---|---|
| active ingredient used according to the invention | 0.500 mg |
| lactose Ph.Eur. | to 2.5 mg or |
| | to 5.0 mg |

### e) Dosage-Dry Powder Formulation

| | mg/dose |
|---|---|
| active ingredient used according to the invention | 0.250 mg |
| lactose Ph.Eur. | to 2.5 mg or |
| | to 5.0 mg |

In example c) the active ingredient is formulated after micronization under addition of steam as pellets with an MMAD between 0,1 and 0,3 mm diameter and brought to use in a multi-dose powder applicator.

In the examples d) and e) the active ingredient is micronized, thereafter, bulk material is mixed with the lactose in the given amounts, and subsequently, filled in a multi-dose powder inhalator.

In all of the examples set forth above, the active ingredient or the medicinal agent in the form of the respective suitable pharmaceutical acceptable salt and/or acid addition salts can be present, insofar as the base is not preferred in each case.

### PHARMACEUTICAL EXPERIMENTAL SECTION

### 1. Growth Inhibition of Human Tumor Cells

The tumor growth inhibiting activity of the substances was determined on human tumor cells in standardized in vitro test systems. In the screening tests, the substances gave IC₅₀-values in a concentration range of 0.1 nM to 10 µM.

### Example 1

HepG2 cells derived from a human liver carcinoma plated at a density of 20,000 cells/ml in 12-well plastic dishes. Cultivation occurred in Richters IMEM-ZO nutrient medium with 5% foetal calf serum (FCS) in a tissue culture incubator with a gas mixture of 5% CO₂ and 95% air at a temperature of 37°C. One day after plating, the culture medium was aspirated from the cells and replaced by fresh medium which contained the respective concentrations of the test substances. For the individual concentrations and the controls without test substances, three-fold batches were done for each.

Three days after the beginning of treatment, the medium was again renewed with the test compounds. After six days of substance incubation, the test was ended and the protein amount in the individual wells was determined with the sulforhodamin-B-method (according to P. Skehan et al.: New Colorimetric Cytotoxicity Assay for Anticancer-Drug Screening. J. Natl. Cancer Inst. 82: 1107-1112, 1990). The IC₅₀-values (defined as that concentration in which the cell growth was inhibited by 50%) was taken from the dose-response curves and given as a comparative measurement for the activity of the test compounds.

The results obtained are depicted in the following Table:

| Test substance No. | IC₅₀-value [µM] |
|---|---|
| 29 | 0.6 |
| 31 | 0.8 |
| 32 | 0.5 |
| 37 | 0.5 |
| 47 | 1 |
| 158 | 0.2 |
| 208 | 0.3 |
| 227 | 0.6 |

### Example 2

A549 cells derived from a human lung carcinoma plated at a density of 20,000 cells/ml in 12-well plastic dishes. Cultivation occurred in Richters IMEM-ZO nutrient medium with 5% foetal calf serum (FCS) in a tissue culture incubator with a gas mixture of 5% CO₂ and 95% air at a temperature of 37°C. One day after plating, the culture medium was aspirated from the cells and replaced by fresh medium which contained the respective concentrations of the test substances. For the individual concentrations and the controls without test substances, three-fold batches were done for each. Three days after the beginning of treatment, the medium was again renewed with the test compounds.

After four days of substance incubation, the test was ended and the protein amount in the individual wells was determined with the sulforhodamin-B-method (according to P. Skehan et al.: New Colorimetric Cytotoxicity Assay for Anticancer-Drug Screening. J. Natl. Cancer Inst. 82: 1107-1112, 1990). The IC₅₀-values (defined as that concentration in which the cell growth was inhibited by 50%) was taken from the dose-response curves and given as a comparative measurement for the activity of the test compounds.

The following results were obtained:

| Test substance No. | IC₅₀-value [µM] |
|---|---|
| 32 | 0.5 |
| 60 | 2 |
| 87 | 0.5 |
| 94 | 2 |
| 101 | 0.4 |
| 138 | 4 |
| 215 | 5 |
| 234 | 0.1 |

### Example 3

HT-29 cells derived from a human colon carcinoma plated at a density of 20,000 cells/ml in 12-well plastic dishes. Cultivation occurred in Richters IMEM-ZO nutrient medium with 5% foetal calf serum (FCS) in a tissue culture incubator with a gas mixture of 5% CO₂ and 95% air at a temperature of 37°C. One day after plating, the culture medium was aspirated from the cells and replaced by fresh medium which contained the respective concentrations of the test substances. For the individual concentrations and the controls without test substances, three-fold batches were done for each. Three days after the beginning of treatment, the medium was again renewed with the test compounds. After four days of substance incubation, the test was ended and the protein amount in the individual wells was determined with the sulforhodamin-B-method (according to P. Skehan et al.: New Colorimetric Cytotoxicity Assay for Anticancer-Drug Screening. J. Natl. Cancer Inst. 82: 1107-1112, 1990). The IC₅₀-values (defined as that concentration in which the cell growth was inhibited by 50%) was taken from the dose-response curves and given as a comparative measurement for the activity of the test compounds.

The following results were obtained:

| Test substance No. | IC₅₀-value [µM] |
|---|---|
| 30 | 0.7 |
| 35 | 3 |
| 38 | 0.4 |
| 97 | 0.1 |
| 119 | 0.2 |

### Example 4

THP-1 cells derived from a human monocytic leukemia plated at a density of 200,000 cells/ml in 96-well plastic dishes. Cultivation occurred in RPMI 1640 nutrient medium with 10% foetal calf serum (FCS) in a tissue culture incubator with a gas mixture of 5% CO₂ and 95% air at a temperature of 37°C. For the individual concentrations and the controls without test substances as well as for the background with nutrient medium but without cells, three-fold batches were done for each. After four days of substance incubation 20 µl WST-1 reagent (Boehringer Mannheim) was respectfully pipetted in each individual well. After 30 to 60 minute incubation in the tissue culture incubator at 37°C and 5% CO₂, the light extinction was measured in an ELISA reader at 450 nm wave length. The backgrounds were each subtracted from the typical measured valves. (The IC₅₀-values (defined as that concentration in which the cell growth was inhibited by 50%) was taken from the dose-response curves and given as a comparative measurement for the activity of the test compounds.

The following results were obtained:

| Test substance No. | IC₅₀-value [µM] |
|---|---|
| 68 | 0.01 |
| 87 | 0.02 |
| 101 | 0.3 |
| 119 | 0.02 |

### 2. Indications

The compounds of formula (I) and their salts permit a therapeutic use in malignant illnesses of humans and animals through their excellent inhibition of the growth of tumor cells. The anti-neoplastic activity of the described substances can be used for prophylactic, adjunct, palliative, and curative treatment of solid tumors, leukemic illnesses and lymphomas as well as for decreasing or preventing metastasis formation in humans and animals. The therapeutic use is possible in the following illnesses for example: gynaecological tumors, ovarian carcinomas, testicle tumors, prostate carcinomas, skin cancer, kidney cancer, bladder tumors, oesophagus carcinomas, stomach cancer, rectal carcinomas, pancreas carcinomas, thyroid cancer, adrenal tumors, leukemia and lymphomas, Hodgkin's disease, tumor illnesses of the CNS, soft-tissue sarcomas, bone sarcomas, benign and malignant mesotheliomas, but especially intestine cancer, liver cancer, breast cancer, bronchial and lung carcinomas, melanomas, acute and chronic leukemias. Benign papillomatosis tumors can also be limited in their growth with the named substances. The broad effectiveness of the new compounds were tested for example in very different human tumor cells in vitro according to the methods described in point 1. Thereby, the following IC₅₀ valves were obtained for the compound Nr. 119:

| Cell line | Source | IC₅₀-values [mM] |
|---|---|---|
| HT-29 | colon carcinoma | 0.2 |
| A549 | lung carcinoma | 0.2 |
| HepG2 | hepatocelluar carcinoma | 0.08 |
| THP-1 | monocytic leukemia | 0.02 |

The novelty of the compounds can be expected to have an independent activity profile in the effectiveness against the various tumor types. Thus, tumors which are resistant to customary cytostatic agents, for example, can respond entirely to these substances. In addition, based on the independent characteristics, combinations of the new compounds with known pharmaceuticals used in chemotherapy are promising as long as their properties are complimented in a suitable manner. The integration of the new structures in a therapy scheme could be successful with one or more substances from the following classes for example: antimetabolites (for example cytarabine, 5-fluorouracil, 6-mercaptopurine, methotrexate), alkylating agents (for example busulfane, carmustine, cisplatin, carboplatin, cyclophosphamide, dacarbazine, melphalane, thiotepa), DNA-intercalating substances and topoisomerase inhibitors (for example actinomycin D, daunorubicin, doxorubicin, mitomycin C, mitoxantrone, etoposide, teniposide, topotecane, irinotecane), spindle poisons (for example vincristine, navelbine, taxol, taxoter), hormonally active agents (for example tamoxifene, flutamide, formestane, gosereline) or other cytostatic agents with complex modes of action (for example L-asparaginase, bleomycin, hydroxyurea). Resistant tumor cells can be made sensitive again for example by interaction of the new compounds with a mechanism of resistance for common cytostatic agents (for example P-glycoprotein, MRP, glutathione-S-transferase, metallothionein).

### 3. Immuno Suppressing Activity

Many anti-tumor agents have not only a cytotoxic effect on tumor cells, but also on the blood cell system. This leads to a weakening of the immune defence, which can, in turn, be specifically employed to suppress the rejection reaction after an organ transplantation for example. Also use of the main compounds, optionally in combination with other immunological diseases (for example, psoriasis or autoimmune diseases) seems likely. In order to test the possibility for a therapeutic use in illnesses of this type, the substance activity was tested on freshly isolated lymphocytes as follows:

The spleen of a Swiss mouse served as a lymphocyte source. The lymphocyte population was isolated from the spleen cell suspension over a ficoll gradient and taken up in IMEM-ZO culture medium with 0,1% dextran 70,000 and 2% foetal calf serum. The cells were plated at a density of ca. 500,000 cells/well/ml in a 12-well plate, 1 ml doubly concentrated test substance solution was pipetted per well and this was subsequently incubated in a tissue culture incubator at 37°C and 5% CO₂. After 2 days, a 1 ml-aliquot with 5 µl of the fluorescent dye solutions propidium iodide (8 mg/ml) and 3,3'-dihexyloxacarbocyanin iodide (40 µg/ml) each was added per well, and incubated for 3 minutes at room temperature. Subsequently, 10,000 cells per each sample were measured on a flow-through cytometer and the percentage amount of vital cells in the population was determined. By means of the dose-response curves, IC₅₀-values were calculated which were also employed in the following Tables for the characterization of the individual substances:

| Test Substance No. | IC₅₀ value [µM] |
|---|---|
| 87 | 0.09 |
| 101 | 0.07 |
| 119 | 0.03 |

The independent structural class of the compounds can also be expected to be successful for an efficient combination with known immunosuppressive agents such as for example cyclosporin A, tacrolimus, rapamycin, azathioprine and glucocorticoids.

## Claims

1. A pyridylalkane, pyridylalkene or pyridylalkine carboxamide according to formula (I) 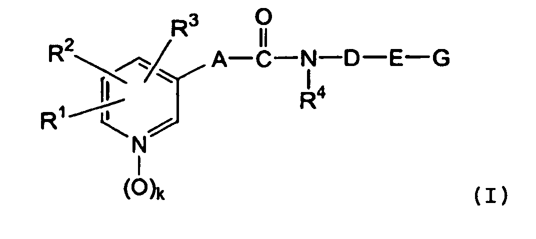 wherein:
**R**^{**1**} is selected from
hydrogen, halogen, cyano, C₁-C₆-alkyl, C₃-C₆-alkenyl, C₂-C₆-alkinyl, trifluoromethyl, C₃-C₈-cycloalkyl, C₁-C₆-hydroxyalkyl, hydroxy, C₁-C₆-alkoxy, C₃-C₆-alkenyloxy, C₃-C₆-alkinyloxy, benzyloxy, C₁-C₇-alkanoyloxy, C₂-C₇-alkoxycarbonyloxy, C₁-C₆-alkylthio, C₃-C₆-alkenylthio, C₃-C₆-alkinylthio, C₃-C₈-cycloalkyloxy, C₃-C₈-cycloalkylthio, C₂-C₇-alkoxycarbonyl, aminocarbonyl, C₂-C₇-alkylaminocarbonyl, C₃-C₁₃-dialkylaminocarbonyl, carboxy, phenyl, phenoxy, phenylthio, pyridyloxy, pyridylthio, and **NR**^{**5**}**R**^{**6**}**,** wherein
**R**^{**5**} and **R**^{**6**} are selected independently of each other from
hydrogen, C₁-C₆-alkyl, C₃-C₆-alkenyl, C₃-C₆-alkinyl, benzyl and phenyl;
**R**^{**2**} is selected from
hydrogen, halogen, cyano, C₁-C₆-alkyl, trifluoromethyl, hydroxy, C₁-C₆-alkoxy, benzyloxy and C₁-C₇-alkanoyloxy; or
**R**^{**1**} and **R**^{**2**}, if adjacent, optionally form a bridge selected from -(CH₂)₄-, -(CH=CH)₂- and -CH₂O-C**R**^{**7**}**R**^{**8**}-O-, wherein
**R**^{**7**} and **R**^{**8**} are selected independently from each other from hydrogen and C₁-C₆-alkyl;
**R**^{**3**} is selected from
hydrogen, halogen, C₁-C₆-alkyl, trifluoromethyl and C₁-C₆-hydroxyalkyl;
**R**^{**4**} is selected from
hydrogen, C₁-C₆-alkyl, C₃-C₆-alkenyl, C₃-C₆-alkinyl, C₃-C₆-cycloalkyl, hydroxy, C₁-C₆-alkoxy and benzyloxy;
**k** is 0 or 1,
**A** is selected from
C₁-C₆-alkylene, optionally substituted once to threefold by C₁-C₃-alkyl, hydroxy, C₁-C₃-alkoxy, fluorine, or phenyl,
C₂-C₆-alkylene, in which a methylene unit is isosterically replaced by O, S, **NR**^{**9**}**,** CO, SO or SO₂, wherein, with the exception of CO, the isosteric substitution is not adjacent to the amide group and **R**^{**9**} is selected from hydrogen, C₁-C₆-alkyl, C₃-C₆-alkenyl, C₃-C₆-alkinyl, C₁-C₆-acyl and C₁-C₆-alkanesulfonyl,
1,2-cyclopropylene,
C₂-C₆-alkenylene, optionally substituted once to threefold by C₁-C₃-alkyl, hydroxy, C₁-C₃-alkoxy, fluorine, cyano or phenyl,
C₄-C₆-alkadienylene, optionally substituted once or twice by C₁-C₃-alkyl, fluorine, cyano or phenyl;
1,3,5-hexatrienylene, optionally substituted by C₁-C₃-alkyl, fluorine, cyano or phenyl, and
ethinylene
**D** is selected from
C₂-C₁₀-alkylene, optionally substituted once or twice by C₁-C₆-alkyl, hydroxy, or C₁-C₆-alkoxy;
C₄-C₁₀-alkenylene, optionally substituted once or twice by C₁-C₆-alkyl, hydroxy, or C₁-C₆-alkoxy;
C₄-C₁₀-alkinylene, optionally substituted once or twice by C₁-C₆-alkyl, hydroxy, or C₁-C₆-alkoxy; and
the group consisting of C₂-C₁₀-alkylene, C₄-C₁₀-alkenylene and C₄-C₁₀-alkinylene, wherein one to three methylene units are isosterically replaced by O, S, **NR**^{**10**}, CO, SO or SO₂, wherein
**R**^{**10**} has the same meaning as **R**^{**9**}, but is selected independently thereof;
**E** represents 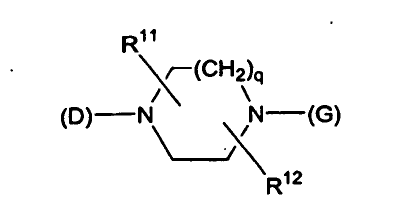 wherein
**q** is 1, 2 or 3;
**R**^{**11**} is selected from
hydrogen, C₁-C₆-alkyl, hydroxy, hydroxymethyl, carboxy, and C₂-C₇-alkoxycarbonyl, and
**R**^{**12**} is selected from
hydrogen, C₁-C₆-alkyl and an oxo group adjacent to a nitrogen atom,
or
**R**^{**11**} and **R**^{**12**} together form a C₁-C₃-alkylene bridge under formation of a bicyclic ring system;
**G** is selected from G1, G2, G3, G4 and G5, wherein
**G1** represents
―(CH₂)ᵣ―(CR¹⁴R¹⁵)ₛ―R¹³,
**r** is 0, 1, 2 or 3,
**s** is 0 or 1,
**R**^{**13**} is selected from
hydrogen, C₁-C₆-alkyl, C₃-C₆-alkenyl, C₃-C₆-alkinyl, C₃-C₈-cycloalkyl;
the group consisting of saturated and unsaturated, four to eight-membered heterocycles, which optionally contain one or two hetero-atoms selected from N, S and O;
benzyl, phenyl;
the group consisting monocyclic aromatic five to six-membered heterocycles, which optionally contain one to
three hetero-atoms selected from the N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms:are selected from N, S and O and the linkage occurs either over an aromatic ring or a hydrogenated ring and either directly or over a methylene group,
**R**^{**14**} has the same meaning as **R**^{**13**}**,** but is selected independently thereof;
**R**^{**15**} is selected from
hydrogen, hydroxy, methyl, benzyl, phenyl,
the group consisting of monocyclic aromatic five to six-membered heterocycles, which contain one to three hetero-atoms from the group N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group,
**G2** is selected from 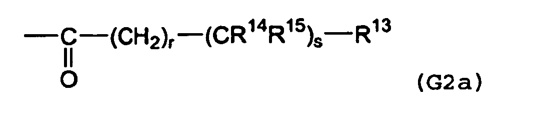 and 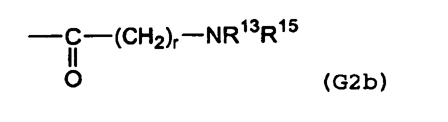 wherein **r, s** and the substituents **R**^{**13**} to **R**^{**15**} have the above meaning, or the group
―NR¹³R¹⁵
represents a nitrogen-containing heterocycle bound over the nitrogen atom which nitrogen-containing heterocycle is selected from
the group consisting of saturated and unsaturated monocyclic, four to eight-membered heterocycles, which, aside from the essential nitrogen atom, optionally contain one or two further hetero-atoms selected from N, S and O, and
the group consisting of saturated and unsaturated bi- or tricyclic, anellated or bridged heterocycles with 8 to 16 ring atoms, that aside from the essential nitrogen atom, optionally contain one or two further hetero-atoms selected from N, S and O;
**G3** represents
―SO₂―(CH₂)ᵣ―R¹³,
wherein **r** and **R**^{**13**} have the above meanings,
**G4** represents 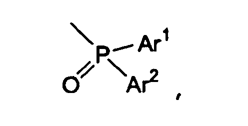 wherein
**Ar**^{**1**} and **Ar**^{**2**} are selected independently from each other from phenyl, pyridyl and naphthyl;
**G5** represents
―COR¹⁶ (G5)
wherein
**R**^{**16**} is selected from
trifluoromethyl, C₁-C₆-alkoxy, C₃-C₆-alkenyloxy, and benzyloxy,
wherein aromatic ring systems in the substituents **R**^{**1**}**, R**^{**2**}**, R**^{**4**}**, R**^{**5**}**, R**^{**6**}**, R**^{**13**}**, R**^{**14**}**, R**^{**15**}**, R**^{**16**}**, Ar**^{**1**} and **Ar**^{**2**} and/or in the ring system ―NR¹³R¹⁵ optionally are substituted independently from each other by one to three of the same or different groups selected from
halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide for two adjacent residues on the aromatic ring, and wherein
alkyl-, alkenyl- and cycloalkyl residues in the groups **G1, G2** and **G3** optionally are substituted by one or two of the same or different groups which are selected from hydroxy, carboxy, C₂-C₇-alkoxycarbonyl, benzyloxycarbonyl, amino, mono-C₁-C₆-alkylamino and di-(C₁-C₆-alkyl-amino);
their cis- and trans-isomers, E- and Z-isomers, enantiomers, diastereomers and other isomers as well as their racemic or non-racemic mixtures and the corresponding endo- and exo-isomers for the case that the ring system E is bicyclic;
their tautomeres; as well as
their acid addition salts including their hydrates and solvates,
wherein **G** does not represent
―(CH₂)ᵣ―(CR¹⁴R¹⁵)ₛ―R¹³,
in the case that simultaneously
**R**^{**13**} represents pyridyl or phenyl which is optionally substituted by halogen, alkyl, alkoxy or trifluoromethyl,
**R**^{**14**} represents hydrogen or phenyl, which is optionally substituted by halogen, alkyl, alkoxy or trifluoromethyl,
**R**^{**15**} represents hydrogen,
**A** represents alkylene, optionally substituted ethenylene or butadienylene,
**D** represents alkylene or alkenylene,
**E** represents piperazine or homopiperazine and
**s** is 1;
and wherein **G** does not represent
a member of the group consisting of phenyl, N-containing heteroaryl and 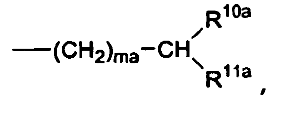 wherein: R^{10a} is hydrogen or phenyl, R^{11a} is a phenyl or a pyridyl, and ma is an integer of 0 to 2, provided that the phenyl group may optionally be substituted by one or two members selected from the group consisting of a halogen, a C₁-C₆ alkyl, trifluoromethyl and a C₁-C₆ alkoxy;
in the case that simultaneously
**R**^{**1**} is hydrogen, a halogen, a C₁-C₆-alkyl, a C₁-C₆-alkoxy, a C₁-C₆-alkylthio, a C₃-C₈-cycloalkyloxy, a C₃-C₈-cycloalkylthio, a C₂-C₇-alkoxycarbonyl, carboxy, a phenyl, a phenoxy, a phenylthio, 3-pyridyloxy or 3-pyridylthio;
**R**^{**2**} is hydrogen, hydroxy, a C₁-C₇-alkanoyloxy or a C₂-C₇-alkoxycarbonyloxy, or when R¹ and R₂ are adjacent to each other, they may combine to form tetramethylene or -CH₂OCR^{8a}R^{9a}O-, wherein R^{8a} and R^{9a} are the same or different and are each a C₁-C₆-alkyl;
**R**^{**3**} is hydrogen, a C₁-C₆-alkyl or a hydroxy-C₁-C₆-alkyl;
**A** is a C₁-C₆-alkylene or -(CR^{6a}=CR^{7a})ᵣₐ-, wherein R^{6a} is hydrogen, a C₁-C₃-alkyl or a phenyl, R^{7a} is hydrogen, a C₁-C₃-alkyl, cyano or a phenyl, and ra is 1 or 2;
**R**^{**4**} is hydrogen;
**D** represents a C₂-C₁₀-alkylene or a C₄-C₁₀-alkylene interrupted by one double bond; and
**E** is selected from piperazine, piperazine, which is substituted by C₁-C₆-alkyl, homopiperazine, and homopiperazine, which is substituted by C₁-C₆-alkyl.

2. A compound according to formula (I) 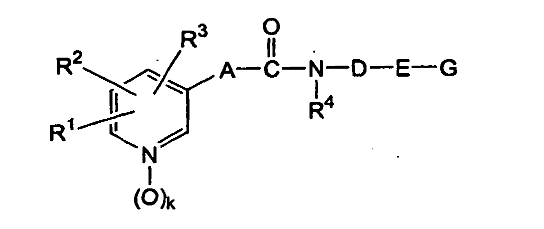 wherein
**R**^{**1**} is selected from
hydrogen, halogen, cyano, C₁-C₆-alkyl, trifluoromethyl, C₃-C₈-cycloalkyl, C₁-C₆-hydroxyalkyl, hydroxy, C₁-C₄-alkoxy, benzyloxy, C₁-C₄-alkylthio, C₁-C₅-alkanoyloxy, C₂-C₅-alkoxycarbonyl, aminocarbonyl, C₂-C₅-alkylaminocarbonyl, C₃-C₉-dialkylaminocarbonyl, carboxy, phenyl, phenoxy, phenylthio, pyridyloxy, and N**R**^{**5**}**R**^{**6**}, wherein
**R**^{**5**} and **R**^{**6**} are selected independently from each other from hydrogen and C₁-C₆-alkyl;
**R**^{**2**} is selected from
hydrogen, halogen, cyano, C₁-C₆-alkyl, trifluoromethyl, hydroxy, and C₁-C₄-alkoxy;
**R**^{**3**} is selected from
hydrogen, halogen and C₁-C₆-alkyl;
**R**^{**4**} is selected from
hydrogen, C₁-C₆-alkyl, C₃-C₆-alkenyl, C₃-C₆-cycloalkyl, hydroxy, C₁-C₆-alkoxy and benzyloxy;
**k** is 0 or 1,
**A** is selected from
C₁-C₆-alkylene, optionally substituted once to three-fold by C₁-C₃-alkyl, hydroxy, fluorine, or phenyl,
C₂-C₆-alkylene, in which a methylene unit is isosterically replaced by O, S, **NR**^{**9**}, CO, SO or SO₂, wherein, with the exception of CO, the isosteric substitution is not adjacent to the amide group and the residue **R**^{**9**} is selected from hydrogen, C₁-C₆-alkyl, C₁-C₆-acyl and methane sulfonyl;
1,2-cyclopropylene,
C₂-C₆-alkenylene, optionally substituted once to threefold by C₁-C₃-alkyl, hydroxy, fluorine, cyano or phenyl,
C₄-C₆-alkadienylene, optionally substituted once or twice by C₁-C₃-alkyl, fluorine, cyano or phenyl;
1,3,5-hexatrienylene, optionally substituted by C₁-C₃-alkyl, fluorine, or cyano, and
ethinylene
**D** is selected from
C₂-C₁₀-alkylene, optionally substituted once or twice by C₁-C₃-alkyl or hydroxy;
C₄-C₁₀-alkenylene, optionally substituted once or twice by C₁-C₃-alkyl or hydroxy;
C₄-C₁₀-alkinylene, optionally substituted once or twice by C₁-C₃-alkyl or hydroxy; and
the group consisting of C₂-C₁₀-alkylene, C₄-C₁₀-alkenylene and C₄-C₁₀-alkinylene, wherein one to three methylene units are isosterically replaced by O, S, **NR**^{**10**}**,** CO, SO or SO₂, wherein
**R**^{**10**} has the same meaning as **R**^{**9**}**,** but is selected independently thereof;
**E** represents 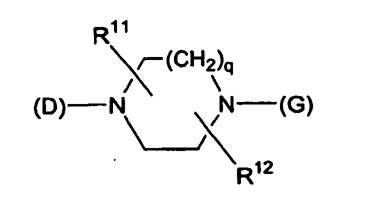 wherein
**q** is 1, 2 or 3;
**R**^{**11**} is selected from
hydrogen, C₁-C₃-alkyl, hydroxy, hydroxymethyl, carboxy, and C₂-C₇-alkoxycarbonyl, and
**R**^{**12**} is selected from hydrogen and an oxo group adjacent to a nitrogen atom or
**R**^{**11**} and **R**^{**12**}, together, form a C₁-C₃-alkylene bridge under formation of a bicyclic ring system;
**G** is selected from G1, G2, G3, G4 and G5, wherein
**G1** represents
―(CH₂)ᵣ―(CR¹⁴R¹⁵)ₛ―R¹³,
**r** is 0, 1 or 2,
**s** is 0 or 1,
**R**^{**13**} is selected from
hydrogen, C₁-C₆-alkyl, C₃-C₆-alkenyl, C₃-C₆-alkinyl, C₃-C₈-cycloalkyl; benzyl, phenyl;
the group consisting of monocyclic aromatic five to six-membered heterocycles, which contain one to three hetero-atoms selected from N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group,
**R**^{**14**} has the same meaning as **R**^{**13**}**,** but is selected independently thereof;
**R**^{**15**} is selected from
hydrogen, hydroxy, methyl, benzyl, phenyl;
the group consisting of monocyclic aromatic five to six-membered heterocycles, which contain one to three hetero-atoms selected from N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group, and
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group;
**G2** is selected from 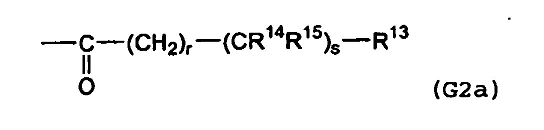 and 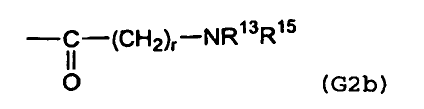 wherein **r, s** and the substituents **R**^{**13**} to **R**^{**15**} have the above meaning, or the group
―NR¹³R¹⁵
is a nitrogen-containing heterocycle bound over the nitrogen atom which nitrogen-containing heterocycle is selected from
the group consisting of saturated and unsaturated monocyclic, four to eight-membered heterocycles, which, aside from the essential nitrogen atom, optionally contain one or two further hetero-atoms selected from N, S and O, and
the group consisting of saturated and unsaturated bi- or tricyclic, anellated or bridged heterocycles with 8 to 16 ring atoms, that aside from the essential nitrogen atom, optionally contain one or two further hetero-atoms selected from N, S and O;
**G3** represents
―SO₂―(CH₂)ᵣ―R¹³,
wherein **r** and **R**^{**13**} have the above meaning,
**G4** represents 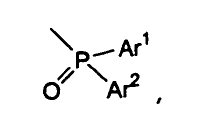 wherein
**Ar**^{**1**} and **Ar**^{**2**} are selected independently from each other from phenyl, pyridyl and naphthyl;
**G5** represents
―COR¹⁶,
wherein
**R**^{**16**} is selected from
trifluoromethyl, C₁-C₆-alkoxy, C₃-C₆-alkenyloxy, and benzyloxy,
wherein aromatic ring systems in the substituents **R**^{**1**}**, R**^{**2**}**, R**^{**4**}**, R**^{**5**}**, R**^{**6**}**, R**^{**13**}**, R**^{**14**}**, R**^{**15**}**, R**^{**16**}**, Ar**^{**1**} and **Ar**^{**2**} and/or in the ring system ―NR¹³R¹⁵ optionally are substituted independently from each other by one to three of the same or different groups selected from
halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide in the case of two adjacent residues on the aromatic ring,
wherein alkyl, alkenyl and cycloalkyl residues in the groups **G**^{**1**}**, G**^{**2**} and **G**^{**3**} optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl-amino),
wherein **G** does not represent
a member of the group consisting of phenyl, N-containing heteroaryl and 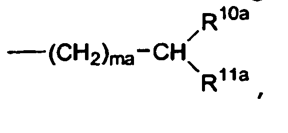 wherein: R^{10a} is hydrogen or phenyl, R^{11a} is a phenyl or a pyridyl, and ma is an integer of 0 to 2, provided that the phenyl group may optionally be substituted by one or two members selected from the group consisting of a halogen, a C₁-C₆ alkyl, trifluoromethyl and a C₁-C₆ alkoxy,
in the case that simultaneously
**R**^{**1**} is hydrogen, a halogen, a C₁-C₆-alkyl, a C₁-C₆-alkoxy, a C₁-C₆-alkylthio, a C₃-C₈-cycloalkyloxy, a C₃-C₈-cycloalkylthio, a C₂-C₇-alkoxycarbonyl, carboxy, a phenyl, a phenoxy, a phenylthio, 3-pyridyloxy or 3-pyridylthio;
**R**^{**2**} is hydrogen, hydroxy, a C₁-C₇-alkanoyloxy or a C₂-C₇-alkoxycarbonyloxy, or when R¹ and R₂ are adjacent to each other, they may combine to form tetramethylene or -CH₂OCR^{8a}R^{9a}O-, wherein R^{8a} and R^{9a} are the same or different and are each a C₁-C₆-alkyl;
**R**^{**3**} is hydrogen, a C₁-C₆-alkyl or a hydroxy-C₁-C₆-alkyl;
**A** is a C₁-C₆-alkylene or -(CR^{6a}=CR^{7a})ᵣₐ-, wherein R^{6a} is hydrogen, a C₁-C₃-alkyl or a phenyl, R^{7a} is hydrogen, a C₁-C₃-alkyl, cyano or a phenyl, and ra is 1 or 2;
**R**^{**4**} is hydrogen;
**D** represents a C₂-C₁₀-alkylene or a C₄-C₁₀-alkylene interrupted by one double bond; and
**E** is selected from piperazine, piperazine, which is substituted by C₁-C₆-alkyl, homopiperazine, and homopiperazine, which is substituted by C₁-C₆-alkyl.

3. The compound according to claim 2, wherein
**R**^{**1**} is selected from
hydrogen, halogen, cyano, methyl, ethyl, trifluoromethyl, hydroxy, C₁-C₄-alkoxy, benzyloxy, C₁-C₅-alkanoyloxy, methylthio, ethylthio, methoxycarbonyl, tert-butoxycarbonyl aminocarbonyl, carboxy, phenoxy, and phenylthio,
**R**^{**2**} is selected from
hydrogen, halogen, trifluoromethyl, and hydroxy,
**R**^{**3**} is selected from
hydrogen, and halogen,
**R**^{**4**} is selected from
hydrogen, C₁-C₃-alkyl, allyl, hydroxy, and C₁-C₃-alkoxy;
**k** is 0 or 1,
**A** is selected from
C₁-C₆-alkylene, optionally substituted once or twice by C₁-C₃-alkyl, hydroxy or fluorine;
C₂-C₆-alkylene, in which a methylene unit is isosterically replaced by O, S, **NR**^{**9**}, CO, SO or SO₂, wherein, with the exception of CO, the isosteric substitution is not adjacent to the amide group,
C₂-C₆-alkenylene, optionally substituted once or twice by C₁-C₃ alkyl, hydroxy and/or fluorine;
C₄-C₆-alkadienylene, optionally substituted by C₁-C₃-alkyl or one or two fluorine atoms; and
1,3,5-hexatrienylene, optionally substituted by fluorine;
**D** is selected from
C₂-C₈-alkylene, optionally substituted once or twice by methyl or hydroxy;
C₄-C₈-alkenylene, optionally substituted once or twice by methyl or hydroxy;
C₄-C₈-alkinylene, optionally substituted once or twice by methyl or hydroxy; and
the group consisting of C₂-C₈-alkylene, C₄-C₈-alkenylene and C₄-C₈-alkinylene, wherein one to three methylene units are each isosterically replaced by O, S, NH, N(CH₃) N(COCH₃), N(SO₂CH₃), CO, SO or SO₂,
**E** represents 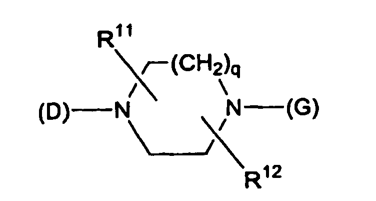 wherein
**q** is 1 or 2;
**R**^{**11**} is selected from
hydrogen, C₁-C₃-alkyl, hydroxymethyl, and carboxy, and
**R**^{**12**} is selected from
hydrogen and an oxo group adjacent to a nitrogen atom
**G** is selected from G1, G2, G3, G4 and G5, wherein
**G1** represents
―(CH₂)ᵣ―(CR¹⁴R¹⁵)ₛ―R¹³
**r** is 0, 1 or 2,
**s** is 0 or 1;
**R**^{**13**} is selected from
hydrogen, C₁-C₆-alkyl, C₃-C₈-cycloalkyl, benzyl, phenyl,
the group consisting of benzocyclobutyl, indanyl, indenyl, oxoindanyl, naphthyl, dihydronaphthyl, tetrahydronaphthyl, oxotetrahydronaphthyl, biphenylenyl, fluorenyl, oxofluorenyl, anthryl, dihydroanthryl, oxodihydroanthryl, dioxodihydroanthryl, phenanthryl, dihydrophenanthryl, oxodihydrophenanthryl, dibenzocycloheptenyl, oxodibenzocycloheptenyl, dihydrodibenzocycloheptenyl, oxodihydrodibenzocycloheptenyl, dihydrodibenzocyclooctenyl, tetrahydrodibenzocyclooctenyl and oxotetrahydrodibenzocyclooctenyl, bound directly or over a methylene group; and
the group consisting of furyl, thienyl, pyrrolyl, oxazolyl, isoxazolyl, thiazolyl, iso-thiazolyl, pyrazolyl, imidazolyl, oxadiazolyl, thiadiazolyl, triazolyl, pyridyl, pyrazinyl, pyridazinyl, pyrimidinyl, triazinyl, imidazothiazolyl, benzofuryl, dihydrobenzofuryl, benzothienyl, dihydrobenzothienyl, indolyl, indolinyl, isoindolinyl, oxoindolinyl, dioxoindolinyl, benzoxazolyl, oxobenzooxazolinyl, benzoisooxazolyl, oxobenzoisooxazolinyl, benzothiazolyl, oxobenzthiazolinyl, benzoisothiazolyl, oxobenzoisothiazolinyl, benzoimidazolyl, oxobenzoimidazolinyl, indazolyl, oxoindazolinyl, benzofurazanyl, benzothiadiazolyl, benzotriazolyl, oxazolopyridyl, oxodihydrooxazolopyridyl, thiazolopyridyl, oxodihydrothiazolopyridyl, isothiazolopyridyl, imidazopyridyl, oxodihydroimidazopyridyl, pyrazolopyridyl, oxodihydropyrazolopyridyl, thienopyrimidinyl, chromanyl, chromanonyl, benzopyranyl, chromonyl, quinoloyl, isoquinoloyl, dihydroquinolyl, oxodihydroquinolinyl, tetrahydroquinolyl, oxotetrahydroquinolinyl, benzodioxanyl, quinoxalinyl, quinazolinyl, naphthyridinyl, carbazolyl, tetrahydrocarbazolyl, oxotetrahydrocarbazolyl, pyridoindolyl, acridinyl, oxodihydroacridinyl, phenanthridinyl, dihydrophenanthridinyl, oxodihydrophenanthridinyl, dibenzoisoquinolinyl, dihydrodibenzoisoquinolinyl, oxodihydrodibenzoisoquinolinyl, phenothiazinyl, dihydrodibenzooxepinyl, oxodihydrodibenzooxepinyl, benzocycloheptathienyl, oxobenzocycloheptathienyl, dihydrothienobenzothiepinyl, oxodihydrothienobenzothiepinyl, dihydrodibenzothiepinyl, oxodihydrodibenzothiepinyl, octahydrodibenzothiepinyl, dibenzoazepinyl, dihydrodibenzoazepinyl, oxodihydrodibenzoazepinyl, octahydrodibenzoazepinyl, benzocycloheptapyridyl, oxobenzocycloheptapyridyl, pyridobenzoazepinyl, dihydropyridobenzoazepinyl, oxodihydropyridobenzoazepinyl, dihydropyridobenzodiazepinyl, dihydrodibenzooxazepinyl, dihydropyridobenzooxepinyl, dihydropyridobenzooxazepinyl, oxodihydropyridobenzooxazepinyl, dihydrodibenzothiazepinyl, oxodihydrodibenzothiazepinyl, dihydropyridobenzothiazepinyl and oxodihydropyridobenzothiazepinyl, bound directly or over a methylene group;
**R**^{**14**} has the same meaning as **R**^{**13**} but is selected independently thereof;
**R**^{**15**} is selected from
hydroxy, methyl, benzyl, phenyl, indanyl, indenyl, naphthyl, dihydronaphthyl, tetrahydronaphthyl, furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, triazinyl, benzofuryl, benzothienyl, indolyl, indolinyl, benzooxazolyl, benzothiazolyl, benzoimidazolyl, chromanyl, quinolyl and tetrahydroquinolyl, bound directly or over a methylene group;
**G2** is selected from 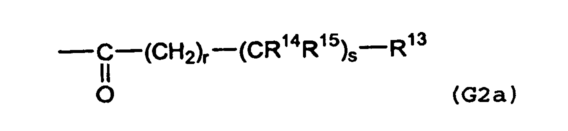 and 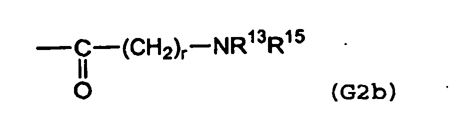 wherein **r, s** and the substituents **R**^{**13**} to **R**^{**15**} have the above meaning, or the group
―NR¹³R¹⁵
represents azetidine, pyrrolidine, piperidine, (1H)-tetrahydropyridine, hexahydroazepine, (1H)-tetrahydroazepine, octahydroazocine, pyrazolidine, piperazine, hexahydrodiazepine, morpholine, hexahydrooxazepine, thiomorpholine, thiomorpholin-1,1-dioxide, of 5-aza-bicyclo-[2.1.1]hexane, 2-aza-bicyclo[2.2.1]heptane, 7-aza-bicyclo-[2.2.1]heptane, 2,5-diaza-bicyclo[2.2.1]heptane, 2-aza-bicyclo[2.2.2]octane, 8-aza-bicyclo[3.2.1]octane, 2,5-diaza-bicyclo[2.2.2]octane, 9-aza-bicyclo[3.3.1]nonane, indoline, isoindoline, (1H)-dihydroquinoline, (1H)-tetrahydroquinolin, (2H)-tetrahydroisoquinoline, (1H)-tetrahydroquinoxaline, (4H)-dihydrobenzooxazine, (4H)-dihydrobenzothiazine, (1H)-tetrahydrobenzo[b]azepine, (1H)-tetrahydrobenzo[c]azepine, (1H)-tetrahydrobenzo[d]azepine, (5H)-tetrahydrobenzo[b]ox-azepine, (5H)-tetrahydrobenzo[b]thiazepine, 1,2,3,4-tetra-hydro-9H-pyrido[3,4-b]indole, (10H)-dihydroacridine, (10H)-dihydrophenanthridine, 1,2,3,4-tetrahydroacridanone, (10H)-phenoxazine, (10H)-phenothiazine, (5H)-dibenzoazepine, (5H)-dihydrodibenzoazepine, (5H)-octahydrodibenzoazepine, dihydrobenzo[d,e]isoquinoline, (5H)-dihydrodibenzodiazepine, (5H)-benzo[b]pyrido-[f]azepine, (5H)-Dihydrobenzo[b]pyri-do[f]azepine, (11H)-Dihydrodibenzo[b,e]oxazepine, (11H)-dihydrodibenzo[b,e]thiazepine, (10H)-dihydrodibenzo[b,f]-oxazepine, (10H)-dihydrodibenzo[b,f]thiazepine, (5H)-tetra-hydrodibenzoazocine, (11H)-dihydrobenzo[e]pyrido[b]-1,4-diazepin-6-one or (11H)-Dihydrobenzo[b]pyrido[e]-1,4-diazepin-5-one,
**G3** represents
―SO₂―(CH₂)ᵣ―R¹³,
wherein **r** and **R**^{**13**} have the above meaning,
**G4** represents 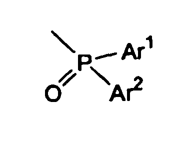 wherein
**Ar**^{**1**} and **Ar**^{**2**} are selected independently from each other from phenyl, pyridyl and naphthyl;
**G5** represents
―COR¹⁶,
wherein
**R**^{**16**} is selected from
trifluoromethyl, C₁-C₆-alkoxy, C₃-C₆-alkenyloxy, and benzyloxy,
wherein aromatic ring systems in the substituents optionally are substituted independently from each other by one to three of the same or different groups selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide in the case of two adjacent residues on the aromatic ring, and
wherein alkyl-, alkenyl- and cycloalkyl residues in the groups **G1, G2** and **G3** optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl-amino).

4. The compound according to claim 2 or 3, wherein
**R**^{**1**} is selected from
hydrogen, fluorine, chlorine, bromine, methyl, ethyl, trifluoromethyl, hydroxy, C₁-C₄-alkoxy, methylthio, ethylthio, carboxy and phenoxy;
**R**^{**2**} is selected from hydrogen, chlorine and methyl;
**R**^{**3**} is hydrogen;
**R**^{**4**} is selected from
hydrogen, C₁-C₃-alkyl and hydroxy,
**k** is 0
**A** is selected from
C₂-C₆-alkylene, which is optionally substituted once or twice by hydroxy or fluorine;
C₂-C₆-alkylene, wherein a methylene unit is isosterically replaced by O, S or CO, wherein, with the exception of CO, the isosteric substitution is not adjacent to the amide group;
C₂-C₆-alkenylene which is optionally substituted by C₁-C₃-alkyl and/or fluorine; and
C₄-C₆-alkadienylene;
**D** is selected from
C₂-C₈-alkylene which is optionally substituted by methyl or hydroxy;
C₄-C₈-alkenylene, which is optionally substituted by hydroxy;
C₄-C₈-alkinylene, which is optionally substituted by hydroxy; and
the group consisting of C₂-C₈-alkylene, C₄-C₈-alkenylene, and C₄-C₈-alkinylene wherein a methylene unit is isosterically replaced by O, NH, N(CH₃), CO or SO₂ or an ethylene group is isosterically replaced by a group NH-CO and/or CO-NH or a propylene group is isosterically replaced by a group NH-CO-O and/or O-CO-NH;
**E** is selected from piperazine and hexahydro-1,4-diazepine, wherein the ring optionally is substituted by one or two methylene groups and/or by an oxo group adjacent to a nitrogen atom;
G is selected from hydrogen, C₃-C₈-cycloalkyl, methoxycarbonyl, tert-butoxycarbonyl, benzyloxycarbonyl, trifluoroacetyl, diphenylphosphinoyl,
―(CH₂)ᵣ―(CR¹⁴R¹⁵)ₛ―R¹³, 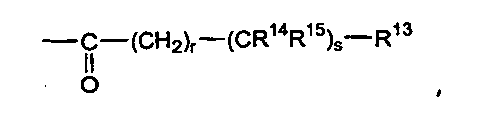 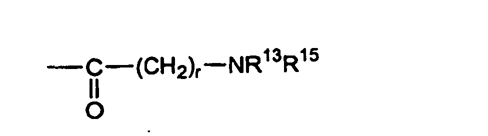
and
―SO₂-(CH₂)ᵣ―R¹³
wherein
**r** is 0 or 1
**s** is 0 or 1,
**R**^{**13**} is selected from hydrogen, methyl, benzyl, phenyl,
the group consisting of indanyl, indenyl, oxoindanyl, naphthyl, tetrahydronaphthyl, fluorenyl, oxofluorenyl, anthryl, dihydroanthryl, oxodihydroanthryl, dioxodihydroanthryl, phenanthryl, dihydrophenanthryl, oxydihydrophenanthryl, dibenzocycloheptenyl, dihydrodibenzocycloheptenyl, and oxodihydrodibenzocycloheptyl, bound directly or over a methylene group, and
the group consisting of furyl, thienyl, oxazolyl, isooxazolyl, thiazolyl, imidazolyl, oxadiazolyl, pyridyl, pyrazinyl, pyrimidinyl, imidazothiazolyl, benzofuryl, benzothienyl, indolyl, indolinyl, oxoindolinyl, dioxoindolinyl, benzoxazolyl, oxobenzooxazolinyl, benzothiazolyl, oxobenzthiazolinyl, benzimidazolyl, oxobenzimidazolinyl, indazolyl, benzofurazanyl, benzotriazolyl, oxazolopyridyl, oxodihydrooxazolopyridyl, thiazolopyridyl, oxodihydrothiazolopyridyl, imidazopyridyl, oxodihydroimidazopyridyl, chromanyl, chromanonyl, benzopyranyl, chromonyl, quinolyl, isoquinolyl, oxodihydroquinolinyl, tetrahydroquinolyl, oxotetrahydroquinolinyl, benzodioxanyl, quinazolinyl, carbazolyl, acridinyl, dihydroacridinyl, oxodihydroacridinyl, dihydrophenanthridinyl dihydroberizoisoquinolinyl, phenothiazinyl, dihydrodibenzooxepinyl, benzocycloheptathienyl, dihydrothienobenzothiepinyl, dihydrodibenzothiepinyl, oxodihydrodibenzothiepinyl, dihydrodibenzoazepinyl, oxodihydrodibenzoazepinyl, octahydrodibenzoazepinyl, benzocycloheptapyridyl, dihydropyridobenzodiazepinyl, and dihydrodibenzothiazepinyl, bound directly or over a methylene group,
**R**^{**14**} is selected from hydrogen, methyl, benzyl, and phenyl,
**R**^{**15**} is selected from
hydrogen, hydroxy, methyl, benzyl, phenyl; and
the group consisting of naphthyl, tetrahydronaphthyl, furyl, thienyl, oxazolyl, thiazolyl, imidazolyl, pyridyl, benzofuryl, benzothienyl, indolyl, indolinyl, benzoxazolyl, benzothiazolyl, benzimidazolyl, chromanyl, quinolyl and tetrahydroquinolyl, bound directly or over a methylene group;
wherein the group
―NR¹³R¹⁵
represents a nitrogen-containing heterocycle bound over the nitrogen atom which nitrogen-containing heterocycle is selected from
pyrrolidine, piperidine, hexahydroazepine, piperazine, hexahydrodiazepine, morpholine, hexahydroxazepine, thiomorpholine, 7-aza-bicyclo[2,2.1]heptane, 2,5-diazabicyclo[2.2.1]heptane, indoline, isoindoline, (1H)-dihydroquinoline, (1H)-tetrahydroquinoline, (2H)-tetrahydroisoquinoline, (4H)-dihydrobenzoxazine, (4H-dihydrobenzothiazine, (1H)-tetrahydrobenzo[b]azepine, (1H)-tetrahydrobenzo[d]azepine, (5H)-tetrahydrobenzo[b]oxazepine, (5H)-tetrahydrobenzo[b]thiazepine, (10H)-dihydroacridine, 1,2,3,4-tetrahydroacridanone, (10H)-dihydrophenanthridine, (1H)-dihydrobenzo[d,e]isoquinoline, (10H)-phenothiazine, (5H)-dibenzo[b,f]azepine, (5H)-dihydrodibenzo[b,f]azepine, (5H)-dihydrodibenzo[c,e]azepine, (5H)-dihydrodibenzodiazepine, (11H)-dihydrodibenzo[b,e]oxazepine (11H)-dihydrodibenzo[b,e]thiazepine, (5H)-dihydrobenzo[b]pyrido[3,2-f]azepine and (11H)-6-oxodihydrobenzo[e]pyrido[3,2-b][1,4]diazepine,
wherein aromatic ring systems in the substituents optionally are substituted, independently of each other, by one to three of the same or different groups selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carbocyalkenyl, C₂-C₇-alkoxycarbonyl, benzyloxycarbonyl, nitro, amino, mono-C₁-C₆-alkylamino, di-(C₁-C₆-alkyl)-amino, and in the case of two adjacent residues on the aromatic ring, methylenedioxy, and
wherein alkyl, alkenyl and cycloalkyl residues in the group G optionally are substituted by one or two of the same or different groups which are selected from hydroxy, carboxy, C₂-C₇-alkoxycarbonyl, benzyloxycarbonyl, amino, mono-C₁-C₆-alkylamino and di-(C₁-C₆-alkyl) amino.

5. The compound according to any one ofclaims 2 to 4, wherein
**R**^{**1**} is selected from
hydrogen, fluorine, methyl, trifluoromethyl and ethylthio;
**R**^{**2**}, **R**^{**3**} and **R**^{**4**} are each hydrogen;
**k** is 0,
**A** is selected from
the group consisting of ethylene, propylene and butylene, which are each optionally substituted by hydroxy or one or two fluorine atoms;
OCH₂, SCH_{2;}
ethenylene, and 1,3-butadienylene;
**D** is selected from
C₂-C₆-alkylene which is optionally substituted by hydroxy;
C₄-C₆ alkenylene;
C₄-C₆ alkinylene; and
the group consisting of C₂-C₆ alkylene, C₄-C₆ alkenylene and C₄-C₆ alkinylene, wherein one or two methylene units are isosterically replaced by O, NH, CO or SO₂;
**E** is piperazine or hexahydro-1,4-diazepine;
**G** is selected from
phenyl, benzyl, phenethyl, diphenylmethyl, naphthyl, tetrahydronaphtyl, naphthylmethyl, fluorenyl fluorenylmethyl, anthrylmethyl, dihydrodibenzocycloheptenyl, furylmethyl, thienylmethyl, thiazolylmethyl, pyridylmethyl, benzothienylmethyl, quinolylmethyl, phenylthienylmethyl, phenylpyridylmethyl, benzocycloheptapyridinyl, dihydrobenzocycloheptapyridinyl, dihydrodibenzooxepinyl, dihydrodibenzothiepinyl, dihydrodibenzoazepinyl, dihdrobenzopyridodiazepinyl, formyl, acetyl, pivaloyl, phenylacetyl, diphenylacetyl, diphenylpropionyl, naphthylacetyl, benzoyl, naphthoyl, oxofluorenylcarbonyl, oxodihydroanthrylcarbonyl, dioxodihydroanthrylcarbonyl, furoyl, pyridylacetyl, pyridylcarbonyl, chromonylcarbonyl, quinolylcarbonyl, phenylylaminocarbonyl, naphthylaminocarbonyl, tetrahydronaphthylaminocarbonyl, dibenzylaminocarbonyl, benzylphenylaminocarbonyl, diphenylaminocarbonyl, indolinyl-N-carbonyl, isoindolinyl-N-carbonyl, tetrahydroquinolinyl-N-carbonyl, carbazolyl-N-carbonyl, tetrahydrobenzoazepinyl-N-carbonyl, dihydrodibenzoazepinyl-N-carbonyl, dihydrobenzopyridoazepinyl-N-carbonyl, oxodihydrobenzopyridoazepinyl-N-carbonyl, methanesulfonyl, toluenesulfonyl, naphthylsulfonyl, quinolinsulfonyl and diphenylphosphinoyl,
wherein aromatic ring systems optionally are substituted independently of each other by one to three of the same or different groups which are selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and in the case of two adjacent residues in the aromatic ring methylenedioxy, and wherein
alkyl, alkenyl and cycloalkyl residues in the group G optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl)-amino.

6. The compound according to any one of claims 2 to 5, which is selected from the group consisting of:
N-[4-(4-diphenylmethylpiperazin-1-yl)-3-hydroxybutyl]-3-pyridin-3-yl-acrylamide;
N-[3-(4-diphenylmethylpiperazin-1-yl)-propoxy]-3-pyridin-3-yl-acrylamide;
N-[4-(4-diphenylmethylpiperazin-1-yl)-4-oxo-butyl]-3-pyridin-3-yl-acrylamide;
N-[3-(4-diphenylmethylpiperazin-1-sulfonyl)-propyl]-3-pyridin-3-yl-acrylamide;
N-{2-[2-(4-diphenylmethylpiperazin-1-yl)-ethoxy]-ethyl}-3-pyridin-3-yl-acrylamide;
N-(4-{4-[bis-(4-fluorophenyl)-methyl]-piperazin-1-yl}-but-2-in-yl)-3-pyridin-3-yl-acrylamide;
N-{4-[4-(4-carboxyphenyl-phenylmethyl)-piperazin-1-yl]-butyl}-3-pyridin-3-yl-acrylamide;
N-(4-{4-[(4-aminophenyl)-phenylmethyl]-piperazin-1-yl}-butyl)-3-pyridin-3-yl-acrylamide;
N-{4-[4-(9H-fluoren-9-yl)-piperazin-1-yl]-butyl}-2-(pyridin-3-yloxy)-acetamide;
N-{5-[4-(9H-fluoren-9-yl)-piperazin-1-yl]-pentyl}-3-pyridin-3-yl-acrylamide;
N-{6-[4-(9H-fluoren-9-yl)-piperazin-1-yl]-hexyl}-3-pyridin-3-yl-acrylamide;
3-pyridin-3-yl-N-{4-[4-(1,2,3,4-tetrahydronaphthalen-1-yl)-piperazin-1-yl]-butyl}-acrylamide;
3-pyridin-3-yl-N-{4-[4-(5,6,7,8-tetrahydronaphthalen-1-yl)-piperazin-1-yl]-butyl}-acrylamide;
N-{4-[4-(naphthalen-1-yl)-piperazin-1-yl]-butyl}-3-pyridin-3-yl-acrylamide;
N-[4-(4-biphenyl-2-yl-piperazin-1-yl)-butyl]-3-pyridin-3-yl-propionamide;
N-[5-(4-biphenyl-2-yl-piperazin-1-yl)-pentyl]-3-pyridin-3-yl-acrylamide;
N-[6-(4-biphenyl-2-yl-piperazin-1-yl)-hexyl]-3-pyridin-3-yl-acrylamide;
N-[4-(4-biphenyl-2-yl-piperazin-1-yl)-butyl]-2-(pyridin-3-yloxy)-acetamide;
N-[4-(4-biphenyl-2-yl-piperazin-1-yl)-butyl]-5-(pyridin-3-yl)-penta-2,4-dienoic acid amide;
N-{4-[4-(10,11-dihydro-5H-dibenzo[a,d]cyclohepten-5-yl)-piperazin-1-yl]-butyl}-3-pyridin-3-yl-propionamide;
N-{5-[4-(10,11-dihydro-5H-dibenzo[a,d]cyclohepten-5-yl)-piperazin-1-yl]-pentyl}-3-pyridin-3-yl-acrylamide;
N-{6-[4-(10,11-dihydro-5H-dibenzo[a,d]cyclohepten-5-yl)-piperazin-1-yl]-hexyl}-3-pyridin-3-yl-acrylamide;
N-{4-[4-(10,11-dihydro-5H-dibenzo[a,d]cyclohepten-5-yl)-piperazin-1-yl]-butyl}-5-(pyridin-3-yl)-penta-2,4-dienoic acid amide;
N-{4-[4-(6,11-dihydro-dibenzo[b,e]oxepin-11-yl)-piperazin-1-yl]-butyl-3-pyridin-3-yl-propionamide;
N-{2-[4-(6,11-dihydrodibenzo[b,e]thiepin-11-yl)-piperazin-1-yl]-ethyl}-3-pyridin-3-yl-acrylamide;
N-[4-(4-diphenylacetyl-piperazin-1-yl)-butyl]-3-pyridin-3-yl-acrylamide;
N-[4-(4-benzoylpiperazin-1-yl)-butyl]-3-pyridin-3-yl-acrylamide;
N-{4-[4-(2-aminobenzoyl)-piperazin-1-yl]-butyl}-3-pyridin-3-yl-acrylamide;
N-{4-[4-(4-carboxybenzoyl)-piperazin-1-yl]-butyl}-3-pyridin-3-yl-acrylamide;
N-{4-[4-(biphenyl-2-carbonyl)-piperazin-1-yl]-butyl}-3-pyridin-3-yl-acrylamide;
N-{4-[4-(9-oxo-9H-fluoren-4-carbonyl)-piperazin-1-yl]-butyl}-3-pyridin-3-yl-acrylamide;
N-{4-[4-(furan-2-carbonyl)-piperazin-1-yl]-butyl}-3-pyridin-3-yl-acrylamide;
N-{4-[4-(naphthalen-1-yl-aminocarbonyl)-piperazin-1-yl]-butyl}-3-pyridin-3-yl-propionamide;
N-{4-[4-(diphenylaminocarbonyl)-piperazin-1-yl]-butyl}-3-pyridin-3-yl-acrylamide;
N-{4-[4-(naphthalen-2-sulfonyl)-piperazin-1-yl]-butyl}-3-pyridin-3-yl-acrylamide;
N-[4-(4-diphenylphosphinoyl-piperazin-1-yl)-butyl]-3-pyridin-3-yl-acrylamide;
N-[4-(4-biphenyl-2-yl-piperazin-1-yl)-butyl]-3-pyridin-3-yl-acrylamide;
N-{4-[4-(9H-fluoren-9-yl)-piperazin-1-yl]-butyl}-3-pyridin-3-yl-acrylamide; and
N-{4-[4-(10,11-dihydro-5H-dibenzo[a,d]cyclohepten-5-yl)-piperazin-1-yl]-butyl}-3-pyridin-3-yl-acrylamide.

7. The compound according to any one of claims 2 to 6, which is present
as cis- and/or trans-isomer, E- and/or Z-isomer, pure isomer and/or mixture of isomers, as enantiomer and/or diastereomer, as racemic or non-racemic mixture, as endo/exo-isomer in case the ring system E is bicyclic,
as tautomere, when G is a heterocyclic aromatic ring or contains such in an anellated ring system, when this heterocyclic ring is substituted by free hydroxy-, mercapto- or amino groups,
as pharmacologically acceptable acid addition salt with inorganic or organic acids, such as hydrochlorides, hydrobromides, hydroiodides, sulfates, phosphates, acetates, benzoates, citrates, fumarates, gluconates, malates, maleates, methanesulfonates, lactates, oxalates, succinates, tartrates and/or tosylates, and/or
as hydrate or other solvate.

8. A method for the production of a compound according to any one of claims 1 to 7, which method is selected from the following (A), (B), (B1), (B2), (B3) and (B4):
(A) carboxylic acids of formula (II) 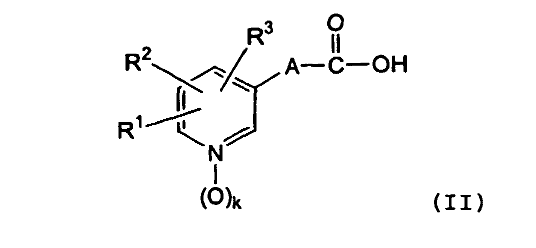 in which R¹, R², R³, A and k have the meaning given in any one of claims 1 to 7 or their reactive derivatives, especially their acid chlorides or activated esters, are reacted, optionally in the presence of condensation agents, with compounds of formula (III) 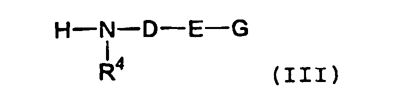 wherein D, E, G and R⁴ are defined as in claims 1 to 7 in the form of the respective free base or the respective acid addition salt, preferably in one or more inert solvents, at a temperature between -40°C and 180°C optionally in the presence of an auxiliary base;
(B) compounds of the formula (I), wherein G is hydrogen, are reacted with a compound of formula (IV)
L―G (IV)
wherein **G** has the meaning given in any one of claims 1 to 7, with the exception of hydrogen, and L is a suitable nucleofuge or reactive group,
(B1) compounds of formula (I), in which **G** is hydrogen, are reacted with a suitable alkylation agent and/or arylation agent of formula (IV),
L―G (IV)
wherein **G** is an alkyl, alkenyl, alkinyl, cycloalkyl, aryl, aralkyl, heteroaryl or heteroaralkyl residue and the leaving group L represents a reactive derivative of an alcohol selected from a halogen atom, such as a chlorine, bromine, or iodine; a sulfonic acid ester, methanesulfonyloxy group, trifluoromethanesulfonyloxy group, ethanesulfonyloxy group, benzenesulfonyloxy group, p-toluenesulfonyloxy group, p-bromobenzenesulfonyloxy group, m-nitrobenzenesulfonyloxy group and a terminal epoxide group,
wherein this reaction occurs in a suitable inert solvent at a temperature between 0°C and 180°C, depending on the reactivity of the educt,
(B2) compounds of formula (I), wherein **G** represents a hydrogen, are reacted with a carboxylic acid, carbamic acid, sulfonic acid and/or phosphinic acid of formula (V),
HO―G (V)
wherein **G** is an acyl residue, carbamoyl residue, sulfonyl residue or phosphinoyl residue, or their derivatives capable of reaction, which reaction preferably occurs in the presence of auxiliary bases in solvents and under conditions as they are described in method (A);
(B3) compounds of formula (I), wherein **G** is hydrogen, are reacted with a carbonyl group transmitter to an intermediate product and subsequently with a primary or secondary amine with the formula (VI)
H―NR¹³R¹⁵ (VI)
wherein R¹³ and R¹⁵ and/or the group ―NR¹³R¹⁵ have the meaning given in any one of claims 1 to 7 without purifying or isolating the intermediate product, preferably compound (VI) is added in a stoichiometric amount or in excess as a solution or a solid and the reaction is completed, wherein the reaction temperatures lie between -40°C and 50°C for the first partial reaction and between 0°C and 150°C for the second partial reaction,
(B4) a compound of formula (I) according to any one of claims 1 to 7, wherein G is hydrogen, is reacted with an isocyanate of formula (VII)
O=C=N-R¹³ (VII)
wherein R¹³ has the meaning given in any one of claims 1 to 7, in an absolute, inert solvent at a temperature from -20°C to 150°C.

9. A pharmaceutical composition comprising one or more of the compounds according to any one of claims 1 to 7 optionally together with (a) pharmaceutically acceptable carrier(s), (a) toxicologically safe adjuvant(s), and/or in combination with other active ingredients.

10. The pharmaceutical composition according to claim 9, which is present in a solid, peroral administrable form as a tablet, capsule, coated tablet, or as a liquid, peroral administrable solution, suspension, effervescent tablet, in the form of tabs or sachets, optionally in sustained action, and/or in gastric fluid-resistant form,
in the form of a suitable injection or infusion preparation together with suitable pharmaceutically acceptable carriers and adjuvants, optionally in sustained action form and or as a parenteral depot medicinal form or implant or in the form of a concentrate, powder or lyophilisate,
in the form of a transdermal therapeutic system for systemic treatment,
in the form of a gastrointestinal therapeutic system (GITS) for systemic treatment,
in the form of a salve, suspension, emulsion, a balm or plaster or in the form of an externally applicable solution,
in the form of a rectal, genital, or transurethral administration emulsion, a solution, a liposomal solution, an implant, suppository or a capsule,
in the form of a composition capable of being applied nasally, otologically or ophthalmologically, or
in a buccally applicable form.

11. The pharmaceutical composition according to claim 9, wherein it is in the form of an inhalation therapeutic agent, optionally in the form of a spray together with suitable pharmaceutically acceptable propellants, carriers and adjuvants.

12. The pharmaceutical composition according to claim 11 for administration by means of a controlled dosage aerosol or in the form of a dry powder dosage formulation.

13. The pharmaceutical composition according to any one of claims 9 to 12, wherein a dosage unit for single administration contains 0.001 to 2.0 mg of one or more of the compounds according to any one of claims 1 to 7.

14. The pharmaceutical composition according to any one of claims 9 to 12, wherein a dosage unit for single administration contains 0.1, 1, 2, 5, 10, 20, 25, 30, 50, 100, 200, 300, 500, 600, 800, 1000, 2000, 3000, 4000 or 5000 mg of one or more of the compounds according to any one of claims 1 to 7.

15. Use of one or more compounds according to formula (I): 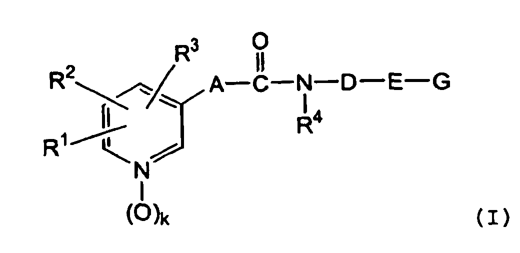 for the manufacture of a pharmaceutical composition for the treatment of cancer and/or abnormal cell growth, and/or the prevention of proliferation and/or the formation of metastases, in the human or animal body, optionally in combination with a further cytostatic agent or immunosuppressive agent, wherein:
**R**^{**1**} is selected from
hydrogen, halogen, cyano, C₁-C₆-alkyl, C₃-C₆-alkenyl, C₂-C₆-alkinyl, trifluoromethyl, C₃-C₈-cycloalkyl, C₁-C₆-hydroxyalkyl, hydroxy, C₁-C₆-alkoxy, C₃-C₆-alkenyloxy, C₃-C₆-alkinyloxy, benzyloxy, C₁-C₇-alkanoyloxy, C₂-C₇-alkoxycarbonyloxy, C₁-C₆-alkylthio, C₃-C₆-alkenylthio, C₃-C₆-alkinylthio, C₃-C₈-cycloalkyloxy, C₃-C₈-cycloalkylthio, C₂-C₇-alkoxycarbonyl, aminocarbonyl, C₂-C₇-alkylaminocarbonyl, C₃-C₁₃-dialkylaminocarbonyl, carboxy, phenyl, phenoxy, phenylthio, pyridyloxy, pyridylthio, and N**R**^{**5**}**R**^{**6**}**,** wherein
**R**^{**5**} and **R**^{**6**} are selected independently of each other from
hydrogen, C₁-C₆-alkyl, C₃-C₆-alkenyl, C₃-C₆-alkinyl, benzyl and phenyl;
**R**^{**2**} is selected from
hydrogen, halogen, cyano, C₁-C₆-alkyl, trifluoromethyl, hydroxy, C₁-C₆-alkoxy, benzyloxy and C₁-C₇-alkanoyloxy; or
**R**^{**1**} and **R**^{**2**}, if adjacent, optionally form a bridge selected from -(CH₂)₄-, -(CH=CH)₂- and -CH₂O-C**R**^{**7**}**R**^{**8**}-O-, wherein
**R**^{**7**} and **R**^{**8**} are selected independently from each other from hydrogen and C₁-C₆-alkyl;
**R**^{**3**} is selected from
hydrogen, halogen, C₁-C₆-alkyl, trifluoromethyl and C₁-C₆-hydroxyalkyl;
**R**^{**4**} is selected from
hydrogen, C₁-C₆-alkyl, C₃-C₆-alkenyl, C₃-C₆-alkinyl, C₃-C₆-cycloalkyl, hydroxy, C₁-C₆-alkoxy and benzyloxy;
**k** is 0 or 1,
**A** is selected from
C₁-C₆-alkylene, optionally substituted once to threefold by C₁-C₃-alkyl, hydroxy, C₁-C₃-alkoxy, fluorine, or phenyl,
C₂-C₆-alkylene, in which a methylene unit is isosterically replaced by O, S, **NR**^{**9**}**,** CO, SO or SO₂, wherein, with the exception of CO, the isosteric substitution is not adjacent to the amide group and **R**^{**9**} is selected from hydrogen, C₁-C₆-alkyl, C₃-C₆-alkenyl, C₃-C₆-alkinyl, C₁-C₆-acyl and C₁-C₆-alkanesulfonyl,
1,2-cyclopropylene,
C₂-C₆-alkenylene, optionally substituted once to threefold by C₁-C₃-alkyl, hydroxy, C₁-C₃-alkoxy, fluorine, cyano or phenyl,
C₄-C₆-alkadienylene, optionally substituted once or twice by C₁-C₃-alkyl, fluorine, cyano or phenyl;
1,3,5-hexatrienylene, optionally substituted by C₁-C₃-alkyl, fluorine, cyano or phenyl, and
ethinylene
**D** is selected from
C₂-C₁₀-alkylene, optionally substituted once or twice by C₁-C₆-alkyl, hydroxy, or C₁-C₆-alkoxy;
C₄-C₁₀-alkenylene, optionally substituted once or twice by C₁-C₆-alkyl, hydroxy, or C₁-C₆-alkoxy;
C₄-C₁₀-alkinylene, optionally substituted once or twice by C₁-C₆-alkyl, hydroxy, or C₁-C₆-alkoxy; and
the group consisting of C₂-C₁₀-alkylene, C₄-C₁₀-alkenylene and C₄-C₁₀-alkinylene, wherein one to three methylene units are isosterically replaced by O, S, **NR**^{**10**}**,** CO, SO or SO₂, wherein
**R**^{**10**} has the same meaning as **R**^{**9**}**,** but is selected independently thereof;
**E** represents 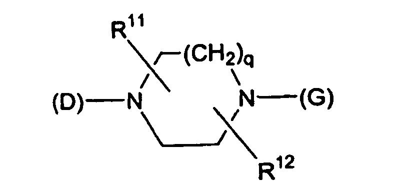 wherein
**q** is 1, 2 or 3;
**R**^{**11**} is selected from
hydrogen, C₁-C₆-alkyl, hydroxy, hydroxymethyl, carboxy, and C₂-C₇-alkoxycarbonyl, and
**R**^{**12**} is selected from
hydrogen, C₁-C₆-alkyl and an oxo group adjacent to a nitrogen atom, or
**R**^{**11**} and **R**^{**12**} together form a C₁-C₃-alkylene bridge under formation of a bicyclic ring system;
**G** is selected from G1, G2, G3, G4 and G5, wherein
**G1** represents
―(CH₂)ᵣ―(CR¹⁴R¹⁵)ₛ―R¹³,
**r** is 0, 1, 2 or 3,
**s** is 0 or 1,
**R**^{**13**} is selected from
hydrogen, C₁-C₆-alkyl, C₃-C₆-alkenyl, C₃-C₆-alkinyl, C₃-C₈-cycloalkyl;
the group consisting of saturated and unsaturated, four to eight-membered heterocycles, which optionally contain one or two hetero-atoms selected from N, S and O;
benzyl, phenyl;
the group consisting monocyclic aromatic five to six-membered heterocycles, which optionally contain one to three hetero-atoms selected from the N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms are selected from N, S and O and the linkage occurs either over an aromatic ring or a hydrogenated ring and either directly or over a methylene group,
**R**^{**14**} has the same meaning as **R**^{**13**}**,** but is selected independently thereof;
**R**^{**15**} is selected from
hydrogen, hydroxy, methyl, benzyl, phenyl,
the group consisting of monocyclic aromatic five to six-membered heterocycles, which contain one to three hetero-atoms from the group N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group,
**G2** is selected from 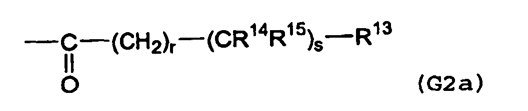 and 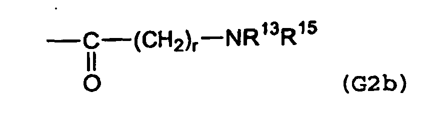 wherein **r, s** and the substituents **R**^{**13**} to **R**^{**15**} have the above meaning, or the group
―NR¹³R¹⁵
represents a nitrogen-containing heterocycle bound over the nitrogen atom which nitrogen-containing heterocycle is selected from
the group consisting of saturated and unsaturated monocyclic, four to eight-membered heterocycles, which, aside from the essential nitrogen atom, optionally contain one or two further hetero-atoms selected from N, S and O, and
the group consisting of saturated and unsaturated bi- or tricyclic, anellated or bridged heterocycles with 8 to 16 ring atoms, that aside from the essential nitrogen atom, optionally contain one or two further hetero-atoms selected from N, S and O;
**G3** represents
―SO₂―(CH₂)ᵣ―R¹³,
wherein **r** and **R**^{**13**} have the above meanings,
**G4** represents 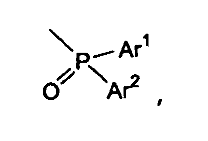 wherein
**Ar**^{**1**} and **Ar**^{**2**} are selected independently from each other from phenyl, pyridyl and naphthyl;
**G5** represents
―COR¹⁶ (G5)
wherein
**R**^{**16**} is selected from
trifluoromethyl, C₁-C₆-alkoxy, C₃-C₆-alkenyloxy, and benzyloxy,
wherein aromatic ring systems in the substituents **R**^{**1**}**, R**^{**2**}**, R**^{**4**}**, R**^{**5**}**, R**^{**6**}**, R**^{**13**}**, R**^{**14**}**, R**^{**15**}**, R**^{**16**}**, Ar**^{**1**} and **Ar**^{**2**} and/or in the ring system ―NR¹³R¹⁵ optionally are substituted independently from each other by one to three of the same or different groups selected from
halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide for two adjacent residues on the aromatic ring, and wherein
alkyl-, alkenyl- and cycloalkyl residues in the groups **G1, G2** and **G**^{**3**} optionally are substituted by one or two of the same or different groups which are selected from hydroxy, carboxy, C₂-C₇-alkoxycarbonyl, benzyloxycarbonyl, amino, mono-C₁-C₆-alkylamino and di-(C₁-C₆-alkyl-amino);
their cis- and trans-isomers, E- and Z-isomers, enantiomers, diastereomers and other isomers as well as their racemic or non-racemic mixtures and the corresponding endo- and exo-isomers for the case that the ring system E is bicyclic;
their tautomeres; as well as
their acid addition salts including their hydrates and solvates.

16. The use according to claim 15, wherein
**R**^{**1**} is selected from
hydrogen, halogen, cyano, C₁-C₆-alkyl, trifluoromethyl, C₃-C₈-cycloalkyl, C₁-C₆-hydroxyalkyl, hydroxy, C₁-C₄-alkoxy, benzyloxy, C₁-C₄-alkylthio, C₁-C₅-alkanoyloxy, C₁-C₄-alkylthio, C₂-C₅-alkoxycarbonyl, aminocarbonyl, C₂-C₅-alkylaminocarbonyl, C₃-C₉-dialkylaminocarbonyl, carboxy, phenyl, phenoxy, phenylthio, pyridyloxy, and **NR**^{**5**}**R**^{**6**}**,** wherein
**R**^{**5**} and **R**^{**6**} are selected independently from each other from hydrogen and C₁-C₆-alkyl;
**R**^{**2**} is selected from
hydrogen, halogen, cyano, C₁-C₆-alkyl, trifluoromethyl, hydroxy, and C₁-C₄-alkoxy;
**R**^{**3**} is selected from
hydrogen, halogen and C₁-C₆-alkyl;
**R**^{**4**} is selected from
hydrogen, C₁-C₆-alkyl, C₃-C₆-alkenyl, C₃-C₆-cycloalkyl, hydroxy, C₁-C₆-alkoxy and benzyloxy;
**k** is 0 or 1,
**A** is selected from
C₁-C₆-alkylene, optionally substituted once to threefold by C₁-C₃-alkyl, hydroxy, fluorine, or phenyl,
C₂-C₆-alkylene, in which a methylene unit is isosterically replaced by O S, **NR**^{**9**}, CO, SO or SO₂, wherein, with the exception of CO, the isosteric substitution is not adjacent to the amide group and the residue **R**^{**9**} is selected from hydrogen, C₁-C₆-alkyl, C₁-C₆-acyl and methane sulfonyl;
1,2-cyclopropylene,
C₂-C₆-alkenylene, optionally substituted once to threefold by C₁-C₃-alkyl, hydroxy, fluorine, cyano or phenyl,
C₄-C₆-alkadienylene, optionally substituted once or twice by C₁-C₃-alkyl, fluorine, cyano or phenyl;
1,3,5-hexatrienylene, optionally substituted by C₁-C₃-alkyl, fluorine, or cyano, and
ethinylene
**D** is selected from
C₂-C₁₀-alkylene, optionally substituted once or twice by C₁-C₃-alkyl or hydroxy;
C₄-C₁₀-alkenylene, optionally substituted once or twice by C₁-C₃-alkyl or hydroxy;
C₄-C₁₀-alkinylene, optionally substituted once or twice by C₁-C₃-alkyl or hydroxy; and
the group consisting of C₂-C₁₀-alkylene, C₄-C₁₀-alkenylene and C₄-C₁₀-alkinylene, wherein one to three methylene units are isosterically replaced by O, S, **NR**^{**10**}**,** CO, SO or SO₂, wherein
**R**^{**10**} has the same meaning as **R**^{**9**}**,** but is selected independently thereof;
**E** represents 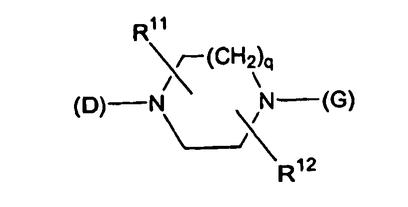 wherein
**q** is 1, 2 or 3;
**R**^{**11**} is selected from
hydrogen, C₁-C₃-alkyl, hydroxy, hydroxymethyl, carboxy, and C₂-C₇-alkoxycarbonyl, and
**R**^{**12**} is selected from hydrogen and an oxo group adjacent to a nitrogen atom or
**R**^{**11**} and **R**^{**12**}, together, form a C₁-C₃-alkylene bridge under formation of a bicyclic ring system;
**G** is selected from G1, G2, G3, G4 and G5, wherein
**G1** represents
―(CH₂)ᵣ―(CR¹⁴R¹⁵)ₛ―R¹³,
**r** is 0, 1 or 2,
**s** is 0 or 1,
**R**^{**13**} is selected from
hydrogen, C₁-C₆-alkyl, C₃-C₆-alkenyl, C₃-C₆-alkinyl, C₃-C₈-cycloalkyl; benzyl, phenyl;
the group consisting of monocyclic aromatic five to six-membered heterocycles, which contain one to three hetero-atoms selected from N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group,
**R**^{**14**} has the same meaning as **R**^{**13**}, but is selected independently thereof;
**R**^{**15**} is selected from
hydrogen, hydroxy, methyl, benzyl, phenyl;
the group consisting of monocyclic aromatic five to six-membered heterocycles, which contain one to three hetero-atoms selected from N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group, and
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group;
**G2** is selected from 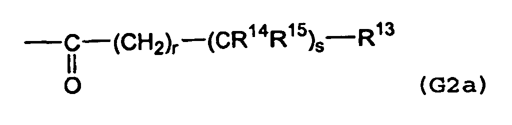 and 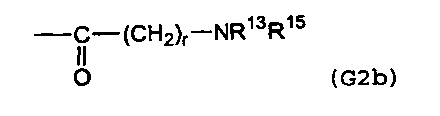 wherein **r, s** and the substituents **R**^{**13**} to **R**^{**15**} have the above meaning, or the group
―NR¹³R¹⁵
is a nitrogen-containing heterocycle bound over the nitrogen atom which nitrogen-containing heterocycle is selected from
the group consisting of saturated and unsaturated monocyclic, four to eight-membered heterocycles, which, aside from the essential nitrogen atom, optionally contain one or two further hetero-atoms selected from N, S and O, and
the group consisting of saturated and unsaturated bi- or tricyclic, anellated or bridged heterocycles with 8 to 16 ring atoms, that aside from the essential nitrogen atom, optionally contain one or two further hetero-atoms selected from N, S and O;
**G3** represents
―SO₂―(CH₂)ᵣ―R¹³,
wherein **r** and **R**^{**13**} have the above meaning,
**G4** represents 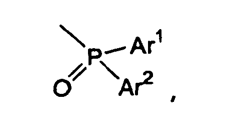 wherein
**Ar**^{**1**} and **Ar**^{**2**} are selected independently from each other from phenyl, pyridyl and naphthyl;
**G5** represents
―COR¹⁶,
wherein
**R**^{**16**} is selected from
trifluoromethyl, C₁-C₆-alkoxy, C₃-C₆-alkenyloxy, and benzyloxy,
wherein aromatic ring systems in the substituents **R**^{**1**}**, R**^{**2**}**, R**^{**4**}**, R**^{**5**}**, R**^{**6**}**, R**^{**13**}**, R**^{**14**}**, R**^{**15**}**, R**^{**16**}**, Ar**^{**1**} and **Ar**^{**2**} and/or in the ring system ―NR¹³R¹⁵ optionally are substituted independently from each other by one to three of the same or different groups selected from
halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide in the case of two adjacent residues on the aromatic ring,
wherein alkyl, alkenyl and cycloalkyl residues in the groups **G**^{**1**}**, G**^{**2**} and **G**^{**3**} optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl-amino).

17. The use according to claim 15 or 16, wherein
**R**^{**1**} is selected from
hydrogen, halogen, cyano, methyl, ethyl, trifluoromethyl, hydroxy, C₁-C₄-alkoxy, benzyloxy, C₁-C₅-alkanoyloxy, methylthio, ethylthio, methoxycarbonyl, tert-butoxycarbonyl aminocarbonyl, carboxy, phenoxy, and phenylthio,
**R**^{**2**} is selected from
hydrogen, halogen, trifluoromethyl, and hydroxy,
**R**^{**3**} is selected from
hydrogen, and halogen,
**R**^{**4**} is selected from
hydrogen, C₁-C₃-alkyl, allyl, hydroxy, and C₁-C₃-alkoxy;
**k** is 0 or 1,
**A** is selected from
C₁-C₆-alkylene, optionally substituted once or twice by C₁-C₃-alkyl, hydroxy or fluorine;
C₂-C₆-alkylene, in which a methylene unit is isosterically replaced by O, S, **NR**^{**9**}, CO, SO or SO₂, wherein, with the exception of CO, the isosteric substitution is not adjacent to the amide group,
C₂-C₆-alkylene, optionally substituted once or twice by C₁-C₃ alkyl, hydroxy and/or fluorine;
C₄-C₆-alkadienylene, optionally substituted by C₁-C₃-alkyl or one or two fluorine atoms; and
1,3,5-hexatrienylene, optionally substituted by fluorine;
**D** is selected from
C₂-C₈-alkylene, optionally substituted once or twice by methyl or hydroxy;
C₄-C₈-alkenylene, optionally substituted once or twice by methyl or hydroxy;
C₄-C₈-alkinylene, optionally substituted once or twice by methyl or hydroxy; and
the group consisting of C₂-C₈-alkylene, C₄-C₈-alkenylene and C₄-C₈-alkinylene, wherein one to three methylene units are each isosterically replaced by O, S, NH, N(CH₃) N(COCH₃), N(SO₂CH₃), CO, SO or SO₂,
**E** represents 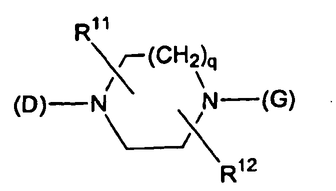 wherein
**q** is 1 or 2;
**R**^{**11**} is selected from
hydrogen, C₁-C₃-alkyl, hydroxymethyl, and carboxy, and
**R**^{**12**} is selected from
hydrogen and an oxo group adjacent to a nitrogen atom
**G** is selected from G1, G2, G3, G4 and G5, wherein
**G1** represents
―(CH₂)ᵣ―(CR¹⁴R¹⁵)ₛ―R¹³
**r** is 0, 1 or 2,
**s** is 0 or 1;
**R**^{**13**} is selected from
hydrogen, C₁-C₆-alkyl, C₃-C₈-cycloalkyl, benzyl, phenyl,
the group consisting of benzocyclobutyl, indanyl, indenyl, oxoindanyl, naphthyl, dihydronaphthyl, tetrahydronaphthyl, oxotetrahydronaphthyl, biphenylenyl, fluorenyl, oxofluorenyl, anthryl, dihydroanthryl, oxodihydroanthryl, dioxodihydroanthryl, phenanthryl, dihydrophenanthryl, oxodihydrophenanthryl, dibenzocycloheptenyl, oxodibenzocycloheptenyl, dihydrodibenzocycloheptenyl, oxodihydrodibenzocycloheptenyl, dihydrodibenzocyclooctenyl, tetrahydrodibenzocyclooctenyl and oxotetrahydrodibenzocyclooctenyl, bound directly or over a methylene group; and
the group consisting of furyl, thienyl, pyrrolyl, oxazolyl, isoxazolyl, thiazolyl, iso-thiazolyl, pyrazolyl, imidazolyl, oxadiazolyl, thiadiazolyl, triazolyl, pyridyl, pyrazinyl, pyridazinyl, pyrimidinyl, triazinyl, imidazothiazolyl, benzofuryl, dihydrobenzofuryl, benzothienyl, dihydrobenzothienyl, indolyl, indolinyl, isoindolinyl, oxoindolinyl, dioxoindolinyl, benzoxazolyl, oxobenzooxazolinyl, benzoisooxazolyl, oxobenzoisooxazolinyl, benzothiazolyl, oxobenzthiazolinyl, benzoisothiazolyl, oxobenzoisothiazolinyl, benzoimidazolyl, oxobenzoimidazolinyl, indazolyl, oxoindazolinyl, benzofurazanyl, benzothiadiazolyl, benzotriazolyl, oxazolopyridyl, oxodihydrooxazolopyridyl, thiazolopyridyl, oxodihydrothiazolopyridyl, isothiazolopyridyl, imidazopyridyl, oxodihydroimidazopyridyl, pyrazolopyridyl, oxodihydropyrazolopyridyl, thienopyrimidinyl, chromanyl, chromanonyl, benzopyranyl, chromonyl, quinoloyl, isoquinoloyl, dihydroquinolyl, oxodihydroquinolinyl, tetrahydroquinolyl, oxotetrahydroquinolinyl, benzodioxanyl, quinoxalinyl, quinazolinyl, naphthyridinyl, carbazolyl, tetrahydrocarbazolyl, oxotetrahydrocarbazolyl, pyridoindolyl, acridinyl, oxodihydroacridinyl, phenanthridinyl, dihydrophenanthridinyl, oxodihydrophenanthridinyl, dibenzoisoquinolinyl, dihydrodibenzoisoquinolinyl, oxodihydrodibenzoisoquinolinyl, phenothiazinyl, dihydrodibenzooxepinyl, oxodihydrodibenzooxepinyl, benzocycloheptathienyl, oxobenzocycloheptathienyl, dihydrothienobenzothiepinyl, oxodihydrothienobenzothiepinyl, dihydrodibenzothiepinyl, oxodihydrodibenzothiepinyl, octahydrodibenzothiepinyl, dibenzoazepinyl, dihydrodibenzoazepinyl, oxodihydrodibenzoazepinyl, octahydrodibenzoazepinyl, benzocycloheptapyridyl, oxobenzocycloheptapyridyl, pyridobenzoazepinyl, dihydropyridobenzoazepinyl, oxodihydropyridobenzoazepinyl, dihydropyridobenzodiazepinyl, dihydrodibenzooxazepinyl, dihydropyridobenzooxepinyl, dihydropyridobenzooxazepinyl, oxodihydropyridobenzooxazepinyl, dihydrodibenzothiazepinyl, oxodihydrodibenzothiazepinyl, dihydropyridobenzothiazepinyl and oxodihydropyridobenzothiazepinyl, bound directly or over a methylene group;
**R**^{**14**} has the same meaning as **R**^{**13**} but is selected independently thereof;
**R**^{**15**} is selected from
hydroxy, methyl, benzyl, phenyl, indanyl, indenyl, naphthyl, dihydronaphthyl, tetrahydronaphthyl, furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, triazinyl, benzofuryl, benzothienyl, indolyl, indolinyl, benzooxazolyl, benzothiazolyl, benzoimidazolyl, chromanyl, quinolyl and tetrahydroquinolyl, bound directly or over a methylene group;
**G2** is selected from 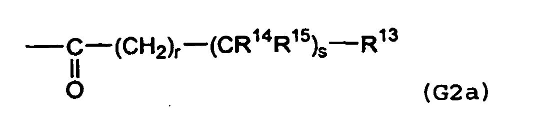 and 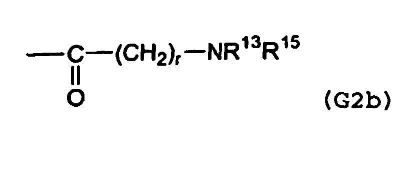 wherein **r, s** and the substituents **R**^{**13**} to **R**^{**15**} have the above meaning, or the group
―NR¹³R¹⁵
represents azetidine, pyrrolidine, piperidine, (1H)-tetrahydropyridine, hexahydroazepine, (1H)-tetrahydroazepine, octahydroazocine, pyrazolidine, piperazine, hexahydrodiazepine, morpholine, hexahydrooxazepine, thiomorpholine, thiomorpholin-1,1-dioxide, of 5-aza-bicyclo-[2.1.1]hexane, 2-aza-bicyclo[2.2.1]heptane, 7-aza-bicyclo-[2.2.1]heptane, 2,5-diaza-bicyclo[2.2.1]heptane, 2-aza-bicyclo[2.2.2]octane, 8-aza-bicyclo[3.2.1]octane, 2,5-diaza-bicyclo[2.2.2]octane, 9-aza-bicyclo[3.3.1]nonane, indoline, isoindoline, (1H)-dihydroquinoline, (1H)-tetrahydroquinolin, (2H)-tetrahydroisoquinoline, (1H)-tetrahydroquinoxaline, (4H)-dihydrobenzooxazine, (4H)-dihydrobenzothiazine, (1H)-tetrahydrobenzo[b]azepine, (1H)-tetrahydrobenzo[c]azepine, (1H)-tetrahydrobenzo[d]azepine, (5H)-tetrahydrobenzo[b]ox-azepine, (5H)-tetrahydrobenzo[b]thiazepine, 1,2,3,4-tetra-hydro-9H-pyrido[3,4-b]indole, (10H)-dihydroacridine, (10H)-dihydrophenanthridine, 1,2,3,4-tetrahydroacridanone, (10H)-phenoxazine, (10H)-phenothiazine, (5H)-dibenzoazepine, (5H)-dihydrodibenzoazepine, (5H)-octahydrodibenzoazepine, dihydrobenzo[d,e]isoquinoline, (5H)-dihydrodibenzodiazepine, (5H)-benzo[b]pyrido-[f]azepine, (5H)-Dihydrobenzo[b]pyri-do[f]azepine, (11H)-Dihydrodibenzo[b,e]oxazepine, (11H)-dihydrodibenzo[b,e]thiazepine, (10H)-dihydrodibenzo[b,f]-oxazepine, (10H)-dihydrodibenzo[b,f]thiazepine, (5H)-tetra-hydrodibenzoazocine, (11H)-dihydrobenzo[e]pyrido[b]-1,4-diazepin-6-one or (11H)-Dihydrobenzo[b]pyrido[e]-1,4-diazepin-5-one,
**G3** represents
―SO₂―(CH₂)ᵣ―R¹³,
wherein **r** and **R**^{**13**} have the above meaning,
**G4** represents 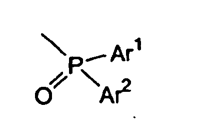 wherein
**Ar**^{**1**} and **Ar**^{**2**} are selected independently from each other from phenyl, pyridyl and naphthyl;
**G5** represents
―COR¹⁶,
wherein
**R**^{**16**} is selected from
trifluoromethyl, C₁-C₆-alkoxy, C₃-C₆-alkenyloxy, and benzyloxy,
wherein aromatic ring systems in the substituents optionally are substituted independently from each other by one to three of the same or different groups selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide in the case of two adjacent residues on the aromatic ring, and
wherein alkyl-, alkenyl- and cycloalkyl residues in the groups **G1, G2** and **G3** optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl-amino).

18. The use according to any one of claims 15 to 17, wherein
**R**^{**1**} is selected from
hydrogen, fluorine, chlorine, bromine, methyl, ethyl, trifluoromethyl, hydroxy, C₁-C₄-alkoxy, methylthio, ethylthio, carboxy and phenoxy;
**R**^{**2**} is selected from hydrogen, chlorine and methyl;
**R**^{**3**} is hydrogen;
**R**^{**4**} is selected from
hydrogen, C₁-C₃-alkyl and hydroxy,
**k** is 0
**A** is selected from
C₂-C₆-alkylene, which is optionally substituted once or twice by hydroxy or fluorine;
C₂-C₆-alkylene, wherein a methylene unit is isosterically replaced by O, S or CO, wherein, with the exception of CO, the isosteric substitution is not adjacent to the amide group;
C₂-C₆-alkenylene which is optionally substituted by C₁-C₃-alkyl and/or fluorine; and
C₄-C₆-alkadienylene;
**D** is selected from
C₂-C₈-alkylene which is optionally substituted by methyl or hydroxy;
C₄-C₈-alkenylene, which is optionally substituted by hydroxy;
C₄-C₈-alkinylene, which is optionally substituted by hydroxy; and
the group consisting of C₂-C₈-alkylene, C₄-C₈-alkenylene, and C₄-C₈-alkinylene wherein a methylene unit is isosterically replaced by O, NH, N(CH₃), CO or SO₂ or an ethylene group is isosterically replaced by a group NH-CO and/or CO-NH or a propylene group is isosterically replaced by a group NH-CO-O and/or O-CO-NH;
**E** is selected from piperazine and hexahydro-1,4-diazepine, wherein the ring optionally is substituted by one or two methylene groups and/or by an oxo group adjacent to a nitrogen atom;
**G** is selected from hydrogen, C₃-C₈-cycloalkyl, methoxycarbonyl, tert-butoxycarbonyl, benzyloxycarbonyl, trifluoroacetyl, diphenylphosphinoyl,
―(CH₂)ᵣ―(CR¹⁴R¹⁵)ₛ―R¹³, 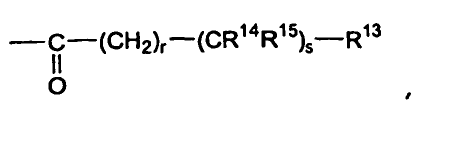 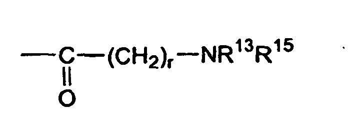
and
―SO₂―(CH₂)ᵣ―R¹³
wherein
**r** is 0 or 1
**s** is 0 or 1,
**R**^{**13**} is selected from hydrogen, methyl, benzyl, phenyl,
the group consisting of indanyl, indenyl, oxoindanyl, naphthyl, tetrahydronaphthyl, fluorenyl, oxofluorenyl, anthryl, dihydroanthryl, oxodihydroanthryl, dioxodihydroanthryl, phenanthryl, dihydrophenanthryl, oxydihydrophenanthryl, dibenzocycloheptenyl, dihydrodibenzocycloheptenyl, and oxodihydrodibenzocycloheptyl, bound directly or over a methylene group, and
the group consisting of furyl, thienyl, oxazolyl, isooxazolyl, thiazolyl, imidazolyl, oxadiazolyl, pyridyl, pyrazinyl, pyrimidinyl, imidazothiazolyl, benzofuryl, benzothienyl, indolyl, indolinyl, oxoindolinyl, dioxoindolinyl, benzoxazolyl, oxobenzooxazolinyl, benzothiazolyl, oxobenzthiazolinyl, benzimidazolyl, oxobenzimidazolinyl, indazolyl, benzofurazanyl, benzotriazolyl, oxazolopyridyl, oxodihydrooxazolopyridyl, thiazolopyridyl, oxodihydrothiazolopyridyl, imidazopyridyl, oxodihydroimidazopyridyl, chromanyl, chromanonyl, benzopyranyl, chromonyl, quinolyl, isoquinolyl, oxodihydroquinolinyl, tetrahydroquinolyl, oxotetrahydroquinolinyl, benzodioxanyl, quinazolinyl, carbazolyl, acridinyl, dihydroacridinyl, oxodihydroacridinyl, dihydrophenanthridinyl dihydrobenzoisoquinolinyl, phenothiazinyl, dihydrodibenzooxepinyl, benzocycloheptathienyl, dihydrothienobenzothiepinyl, dihydrodibenzothiepinyl, oxodihydrodibenzothiepinyl, dihydrodibenzoazepinyl, oxodihydrodibenzoazepinyl, octahydrodibenzoazepinyl, benzocycloheptapyridyl, dihydropyridobenzodiazepinyl, and dihydrodibenzothiazepinyl, bound directly or over a methylene group,
**R**^{**14**} is selected from hydrogen, methyl, benzyl, and phenyl,
**R**^{**15**} is selected from
hydrogen, hydroxy, methyl, benzyl, phenyl; and ,
the group consisting of naphthyl, tetrahydronaphthyl, furyl, thienyl, oxazolyl, thiazolyl, imidazolyl, pyridyl, benzofuryl, benzothienyl, indolyl, indolinyl, benzoxazolyl, benzothiazolyl, benzimidazolyl, chromanyl, quinolyl and tetrahydroquinolyl, bound directly or over a methylene group;
wherein the group
―NR¹³R¹⁵
represents a nitrogen-containing heterocycle bound over the nitrogen atom which nitrogen-containing heterocycle is selected from
pyrrolidine, piperidine, hexahydroazepine, piperazine, hexahydrodiazepine, morpholine, hexahydroxazepine, thiomorpholine, 7-aza-bicyclo[2,2.1]heptane, 2,5-diaza-bicyclo[2.2.1]heptane, indoline, isoindoline, (1H)-dihydroquinoline, (1H)-tetrahydroquinoline, (2H)-tetrahydroisoquinoline, (4H)-dihydrobenzoxazine, (4H)-dihydrobenzothiazine, (1H)-tetrahydrobenzo[b]azepine, (1H)-tetrahydrobenzo[d]azepine, (5H)-tetrahydrobenzo[b]oxazepine, (5H)-tetrahydrobenzo[b]thiazepine, (10H)-dihydroacridine, 1,2,3,4-tetrahydroacridanone, (10H)-dihydrophenanthridine, (1H)-dihydrobenzo-[d,e]isoquinoline, (10H)-phenothiazine, (5H)-dibenzo[b,f]azepine, (5H)-dihydrodibenzo[b,f]azepine, (5H)-dihydrodibenzo[c,e]azepine, (5H)-dihydrodibenzo-diazepine, (11H)-dihydrodibenzo[b,e]oxazepine (11H)-dihydrodibenzo[b,e]thiazepine, (5H)-dihydrobenzo[b]pyrido[3,2-f]azepine and (11H)-6-oxodihydrobenzo[e]pyrido[3,2-b][1,4]diazepine,
wherein aromatic ring systems in the substituents optionally are substituted, independently of each other, by one to three of the same or different groups selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carbocyalkenyl, C₂-C₇-alkoxycarbonyl, benzyloxycarbonyl, nitro, amino, mono-C₁-C₆-alkylamino, di-(C₁-C₆-alkyl)-amino, and in the case of two adjacent residues on the aromatic ring, methylenedioxy, and
wherein alkyl, alkenyl and cycloalkyl residues in the group G optionally are substituted by one or two of the same or different groups which are selected from hydroxy, carboxy, C₂-C₇-alkoxycarbonyl, benzyloxycarbonyl, amino, mono-C₁-C₆-alkylamino and di-(C₁-C₆-alkyl) amino.

19. The use according to any one of claims 15 to 18, wherein
**R**^{**1**} is selected from
hydrogen, fluorine, methyl, trifluoromethyl and ethylthio;
**R**^{**2**}, **R**^{**3**} and **R**^{**4**} are each hydrogen;
**k** is 0,
**A** is selected from
the group consisting of ethylene, propylene and butylene, which are each optionally substituted by hydroxy or one or two fluorine atoms;
OCH₂, SCH_{2;}
ethenylene, and 1,3-butadienylene;
**D** is selected from
C₂-C₆-alkylene which is optionally substituted by hydroxy;
C₄-C₆ alkenylene;
C₄-C₆ alkinylene; and
the group consisting of C₂-C₆ alkylene, C₄-C₆ alkenylene and C₄-C₆ alkinylene, wherein one or two methylene units are isosterically replaced by O, NH, CO or SO₂;
**E** is piperazine or hexahydro-1,4-diazepine;
**G** is selected from
phenyl, benzyl, phenethyl, diphenylmethyl, naphthyl, tetrahydronaphtyl, naphthylmethyl, fluorenyl fluorenylmethyl, anthrylmethyl, dihydrodibenzocycloheptenyl, furylmethyl, thienylmethyl, thiazolylmethyl, pyridylmethyl, benzothienylmethyl, quinolylmethyl, phenylthienylmethyl, phenylpyridylmethyl, benzocycloheptapyridinyl, dihydrobenzocycloheptapyridinyl, dihydrodibenzooxepinyl, dihydrodibenzothiepinyl, dihydrodibenzoazepinyl, dihdrobenzopyridodiazepinyl, formyl, acetyl, pivaloyl, phenylacetyl, diphenylacetyl, diphenylpropionyl, naphthylacetyl, benzoyl, naphthoyl, oxofluorenylcarbonyl, oxodihydroanthrylcarbonyl, dioxodihydroanthrylcarbonyl, furoyl, pyridylacetyl, pyridylcarbonyl, chromonylcarbonyl, quinolylcarbonyl, phenylylaminocarbonyl, naphthylaminocarbonyl, tetrahydronaphthylaminocarbonyl, dibenzylaminocarbonyl, benzylphenylaminocarbonyl, diphenylaminocarbonyl, indolinyl-N-carbonyl, isoindolinyl-N-carbonyl, tetrahydroquinolinyl-N-carbonyl, carbazolyl-N-carbonyl, tetrahydrobenzoazepinyl-N-carbonyl, dihydrodibenzoazepinyl-N-carbonyl, dihydrobenzopyridoazepinyl-N-carbonyl, oxodihydrobenzopyridoazepinyl-N-carbonyl, methanesulfonyl, toluenesulfonyl, naphthylsulfonyl, quinolinsulfonyl and diphenylphosphinoyl,
wherein aromatic ring systems optionally are substituted independently of each other by one to three of the same or different groups which are selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and in the case of two adjacent residues in the aromatic ring methylenedioxy, and wherein alkyl, alkenyl and cycloalkyl residues in the group **G** optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl)-amino.

20. The use according to any one of claims 15 to 19, wherein the compound(s) of formula (I) is/are selected from the group consisting of:
N-[4-(4-diphenylmethylpiperazin-1-yl)-3-hydroxybutyl]-3-pyridin-3-yl-acrylamide;
N-[3-(4-diphenylmethylpiperazin-1-yl)-propoxy]-3-pyridin-3-yl-acrylamide;
N- [4-(4-diphenylmethylpiperazin-1-yl)-4-oxo-butyl] -3-pyridin-3-yl-acrylamide;
N-[3-(4-diphenylmethylpiperazin-1-sulfonyl)-propyl]-3-pyridin-3-yl-acrylamide;
N-{2-[2-(4-diphenylmethylpiperazin-1-yl)-ethoxy]-ethyl}-3-pyridin-3-yl-acrylamide;
N-(4-{4-[bis-(4-fluorophenyl)-methyl]-piperazin-1-yl}-but-2-in-yl)-3-pyridin-3-yl-acrylamide;
N-{4-[4-(4-carboxyphenyl-phenylmethyl)-piperazin-1-yl]-butyl}-3-pyridin-3-yl-acrylamide;
N-(4-{4-[(4-aminophenyl)-phenylmethyl]-piperazin-1-yl}-butyl)-3-pyridin-3-yl-acrylamide;
N-{4-[4-(9H-fluoren-9-yl)-piperazin-1-yl]-butyl}-2-(pyridin-3-yloxy)-acetamide;
N-{5-[4-(9H-fluoren-9-yl)-piperazin-1-yl]-pentyl}-3-pyridin-3-yl-acrylamide;
N-{6-[4-(9H-fluoren-9-yl)-piperazin-1-yl]-hexyl}-3-pyridin-3-yl-acrylamide;
3-pyridin-3-yl-N-{4-[4-(1,2,3,4-tetrahydronaphthalen-1-yl)-piperazin-1-yl]-butyl}-acrylamide;
3-pyridin-3-yl-N-{4-[4-(5,6,7,8-tetrahydronaphthalen-1-yl)-piperazin-1-yl]-butyl}-acrylamide;
N-{4-[4-(naphthalen-1-yl)-piperazin-1-yl]-butyl}-3-pyridin-3-yl-acrylamide;
N-[4-(4-biphenyl-2-yl-piperazin-1-yl)-butyl]-3-pyridin-3-yl-propionamide;
N-[5-(4-biphenyl-2-yl-piperazin-1-yl)-pentyl]-3-pyridin-3-yl-acrylamide;
N-[6-(4-biphenyl-2-yl-piperazin-1-yl)-hexyl]-3-pyridin-3-yl-acrylamide;
N-[4-(4-biphenyl-2-yl-piperazin-1-yl)-butyl]-2-(pyridin-3-yloxy)-acetamide;
N-[4-(4-biphenyl-2-yl-piperazin-1-yl)-butyl]-5-(pyridin-3-yl)-penta-2,4-dienoic acid amide;
N-{4-[4-(10,11-dihydro-5H-dibenzo[a,d]cyclohepten-5-yl)-piperazin-1-yl]-butyl}-3-pyridin-3-yl-propionamide;
N-{5-[4-(10,11-dihydro-5H-dibenzo[a,d]cyclohepten-5-yl)-piperazin-1-yl]-pentyl}-3-pyridin-3-yl-acrylamide;
N-{6-[4-(10,11-dihydro-5H-dibenzo[a,d]cyclohepten-5-yl)-piperazin-1-yl]-hexyl}-3-pyridin-3-yl-acrylamide;
N-{4-[4-(10,11-dihydro-5H-dibenzo[a,d]cyclohepten-5-yl)-piperazin-1-yl]-butyl}-5-(pyridin-3-yl)-penta-2,4-dienoic acid amide;
N-{4-[4-(6,11-dihydro-dibenzo[b,e]oxepin-11-yl)-piperazin-1-yl]-butyl-3-pyridin-3-yl-propionamide;
N-{2-[4-(6,11-dihydrodibenzo[b,e]thiepin-11-yl)-piperazin-1-yl]-ethyl}-3-pyridin-3-yl-acrylamide;
N-[4-(4-diphenylacetyl-piperazin-1-yl)-butyl]-3-pyridin-3-yl-acrylamide;
N-[4-(4-benzoylpiperazin-1-yl)-butyl]-3-pyridin-3-yl-acrylamide;
N-{4-[4-(2-aminobenzoyl)-piperazin-1-yl]-butyl}-3-pyridin-3-yl-acrylamide;
N-{4-[4-(4-carboxybenzoyl)-piperazin-1-yl]-butyl}-3-pyridin-3-yl-acrylamide;
N-{4-[4-(biphenyl-2-carbonyl)-piperazin-1-yl]-butyl}-3-pyridin-3-yl-acrylamide;
N-{4-[4-(9-oxo-9H-fluoren-4-carbonyl)-piperazin-1-yl]-butyl}-3-pyridin-3-yl-acrylamide;
N-{4-[4-(furan-2-carbonyl)-piperazin-1-yl]-butyl}-3-pyridin-3-yl-acrylamide;
N-{4-[4-(naphthalen-1-yl-aminocarbonyl)-piperazin-1-yl]-butyl}-3-pyridin-3-yl-propionamide;
N-{4-[4-(diphenylaminocarbonyl)-piperazin-1-yl]-butyl}-3-pyridin-3-yl-acrylamide;
N-{4-[4-(naphthalen-2-sulfonyl)-piperazin-1-yl]-butyl}-3-pyridin-3-yl-acrylamide;
N-[4-(4-diphenylphosphinoyl-piperazin-1-yl)-butyl]-3-pyridin-3-yl-acrylamide;
N-[4-(4-biphenyl-2-yl-piperazin-1-yl)-butyl]-3-pyridin-3-yl-acrylamide;
N-{4-[4-(9H-fluoren-9-yl)-piperazin-1-yl]-butyl}-3-pyridin-3-yl-acrylamide; and
N-{4-[4-(10,11-dihydro-5H-dibenzo[a,d]cyclohepten-5-yl)-piperazin-1-yl]-butyl}-3-pyridin-3-yl-acrylamide.

21. Use of one or more compounds according to formula (I): 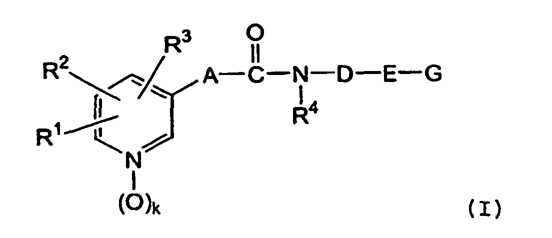 for the manufacture of a pharmaceutical composition for the suppression of the rejection reaction after an organ transplantation in the human or animal body, optionally in combination with a further cytostatic agent or immunosuppressive agent and/or further pharmaceutical compositions suitable for these indications,
wherein:
**R**^{**1**} is selected from
hydrogen, halogen, cyano, C₁-C₆-alkyl, C₃-C₆-alkenyl, C₂-C₆-alkinyl, trifluoromethyl, C₃-C₈-cycloalkyl, C₁-C₆-hydroxyalkyl, hydroxy, C₁-C₆-alkoxy, C₃-C₆-alkenyloxy, C₃-C₆-alkinyloxy, benzyloxy, C₁-C₇-alkanoyloxy, C₂-C₇-alkoxycarbonyloxy, C₁-C₆-alkylthio, C₃-C₆-alkenylthio, C₃-C₆-alkinylthio, C₃-C₈-cycloalkyloxy, C₃-C₈-cycloalkylthio, C₂-C₇-alkoxycarbonyl, aminocarbonyl, C₂-C₇-alkylaminocarbonyl, C₃-C₁₃-dialkylaminocarbonyl, carboxy, phenyl, phenoxy, phenylthio, pyridyloxy, pyridylthio, and **NR**^{**5**}**R**^{**6**}**,** wherein
**R**^{**5**} and **R**^{**6**} are selected independently of each other from
hydrogen, C₁-C₆-alkyl, C₃-C₆-alkenyl, C₃-C₆-alkinyl, benzyl and phenyl;
**R**^{**2**} is selected from
hydrogen, halogen, cyano, C₁-C₆-alkyl, trifluoromethyl, hydroxy, C₁-C₆-alkoxy, benzyloxy and C₁-C₇-alkanoyloxy; or
**R**^{**1**} and **R**^{**2**}, if adjacent, optionally form a bridge selected from -(CH₂)₄-, -(CH=CH)₂- and -CH₂O-C**R**^{**7**}**R**^{**8**}-O-, wherein
**R**^{**7**} and **R**^{**8**} are selected independently from each other from hydrogen and C₁-C₆-alkyl;
**R**^{**3**} is selected from
hydrogen, halogen, C₁-C₆-alkyl, trifluoromethyl and C₁-C₆-hydroxyalkyl;
**R**^{**4**} is selected from
hydrogen, C₁-C₆-alkyl, C₃-C₆-alkenyl, C₃-C₆-alkinyl, C₃-C₆-cycloalkyl, hydroxy, C₁-C₆-alkoxy and benzyloxy;
**k** is 0 or 1,
**A** is selected from
C₁-C₆-alkylene, optionally substituted once to threefold by C₁-C₃-alkyl, hydroxy, C₁-C₃-alkoxy, fluorine, or phenyl,
C₂-C₆-alkylene, in which a methylene unit is isosterically replaced by O, S, **NR**^{**9**}**,** CO, SO or SO₂, wherein, with the exception of CO, the isosteric substitution is not adjacent to the amide group and **R**^{**9**} is selected from hydrogen, C₁-C₆-alkyl, C₃-C₆-alkenyl, C₃-C₆-alkinyl, C₁-C₆-acyl and C₁-C₆-alkanesulfonyl,
1,2-cyclopropylene,
C₂-C₆-alkenylene, optionally substituted once to threefold by C₁-C₃-alkyl, hydroxy, C₁-C₃-alkoxy, fluorine, cyano or phenyl,
C₄-C₆-alkadienylene, optionally substituted once or twice by C₁-C₃-alkyl, fluorine, cyano or phenyl;
1,3,5-hexatrienylene, optionally substituted by C₁-C₃-alkyl, fluorine, cyano or phenyl, and
ethinylene
**D** is selected from
C₂-C₁₀-alkylene, optionally substituted once or twice by C₁-C₆-alkyl, hydroxy, or C₁-C₆-alkoxy;
C₄-C₁₀-alkenylene, optionally substituted once or twice by C₁-C₆-alkyl, hydroxy, or C₁-C₆-alkoxy;
C₄-C₁₀-alkinylene, optionally substituted once or twice by C₁-C₆-alkyl, hydroxy, or C₁-C₆-alkoxy; and
the group consisting of C₂-C₁₀-alkylene, C₄-C₁₀-alkenylene and C₄-C₁₀-alkinylene, wherein one to three methylene units are isosterically replaced by O S, **NR**^{**10**}, CO, SO or SO₂, wherein ,
**R**^{**10**} has the same meaning as **R**^{**9**}, but is selected independently thereof;
**E** represents 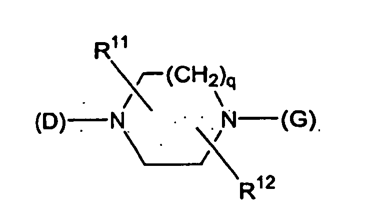 wherein
**q** is 1, 2 or 3;
**R**^{**11**} is selected from
hydrogen, C₁-C₆-alkyl, hydroxy, hydroxymethyl, carboxy, and C₂-C₇-alkoxycarbonyl, and
**R**^{**12**} is selected from
hydrogen, C₁-C₆-alkyl and an oxo group adjacent to a nitrogen atom,
or
**R**^{**11**} and **R**^{**12**} together form a C₁-C₃-alkylene bridge under formation of a bicyclic ring system;
**G** is selected from G1, G2, G3, G4 and G5, wherein
**G1** represents
―(CH₂)ᵣ―(CR¹⁴R¹⁵)ₛ―R¹³,
**r** is 0, 1, 2 or 3,
**s** is 0 or 1,
**R**^{**13**} is selected from
hydrogen, C₁-C₆-alkyl, C₃-C₆-alkenyl, C₃-C₆-alkinyl, C₃-C₈-cycloalkyl;
the group consisting of saturated and unsaturated, four to eight-membered heterocycles, which optionally contain one or two hetero-atoms selected from N, S and O;
benzyl, phenyl;
the group consisting monocyclic aromatic five to six-membered heterocycles, which optionally contain one to three hetero-atoms selected from the N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms are selected from N, S and O and the linkage occurs either over an aromatic ring or a hydrogenated ring and either directly or over a methylene group,
**R**^{**14**} has the same meaning as **R**^{**13**}, but is selected independently thereof;
**R**^{**15**} is selected from
hydrogen, hydroxy, methyl, benzyl, phenyl,
the group consisting of monocyclic aromatic five to six-membered heterocycles, which contain one to three hetero-atoms from the group N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group,
**G2** is selected from 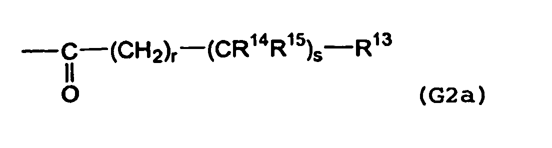 and 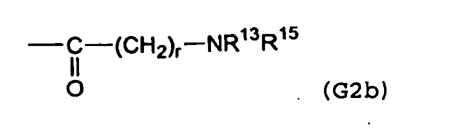 wherein **r, s** and the substituents **R**^{**13**} to **R**^{**15**} have the above meaning, or the group
―NR¹³R¹⁵
represents a nitrogen-containing heterocycle bound over the nitrogen atom which nitrogen-containing heterocycle is selected from
the group consisting of saturated and unsaturated monocyclic, four to eight-membered heterocycles, which, aside from the essential nitrogen atom, optionally contain one or two further hetero-atoms selected from N, S and O, and
the group consisting of saturated and unsaturated bi- or tricyclic, anellated or bridged heterocycles with 8 to 16 ring atoms, that aside from the essential nitrogen atom, optionally contain one or two further hetero-atoms selected from N, S and O;
**G3** represents
―SO₂―(CH₂)ᵣ―R¹³,
wherein **r** and **R**^{**13**} have the above meanings,
**G4** represents 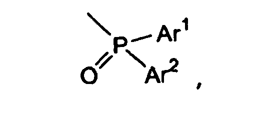 wherein
**Ar**^{**1**} and **Ar**^{**2**} are selected independently from each other from phenyl, pyridyl and naphthyl;
**G5** represents
―COR¹⁶ (G5)
wherein
**R**^{**16**} is selected from
trifluoromethyl, C₁-C₆-alkoxy, C₃-C₆-alkenyloxy, and benzyloxy,
wherein aromatic ring systems in the substituents **R**^{**1**}**, R**^{**2**}**, R**^{**4**}**, R**^{**5**}**, R**^{**6**}**, R**^{**13**}**, R**^{**14**}**, R**^{**15**}**, R**^{**16**}**, Ar**^{**1**} and **Ar**^{**2**} and/or in the ring system ―NR¹³R¹⁵ optionally are substituted independently from each other by one to three of the same or different groups selected from
halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide for two adjacent residues on the aromatic ring, and wherein
alkyl-, alkenyl- and cycloalkyl residues in the groups **G1, G2** and **G**^{**3**} optionally are substituted by one or two of the same or different groups which are selected from hydroxy, carboxy, C₂-C₇-alkoxycarbonyl, benzyloxycarbonyl, amino, mono-C₁-C₆-alkylamino and di-(C₁-C₆-alkyl-amino); their cis- and trans-isomers, E- and Z-isomers, enantiomers, diastereomers and other isomers as well as their racemic or non-racemic mixtures and the corresponding endo- and exo-isomers for the case that the ring system E is bicyclic;
their tautomeres; as well as
their acid addition salts including their hydrates and solvates.

22. The use according to claim 21, wherein
**R**^{**1**} is selected from
hydrogen, halogen, cyano, C₁-C₆-alkyl, trifluoromethyl, C₃-C₈-cycloalkyl, C₁-C₆-hydroxyalkyl, hydroxy, C₁-C₄-alkoxy, benzyloxy, C₁-C₄-alkylthio, C₁-C₅-alkanoyloxy, C₁-C₄-alkylthio, C₂-C₅-alkokycarbonyl, aminocarbonyl, C₂-C₅-alkylaminocarbonyl, C₃-C₉-dialkylaminocarbonyl, carboxy, phenyl, phenoxy, phenylthio, pyridyloxy, and **NR**^{**5**}**R**^{**6**}**,** wherein
**R**^{**5**} and **R**^{**6**} are selected independently from each other from hydrogen and C₁-C₆-alkyl;
**R**^{**2**} is selected from
hydrogen, halogen, cyano, C₁-C₆-alkyl, trifluoromethyl, hydroxy, and C₁-C₄-alkoxy;
**R**^{**3**} is selected from
hydrogen, halogen and C₁-C₆-alkyl;
**R**^{**4**} is selected from
hydrogen, C₁-C₆-alkyl, C₃-C₆-alkenyl, C₃-C₆-cycloalkyl, hydroxy, C₁-C₆-alkoxy and benzyloxy;
**k** is 0 or 1,
**A** is selected from
C₁-C₆-alkylene, optionally substituted once to threefold by C₁-C₃-alkyl, hydroxy, fluorine, or phenyl,
C₂-C₆-alkylene, in which a methylene unit is isosterically replaced by O, S, **NR**^{**9**}**,** CO, SO or SO₂, wherein, with the exception of CO, the isosteric substitution is not adjacent to the amide group and the residue **R**^{**9**} is selected from hydrogen, C₁-C₆-alkyl, C₁-C₆-acyl and methane sulfonyl;
1,2-cyclopropylene,
C₂-C₆-alkenylene, optionally substituted once to threefold by C₁-C₃-alkyl, hydroxy, fluorine, cyano or phenyl,
C₄-C₆-alkadienylene, optionally substituted once or twice by C₁-C₃-alkyl, fluorine, cyano or phenyl;
1,3,5-hexatrienylene, optionally substituted by C₁-C₃-alkyl, fluorine, or cyano, and
ethinylene
**D** is selected from
C₂-C₁₀-alkylene, optionally substituted once or twice by C₁-C₃-alkyl or hydroxy;
C₄-C₁₀-alkenylene, optionally substituted once or twice by C₁-C₃-alkyl or hydroxy;
C₄-C₁₀-alkinylene, optionally substituted once or twice by C₁-C₃-alkyl or hydroxy; and
the group consisting of C₂-C₁₀-alkylene, C₄-C₁₀-alkenylene and C₄-C₁₀-alkinylene, wherein one to three methylene units are isosterically replaced by O, S, **NR**^{**10**}**,** CO, SO or SO₂, wherein
**R**^{**10**} has the same meaning as **R**^{**9**}**,** but is selected independently thereof;
**E** represents 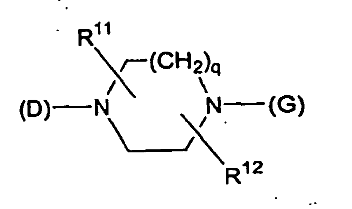 wherein
**q** is 1, 2 or 3;
**R**^{**11**} is selected from
hydrogen, C₁-C₃-alkyl, hydroxy, hydroxymethyl, carboxy, and C₂-C₇-alkoxycarbonyl, and
**R**^{**12**} is selected from hydrogen and an oxo group adjacent to a nitrogen atom or
**R**^{**11**} and **R**^{**12**}, together, form a C₁-C₃-alkylene bridge under formation of a bicyclic ring system;
**G** is selected from G1, G2, G3, G4 and G5, wherein
**G1** represents
―(CH₂)ᵣ―(CR¹⁴R¹⁵)ₛ―R¹³,
**r** is 0, 1 or 2,
**s** is 0 or 1,
**R**^{**13**} is selected from
hydrogen, C₁-C₆-alkyl, C₃-C₆-alkenyl, C₃-C₆-alkinyl, C₃-C₈-cycloalkyl; benzyl, phenyl;
the group consisting of monocyclic aromatic five to six-membered heterocycles, which contain one to three hetero-atoms selected from N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group,
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group,
**R**^{**14**} has the same meaning as **R**^{**13**}**,** but is selected independently thereof;
**R**^{**15**} is selected from
hydrogen, hydroxy, methyl, benzyl, phenyl;
the group consisting of monocyclic aromatic five to six-membered heterocycles, which contain one to three hetero-atoms selected from N, S and O and are either bound directly or over a methylene group,
the group consisting of anellated bi- and tricyclic aromatic or partially hydrogenated carbocyclic ring systems with 8 to 16 ring atoms and at least one aromatic ring, wherein the linkage occurs either over an aromatic or a hydrogenated ring and either directly or over a methylene group, and
the group consisting of anellated bi- and tricyclic aromatic or partially hydrogenated heterocyclic ring systems with 8 to 16 ring atoms and at least one aromatic ring, wherein one to three ring atoms optionally are selected from N, S and O and the linkage occurs either over an aromatic ring or a hydrogenated ring and either directly or over a methylene group;
**G2** is selected from 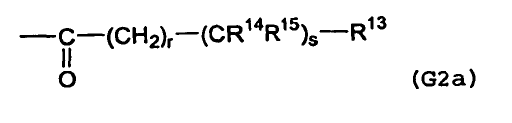 and 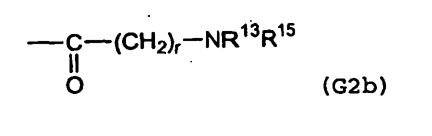 wherein **r, s** and the substituents **R**^{**13**} to **R**^{**15**} have the above meaning, or the group
―NR¹³R¹⁵
is a nitrogen-containing heterocycle bound over the nitrogen atom which nitrogen-containing heterocycle is selected from
the group consisting of saturated and unsaturated monocyclic, four to eight-membered heterocycles, which, aside from the essential nitrogen atom, optionally contain one or two further hetero-atoms selected from N, S and O, and
the group consisting of saturated and unsaturated bi- or tricyclic, anellated or bridged heterocycles with 8 to 16 ring atoms, that aside from the essential nitrogen atom, optionally contain one or two further hetero-atoms selected from N, S and O;
**G3** represents
―SO₂―(CH₂)ᵣ―R¹³,
wherein **r** and **R**^{**13**} have the above meaning,
**G4** represents 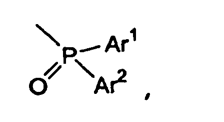 wherein
**Ar**^{**1**} and **Ar**^{**2**} are selected independently from each other from phenyl, pyridyl and naphthyl;
**G5** represents
―COR¹⁶,
wherein
**R**^{**16**} is selected from
trifluoromethyl, C₁-C₆-alkoxy, C₃-C₆-alkenyloxy, and benzyloxy,
wherein aromatic ring systems in the substituents **R**^{**1**}**, R**^{**2**}**, R**^{**4**}**, R**^{**5**}**, R**^{**6**}**, R**^{**13**}**, R**^{**14**}**, R**^{**15**}**, R**^{**16**}**, Ar**^{**1**} and **Ar**^{**2**} and/or in the ring system ―NR¹³R¹⁵ optionally are substituted independently from each other by one to three of the same or different groups selected from
halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide in the case of two adjacent residues on the aromatic ring,
wherein alkyl, alkenyl and cycloalkyl residues in the groups **G**^{**1**}**, G**^{**2**} and **G**^{**3**} optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl-amino).

23. The use according to claim 21 or 22, wherein
**R**^{**1**} is selected from
hydrogen, halogen, cyano, methyl, ethyl, trifluoromethyl, hydroxy, C₁-C₄-alkoxy, benzyloxy, C₁-C₅-alkanoyloxy, methylthio, ethylthio, methoxycarbonyl, tert-butoxycarbonyl aminocarbonyl, carboxy, phenoxy, and phenylthio,
**R**^{**2**} is selected from
hydrogen, halogen, trifluoromethyl, and hydroxy,
**R**^{**3**} is selected from
hydrogen, and halogen,
**R**^{**4**} is selected from
hydrogen, C₁-C₃-alkyl, allyl, hydroxy, and C₁-C₃-alkoxy;
**k** is 0 or 1,
**A** is selected from
C₁-C₆-alkylene, optionally substituted once or twice by C₁-C₃-alkyl, hydroxy or fluorine;
C₂-C₆-alkylene, in which a methylene unit is isosterically replaced by O S, **NR**^{**9**}, CO, SO or SO₂, wherein, with the exception of CO, the isosteric substitution is not adjacent to the amide group,
C₂-C₆-alkylene, optionally substituted once or twice by C₁-C₃ alkyl, hydroxy and/or fluorine;
C₄-C₆-alkadienylene, optionally substituted by C₁-C₃-alkyl or one or two fluorine atoms; and
1,3,5-hexatrienylene, optionally substituted by fluorine;
**D** is selected from
C₂-C₈-alkylene, optionally substituted once or twice by methyl or hydroxy;
C₄-C₈-alkenylene, optionally substituted once or twice by methyl or hydroxy;
C₄-C₈-alkinylene, optionally substituted once or twice by methyl or hydroxy; and
the group consisting of C₂-C₈-alkylene, C₄-C₈-alkenylene and C₄-C₈-alkinylene, wherein one to three methylene units are each isosterically replaced by O, S, NH, N(CH₃) N(COCH₃), N(SO₂CH₃), CO, SO or SO₂,
**E** represents 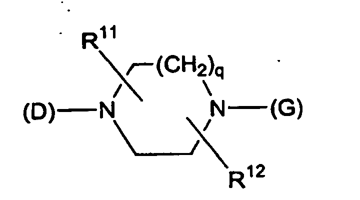 wherein
**q** is 1 or 2;
**R**^{**11**} is selected from
hydrogen, C₁-C₃-alkyl, hydroxymethyl, and carboxy, and
**R**^{**12**} is selected from
hydrogen and an oxo group adjacent to a nitrogen atom
**G** is selected from G1, G2, G3, G4 and G5, wherein
**G1** represents
―(CH₂)ᵣ―(CR¹⁴R¹⁵)ₛ―R¹³
**r** is 0, 1 or 2,
**s** is 0 or 1;
**R**^{**13**} is selected from
hydrogen, C₁-C₆-alkyl, C₃-C₈-cycloalkyl, benzyl, phenyl,
the group consisting of benzocyclobutyl, indanyl, indenyl, oxoindanyl, naphthyl, dihydronaphthyl, tetrahydronaphthyl, oxotetrahydronaphthyl, biphenylenyl, fluorenyl, oxofluorenyl, anthryl, dihydroanthryl, oxodihydroanthryl, dioxodihydroanthryl, phenanthryl, dihydrophenanthryl, oxodihydrophenanthryl, dibenzocycloheptenyl, oxodibenzocycloheptenyl, dihydrodibenzocycloheptenyl, oxodihydrodibenzocycloheptenyl, dihydrodibenzocyclooctenyl, tetrahydrodibenzocyclooctenyl and oxotetrahydrodibenzocyclooctenyl, bound directly or over a methylene group; and
the group consisting of furyl, thienyl, pyrrolyl, oxazolyl, isoxazolyl, thiazolyl, iso-thiazolyl, pyrazolyl, imidazolyl, oxadiazolyl, thiadiazolyl, triazolyl, pyridyl, pyrazinyl, pyridazinyl, pyrimidinyl, triazinyl, imidazothiazolyl, benzofuryl, dihydrobenzofuryl, benzothienyl, dihydrobenzothienyl, indolyl, indolinyl, isoindolinyl, oxoindolinyl, dioxoindolinyl, benzoxazolyl, oxobenzooxazolinyl, benzoisooxazolyl, oxobenzoisooxazolinyl, benzothiazolyl, oxobenzthiazolinyl, benzoisothiazolyl, oxobenzoisothiazolinyl, benzoimidazolyl, oxobenzoimidazolinyl, indazolyl, oxoindazolinyl, benzofurazanyl, benzothiadiazolyl, benzotriazolyl, oxazolopyridyl, oxodihydrooxazolopyridyl, thiazolopyridyl, oxodihydrothiazolopyridyl, isothiazolopyridyl, imidazopyridyl, oxodihydroimidazopyridyl, pyrazolopyridyl, oxodihydropyrazolopyridyl, thienopyrimidinyl, chromanyl, chromanonyl, benzopyranyl, chromonyl, quinoloyl, isoquinoloyl, dihydroquinolyl, oxodihydroquinolinyl, tetrahydroquinolyl, oxotetrahydroquinolinyl, benzodioxanyl, quinoxalinyl, quinazolinyl, naphthyridinyl, carbazolyl, tetrahydrocarbazolyl, oxotetrahydrocarbazolyl, pyridoindolyl, acridinyl, oxodihydroacridinyl, phenanthridinyl, dihydrophenanthridinyl, oxodihydrophenanthridinyl, dibenzoisoquinolinyl, dihydrodibenzoisoquinolinyl, oxodihydrodibenzoisoquinolinyl, phenothiazinyl, dihydrodibenzooxepinyl, oxodihydrodibenzooxepinyl, benzocycloheptathienyl, oxobenzocycloheptathienyl, dihydrothienobenzothiepinyl, oxodihydrothienobenzothiepinyl, dihydrodibenzothiepinyl, oxodihydrodibenzothiepinyl, octahydrodibenzothiepinyl, dibenzoazepinyl, dihydrodibenzoazepinyl, oxodihydrodibenzoazepinyl, octahydrodibenzoazepinyl, benzocycloheptapyridyl, oxobenzocycloheptapyridyl, pyridobenzoazepinyl, dihydropyridobenzoazepinyl, oxodihydropyridobenzoazepinyl, dihydropyridobenzodiazepinyl, dihydrodibenzooxazepinyl, dihydropyridobenzooxepinyl, dihydropyridobenzooxazepinyl, oxodihydropyridobenzooxazepinyl, dihydrodibenzothiazepinyl, oxodihydrodibenzothiazepinyl, dihydropyridobenzothiazepinyl and oxodihydropyridobenzothiazepinyl, bound directly or over a methylene group;
**R**^{**14**} has the same meaning as **R**^{**13**} but is selected independently thereof;
**R**^{**15**} is selected from
hydroxy, methyl, benzyl, phenyl, indanyl, indenyl, naphthyl, dihydronaphthyl, tetrahydronaphthyl, furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, triazinyl, benzofuryl, benzothienyl, indolyl, indolinyl, benzooxazolyl, benzothiazolyl, benzoimidazolyl, chromanyl, quinolyl and tetrahydroquinolyl, bound directly or over a methylene group;
**G2** is selected from 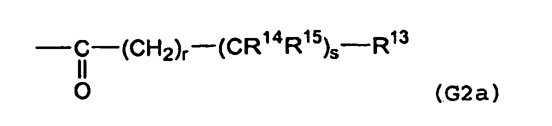 and 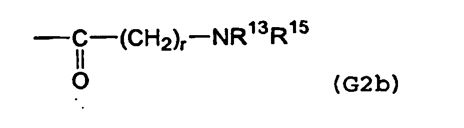 wherein **r, s** and the substituents **R**^{**13**} to **R**^{**15**} have the above meaning, or the group
―NR¹³R¹⁵
represents azetidine, pyrrolidine, piperidine, (1H)-tetrahydropyridine, hexahydroazepine, (1H)-tetrahydroazepine, octahydroazocine, pyrazolidine, piperazine, hexahydrodiazepine, morpholine, hexahydrooxazepine, thiomorpholine, thiomorpholin-1,1-dioxide, of 5-aza-bicyclo-[2.1.1]hexane, 2-aza-bicyclo[2.2.1]heptane, 7-aza-bicyclo-[2.2.1]heptane, 2,5-diaza-bicyclo[2.2.1]heptane, 2-aza-bicyclo[2.2.2]octane, 8-aza-bicyclo[3.2.1]octane, 2,5-diaza-bicyclo[2.2.2.]octane, 9-aza-bicyclo[3.3.1]nonane, indoline, isoindoline, (1H)-dihydroquinoline, (1H)-tetrahydroquinolin, (2H)-tetrahydroisoquinoline, (1H)-tetrahydroquinoxaline, (4H)-dihydrobenzooxazine, (4H)-dihydrobenzothiazine, (1H)-tetrahydrobenzo[b]azepine, (1H)-tetrahydrobenzo[c]azepine, (1H)-tetrahydrobenzo[d]azepine, (5H)-tetrahydrobenzo[b]ox-azepine, (5H)-tetrahydrobenzo[b]thiazepine, 1,2,3,4-tetra-hydro-9H-pyrido[3,4-b]indole, (10H)-dihydroacridine, (10H)-dihydrophenanthridine, 1,2,3,4-tetrahydroacridanone, (10H)-phenoxazine, (10H)-phenothiazine, (5H)-dibenzoazepine, (5H)-dihydrodibenzoazepine, (5H)-octahydrodibenzoazepine, dihydrobenzo[d,e]isoquinoline, (5H)-dihydrodibenzodiazepine, (5H)-benzo[b]pyrido[f]azepine, (5H)-Dihydrobenzo[b]pyri-do[f]azepine, (11H)-Dihydrodibenzo[b,e]oxazepine, (11H)-dihydrodibenzo[b,e]thiazepine, (10H)-dihydrodibenzo[b,f]-oxazepine, (10H)-dihydrodibenzo[b,f]thiazepine, (5H)-tetra-hydrodibenzoazocine, (11H)-dihydrobenzo[e]pyrido[b]-1,4-diazepin-6-one or (11H)-Dihydrobenzo[b]pyrido[e]-1,4-diazepin-5-one,
**G3** represents
―SO₂―(CH₂)ᵣ―R¹³,
wherein **r** and **R**^{**13**} have the above meaning,
**G4** represents 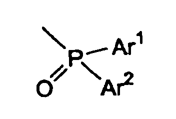 wherein
**Ar**^{**1**} and **Ar**^{**2**} are selected independently from each other from phenyl, pyridyl and naphthyl;
**G5** represents
―COR¹⁶,
wherein
**R**^{**16**} is selected from
trifluoromethyl, C₁-C₆-alkoxy, C₃-C₆-alkenyloxy, and benzyloxy,
wherein aromatic ring systems in the substituents optionally are substituted independently from each other by one to three of the same or different groups selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and methylene dioxide in the case of two adjacent residues on the aromatic ring, and
wherein alkyl-, alkenyl- and cycloalkyl residues in the groups **G1, G2** and **G3** optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl-amino).

24. The use according to any one of claims 21 to 23, wherein
**R**^{**1**} is selected from
hydrogen, fluorine, chlorine, bromine, methyl, ethyl, trifluoromethyl, hydroxy, C₁-C₄-alkoxy, methylthio, ethylthio, carboxy and phenoxy;
**R**^{**2**} is selected from hydrogen, chlorine and methyl;
**R**^{**3**} is hydrogen;
**R**^{**4**} is selected from
hydrogen, C₁-C₃-alkyl and hydroxy,
**k** is 0
**A** is selected from
C₂-C₆-alkylene, which is optionally substituted once or twice by hydroxy or fluorine;
C₂-C₆-alkylene, wherein a methylene unit is isosterically replaced by O, S or CO, wherein, with the exception of CO, the isosteric substitution is not adjacent to the amide group;
C₂-C₆-alkenylene which is optionally substituted by C₁-C₃-alkyl and/or fluorine; and
C₄-C₆-alkadienylene;
**D** is selected from
C₂-C₈-alkylene which is optionally substituted by methyl or hydroxy;
C₄-C₈-alkenylene, which is optionally substituted by hydroxy;
C₄-C₈-alkinylene, which is optionally substituted by hydroxy; and
the group consisting of C₂-C₈-alkylene, C₄-C₈-alkenylene, and C₄-C₈-alkinylene wherein a methylene unit is isosterically replaced by O, NH, N(CH₃), CO or SO₂ or an ethylene group is isosterically replaced by a group NH-CO and/or CO-NH or a propylene group is isosterically replaced by a group NH-CO-O and/or O-CO-NH;
**E** is selected from piperazine and hexahydro-1,4-diazepine, wherein the ring optionally is substituted by one or two methylene groups and/or by an oxo group adjacent to a nitrogen atom;
**G** is selected from hydrogen, C₃-C₈-cycloalkyl, methoxycarbonyl, tert-butoxycarbonyl, benzyloxycarbonyl, trifluoroacetyl, diphenylphosphinoyl,
―(CH₂)ᵣ―(CR¹⁴R¹⁵)ₛ―R¹³, 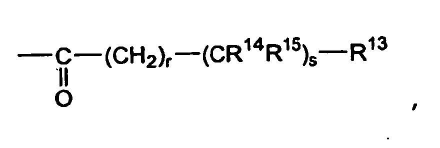 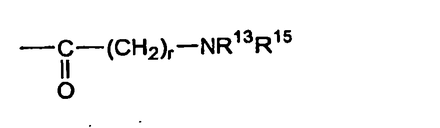
and
―SO₂―(CH₂)ᵣ―R¹³
wherein
**r** is 0 or 1
**s** is 0 or 1,
**R**^{**13**} is selected from hydrogen, methyl, benzyl, phenyl,
the group consisting of indanyl, indenyl, oxoindanyl, naphthyl, tetrahydronaphthyl, fluorenyl, oxofluorenyl, anthryl, dihydroanthryl, oxodihydroanthryl, dioxodihydroanthryl, phenanthryl, dihydrophenanthryl, oxydihydrophenanthryl, dibenzocycloheptenyl, dihydrodibenzocycloheptenyl, and oxodihydrodibenzocycloheptyl, bound directly or over a methylene group, and
the group consisting of furyl, thienyl, oxazolyl, isooxazolyl, thiazolyl, imidazolyl, oxadiazolyl, pyridyl, pyrazinyl, pyrimidinyl, imidazothiazolyl, benzofuryl, benzothienyl, indolyl, indolinyl, oxoindolinyl, dioxoindolinyl, benzoxazolyl, oxobenzooxazolinyl, benzothiazolyl, oxobenzthiazolinyl, benzimidazolyl, oxobenzimidazolinyl, indazolyl, benzofurazanyl, benzotriazolyl, oxazolopyridyl, oxodihydrooxazolopyridyl, thiazolopyridyl, oxodihydrothiazolopyridyl, imidazopyridyl, oxodihydroimidazopyridyl, chromanyl, chromanonyl, benzopyranyl, chromonyl, quinolyl, isoquinolyl, oxodihydroquinolinyl, tetrahydroquinolyl, oxotetrahydroquinolinyl, benzodioxanyl, quinazolinyl, carbazolyl, acridinyl, dihydroacridinyl, oxodihydroacridinyl, dihydrophenanthridinyl dihydrobenzoisoquinolinyl, phenothiazinyl, dihydrodibenzooxepinyl, benzocycloheptathienyl, dihydrothienobenzothiepinyl, dihydrodibenzothiepinyl, oxodihydrodibenzothiepinyl, dihydrodibenzoazepinyl, oxodihydrodibenzoazepinyl, octahydrodibenzoazepinyl, benzocycloheptapyridyl, dihydropyridobenzodiazepinyl, and dihydrodibenzothiazepinyl, bound directly or over a methylene group,
**R**^{**14**} is selected from hydrogen, methyl, benzyl, and phenyl,
**R**^{**15**} is selected from
hydrogen, hydroxy, methyl, benzyl, phenyl; and
the group consisting of naphthyl, tetrahydronaphthyl, furyl, thienyl, oxazolyl, thiazolyl, imidazolyl, pyridyl, benzofuryl, benzothienyl, indolyl, indolinyl, benzoxazolyl, benzothiazolyl, benzimidazolyl, chromanyl, quinolyl and tetrahydroquinolyl, bound directly or over a methylene group;
wherein the group
―NR¹³R¹⁵
represents a nitrogen-containing heterocycle bound over the nitrogen atom which nitrogen-containing heterocycle is selected from
pyrrolidine, piperidine, hexahydroazepine, piperazine, hexahydrodiazepine, morpholine, hexahydroxazepine, thiomorpholine, 7-aza-bicyclo[2,2.1]heptane, 2,5-diaza-bicyclo[2.2.1]heptane, indoline, isoindoline, (1H)-dihydroquinoline, (1H)-tetrahydroquinoline, (2H)-tetrahydroisoquinoline, (4H)-dihydrobenzoxazine, (4H)-dihydrobenzothiazine, (1H)-tetrahydrobenzo[b]azepine, (1H)-tetrahydrobenzo[d]azepine, (5H)-tetrahydrobenzo[b]oxazepine, (5H) -tetrahydrobenzo [b] thiazepine, (10H)-dihydroacridine, 1,2,3,4-tetrahydroacridanone, (10H)-dihydrophenanthridine, (1H)-dihydrobenzo-[d,e]isoquinoline, (10H)-phenothiazine, (5H)-dibenzo[b,f]azepine, (5H)-dihydrodibenzo[b,f]azepine, (5H)-dihydrodibenzo[c,e]azepine, (5H)-dihydrodibenzo-diazepine, (11H)-dihydrodibenzo[b,e]oxazepine (11H)-dihydrodibenzo[b,e]thiazepine, (5H)-dihydrobenzo[b]pyrido[3,2-f]azepine and (11H)-6-oxodihydrobenzo[e]pyrido[3,2-b][1,4]diazepine,
wherein aromatic ring systems in the substituents optionally are substituted, independently of each other, by one to three of the same or different groups selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carbocyalkenyl, C₂-C₇-alkoxycarbonyl, benzyloxycarbonyl, nitro, amino, mono-C₁-C₆-alkylamino, di-(C₁-C₆-alkyl)-amino, and in the case of two adjacent residues on the aromatic ring, methylenedioxy, and
wherein alkyl, alkenyl and cycloalkyl residues in the group **G** optionally are substituted by one or two of the same or different groups which are selected from hydroxy, carboxy, C₂-C₇-alkoxycarbonyl, benzyloxycarbonyl, amino, mono-C₁-C₆-alkylamino and di-(C₁-C₆-alkyl) amino.

25. The use according to any one of claims 21 to 24, wherein
**R**^{**1**} is selected from
hydrogen, fluorine, methyl, trifluoromethyl and ethylthio;
**R**^{**2**}, **R**^{**3**} and **R**^{**4**} are each hydrogen;
**k** is 0,
**A** is selected from
the group consisting of ethylene, propylene and butylene, which are each optionally substituted by hydroxy or one or two fluorine atoms;
OCH₂, SCH_{2;}
ethenylene, and 1,3-butadienylene;
**D** is selected from
C₂-C₆-alkylene which is optionally substituted by hydroxy;
C₄-C₆ alkenylene;
C₄-C₆ alkinylene; and
the group consisting of C₂-C₆ alkylene, C₄-C₆ alkenylene and C₄-C₆ alkinylene, wherein one or two methylene units are isosterically replaced by O, NH, CO or SO₂;
**E** is piperazine or hexahydro-1,4-diazepine;
**G** is selected from
phenyl, benzyl, phenethyl, diphenylmethyl, naphthyl, tetrahydronaphtyl, naphthylmethyl, fluorenyl fluorenylmethyl, anthrylmethyl, dihydrodibenzocycloheptenyl, furylmethyl, thienylmethyl, thiazolylmethyl, pyridylmethyl, benzothienylmethyl, quinolylmethyl, phenylthienylmethyl, phenylpyridylmethyl, benzocycloheptapyridinyl, dihydrobenzocycloheptapyridinyl, dihydrodibenzooxepinyl, dihydrodibenzothiepinyl, dihydrodibenzoazepinyl, dihdrobenzopyridodiazepinyl, formyl, acetyl, pivaloyl, phenylacetyl, diphenylacetyl, diphenylpropionyl, naphthylacetyl, benzoyl, naphthoyl, oxofluorenylcarbonyl, oxodihydroanthrylcarbonyl, dioxodihydroanthrylcarbonyl, furoyl, pyridylacetyl, pyridylcarbonyl, chromonylcarbonyl, quinolylcarbonyl, phenylylaminocarbonyl, naphthylaminocarbonyl, tetrahydronaphthylaminocarbonyl, dibenzylaminocarbonyl, benzylphenylaminocarbonyl, diphenylaminocarbonyl, indolinyl-N-carbonyl, isoindolinyl-N-carbonyl, tetrahydroquinolinyl-N-carbonyl, carbazolyl-N-carbonyl, tetrahydrobenzoazepinyl-N-carbonyl, dihydrodibenzoazepinyl-N-carbonyl, dihydrobenzopyridoazepinyl-N-carbonyl, oxodihydrobenzopyridoazepinyl-N-carbonyl, methanesulfonyl, toluenesulfonyl, naphthylsulfonyl, quinolinsulfonyl and diphenylphosphinoyl,
wherein aromatic ring systems optionally are substituted independently of each other by one to three of the same or different groups which are selected from halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy which is entirely or partially substituted by fluorine; benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, di-(C₁-C₆-alkyl)-amino, and in the case of two adjacent residues in the aromatic ring methylenedioxy, and wherein
alkyl, alkenyl and cycloalkyl residues in the group **G** optionally are substituted by one or two of the same or different groups which are selected from
hydroxy, carboxy, C₂-C₇-alkoxycarbonyl, benzyloxycarbonyl, amino, mono-C₁-C₆-alkylamino and di-(C₁-C₆-alkyl)-amino.

26. The use according to any one of claims 21 to 25, wherein the compound(s) of formula (I) is/are selected from the group consisting of:
N-[4-(4-diphenylmethylpiperazin-1-yl)-3-hydroxybutyl]-3-pyridin-3-yl-acrylamide;
N-[3-(4-diphenylmethylpiperazin-1-yl)-propoxy]-3-pyridin-3-yl-acrylamide;
N-[4-(4-diphenylmethylpiperazin-1-yl)-4-oxo-butyl]-3-pyridin-3-yl-acrylamide;
N-[3-(4-diphenylmethylpiperazin-1-sulfonyl)-propyl]-3-pyridin-3-yl-acrylamide;
N-{2-[2-(4-diphenylmethylpiperazin-1-yl)-ethoxy]-ethyl}-3-pyridin-3-yl-acrylamide;
N-(4-{4-[bis-(4-fluorophenyl)-methyl]-piperazin-1-yl}-but-2-in-yl)-3-pyridin-3-yl-acrylamide;
N-{4-[4-(4-carboxyphenyl-phenylmethyl)-piperazin-1-yl]-butyl}-3-pyridin-3-yl-acrylamide;
N-(4-{4-[(4-aminophenyl)-phenylmethyl]-piperazin-1-yl}-butyl)-3-pyridin-3-yl-acrylamide;
N-{4-[4-(9H-fluoren-9-yl)-piperazin-1-yl]-butyl}-2-(pyridin-3-yloxy)-acetamide;
N-{5-[4-(9H-fluoren-9-yl)-piperazin-1-yl]-pentyl}-3-pyridin-3-yl-acrylamide;
N-{6-[4-(9H-fluoren-9-yl)-piperazin-1-yl]-hexyl}-3-pyridin-3-yl-acrylamide;
3-pyridin-3-yl-N-{4-[4-(1,2,3,4-tetrahydronaphthalen-1-yl)-piperazin-1-yl]-butyl}-acrylamide;
3-pyridin-3-yl-N-{4-[4-(5,6,7,8-tetrahydronaphthalen-1-yl)-piperazin-1-yl]-butyl}-acrylamide;
N-{4-[4-(naphthalen-1-yl)-piperazin-1-yl]-butyl}-3-pyridin-3-yl-acrylamide;
N-[4-(4-biphenyl-2-yl-piperazin-1-yl)-butyl]-3-pyridin-3-yl-propionamide;
N-[5-(4-biphenyl-2-yl-piperazin-1-yl)-pentyl]-3-pyridin-3-yl-acrylamide;
N-[6-(4-biphenyl-2-yl-piperazin-1-yl)-hexyl]-3-pyridin-3-yl-acrylamide;
N-[4-(4-biphenyl-2-yl-piperazin-1-yl)-butyl]-2-(pyridin-3-yloxy)-acetamide;
N-[4-(4-biphenyl-2-yl-piperazin-1-yl)-butyl]-5-(pyridin-3-yl)-penta-2,4-dienoic acid amide;
N-{4-[4-(10,11-dihydro-5H-dibenzo[a,d]cyclohepten-5-yl)-piperazin-1-yl]-butyl}-3-pyridin-3-yl-propionamide;
N-{5-[4-(10,11-dihydro-5H-dibenzo[a,d]cyclohepten-5-yl)-piperazin-1-yl]-pentyl}-3-pyridin-3-yl-acrylamide;
N-{6-[4-(10,11-dihydro-5H-dibenzo[a,d]cyclohepten-5-yl)-piperazin-1-yl]-hexyl}-3-pyridin-3-yl-acrylamide;
N-{4-[4-(10,11-dihydro-5H-dibenzo[a,d]cyclohepten-5-yl)-piperazin-1-yl]-butyl}-5-(pyridin-3-yl)-penta-2,4-dienoic acid amide;
N-{4-[4-(6,11-dihydro-dibenzo[b,e]oxepin-11-yl)-piperazin-1-yl]-butyl-3-pyridin-3-yl-propionamide;
N-{2-[4- (6,11-dihydrodibenzo[b,e]thiepin-11-yl)-piperazin-1-yl]-ethyl}-3-pyridin-3-yl-acrylamide;
N-[4-(4-diphenylacetyl-piperazin-1-yl)-butyl]-3-pyridin-3-yl-acrylamide;
N-[4-(4-benzoylpiperazin-1-yl)-butyl]-3-pyridin-3-yl-acrylamide;
N-{4-[4-(2-aminobenzoyl)-piperazin-1-yl]-butyl}-3-pyridin-3-yl-acrylamide;
N-{4-[4-(4-carboxybenzoyl)-piperazin-1-yl]-butyl}-3-pyridin-3-yl-acrylamide;
N-{4-[4-(biphenyl-2-carbonyl)-piperazin-1-yl]-butyl}-3-pyridin-3-yl-acrylamide;
N-{4-[4- (9-oxo-9H-fluoren-4-carbonyl)-piperazin-1-yl]-butyl}-3-pyridin-3-yl-acrylamide;
N-{4-[4-(furan-2-carbonyl)-piperazin-1-yl]-butyl}-3-pyridin-3-yl-acrylamide;
N-{4-[4-(naphthalen-1-yl-aminocarbonyl)-piperazin-1-yl]-butyl}-3-pyridin-3-yl-propionamide;
N-{4-[4-(diphenylaminocarbonyl)-piperazin-1-yl]-butyl}-3-pyridin-3-yl-acrylamide;
N-{4-[4-(naphthalen-2-sulfonyl)-piperazin-1-yl]-butyl}-3-pyridin-3-yl-acrylamide;
N-[4-(4-diphenylphosphinoyl-piperazin-1-yl)-butyl]-3-pyridin-3-yl-acrylamide;
N-[4-(4-biphenyl-2-yl-piperazin-1-yl)-butyl]-3-pyridin-3-yl-acrylamide;
N-{4-[4-(9H-fluoren-9-yl)-piperazin-1-yl]-butyl}-3-pyridin-3-yl-acrylamide; and
N-{4-[4-(10,11-dihydro-5H-dibenzo[a,d]cyclohepten-5-yl)-piperazin-1-yl]-butyl}-3-pyridin-3-yl-acrylamide.

27. Use of one or more compounds according to any one of claims 1 to 7 for the manufacture of a pharmaceutical composition for the immunosuppression in the human or animal body, optionally in combination with a further cytostatic agent or immunosuppressive agent and/or further pharmaceutical compositions suitable for this indication.

## Patentansprüche

1. Pyridylalkan-, Pyridylalken- oder Pyridylalkincarboxamid gemäss Formel (I): 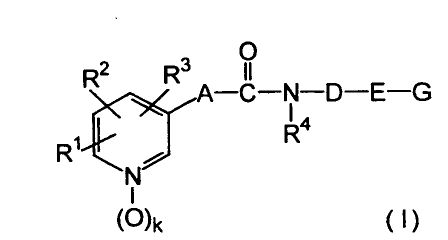 worin:
R¹ ausgewählt ist aus
Wasserstoff, Halogen, Cyano, C₁₋₆-Alkyl, C₃₋₆-Alkenyl, C₂₋₆-Alkinyl, Trifluormethyl, C₃₋₈-Cycloalkyl, C₁₋₆-Hydroxyalkyl, Hydroxy, C₁₋₆-Alkoxy, C₃₋₆-Alkenyloxy, C₃₋₆-Alkinyloxy, Benzyloxy, C₁₋₇-Alkanoyloxy, C₂₋₇-Alkoxycarbonyloxy, C₁₋₆-Alkylthio, C₃₋₆-Alkenylthio, C₃₋₆-Alkinylthio, C₃₋₈-Cycloalkyloxy, C₃₋₈-Cycloalkylthio, C₂₋₇-Alkoxycarbonyl, Aminocarbonyl, C₂₋₇-Alkylaminocarbonyl, C₃₋₁₃-Dialkylaminocarbonyl, Carboxy, Phenyl, Phenoxy, Phenylthio, Pyridyloxy, Pyridylthio und NR⁵R⁶, worin
R⁵ und R⁶ unabhängig voneinander ausgewählt sind aus Wasserstoff, C₁₋₆-Alkyl, C₃₋₆-Alkenyl, C₃₋₆-Alkinyl, Benzyl und Phenyl;
R² ausgewählt ist aus
Wasserstoff, Halogen, Cyano, C₁₋₆-Alkyl, Trifluormethyl, Hydroxy, C₁₋₆-Alkoxy, Benzyloxy und C₁₋₇-Alkanoyloxy; oder
R¹ und R², falls benachbart, bilden gegebenenfalls eine Brücke, ausgewählt aus -(CH₂)₄-, -(CH=CH)₂-und -CH₂O-CR⁷R⁸-O-, worin
R⁷ und R⁸ unabhängig voneinander ausgewählt sind aus Wasserstoff oder C₁₋₆-Alkyl;
R³ ausgewählt ist aus
Wasserstoff, Halogen, C₁₋₆-Alkyl, Trifluormethyl und C₁₋₆-Hydroxyalkyl;
R⁴ ausgewählt ist aus
Wasserstoff, C₁₋₆-Alkyl, C₃₋₆-Alkenyl, C₃₋₆-Alkinyl, C₃₋₆-Cycloalkyl, Hydroxy, C₁₋₆-Alkoxy und Benzyloxy;
k 0 oder 1 ist;
A ausgewählt ist aus
C₁₋₆-Alkylen, gegebenenfalls ein- bis dreimal substituiert durch C₁₋₃-Alkyl, Hydroxy, C₁₋₃-Alkoxy, Fluor oder Phenyl,
C₂₋₆-Alkylen, worin eine Methyleneinheit isosterisch ersetzt ist durch O, S, NR⁹, CO, SO oder SO₂, worin, mit Ausnahme von CO, die isosterische Substitution nicht benachbart zur Amidgruppe ist und R⁹ ausgewählt ist aus Wasserstoff, C₁₋₆-Alkyl, C₃₋₆-Alkenyl, C₃₋₆-Alkinyl, C₁₋₆-Acyl und C₁₋₆-Alkansulfonyl,
1,2-Cyclopropylen,
C₂₋₆-Alkenylen, gegebenenfalls ein- bis dreimal substituiert durch C₁₋₃-Alkyl, Hydroxy, C₁₋₃-Alkoxy, Fluor, Cyano oder Phenyl,
C₄₋₆-Alkadienylen, gegebenenfalls ein- oder zweimal substituiert durch C₁₋₃-Alkyl, Fluor, Cyano oder Phenyl,
1,3,5-Hexatrienylen, gegebenenfalls substituiert durch C₁₋₃-Alkyl, Fluor, Cyano oder Phenyl, und
Ethinylen;
D ausgewählt ist aus
C₂₋₁₀-Alkylen, gegebenenfalls ein- oder zweimal substituiert durch C₁₋₆-Alkyl, Hydroxy oder C₁₋₆-Alkoxy,
C₄₋₁₀-Alkenylen, gegebenenfalls ein- oder zweimal substituiert durch C₁₋₆-Alkyl, Hydroxy oder C₁₋₆-Alkoxy,
C₄₋₁₀-Alkinylen, gegebenenfalls ein- oder zweimal substituiert durch C₁₋₆-Alkyl, Hydroxy oder C₁₋₆-Alkoxy, und
der Gruppe, bestehend aus C₂₋₁₀-Alkylen, C₄₋₁₀-Alkenylen und C₄₋₁₀-Alkinylen, worin ein bis drei Methyleneinheiten isosterisch ersetzt sind durch O, S, NR¹⁰, CO, SO oder SO₂, worin
R¹⁰ die gleiche Bedeutung wie R⁹ aufweist, aber unabhängig davon ausgewählt ist;
E darstellt: 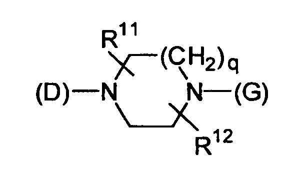 worin:
q 1, 2 oder 3 ist;
R¹¹ ausgewählt ist aus
Wasserstoff, C₁₋₆-Alkyl, Hydroxy, Hydroxymethyl, Carboxy und C₂₋₇-Alkoxycarbonyl, und
R¹² ausgewählt ist aus
Wasserstoff, C₁₋₆-Alkyl und einer zu einem Stickstoffatom benachbarten Oxogruppe,
oder
R¹¹ und R¹² bilden zusammen eine C₁₋₃-Alkylenbrücke unter Bildung eines bicyclischen Ringsystems;
G ist ausgewählt aus G1, G2, G3, G4 und G5, worin
G1 darstellt: 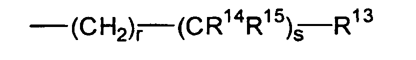
r 0, 1, 2 oder 3 ist;
s 0 oder 1 ist;
R¹³ ausgewählt ist aus
Wasserstoff, C₁₋₆-Alkyl, C₃₋₆-Alkenyl, C₃₋₆-Alkinyl, C₃₋₈-Cycloalkyl,
der Gruppe bestehend aus gesättigten und ungesättigten, 4- bis 8-gliedrigen Heterocyclen, die gegebenenfalls ein oder zwei Heteroatome, ausgewählt aus N, S und O, enthalten,
Benzyl, Phenyl,
der Gruppe bestehend aus monocyclischen aromatischen 5- bis 6-gliedrigen Heterocyclen, die gegebenenfalls ein bis drei Heteroatome enthalten, ausgewählt aus N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht,
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome ausgewählt sind aus N, S und O, und die Verknüpfung entweder über einen aromatischen Ring oder einen hydrierten Ring und entweder direkt oder über eine Methylengruppe geschieht;
R¹⁴ die gleiche Bedeutung wie R¹³ hat, aber unabhängig davon ausgewählt ist;
R¹⁵ ausgewählt ist aus
Wasserstoff, Hydroxy, Methyl, Benzyl, Phenyl,
der Gruppe bestehend aus monocyclischen aromatischen 5- bis 6-gliedrigen Heterocyclen, die ein bis drei Heteroatome enthalten, ausgewählt aus der Gruppe N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht,
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome gegebenenfalls ausgewählt sind aus N, S und O, und die Verknüpfung entweder über einen aromatischen Ring oder einen hydrierten Ring und entweder direkt oder über eine Methylengruppe geschieht;
G2 ausgewählt ist aus 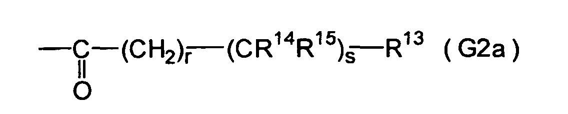 und 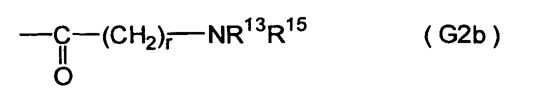 worin r, s und die Substituenten R¹³ bis R¹⁵ obige Bedeutungen aufweisen, oder die Gruppe
―NR¹³R¹⁵
einen über das Stickstoffatom gebundenen stickstoffhaltigen Heterocyclus darstellt, wobei der stickstoffhaltige Heterocyclus ausgewählt ist aus
der Gruppe bestehend aus gesättigten und ungesättigten, monocyclischen, 4- bis 8-gliedrigen Heterocyclen, die neben dem essentiellen Stickstoffatom gegebenenfalls ein oder zwei weitere Heteroatome, ausgewählt aus N, S und O, enthalten, und
der Gruppe bestehend aus gesättigten und ungesättigten, bi- oder tricyclischen, kondensierten oder verbrückten Heterocyclen mit 8 bis 16 Ringatomen, die neben dem essentiellen Stickstoffatom gegebenenfalls ein oder zwei weitere Heteroatome, ausgewählt aus N, S und O, enthalten;
G3 darstellt: 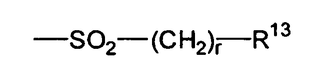 worin r und R¹³ die obigen Bedeutungen aufweisen;
G⁴ darstellt: 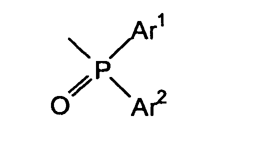 worin
Ar¹ und Ar² unabhängig voneinander ausgewählt sind aus Phenyl, Pyridyl und Naphthyl;
G5 darstellt:
―COR¹⁶ (G5)
worin
R¹⁶ ausgewählt ist aus Trifluormethyl, C₁₋₆-Alkoxy, C₃₋₆-Alkenyloxy und Benzyloxy,
worin aromatische Ringsysteme in den Substituenten R¹, R², R⁴, R⁵, R⁶, R¹³, R¹⁴, R¹⁵, R¹⁶, Ar¹ und Ar² und/oder in dem Ringsystem -NR¹³R¹⁵ unabhängig voneinander gegebenenfalls durch ein bis drei der gleichen oder unterschiedlichen Gruppen substituiert sind, unabhängig ausgewählt aus
Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und worin
Alkyl-, Alkenyl- und Cycloalkylreste in den Gruppen G1, G2 und G3 gegebenenfalls durch ein oder zwei der gleichen oder unterschiedlichen Gruppen substituiert sind, die ausgewählt sind aus Hydroxy, Carboxy, C₂₋₇-Alkoxycarbonyl, Benzyloxycarbonyl, Amino, Mono-C₁₋₆-alkylamino und Di-(C₁₋₆-alkyl-amino);
ihre cis- und trans-Isomere, E- und Z-Isomere, Enantiomere, Diastereomere und andere Isomere sowie ihre racemischen oder nicht-racemischen Mischungen und die entsprechenden endo- und exo-Isomere für den Fall, dass das Ringsystem E bicyclisch ist;
ihre Tautomere; sowie
ihre Säureadditionssalze einschliesslich ihrer Hydrate und Solvate,
worin G nicht darstellt: 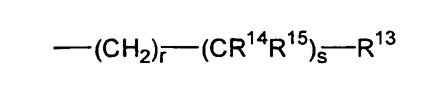 für den Fall, dass gleichzeitig
R¹³ Pyridyl oder Phenyl, das gegebenenfalls durch Halogen, Alkyl, Alkoxy oder Trifluormethyl substituiert ist, darstellt,
R¹⁴ Wasserstoff oder Phenyl darstellt, das gegebenenfalls durch Halogen, Alkyl, Alkoxy oder Trifluormethyl substituiert ist,
R¹⁵ Wasserstoff darstellt,
A Alkylen, gegebenenfalls substituiertes Ethenylen oder Butadienylen darstellt,
D Alkylen oder Alkenylen darstellt,
E Piperazin oder Homopiperazin darstellt, und
s 1 ist;
und worin G nicht darstellt:
ein Mitglied der Gruppe, bestehend aus Phenyl, N-haltigem Heteroaryl und 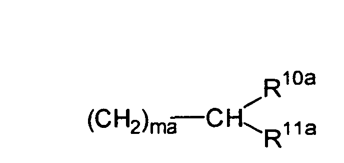 worin: R¹⁰ Wasserstoff oder Phenyl ist, R^{11a} Phenyl oder Pyridyl ist, und ma eine ganze Zahl von 0 bis 2 ist, vorausgestzt dass die Phenylgruppe gegebenenfalls durch ein oder zwei Mitglieder, ausgewählt aus der Gruppe bestehend aus Halogen, C₁₋₆-Alkyl, Trifluormethyl und C₁₋₆-Alkoxy, substituiert ist;
für den Fall, dass gleichzeitig
R¹ Wasserstoff, Halogen, C₁₋₆-Alkyl, C₁₋₆-Alkoxy, C₁₋₆-Alkylthio, C₃₋₈-Cycloalkyloxy, C₃₋₈-Cycloalkylthio, C₂₋₇-Alkoxycarbonyl, Carboxy, Phenyl, Phenoxy, Phenylthio, 3-Pyridyloxy oder 3-Pyridylthio bedeutet;
R² Wasserstoff, Hydroxy, C₁₋₇-Alkanoyloxy oder C₂₋₇-Alkoxycarbonyloxy bedeutet, oder, wenn R¹ und R² zueinander benachbart sind, sie zur Bildung von Tetramethylen oder -CH₂OCR^{8a}R^{9a}O- verknüpft sein können, worin R^{8a} und R^{9a} gleich oder unterschiedlich sind und jeweils C₁₋₆-Alkyl bedeuten;
R³ Wasserstoff, C₁₋₆-Alkyl oder Hydroxy-C₁₋₆-alkyl bedeutet;
A C₁₋₆-Alkylen oder -(CR^{6a}=CR^{7a})ᵣₐ- bedeutet, worin R^{6a} Wasserstoff, C₁₋₃-Alkyl oder Phenyl ist, R^{7a} Wasserstoff, C₁₋₃-Alkyl, Cyano oder Phenyl ist, und ra 1 oder 2 ist;
R⁴ Wasserstoff ist;
D C₂₋₁₀-Alkylen oder C₄₋₁₀-Alkylen, unterbrochen durch eine Doppelbindung, ist; und
E ausgewählt ist aus Piperazin, Piperazin, das durch C₁₋₆-Alkyl substituiert ist, Homopiperazin und Homopiperazin, das durch C₁₋₆-Alkyl substituiert ist.

2. Verbindung gemäss Formel (I) 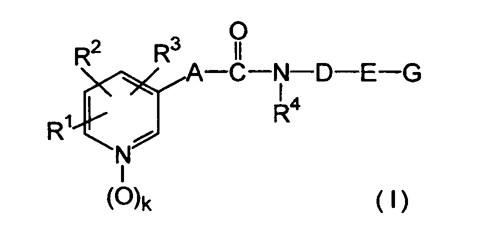 worin:
R¹ ausgewählt ist aus
Wasserstoff, Halogen, Cyano, C₁₋₆-Alkyl, Trifluormethyl, C₃₋₈-Cycloalkyl, C₁₋₆-Hydroxyalkyl, Hydroxy, C₁₋₄-Alkoxy, Benzyloxy, C₁₋₄-Alkylthio, C₁₋₅-Alkanoyloxy, C₂₋₅-Alkoxycarbonyl, Aminocarbonyl, C₂₋₅-Alkylaminocarbonyl, C₃₋₉-Dialkylaminocarbonyl, Carboxy, Phenyl, Phenoxy, Phenylthio, Pyridyloxy und NR⁵R⁶, worin
R⁵ und R⁶ unabhängig voneinander ausgewählt sind aus Wasserstoff und C₁₋₆-Alkyl;
R² ausgewählt ist aus
Wasserstoff, Halogen, Cyano, C₁₋₆-Alkyl, Trifluormethyl, Hydroxy und C₁₋₄-Alkoxy;
R³ ausgewählt ist aus
Wasserstoff, Halogen und C₁₋₆-Alkyl;
R⁴ ausgewählt ist aus
Wasserstoff, C₁₋₆-Alkyl, C₃₋₆-Alkenyl, C₃₋₆-Cycloalkyl, Hydroxy, C₁₋₆-Alkoxy und Benzyloxy;
k 0 oder 1 ist;
A ausgewählt ist aus
C₁₋₆-Alkylen, gegebenenfalls ein- bis dreimal substituiert durch C₁₋₃-Alkyl, Hydroxy, Fluor oder Phenyl,
C₂₋₆-Alkylen, worin eine Methyleneinheit isosterisch ersetzt ist durch O, S, NR⁹, CO, SO oder SO₂, worin, mit Ausnahme von CO, die isosterische Substitution nicht benachbart zur Amidgruppe ist und der Rest R⁹ ausgewählt ist aus Wasserstoff, C₁₋₆-Alkyl, C₁₋₆-Acyl und Methansulfonyl,
1,2-Cyclopropylen,
C₂₋₆-Alkenylen, gegebenenfalls ein- bis dreimal substituiert durch C₁₋₃-Alkyl, Hydroxy, Fluor, Cyano oder Phenyl,
C₄₋₆-Alkadienylen, gegebenenfalls ein- oder zweimal substituiert durch C₁₋₃-Alkyl, Fluor, Cyano oder Phenyl,
1,3,5-Hexatrienylen, gegebenenfalls substituiert durch C₁₋₃-Alkyl, Fluor oder Cyano, und
Ethinylen;
D ausgewählt ist aus
C₂₋₁₀-Alkylen, gegebenenfalls ein- oder zweimal substituiert durch C₁₋₃-Alkyl oder Hydroxy,
C₄₋₁₀-Alkenylen, gegebenenfalls ein- oder zweimal substituiert durch C₁₋₃-Alkyl oder Hydroxy,
C₄₋₁₀-Alkinylen, gegebenenfalls ein- oder zweimal substituiert durch C₁₋₃-Alkyl oder Hydroxy, und
der Gruppe, bestehend aus C₂₋₁₀-Alkylen, C₄₋₁₀-Alkenylen und C₄₋₁₀-Alkinylen, worin ein bis drei Methyleneinheiten isosterisch ersetzt sind durch O, S, NR¹⁰, CO, SO oder SO₂, worin
R¹⁰ die gleiche Bedeutung wie R⁹ aufweist, aber unabhängig davon ausgewählt ist;
E darstellt: 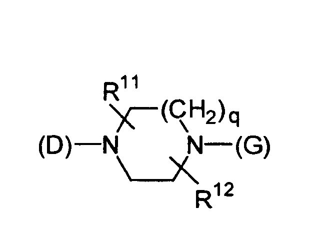 worin:
q 1, 2 oder 3 ist;
R¹¹ ausgewählt ist aus
Wasserstoff, C₁₋₃-Alkyl, Hydroxy, Hydroxymethyl, Carboxy und C₂₋₇-Alkoxycarbonyl; und
R¹² ausgewählt ist aus
Wasserstoff und einer zu einem Stickstoffatom benachbarten Oxogruppe,
oder
R¹¹ und R¹² bilden zusammen eine C₁₋₃-Alkylenbrücke unter Bildung eines bicyclischen Ringsystems;
G ist ausgewählt aus G1, G2, G3, G4 und G5, worin
G1 darstellt: 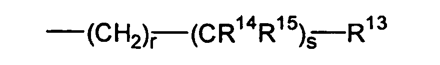
r 0, 1 oder 2 ist;
s 0 oder 1 ist;
R¹³ ausgewählt ist aus
Wasserstoff, C₁₋₆-Alkyl, C₃₋₆-Alkenyl, C₃₋₆-Alkinyl, C₃₋₈-Cycloalkyl, Benzyl, Phenyl,
der Gruppe bestehend aus monocyclischen aromatischen 5- bis 6-gliedrigen Heterocyclen, die ein bis drei Heteroatome enthalten, ausgewählt aus N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht,
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome gegebenenfalls ausgewählt sind aus N, S und O, und die Bindung entweder über einen aromatischen Ring oder einen hydrierten Ring und entweder direkt oder über eine Methylengruppe geschieht;
R¹⁴ die gleiche Bedeutung wie R¹³ hat, aber unabhängig davon ausgewählt ist;
R¹⁵ ausgewählt ist aus
Wasserstoff, Hydroxy, Methyl, Benzyl, Phenyl, der Gruppe bestehend aus monocyclischen aromatischen 5- bis 6-gliedrigen Heterocyclen, die ein bis drei Heteroatome enthalten, ausgewählt aus N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht, und
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome gegebenenfalls ausgewählt sind aus N, S und O, und die Verknüpfung entweder über einen aromatischen Ring oder einen hydrierten Ring und entweder direkt oder über eine Methylengruppe geschieht;
G2 ausgewählt ist aus 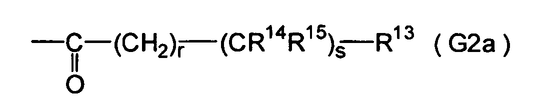 und 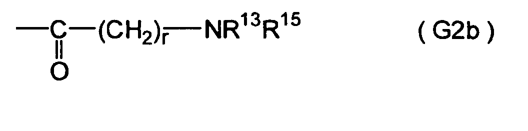 worin r, s und die Substituenten R¹³ bis R¹⁵ obige Bedeutungen aufweisen, oder die Gruppe
―N¹³R¹⁵
ist ein über das Stickstoffatom gebundener stickstoffhaltiger Heterocyclus, wobei der stickstoffhaltige Heterocyclus ausgewählt ist aus
der Gruppe bestehend aus gesättigten und ungesättigten, monocyclischen, 4- bis 8-gliedrigen Heterocyclen, die neben dem essentiellen Stickstoffatom gegebenenfalls ein oder zwei weitere Heteroatome, ausgewählt aus N, S und O, enthalten, und
der Gruppe bestehend aus gesättigten und ungesättigten, bi- oder tricyclischen, kondensierten oder verbrückten Heterocyclen mit 8 bis 16 Ringatomen, die neben dem essentiellen Stickstoffatom gegebenenfalls ein oder zwei weitere Heteroatome, ausgewählt aus N, S und O, enthalten;
G3 darstellt: 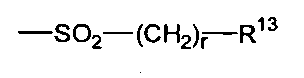 worin r und R¹³ die obigen Bedeutungen aufweisen;
G⁴ darstellt: 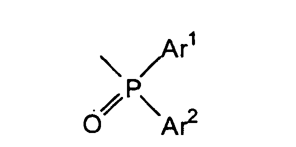 worin
Ar¹ und Ar² unabhängig voneinander ausgewählt sind aus Phenyl, Pyridyl und Naphthyl;
G5 darstellt:
―COR¹⁶
worin
R¹⁶ ausgewählt ist aus
Trifluormethyl, C₁₋₆-Alkoxy, C₃₋₆-Alkenyloxy und Benzyloxy,
worin aromatische Ringsysteme in den Substituenten R¹, R², R⁴, R⁵, R⁶, R¹³, R¹⁴, R¹⁵, R¹⁶, Ar¹ und Ar² und/oder in dem Ringsystem -NR¹³R¹⁵ unabhängig voneinander gegebenenfalls durch ein bis drei der gleichen oder unterschiedlichen Gruppen substituiert sind, ausgewählt aus
Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid,
worin Alkyl-, Alkenyl- und Cycloalkylreste in den Gruppen G1, G2 und G3 gegebenenfalls durch ein oder zwei der gleichen oder unterschiedlichen Gruppen substituiert sind, die ausgewählt sind aus
Hydroxy, Carboxy, C₂₋₇-Alkoxycarbonyl, Benzyloxycarbonyl, Amino, Mono-C₁₋₆-alkylamino und Di-(C₁₋₆-alkyl-amino);
worin G nicht darstellt:
ein Mitglied der Gruppe, bestehend aus Phenyl, N-haltiges Heteroaryl und 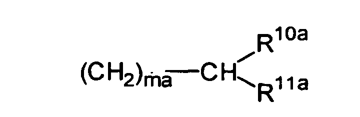 worin: R¹⁰ Wasserstoff oder Phenyl ist, R^{11a} Phenyl oder Pyridyl ist, und ma eine ganze Zahl von 0 bis 2 ist, vorausgesetzt dass die Phenylgruppe gegebenenfalls durch ein oder zwei Mitglieder, ausgewählt aus der Gruppe bestehend aus Halogen, C₁₋₆-Alkyl, Trifluormethyl und C₁₋₆-Alkoxy, substituiert ist;
für den Fall, dass gleichzeitig
R¹ Wasserstoff, Halogen, C₁₋₆-Alkyl, C₁₋₆-Alkoxy, C₁₋₆-Alkylthio, C₃₋₈-Cycloalkyloxy, C₃₋₈-Cycloalkylthio, C₂₋₇-Alkoxycarbonyl, Carboxy, Phenyl, Phenoxy, Phenylthio, 3-Pyridyloxy oder 3-Pyridylthio bedeutet;
R² Wasserstoff, Hydroxy, C₁₋₇-Alkanoyloxy oder C₂₋₇-Alkoxycarbonyloxy bedeutet, oder, wenn R¹ und R² zueinander benachbart sind, sie zur Bildung von Tetramethylen oder -CH₂OCR^{8a}R^{9a}O- verknüpft sein können, worin R^{8a} und R^{9a} gleich oder unterschiedlich sind und jeweils C₁₋₆-Alkyl bedeuten;
R³ Wasserstoff, C₁₋₆-Alkyl oder Hydroxy-C₁₋₆-alkyl bedeutet;
A C₁₋₆-Alkylen oder -(CR^{6a}=CR^{7a})ᵣₐ- bedeutet, worin R^{6a} Wasserstoff, C₁₋₃-Alkyl oder Phenyl ist, R^{7a} Wasserstoff, C₁₋₃-Alkyl, Cyano oder Phenyl ist, und ra 1 oder 2 ist;
R⁴ Wasserstoff ist;
D C₂₋₁₀-Alkylen oder C₄₋₁₀-Alkylen, unterbrochen durch eine Doppelbindung, ist; und
E ausgewählt ist aus Piperazin, Piperazin, das durch C₁₋₆-Alkyl substituiert ist, Homopiperazin und Homopiperazin, das durch C₁₋₆-Alkyl substituiert ist.

3. Verbindung gemäss Anspruch 2, worin
R¹ ausgewählt ist aus
Wasserstoff, Halogen, Cyano, Methyl, Ethyl, Trifluormethyl, Hydroxy, C₁₋₄-Alkoxy, Benzyloxy, C₁₋₅-Alkanoyloxy, Methylthio, Ethylthio, Methoxycarbonyl, tert-Butoxycarbonyl, Aminocarbonyl, Carboxy, Phenoxy und Phenylthio;
R² ausgewählt ist aus
Wasserstoff, Halogen, Trifluormethyl und Hydroxy;
R³ ausgewählt ist aus
Wasserstoff und Halogen;
R⁴ ausgewählt ist aus
Wasserstoff, C₁₋₃-Alkyl, Allyl, Hydroxy und C₁₋₃-Alkoxy;
k 0 oder 1 ist;
A ausgewählt ist aus
C₁₋₆-Alkylen, gegebenenfalls ein- oder zweimal substituiert durch C₁₋₃-Alkyl, Hydroxy oder Fluor,
C₂₋₆-Alkylen, worin eine Methyleneinheit isosterisch ersetzt ist durch O, S, NR⁹, CO, SO oder SO₂, worin, mit Ausnahme von CO, die isosterische Substitution nicht benachbart zur Amidgruppe ist,
C₂₋₆-Alkenylen, gegebenenfalls ein- oder zweimal substituiert durch C₁₋₃-Alkyl, Hydroxy und/oder Fluor,
C₄₋₆-Alkadienylen, gegebenenfalls durch C₁₋₃-Alkyl oder ein oder zwei Fluoratome substituiert, und
1,3,5-Hexatrienylen, gegebenenfalls durch Fluor substituiert;
D ausgewählt ist aus
C₂₋₈-Alkylen, gegebenenfalls ein- oder zweimal substituiert durch Methyl oder Hydroxy,
C₄₋₈-Alkenylen, gegebenenfalls ein- oder zweimal substituiert durch Methyl oder Hydroxy,
C₄₋₈-Alkinylen, gegebenenfalls ein- oder zweimal substituiert durch Methyl oder Hydroxy, und
der Gruppe, bestehend aus C₂₋₈-Alkylen, C₄₋₈-Alkenylen und C₄₋₈-Alkinylen, worin ein bis drei Methyleneinheiten jeweils isosterisch ersetzt sind durch O, S, NH, N(CH₃), N(COCH₃), N(SO₂CH₃), CO, SO oder SO₂,
E darstellt: 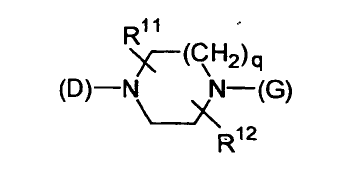 worin:
q 1 oder 2 ist;
R¹¹ ausgewählt ist aus
Wasserstoff, C₁₋₃-Alkyl, Hydroxymethyl und Carboxy; und
R¹² ausgewählt ist aus
Wasserstoff und einer zu einem Stickstoffatom benachbarten Oxogruppe;
G ausgewählt ist aus G1, G2, G3, G4 und G5, worin
G1 darstellt: 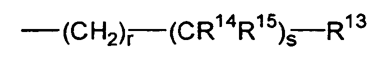
r 0, 1 oder 2 ist;
s 0 oder 1 ist;
R¹³ ausgewählt ist aus
Wasserstoff, C₁₋₆-Alkyl, C₃₋₈-Cycloalkyl, Benzyl, Phenyl,
der Gruppe bestehend aus Benzocyclobutyl, Indanyl, Indenyl, Oxoindanyl, Naphthyl, Dihydronaphthyl, Tetrahydronaphthyl, Oxotetrahydronaphthyl, Biphenylenyl, Fluorenyl, Oxofluorenyl, Anthryl, Dihydroanthryl, Oxodihydroanthryl, Dioxodihydroanthryl, Phenanthryl, Dihydrophenanthryl, Oxodihydrophenanthryl, Dibenzocycloheptenyl, Oxodibenzocycloheptenyl, Dihydrodibenzocycloheptenyl, Oxodihydrodibenzocycloheptenyl, Dihydrodibenzocyclooctenyl, Tetrahydrodibenzocyclooctenyl und Oxotetrahydrodibenzocyclooctenyl, direkt oder über eine Methylengruppe gebunden; und
der Gruppe bestehend aus Furyl, Thienyl, Pyrrolyl, Oxazolyl, Isoxazolyl, Thiazolyl, Isothiazolyl, Pyrazolyl, Imidazolyl, Oxadiazolyl, Thiadiazolyl, Triazolyl, Pyridyl, Pyrazinyl, Pyridazinyl, Pyrimidinyl, Triazinyl, Imidazothiazolyl, Benzofuryl, Dihydrobenzofuryl, Benzothienyl, Dihydrobenzothienyl, Indolyl, Indolinyl, Isoindolinyl, Oxoindolinyl, Dioxoindolinyl, Benzoxazolyl, Oxobenzooxazolinyl, Benzoisooxazolyl, Oxobenzoisooxazolinyl, Benzothiazolyl, Oxobenzothiazolinyl, Benzoisothiazolyl, Oxobenzoisothiazolinyl, Benzoimidazolyl, Oxobenzoimidazolinyl, Indazolyl, Oxoindazolinyl, Benzofurazanyl, Benzothiadiazolyl, Benzotriazolyl, Oxazolopyridyl, Oxodihydrooxazolopyridyl, Thiazolopyridyl, Oxodihydrothiazolopyridyl, Isothiazolopyridyl, Imidazopyridyl, Oxodihydroimidazopyridyl, Pyrazolopyridyl, Oxodihydropyrazolopyridyl, Thienopyrimidinyl, Chromanyl, Chromanonyl, Benzopyranyl, Chromonyl, Chinoloyl, Isochinoloyl, Dihydrochinoloyl, Oxodihydrochinoloyl, Tetrahydrochinoloyl, Oxotetrahydrochinolinyl, Benzodioxanyl, Chinoxalinyl, Chinazolinyl, Naphthyridinyl, Carbazolyl, Tetrahydrocarbazolyl, Oxotetrahydrocarbazolyl, Pyridoindolyl, Acridinyl, Oxodihydroacridinyl, Phenanthridinyl, Dihydrophenanthridinyl, Oxodihydrophenanthridinyl, Dibenzoisochinolinyl, Dihydrodibenzoisochinolinyl, Oxodihydrodibenzoisochinolinyl, Phenothiazinyl, Dihydrodibenzooxepinyl, Oxodihydrodibenzooxepinyl; Benzocycloheptathienyl, Oxobenzocycloheptathienyl, Dihydrothienobenzothiepinyl, Oxodihydrothienobenzothiepinyl, Dihydrodibenzothiepinyl, Oxodihydrodibenzothiepinyl, Octahydrodibenzothiepinyl, Dibenzoazepinyl, Dihydrodibenzoazepinyl, Oxodihydrodibenzoazepinyl, Octahydrodibenzoazepinyl, Benzocycloheptapyridyl, Oxobenzocycloheptapyridyl, Pyridobenzoazepinyl, Dihydropyridobenzoazepinyl, Oxodihydropyridobenzoazepinyl, Dihydropyridobenzodiazepinyl, Dihydrodibenzooxazepinyl, Dihydropyridobenzooxepinyl, Dihydropyridobenzooxazepinyl, Oxodihydropyridobenzooxazepinyl, Dihydrodibenzothiazepinyl, Oxodihydrodibenzothiazepinyl, Dihydropyridobenzothiazepinyl und Oxodihydropyridobenzothiazepinyl, direkt oder über eine Methylengruppe gebunden;
R¹⁴ die gleiche Bedeutung wie R¹³ hat, aber unabhängig davon ausgewählt ist;
R¹⁵ ausgewählt ist aus
Hydroxy, Methyl, Benzyl, Phenyl, Indanyl, Indenyl, Naphthyl, Dihydronaphthyl, Tetrahydronaphthyl, Furyl, Thienyl, Pyrrolyl, Oxazolyl, Isoxazolyl, Thiazolyl, Isothiazolyl, Pyrazolyl, Imidazolyl, Oxadiazolyl, Thiadiazolyl, Triazolyl, Pyridyl, Pyrazinyl, Pyridazinyl, Pyrimidinyl, Triazinyl, Benzofuryl, Benzothienyl, Indolyl, Indolinyl, Benzooxazolyl, Benzothiazolyl, Benzoimidazolyl, Chromanyl, Chinolyl und Tetrahydrochinolyl, direkt oder über eine Methylengruppe gebunden,
G2 ausgewählt ist aus 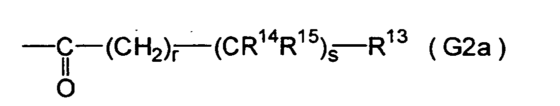 und 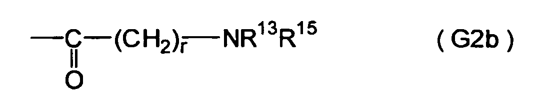 worin r, s und die Substituenten R¹³ bis R¹⁵ obige Bedeutungen aufweisen oder die Gruppe
―NR¹³R¹⁵
darstellt: Azetidin, Pyrrolidin, Piperidin, (1H)-Tetrahydropyridin, Hexahydroazepin, (1H)-Tetrahydroazepin, Octahydroazocin, Pyrazolidin, Piperazin, Hexahydrodiazepin, Morpholin, Hexahydrooxazepin, Thiomorpholin, Thiomorpholin-1,1-dioxid, 5-Aza-bicyclo[2.1.1]hexan, 2-Aza-bicyclo[2.2.1]heptan, 7-Aza-bicyclo[2.2.1]heptan, 2,5-Diaza-bicyclo[2.2.1]heptan, 2-Aza-bicyclo[2.2.2]octan, 8-Aza-bicyclo[3.2.1]octan, 2,5-Diaza-bicyclo[2.2.2]octan, 9-Aza-bicyclo[3.3.1]nonan, Indolin, Isoindolin, (1H)-Dihydrochinolin, (1H)-Tetrahydrochinolin, (2H)-Tetrahydroisochinolidin, (1H)-Tetrahydrochinoxalin, (4H)-Dihydrobenzooxazin, (4H)-Dihydrobenzothiazin, (1H)-Tetrahydrobenzo[b]azepin, (1H)-Tetrahydrobenzo[c]azepin, (1H)-Tetrahydrobenzo[d]azepin, (5H)-Tetrahydrobenzo[b]oxazepin, (5H)-Tetrahydrobenzo[b]thiazepin, 1,2,3,4-Tetrahydro-9H-pyrido[3,4-b]indol, (10H)-Dihydroacridin, (10H)-Dihydrophenanthridin, 1,2,3,4-Tetrahydroacridanon, (10H)-Phenoxazin, (10H)-Phenothiazin, (5H)-Dibenzoazepin, (5H)-Dihydrodibenzoazepin, (5H)-Octahydrodibenzoazepin, Dihydrobenzo[d,e]isochinolin, (5H)-Dihydrodibenzodiazepin, (5H)-Benzo[b]pyrido[f]azepin, (5H)-Dihydrodibenzo[b]pyrido[f]azepin, (11H)-Dihydrodibenzo[b,e]oxazepin, (11H)-Dihydrodibenzo[b,e]thiazepin, (10H)-Dihydrodibenzo[b,f]oxazepin, (10H)-Dihydrodibenzo[b,f]thiazepin, (5H)-Tetrahydrodibenzoazoacin, (11H)-Dihydrobenzo[e]pyrido[b]-1,4-diazepin-6-on oder (11H)-Dihydrobenzo[b]pyrido[e]-1,4-diazepin-5-on;
G3 darstellt: 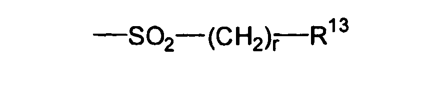 worin r und R¹³ die obigen Bedeutungen aufweisen;
G⁴ darstellt: 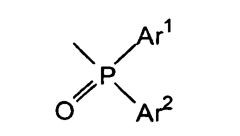 worin
Ar¹ und Ar² unabhängig voneinander ausgewählt sind aus Phenyl, Pyridyl und Naphthyl;
G5 darstellt:
―COR¹⁶
worin
R¹⁶ ausgewählt ist aus
Trifluormethyl, C₁₋₆-Alkoxy, C₃₋₆-Alkenyloxy und Benzyloxy,
worin aromatische Ringsysteme in den Substituenten unabhängig voneinander gegebenenfalls durch ein bis drei der gleichen oder unterschiedlichen Gruppen substituiert sind, ausgewählt aus
Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und
worin Alkyl-, Alkenyl- und Cycloalkylreste in den Gruppen G1, G2 und G3 gegebenenfalls durch ein oder zwei gleiche oder unterschiedliche Gruppen substituiert sind, die ausgewählt sind aus
Hydroxy, Carboxy, C₂₋₇-Alkoxycarbonyl, Benzyloxycarbonyl, Amino, Mono-C₁₋₆-alkylamino und Di-(C₁₋₆-alkyl-amino).

4. Verbindung gemäss Anspruch 2 oder 3, worin
R¹ ausgewählt ist aus
Wasserstoff, Fluor, Chlor, Brom, Methyl, Ethyl, Trifluormethyl, Hydroxy, C₁₋₄-Alkoxy, Methylthio, Ethylthio, Carboxy und Phenoxy;
R² ausgewählt ist aus Wasserstoff, Chlor und Methyl;
R³ Wasserstoff ist;
R⁴ ausgewählt ist aus Wasserstoff, C₁₋₃-Alkyl und Hydroxy;
k 0 ist;
A ausgewählt ist aus
C₂₋₆-Alkylen, gegebenenfalls ein- oder zweimal substituiert durch Hydroxy oder Fluor,
C₂₋₆-Alkylen, worin eine Methyleneinheit isosterisch ersetzt ist durch O, S oder CO, worin, mit Ausnahme von CO, die isosterische Substitution nicht benachbart zur Amidgruppe ist,
C₂₋₆-Alkenylen, gegebenenfalls ein- oder zweimal substituiert durch C₁₋₃-Alkyl und/oder Fluor, und
C₄₋₆-Alkadienylen;
D ausgewählt ist aus
C₂₋₈-Alkylen, gegebenenfalls substituiert durch Methyl oder Hydroxy,
C₄₋₈-Alkenylen, gegebenenfalls substituiert durch Hydroxy,
C₄₋₈-Alkinylen, gegebenenfalls substituiert durch Hydroxy, und
der Gruppe, bestehend aus C₂₋₈-Alkylen, C₄₋₈-Alkenylen und C₄₋₈-Alkinylen, worin eine Methyleneinheit isosterisch ersetzt ist durch O, NH, N(CH₃), CO oder SO₂, oder eine Ethylengruppe isosterisch ersetzt ist durch eine Gruppe NH-CO und/oder CO-NH, oder eine Propylengruppe isosterisch ersetzt ist durch eine Gruppe NH-CO-O und/oder O-CO-NH;
E ausgewählt ist aus Piperazin und Hexahydro-1,4-diazepin, worin der Ring gegebenenfalls durch ein oder zwei Methylengruppen und/oder durch eine zu einem Stickstoffatom benachbarte Oxogruppe substituiert ist;
G ausgewählt ist aus Wasserstoff, C₃₋₈-Cycloalkyl, Methoxycarbonyl, tert-Butoxycarbonyl, Benzyloxycarbonyl, Trifluoracetyl, Diphenylphosphinoyl, 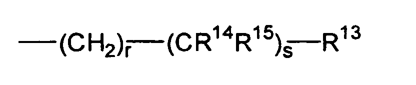 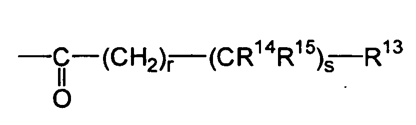 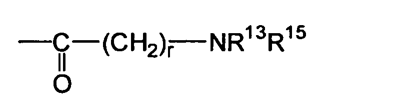 und 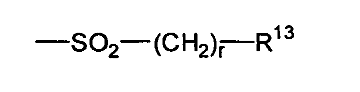 worin
r 0 oder 1 ist;
s 0 oder 1 ist;
R¹³ ausgewählt ist aus Wasserstoff, Methyl, Benzyl, Phenyl,
der Gruppe bestehend aus Indanyl, Indenyl, Oxoindanyl, Naphthyl, Tetrahydronaphthyl, Fluorenyl, Oxofluorenyl, Anthryl, Dihydroanthryl, Oxodihydroanthryl, Dioxodihydroanthryl, Phenanthryl, Dihydrophenanthryl, Oxodihydrophenanthryl, Dibenzocycloheptenyl, Dihydrodibenzocycloheptenyl und Oxodihydrodibenzocycloheptenyl, direkt oder über eine Methylengruppe gebunden; und
der Gruppe bestehend aus Furyl, Thienyl, Oxazolyl, Isooxazolyl, Thiazolyl, Imidazolyl, Oxadiazolyl, Pyridyl, Pyrazinyl, Pyrimidinyl, Imidazothiazolyl, Benzofuryl, Benzothienyl, Indolyl, Indolinyl, Oxoindolinyl, Dioxoindolinyl, Benzoxazolyl, Oxobenzooxazolinyl, Benzothiazolyl, Oxobenzothiazolinyl, Benzoimidazolyl, Oxobenzimidazolinyl, Indazolyl, Benzofurazanyl, Benzotriazolyl, Oxazolopyridyl, Oxodihydrooxazolopyridyl, Thiazolopyridyl, Oxodihydrothiazolopyridyl, Imidazopyridyl, Oxodihydroimidazopyridyl, Chromanyl, Chromanonyl, Benzopyranyl, Chromonyl, Chinolyl, Isochinolyl, Oxodihydrochinolinyl, Tetrahydrochinolyl, Oxotetrahydrochinolinyl, Benzodioxanyl, Chinazolinyl, Carbazolyl, Acridinyl, Dihydroacridinyl, Oxodihydroacridinyl, Dihydrophenanthridinyl, Dihydrodibenzoisochinolinyl, Phenothiazinyl, Dihydrodibenzooxepinyl, Benzocycloheptathienyl, Dihydrothienobenzothiepinyl, Dihydrodibenzothiepinyl, Oxodihydrodibenzothiepinyl, Dihydrodibenzoazepinyl, Oxodihydrodibenzoazepinyl, Octahydrodibenzoazepinyl, Benzocycloheptapyridyl, Dihydropyridobenzodiazepinyl und Dihydrodibenzothiazepinyl, direkt oder über eine Methylengruppe gebunden;
R¹⁴ ausgewählt ist aus Wasserstoff, Methyl, Benzyl und Phenyl;
R¹⁵ ausgewählt ist aus
Wasserstoff, Hydroxy, Methyl, Benzyl, Phenyl; und
der Gruppe bestehend aus Naphthyl, Tetrahydronaphthyl, Furyl, Thienyl, Oxazolyl, Thiazolyl, Imidazolyl, Pyridyl, Benzofuryl, Benzothienyl, Indolyl, Indolinyl, Benzoxazolyl, Benzothiazolyl, Benzoimidazolyl, Chromanyl, Chinolyl und Tetrahydrochinolyl, direkt oder über eine Methylengruppe gebunden,
worin die Gruppe
―NR¹³R¹⁵
einen über das Stickstoffatom gebundenen stickstoffhaltigen Heterocyclus darstellt, wobei der stickstoffhaltige Heterocyclus ausgewählt ist aus
Pyrrolidin, Piperid, Hexahydroazepin, Piperazin, Hexahydrodiazepin, Morpholin, Hexahydroxazepin, Thiomorpholin, 7-Aza-bicyclo[2.2.1]heptan, 2,5-Diaza-bicyclo[2.2.1]heptan, Indolin, Isoindolin, (1H)-Dihydrochinolin, (1H)-Tetrahydrochinolin, (2H)-Tetrahydroisochinolin, (4H)-Dihydrobenzoxazin, (4H)-Dihydrobenzothiazin, (1H)-Tetrahydrobenzo[b]azepin, (1H)-Tetrahydrobenzo[d]azepin, (5H)-Tetrahydrobenzo[b]oxazepin, (5H)-Tetrahydrobenzo[b]thiazepin, (10H)-Dihydroacridin, 1,2,3,4-Tetrahydroacridanon, (10H)-Dihydrophenanthridin, (1H)-Dihydrobenzo[d,e]isochinolin, (10H)-Phenothiazin, (5H)-Dibenzo[b,f]azepin, (5H)-Dihydrodibenzo[b,f]azepin, (5H)-Dihydrodibenzo[c,e]azepin, (5H)-Dihydrodibenzodiazepin, (11H)-Dihydrodibenzo[b,e]oxazepin, (11H)-Dihydrodibenzo[b,e]thiazepin, (5H)-Dihydrobenzo[b]pyrido[3,2-f]azepin und (11H)-6-Oxodihydrobenzo[e]pyrido[3,2-b][1,4]-diazepin,
worin aromatische Ringsysteme in den Substituenten unabhängig voneinander gegebenenfalls durch ein bis drei der gleichen oder unterschiedlichen Gruppen substituiert sind, ausgewählt aus Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und
worin Alkyl-, Alkenyl- und Cycloalkylreste in der Gruppe G gegebenenfalls durch ein oder zwei gleiche oder unterschiedliche Gruppen substituiert sind, die ausgewählt sind aus Hydroxy, Carboxy, C₂₋₇-Alkoxycarbonyl, Benzyloxycarbonyl, Amino, Mono-C₁₋₆-alkylamino und Di-(C₁₋₆-alkyl-amino).

5. Verbindung gemäss einem der Ansprüche 2 bis 4, worin
R¹ ausgewählt ist aus
Wasserstoff, Fluor, Methyl, Trifluormethyl und Ethylthio;
R², R³ und R⁴ jeweils Wasserstoff sind;
k 0 ist;
A ausgewählt ist aus
der Gruppe bestehend aus Ethylen, Propylen und Butylen, die jeweils gegebenenfalls durch Hydroxy oder ein oder zwei Fluoratome substituiert sind;
OCH₂, SCH₂;
Ethenylen und 1,3-Butadienylen;
D ausgewählt ist aus
C₂₋₆-Alkylen, gegebenenfalls substituiert durch Hydroxy,
C₄₋₆-Alkenylen,
C₄₋₆-Alkinylen und
der Gruppe, bestehend aus C₂₋₆-Alkylen, C₄₋₆-Alkenylen und C₄₋₆-Alkinylen, worin ein oder zwei Methyleneinheiten isosterisch ersetzt ist durch O, NH, CO oder SO₂;
E Piperazin oder Hexahydro-1,4-diazepin ist;
G ausgewählt ist aus
Phenyl, Benzyl, Phenethyl, Diphenylmethyl, Naphthyl, Tetrahydronaphthyl, Naphthylmethyl, Fluorenyl, Fluorenylmethyl, Anthrylmethyl, Dihydrodibenzocycloheptenyl, Furylmethyl, Thienylmethyl, Thiazolylmethyl, Pyridylmethyl, Benzothienylmethyl, Chinolylmethyl, Phenylthienylmethyl, Phenylpyridylmethyl, Benzocycloheptapyridinyl, Dihydrobenzocycloheptapyridinyl, Dihydrodibenzooxepinyl, Dihydrodibenzothiepinyl, Dihydrodibenzoazepinyl, Dihydrobenzopyridodiazepinyl, Formyl, Acetyl, Pivaloyl, Phenylacetyl, Diphenylacetyl, Diphenylpropionyl, Naphthylacetyl, Benzoyl, Naphthoyl, Oxofluorenylcarbonyl, Oxodihydroanthrylcarbonyl, Dioxodihydroanthrylcarbonyl, Furoyl, Pyridylacetyl, Pyridylcarbonyl, Chromonylcarbonyl, Chinolylcarbonyl, Phenylylaminocarbonyl, Naphthylaminocarbonyl, Tetrahydronaphthylaminocarbonyl, Dibenzylaminocarbonyl, Benzylphenylaminocarbonyl, Diphenylaminocarbonyl, Indolinyl-N-carbonyl, Isoindolinyl-N-carbonyl, Tetrahydrochinolinyl-N-carbonyl, Carbazolyl-N-carbonyl, Tetrahydrobenzoazepinyl-N-carbonyl, Dihydrodibenzoazepinyl-N-carbonyl, Dihydrobenzopyridoazepinyl-N-carbonyl, Oxodihydrobenzopyridoazepinyl-N-carbonyl, Methansulfonyl, Toluolsulfonyl, Naphthylsulfonyl, Chinolinsulfonyl und Diphenylphosphinoyl,
worin aromatische Ringsysteme in den Substituenten unabhängig voneinander gegebenenfalls durch ein bis drei der gleichen oder unterschiedlichen Gruppen substituiert sind, ausgewählt aus Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und worin
Alkyl-, Alkenyl- und Cycloalkylreste in der Gruppe G gegebenenfalls durch ein oder zwei gleiche oder unterschiedliche Gruppen substituiert sind, die ausgewählt sind aus
Hydroxy, Carboxy, C₂₋₇-Alkoxycarbonyl, Benzyloxycarbonyl, Amino, Mono-C₁₋₆-alkylamino und Di-(C₁₋₆-alkyl)-amino.

6. Verbindung gemäss einem der Ansprüche 2 bis 5, die ausgewählt ist aus der Gruppe bestehend aus
N-[4-(4-Diphenylmethylpiperazin-1-yl)-3-hydroxybutyl]-3-pyridin-3-yl-acrylamid,
N-[3-(4-Diphenylmethylpiperazin-1-yl)-propoxy]-3-pyridin-3-yl-acrylamid,
N-[4-(4-Diphenylmethylpiperazin-1-yl)-4-oxo-butyl]-3-pyridin-3-yl-acrylamid,
N-[3-(4-Diphenylmethylpiperazin-1-sulfonyl)-propyl]-3-pyridin-3-yl-acrylamid,
N-{2-[2-(4-Diphenylmethylpiperazin-1-yl)-ethoxy]-ethyl}-3-pyridin-3-yl-acrylamid,
N-(4-{4-[Bis-(4-fluorphenyl)-methyl]-piperazin-1-yl}-but-2-inyl)-3-pyridin-3-yl-acrylamid,
N-{4-[4-(4-Carboxyphenyl-phenylmethyl)-piperazin-1-yl]-butyl}-3-pyridin-3-yl-acrylamid,
N-(4-{4-[(4-Aminophenyl)-phenylmethyl]-piperazin-1-yl}-butyl)-3-pyridin-3-yl-acrylamid,
N-{4-[4-(9H-Fluoren-9-yl)-piperazin-1-yl]-butyl}-2-(pyridin-3-yloxy)-acetamid,
N-{5-[4-(9H-Fluoren-9-yl)-piperazin-1-yl]-phenyl}-3-pyridin-3-yl-acrylamid,
N-{6-[4-(9H-Fluoren-9-yl)-piperazin-1-yl]-hexyl]-3-pyridin-3-yl-acrylamid,
3-Pyridin-3-yl-N-{4-[4-(1,2,3,4-tetrahydronaphthalin-1-yl)-piperazin-1-yl]-butyl}-acrylamid,
3-Pyridin-3-yl-N-{4-[4-(5,6,7,8-tetrahydronaphthalin-1-yl)-piperazin-1-yl]-butyl}-acrylamid,
N-{4-[4-(Naphthalin-1-yl)-piperazin-1-yl]-butyl}-3-pyridin-3-yl-acrylamid,
N-[4-(4-Biphenyl-2-yl-piperazin-1-yl)-butyl]-3-pyridin-3-yl-propionamid,
N-[5-(4-Biphenyl-2-yl-piperazin-1-yl)-pentyl]-3-pyridin-3-yl-acrylamid,
N-[6-(4-Biphenyl-2-yl-piperazin-1-yl)-hexyl]-3-pyridin-3-yl-acrylamid,
N-[4-(4-Biphenyl-2-yl-piperazin-1-yl)-butyl]-2-(pyridin-3-yloxy)-acetamid,
N-[4-(4-Biphenyl-2-yl-piperazin-1-yl)-butyl]-5-(pyridin-3-yl)-penta-2,4-diensäureamid,
N-{4-[4-(10,11-Dihydro-5H-dibenzo[a,d]cyclohepten-5-yl)-piperazin-1-yl]-butyl}-3-pyridin-3-yl-propionamid,
N-{5-[4-(10,11-Dihydro-5H-dibenzo[a,d]cyclohepten-5-yl)-piperazin-1-yl]-pentyl}-3-pyridin-3-yl-acrylamid,
N-{6-[4-(10,11-Dihydro-5H-dibenzo[a,d]cyclohepten-5-yl)-piperazin-1-yl]-hexyl}-3-pyridin-3-yl-acrylamid,
N-{4-[4-(10,11-Dihydro-5H-dibenzo[a,d]cyclohepten-5-yl)-piperazin-1-yl]-butyl}-5-(pyridin-3-yl)-penta-2,4-diensäureamid,
N-{4-[4-(6,11-Dihydrodibenzo[b,e]oxepin-11-yl)-piperazin-1-yl]-butyl-3-pyridin-3-yl-propionamid,
N-{2-[4-(6,11-Dihydrodibenzo[b,e]thiepin-11-yl)-piperazin-1-yl]-ethyl}-3-pyridin-3-yl-acrylamid,
N-[4-(4-Diphenylacetyl-piperazin-1-yl)-butyl]-3-pyridin-3-yl-acrylamid,
N-[4-(4-Benzoylpiperazin-1-yl)-butyl]-3-pyridin-3-yl-acrylamid,
N-{4-[4-(2-Aminobenzoyl)-piperazin-1-yl]-butyl}-3-pyridin-3-yl-acrylamid,
N-{4-[4-(4-Carboxybenzoyl)-piperazin-1-yl]-butyl}-3-pyridin-3-yl-acrylamid,
N-{4-[4-(Biphenyl-2-carbonyl)-piperazin-1-yl]-butyl}-3-pyridin-3-yl-acrylamid,
N-{4-[4-(9-Oxo-9H-fluor-4-carbonyl)-piperazin-1-yl]-butyl}-3-pyridin-3-yl-acrylamid,
N-{4-[4-(Furan-2-carbonyl)-piperazin-1-yl]-butyl}-3-pyridin-3-yl-acrylamid,
N-{4-[4-(Naphthalin-1-yl-aminocarbonyl)-piperazin-1-yl]-butyl}-3-pyridin-3-yl-propionamid,
N-{4-[4-(Diphenylaminocarbonyl)-piperazin-1-yl]-butyl}-3-pyridin-3-yl-acrylamid,
N-{4-[4-(Naphthalin-2-sulfonyl)-piperazin-1-yl]-butyl}-3-pyridin-3-yl-acrylamid,
N-[4-(4-Diphenylphosphinoyl-piperazin-1-yl)-butyl]-3-pyridin-3-yl-acrylamid,
N-[4-(4-Biphenyl-2-yl-piperazin-1-yl)-butyl]-3-pyridin-3-yl-acrylamid,
N-{4-[4-(9H-Fluoren-9-yl)-piperazin-1-yl]-butyl}-3-pyridin-3-yl-acrylamid und
N-{4-[4-(10,11-Dihydro-5H-dibenzo[a,d]cyclohepten-5-yl)-piperazin-1-yl]-butyl}-3-pyridin-3-yl-acrylamid.

7. Verbindung gemäss einem der Ansprüche 2 bis 6, die vorhanden ist als
cis- und/oder trans-Isomer, E- und/oder Z-Isomer, reines Isomer und/oder Mischung von Isomeren, als Enantiomer und/oder Diastereomer, als racemische oder nicht-racemische Mischung, als endo/exo-Isomer im Fall, dass das Ringsystem E bicyclisch ist,
als Tautomer, wenn G ein heterocyclischer aromatischer Ring ist, oder dieses in einem kondensierten Ringsystem enthält, wenn dieser heterocyclische Ring durch freie Hydroxy-, Mercapto- oder Aminogruppen substituiert ist,
als pharmakologisch annehmbares Säureadditionssalz mit anorganischen oder organischen Säuren, wie Hydrochloriden, Hydrobromiden, Hydroiodiden, Sulfaten, Phosphaten, Acetaten, Benzoaten, Citraten, Fumaraten, Malaten, Maleaten, Methansulfonaten, Lactaten, Oxalaten, Succinaten, Tartraten und/oder Tosylaten, und/oder
als Hydrat oder anderes Solvat.

8. Verfahren zur Herstellung einer Verbindung gemäss einem der Ansprüche 1 bis 7, worin das Verfahren ausgewählt ist aus den folgenden (A), (B), (B1), (B2), (B3) und (B4) :
(A) Carbonsäuren der Formel (II): 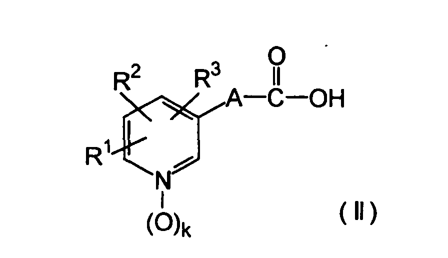 worin R¹, R², R³, A und k die Bedeutungen gemäss einem der Ansprüche 1 bis 7 aufweisen, oder ihre reaktiven Derivate, insbesondere ihre Säurechloride oder aktivierten Ester, werden gegebenenfalls in Gegenwart von Kondensationsmitteln mit Verbindungen der Formel (III) umgesetzt: 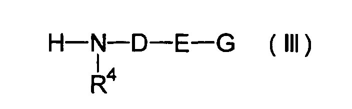 worin D, E, G und R⁴ wie in den Ansprüchen 1 bis 7 definiert sind, in Form der entsprechenden freien Base oder des entsprechenden Säureadditionssalzes, vorzugsweise in einem oder mehreren inerten Lösungsmitteln bei einer Temperatur zwischen -40 und 180°C, gegebenenfalls in Gegenwart einer Hilfsbase;
(B) Verbindungen der Formel (I), worin G Wasserstoff ist, werden mit einer Verbindung der Formel (IV) umgesetzt:
L―G (IV)
worin G die Bedeutung gemäss einem der Ansprüche 1 bis 7, mit der Ausnahme von Wasserstoff, aufweist und L eine geeignete Abgangsgruppe oder reaktive Gruppe ist;
(B1) Verbindungen der Formel (I), worin G Wasserstoff ist, werden mit einem geeigneten Alkylierungsmittel und/oder Arylierungsmittel der Formel (IV) umgesetzt:
L―G (IV)
worin G ein Alkyl-, Alkenyl-, Alkinyl-, Cycloalkyl-, Aryl-, Aralkyl-, Heteroaryl- oder Heteroaralkylrest ist und die Abgangsgruppe L ein reaktives Derivat eines Alkohols darstellt, ausgewählt aus einem Halogenatom, wie Chlor, Brom oder Iod, einem Sulfonsäureester, einer Methansulfonyloxygruppe, einer Trifluormethansulfonyloxygruppe, einer Ethansulfonyloxygruppe, einer Benzolsulfonyloxygruppe, einer p-Toluolsulfonyloxygruppe, einer p-Brombenzolsulfonyloxygruppe, einer m-Nitrobenzolsulfonyloxygruppe und einer terminalen Epoxidgruppe,
worin diese Reaktion abhängig von der Reaktion des Edukts in einem geeigneten inerten Lösungsmittel bei einer Temperatur zwischen 0 und 180°C durchgeführt wird;
(B2) Verbindungen der Formel (I), worin G Wasserstoff darstellt, werden mit einer Carbonsäure, Carbaminsäure, Sulfonsäure und/oder Phosphinsäure der Formel (V) umgesetzt:
HO―G (V)
worin G ein Acylrest, Carbamoylrest, Sulfonylrest oder Phosphinoylrest ist, oder ihren zur Reaktion geeigneten Derivaten, wobei die Reaktion vorzugsweise in Gegenwart von Hilfsbasen in Lösungsmitteln und unter den in Verfahren (A) beschriebenen Bedingungen durchgeführt wird;
(B3) Verbindungen der Formel (I), worin G Wasserstoff ist, werden mit einem Carbonylgruppenüberträger zu einem Zwischenprodukt und anschliessend mit einem primären oder sekundären Amin der Formel (VI) umgesetzt:
H―NR¹³R¹⁵ (VI)
worin R¹³ und R¹⁵ und/oder die Gruppe -NR¹³R¹⁵ die Bedeutungen gemäss einem der Ansprüche 1 bis 7 besitzen, ohne Reinigung oder Isolierung des Zwischenprodukts, vorzugsweise wird die Verbindung (VI) in einer stöchiometrischen Menge oder in einem Überschuss als Lösung oder Feststoff zugegeben, und die Reaktion ist abgeschlossen, worin die Reaktionstemperaturen für die erste Teilreaktion zwischen -40 und 50°C und für die zweite Teilreaktion zwischen 0 und 150°C liegen;
(B4) eine Verbindung der Formel (I) gemäss einem der Ansprüche 1 bis 7, worin G Wasserstoff ist, wird mit einem Isocyanat der Formel (VII) umgesetzt:
O=C=N―R¹³ (VII)
worin R¹³ die Bedeutung gemäss einem der Ansprüche 1 bis 7 aufweist, in einem absoluten, inerten Lösungsmittel bei einer Temperatur von -20 bis 150°C.

9. Pharmazeutische Zusammensetzung, umfassend eine oder mehrere der Verbindungen gemäss einem der Ansprüche 1 bis 7, gegebenenfalls zusammen mit (einem) pharmazeutisch annehmbaren Träger(n), (einem) toxikologisch unbedenklichen Hilfsstoff(en) und/oder in Kombination mit anderen Wirkstoffen.

10. Pharmazeutische Zusammensetzung gemäss Anspruch 9, die vorliegt in einer festen, peroral verabreichbaren Form als Tablette, Kapsel, beschichtete Tablette oder als Flüssigkeit; peroral verabreichbare Lösung, Suspension, Brausetablette, in Form von Tabs oder Sachets, gegebenenfalls in retardierter Form und/oder in magensaftresistenter Form,
in Form einer geeigneten Injektions- oder Infusionszubereitung, zusammen mit geeigneten, pharmazeutisch annehmbaren Trägern und Hilfsstoffen, gegebenenfalls in retardierter Form und/oder als parenterale Depotarzneiform oder Implantat oder in Form eines Konzentrats, Pulvers oder Lyophilisats,
in Form eines transdermalen therapeutischen Systems zur systemischen Behandlung,
in Form eines gastrointestinalen therapeutischen Systems (GITS) zur systemischen Behandlung.
in Form einer Salbe, Suspension, Emulsion, eines Balsams oder Pflasters oder in Form einer äusserlich applizierbaren Lösung,
in Form einer rektal, genital oder transurethral verabreichbaren Emulsion, Lösung, liposomalen Lösung, eines Implantats, Suppositoriums oder einer Kapsel,
in Form einer Zusammensetzung, die nasal, otologisch oder ophthalmologisch applizierbar ist oder
in bukkal applizierbarer Form.

11. Pharmazeutische Zusammensetzung gemäss Anspruch 9, die in Form eines Inhalationstherapeutikums, gegebenenfalls in Form eines Sprays, zusammen mit geeigneten, pharmazeutisch annehmbaren Treibmitteln, Trägern und Hilfsstoffen vorliegt.

12. Pharmazeutische Zusammensetzung gemäss Anspruch 11 zur Verabreichung mittels Dosieraerosol oder in Form einer Trockenpulverdosierformulierung.

13. Pharmazeutische Zusammensetzung gemäss einem der Ansprüche 9 bis 12, worin eine Dosiseinheit zur Einzelverabreichung 0,001 bis 2,0 mg einer oder mehrerer der Verbindungen gemäss einem der Ansprüche 1 bis 7 enthält.

14. Pharmazeutische Zusammensetzung gemäss einem der Ansprüche 9 bis 12, worin eine Dosiseinheit zur Einzelverabreichung 0,1, 1, 2, 5, 10, 20, 25, 30, 50, 100, 200, 300, 500, 600, 800, 1.000, 2.000, 3.000, 4.000 oder 5.000 mg einer oder mehrerer der Verbindungen gemäss einem der Ansprüche 1 bis 7 enthält.

15. Verwendung einer oder mehrerer Verbindungen gemäss Formel (I) 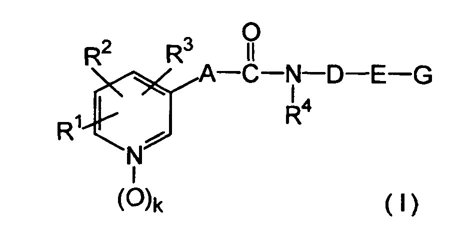 zur Herstellung einer pharmazeutischen Zusammensetzung zur Behandlung von Krebs und/oder abnormem Zellwachstum und/oder zur Prävention der Proliferation und/oder der Bildung von Metastasen im menschlichen oder tierischen Körper, gegebenenfalls in Kombination mit einem weiteren Cytostatikum oder Immunsuppressivum, worin
R¹ ausgewählt ist aus
Wasserstoff, Halogen, Cyano, C₁₋₆-Alkyl, C₃₋₆-Alkenyl, C₂₋₆-Alkinyl, Trifluormethyl, C₃₋₈-Cycloalkyl, C₁₋₆-Hydroxyalkyl, Hydroxy, C₁₋₆-Alkoxy, C₃₋₆-Alkenyloxy, C₃₋₆-Alkinyloxy, Benzyloxy, C₁₋₇-Alkanoyloxy, C₂₋₇-Alkoxycarbonyloxy, C₁₋₆-Alkylthio, C₃₋₆-Alkenylthio, C₃₋₆-Alkinylthio, C₃₋₈-Cycloalkyloxy, C₃₋₈-Cycloalkylthio, C₂₋₇-Alkoxycarbonyl, Aminocarbonyl, C₂₋₇-Alkylaminocarbonyl, C₃₋₁₃-Dialkylaminocarbonyl, Carboxy, Phenyl, Phenoxy, Phenylthio, Pyridyloxy, Pyridylthio und NR⁵R⁶, worin
R⁵ und R⁶ unabhängig voneinander ausgewählt sind aus
Wasserstoff, C₁₋₆-Alkyl, C₃₋₆-Alkenyl, C₃₋₆-Alkinyl, Benzyl und Phenyl;
R² ausgewählt ist aus
Wasserstoff, Halogen, Cyano, C₁₋₆-Alkyl, Trifluormethyl, Hydroxy, C₁₋₆-Alkoxy, Benzyloxy und C₁₋₇-Alkanoyloxy; oder
R¹ und R², falls benachbart, bilden gegebenenfalls eine Brücke, ausgewählt aus -(CH₂)₄-, -(CH=CH)₂- und -CH₂O-CR⁷R⁸-O-, worin
R⁷ und R⁸ unabhängig voneinander ausgewählt sind aus Wasserstoff oder C₁₋₆-Alkyl;
R³ ausgewählt ist aus
Wasserstoff, Halogen, C₁₋₆-Alkyl, Trifluormethyl und C₁₋₆-Hydroxyalkyl;
R⁴ ausgewählt ist aus
Wasserstoff, C₁₋₆-Alkyl, C₃₋₆-Alkenyl, C₃₋₆-Alkinyl, C₃₋₆-Cycloalkyl, Hydroxy, C₁₋₆-Alkoxy und Benzyloxy;
k 0 oder 1 ist;
A ausgewählt ist aus
C₁₋₆-Alkylen, gegebenenfalls ein- bis dreimal substituiert durch C₁₋₃-Alkyl, Hydroxy, C₁₋₃-Alkoxy, Fluor oder Phenyl,
C₂₋₆-Alkylen, worin eine Methyleneinheit isosterisch ersetzt ist durch O, S, NR⁹, CO, SO oder SO₂, worin, mit Ausnahme von CO, die isosterische Substitution nicht benachbart zur Amidgruppe ist und R⁹ ausgewählt ist aus Wasserstoff, C₁₋₆-Alkyl, C₃₋₆-Alkenyl, C₃₋₆-Alkinyl, C₁₋₆-Acyl und C₁₋₆-Alkansulfonyl,
1,2-Cyclopropylen,
C₂₋₆-Alkenylen, gegebenenfalls ein- bis dreimal substituiert durch C₁₋₃-Alkyl, Hydroxy, C₁₋₃-Alkoxy, Fluor, Cyano oder Phenyl,
C₄₋₆-Alkadienylen, gegebenenfalls ein- oder zweimal substituiert durch C₁₋₃-Alkyl, Fluor, Cyano oder Phenyl,
1,3,5-Hexatrienylen, gegebenenfalls substituiert durch C₁₋₃-Alkyl, Fluor, Cyano oder Phenyl, und
Ethinylen;
D ausgewählt ist aus
C₂₋₁₀-Alkylen, gegebenenfalls ein- oder zweimal substituiert durch C₁₋₆-Alkyl, Hydroxy oder C₁₋₆-Alkoxy,
C₄₋₁₀-Alkenylen, gegebenenfalls ein- oder zweimal substituiert durch C₁₋₆-Alkyl, Hydroxy oder C₁₋₆-Alkoxy,
C₄₋₁₀-Alkinylen, gegebenenfalls ein- oder zweimal substituiert durch C₁₋₆-Alkyl, Hydroxy oder C₁₋₆-Alkoxy, und
der Gruppe, bestehend aus C₂₋₁₀-Alkylen, C₄₋₁₀-Alkenylen und C₄₋₁₀-Alkinylen, worin ein bis drei Methyleneinheiten isosterisch ersetzt sind durch O, S, NR¹⁰, CO, SO oder SO₂, worin
R¹⁰ die gleiche Bedeutung wie R⁹ aufweist, aber unabhängig davon ausgewählt ist;
E darstellt: 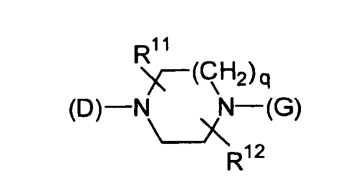 worin:
q 1, 2 oder 3 ist;
R¹¹ ausgewählt ist aus
Wasserstoff, C₁₋₆-Alkyl, Hydroxy, Hydroxymethyl, Carboxy und C₂₋₇-Alkoxycarbonyl, und
R¹² ausgewählt ist aus
Wasserstoff, C₁₋₆-Alkyl und einer zu einem Stickstoffatom benachbarten Oxogruppe,
oder
R¹¹ und R¹² bilden zusammen eine C₁₋₃-Alkylenbrücke unter Bildung eines bicyclischen Ringsystems;
G ist ausgewählt aus G1, G2, G3, G4 und G5, worin
G1 darstellt: 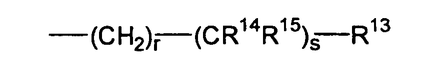
r 0, 1, 2 oder 3 ist;
s 0 oder 1 ist;
R¹³ ausgewählt ist aus
Wasserstoff, C₁₋₆-Alkyl, C₃₋₆-Alkenyl, C₃₋₆-Alkinyl, C₃₋₈-Cycloalkyl,
der Gruppe bestehend aus gesättigten und ungesättigten, 4- bis 8-gliedrigen Heterocyclen, die gegebenenfalls ein oder zwei Heteroatome, ausgewählt aus N, S und O, enthalten,
Benzyl, Phenyl,
der Gruppe bestehend aus monocyclischen aromatischen 5- bis 6-gliedrigen Heterocyclen, die gegebenenfalls ein bis drei Heteroatome enthalten, ausgewählt aus N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht,
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome ausgewählt sind aus N, S und O, und die Verknüpfung entweder über einen aromatischen Ring oder einen hydrierten Ring und entweder direkt oder über eine Methylengruppe geschieht;
R¹⁴ die gleiche Bedeutung wie R¹³ hat, aber unabhängig davon ausgewählt ist;
R¹⁵ ausgewählt ist aus
Wasserstoff, Hydroxy, Methyl, Benzyl, Phenyl,
der Gruppe bestehend aus monocyclischen aromatischen 5- bis 6-gliedrigen Heterocyclen, die ein bis drei Heteroatome enthalten, ausgewählt aus der Gruppe N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht,
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome gegebenenfalls ausgewählt sind aus N, S und O, und die Verknüpfung entweder über einen aromatischen Ring oder einen hydrierten Ring und entweder direkt oder über eine Methylengruppe geschieht;
G2 ausgewählt ist aus 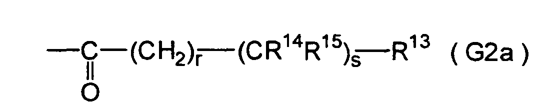 und 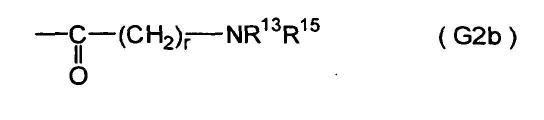 worin r, s und die Substituenten R¹³ bis R¹⁵ obige Bedeutungen aufweisen, oder die Gruppe
―NR¹³R¹⁵
einen über das Stickstoffatom gebundenen stickstoffhaltigen Heterocyclus darstellt, wobei der stickstoffhaltige Heterocyclus ausgewählt ist aus
der Gruppe bestehend aus gesättigten und ungesättigten, monocyclischen, 4- bis 8-gliedrigen Heterocyclen, die neben dem essentiellen Stickstoffatom gegebenenfalls ein oder zwei weitere Heteroatome, ausgewählt aus N, S und O, enthalten, und
der Gruppe bestehend aus gesättigten und ungesättigten, bi- oder tricyclischen, kondensierten oder verbrückten Heterocyclen mit 8 bis 16 Ringatomen, die neben dem essentiellen Stickstoffatom gegebenenfalls ein oder zwei weitere Heteroatome, ausgewählt aus N, S und O, enthalten;
G3 darstellt: 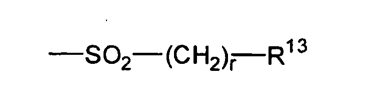 worin r und R¹³ die obigen Bedeutungen aufweisen;
G⁴ darstellt: 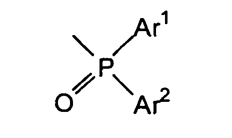 worin
Ar¹ und Ar² unabhängig voneinander ausgewählt sind aus Phenyl, Pyridyl und Naphthyl;
G5 darstellt:
―COR¹⁶ (G5)
worin
R¹⁶ ausgewählt ist aus
Trifluormethyl, C₁₋₆-Alkoxy, C₃₋₆-Alkenyloxy und Benzyloxy,
worin aromatische Ringsysteme in den Substituenten R¹, R², R⁴, R⁵, R⁶, R¹³, R¹⁴, R¹⁵, R¹⁶, Ar¹ und Ar² und/oder in dem Ringsystem -NR¹³R¹⁵ unabhängig voneinander gegebenenfalls durch ein bis drei der gleichen oder unterschiedlichen Gruppen substituiert sind, unabhängig ausgewählt aus
Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und worin
Alkyl-, Alkenyl- und Cycloalkylreste in den Gruppen G1, G2 und G3 gegebenenfalls durch ein oder zwei der gleichen oder unterschiedlichen Gruppen substituiert sind, die ausgewählt sind aus Hydroxy, Carboxy, C₂₋₇-Alkoxycarbonyl, Benzyloxycarbonyl, Amino, Mono-C₁₋₆-alkylamino und Di-(C₁₋₆-alkyl-amino);
ihre cis- und trans-Isomere, E- und Z-Isomere, Enantiomere, Diastereomere und andere Isomere sowie ihre racemischen oder nicht-racemischen Mischungen und die entsprechenden endo- und exo-Isomere für den Fall, dass das Ringsystem E bicyclisch ist;
ihre Tautomere; sowie
ihre Säureadditionssalze einschliesslich ihrer Hydrate und Solvate.

16. Verwendung gemäss Anspruch 15, worin
R¹ ausgewählt ist aus
Wasserstoff, Halogen, Cyano, C₁₋₆-Alkyl, Trifluormethyl, C₃₋₈-Cycloalkyl, C₁₋₆-Hydroxyalkyl, Hydroxy, C₁₋₄-Alkoxy, Benzyloxy, C₁₋₄-Alkylthio, C₁₋₅-Alkanoyloxy, C₂₋₅-Alkoxycarbonyl, Aminocarbonyl, C₂₋₅-Alkylaminocarbonyl, C₃₋₉-Dialkylaminocarbonyl, Carboxy, Phenyl, Phenoxy, Phenylthio, Pyridyloxy und NR⁵R⁶, worin
R⁵ und R⁶ unabhängig voneinander ausgewählt sind aus Wasserstoff und C₁₋₆-Alkyl;
R² ausgewählt ist aus
Wasserstoff, Halogen, Cyano, C₁₋₆-Alkyl, Trifluormethyl, Hydroxy und C₁₋₄-Alkoxy;
R³ ausgewählt ist aus
Wasserstoff, Halogen und C₁₋₆-Alkyl;
R⁴ ausgewählt ist aus
Wasserstoff, C₁₋₆-Alkyl, C₃₋₆-Alkenyl, C₃₋₆-Cycloalkyl, Hydroxy, C₁₋₆-Alkoxy und Benzyloxy;
k 0 oder 1 ist;
A ausgewählt ist aus
C₁₋₆-Alkylen, gegebenenfalls ein- bis dreimal substituiert durch C₁₋₃-Alkyl, Hydroxy, Fluor oder Phenyl,
C₂₋₆-Alkylen, worin eine Methyleneinheit isosterisch ersetzt ist durch O, S, NR⁹, CO, SO oder SO₂, worin, mit Ausnahme von CO, die isosterische Substitution nicht benachbart zur Amidgruppe ist und der Rest R⁹ ausgewählt ist aus Wasserstoff, C₁₋₆-Alkyl, C₁₋₆-Acyl und Methansulfonyl,
1,2-Cyclopropylen,
C₂₋₆-Alkenylen, gegebenenfalls ein- bis dreimal substituiert durch C₁₋₃-Alkyl, Hydroxy, Fluor, Cyano oder Phenyl,
C₄₋₆-Alkadienylen, gegebenenfalls ein- oder zweimal substituiert durch C₁₋₃-Alkyl, Fluor, Cyano oder Phenyl,
1,3,5-Hexatrienylen, gegebenenfalls substituiert durch C₁₋₃-Alkyl, Fluor oder Cyano, und
Ethinylen;
D ausgewählt ist aus
C₂₋₁₀-Alkylen, gegebenenfalls ein- oder zweimal substituiert durch C₁₋₃-Alkyl oder Hydroxy,
C₄₋₁₀-Alkenylen, gegebenenfalls ein- oder zweimal substituiert durch C₁₋₃-Alkyl oder Hydroxy,
C₄₋₁₀-Alkinylen, gegebenenfalls ein- oder zweimal substituiert durch C₁₋₃-Alkyl oder Hydroxy, und
der Gruppe, bestehend aus C₂₋₁₀-Alkylen, C₄₋₁₀-Alkenylen und C₄₋₁₀-Alkinylen, worin ein bis drei Methyleneinheiten isosterisch ersetzt sind durch O, S, NR¹⁰, CO, SO oder SO₂, worin
R¹⁰ die gleiche Bedeutung wie R⁹ aufweist, aber unabhängig davon ausgewählt ist;
E darstellt: 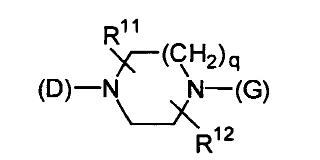 worin:
q 1, 2 oder 3 ist;
R¹¹ ausgewählt ist aus
Wasserstoff, C₁₋₃-Alkyl, Hydroxy, Hydroxymethyl, Carboxy und C₂₋₇-Alkoxycarbonyl; und
R¹² ausgewählt ist aus
Wasserstoff und einer zu einem Stickstoffatom benachbarten Oxogruppe,
oder
R¹¹ und R¹² bilden zusammen eine C₁₋₃-Alkylenbrücke unter Bildung eines bicyclischen Ringsystems;
G ist ausgewählt aus G1, G2, G3, G4 und G5, worin
G1 darstellt: 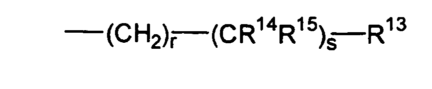
r 0, 1 oder 2 ist;
s 0 oder 1 ist;
R¹³ ausgewählt ist aus
Wasserstoff, C₁₋₆-Alkyl, C₃₋₆-Alkenyl, C₃₋₆-Alkinyl, C₃₋₈-Cycloalkyl, Benzyl, Phenyl,
der Gruppe bestehend aus monocyclischen aromatischen 5- bis 6-gliedrigen Heterocyclen, die ein bis drei Heteroatome enthalten, ausgewählt aus N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht,
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome gegebenenfalls ausgewählt sind aus N, S und O, und die Bindung entweder über einen aromatischen Ring oder einen hydrierten Ring und entweder direkt oder über eine Methylengruppe geschieht;
R¹⁴ die gleiche Bedeutung wie R¹³ hat, aber unabhängig davon ausgewählt ist;
R¹⁵ ausgewählt ist aus
Wasserstoff, Hydroxy, Methyl, Benzyl, Phenyl,
der Gruppe bestehend aus monocyclischen aromatischen 5- bis 6-gliedrigen Heterocyclen, die ein bis drei Heteroatome enthalten, ausgewählt aus N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht, und
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome gegebenenfalls ausgewählt sind aus N, S und O, und die Verknüpfung entweder über einen aromatischen Ring oder einen hydrierten Ring und entweder direkt oder über eine Methylengruppe geschieht;
G2 ausgewählt ist aus 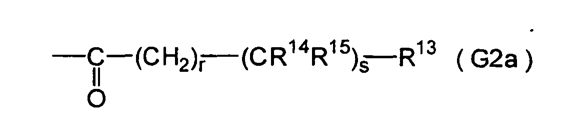 und 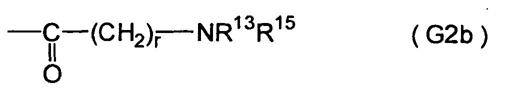 worin r, s und die Substituenten R¹³ bis R¹⁵ obige Bedeutungen aufweisen, oder die Gruppe
―NR¹³R¹⁵
ist ein über das Stickstoffatom gebundener stickstoffhaltiger Heterocyclus, wobei der stickstoffhaltige Heterocyclus ausgewählt ist aus
der Gruppe bestehend aus gesättigten und ungesättigten, monocyclischen, 4- bis 8-gliedrigen Heterocyclen, die neben dem essentiellen Stickstoffatom gegebenenfalls ein oder zwei weitere Heteroatome, ausgewählt aus N, S und O, enthalten, und
der Gruppe bestehend aus gesättigten und ungesättigten, bi- oder tricyclischen, kondensierten oder verbrückten Heterocyclen mit 8 bis 16 Ringatomen, die neben dem essentiellen Stickstoffatom gegebenenfalls ein oder zwei weitere Heteroatome, ausgewählt aus N, S und O, enthalten;
G3 darstellt: 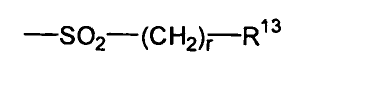 worin r und R¹³ die obigen Bedeutungen aufweisen;
G⁴ darstellt: 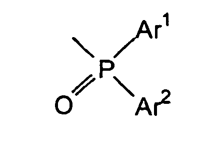 worin
Ar¹ und Ar² unabhängig voneinander ausgewählt sind aus Phenyl, Pyridyl und Naphthyl;
G5 darstellt:
―COR¹⁶
worin
R¹⁶ ausgewählt ist aus
Trifluormethyl, C₁₋₆-Alkoxy, C₃₋₆-Alkenyloxy und Benzyloxy,
worin aromatische Ringsysteme in den Substituenten R¹, R², R⁴, R⁵, R⁶, R¹³, R¹⁴, R¹⁵, R¹⁶, Ar¹ und Ar² und/oder in dem Ringsystem -NR¹³R¹⁵ unabhängig voneinander gegebenenfalls durch ein bis drei der gleichen oder unterschiedlichen Gruppen substituiert sind, ausgewählt aus
Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid,
worin Alkyl-, Alkenyl- und Cycloalkylreste in den Gruppen G1, G2 und G3 gegebenenfalls durch ein oder zwei der gleichen oder unterschiedlichen Gruppen substituiert sind, die ausgewählt sind aus
Hydroxy, Carboxy, C₂₋₇-Alkoxycarbonyl, Benzyloxycarbonyl, Amino, Mono-C₁₋₆-alkylamino und Di-(C₁₋₆-alkyl-amino).

17. Verwendung gemäss Anspruch 15 oder 16, worin
R¹ ausgewählt ist aus
Wasserstoff, Halogen, Cyano, Methyl, Ethyl, Trifluormethyl, Hydroxy, C₁₋₄-Alkoxy, Benzyloxy, C₁₋₅-Alkanoyloxy, Methylthio, Ethylthio, Methoxycarbonyl, tert-Butoxycarbonyl, Aminocarbonyl, Carboxy, Phenoxy und Phenylthio;
R² ausgewählt ist aus
Wasserstoff, Halogen, Trifluormethyl und Hydroxy;
R³ ausgewählt ist aus
Wasserstoff und Halogen;
R⁴ ausgewählt ist aus
Wasserstoff, C₁₋₃-Alkyl, Allyl, Hydroxy und C₁₋₃-Alkoxy;
k 0 oder 1 ist;
A ausgewählt ist aus
C₁₋₆-Alkylen, gegebenenfalls ein- oder zweimal substituiert durch C₁₋₃-Alkyl, Hydroxy oder Fluor,
C₂₋₆-Alkylen, worin eine Methyleneinheit isosterisch ersetzt ist durch O, S, NR⁹, CO, SO oder SO₂, worin, mit Ausnahme von CO, die isosterische Substitution nicht benachbart zur Amidgruppe ist,
C₂₋₆-Alkenylen, gegebenenfalls ein- oder zweimal substituiert durch C₁₋₃-Alkyl, Hydroxy und/oder Fluor,
C₄₋₆-Alkadienylen, gegebenenfalls durch C₁₋₃-Alkyl oder ein oder zwei Fluoratome substituiert, und
1,3,5-Hexatrienylen, gegebenenfalls durch Fluor substituiert;
D ausgewählt ist aus
C₂₋₈-Alkylen, gegebenenfalls ein- oder zweimal substituiert durch Methyl oder Hydroxy,
C₄₋₈-Alkenylen, gegebenenfalls ein- oder zweimal substituiert durch Methyl oder Hydroxy,
C₄₋₈-Alkinylen, gegebenenfalls ein- oder zweimal substituiert durch Methyl oder Hydroxy, und
der Gruppe, bestehend aus C₂₋₈-Alkylen, C₄₋₈-Alkenylen und C₄₋₈-Alkinylen, worin ein bis drei Methyleneinheiten jeweils isosterisch ersetzt sind durch O, S, NH, N(CH₃), N(COCH₃), N(SO₂CH₃), CO, SO oder SO₂,
E darstellt: 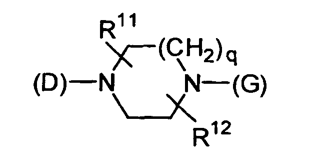 worin:
q 1 oder 2 ist;
R¹¹ ausgewählt ist aus
Wasserstoff, C₁₋₃-Alkyl, Hydroxymethyl und Carboxy; und
R¹² ausgewählt ist aus
Wasserstoff und einer zu einem Stickstoffatom benachbarten Oxogruppe;
G ausgewählt ist aus G1, G2, G3, G4 und G5, worin
G1 darstellt: 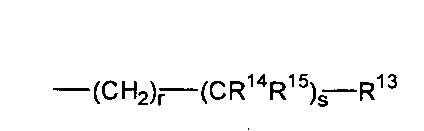
r 0, 1 oder 2 ist;
s 0 oder 1 ist;
R¹³ ausgewählt ist aus
Wasserstoff, C₁₋₆-Alkyl, C₃₋₈-Cycloalkyl, Benzyl, Phenyl,
der Gruppe bestehend aus Benzocyclobutyl, Indanyl, Indenyl, Oxoindanyl, Naphthyl, Dihydronaphthyl, Tetrahydronaphthyl, Oxotetrahydronaphthyl, Biphenylenyl, Fluorenyl, Oxofluorenyl, Anthryl, Dihydroanthryl, Oxodihydroanthryl, Dioxodihydroanthryl, Phenanthryl, Dihydrophenanthryl, Oxodihydrophenanthryl, Dibenzocycloheptenyl, Oxodibenzocycloheptenyl, Dihydrodibenzocycloheptenyl, Oxodihydrodibenzocycloheptenyl, Dihydrodibenzocyclooctenyl, Tetrahydrodibenzocyclooctenyl und Oxotetrahydrodibenzocyclooctenyl, direkt oder über eine Methylengruppe gebunden; und
der Gruppe bestehend aus Furyl, Thienyl, Pyrrolyl, Oxazolyl, Isoxazolyl, Thiazolyl, Isothiazolyl, Pyrazolyl, Imidazolyl, Oxadiazolyl, Thiadiazolyl, Triazolyl, Pyridyl, Pyrazinyl, Pyridazinyl, Pyrimidinyl, Triazinyl, Imidazothiazolyl, Benzofuryl, Dihydrobenzofuryl, Benzothienyl, Dihydrobenzothienyl, Indolyl, Indolinyl, Isoindolinyl, Oxoindolinyl, Dioxoindolinyl, Benzoxazolyl, Oxobenzooxazolinyl, Benzoisooxazolyl, Oxobenzoisooxazolinyl, Benzothiazolyl, Oxobenzothiazolinyl, Benzoisothiazolyl, Oxobenzoisothiazolinyl, Benzoimidazolyl, Oxobenzoimidazolinyl, Indazolyl, Oxoindazolinyl, Benzofurazanyl, Benzothiadiazolyl, Benzotriazolyl, Oxazolopyridyl, Oxodihydrooxazolopyridyl, Thiazolopyridyl, Oxodihydrothiazolopyridyl, Isothiazolopyridyl, Imidazopyridyl, Oxodihydroimidazopyridyl, Pyrazolopyridyl, Oxodihydropyrazolopyridyl, Thienopyrimidinyl, Chromanyl, Chromanonyl, Benzopyranyl, Chromonyl, Chinoloyl, Isochinoloyl, Dihydrochinoloyl, Oxodihydrochinoloyl, Tetrahydrochinoloyl, Oxotetrahydrochinolinyl, Benzodioxanyl, Chinoxalinyl, Chinazolinyl, Naphthyridinyl, Carbazolyl, Tetrahydrocarbazolyl, Oxotetrahydrocarbazolyl, Pyridoindolyl, Acridinyl, Oxodihydroacridinyl, Phenanthridinyl, Dihydrophenanthridinyl, Oxodihydrophenanthridinyl, Dibenzoisochinolinyl, Dihydrodibenzoisochinolinyl, Oxodihydrodibenzoisochinolinyl, Phenothiazinyl, Dihydrodibenzooxepinyl, Oxodihydrodibenzooxepinyl, Benzocycloheptathienyl, Oxobenzocycloheptathienyl, Dihydrothienobenzothiepinyl, Oxodihydrothienobenzothiepinyl, Dihydrodibenzothiepinyl, Oxodihydrodibenzothiepinyl, Octahydrodibenzothiepinyl, Dibenzoazepinyl, Dihydrodibenzoazepinyl, Oxodihydrodibenzoazepinyl, Octahydrodibenzoazepinyl, Benzocycloheptapyridyl, Oxobenzocycloheptapyridyl, Pyridobenzoazepinyl, Dihydropyridobenzoazepinyl, Oxodihydropyridobenzoazepinyl, Dihydropyridobenzodiazepinyl, Dihydrodibenzooxazepinyl, Dihydropyridobenzooxepinyl, Dihydropyridobenzooxazepinyl, Oxodihydropyridobenzooxazepinyl, Dihydrodibenzothiazepinyl, Oxodihydrodibenzothiazepinyl, Dihydropyridobenzothiazepinyl und Oxodihydropyridobenzothiazepinyl, direkt oder über eine Methylengruppe gebunden;
R¹⁴ die gleiche Bedeutung wie R¹³ hat, aber unabhängig davon ausgewählt ist;
R¹⁵ ausgewählt ist aus
Hydroxy, Methyl, Benzyl, Phenyl, Indanyl, Indenyl, Naphthyl, Dihydronaphthyl, Tetrahydronaphthyl, Furyl, Thienyl, Pyrrolyl, Oxazolyl, Isoxazolyl, Thiazolyl, Isothiazolyl, Pyrazolyl, Imidazolyl, Oxadiazolyl, Thiadiazolyl, Triazolyl, Pyridyl, Pyrazinyl, Pyridazinyl, Pyrimidinyl, Triazinyl, Benzofuryl, Benzothienyl, Indolyl, Indolinyl, Benzooxazolyl, Benzothiazolyl, Benzoimidazolyl, Chromanyl, Chinolyl und Tetrahydrochinolyl, direkt oder über eine Methylengruppe gebunden,
G2 ausgewählt ist aus 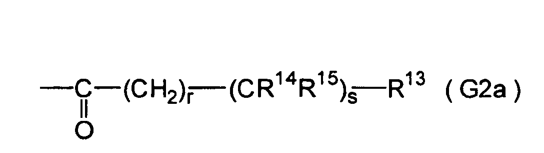 und 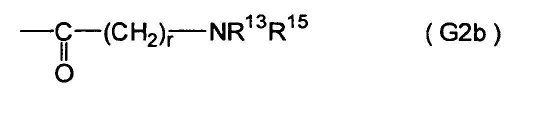 worin r, s und die Substituenten R¹³ bis R¹⁵ obige Bedeutungen aufweisen oder die Gruppe
―NR¹³R¹⁵
darstellt: Azetidin, Pyrrolidin, Piperidin, (1H)-Tetrahydropyridin, Hexahydroazepin, (1H)-Tetrahydroazepin, Octahydroazocin, Pyrazolidin, Piperazin, Hexahydrodiazepin, Morpholin, Hexahydrooxazepin, Thiomorpholin, Thiomorpholin-1,1-dioxid, 5-Aza-bicyclo[2.1.1]hexan, 2-Aza-bicyclo[2.2.1]heptan, 7-Aza-bicyclo[2.2.1]heptan, 2,5-Diaza-bicyclo[2.2.1]heptan, 2-Aza-bicyclo[2.2.2]octan, 8-Aza-bicyclo[3.2.1]octan, 2,5-Diaza-bicyclo[2.2.2]octan, 9-Aza-bicyclo[3.3.1]nonan, Indolin, Isoindolin, (1H)-Dihydrochinolin, (1H)-Tetrahydrochinolin, (2H)-Tetrahydroisochinolidin, (1H)-Tetrahydrochinoxalin, (4H)-Dihydrobenzooxazin, (4H)-Dihydrobenzothiazin, (1H)-Tetrahydrobenzo[b]azepin, (1H)-Tetrahydrobenzo[c]azepin, (1H)-Tetrahydrobenzo[d]azepin, (5H)-Tetrahydrobenzo[b]oxazepin, (5H)-Tetrahydrobenzo[b]thiazepin, 1,2,3,4-Tetrahydro-9H-pyrido[3,4-b]indol, (10H)-Dihydroacridin, (10H)-Dihydrophenanthridin, 1,2,3,4-Tetrahydroacridanon, (10H)-Phenoxazin, (10H)-Phenothiazin, (5H)-Dibenzoazepin, (5H)-Dihydrodibenzoazepin, (5H)-Octahydrodibenzoazepin, Dihydrobenzo[d,e]isochinolin, (5H)-Dihydrodibenzodiazepin, (5H)-Benzo[b]pyrido[f]azepin, (5H)-Dihydrodibenzo[b]pyrido[f]azepin, (11H)-Dihydrodibenzo[b,e]oxazepin, (11H)-Dihydrodibenzo[b,e]thiazepin, (10H)-Dihydrodibenzo[b,f]oxazepin, (10H)-Dihydrodibenzo[b,f]thiazepin, (5H)-Tetrahydrodibenzoazoacin, (11H)-Dihydrobenzo[e]pyrido[b]-1,4-diazepin-6-on oder (11H)-Dihydrobenzo[b]pyrido[e]-1,4-diazepin-5-on;
G3 darstellt: 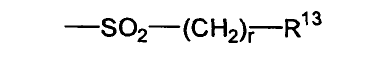 worin r und R¹³ die obigen Bedeutungen aufweisen;
G⁴ darstellt: 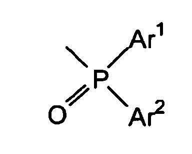 worin
Ar¹ und Ar² unabhängig voneinander ausgewählt sind aus Phenyl, Pyridyl und Naphthyl;
G5 darstellt:
―COR¹⁶
worin
R¹⁶ ausgewählt ist aus
Trifluormethyl, C₁₋₆-Alkoxy, C₃₋₆-Alkenyloxy und Benzyloxy,
worin aromatische Ringsysteme in den Substituenten unabhängig voneinander gegebenenfalls durch ein bis drei der gleichen oder unterschiedlichen Gruppen substituiert sind, ausgewählt aus
Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und
worin Alkyl-, Alkenyl- und Cycloalkylreste in den Gruppen G1, G2 und G3 gegebenenfalls durch ein oder zwei gleiche oder unterschiedliche Gruppen substituiert sind, die ausgewählt sind aus
Hydroxy, Carboxy, C₂₋₇-Alkoxycarbonyl, Benzyloxycarbonyl, Amino, Mono-C₁₋₆-alkylamino und Di-(C₁₋₆-alkyl-amino).

18. Verwendung gemäss einem der Ansprüche 15 bis 17, worin
R¹ ausgewählt ist aus
Wasserstoff, Fluor, Chlor, Brom, Methyl, Ethyl, Trifluormethyl, Hydroxy, C₁₋₄-Alkoxy, Methylthio, Ethylthio, Carboxy und Phenoxy;
R² ausgewählt ist aus Wasserstoff, Chlor und Methyl;
R³ Wasserstoff ist;
R⁴ ausgewählt ist aus Wasserstoff, C₁₋₃-Alkyl und Hydroxy;
k 0 ist;
A ausgewählt ist aus
C₂₋₆-Alkylen, gegebenenfalls ein- oder zweimal substituiert durch Hydroxy oder Fluor,
C₂₋₆-Alkylen, worin eine Methyleneinheit isosterisch ersetzt ist durch O, S oder CO, worin, mit Ausnahme von CO, die isosterische Substitution nicht benachbart zur Amidgruppe ist,
C₂₋₆-Alkenylen, gegebenenfalls ein- oder zweimal substituiert durch C₁₋₃-Alkyl und/oder Fluor, und
C₄₋₆-Alkadienylen;
D ausgewählt ist aus
C₂₋₈-Alkylen, gegebenenfalls substituiert durch Methyl oder Hydroxy,
C₄₋₈-Alkenylen, gegebenenfalls substituiert durch Hydroxy,
C₄₋₈-Alkinylen, gegebenenfalls substituiert durch Hydroxy, und
der Gruppe, bestehend aus C₂₋₈-Alkylen, C₄₋₈-Alkenylen und C₄₋₈-Alkinylen, worin eine Methyleneinheit isosterisch ersetzt ist durch O, NH, N(CH₃), CO oder SO₂, oder eine Ethylengruppe isosterisch ersetzt ist durch eine Gruppe NH-CO und/oder CO-NH, oder eine Propylengruppe isosterisch ersetzt ist durch eine Gruppe NH-CO-O und/oder O-CO-NH;
E ausgewählt ist aus Piperazin und Hexahydro-1,4-diazepin, worin der Ring gegebenenfalls durch ein oder zwei Methylengruppen und/oder durch eine zu einem Stickstoffatom benachbarte Oxogruppe substituiert ist;
G ausgewählt ist aus Wasserstoff, C₃₋₈-Cycloalkyl, Methoxycarbonyl, tert-Butoxycarbonyl, Benzyloxycarbonyl, Trifluoracetyl, Diphenylphosphinoyl, 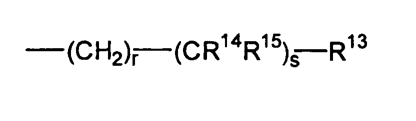 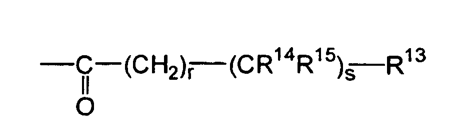 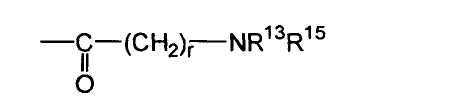 und 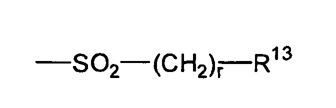 worin
r 0 oder 1 ist;
s 0 oder 1 ist;
R¹³ ausgewählt ist aus Wasserstoff, Methyl, Benzyl, Phenyl,
der Gruppe bestehend aus Indanyl, Indenyl, Oxoindanyl, Naphthyl, Tetrahydronaphthyl, Fluorenyl, Oxofluorenyl, Anthryl, Dihydroanthryl, Oxodihydroanthryl, Dioxodihydroanthryl, Phenanthryl, Dihydrophenanthryl, Oxodihydrophenanthryl, Dibenzocycloheptenyl, Dihydrodibenzocycloheptenyl und Oxodihydrodibenzocycloheptenyl, direkt oder über eine Methylengruppe gebunden; und
der Gruppe bestehend aus Furyl, Thienyl, Oxazolyl, Isooxazolyl, Thiazolyl, Imidazolyl, Oxadiazolyl, Pyridyl, Pyrazinyl, Pyrimidinyl, Imidazothiazolyl, Benzofuryl, Benzothienyl, Indolyl, Indolinyl, Oxoindolinyl, Dioxoindolinyl, Benzoxazolyl, Oxobenzooxazolinyl, Benzothiazolyl, Oxobenzothiazolinyl, Benzoimidazolyl, Oxobenzimidazolinyl, Indazolyl, Benzofurazanyl, Benzotriazolyl, Oxazolopyridyl, Oxodihydrooxazolopyridyl, Thiazolopyridyl, Oxodihydrothiazolopyridyl, Imidazopyridyl, Oxodihydroimidazopyridyl, Chromanyl, Chromanonyl, Benzopyranyl, Chromonyl, Chinolyl, Isochinolyl, Oxodihydrochinolinyl, Tetrahydrochinolyl, Oxotetrahydrochinolinyl, Benzodioxanyl, Chinazolinyl, Carbazolyl, Acridinyl, Dihydroacridinyl, Oxodihydroacridinyl, Dihydrophenanthridinyl, Dihydrodibenzoisochinolinyl, Phenothiazinyl, Dihydrodibenzooxepinyl, Benzocycloheptathienyl, Dihydrothienobenzothiepinyl, Dihydrodibenzothiepinyl, Oxodihydrodibenzothiepinyl, Dihydrodibenzoazepinyl, Oxodihydrodibenzoazepinyl, Octahydrodibenzoazepinyl, Benzocycloheptapyridyl, Dihydropyridobenzodiazepinyl und Dihydrodibenzothiazepinyl, direkt oder über eine Methylengruppe gebunden;
R¹⁴ ausgewählt ist aus Wasserstoff, Methyl, Benzyl und Phenyl;
R¹⁵ ausgewählt ist aus
Wasserstoff, Hydroxy, Methyl, Benzyl, Phenyl; und
der Gruppe bestehend aus Naphthyl, Tetrahydronaphthyl, Furyl, Thienyl, Oxazolyl, Thiazolyl, Imidazolyl, Pyridyl, Benzofuryl, Benzothienyl, Indolyl, Indolinyl, Benzoxazolyl, Benzothiazolyl, Benzoimidazolyl, Chromanyl, Chinolyl und Tetrahydrochinolyl, direkt oder über eine Methylengruppe gebunden,
worin die Gruppe
―NR¹³R¹⁵
einen über das Stickstoffatom gebundenen stickstoffhaltigen Heterocyclus darstellt, wobei der stickstoffhaltige Heterocyclus ausgewählt ist aus
Pyrrolidin, Piperid, Hexahydroazepin, Piperazin, Hexahydrodiazepin, Morpholin, Hexahydroxazepin, Thiomorpholin, 7-Aza-bicyclo[2.2.1]heptan, 2,5-Diaza-bicyclo[2.2.1]heptan, Indolin, Isoindolin, (1H)-Dihydrochinolin, (1H)-Tetrahydrochinolin, (2H)-Tetrahydroisochinolin, (4H)-Dihydrobenzoxazin, (4H)-Dihydrobenzothiazin, (1H)-Tetrahydrobenzo[b]azepin, (1H)-Tetrahydrobenzo[d]azepin, (5H)-Tetrahydrobenzo[b]oxazepin, (5H)-Tetrahydrobenzo[b]thiazepin, (10H)-Dihydroacridin, 1,2,3,4-Tetrahydroacridanon, (10H)-Dihydrophenanthridin, (1H)-Dihydrobenzo[d,e]isochinolin, (10H)-Phenothiazin, (5H)-Dibenzo[b,f]azepin, (5H)-Dihydrodibenzo[b,f]azepin, (5H)-Dihydrodibenzo[c,e]azepin, (5H)-Dihydrodibenzodiazepin, (11H)-Dihydrodibenzo[b,e]oxazepin, (11H)-Dihydrodibenzo[b,e]thiazepin, (5H)-Dihydrobenzo[b]pyrido[3,2-f]azepin und (11H)-6-Oxodihydrobenzo[e]pyrido[3,2-b][1,4]-diazepin,
worin aromatische Ringsysteme in den Substituenten unabhängig voneinander gegebenenfalls durch ein bis drei der gleichen oder unterschiedlichen Gruppen substituiert sind, ausgewählt aus Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und
worin Alkyl-, Alkenyl- und Cycloalkylreste in der Gruppe G gegebenenfalls durch ein oder zwei gleiche oder unterschiedliche Gruppen substituiert sind, die ausgewählt sind aus Hydroxy, Carboxy, C₂₋₇-Alkoxycarbonyl, Benzyloxycarbonyl, Amino, Mono-C₁₋₆-alkylamino und Di-(C₁₋₆-alkyl-amino).

19. Verwendung gemäss einem der Ansprüche 15 bis 18, worin
R¹ ausgewählt ist aus
Wasserstoff, Fluor, Methyl, Trifluormethyl und Ethylthio;
R², R³ und R⁴ jeweils Wasserstoff sind;
k 0 ist;
A ausgewählt ist aus
der Gruppe bestehend aus Ethylen, Propylen und Butylen, die jeweils gegebenenfalls durch Hydroxy oder ein oder zwei Fluoratome substituiert sind;
OCH₂, SCH₂;
Ethenylen und 1,3-Butadienylen;
D ausgewählt ist aus
C₂₋₆-Alkylen, gegebenenfalls substituiert durch Hydroxy,
C₄₋₆-Alkenylen,
C₄₋₆-Alkinylen und
der Gruppe, bestehend aus C₂₋₆-Alkylen, C₄₋₆-Alkenylen und C₄₋₆-Alkinylen, worin ein oder zwei Methyleneinheiten isosterisch ersetzt ist durch O, NH, CO oder SO₂;
E Piperazin oder Hexahydro-1,4-diazepin ist;
G ausgewählt ist aus
Phenyl, Benzyl, Phenethyl, Diphenylmethyl, Naphthyl, Tetrahydronaphthyl, Naphthylmethyl, Fluorenyl, Fluorenylmethyl, Anthrylmethyl, Dihydrodibenzocycloheptenyl, Furylmethyl, Thienylmethyl, Thiazolylmethyl, Pyridylmethyl, Benzothienylmethyl, Chinolylmethyl, Phenylthienylmethyl, Phenylpyridylmethyl, Benzocycloheptapyridinyl, Dihydrobenzocycloheptapyridinyl, Dihydrodibenzooxepinyl, Dihydrodibenzothiepinyl, Dihydrodibenzoazepinyl, Dihydrobenzopyridodiazepinyl, Formyl, Acetyl, Pivaloyl, Phenylacetyl, Diphenylacetyl, Diphenylpropionyl, Naphthylacetyl, Benzoyl, Naphthoyl, Oxofluorenylcarbonyl, Oxodihydroanthrylcarbonyl, Dioxodihydroanthrylcarbonyl, Furoyl, Pyridylacetyl, Pyridylcarbonyl, Chromonylcarbonyl, Chinolylcarbonyl, Phenylylaminocarbonyl, Naphthylaminocarbonyl, Tetrahydronaphthylaminocarbonyl, Dibenzylaminocarbonyl, Benzylphenylaminocarbonyl, Diphenylaminocarbonyl, Indolinyl-N-carbonyl, Isoindolinyl-N-carbonyl, Tetrahydrochinolinyl-N-carbonyl, Carbazolyl-N-carbonyl, Tetrahydrobenzoazepinyl-N-carbonyl, Dihydrodibenzoazepinyl-N-carbonyl, Dihydrobenzopyridoazepinyl-N-carbonyl, Oxodihydrobenzopyridoazepinyl-N-carbonyl, Methansulfonyl, Toluolsulfonyl, Naphthylsulfonyl, Chinolinsulfonyl und Diphenylphosphinoyl,
worin aromatische Ringsysteme in den Substituenten unabhängig voneinander gegebenenfalls durch ein bis drei der gleichen oder unterschiedlichen Gruppen substituiert sind, ausgewählt aus Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und worin
Alkyl-, Alkenyl- und Cycloalkylreste in der Gruppe G gegebenenfalls durch ein oder zwei gleiche oder unterschiedliche Gruppen substituiert sind, die ausgewählt sind aus
Hydroxy, Carboxy, C₂₋₇-Alkoxycarbonyl, Benzyloxycarbonyl, Amino, Mono-C₁₋₆-alkylamino und Di-(C₁₋₆-alkyl)-amino.

20. Verwendung gemäss einem der Ansprüche 15 bis 19, worin die Verbindung(en) der Formel (I) ausgewählt ist/sind aus der Gruppe bestehend aus:
N-[4-(4-Diphenylmethylpiperazin-1-yl)-3-hydroxybutyl]-3-pyridin-3-yl-acrylamid,
N-[3-(4-Diphenylmethylpiperazin-1-yl)-propoxy]-3-pyridin-3-yl-acrylamid,
N-[4-(4-Diphenylmethylpiperazin-1-yl)-4-oxo-butyl]-3-pyridin-3-yl-acrylamid,
N-[3-(4-Diphenylmethylpiperazin-1-sulfonyl)-propyl]-3-pyridin-3-yl-acrylamid,
N-{2-[2-(4-Diphenylmethylpiperazin-1-yl)-ethoxy]-ethyl}-3-pyridin-3-yl-acrylamid,
N-(4-{4-[Bis-(4-fluorphenyl)-methyl]-piperazin-1-yl}-but-2-inyl)-3-pyridin-3-yl-acrylamid,
N-{4-[4-(4-Carboxyphenyl-phenylmethyl)-piperazin-1-yl]-butyl}-3-pyridin-3-yl-acrylamid,
N-(4-{4-[(4-Aminophenyl)-phenylmethyl]-piperazin-1-yl}-butyl)-3-pyridin-3-yl-acrylamid,
N-{4-[4-(9H-Fluoren-9-yl)-piperazin-1-yl]-butyl}-2-(pyridin-3-yloxy)-acetamid,
N-{5-[4-(9H-Fluoren-9-yl)-piperazin-1-yl]-phenyl}-3-pyridin-3-yl-acrylamid,
N-{6-[4-(9H-Fluoren-9-yl)-piperazin-1-yl]-hexyl]-3-pyridin-3-yl-acrylamid,
3-Pyridin-3-yl-N-{4-[4-(1,2,3,4-tetrahydronaphthalin-1-yl)-piperazin-1-yl]-butyl}-acrylamid,
3-Pyridin-3-yl-N-{4-[4-(5,6,7,8-tetrahydronaphthalin-1-yl)-piperazin-1-yl]-butyl}-acrylamid,
N-{4-[4-(Naphthalin-1-yl)-piperazin-1-yl]-butyl}-3-pyridin-3-yl-acrylamid,
N-[4-(4-Biphenyl-2-yl-piperazin-1-yl)-butyl]-3-pyridin-3-yl-propionamid,
N-[5-(4-Biphenyl-2-yl-piperazin-1-yl)-pentyl]-3-pyridin-3-yl-acrylamid,
N-[6-(4-Biphenyl-2-yl-piperazin-1-yl)-hexyl]-3-pyridin-3-yl-acrylamid,
N-[4-(4-Biphenyl-2-yl-piperazin-1-yl)-butyl]-2-(pyridin-3-yloxy)-acetamid,
N-[4-(4-Biphenyl-2-yl-piperazin-1-yl)-butyl]-5-(pyridin-3-yl)-penta-2,4-diensäureamid,
N-{4-[4-(10,11-Dihydro-5H-dibenzo[a,d]cyclohepten-5-yl)-piperazin-1-yl]-butyl}-3-pyridin-3-yl-propionamid,
N-{5-[4-(10,11-Dihydro-5H-dibenzo[a,d]cyclohepten-5-yl)-piperazin-1-yl]-pentyl}-3-pyridin-3-yl-acrylamid,
N-{6-[4-(10,11-Dihydro-5H-dibenzo[a,d]cyclohepten-5-yl)-piperazin-1-yl]-hexyl}-3-pyridin-3-yl-acrylamid,
N-{4-[4-(10,11-Dihydro-5H-dibenzo[a,d]cyclohepten-5-yl)-piperazin-1-yl]-butyl}-5-(pyridin-3-yl)-penta-2,4-diensäureamid,
N-{4-[4-(6,11-Dihydrodibenzo[b,e]oxepin-11-yl)-piperazin-1-yl]-butyl-3-pyridin-3-yl-propionamid,
N-{2-[4-(6,11-Dihydrodibenzo[b,e]thiepin-11-yl)-piperazin-1-yl]-ethyl}-3-pyridin-3-yl-acrylamid,
N-[4-(4-Diphenylacetyl-piperazin-1-yl)-butyl]-3-pyridin-3-yl-acrylamid,
N-[4-(4-Benzoylpiperazin-1-yl)-butyl]-3-pyridin-3-yl-acrylamid,
N-{4-[4-(2-Aminobenzoyl)-piperazin-1-yl]-butyl}-3-pyridin-3-yl-acrylamid,
N-{4-[4-(4-Carboxybenzoyl)-piperazin-1-yl]-butyl}-3-pyridin-3-yl-acrylamid,
N-{4-[4-(Biphenyl-2-carbonyl)-piperazin-1-yl]-butyl}-3-pyridin-3-yl-acrylamid,
N-{4-[4-(9-Oxo-9H-fluor-4-carbonyl)-piperazin-1-yl]-butyl}-3-pyridin-3-yl-acrylamid,
N-{4-[4-(Furan-2-carbonyl)-piperazin-1-yl]-butyl}-3-pyridin-3-yl-acrylamid,
N-{4-[4-(Naphthalin-1-yl-aminocarbonyl)-piperazin-1-yl]-butyl}-3-pyridin-3-yl-propionamid,
N-{4-[4-(Diphenylaminocarbonyl)-piperazin-1-yl]-butyl}-3-pyridin-3-yl-acrylamid,
N-{4-[4-(Naphthalin-2-sulfonyl)-piperazin-1-yl]-butyl}-3-pyridin-3-yl-acrylamid,
N-[4-(4-Diphenylphosphinoyl-piperazin-1-yl)-butyl]-3-pyridin-3-yl-acrylamid,
N-[4-(4-Biphenyl-2-yl-piperazin-1-yl)-butyl]-3-pyridin-3-yl-acrylamid,
N-{4-[4-(9H-Fluoren-9-yl)-piperazin-1-yl]-butyl}-3-pyridin-3-yl-acrylamid und
N-{4-[4-(10,11-Dihydro-5H-dibenzo[a,d]cyclohepten-5-yl)-piperazin-1-yl]-butyl}-3-pyridin-3-yl-acrylamid.

21. Verwendung einer oder mehrerer Verbindungen gemäss Formel (I) 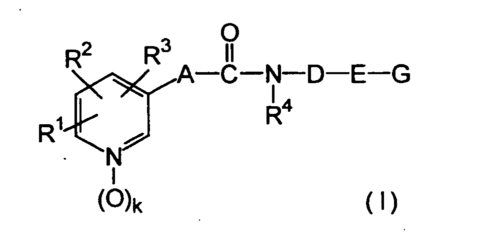 zur Herstellung einer pharmazeutischen Zusammensetzung zur Unterdrückung einer Abstossungsreaktion nach einer Organtransplantation im menschlichen oder tierischen Körper, gegebenenfalls in Kombination mit einem weiteren Cytostatikum oder Immunsuppressivum und/oder weiteren, für diese Indikationen geeigneten pharmazeutischen Zusammensetzungen, worin
R¹ ausgewählt ist aus
Wasserstoff, Halogen, Cyano, C₁₋₆-Alkyl, C₃₋₆-Alkenyl, C₂₋₆-Alkinyl, Trifluormethyl, C₃₋₈-Cycloalkyl, C₁₋₆-Hydroxyalkyl, Hydroxy, C₁₋₆-Alkoxy, C₃₋₆-Alkenyloxy, C₃₋₆-Alkinyloxy, Benzyloxy, C₁₋₇-Alkanoyloxy, C₂₋₇-Alkoxycarbonyloxy, C₁₋₆-Alkylthio, C₃₋₆-Alkenylthio, C₃₋₆-Alkinylthio, C₃₋₈-Cycloalkyloxy, C₃₋₈-Cycloalkylthio, C₂₋₇-Alkoxycarbonyl, Aminocarbonyl, C₂₋₇-Alkylaminocarbonyl, C₃₋₁₃-Dialkylaminocarbonyl, Carboxy, Phenyl, Phenoxy, Phenylthio, Pyridyloxy, Pyridylthio und NR⁵R⁶, worin
R⁵ und R⁶ unabhängig voneinander ausgewählt sind aus
Wasserstoff, C₁₋₆-Alkyl, C₃₋₆-Alkenyl, C₃₋₆-Alkinyl, Benzyl und Phenyl;
R² ausgewählt ist aus
Wasserstoff, Halogen, Cyano, C₁₋₆-Alkyl, Trifluormethyl, Hydroxy, C₁₋₆-Alkoxy, Benzyloxy und C₁₋₇-Alkanoyloxy; oder
R¹ und R², falls benachbart, bilden gegebenenfalls eine Brücke, ausgewählt aus -(CH₂)₄-, -(CH=CH)₂- und -CH₂O-CR⁷R⁸-O-, worin
R⁷ und R⁸ unabhängig voneinander ausgewählt sind aus Wasserstoff oder C₁₋₆-Alkyl;
R³ ausgewählt ist aus
Wasserstoff, Halogen, C₁₋₆-Alkyl, Trifluormethyl und C₁₋₆-Hydroxyalkyl;
R⁴ ausgewählt ist aus
Wasserstoff, C₁₋₆-Alkyl, C₃₋₆-Alkenyl, C₃₋₆-Alkinyl, C₃₋₆-Cycloalkyl, Hydroxy, C₁₋₆-Alkoxy und Benzyloxy;
k 0 oder 1 ist;
A ausgewählt ist aus
C₁₋₆-Alkylen, gegebenenfalls ein- bis dreimal substituiert durch C₁₋₃-Alkyl, Hydroxy, C₁₋₃-Alkoxy, Fluor oder Phenyl,
C₂₋₆-Alkylen, worin eine Methyleneinheit isosterisch ersetzt ist durch O, S, NR⁹, CO, SO oder SO₂, worin, mit Ausnahme von CO, die isosterische Substitution nicht benachbart zur Amidgruppe ist und R⁹ ausgewählt ist aus Wasserstoff, C₁₋₆-Alkyl, C₃₋₆-Alkenyl, C₃₋₆-Alkinyl, C₁₋₆-Acyl und C₁₋₆-Alkansulfonyl,
1,2-Cyclopropylen,
C₂₋₆-Alkenylen, gegebenenfalls ein- bis dreimal substituiert durch C₁₋₃-Alkyl, Hydroxy, C₁₋₃-Alkoxy, Fluor, Cyano oder Phenyl,
C₄₋₆-Alkadienylen, gegebenenfalls ein- oder zweimal substituiert durch C₁₋₃-Alkyl, Fluor, Cyano oder Phenyl,
1,3,5-Hexatrienylen, gegebenenfalls substituiert durch C₁₋₃-Alkyl, Fluor, Cyano oder Phenyl, und
Ethinylen;
D ausgewählt ist aus
C₂₋₁₀-Alkylen, gegebenenfalls ein- oder zweimal substituiert durch C₁₋₆-Alkyl, Hydroxy oder C₁₋₆-Alkoxy,
C₄₋₁₀-Alkenylen, gegebenenfalls ein- oder zweimal substituiert durch C₁₋₆-Alkyl, Hydroxy oder C₁₋₆-Alkoxy,
C₄₋₁₀-Alkinylen, gegebenenfalls ein- oder zweimal substituiert durch C₁₋₆-Alkyl, Hydroxy oder C₁₋₆-Alkoxy, und
der Gruppe, bestehend aus C₂₋₁₀-Alkylen, C₄₋₁₀-Alkenylen und C₄₋₁₀-Alkinylen, worin ein bis drei Methyleneinheiten isosterisch ersetzt sind durch O, S, NR¹⁰, CO, SO oder SO₂, worin
R¹⁰ die gleiche Bedeutung wie R⁹ aufweist, aber unabhängig davon ausgewählt ist;
E darstellt: 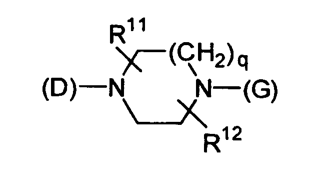 worin:
q 1, 2 oder 3 ist;
R¹¹ ausgewählt ist aus
Wasserstoff, C₁₋₆-Alkyl, Hydroxy, Hydroxymethyl, Carboxy und C₂₋₇-Alkoxycarbonyl, und
R¹² ausgewählt ist aus
Wasserstoff, C₁₋₆-Alkyl und einer zu einem Stickstoffatom benachbarten Oxogruppe,
oder
R¹¹ und R¹² bilden zusammen eine C₁₋₃-Alkylenbrücke unter Bildung eines bicyclischen Ringsystems;
G ist ausgewählt aus G1, G2, G3, G4 und G5, worin
G1 darstellt: 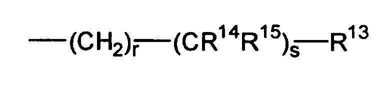
r 0, 1, 2 oder 3 ist;
s 0 oder 1 ist;
R¹³ ausgewählt ist aus
Wasserstoff, C₁₋₆-Alkyl, C₃₋₆-Alkenyl, C₃₋₆-Alkinyl, C₃₋₈-Cycloalkyl,
der Gruppe bestehend aus gesättigten und ungesättigten, 4- bis 8-gliedrigen Heterocyclen, die gegebenenfalls ein oder zwei Heteroatome, ausgewählt aus N, S und O, enthalten,
Benzyl, Phenyl,
der Gruppe bestehend aus monocyclischen aromatischen 5- bis 6-gliedrigen Heterocyclen, die gegebenenfalls ein bis drei Heteroatome enthalten, ausgewählt aus N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht,
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome ausgewählt sind aus N, S und O, und die Verknüpfung entweder über einen aromatischen Ring oder einen hydrierten Ring und entweder direkt oder über eine Methylengruppe geschieht;
R¹⁴ die gleiche Bedeutung wie R¹³ hat, aber unabhängig davon ausgewählt ist;
R¹⁵ ausgewählt ist aus
Wasserstoff, Hydroxy, Methyl, Benzyl, Phenyl,
der Gruppe bestehend aus monocyclischen aromatischen 5- bis 6-gliedrigen Heterocyclen, die ein bis drei Heteroatome enthalten, ausgewählt aus der Gruppe N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht,
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome gegebenenfalls ausgewählt sind aus N, S und O, und die Verknüpfung entweder über einen aromatischen Ring oder einen hydrierten Ring und entweder direkt oder über eine Methylengruppe geschieht;
G2 ausgewählt ist aus 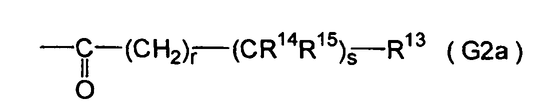 und 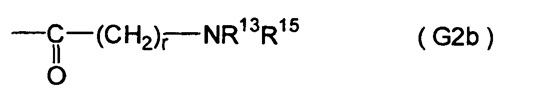 worin r, s und die Substituenten R¹³ bis R¹⁵ obige Bedeutungen aufweisen, oder die Gruppe
―NR¹³R¹⁵
einen über das Stickstoffatom gebundenen stickstoffhaltigen Heterocyclus darstellt, wobei der stickstoffhaltige Heterocyclus ausgewählt ist aus
der Gruppe bestehend aus gesättigten und ungesättigten, monocyclischen, 4- bis 8-gliedrigen Heterocyclen, die neben dem essentiellen Stickstoffatom gegebenenfalls ein oder zwei weitere Heteroatome, ausgewählt aus N, S und O, enthalten, und
der Gruppe bestehend aus gesättigten und ungesättigten, bi- oder tricyclischen, kondensierten oder verbrückten Heterocyclen mit 8 bis 16 Ringatomen, die neben dem essentiellen Stickstoffatom gegebenenfalls ein oder zwei weitere Heteroatome, ausgewählt aus N, S und O, enthalten;
G3 darstellt: 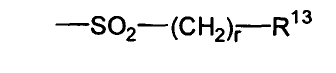 worin r und R¹³ die obigen Bedeutungen aufweisen;
G⁴ darstellt: 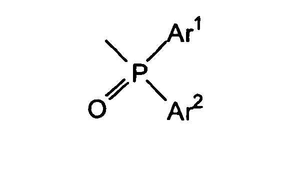 worin
Ar¹ und Ar² unabhängig voneinander ausgewählt sind aus Phenyl, Pyridyl und Naphthyl;
G5 darstellt:
―COR¹⁶ (G5)
worin
R¹⁶ ausgewählt ist aus
Trifluormethyl, C₁₋₆-Alkoxy, C₃₋₆-Alkenyloxy und Benzyloxy,
worin aromatische Ringsysteme in den Substituenten R¹, R², R⁴, R⁵, R⁶, R¹³, R¹⁴, R¹⁵, R¹⁶, Ar¹ und Ar² und/oder in dem Ringsystem -NR¹³R¹⁵ unabhängig voneinander gegebenenfalls durch ein bis drei der gleichen oder unterschiedlichen Gruppen substituiert sind, unabhängig ausgewählt aus
Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und worin
Alkyl-, Alkenyl- und Cycloalkylreste in den Gruppen G1, G2 und G3 gegebenenfalls durch ein oder zwei der gleichen oder unterschiedlichen Gruppen substituiert sind, die ausgewählt sind aus Hydroxy, Carboxy, C₂₋₇-Alkoxycarbonyl, Benzyloxycarbonyl, Amino, Mono-C₁₋₆-alkylamino und Di-(C₁₋₆-alkyl-amino);
ihre cis- und trans-Isomere, E- und Z-Isomere, Enantiomere, Diastereomere und andere Isomere sowie ihre racemischen oder nicht-racemischen Mischungen und die entsprechenden endo- und exo-Isomere für den Fall, dass das Ringsystem E bicyclisch ist;
ihre Tautomere; sowie
ihre Säureadditionssalze einschliesslich ihrer Hydrate und Solvate.

22. Verwendung gemäss Anspruch 21, worin
R¹ ausgewählt ist aus
Wasserstoff, Halogen, Cyano, C₁₋₆-Alkyl, Trifluormethyl, C₃₋₈-Cycloalkyl, C₁₋₆-Hydroxyalkyl, Hydroxy, C₁₋₄-Alkoxy, Benzyloxy, C₁₋₄-Alkylthio, C₁₋₅-Alkanoyloxy, C₂₋₅-Alkoxycarbonyl, Aminocarbonyl, C₂₋₅-Alkylaminocarbonyl, C₃₋₉-Dialkylaminocarbonyl, Carboxy, Phenyl, Phenoxy, Phenylthio, Pyridyloxy und NR⁵R⁶, worin
R⁵ und R⁶ unabhängig voneinander ausgewählt sind aus Wasserstoff und C₁₋₆-Alkyl;
R² ausgewählt ist aus
Wasserstoff, Halogen, Cyano, C₁₋₆-Alkyl, Trifluormethyl, Hydroxy und C₁₋₄-Alkoxy;
R³ ausgewählt ist aus
Wasserstoff, Halogen und C₁₋₆-Alkyl;
R⁴ ausgewählt ist aus
Wasserstoff, C₁₋₆-Alkyl, C₃₋₆-Alkenyl, C₃₋₆-Cycloalkyl, Hydroxy, C₁₋₆-Alkoxy und Benzyloxy;
k 0 oder 1 ist;
A ausgewählt ist aus
C₁₋₆-Alkylen, gegebenenfalls ein- bis dreimal substituiert durch C₁₋₃-Alkyl, Hydroxy, Fluor oder Phenyl,
C₂₋₆-Alkylen, worin eine Methyleneinheit isosterisch ersetzt ist durch O, S, NR⁹, CO, SO oder SO₂, worin, mit Ausnahme von CO, die isosterische Substitution nicht benachbart zur Amidgruppe ist und der Rest R⁹ ausgewählt ist aus Wasserstoff, C₁₋₆-Alkyl, C₁₋₆-Acyl und Methansulfonyl,
1,2-Cyclopropylen,
C₂₋₆-Alkenylen, gegebenenfalls ein- bis dreimal substituiert durch C₁₋₃-Alkyl, Hydroxy, Fluor, Cyano oder Phenyl,
C₄₋₆-Alkadienylen, gegebenenfalls ein- oder zweimal substituiert durch C₁₋₃-Alkyl, Fluor, Cyano oder Phenyl,
1,3,5-Hexatrienylen, gegebenenfalls substituiert durch C₁₋₃-Alkyl, Fluor oder Cyano, und
Ethinylen;
D ausgewählt ist aus
C₂₋₁₀-Alkylen, gegebenenfalls ein- oder zweimal substituiert durch C₁₋₃-Alkyl oder Hydroxy,
C₄₋₁₀-Alkenylen, gegebenenfalls ein- oder zweimal substituiert durch C₁₋₃-Alkyl oder Hydroxy,
C₄₋₁₀-Alkinylen, gegebenenfalls ein- oder zweimal substituiert durch C₁₋₃-Alkyl oder Hydroxy, und
der Gruppe, bestehend aus C₂₋₁₀-Alkylen, C₄₋₁₀-Alkenylen und C₄₋₁₀-Alkinylen, worin ein bis drei Methyleneinheiten isosterisch ersetzt sind durch O, S, NR¹⁰, CO, SO oder SO₂, worin
R¹⁰ die gleiche Bedeutung wie R⁹ aufweist, aber unabhängig davon ausgewählt ist;
E darstellt: 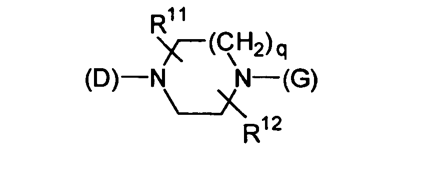 worin:
q 1, 2 oder 3 ist;
R¹¹ ausgewählt ist aus
Wasserstoff, C₁₋₃-Alkyl, Hydroxy, Hydroxymethyl, Carboxy und C₂₋₇-Alkoxycarbonyl; und
R¹² ausgewählt ist aus
Wasserstoff und einer zu einem Stickstoffatom benachbarten Oxogruppe,
oder
R¹¹ und R¹² bilden zusammen eine C₁₋₃-Alkylenbrücke unter Bildung eines bicyclischen Ringsystems;
G ist ausgewählt aus G1, G2, G3, G4 und G5, worin
G1 darstellt: 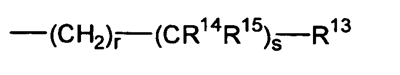
r 0, 1 oder 2 ist;
s 0 oder 1 ist;
R¹³ ausgewählt ist aus
Wasserstoff, C₁₋₆-Alkyl, C₃₋₆-Alkenyl, C₃₋₆-Alkinyl, C₃₋₈-Cycloalkyl, Benzyl, Phenyl,
der Gruppe bestehend aus monocyclischen aromatischen 5- bis 6-gliedrigen Heterocyclen, die ein bis drei Heteroatome enthalten, ausgewählt aus N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht,
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome gegebenenfalls ausgewählt sind aus N, S und O, und die Bindung entweder über einen aromatischen Ring oder einen hydrierten Ring und entweder direkt oder über eine Methylengruppe geschieht;
R¹⁴ die gleiche Bedeutung wie R¹³ hat, aber unabhängig davon ausgewählt ist;
R¹⁵ ausgewählt ist aus
Wasserstoff, Hydroxy, Methyl, Benzyl, Phenyl,
der Gruppe bestehend aus monocyclischen aromatischen 5- bis 6-gliedrigen Heterocyclen, die ein bis drei Heteroatome enthalten, ausgewählt aus N, S und O, und entweder direkt oder über eine Methylengruppe gebunden sind,
der Gruppe bestehend aus kondensierten bi- und tricyclischen, aromatischen oder teilweise hydrierten carbocyclischen Ringsystemen mit 8 bis 16 Ringatomen und mindestens einem aromatischen Ring, worin die Verknüpfung entweder über einen aromatischen oder einen hydrierten Ring und entweder direkt oder über eine Methylengruppe geschieht, und
der Gruppe bestehend aus kondensierten bi- und tricyclischen, aromatischen oder teilweise hydrierten heterocyclischen Ringsystemen mit 8 bis 16 Ringatomen und mindestens einem aromatischen Ring, worin ein bis drei Ringatome gegebenenfalls ausgewählt sind aus N, S und O, und die Verknüpfung entweder über einen aromatischen Ring oder einen hydrierten Ring und entweder direkt oder über eine Methylengruppe geschieht;
G2 ausgewählt ist aus 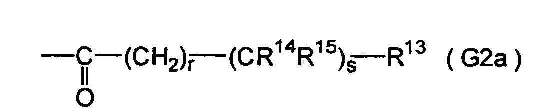 und 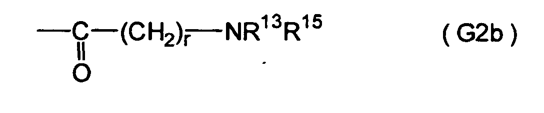 worin r, s und die Substituenten R¹³ bis R¹⁵ obige Bedeutungen aufweisen, oder die Gruppe
―NR¹³R¹⁵
ist ein über das Stickstoffatom gebundener stickstoffhaltiger Heterocyclus, wobei der stickstoffhaltige Heterocyclus ausgewählt ist aus
der Gruppe bestehend aus gesättigten und ungesättigten, monocyclischen, 4- bis 8-gliedrigen Heterocyclen, die neben dem essentiellen Stickstoffatom gegebenenfalls ein oder zwei weitere Heteroatome, ausgewählt aus N, S und O, enthalten, und
der Gruppe bestehend aus gesättigten und ungesättigten, bi- oder tricyclischen, kondensierten oder verbrückten Heterocyclen mit 8 bis 16 Ringatomen, die neben dem essentiellen Stickstoffatom gegebenenfalls ein oder zwei weitere Heteroatome, ausgewählt aus N, S und O, enthalten;
G3 darstellt: 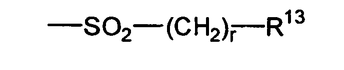 worin r und R¹³ die obigen Bedeutungen aufweisen;
G⁴ darstellt: 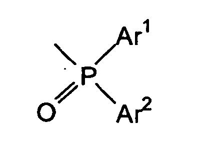 worin
Ar¹ und Ar² unabhängig voneinander ausgewählt sind aus Phenyl, Pyridyl und Naphthyl;
G5 darstellt:
―COR¹⁶
worin
R¹⁶ ausgewählt ist aus
Trifluormethyl, C₁₋₆-Alkoxy, C₃₋₆-Alkenyloxy und Benzyloxy,
worin aromatische Ringsysteme in den Substituenten R¹, R², R⁴, R⁵, R⁶, R¹³, R¹⁴, R¹⁵, R¹⁶, Ar¹ und Ar² und/oder in dem Ringsystem -NR¹³R¹⁵ unabhängig voneinander gegebenenfalls durch ein bis drei der gleichen oder unterschiedlichen Gruppen substituiert sind, ausgewählt aus
Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid,
worin Alkyl-, Alkenyl- und Cycloalkylreste in den Gruppen G1, G2 und G3 gegebenenfalls durch ein oder zwei der gleichen oder unterschiedlichen Gruppen substituiert sind, die ausgewählt sind aus Hydroxy, Carboxy, C₂₋₇-Alkoxycarbonyl, Benzyloxycarbonyl, Amino, Mono-C₁₋₆-alkylamino und Di-(C₁₋₆-alkyl-amino).

23. Verwendung gemäss Anspruch 21 oder 22, worin
R¹ ausgewählt ist aus
Wasserstoff, Halogen, Cyano, Methyl, Ethyl, Trifluormethyl, Hydroxy, C₁₋₄-Alkoxy, Benzyloxy, C₁₋₅-Alkanoyloxy, Methylthio, Ethylthio, Methoxycarbonyl, tert-Butoxycarbonyl, Aminocarbonyl, Carboxy, Phenoxy und Phenylthio;
R² ausgewählt ist aus
Wasserstoff, Halogen, Trifluormethyl und Hydroxy;
R³ ausgewählt ist aus
Wasserstoff und Halogen;
R⁴ ausgewählt ist aus
Wasserstoff, C₁₋₃-Alkyl, Allyl, Hydroxy und C₁₋₃-Alkoxy;
k 0 oder 1 ist;
A ausgewählt ist aus
C₁₋₆-Alkylen, gegebenenfalls ein- oder zweimal substituiert durch C₁₋₃-Alkyl, Hydroxy oder Fluor,
C₂₋₆-Alkylen, worin eine Methyleneinheit isosterisch ersetzt ist durch O, S, NR⁹, CO, SO oder SO₂, worin, mit Ausnahme von CO, die isosterische Substitution nicht benachbart zur Amidgruppe ist,
C₂₋₆-Alkenylen, gegebenenfalls ein- oder zweimal substituiert durch C₁₋₃-Alkyl, Hydroxy und/oder Fluor,
C₄₋₆-Alkadienylen, gegebenenfalls durch C₁₋₃-Alkyl oder ein oder zwei Fluoratome substituiert, und
1,3,5-Hexatrienylen, gegebenenfalls durch Fluor substituiert;
D ausgewählt ist aus
C₂₋₈-Alkylen, gegebenenfalls ein- oder zweimal substituiert durch Methyl oder Hydroxy,
C₄₋₈-Alkenylen, gegebenenfalls ein- oder zweimal substituiert durch Methyl oder Hydroxy,
C₄₋₈-Alkinylen, gegebenenfalls ein- oder zweimal substituiert durch Methyl oder Hydroxy, und
der Gruppe, bestehend aus C₂₋₈-Alkylen, C₄₋₈-Alkenylen und C₄₋₈-Alkinylen, worin ein bis drei Methyleneinheiten jeweils isosterisch ersetzt sind durch O, S, NH, N(CH₃), N(COCH₃), N(SO₂CH₃), CO, SO oder SO₂,
E darstellt: 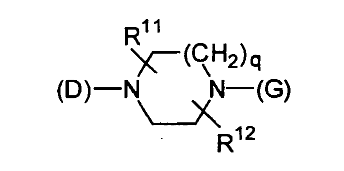 worin:
q 1 oder 2 ist;
R¹¹ ausgewählt ist aus
Wasserstoff, C₁₋₃-Alkyl, Hydroxymethyl und Carboxy; und
R¹² ausgewählt ist aus
Wasserstoff und einer zu einem Stickstoffatom benachbarten Oxogruppe;
G ausgewählt ist aus G1, G2, G3, G4 und G5, worin
G1 darstellt: 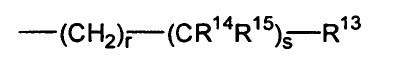
r 0, 1 oder 2 ist;
s 0 oder 1 ist;
R¹³ ausgewählt ist aus .
Wasserstoff, C₁₋₆-Alkyl, C₃₋₈-Cycloalkyl, Benzyl, Phenyl,
der Gruppe bestehend aus Benzocyclobutyl, Indanyl, Indenyl, Oxoindanyl, Naphthyl, Dihydronaphthyl, Tetrahydronaphthyl, Oxotetrahydronaphthyl, Biphenylenyl, Fluorenyl, Oxofluorenyl, Anthryl, Dihydroanthryl, Oxodihydroanthryl, Dioxodihydroanthryl, Phenanthryl, Dihydrophenanthryl, Oxodihydrophenanthryl, Dibenzocycloheptenyl, Oxodibenzocycloheptenyl, Dihydrodibenzocycloheptenyl, Oxodihydrodibenzocycloheptenyl, Dihydrodibenzocyclooctenyl, Tetrahydrodibenzocyclooctenyl und Oxotetrahydrodibenzocyclooctenyl, direkt oder über eine Methylengruppe gebunden; und
der Gruppe bestehend aus Furyl, Thienyl, Pyrrolyl, Oxazolyl, Isoxazolyl, Thiazolyl, Isothiazolyl, Pyrazolyl, Imidazolyl, Oxadiazolyl, Thiadiazolyl, Triazolyl, Pyridyl, Pyrazinyl, Pyridazinyl, Pyrimidinyl, Triazinyl, Imidazothiazolyl, Benzofuryl, Dihydrobenzofuryl, Benzothienyl, Dihydrobenzothienyl, Indolyl, Indolinyl, Isoindolinyl, Oxoindolinyl, Dioxoindolinyl, Benzoxazolyl, Oxobenzooxazolinyl, Benzoisooxazolyl, Oxobenzoisooxazolinyl, Benzothiazolyl, Oxobenzothiazolinyl, Benzoisothiazolyl, Oxobenzoisothiazolinyl, Benzoimidazolyl, Oxobenzoimidazolinyl, Indazolyl, Oxoindazolinyl, Benzofurazanyl, Benzothiadiazolyl, Benzotriazolyl, Oxazolopyridyl, Oxodihydrooxazolopyridyl, Thiazolopyridyl, Oxodihydrothiazolopyridyl, Isothiazolopyridyl, Imidazopyridyl, Oxodihydroimidazopyridyl, Pyrazolopyridyl, Oxodihydropyrazolopyridyl, Thienopyrimidinyl, Chromanyl, Chromanonyl, Benzopyranyl, Chromonyl, Chinoloyl, Isochinoloyl, Dihydrochinoloyl, Oxodihydrochinoloyl, Tetrahydrochinoloyl, Oxotetrahydrochinolinyl, Benzodioxanyl, Chinoxalinyl, Chinazolinyl, Naphthyridinyl, Carbazolyl, Tetrahydrocarbazolyl, Oxotetrahydrocarbazolyl, Pyridoindolyl, Acridinyl, Oxodihydroacridinyl, Phenanthridinyl, Dihydrophenanthridinyl, Oxodihydrophenanthridinyl, Dibenzoisochinolinyl, Dihydrodibenzoisochinolinyl, Oxodihydrodibenzoisochinolinyl, Phenothiazinyl, Dihydrodibenzooxepinyl, Oxodihydrodibenzooxepinyl, Benzocycloheptathienyl, Oxobenzocycloheptathienyl, Dihydrothienobenzothiepinyl, Oxodihydrothienobenzothiepinyl, Dihydrodibenzothiepinyl, Oxodihydrodibenzothiepinyl, Octahydrodibenzothiepinyl, Dibenzoazepinyl, Dihydrodibenzoazepinyl, Oxodihydrodibenzoazepinyl, Octahydrodibenzoazepinyl, Benzocycloheptapyridyl, Oxobenzocycloheptapyridyl, Pyridobenzoazepinyl, Dihydropyridobenzoazepinyl, Oxodihydropyridobenzoazepinyl, Dihydropyridobenzodiazepinyl, Dihydrodibenzooxazepinyl, Dihydropyridobenzooxepinyl, Dihydropyridobenzooxazepinyl, Oxodihydropyridobenzooxazepinyl, Dihydrodibenzothiazepinyl, Oxodihydrodibenzothiazepinyl, Dihydropyridobenzothiazepinyl und Oxodihydropyridobenzothiazepinyl, direkt oder über eine Methylengruppe gebunden;
R¹⁴ die gleiche Bedeutung wie R¹³ hat, aber unabhängig davon ausgewählt ist;
R¹⁵ ausgewählt ist aus
Hydroxy, Methyl, Benzyl, Phenyl, Indanyl, Indenyl, Naphthyl, Dihydronaphthyl, Tetrahydronaphthyl, Furyl, Thienyl, Pyrrolyl, Oxazolyl, Isoxazolyl, Thiazolyl, Isothiazolyl, Pyrazolyl, Imidazolyl, Oxadiazolyl, Thiadiazolyl, Triazolyl, Pyridyl, Pyrazinyl, Pyridazinyl, Pyrimidinyl, Triazinyl, Benzofuryl, Benzothienyl, Indolyl, Indolinyl, Benzooxazolyl, Benzothiazolyl, Benzoimidazolyl, Chromanyl, Chinolyl und Tetrahydrochinolyl, direkt oder über eine Methylengruppe gebunden,
G2 ausgewählt ist aus 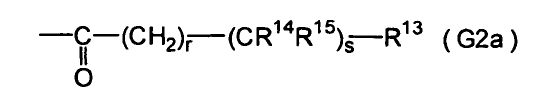 und 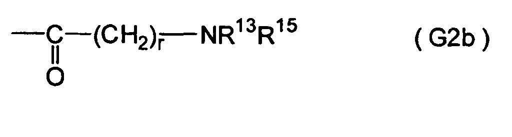 worin r, s und die Substituenten R¹³ bis R¹⁵ obige Bedeutungen aufweisen oder die Gruppe
―NR¹³R¹⁵
darstellt: Azetidin, Pyrrolidin, Piperidin, (1H)-Tetrahydropyridin, Hexahydroazepin, (1H)-Tetrahydroazepin, Octahydroazocin, Pyrazolidin, Piperazin, Hexahydrodiazepin, Morpholin, Hexahydrooxazepin, Thiomorpholin, Thiomorpholin-1,1-dioxid, 5-Aza-bicyclo[2.1.1]hexan, 2-Aza-bicyclo[2.2.1]heptan, 7-Aza-bicyclo[2.2.1]heptan, 2,5-Diaza-bicyclo[2.2.1]heptan, 2-Aza-bicyclo[2.2.2]octan, 8-Aza-bicyclo[3.2.1]octan, 2,5-Diaza-bicyclo[2.2.2]octan, 9-Aza-bicyclo[3.3.1]nonan, Indolin, Isoindolin, (1H)-Dihydrochinolin, (1H)-Tetrahydrochinolin, (2H)-Tetrahydroisochinolidin, (1H)-Tetrahydrochinoxalin, (4H)-Dihydrobenzooxazin, (4H)-Dihydrobenzothiazin, (1H)-Tetrahydrobenzo[b]azepin, (1H)-Tetrahydrobenzo[c]azepin, (1H)-Tetrahydrobenzo[d]azepin, (5H)-Tetrahydrobenzo[b]oxazepin, (5H)-Tetrahydrobenzo[b]thiazepin, 1,2,3,4-Tetrahydro-9H-pyrido[3,4-b]indol, (10H)-Dihydroacridin, (10H)-Dihydrophenanthridin, 1,2,3,4-Tetrahydroacridanon, (10H)-Phenoxazin, (10H)-Phenothiazin, (5H)-Dibenzoazepin, (5H)-Dihydrodibenzoazepin, (5H)-Octahydrodibenzoazepin, Dihydrobenzo[d,e]isochinolin, (5H)-Dihydrodibenzodiazepin, (5H)-Benzo[b]pyrido[f]azepin, (5H)-Dihydrodibenzo[b]pyrido[f]azepin, (11H)-Dihydrodibenzo[b,e]oxazepin, (11H)-Dihydrodibenzo[b,e]thiazepin, (10H)-Dihydrodibenzo[b,f]oxazepin, (10H)-Dihydrodibenzo[b,f]thiazepin, (5H)-Tetrahydrodibenzoazoacin, (11H)-Dihydrobenzo[e]pyrido[b]-1,4-diazepin-6-on oder (11H)-Dihydrobenzo[b]pyrido[e]-1,4-diazepin-5-on;
G3 darstellt: 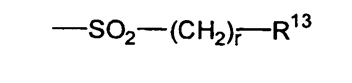 worin r und R¹³ die obigen Bedeutungen aufweisen;
G⁴ darstellt: 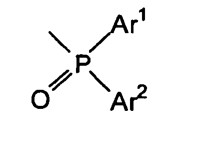 worin
Ar¹ und Ar² unabhängig voneinander ausgewählt sind aus Phenyl, Pyridyl und Naphthyl;
G5 darstellt:
―COR¹⁶
worin
R¹⁶ ausgewählt ist aus
Trifluormethyl, C₁₋₆-Alkoxy, C₃₋₆-Alkenyloxy und Benzyloxy,
worin aromatische Ringsysteme in den Substituenten unabhängig voneinander gegebenenfalls durch ein bis drei der gleichen oder unterschiedlichen Gruppen substituiert sind, ausgewählt aus
Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und
worin Alkyl-, Alkenyl- und Cycloalkylreste in den Gruppen G1, G2 und G3 gegebenenfalls durch ein oder zwei gleiche oder unterschiedliche Gruppen substituiert sind, die ausgewählt sind aus
Hydroxy, Carboxy, C₂₋₇-Alkoxycarbonyl, Benzyloxycarbonyl, Amino, Mono-C₁₋₆-alkylamino und Di-(C₁₋₆-alkyl-amino).

24. Verwendung gemäss einem der Ansprüche 21 bis 23, worin
R¹ ausgewählt ist aus
Wasserstoff, Fluor, Chlor, Brom, Methyl, Ethyl, Trifluormethyl, Hydroxy, C₁₋₄-Alkoxy, Methylthio, Ethylthio, Carboxy und Phenoxy;
R² ausgewählt ist aus Wasserstoff, Chlor und Methyl;
R³ Wasserstoff ist;
R⁴ ausgewählt ist aus Wasserstoff, C₁₋₃-Alkyl und Hydroxy;
k 0 ist;
A ausgewählt ist aus
C₂₋₆-Alkylen, gegebenenfalls ein- oder zweimal substituiert durch Hydroxy oder Fluor,
C₂₋₆-Alkylen, worin eine Methyleneinheit isosterisch ersetzt ist durch O, S oder CO, worin, mit Ausnahme von CO, die isosterische Substitution nicht benachbart zur Amidgruppe ist,
C₂₋₆-Alkenylen, gegebenenfalls ein- oder zweimal substituiert durch C₁₋₃-Alkyl und/oder Fluor, und
C₄₋₆-Alkadienylen;
D ausgewählt ist aus
C₂₋₈-Alkylen, gegebenenfalls substituiert durch Methyl oder Hydroxy,
C₄₋₈-Alkenylen, gegebenenfalls substituiert durch Hydroxy,
C₄₋₈-Alkinylen, gegebenenfalls substituiert durch Hydroxy, und
der Gruppe, bestehend aus C₂₋₈-Alkylen, C₄₋₈-Alkenylen und C₄₋₈-Alkinylen, worin eine Methyleneinheit isosterisch ersetzt ist durch O, NH, N(CH₃), CO oder SO₂, oder eine Ethylengruppe isosterisch ersetzt ist durch eine Gruppe NH-CO und/oder CO-NH, oder eine Propylengruppe isosterisch ersetzt ist durch eine Gruppe NH-CO-O und/oder O-CO-NH;
E ausgewählt ist aus Piperazin und Hexahydro-1,4-diazepin, worin der Ring gegebenenfalls durch ein oder zwei Methylengruppen und/oder durch eine zu einem Stickstoffatom benachbarte Oxogruppe substituiert ist;
G ausgewählt ist aus Wasserstoff, C₃₋₈-Cycloalkyl, Methoxycarbonyl, tert-Butoxycarbonyl, Benzyloxycarbonyl, Trifluoracetyl, Diphenylphosphinoyl, 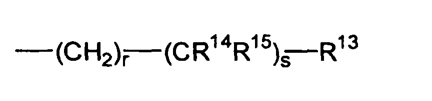 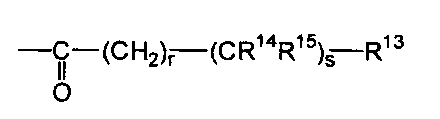 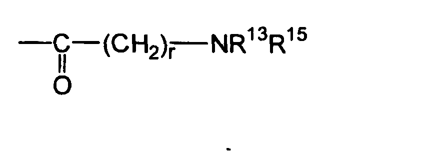 und 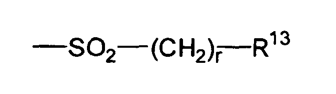 worin
r 0 oder 1 ist;
s 0 oder 1 ist;
R¹³ ausgewählt ist aus Wasserstoff, Methyl, Benzyl, Phenyl,
der Gruppe bestehend aus Indanyl, Indenyl, Oxoindanyl, Naphthyl, Tetrahydronaphthyl, Fluorenyl, Oxofluorenyl, Anthryl, Dihydroanthryl, Oxodihydroanthryl, Dioxodihydroanthryl, Phenanthryl, Dihydrophenanthryl, Oxodihydrophenanthryl, Dibenzocycloheptenyl, Dihydrodibenzocycloheptenyl und Oxodihydrodibenzocycloheptenyl, direkt oder über eine Methylengruppe gebunden; und
der Gruppe bestehend aus Furyl, Thienyl, Oxazolyl, Isooxazolyl, Thiazolyl, Imidazolyl, Oxadiazolyl, Pyridyl, Pyrazinyl, Pyrimidinyl, Imidazothiazolyl, Benzofuryl, Benzothienyl, Indolyl, Indolinyl, Oxoindolinyl, Dioxoindolinyl, Benzoxazolyl, Oxobenzooxazolinyl, Benzothiazolyl, Oxobenzothiazolinyl, Benzoimidazolyl, Oxobenzimidazolinyl, Indazolyl, Benzofurazanyl, Benzotriazolyl, Oxazolopyridyl, Oxodihydrooxazolopyridyl, Thiazolopyridyl, Oxodihydrothiazolopyridyl, Imidazopyridyl, Oxodihydroimidazopyridyl, Chromanyl, Chromanonyl, Benzopyranyl, Chromonyl, Chinolyl, Isochinolyl, Oxodihydrochinolinyl, Tetrahydrochinolyl, Oxotetrahydrochinolinyl, Benzodioxanyl, Chinazolinyl, Carbazolyl, Acridinyl, Dihydroacridinyl, Oxodihydroacridinyl, Dihydrophenanthridinyl, Dihydrodibenzoisochinolinyl, Phenothiazinyl, Dihydrodibenzooxepinyl, Benzocycloheptathienyl, Dihydrothienobenzothiepinyl, Dihydrodibenzothiepinyl, Oxodihydrodibenzothiepinyl, Dihydrodibenzoazepinyl, Oxodihydrodibenzoazepinyl, Octahydrodibenzoazepinyl, Benzocycloheptapyridyl, Dihydropyridobenzodiazepinyl und Dihydrodibenzothiazepinyl, direkt oder über eine Methylengruppe gebunden;
R¹⁴ ausgewählt ist aus Wasserstoff, Methyl, Benzyl und Phenyl;
R¹⁵ ausgewählt ist aus
Wasserstoff, Hydroxy, Methyl, Benzyl, Phenyl; und
der Gruppe bestehend aus Naphthyl, Tetrahydronaphthyl, Furyl, Thienyl, Oxazolyl, Thiazolyl, Imidazolyl, Pyridyl, Benzofuryl, Benzothienyl, Indolyl, Indolinyl, Benzoxazolyl, Benzothiazolyl, Benzoimidazolyl, Chromanyl, Chinolyl und Tetrahydrochinolyl, direkt oder über eine Methylengruppe gebunden,
worin die Gruppe
―NR¹³R¹⁵
einen über das Stickstoffatom gebundenen stickstoffhaltigen Heterocyclus darstellt, wobei der stickstoffhaltige Heterocyclus ausgewählt ist aus
Pyrrolidin, Piperid, Hexahydroazepin, Piperazin, Hexahydrodiazepin, Morpholin, Hexahydroxazepin, Thiomorpholin, 7-Aza-bicyclo[2.2.1]heptan, 2,5-Diaza-bicyclo[2.2.1]heptan, Indolin, Isoindolin, (1H)-Dihydrochinolin, (1H)-Tetrahydrochinolin, (2H)-Tetrahydroisochinolin, (4H)-Dihydrobenzoxazin, (4H)-Dihydrobenzothiazin, (1H)-Tetrahydrobenzo[b]azepin, (1H)-Tetrahydrobenzo[d]azepin, (5H)-Tetrahydrobenzo[b]oxazepin, (5H)-Tetrahydrobenzo[b]thiazepin, (10H)-Dihydroacridin, 1,2,3,4-Tetrahydroacridanon, (10H)-Dihydrophenanthridin, (1H)-Dihydrobenzo[d,e]isochinolin, (10H)-Phenothiazin, (5H)-Dibenzo[b,f]azepin, (5H)-Dihydrodibenzo[b,f]azepin, (5H)-Dihydrodibenzo[c,e,]azepin, (5H)-Dihydrodibenzodiazepin, (11H)-Dihydrodibenzo[b,e]oxazepin, (11H)-Dihydrodibenzo[b,e]thiazepin, (5H)-Dihydrobenzo[b]pyrido[3,2-f]azepin und (11H)-6-Oxodihydrobenzo[e]pyrido[3,2-b][1,4]-diazepin,
worin aromatische Ringsysteme in den Substituenten unabhängig voneinander gegebenenfalls durch ein bis drei der gleichen oder unterschiedlichen Gruppen substituiert sind, ausgewählt aus Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und
worin Alkyl-, Alkenyl- und Cycloalkylreste in der Gruppe G gegebenenfalls durch ein oder zwei gleiche oder unterschiedliche Gruppen substituiert sind, die ausgewählt sind aus Hydroxy, Carboxy, C₂₋₇-Alkoxycarbonyl, Benzyloxycarbonyl, Amino, Mono-C₁₋₆-alkylamino und Di-(C₁₋₆-alkyl-amino).

25. Verwendung gemäss einem der Ansprüche 21 bis 24, worin
R¹ ausgewählt ist aus
Wasserstoff, Fluor, Methyl, Trifluormethyl und Ethylthio;
R², R³ und R⁴ jeweils Wasserstoff sind;
k 0 ist;
A ausgewählt ist aus
der Gruppe bestehend aus Ethylen, Propylen und Butylen, die jeweils gegebenenfalls durch Hydroxy oder ein oder zwei Fluoratome substituiert sind;
OCH₂, SCH₂;
Ethenylen und 1,3-Butadienylen;
D ausgewählt ist aus
C₂₋₆-Alkylen, gegebenenfalls substituiert durch Hydroxy,
C₄₋₆-Alkenylen,
C₄₋₆-Alkinylen und
der Gruppe, bestehend aus C₂₋₆-Alkylen, C₄₋₆-Alkenylen und C₄₋₆-Alkinylen, worin ein oder zwei Methyleneinheiten isosterisch ersetzt ist durch O, NH, CO oder SO₂;
E Piperazin oder Hexahydro-1,4-diazepin ist;
G ausgewählt ist aus
Phenyl, Benzyl, Phenethyl, Diphenylmethyl, Naphthyl, Tetrahydronaphthyl, Naphthylmethyl, Fluorenyl, Fluorenylmethyl, Anthrylmethyl, Dihydrodibenzocycloheptenyl, Furylmethyl, Thienylmethyl, Thiazolylmethyl, Pyridylmethyl, Benzothienylmethyl, Chinolylmethyl, Phenylthienylmethyl, Phenylpyridylmethyl, Benzocycloheptapyridinyl, Dihydrobenzocycloheptapyridinyl, Dihydrodibenzooxepinyl, Dihydrodibenzothiepinyl, Dihydrodibenzoazepinyl, Dihydrobenzopyridodiazepinyl, Formyl, Acetyl, Pivaloyl, Phenylacetyl, Diphenylacetyl, Diphenylpropionyl, Naphthylacetyl, Benzoyl, Naphthoyl, Oxofluorenylcarbonyl, Oxodihydroanthrylcarbonyl, Dioxodihydroanthrylcarbonyl, Furoyl, Pyridylacetyl, Pyridylcarbonyl, Chromonylcarbonyl, Chinolylcarbonyl, Phenylylaminocarbonyl, Naphthylaminocarbonyl, Tetrahydronaphthylaminocarbonyl, Dibenzylaminocarbonyl, Benzylphenylaminocarbonyl, Diphenylaminocarbonyl, Indolinyl-N-carbonyl, Isoindolinyl-N-carbonyl, Tetrahydrochinolinyl-N-carbonyl, Carbazolyl-N-carbonyl, Tetrahydrobenzoazepinyl-N-carbonyl, Dihydrodibenzoazepinyl-N-carbonyl, Dihydrobenzopyridoazepinyl-N-carbonyl, Oxodihydrobenzopyridoazepinyl-N-carbonyl, Methansulfonyl, Toluolsulfonyl, Naphthylsulfonyl, Chinolinsulfonyl und Diphenylphosphinoyl,
worin aromatische Ringsysteme in den Substituenten unabhängig voneinander gegebenenfalls durch ein bis drei der gleichen oder unterschiedlichen Gruppen substituiert sind, ausgewählt aus Halogen, Cyano, C₁₋₆-Alkyl, Trifluormethyl, C₃₋₈-Cycloalkyl, Phenyl, Benzyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Carboxy, C₂₋₇-Carboxyalkyl, C₂₋₇-Carboxyalkenyl, C₂₋₇-Alkoxycarbonyl, Benzyloxycarbonyl, Nitro, Amino, Mono-C₁₋₆-alkylamino, Di-(C₁₋₆-alkyl)-amino und, für zwei benachbarte Reste des aromatischen Rings, Methylendioxid, und worin
Alkyl-, Alkenyl- und Cycloalkylreste in der Gruppe G gegebenenfalls durch ein oder zwei gleiche oder unterschiedliche Gruppen substituiert sind, die ausgewählt sind aus
Hydroxy, Carboxy, C₂₋₇-Alkoxycarbonyl, Benzyloxycarbonyl, Amino, Mono-C₁₋₆-alkylamino und Di-(C₁₋₆-alkyl)-amino.

26. Verwendung gemäss einem der Ansprüche 21 bis 25, worin die Verbindung(en) der Formel (I) ausgewählt ist/sind aus der Gruppe bestehend aus
N-[4-(4-Diphenylmethylpiperazin-1-yl)-3-hydroxybutyl]-3-pyridin-3-yl-acrylamid,
N-[3-(4-Diphenylmethylpiperazin-1-yl)-propoxy]-3-pyridin-3-yl-acrylamid,
N-[4-(4-Diphenylmethylpiperazin-1-yl)-4-oxo-butyl]-3-pyridin-3-yl-acrylamid,
N-[3-(4-Diphenylmethylpiperazin-1-sulfonyl)-propyl]-3-pyridin-3-yl-acrylamid,
N-{2-[2-(4-Diphenylmethylpiperazin-1-yl)-ethoxy]-ethyl}-3-pyridin-3-yl-acrylamid,
N-(4-{4-[Bis-(4-fluorphenyl)-methyl]-piperazin-1-yl}-but-2-inyl)-3-pyridin-3-yl-acrylamid,
N-{4-[4-(4-Carboxyphenyl-phenylmethyl)-piperazin-1-yl]-butyl}-3-pyridin-3-yl-acrylamid,
N-(4-{4-[(4-Aminophenyl)-phenylmethyl]-piperazin-1-yl}-butyl)-3-pyridin-3-yl-acrylamid,
N-{4-[4-(9H-Fluoren-9-yl)-piperazin-1-yl]-butyl}-2-(pyridin-3-yloxy)-acetamid,
N-{5-[4-(9H-Fluoren-9-yl)-piperazin-1-yl]-phenyl}-3-pyridin-3-yl-acrylamid,
N-{6-[4-(9H-Fluoren-9-yl)-piperazin-1-yl]-hexyl]-3-pyridin-3-yl-acrylamid,
3-Pyridin-3-yl-N-{4-[4-(1,2,3,4-tetrahydronaphthalin-1-yl)-piperazin-1-yl]-butyl}-acrylamid,
3-Pyridin-3-yl-N-{4-[4-(5,6,7,8-tetrahydronaphthalin-1-yl)-piperazin-1-yl]-butyl}-acrylamid,
N-{4-[4-(Naphthalin-1-yl)-piperazin-1-yl]-butyl}-3-pyridin-3-yl-acrylamid,
N-[4-(4-Biphenyl-2-yl-piperazin-1-yl)-butyl]-3-pyridin-3-yl-propionamid,
N-[5-(4-Biphenyl-2-yl-piperazin-1-yl)-pentyl]-3-pyridin-3-yl-acrylamid,
N-[6-(4-Biphenyl-2-yl-piperazin-1-yl)-hexyl]-3-pyridin-3-yl-acrylamid,
N-[4-(4-Biphenyl-2-yl-piperazin-1-yl)-butyl]-2-(pyridin-3-yloxy)-acetamid,
N-[4-(4-Biphenyl-2-yl-piperazin-1-yl)-butyl]-5-(pyridin-3-yl)-penta-2,4-diensäureamid,
N-{4-[4-(10,11-Dihydro-5H-dibenzo[a,d]cyclohepten-5-yl)-piperazin-1-yl]-butyl}-3-pyridin-3-yl-propionamid,
N-{5-[4-(10,11-Dihydro-5H-dibenzo[a,d]cyclohepten-5-yl)-piperazin-1-yl]-pentyl}-3-pyridin-3-yl-acrylamid,
N-{6-[4-(10,11-Dihydro-5H-dibenzo[a,d]cyclohepten-5-yl)-piperazin-1-yl]-hexyl}-3-pyridin-3-yl-acrylamid,
N-{4-[4-(10,11-Dihydro-5H-dibenzo[a,d]cyclohepten-5-yl)-piperazin-1-yl]-butyl}-5-(pyridin-3-yl)-penta-2,4-diensäureamid,
N-{4-[4-(6,11-Dihydrodibenzo[b,e]oxepin-11-yl)-piperazin-1-yl]-butyl-3-pyridin-3-yl-propionamid,
N-{2-[4-(6,11-Dihydrodibenzo[b,e]thiepin-11-yl)-piperazin-1-yl]-ethyl}-3-pyridin-3-yl-acrylamid,
N-[4-(4-Diphenylacetyl-piperazin-1-yl)-butyl]-3-pyridin-3-yl-acrylamid,
N-[4-(4-Benzoylpiperazin-1-yl)-butyl]-3-pyridin-3-yl-acrylamid,
N-{4-[4-(2-Aminobenzoyl)-piperazin-1-yl]-butyl}-3-pyridin-3-yl-acrylamid,
N-{4-[4-(4-Carboxybenzoyl)-piperazin-1-yl]-butyl}-3-pyridin-3-yl-acrylamid,
N-{4-[4-(Biphenyl-2-carbonyl)-piperazin-1-yl]-butyl}-3-pyridin-3-yl-acrylamid,
N-{4-[4-(9-Oxo-9H-fluor-4-carbonyl)-piperazin-1-yl]-butyl}-3-pyridin-3-yl-acrylamid,
N-{4-[4-(Furan-2-carbonyl)-piperazin-1-yl]-butyl}-3-pyridin-3-yl-acrylamid,
N-{4-[4-(Naphthalin-1-yl-aminocarbonyl)-piperazin-1-yl]-butyl}-3-pyridin-3-yl-propionamid,
N-{4-[4-(Diphenylaminocarbonyl)-piperazin-1-yl]-butyl}-3-pyridin-3-yl-acrylamid,
N-{4-[4-(Naphthalin-2-sulfonyl)-piperazin-1-yl]-butyl}-3-pyridin-3-yl-acrylamid,
N-[4-(4-Diphenylphosphinoyl-piperazin-1-yl)-butyl]-3-pyridin-3-yl-acrylamid,
N-[4-(4-Biphenyl-2-yl-piperazin-1-yl)-butyl]-3-pyridin-3-yl-acrylamid,
N-{4-[4-(9H-Fluoren-9-yl)-piperazin-1-yl]-butyl}-3-pyridin-3-yl-acrylamid und
N-{4-[4-(10,11-Dihydro-5H-dibenzo[a,d]cyclohepten-5-yl)-piperazin-1-yl]-butyl}-3-pyridin-3-yl-acrylamid.

27. Verwendung einer oder mehrerer Verbindungen gemäss einem der Ansprüche 1 bis 7 zur Herstellung einer pharmazeutischen Zusammensetzung zur Immunsuppression im menschlichen oder tierischen Körper, gegebenenfalls mit einem weiteren Cytostatikum oder Immunsuppressivum und/oder weiteren für diese Indikation geeigneten pharmazeutischen Zusammensetzungen.

## Revendications

1. Carboxamide de pyridylalcane, de pyridylalcène ou de pyridylalcyne selon la formule (I) 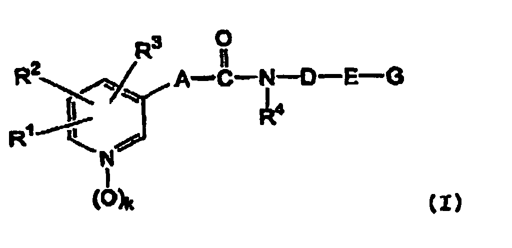 dans laquelle :
R¹ est choisi parmi
l'hydrogène, un halogène, un groupe cyano, un groupe alkyle en C₁ à C₆, un groupe alcényle en C₃ à C₆, un groupe alcynyle en C₂ à C₆, un groupe trifluorométhyle, un groupe cycloalkyle en C₃ à C₈, un groupe hydroxyalkyle en C₁ à C₆, un groupe hydroxy, un groupe alcoxy en C₁ à C₆, un groupe alcényloxy en C₃ à C₆, un groupe alcynyloxy en C₃ à C₆, un groupe benzyloxy, un groupe alcanoyloxy en C₁ à C₇, un groupe alcoxycarbonyloxy en C₂ à C₇, un groupe alkylthio en C₁ à C₆, un groupe alcénylthio en C₃ à C₆, un groupe alcynylthio en C₃ à C₆, un groupe cycloalkyloxy en C₃ à C₈, un groupe cycloalkylthio en C₃ à C₈, un groupe alcoxycarbonyle en C₂ à C₇, un groupe aminocarbonyle, un groupe alkylaminocarbonyle en C₂ à C₇, un groupe dialkylaminocarbonyle en C₃ à C₁₃, un groupe carboxy, un groupe phényle, un groupe phénoxy, un groupe phénylthio, un groupe pyridyloxy, un groupe pyridylthio, et un groupe NR⁵R⁶, dans lequel
R⁵ et R⁶ sont choisis indépendamment l'un de l'autre parmi
l'hydrogène, un groupe alkyle en C₁ à C₆, un groupe alcényle en C₃ à C₆, un groupe alcynyle en C₃ à C₆, un groupe benzyle et un groupe phényle ;
R² est choisi parmi
l'hydrogène, un halogène, un groupe cyano, un groupe alkyle en C₁ à C₆, un groupe trifluorométhyle, un groupe hydroxy, un groupe alcoxy en C₁ à C₆, un groupe benzyloxy et un groupe alcanoyloxy en C₁ à C₇
ou
R¹ et R², s'ils sont adjacents, forment de façon facultative un pont choisi parmi -(CH₂)₄-, -(CH=CH)₂- et -CH₂O-CR⁷R⁸-O-, dans lequel
R⁷ et R⁸ sont choisis indépendamment l'un de l'autre parmi l'hydrogène et un groupe alkyle en C₁ à C₆ ;
R³ est choisi parmi
l'hydrogène, un halogène, un groupe alkyle en C₁ à C₆, un groupe trifluorométhyle et un groupe hydroxyalkyle en C₁ à C₆ ;
R⁴ est choisi parmi
l'hydrogène, un groupe alkyle en C₁ à C₆, un groupe alcényle en C₃ à C₆, un groupe alcynyle en C₃ à C₆, un groupe cycloalkyle en C₃ à C₆, un groupe hydroxy, un groupe alcoxy en C₁ à C₆ et un groupe benzyloxy ;
k est 0 ou 1,
A est choisi parmi
un groupe alkylène en C₁ à C₆, éventuellement substitué de une à trois fois par un groupe alkyle en C₁ à C₃, un groupe hydroxy, un groupe alcoxy en C₁ à C₃, le fluor, ou un groupe phényle,
un groupe alkylène en C₂ à C₆, dans lequel une unité méthylène est remplacée de façon isostérique par O, S, NR⁹, CO, SO ou SO₂, dans lequel, à l'exception de CO, la substitution isostérique n'est pas adjacente au groupe amide et R⁹ est choisi parmi l'hydrogène, un groupe alkyle en C₁ à C₆, un groupe alcényle en C₃ à C₆, un groupe alcynyle en C₃ à C₆, un groupe acyle en C₁ à C₆ et un groupe alcanesulfonyle en C₁ à C₆,
un groupe 1,2-cyclopropylène,
un groupe alcénylène en C₂ à C₆, éventuellement substitué de une à trois fois par un groupe alkyle en C₁ à C₃, un groupe hydroxy, un groupe alcoxy en C₁ à C₃, le fluor, un groupe cyano ou un groupe phényle,
un groupe alcadiénylène en C₄ à C₆, éventuellement substitué une fois ou deux fois par un groupe alkyle en C₁ à C₃, le fluor, un groupe cyano ou un groupe phényle ;
un groupe 1,3,5-hexatriénylène, éventuellement substitué par un groupe alkyle en C₁ à C₃, le fluor, un groupe cyano ou un groupe phényle, et
un groupe éthynylène
D est choisi parmi
un groupe alkylène en C₂ à C₁₀, éventuellement substitué une fois ou deux fois par un groupe alkyle en C₁ à C₆, un groupe hydroxy, ou un groupe alcoxy en C₁ à C₆ ;
un groupe alcénylène en C₉ à C₁₀, éventuellement substitué une fois ou deux fois par un groupe alkyle en C₁ à C₆, un groupe hydroxy, ou un groupe alcoxy en C₁ à C₆ ;
un groupe alcynylène en C₄ à C₁₀, éventuellement substitué une fois ou deux fois par un groupe alkyle en C₁ à C₆, un groupe hydroxy, ou un groupe alcoxy en C₁ à C₆ ; et
le groupe constitué par un groupe alkylène en C₂ à C₁₀, un groupe alcénylène en C₄ à C₁₀ et un groupe alcynylène en C₄ à C₁₀, dans lequel une à trois unités méthylène sont remplacées de façon isostérique par O, S, NR¹⁰, CO, SO ou SO₂, dans lequel
R¹⁰ a la même signification que R⁹, mais est choisi indépendamment de celui-ci;
E représente 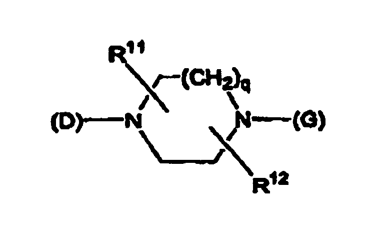 dans lequel
q est 1, 2 ou 3 ;
R¹¹ est choisi parmi
l'hydrogène, un groupe alkyle en C₁ à C₆, un groupe hydroxy, un groupe hydroxyméthyle, un groupe carboxy, et un groupe alcoxycarbonyle en C₂ à C₇, et
R¹² est choisi parmi
l'hydrogène, un groupe alkyle en C₁ à C₆ et un groupe oxo adjacent à un atome d'azote,
ou R¹¹ et R¹² forment ensemble un pont alkylène en C₁ à C₃ lors d'une formation d'un système à noyau bicyclique ;
G est choisi parmi G1, G2, G3, G4 et G5, dans lesquels
G1 représente
-(CH₂)ᵣ-(CR¹⁴R¹⁵)ₛ-R¹³,
r est 0, 1, 2 ou 3,
s est 0 ou 1
R¹³ est choisi parmi
l'hydrogène, un groupe alkyle en C₁ à C₆, un groupe alcényle en C₃ à C₆, un groupe alcynyle en C₃ à C₆, un groupe cycloalkyle en C₃ à C₈ ;
le groupe constitué des hétérocycles de quatre à huit membres, saturés et insaturés, qui contiennent éventuellement un ou deux hétéroatomes choisis parmi N, S et O ;
un groupe benzyle, un groupe phényle ;
le groupe constitué des hétérocycles de cinq à six membres aromatiques monocycliques, qui contiennent éventuellement un à trois hétéroatomes choisis parmi N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
R¹⁴ a la même signification que R¹³, mais est choisi indépendamment de celui-ci ;
R¹⁵ est choisi parmi
l'hydrogène, un groupe hydroxy, un groupe méthyle, un groupe benzyle, un groupe phényle,
le groupe constitué des hétérocycles de cinq et six membres aromatiques monocycliques, qui contiennent un à trois hétéroatomes du groupe N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
G2 est choisi parmi 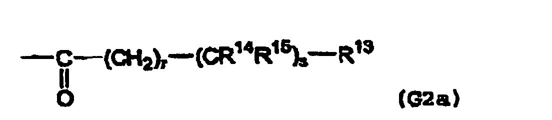 et 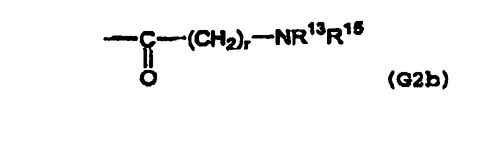 dans lesquels r, s et les substituants R¹³ à R¹⁵ ont la signification ci-dessus, ou le groupe
-NR¹³R¹⁵
représente un hétérocycle contenant de l'azote relié sur l'atome d'azote lequel hétérocycle contenant de l'azote est choisi parmi
le groupe constitué des hétérocycles de quatre à huit membres, monocycliques, saturés et insaturés, qui, en plus de l'atome d'azote essentiel, contiennent éventuellement un ou deux autres hétéroatomes choisis parmi N, S et O, et
le groupe constitué des hétérocycles bi- ou tricyclique condensés ou pontés, saturés et insaturés, avec 8 à 16 atomes de cycle, qui en plus de l'atome d'azote essentiel contiennent éventuellement un ou deux autres hétéroatomes choisis parmi N, S et O ;
G3 représente
-SO₂-(CH₂)ᵣ-R¹³,
dans lequel r et R¹³ ont les significations ci-dessus,
G4 représente 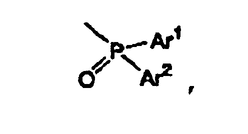 dans lequel
Ar¹ et Ar² sont choisis indépendamment l'un de l'autre parmi un groupe phényle, un groupe pyridyle et un groupe naphtyle ;
G5 représente
-COR¹⁶ (G5)
dans lequel
R¹⁶ est choisi parmi
un groupe trifluorométhyle, un groupe alcoxy en C₁ à C₆, un groupe alcényloxy en C₃ à C₆, et un groupe benzyloxy,
où les systèmes cycliques aromatiques dans les substituants R¹, R², R⁴, R⁵, R⁶, R¹³, R¹⁴, R¹⁶, Ar¹ et Ar² et/ou dans le système cyclique -NR¹³R¹⁵ sont éventuellement substitués indépendamment les uns des autres par un à trois groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl (en C₁ à C₆)amino, et un groupe méthylène dioxyde pour deux résidus adjacents sur le noyau aromatique, et où
les résidus alkyle, alcényle et cycloalkyle dans les groupes G1, G2 et G3 sont éventuellement substitués par un ou deux des groupes identiques ou différents qui sont choisis parmi un groupe hydroxy, un groupe carboxy, un groupe alcoxycarbonyle en C₂ à C₇, un groupe benzyloxycarbonyle, un groupe amino, un groupe monoalkyl(en C₁ à C₆)amino et un groupe dialkyl(en C₁ à C₆)amino ;
leurs isomères cis et trans, leurs isomères E et Z, leurs énantiomères, leurs diastéréoisomères et d'autres isomères ainsi que leurs mélanges racémiques ou non racémiques et les isomères endo et exo correspondants dans le cas où le système cyclique E est bicyclique ;
leurs tautomères ; ainsi que
leurs sels d'addition d'acide comprenant leurs hydrates et produits de solvatation,
où G ne représente pas
- (CH₂)ᵣ-(CR¹⁴R¹⁵)ₛ-R¹³,
dans le cas où simultanément
R¹³ représente un groupe pyridyle ou phényle qui est éventuellement substitué par un halogène, un groupe alkyle, un groupe alcoxy ou un groupe trifluorométhyle,
R¹⁴ représente l'hydrogène ou un groupe phényle, qui est éventuellement substitué par un halogène, un groupe alkyle, un groupe alcoxy ou un groupe trifluorométhyle,
R¹⁵ représente l'hydrogène,
A représente un groupe alkylène, un groupe éthénylène ou butadiénylène éventuellement substitué,
D représente un groupe alkylène ou alcénylène,
E représente une pipérazine ou homopipérazine et
s est 1 ;
et où G ne représente pas
un membre du groupe constitué par un groupe phényle, un hétéroaryle contenant de l'azote et 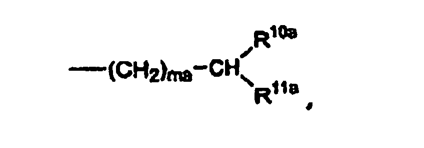 dans lequel : R^{10a} est l'hydrogène ou un groupe phényle, R^{11a} est un groupe phényle ou un groupe pyridyle, et ma est un entier de 0 à 2, à condition que le groupe phényle puisse être éventuellement substitué par un ou deux membres choisis dans le groupe constitué par un halogène, un groupe alkyle en C₁ à C₆, un groupe trifluorométhyle et un groupe alcoxy en C₁ à C₆ ;
dans le cas où simultanément
R¹ est l'hydrogène, un halogène, un groupe alkyle en C₁ à C₆, un groupe alcoxy en C₁ à C₆, un groupe alkylthio en C₁ à C₆, un groupe cycloalkyloxy en C₃ à C₈, un groupe cycloalkylthio en C₃ à C₈, un groupe alcoxycarbonyle en C₂ à C₇, un groupe carboxy, un groupe phényle, un groupe phénoxy, un groupe phénylthio, un groupe 3-pyridyloxy ou un groupe 3-pyridylthio ;
R² est l'hydrogène, un groupe hydroxy, un groupe alcanoyloxy en C₁ à C₇ ou un groupe alcoxycarbonyloxy en C₂ à C₇, ou lorsque R¹ et R² sont adjacents l'un par rapport à l'autre, ils peuvent se combiner pour former un groupe tétraméthylène ou un groupe -CH₂OCR^{8a}R^{9a}O-, dans lequel R^{8a} et R^{9a} sont identiques ou différents et sont chacun un groupe alkyle en C₁ à C₆ ;
R³ est l'hydrogène, un groupe alkyle en C₁ à C₆ ou un groupe hydroxyalkyle en C₁ à C₆ ;
A est un groupe alkylène en C₁ à C₆ ou un groupe
-(CR^{6a}=CR^{7a})ᵣₐ-, dans lequel R^{6a} est l'hydrogène, un groupe alkyle en C₁ à C₃ ou un groupe phényle, R^{7a} est l'hydrogène, un groupe alkyle en C₁ à C₃, un groupe cyano ou un groupe phényle, et ra est 1 ou 2 ;
R⁴ est l'hydrogène ;
D représente un groupe alkylène en C₂ à C₁₀ ou un groupe alkylène en C₄ à C₁₀ interrompu par une double liaison ; et
E est choisi parmi la pipérazine, une pipérazine qui est substituée par un groupe alkyle en C₁ à C₆, l'homopipérazine et une homopipérazine qui est substituée par un groupe alkyle en C₁ à C₆.

2. Composé selon la formule (I) 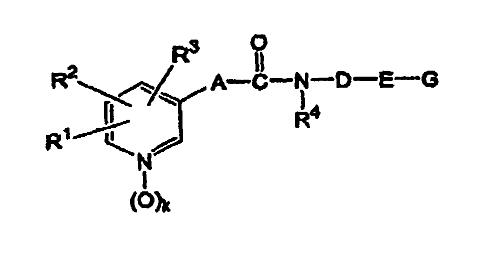 dans laquelle
R¹ est choisi parmi
l'hydrogène, un halogène, un groupe cyano, un groupe alkyle en C₁ à C₆, un groupe trifluorométhyle, un groupe cycloalkyle en C₃ à C₈, un groupe hydroxyalkyle en C₁ à C₆, un groupe hydroxy, un groupe alcoxy en C₁ à C₄, un groupe benzyloxy, un groupe alkylthio en C₁ à C₄, un groupe alcanoyloxy en C₁ à C₅, un groupe alcoxycarbonyle en C₂ à C₅, un groupe aminocarbonyle, un groupe alkylaminocarbonyle en C₂ à C₅, un groupe dialkylaminocarbonyle en C₃ à C₉, un groupe carboxy, un groupe phényle, un groupe phénoxy, un groupe phénylthio, un groupe pyridyloxy, et un groupe NR⁵R⁶, dans lequel
R⁵ et R⁶ sont choisis indépendamment l'un de l'autre parmi l'hydrogène et un groupe alkyle en C₁ à C₆ ;
R² est choisi parmi
l'hydrogène, un halogène, un groupe cyano, un groupe alkyle en C₁ à C₆, un groupe trifluorométhyle, un groupe hydroxy, et un groupe alcoxy en C₁ à C₄ ;
R³ est choisi parmi
l'hydrogène, un halogène et un groupe alkyle en C₁ à C₆ ;
R⁴ est choisi parmi
l'hydrogène, un groupe alkyle en C₁ à C₆, un groupe alcényle en C₃ à C₆, un groupe cycloalkyle en C₃ à C₆, un groupe hydroxy, un groupe alcoxy en C₁ à C₆ et un groupe benzyloxy ;
k est ou 1,
A est choisi parmi
un groupe alkylène en C₁ à C₆, éventuellement substitué une fois à trois fois par un groupe alkyle en C₁ à C₃, un groupe hydroxy, le fluor, ou un groupe phényle,
un groupe alkylène en C₂ à C₆, dans lequel une unité méthylène est remplacée de façon isostérique par O, S, NR⁹, CO, SO ou SO₂, dans lequel, à l'exception de CO, la substitution isostérique n'est pas adjacente au groupe amide et le résidu R⁹ est choisi parmi l'hydrogène, un groupe alkyle en C₁ à C₆, un groupe acyle en C₁ à C₆ et un groupe méthanesulfonyle ;
un groupe 1,2-cyclopropylène,
un groupe alcénylène en C₂ à C₆, éventuellement substitué de une à trois fois par un groupe alkyle en C₁ à C₃, un groupe hydroxy, le fluor, un groupe cyano ou un groupe phényle,
un groupe alcadiénylène en C₄ à C₆, éventuellement substitué une fois ou deux fois par un groupe alkyle en C₁ à C₃, le fluor, un groupe cyano ou un groupe phényle ;
un groupe 1,3,5-hexatriénylène, éventuellement substitué par un groupe alkyle en C₁ à C₃, le fluor ou un groupe cyano, et
un groupe éthynylène
D est choisi parmi
un groupe alkylène en C₂ à C₁₀, éventuellement substitué une fois ou deux fois par un groupe alkyle en C₁ à C₃ ou un groupe hydroxy ;
un groupe alcénylène en C₄ à C₁₀, éventuellement substitué une fois ou deux fois par un groupe alkyle en C₁ à C₃ ou un groupe hydroxy ;
un groupe alcynylène en C₄ à C₁₀, éventuellement substitué une fois ou deux fois par un groupe alkyle en C₁ à C₃ ou un groupe hydroxy ; et
le groupe constitué par un groupe alkylène en C₂ à C₁₀, un groupe alcénylène en C₄ à C₁₀ et un groupe alcynylène en C₄ à C₁₀, dans lequel une à trois unités méthylène sont remplacées de façon isostérique par O, S, NR¹⁰, CO, SO ou SO₂, dans lequel
R¹⁰ a la même signification que R⁹, mais est choisi indépendamment de celui-ci;
E représente 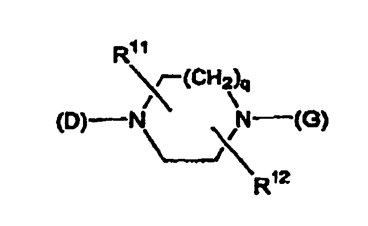 dans lequel
q est 1, 2 ou 3 ;
R¹¹ est choisi parmi
l'hydrogène, un groupe alkyle en C₁ à C₃, un groupe hydroxy, un groupe hydroxyméthyle, un groupe carboxy, et un groupe alcoxycarbonyle en C₂ à C₇, et
R¹² est choisi parmi l'hydrogène et un groupe oxo adjacent à un atome d'azote ou
R¹¹ et R¹², ensemble, forment un pont alkylène en C₁ à C₃ lors d'une formation d'un système à noyau bicyclique ;
G est choisi parmi G1, G2, G3, G4 et G5, dans lesquels
G1 représente
-(CH₂)ᵣ-(CR¹⁴R¹⁵)ₛ-R¹³,
r est 0, 1 ou 2,
s est 0 ou 1
R¹³ est choisi parmi
l'hydrogène, un groupe alkyle en C₁ à C₆, un groupe alcényle en C₃ à C₆, un groupe alcynyle en C₃ à C₆, un groupe cycloalkyle en C₃ à C₈ ; un groupe benzyle, un groupe phényle ;
le groupe constitué des hétérocycles de cinq à six membres aromatiques monocycliques, qui contiennent un à trois hétéroatomes choisis parmi N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
R¹⁴ a la même signification que R¹³, mais est choisi indépendamment de celui-ci ;
R¹⁵ est choisi parmi
l'hydrogène, un groupe hydroxy, un groupe méthyle, un groupe benzyle, un groupe phényle,
le groupe constitué des hétérocycles de cinq et six membres aromatiques monocycliques, qui contiennent un à trois hétéroatomes du groupe N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
G2 est choisi parmi 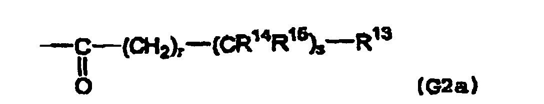 et 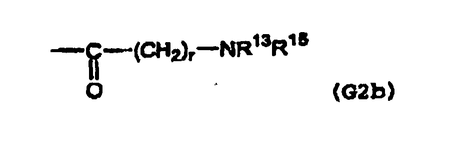 dans lesquels r, s et les substituants R¹³ à R¹⁵ ont la signification ci-dessus, ou le groupe
-NR¹³R¹⁵
représente un hétérocycle contenant de l'azote relié sur l'atome d'azote lequel hétérocycle contenant de l'azote est choisi parmi
le groupe constitué des hétérocycles de quatre à huit membres, monocycliques, saturés et insaturés, qui, en plus de l'atome d'azote essentiel, contiennent éventuellement un ou deux autres hétéroatomes choisis parmi N, S et O, et
le groupe constitué des hétérocycles bi- ou tricyclique condensés ou pontés, saturés et insaturés, avec 8 à 16 atomes de cycle, qui en plus de l'atome d'azote essentiel contiennent éventuellement un ou deux autres hétéroatomes choisis parmi N, S et O ;
G3 représente
-SO₂-(CH₂)ᵣ-R¹³,
dans lequel r et R¹³ ont les significations ci-dessus,
G4 représente 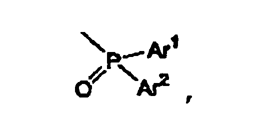 dans lequel
Ar¹ et Ar² sont choisis indépendamment l'un de l'autre parmi un groupe phényle, un groupe pyridyle et un groupe naphtyle ;
G5 représente
-COR¹⁶
dans lequel
R¹⁶ est choisi parmi
un groupe trifluorométhyle, un groupe alcoxy en C₁ à C₆, un groupe alcényloxy en C₃ à C₆, et un groupe benzyloxy,
où les systèmes cycliques aromatiques dans les substituants R¹, R², R⁴, R⁵, R⁶, R¹³, R¹⁴, R¹⁶, Ar¹ et Ar² et/ou dans le système cyclique -NR¹³R¹⁵ sont éventuellement substitués indépendamment les uns des autres par un à trois groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl(en C₁ à C₆)amino, et un groupe méthylène dioxyde dans le cas de deux résidus adjacents sur le noyau aromatique, et où
les résidus alkyle, alcényle et cycloalkyle dans les groupes G1, G2 et G3 sont éventuellement substitués par un ou deux des groupes identiques ou différents qui sont choisis parmi
un groupe hydroxy, un groupe carboxy, un groupe alcoxycarbonyle en C₂ à C₇, un groupe benzyloxycarbonyle, un groupe amino, un groupe monoalkyl(en C₁ à C₆)amino et un groupe dialkyl(en C₁ à C₆)amino ;
où G ne représente pas
un membre du groupe constitué par un groupe phényle, un hétéroaryle contenant de l'azote et 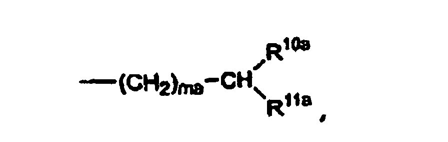 dans lequel : R^{10a} est l'hydrogène ou un groupe phényle,
R^{11a} est un groupe phényle ou un groupe pyridyle, et ma est un entier de 0 à 2, à condition que le groupe phényle puisse être éventuellement substitué par un ou deux membres choisis dans le groupe constitué par un halogène, un groupe alkyle en C₁ à C₆, un groupe trifluorométhyle et un groupe alcoxy en C₁ à C₆ ;
dans le cas où simultanément
R¹ est l'hydrogène, un halogène, un groupe alkyle en C₁ à C₆, un groupe alcoxy en C₁ à C₆, un groupe alkylthio en C₁ à C₆, un groupe cycloalkyloxy en C₃ à C₈, un groupe cycloalkylthio en C₃ à C₈, un groupe alcoxycarbonyle en C₂ à C₇, un groupe carboxy, un groupe phényle, un groupe phénoxy, un groupe phénylthio, un groupe 3-pyridyloxy ou un groupe 3-pyridylthio ;
R² est l'hydrogène, un groupe hydroxy, un groupe alcanoyloxy en C₁ à C₇ ou un groupe alcoxycarbonyloxy en C₂ à C₇, ou lorsque R¹ et R² sont adjacents l'un par rapport à l'autre, ils peuvent se combiner pour former un groupe tétraméthylène ou un groupe -CH₂OCR^{8a}R^{9a}O-, dans lequel R^{8a} et R^{9a} sont identiques ou différents et sont chacun un groupe alkyle en C₁ à C₆ ;
R³ est l'hydrogène, un groupe alkyle en C₁ à C₆ ou un groupe hydroxyalkyle en C₁ à C₆ ;
A est un groupe alkylène en C₁ à C₆ ou un groupe
-(CR^{6a}=CR^{7a})ᵣₐ-, dans lequel R^{6a} est l'hydrogène, un groupe alkyle en C₁ à C₃ ou un groupe phényle, R^{7a} est l'hydrogène, un groupe alkyle en C₁ à C₃, un groupe cyano ou un groupe phényle, et ra est 1 ou 2 ;
R⁴ est l'hydrogène ;
D représente un groupe alkylène en C₂ à C₁₀ ou un groupe alkylène en C₄ à C₁₀ interrompu par une double liaison ; et
E est choisi parmi la pipérazine, une pipérazine qui est substituée par un groupe alkyle en C₁ à C₆, l'homopipérazine et une homopipérazine qui est substituée par un groupe alkyle en C₁ à C₆.

3. Composé selon la revendication 2, dans lequel
R¹ est choisi parmi
l'hydrogène, un halogène, un groupe cyano, un groupe méthyle, un groupe éthyle, un groupe trifluorométhyle, un groupe hydroxy, un groupe alcoxy en C₁ à C₄, un groupe benzyloxy, un groupe alcanoyloxy en C₁ à C₅, un groupe méthylthio, un groupe éthylthio, un groupe méthoxycarbonyle, un groupe tert-butoxycarbonyle, un groupe aminocarbonyle, un groupe carboxy, un groupe phénoxy, et un groupe phénylthio,
R² est choisi parmi
l'hydrogène, un halogène, un groupe trifluorométhyle, et un groupe hydroxy,
R³ est choisi parmi
l'hydrogène et un halogène,
R⁴ est choisi parmi
l'hydrogène, un groupe alkyle en C₁ à C₃, un groupe allyle, un groupe hydroxy, et un groupe alcoxy en C₁ à C₃ ;
k est 0 ou 1,
A est choisi parmi
un groupe alkylène en C₁ à C₆, éventuellement substitué une fois ou deux fois par un groupe alkyle en C₁ à C₃, un groupe hydroxy ou le fluor ;
un groupe alkylène en C₂ à C₆, dans lequel une unité méthylène est remplacée de façon isostérique par O, S, NR⁹, CO, SO ou SO₂, dans lequel, à l'exception de CO, la substitution isostérique n'est pas adjacente au groupe amide,
un groupe alcénylène en C₂ à C₆, éventuellement substitué de une à trois fois par un groupe alkyle en C₁ à C₃, un groupe hydroxy et/ou le fluor ;
un groupe alcadiénylène en C₄ à C₆, éventuellement substitué par un groupe alkyle en C₁ à C₃ ou un ou deux atomes de fluor ; et
un groupe 1,3,5-hexatriénylène, éventuellement substitué par le fluor ;
D est choisi parmi
un groupe alkylène en C₂ à C₈, éventuellement substitué une fois ou deux fois par un groupe méthyle ou un groupe hydroxy ;
un groupe alcénylène en C₄ à C₈, éventuellement substitué une fois ou deux fois par un groupe méthyle ou un groupe hydroxy ;
un groupe alcynylène en C₄ à C₈, éventuellement substitué une fois ou deux fois par un groupe méthyle ou un groupe hydroxy ; et
le groupe constitué par un groupe alkylène en C₂ à C₈, un groupe alcénylène en C₄ à C₈ et un groupe alcynylène en C₄ à C₈, dans lequel une à trois unités méthylène sont remplacées de façon isostérique par O, S, NH, N(CH₃), N(COCH₃), N(SO₂CH₃), CO, SO ou SO₂,
E représente 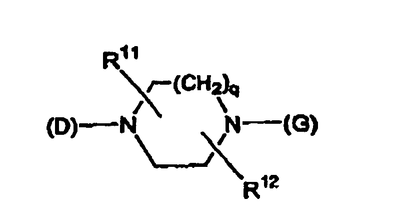 dans lequel
q est 1 ou 2 ;
R¹¹ est choisi parmi
l'hydrogène, un groupe alkyle en C₁ à C₃, un groupe hydroxyméthyle et un groupe carboxy, et
R¹² est choisi parmi
l'hydrogène et un groupe oxo adjacent à un atome d'azote
G est choisi parmi G1, G2, G3, G4 et G5, dans lesquels
G1 représente
- (CH₂)ᵣ-(CR¹⁴R¹⁵)ₛ-R¹³,
r est 0, 1 ou 2,
s est 0 ou 1 ;
R¹³ est choisi parmi
l'hydrogène, un groupe alkyle en C₁ à C₆, un groupe cycloalkyle en C₃ à C₈, un groupe benzyle, un groupe phényl,
le groupe constitué par un groupe benzocyclobutyle, un groupe indanyle, un groupe indényle, un groupe oxoindanyle, un groupe naphtyle, un groupe dihydronaphtyle, un groupe tétrahydronaphtyle, un groupe oxotétrahydronaphtyle, un groupe biphénylényle, un groupe fluorényle, un groupe oxofluorényle, un groupe anthryle, un groupe dihydroanthryle, un groupe oxodihydroanthryle, un groupe dioxodihydroanthryle, un groupe phénanthryle, un groupe dihydrophénanthryle, un groupe oxodihydrophénanthryle, un groupe dibenzocycloheptényle, un groupe oxodibenzocycloheptényle, un groupe dihydrodibenzocycloheptényle, un groupe oxodihydrodibenzocycloheptényle, un groupe dihydrodibenzocyclooctényle, un groupe tétrahydrodibenzocyclooctényle et un groupe oxotétrahydrodibenzocyclooctényle, liés directement ou sur un groupe méthylène ; et
le groupe constitué par un groupe furyle, un groupe thiényle, un groupe pyrrolyle, un groupe oxazolyle, un groupe isoxazolyle, un groupe thiazolyle, un groupe isothiazolyle, un groupe pyrazolyle, un groupe imidazolyle, un groupe oxadiazolyle, un groupe thiadiazolyle, un groupe triazolyle, un groupe pyridyle, un groupe pyrazinyle, un groupe pyridazinyle, un groupe pyrimidinyle, un groupe triazinyle, un groupe imidazothiazolyle, un groupe benzofuryle, un groupe dihydrobenzofuryle, un groupe benzothiényle, un groupe dihydrobenzothiényle, un groupe indolyle, un groupe indolinyle, un groupe isoindolinyle, un groupe oxoindolinyle, un groupe dioxoindolinyle, un groupe benzoxazolyle, un groupe oxobenzooxazolinyle, un groupe benzoisooxazolyle, un groupe oxobenzoisooxazolinyle, un groupe benzothiazolyle, un groupe oxobenzothiazolinyle, un groupe benzoisothiazolyle, un groupe oxobenzoisothiazolinyle, un groupe benzoimidazolyle, un groupe oxobenzoimidazolinyle, un groupe indazolyle, un groupe oxoindazolinyle, un groupe benzofurazanyle, un groupe benzothiadiazolyle, un groupe benzotriazolyle, un groupe oxazolopyridyle, un groupe oxodihydrooxazolopyridyle, un groupe thiazolopyridyle, un groupe oxodihydrothiazolopyridyle, un groupe isothiazolopyridyle, un groupe imidazopyridyle, un groupe oxodihydroimidazopyridyle, un groupe pyrazolopyridyle, un groupe oxodihydropyrazolopyridyle, un groupe thiénopyrimidinyle, un groupe chromanyle, un groupe chromanonyle, un groupe benzopyranyle, un groupe chromonyle, un groupe quinoloyle, un groupe isoquinoloyle, un groupe dihydroquinolyle, un groupe oxodihydroquinolinyle, un groupe tétrahydroquinolyle, un groupe oxotétrahydroquinolinyle, un groupe benzodioxanyle, un groupe quinoxalinyle, un groupe quinazolinyle, un groupe naphtypiridinyle, un groupe carbazolyle, un groupe tétrahydrocarbazolyle, un groupe oxotétrahydrocarbazolyle, un groupe pyridoindolyle, un groupe acridinyle, un groupe oxodihydroacridinyle, un groupe phénanthridinyle, un groupe dihydrophénanthridinyle, un groupe oxodihydrophénanthridinyle, un groupe dibenzoisoquinolinyle, un groupe dihydrodibenzoisoquinolinyle, un groupe oxodihydrodibenzoisoquinolinyle, un groupe phénothiazinyle, un groupe dihydrodibenzooxépinyle, un groupe oxodihydrodibenzooxépinyle, un groupe benzocycloheptathiényle, un groupe oxobenzocycloheptathiényle, un groupe dihydrothiénobenzothiépinyle, un groupe oxodihydrothiénobenzothiépinyle, un groupe dihydrodibenzothiépinyle, un groupe oxodihydrodibenzothiépinyle, un groupe octahydrodibenzothiépinyle, un groupe dibenzoazépinyle, un groupe dihydrodibenzoazépinyle, un groupe oxodihydrodibenzoazépinyle, un groupe octahydrodibenzoazépinyle, un groupe benzocycloheptapyridyle, un groupe oxobenzocycloheptapyridyle, un groupe pyridobenzoazépinyle, un groupe dihydropyridobenzoazépinyle, un groupe oxodihydropyridobenzoazépinyle, un groupe dihydropyridobenzodiazépinyle, un groupe dihydrodibenzooxazépinyle, un groupe dihydropyridobenzooxépinyle, un groupe dihydropyridobenzooxazépinyle, un groupe oxydihydropyridobenzooxazépinyle, un groupe dihydrodibenzothiazépinyle, un groupe oxodihydrodibenzothiazépinyle, un groupe dihydropyridobenzothiazépinyle et un groupe oxodihydropyridobenzothiazépinyle, liés directement ou sur un groupe méthylène ;
R¹⁴ a la même signification que R¹³ mais est choisi indépendamment de celui-ci ;
R¹⁵ est choisi parmi
un groupe hydroxy, un groupe méthyle, un groupe benzyle, un groupe phényle, un groupe indanyle, un groupe indényle, un groupe naphtyle, un groupe dihydronaphtyle, un groupe tétrahydronaphtyle, un groupe furyle, un groupe thiényle, un groupe pyrrolyle, un groupe oxazolyle, un groupe isoxazolyle, un groupe thiazolyle, un groupe isothiazolyle, un groupe pyrazolyle, un groupe imidazolyle, un groupe oxadiazolyle, un groupe thiadiazolyle, un groupe triazolyle, un groupe pyridyle, un groupe pyrazinyle, un groupe pyridazinyle, un groupe pyrimidinyle, un groupe triazinyle, un groupe benzofuryle, un groupe benzothiényle, un groupe indolyle, un groupe indolinyle, un groupe benzooxazolyle, un groupe benzothiazolyle, un groupe benzoimidazolyle, un groupe chromanyle, un groupe quinolyle et un groupe tétrahydroquinolyle, liés directement ou sur un groupe méthylène ;
G2 est choisi parmi 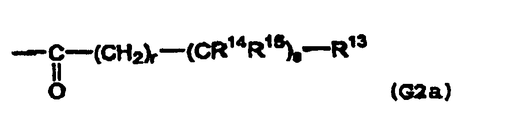 et 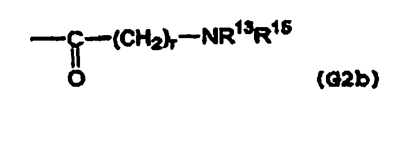 dans lesquelles r, s et les substituants R¹³ à R¹⁵ ont la signification ci-dessus, ou le groupe
-NR¹³R¹⁵
représente les groupes azétidine, pyrrolidine, pipéridine, (1H)-tétrahydropyridine, hexahydroazépine, (1H)-tétrahydroazépine, octahydroazocine, pyrazolidine, pipérazine, hexahydrodiazépine, morpholine, hexahydrooxazépine, thiomorpholine, thiomorpholin-1,1-dioxyde, 5-aza-bicyclo-[2.1.1]hexane, 2-aza-bicyclo[2.2.1]heptane, 7-aza-bicyclo-[2.2.1]heptane, 2,5-diaza-bicyclo[2.2.1]heptane, 2-aza-bicyclo[2.2.2]octane, 8-aza-bicyclo[3.2.1]octane, 2,5-diaza-bicyclo[2.2.2]octane, 9-aza-bicyclo[3.3.1]nonane, indoline, isoindoline, (1H)-dihydroquinoline, (1H)-tétrahydroquinoline, (2H)-tétrahydroisoquinoline, (1H)-tétrahydroquinoxaline, (4H)-dihydrobenzooxazine, (4H)-dihydrobenzothiazine, (1H)-tétrahydrobenzo[b]azépine, (1H)-tétrahydrobenzo[c]azépine, (1H)-tétrahydrobenzo[d]azépine, (5H)-tétrahydrobenzo[b]oxazépine, (5H)-tétrahydrobenzo[b]thiazépine, 1,2,3,4-tétrahydro-9H-pyrido[3,4-b]indole, (10H)-dihydroacridine, (10H)-dihydrophénanthridine, 1,2,3,4-tétrahydroacridanone, (10H)-phénoxazine, (10H)-phénothiazine, (5H)-dibenzoazépine, (5H)-dihydrodibenzoazépine, (5H)-octahydrodibenzoazépine, dihydrobenzo[d,e]isoquinoline, (5H)-dihydrodibenzodiazépine, (5H)-benzo[b]pyrido[f]azépine, (5H)-dihydrobenzo[b]pyrido[f]azépine, (11H)-dihydrodibenzo[b,e]oxazépine, (11H)-dihydrodibenzo[b,e]thiazépine, (10H)-dihydrodibenzo[b,f]oxazépine, (10H)-dihydrodibenzo[b,f]thiazépine, (5H)-tétrahydrodibenzoazocine, (11H)-dihydrobenzo[e]pyrido[b]-1,4-diazépin-6-one ou (11H)-dihydrobenzo[b]pyrido[e]-1,4-diazépin-5-one,
G3 représente
-SO₂-(CH₂)ᵣ-R¹³,
dans lequel r et R¹³ ont la signification ci-dessus,
G4 représente 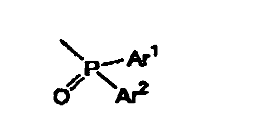 dans lequel
Ar¹ et Ar² sont choisis indépendamment l'un de l'autre parmi un groupe phényle, un groupe pyridyle et un groupe naphtyle ;
G5 représente
-COR¹⁶,
dans lequel
R¹⁶ est choisi parmi
un groupe trifluorométhyle, un groupe alcoxy en C₁ à C₆, un groupe alcényloxy en C₃ à C₆, et un groupe benzyloxy,
où les systèmes cycliques aromatiques dans les substituants sont éventuellement substitués indépendamment les uns des autres par un à trois des groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl (en C₁ à C₆)amino, et un groupe méthylène dioxyde dans le cas de deux résidus adjacents sur le noyau aromatique, et
où les résidus alkyle, alcényle et cycloalkyle dans les groupes G1, G2 et G3 sont éventuellement substitués par un ou deux des groupes identiques ou différents qui sont choisis parmi
un groupe hydroxy, un groupe carboxy, un groupe alcoxycarbonyle en C₂ à C₇, un groupe benzyloxycarbonyle, un groupe amino, un groupe monoalkyl(en C₁ à C₆)amino et un groupe dialkyl(en C₁ à C₆)amino.

4. Composé selon la revendication 2 ou 3, dans lequel
R¹ est choisi parmi
l'hydrogène, le fluor, le chlore, le brome, un groupe méthyl, un groupe éthyle, un groupe trifluorométhyle, un groupe hydroxy, un groupe alcoxy en C₁ à C₄, un groupe méthylthio, un groupe éthylthio, un groupe carboxy et un groupe phénoxy ;
R² est choisi parmi l'hydrogène, le chlore et un groupe méthyle ;
R³ est l'hydrogène ;
R⁴ est choisi parmi
l'hydrogène, un groupe alkyle en C₁ à C₃ et un groupe hydroxy,
k est 0
A est choisi parmi
un groupe alkylène en C₂ à C₆, qui est éventuellement substitué une fois ou deux fois par un groupe hydroxy ou le fluor ;
un groupe alkylène en C₂ à C₆, dans lequel une unité méthylène est remplacée de façon isostérique par O, S ou CO, dans lequel, à l'exception de CO, la substitution isostérique n'est pas adjacente au groupe amide ;
un groupe alcénylène en C₂ à C₆ qui est éventuellement substitué par un groupe alkyle en C₁ à C₃ et/ou le fluor ; et
un groupe alcadiénylène en C₄ à C₆ ;
D est choisi parmi
un groupe alkylène en C₂ à C₈ qui est éventuellement substitué par un groupe méthyle ou un groupe hydroxy ;
un groupe alcénylène en C₄ à C₈, qui est éventuellement substitué par un groupe hydroxy ;
un groupe alcynylène en C₄ à C₈, qui est éventuellement substitué par un groupe hydroxy ; et
le groupe constitué par un groupe alkylène en C₂ à C₈, un groupe alcénylène en C₄ à C₈, et un groupe alcynylène en C₄ à C₈ dans lequel une unité méthylène est remplacée de façon isostérique par O, NH, N(CH₃), CO ou SO₂ ou un groupe éthylène est remplacé de façon isostérique par un groupe NH-CO et/ou CO-NH ou un groupe propylène est remplacé de façon isostérique par un groupe NH-CO-O et/ou O-CO-NH ;
**E** est choisi parmi une pipérazine et une hexahydro-1,4-diazépine, où le cycle est éventuellement substitué par un ou deux groupes méthylène et/ou par un groupe oxo adjacent à un atome d'azote ;
G est choisi parmi l'hydrogène, un groupe cycloalkyle en C₃ à C₈, un groupe méthoxycarbonyle, un groupe tert-butoxycarbonyle, un groupe benzyloxycarbonyle, un groupe trifluoroacétyle, un groupe diphénylphosphinoyle,
- (CH₂)ᵣ-(CR¹⁴R¹⁵)ₛ-R¹³, 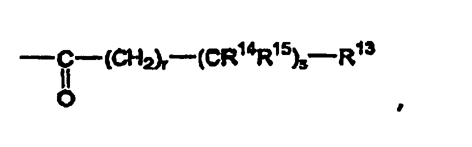 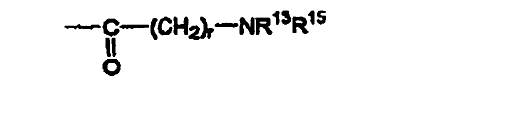
et
-SO₂-(CH₂)ᵣ-R¹³
où
r est 0 ou 1
s est 0 ou 1,
R¹³ est choisi parmi l'hydrogène, un groupe méthyle, un groupe benzyle, un groupe phényle,
le groupe constitué par un groupe indanyle, un groupe indényle, un groupe oxoindanyle, un groupe naphtyle, un groupe tétrahydronaphtyle, un groupe fluorényle, un groupe oxofluorényle, un groupe anthryle, un groupe dihydroanthryle, un groupe oxodihydroanthryle, un groupe dioxodihydroanthryle, un groupe phénanthryle, un groupe dihydrophénanthryle, un groupe oxydihydrophénanthryle, un groupe dibenzocycloheptényle, un groupe dihydrodibenzocycloheptényle, et un groupe oxodihydrodibenzocycloheptyle, liés directement ou sur un groupe méthylène, et
le groupe constitué par un groupe furyle, un groupe thiényle, un groupe oxazolyle, un groupe isooxazolyle, un groupe thiazolyle, un groupe imidazolyle, un groupe oxadiazolyle, un groupe pyridyle, un groupe pyrazinyle, un groupe pyrimidinyle, un groupe imidazothiazolyle, un groupe benzofuryle, un groupe benzothiényle, un groupe indolyle, un groupe indolinyle, un groupe oxoindolinyle, un groupe dioxoindolinyle, un groupe benzoxazolyle, un groupe oxobenzooxazolinyle, un groupe benzothiazolyle, un groupe oxobenzthiazolinyle, un groupe benzimidazolyle, un groupe oxobenzimidazolinyle, un groupe indazolyle, un groupe benzofurazanyle, un groupe benzotriazolyle, un groupe oxazolopyridyle, un groupe oxodihydrooxazolopyridyle, un groupe thiazolopyridyle, un groupe oxodihydrothiazolopyridyle, un groupe imidazopyridyle, un groupe oxodihydroimidazopyridyle, un groupe chromanyle, un groupe chromanonyle, un groupe benzopyranyle, un groupe chromonyle, un groupe quinolyle, un groupe isoquinolyle, un groupe oxodihydroquinolinyle, un groupe tétrahydroquinolyle, un groupe oxotétrahydroquinolinyle, un groupe benzodioxanyle, un groupe quinazolinyle, un groupe carbazolyle, un groupe acridinyle, un groupe dihydroacridinyle, un groupe oxodihydroacridinyle, un groupe dihydrophénanthridinyle, un groupe dihydrobenzoisoquinolinyle, un groupe phénothiazinyle, un groupe dihydrodibenzooxépinyle, un groupe benzocycloheptathiényle, un groupe dihydrothiénobenzothiépinyle, un groupe dihydrodibenzothiépinyle, un groupe oxodihydrodibenzothiépinyle, un groupe dihydrodibenzoazépinyle, un groupe oxodihydrodibenzoazépinyle, un groupe octahydrodibenzoazépinyle, un groupe benzocycloheptapyridyle, un groupe dihydropyridobenzodiazépinyle, et un groupe dihydrodibenzothiazépinyle, liés directement ou sur un groupe méthylène,
R¹⁴ est choisi parmi l'hydrogène, un groupe méthyle, un groupe benzyle, et un groupe phényle,
R¹⁵ est choisi parmi l'hydrogène, un groupe hydroxy, un groupe méthyle, un groupe benzyle, un groupe phényle ; et
le groupe constitué par un groupe naphtyle, un groupe tétrahydronaphtyle, un groupe furyle, un groupe thiényle, un groupe oxazolyle, un groupe thiazolyle, un groupe imidazolyle, un groupe pyridyle, un groupe benzofuryle, un groupe benzothiényle, un groupe indolyle, un groupe indolinyle, un groupe benzoxazolyle, un groupe benzothiazolyle, un groupe benzimidazolyle, un groupe chromanyle, un groupe quinolyle et un groupe tétrahydroquinolyle, liés directement ou sur un groupe méthylène ;
où le groupe
-NR¹³R¹⁵
représente un hétérocycle contenant de l'azote lié sur l'atome d'azote lequel hétérocycle contenant de l'azote est choisi parmi
un groupe pyrrolidine, un groupe pipéridine, un groupe hexahydroazépine, un groupe pipérazine, un groupe hexahydrodiazépine, un groupe morpholine, un groupe hexahydroazépine, un groupe thiomorpholine, un groupe 7-aza-bicyclo[2.2.1]heptane, un groupe 2,5-diaza-bicyclo[2.2.1]heptane, un groupe indoline, un groupe isoindoline, un groupe (1H)-dihydroquinoline, un groupe (1H)-tétrahydroquinoline, un groupe (2H)-tétrahydroisoquinoline, un groupe (4H)-dihydrobenzoxazine, un groupe (4H)-dihydrobenzothiazine, un groupe (1H)-tétrahydrobenzo[b]azépine, un groupe (1H)-tétrahydrobenzo[d]azépine, un groupe (5H)-tétrahydrobenzo[b]oxazépine, un groupe (5H)-tétrahydrobenzo[b]thiazépine, un groupe (10H)-dihydroacridine, un groupe 1,2,3,4-tétrahydroacridanone, un groupe (10H)-dihydrophénanthridine, un groupe (1H)-dihydrobenzo[d,e]isoquinoline, un groupe (10H)-phénothiazine, un groupe (5H)-dibenzo[b,f]azépine, un groupe (5H)-dihydrodibenzo[b,f]azépine, un groupe (5H)-dihydrodibenzo[c,e]azépine, un groupe (5H)-dihydrodibenzodiazépine, un groupe (11H)-dihydrodibenzo[b,e]oxazépine, un groupe (11H)-dihydrodibenzo[b,e]thiazépine, un groupe (5H)-dihydrobenzo[b]pyrido[3,2-f]azépine et un groupe (11H)-6-oxodihydrobenzo[e]pyrido[3,2-b][1,4]diazépine,
où les systèmes cycliques aromatiques dans les substituants sont éventuellement substitués, indépendamment les uns des autres, par un à trois des groupes identiques ou différents choisis parmi un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor, un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl(en C₁ à C₆)amino, et dans le cas de deux résidus adjacents sur le noyau aromatique, un groupe méthylènedioxy, et
où les résidus alkyle, alcényle et cycloalkyle dans le groupe G sont éventuellement substitué par un ou deux des groupes identiques ou différents qui sont choisis parmi un groupe hydroxy, un groupe carboxy, un groupe alcoxycarbonyle en C₂-C₇, un groupe benzyloxycarbonyle, un groupe amino, un groupe monoalkyl(en C₁ à C₆)amino et un groupe dialkyl(en C₁ à C₆)amino.

5. Composé selon l'une quelconque des revendications 2 à 4, dans lequel
R¹ est choisi parmi
l'hydrogène, le fluor, un groupe méthyle, un groupe trifluorométhyle et un groupe éthylthio ;
R², R³ et R⁴ sont chacun un hydrogène ;
k est 0,
A est choisi parmi
le groupe constitué par l'éthylène, le propylène et le butylène, qui sont chacun éventuellement substitués par un groupe hydroxy ou un ou deux atome de fluor ;
OCH₂, SCH₂ ;
un groupe éthénylène, et un groupe 1,3-butadiénylène ;
D est choisi parmi
un groupe alkylène en C₂ à C₆ qui est éventuellement substitué par un groupe hydroxy ;
un groupe alcénylène en C₄ à C₆ ;
un groupe alcynylène en C₄ à C₆ ; et
le groupe constitué par un groupe alkylène en C₂ à C₆, un groupe alcénylène en C₄ à C₆ et un groupe alcynylène en C₄ à C₆, dans lequel une ou deux unités méthylène sont remplacée de façon isostérique par O, NH, CO ou SO₂ ;
E est une pipérazine ou une hexahydro-1,4-diazépine ;
G est choisi parmi
un groupe phényle, un groupe benzyle, un groupe phénéthyle, un groupe diphénylméthyle, un groupe naphtyle, un groupe tétrahydronaphtyle, un groupe naphtylméthyle, un groupe fluorényle, un groupe fluorénylméthyle, un groupe anthrylméthyle, un groupe dihydrodibenzocycloheptényle, un groupe furylméthyle, un groupe thiénylméthyle, un groupe thiazolylméthyle, un groupe pyridylméthyle, un groupe benzothiénylméthyle, un groupe quinolylméthyle, un groupe phénylthiénylméthyle, un groupe phénylpyridylméthyle, un groupe benzocycloheptapyridinyle, un groupe dihydrobenzocycloheptapyridinyle, un groupe dihydrodibenzooxépinyle, un groupe dihydrodibenzothiépinyle, un groupe dihydrodibenzoazépinyle, un groupe dihydrobenzopyridodiazépinyle, un groupe formyle, un groupe acétyle, un groupe pivaloyle, un groupe phénylacétyle, un groupe diphénylacétyle, un groupe diphénylpropionyle, un groupe naphtylacétyle, un groupe benzoyle, un groupe naphtoyle, un groupe oxofluorénylcarbonyle, un groupe oxodihydroanthrylcarbonyle, un groupe dioxodihydroanthrylcarbonyle, un groupe furoyle, un groupe pyridylacétyle, un groupe pyridylcarbonyle, un groupe chromonylcarbonyle, un groupe quinolylcarbonyle, un groupe phénylylaminocarbonyle, un groupe naphtylaminocarbonyle, un groupe tétrahydronaphtylaminocarbonyle, un groupe dibenzylaminocarbonyle, un groupe benzylphénylaminocarbonyle, un groupe diphénylaminocarbonyle, un groupe indolinyl-N-carbonyle, un groupe isoindolinyl-N-carbonyle, un groupe tétrahydroquinolinyl-N-carbonyle, un groupe carbazolyl-N-carbonyle, un groupe tétrahydrobenzoazépinyl-N-carbonyle, un groupe dihydrodibenzoazépinyl-N-carbonyle, un groupe dihydrobenzopyridoazépinyl-N-carbonyle, un groupe oxodihydrobenzopyridoazépinyl-N-carbonyle, un groupe méthanesulfonyle, un groupe toluènesulfonyle, un groupe naphtylsulfonyle, un groupe quinolinesulfonyle et un groupe diphénylphosphinoyle,
où les systèmes cycliques aromatiques sont éventuellement substitués indépendamment les uns des autres par un à trois des groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl(en C₁ à C₆)amino, et dans le cas de deux résidus adjacents sur le noyau aromatique, un groupe méthylènedioxy, et
où les résidus alkyle, alcényle et cycloalkyle dans le groupe G sont éventuellement substitués par un ou deux des groupes identiques ou différents qui sont choisis parmi
un groupe hydroxy, un groupe carboxy, un groupe alcoxycarbonyle en C₂ à C₇, un groupe benzyloxycarbonyle, un groupe amino, un groupe monoalkyl(en C₁ à C₆)amino et un groupe dialkyl(en C₁ à C₆)amino.

6. Composé selon l'une quelconque des revendications 2 à 5, qui est choisi dans le groupe constitué par :
N-[4-(4-diphénylméthylpipérazin-1-yl)-3-hydroxybutyl]-3-pyridin-3-yl-acrylamide ;
N-[3-(4-diphénylméthylpipérazin-1-yl)-propoxy]-3-pyridin-3-yl-acrylamide ;
N-[4-(4-diphénylméthylpipérazin-1-yl)-4-oxo-butyl]-3-pyridin-3-yl-acrylamide ;
N-[3-(4-diphénylméthylpipérazin-1-sulfonyl)-propyl]-3-pyridin-3-yl-acrylamide ;
N-{2-[2-(4-diphénylméthylpipérazin-1-yl)-éthoxy]-éthyl}-3-pyridin-3-yl-acrylamide ;
N-(4-[bis-(4-fluorophényl)-méthyl]-pipérazin-1-1-yl}-but-2-in-yl)-3-yl-acrylamide ;
N-{4-[4-(4-carboxyphényl-phénylméthyl)-pipérazin-1-yl]-butyl}-3-pyridin-3-yl-acrylamide ;
N-[4-{4-[(4-aminophényl)-phénylméthyl]-pipérazin-1-yl}butyl)-3-pyridin-3-yl-acrylamide ;
N-{4-[4-(9H-fluorèn-9-yl)-pipérazin-1-yl]-butyl}-2-(pyridin-3-yloxy)-acétamide ;
N-{5-[4-(9H-fluorèn-9-yl)-pipérazin-1-yl]-pentyl}-3-pyridin-3-yl-acrylamide ;
N-{6-[4-(9H-fluorèn-9-yl)-pipérazin-1-yl]-hexyl}-3-pyridin-3-yl-acrylamide ;
3-pyridin-3-yl-N-{4-[4-(1,2,3,4-tétrahydronaphtalèn-1-yl)-pipérazin-1-yl]-butyl}-acrylamide ;
3-pyridin-3-yl-N-{4-[4-(5,6,7,8-tétrahydronaphtalèn-1-yl)-pipérazin-1-yl]-butyl}-acrylamide ;
N-{4-[4-(naphtalèn-1-yl)-pipérazin-1-yl]-butyl}-3-pyridin-3-yl-acrylamide ;
N-[4-(4-biphényl-2-yl-pipérazin-1-yl)-butyl]-3-pyridin-3-yl-propionamide ;
N-[5-(4-biphényl-2-yl-pipérazin-1-yl)-pentyl]-3-pyridin-3-yl-acrylamide ;
N-[6-(4-biphényl-2-yl-pipérazin-1-yl)-hexyl]-3-pyridin-3-yl-acrylamide ;
N-[4-(4-biphényl-2-yl-pipérazin-1-yl)-butyl]-2-(pyridin-3-yloxy)-acétamide ;
amide de l'acide N-[4-(4-biphényl-2-yl-pipérazin-1-yl)-butyl]-5-(pyridin-3-yl)-penta-2,4-diénoique ;
N-{4-[4-(10,11-dihydro-5H-dibenzo[a,d]cycloheptèn-5-yl)-pipérazin-1-yl]-butyl}-3-pyridin-3-yl-propionamide ;
N-{5-[4-(10,11-dihydro-5H-dibenzo[a,d]cycloheptèn-5-yl)-pipérazin-1-yl]-pentyl}-3-pyridin-3-yl-acrylamide ;
N-{6-[4-(10,11-dihydro-5H-dibenzo[a,d]cycloheptèn-5-yl)-pipérazin-1-yl]-hexyl}-3-pyridin-3-yl-acrylamide ;
amide de l'acide N-{4-[4-(10,11-dihydro-5H-dibenzo[a,d]cycloheptèn-5-yl)-pipérazin-1-yl]-butyl}-5-(pyridin-3-yl)-penta-2,4-diénoïque ;
N-{4-[4-(6,11-dihydro-dibenzo[b,e]oxépin-11-yl)-pipérazin-1-yl]-butyl}-3-pyridin-3-yl-propionamide ;
N-{2-[4-(6,11-dihydrodibenzo[b,e]thiépin-11-yl)-pipérazin-1-yl]-éthyl}-3-pyridin-3-yl-acrylamide ;
N-[4-(4-diphénylacétyl-pipérazin-1-yl)-butyl]-3-pyridin-3-yl-acrylamide ;
N-[4-(4-benzoylpypérazin-1-yl)-butyl]-3-pyridin-3-yl-acrylamide ;
N-{4-[4-(2-aminobenzoyl)-pipérazin-1-yl]-butyl}-3-pyridin-3-yl-acrylamide ;
N-{4-[4-(4-carboxybenzoyl)-pipérazin-1-yl]-butyl}-3-pyridin-3-yl-acrylamide ;
N-{4-[4-(biphényl-2-carbonyl)-pipérazin-1-yl]-butyl}-3-pyridin-3-yl-acrylamide ;
N-{4-[4-(9-oxo-9H-fluorèn-4-carbonyl)-pipérazin-1-yl]-butyl}-3-pyridin-3-yl-acrylamide ;
N-{4-[4-(furan-2-carbonyl)-pipérazin-1-yl]-butyl}-3-pyridin-3-yl-acrylamide ;
N-{4-[4-(naphtalèn-1-yl-aminocarbonyl)-pipérazin-1-yl]-butyl}-3-pyridin-3-yl-propionamide ;
N-{4-[4-(diphénylaminocarbonyl)-pipérazin-1-yl]-butyl}-3-pyridin-3-yl-acrylamide ;
N-{4-[4-(naphtalèn-2-sulfonyl)-pipérazin-1-yl]-butyl}-3-pyridin-3-yl-acrylamide ;
N-[4-(4-diphénylphosphinoyl-pipérazin-1-yl)-butyl]-3-pyridin-3-yl-acrylamide ;
N-[4-(4-biphényl-2-yl-pipérazin-1-yl)-butyl}-3-pyridin-3-yl-acrylamide ;
N-{4-[4-(9H-fluorèn-9-yl)-pipérazin-1-yl]-butyl}-3-pyridin-3-yl-acrylamide ; et
N-{4-[4-(10,11-dihydro-5H-dibenzo[a,d]cyclohepten-5-yl)-pipérazin-1-yl]-butyl}-3-pyridin-3-yl-acrylamide.

7. Composé selon l'une quelconque des revendications 2 à 6, qui est présent
comme isomère cis et/ou trans, isomère E et/ou Z, isomère pur et/ou mélange d'isomères, comme énantiomère et/ou diastéréoisomère, comme mélange racémique ou non racémique, comme endo/exo isomère dans le cas où le système cyclique E est bicyclique,
comme tautomère, lorsque G est un cycle aromatique hétérocyclique ou le contient dans un système cyclique condensé, lorsque cet hétérocycle est substitué par des groupes hydroxy, mercapto ou amino libres.
comme sel d'addition d'acide pharmacologiquement acceptable avec des acides inorganiques ou organiques, tels que les chlorhydrates, les bromhydrates, les iodhydrates, les sulfates, les phosphates, les acétates, les benzoates, les citrates, les fumarates, les gluconates, les malates, les maléates, les méthanesulfonates, les lactates, les oxalates, les succinates, les tartrates et/ou les tosylates, et/ou
comme hydrate ou autre produit de solvatation.

8. Procédé pour la production d'un composé selon l'une quelconque des revendications 1 à 7, lequel procédé est choisi parmi les (A), (B), (B1), (B2), (B3) et (B4) qui suivent :
(A) des acides carboxyliques de formule (II) 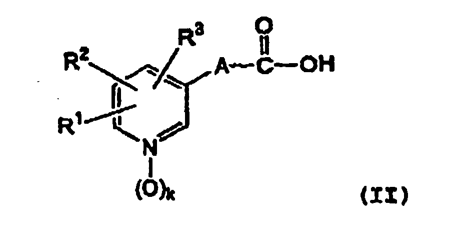 dans laquelle R¹, R², R³, A et k ont la signification donnée dans l'une quelconque des revendications 1 à 7 ou leurs dérivés réactifs, en particulier leurs chlorures d'acide ou esters activés, sont mis à réagir, éventuellement en présence d'agents de condensation, avec des composés de formule (III) 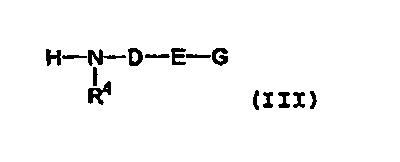 dans laquelle D, E, G et R⁴ sont définis comme dans les revendications 1 à 7 sous la forme de la base libre respective ou du sel d'addition d'acide respectif, de préférence dans un ou plusieurs solvants inertes, à une température entre -40°C et 180°C éventuellement en présence d'une base auxiliaire ;
(B) des composés de formule (I), dans laquelle G est l'hydrogène, sont mis à réagir avec un composé de formule (IV)
L-G (IV)
dans laquelle G a la signification donnée dans l'une quelconque des revendications 1 à 7, à l'exception de l'hydrogène, et L est un nucléofuge ou groupe réactif approprié,
(B1) des composés de formule (I), dans laquelle G est l'hydrogène, sont mis à réagir avec un agent d'alkylation et/ou agent d'arylation approprié de formule (IV)
L-G (IV)
dans laquelle G est un résidu alkyle, alcényle, alcynyle, cycloalkyle, aryle, aralkyle, hétéroaryle ou hétéroaralkyle et le groupe partant L représente un dérivé réactif d'un alcool choisi parmi un atome d'halogène, tel qu'un chlore, un brome, ou un iode ; un ester d'acide sulfonique, un groupe méthanesulfonyloxy, un groupe trifluorométhanesulfonyloxy, un groupe éthanesulfonyloxy, un groupe benzènesulfonyloxy, un groupe p-toluènesulfonyloxy, un groupe p-bromobenzènesulfonyloxy, un groupe m-nitrobenzènesulfonyloxy et un groupe époxy terminal,
dans lequel cette réaction se produit dans un solvant approprié inerte à une température entre 0°C et 180°C, selon la réactivité de l'éduit,
(B2) des composés de formule (I), dans laquelle G représente un hydrogène, sont mis à réagir avec un acide carboxylique, un acide carbamique, un acide sulfonique et/ou un acide phosphinique de formule (V),
HO-G (V)
dans laquelle G est un résidu acyle, un résidu carbamoyle, un résidu sulfonyle ou un résidu phosphinoyle, ou leurs dérivés capables de réaction, laquelle réaction se produit de préférence en présence de bases auxiliaires dans des solvants et dans des conditions telles qu'elles sont décrites dans le procédé (A) ;
(B3) des composés de formule (I), dans laquelle G est un hydrogène, sont mis à réagir avec un transmetteur de groupe carbonyle vers un produit intermédiaire et ensuite avec une amine primaire ou secondaire avec la formule (VI)
H-NR¹³R¹⁵ (VI)
dans laquelle R¹³ et R¹⁵ et/ou le groupe -NR¹³R¹⁵ ont la signification donnée dans l'une quelconque des revendications 1 à 7 sans purification ou isolation du produit intermédiaire, de préférence le composé (VI) est ajouté dans une quantité stoechiométrique ou en excès sous la forme d'une solution ou d'un solide et la réaction est menée à bonne fin, où les températures se situent entre -40°C et 50°C pour la première réaction partielle et entre 0°C et 150°C pour la deuxième réaction partielle.
(B4) un composé de formule (I) selon l'une quelconque des revendications 1 à 7, dans lequel G est l'hydrogène, est mis à réagir avec un isocyanate de formule (VII)
O=C=N-R¹³ (VII)
dans laquelle R¹³ a la signification donnée dans l'une quelconque des revendications 1 à 7, dans un solvant inerte absolu à une température de -20°C à 150°C.

9. Composition pharmaceutique comprenant un ou plusieurs des composés selon l'une quelconque des revendications 1 à 7 éventuellement ensemble avec (a) un(des) support(s) pharmaceutiquement acceptable(s), (a) un adjuvant toxicologiquement sans danger(s), et/ou en combinaison avec d'autres ingrédients actifs.

10. Composition pharmaceutique selon la revendication 9, qui est présente sous une forme solide administrable par voie perorale sous la forme d'un comprimé, d'une capsule, d'un comprimé enrobé, ou en tant que solution, suspension, comprimé effervescent, administrable sous une forme liquide par voie perorale, sous la forme de comprimés ou de sachets, éventuellement sous une forme à action modifiée ou résistante au fluide gastrique,
sous la forme d'une préparation injectable ou perfusable appropriée conjointement avec des supports et adjuvants pharmaceutiquement acceptables appropriés, éventuellement sous une forme à action modifiée ou sous une forme médicinale de dépôt parentéral ou d'implant ou sous la forme d'un concentré, d'une poudre ou d'un produit lyophilisé,
sous la forme d'un système thérapeutique transdermique pour un traitement systémique,
sous la forme d'un système thérapeutique gastro-intestinal (GITS) pour un traitement systémique,
sous la forme d'un onguent, d'une suspension, d'une émulsion, d'un baume ou d'un emplâtre ou sous la forme d'une solution applicable par voie externe,
sous la forme d'émulsions administrables par voie rectale, génitale, ou transuréthrale, d'une solution, d'une solution liposomale, d'un implant, d'un suppositoire ou d'une capsule,
sous la forme d'une composition capable d'être appliquée de façon nasale, otologique ou ophtalmologique, ou
sous une forme applicable buccalement.

11. Composition pharmaceutique selon la revendication 9, laquelle est sous la forme d'un agent thérapeutique d'inhalation, éventuellement sous la forme d'un pulvérisateur avec des propulseurs, supports et adjuvants pharmaceutiquement acceptables appropriés.

12. Composition pharmaceutique selon la revendication 11 pour une administration au moyen d'un aérosol à dosage contrôlé ou sous la forme d'une formulation à dosage de poudre sèche.

13. Composition pharmaceutique selon l'une quelconque des revendications 9 à 12, dans laquelle une unité posologique pour une seule administration contient 0,001 à 2,0 mg, de un ou plusieurs des composés selon l'une quelconque des revendications 1 à 7.

14. Composition pharmaceutique selon l'une quelconque des revendications 9 à 12, dans laquelle une unité posologique pour une seule administration contient 0,1, 1, 2, 5, 10, 20, 25, 30, 50, 100, 200, 300, 500, 600, 800, 1000, 2000, 3000, 4000 ou 5000 mg de un ou plusieurs des composés selon l'une quelconque des revendications 1 à 7.

15. Utilisation d'un ou plusieurs des composés selon la formule (I) : 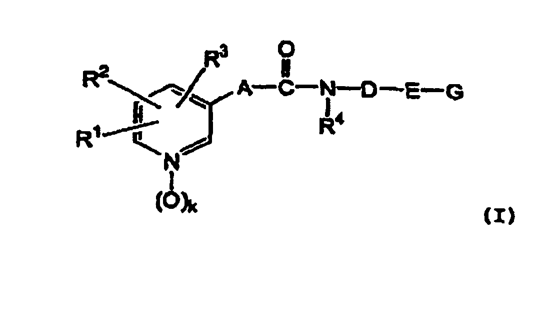 pour la fabrication d'une composition pharmaceutique pour le traitement du cancer et/ou de la croissance cellulaire anormale, et/ou la prévention de la prolifération et/ou la formation de métastases, dans le corps humain ou animal, éventuellement en combinaison avec un autre agent cytostatique ou agent immunosuppresseur,
dans laquelle :
R¹ est choisi parmi
l'hydrogène, un halogène, un groupe cyano, un groupe alkyle en C₁ à C₆, un groupe alcényle en C₃ à C₆, un groupe alcynyle en C₂ à C₆, un groupe trifluorométhyle, un groupe cycloalkyle en C₃ à C₈, un groupe hydroxyalkyle en C₁ à C₆, un groupe hydroxy, un groupe alcoxy en C₁ à C₆, un groupe alcényloxy en C₃ à C₆, un groupe alcynyloxy en C₃ à C₆, un groupe benzyloxy, un groupe alcanoyloxy en C₁ à C₇, un groupe alcoxycarbonyloxy en C₂ à C₇, un groupe alkylthio en C₁ à C₆, un groupe alcénylthio en C₃ à C₆, un groupe alcynylthio en C₃ à C₆, un groupe cycloalkyloxy en C₃ à C₈, un groupe cycloalkylthio en C₃ à C₈, un groupe alcoxycarbonyle en C₂ à C₇, un groupe aminocarbonyle, un groupe alkylaminocarbonyle en C₂ à C₇, un groupe dialkylaminocarbonyle en C₃ à C₁₃, un groupe carboxy, un groupe phényle, un groupe phénoxy, un groupe phénylthio, un groupe pyridyloxy, un groupe pyridylthio, et un groupe NR⁵R⁶, dans lequel
R⁵ et R⁶ sont choisis indépendamment l'un de l'autre parmi
l'hydrogène, un groupe alkyle en C₁ à C₆, un groupe alcényle en C₃ à C₆, un groupe alcynyle en C₃ à C₆, un groupe benzyle et un groupe phényle ;
R² est choisi parmi
l'hydrogène, un halogène, un groupe cyano, un groupe alkyle en C₁ à C₆, un groupe trifluorométhyle, un groupe hydroxy, un groupe alcoxy en C₁ à C₆, un groupe benzyloxy et un groupe alcanoyloxy en C₁ à C₇
ou
R¹ et R², s'ils sont adjacents, forment de façon facultative un pont choisi parmi -(CH₂)₄-, -(CH=CH)₂- et -CH₂O-CR⁷R⁸-O-, dans lequel
R⁷ et R⁸ sont choisis indépendamment l'un de l'autre parmi l'hydrogène et un groupe alkyle en C₁ à C₆ ;
R³ est choisi parmi
l'hydrogène, un halogène, un groupe alkyle en C₁ à C₆, un groupe trifluorométhyle et un groupe hydroxyalkyle en C₁ à C₆ ;
R⁴ est choisi parmi
l'hydrogène, un groupe alkyle en C₁ à C₆, un groupe alcényle en C₃ à C₆, un groupe alcynyle en C₃ à C₆, un groupe cycloalkyle en C₃ à C₆, un groupe hydroxy, un groupe alcoxy en C₁ à C₆ et un groupe benzyloxy ;
k est 0 ou 1,
A est choisi parmi
un groupe alkylène en C₁ à C₆, éventuellement substitué de une à trois fois par un groupe alkyle en C₁ à C₃, un groupe hydroxy, un groupe alcoxy en C₁ à C₃, le fluor, ou un groupe phényle,
un groupe alkylène en C₂ à C₆, dans lequel une unité méthylène est remplacée de façon isostérique par O, S, NR⁹, CO, SO ou SO₂, dans lequel, à l'exception de CO, la substitution isostérique n'est pas adjacente au groupe amide et R⁹ est choisi parmi l'hydrogène, un groupe alkyle en C₁ à C₆, un groupe alcényle en C₃ à C₆, un groupe alcynyle en C₃ à C₆, un groupe acyle en C₁ à C₆ et un groupe alcanesulfonyle en C₁ à C₆,
un groupe 1,2-cyclopropylène,
un groupe alcénylène en C₂ à C₆, éventuellement substitué de une à trois fois par un groupe alkyle en C₁ à C₃, un groupe hydroxy, un groupe alcoxy en C₁ à C₃, le fluor, un groupe cyano ou un groupe phényle,
un groupe alcadiénylène en C₄ à C₆, éventuellement substitué une fois ou deux fois par un groupe alkyle en C₁ à C₃, le fluor, un groupe cyano ou un groupe phényle ;
un groupe 1,3,5-hexatriénylène, éventuellement substitué par un groupe alkyle en C₁ à C₃, le fluor, un groupe cyano ou un groupe phényle, et
un groupe éthynylène
D est choisi parmi
un groupe alkylène en C₂ à C₁₀, éventuellement substitué une fois ou deux fois par un groupe alkyle en C₁ à C₆, un groupe hydroxy, ou un groupe alcoxy en C₁ à C₆ ;
un groupe alcénylène en C₄ à C₁₀, éventuellement substitué une fois ou deux fois par un groupe alkyle en C₁ à C₆, un groupe hydroxy, ou un groupe alcoxy en C₁ à C₆ ;
un groupe alcynylène en C₄ à C₁₀, éventuellement substitué une fois ou deux fois par un groupe alkyle en C₁ à C₆, un groupe hydroxy, ou un groupe alcoxy en C₁ à C₆ ; et
le groupe constitué par un groupe alkylène en C₂ à C₁₀, un groupe alcénylène en C₄ à C₁₀ et un groupe alcynylène en C₄ à C₁₀, dans lequel une à trois unités méthylène sont remplacées de façon isostérique par O, S, NR¹⁰, CO, SO ou SO₂, dans lequel
R¹⁰ a la même signification que R⁹, mais est choisi indépendamment de celui-ci;
E représente 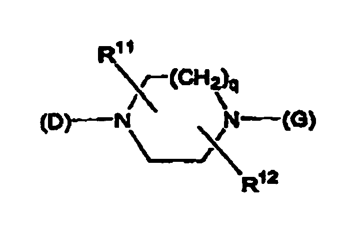 dans lequel
q est 1, 2 ou 3 ;
R¹¹ est choisi parmi
l'hydrogène, un groupe alkyle en C₁ à C₆, un groupe hydroxy, un groupe hydroxyméthyle, un groupe carboxy, et un groupe alcoxycarbonyle en C₂ à C₇, et
R¹² est choisi parmi
l'hydrogène, un groupe alkyle en C₁ à C₆ et un groupe oxo adjacent à un atome d'azote,
ou R¹¹ et R¹² forment ensemble un pont alkylène en C₁ à C₃ lors d'une formation d'un système à noyau bicyclique ;
G est choisi parmi G1, G2, G3, G4 et G5, dans lesquels
G1 représente
- (CH₂)ᵣ-(CR¹⁴R¹⁵)ₛ-R¹³,
r est 0, 1, 2 ou 3,
s est 0 ou 1
R¹³ est choisi parmi
l'hydrogène, un groupe alkyle en C₁ à C₆, un groupe alcényle en C₃ à C₆, un groupe alcynyle en C₃ à C₆, un groupe cycloalkyle en C₃ à C₈ ;
le groupe constitué des hétérocycles de quatre à huit membres, saturés et insaturés, qui contiennent éventuellement un ou deux hétéroatomes choisis parmi N, S et O ;
un groupe benzyle, un groupe phényle ;
le groupe constitué des hétérocycles de cinq à six membres aromatiques monocycliques, qui contiennent éventuellement un à trois hétéroatomes choisis parmi N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
R¹⁴ a la même signification que R¹³, mais est choisi indépendamment de celui-ci ;
R¹⁵ est choisi parmi
l'hydrogène, un groupe hydroxy, un groupe méthyle, un groupe benzyle, un groupe phényle,
le groupe constitué des hétérocycles de cinq et six membres aromatiques monocycliques, qui contiennent un à trois hétéroatomes du groupe N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
G2 est choisi parmi 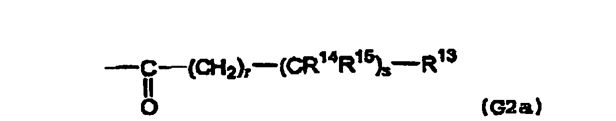 et 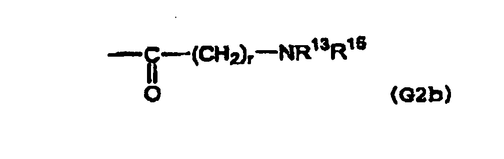 dans lesquels r, s et les substituants R¹³ à R¹⁵ ont la signification ci-dessus, ou le groupe
-NR¹³R¹⁵
représente un hétérocycle contenant de l'azote relié sur l'atome d'azote lequel hétérocycle contenant de l'azote est choisi parmi
le groupe constitué des hétérocycles de quatre à huit membres, monocycliques, saturés et insaturés, qui, en plus de l'atome d'azote essentiel, contiennent éventuellement un ou deux autres hétéroatomes choisis parmi N, S et O, et
le groupe constitué des hétérocycles bi- ou tricyclique condensés ou pontés, saturés et insaturés, avec 8 à 16 atomes de cycle, qui en plus de l'atome d'azote essentiel contiennent éventuellement un ou deux autres hétéroatomes choisis parmi N, S et O ;
G3 représente
-SO₂-(CH₂)ᵣ-R¹³,
dans lequel r et R¹³ ont les significations ci-dessus,
G4 représente 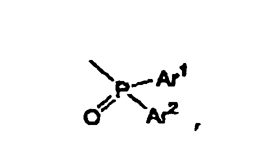 dans lequel
Ar¹ et Ar² sont choisis indépendamment l'un de l'autre parmi un groupe phényle, un groupe pyridyle et un groupe naphtyle ;
G5 représente
-COR¹⁶
dans lequel
R¹⁶ est choisi parmi
un groupe trifluorométhyle, un groupe alcoxy en C₁ à C₆, un groupe alcényloxy en C₃ à C₆, et un groupe benzyloxy,
où les systèmes cycliques aromatiques dans les substituants R¹, R², R⁴, R⁵, R⁶, R¹³, R¹⁴, R¹⁶, Ar¹ et Ar² et/ou dans le système cyclique -NR¹³R¹⁵ sont éventuellement substitués indépendamment les uns des autres par un à trois groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl(en C₁ à C₆)amino, et un groupe méthylène dioxyde pour deux résidus adjacents sur le noyau aromatique, et où
les résidus alkyle, alcényle et cycloalkyle dans les groupes G1, G2 et G3 sont éventuellement substitués par un ou deux des groupes identiques ou différents qui sont choisis parmi un groupe hydroxy, un groupe carboxy, un groupe alcoxycarbonyle en C₂ à C₇, un groupe benzyloxycarbonyle, un groupe amino, un groupe monoalkyl(en C₁ à C₆)amino et un groupe dialkyl(en C₁ à C₆)amino ;
leurs isomères cis et trans, leurs isomères E et Z, leurs énantiomères, leurs diastéréoisomères et d'autres isomères ainsi que leurs mélanges racémiques ou non racémiques et les isomères endo et exo correspondants dans le cas où le système cyclique E est bicyclique ;
leurs tautomères ; ainsi que
leurs sels d'addition d'acide comprenant leurs hydrates et produits de solvatation.

16. Utilisation selon la revendication 15, dans laquelle
dans laquelle
R¹ est choisi parmi
l'hydrogène, un halogène, un groupe cyano, un groupe alkyle en C₁ à C₆, un groupe trifluorométhyle, un groupe cycloalkyle en C₃ à C₈, un groupe hydroxyalkyle en C₁ à C₆, un groupe hydroxy, un groupe alcoxy en C₁ à C₄, un groupe benzyloxy, un groupe alkylthio en C₁ à C₄, un groupe alcanoyloxy en C₁ à C₅, un groupe alcoxycarbonyle en C₂ à C₅, un groupe aminocarbonyle, un groupe alkylaminocarbonyle en C₂ à C₅, un groupe dialkylaminocarbonyle en C₃ à C₉, un groupe carboxy, un groupe phényle, un groupe phénoxy, un groupe phénylthio, un groupe pyridyloxy, et un groupe NR⁵R⁶, dans lequel
R⁵ et R⁶ sont choisis indépendamment l'un de l'autre parmi l'hydrogène et un groupe alkyle en C₁ à C₆ ;
R² est choisi parmi
l'hydrogène, un halogène, un groupe cyano, un groupe alkyle en C₁ à C₆, un groupe trifluorométhyle, un groupe hydroxy, et un groupe alcoxy en C₁ à C₄ ;
R³ est choisi parmi
l'hydrogène, un halogène et un groupe alkyle en C₁ à C₆ ;
R⁴ est choisi parmi
l'hydrogène, un groupe alkyle en C₁ à C₆, un groupe alcényle en C₃ à C₆, un groupe cycloalkyle en C₃ à C₆, un groupe hydroxy, un groupe alcoxy en C₁ à C₆ et un groupe benzyloxy ;
k est ou 1,
A est choisi parmi
un groupe alkylène en C₁ à C₆, éventuellement substitué une fois à trois fois par un groupe alkyle en C₁ à C₃, un groupe hydroxy, le fluor, ou un groupe phényle,
un groupe alkylène en C₂ à C₆, dans lequel une unité méthylène est remplacée de façon isostérique par O, S, NR⁹, CO, SO ou SO₂, dans lequel, à l'exception de CO, la substitution isostérique n'est pas adjacente au groupe amide et le résidu R⁹ est choisi parmi l'hydrogène, un groupe alkyle en C₁ à C₆, un groupe acyle en C₁ à C₆ et un groupe méthanesulfonyle ;
un groupe 1,2-cyclopropylène,
un groupe alcénylène en C₂ à C₆, éventuellement substitué de une à trois fois par un groupe alkyle en C₁ à C₃, un groupe hydroxy, le fluor, un groupe cyano ou un groupe phényle,
un groupe alcadiénylène en C₄ à C₆, éventuellement substitué une fois ou deux fois par un groupe alkyle en C₁ à C₃, le fluor, un groupe cyano ou un groupe phényle ;
un groupe 1,3,5-hexatriénylène, éventuellement substitué par un groupe alkyle en C₁ à C₃, le fluor ou un groupe cyano, et
un groupe éthynylène
D est choisi parmi
un groupe alkylène en C₂ à C₁₀, éventuellement substitué une fois ou deux fois par un groupe alkyle en C₁ à C₃ ou un groupe hydroxy ;
un groupe alcénylène en C₄ à C₁₀, éventuellement substitué une fois ou deux fois par un groupe alkyle en C₁ à C₃ ou un groupe hydroxy ;
un groupe alcynylène en C₄ à C₁₀, éventuellement substitué une fois ou deux fois par un groupe alkyle en C₁ à C₃ ou un groupe hydroxy ; et
le groupe constitué par un groupe alkylène en C₂ à C₁₀, un groupe alcénylène en C₄ à C₁₀ et un groupe alcynylène en C₄ à C₁₀, dans lequel une à trois unités méthylène sont remplacées de façon isostérique par O, S, NR¹⁰, CO, SO ou SO₂, dans lequel
R¹⁰ a la même signification que R⁹, mais est choisi indépendamment de celui-ci;
E représente 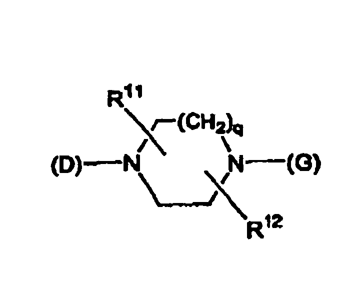 dans lequel
q est 1, 2 ou 3 ;
R¹¹ est choisi parmi
l'hydrogène, un groupe alkyle en C₁ à C₃, un groupe hydroxy, un groupe hydroxyméthyle, un groupe carboxy, et un groupe alcoxycarbonyle en C₂ à C₇, et
R¹² est choisi parmi l'hydrogène et un groupe oxo adjacent à un atome d'azote ou
R¹¹ et R¹², ensemble, forment un pont alkylène en C₁ à C₃ lors d'une formation d'un système à noyau bicyclique ;
G est choisi parmi G1, G2, G3, G4 et G5, dans lesquels
G1 représente
- (CH₂)ᵣ-(CR¹⁴R¹⁵)ₛ-R¹³,
r est 0, 1 ou 2,
s est 0 ou 1
R¹³ est choisi parmi
l'hydrogène, un groupe alkyle en C₁ à C₆, un groupe alcényle en C₃ à C₆, un groupe alcynyle en C₃ à C₆, un groupe cycloalkyle en C₃ à C₈ ; un groupe benzyle, un groupe phényle ;
le groupe constitué des hétérocycles de cinq à six membres aromatiques monocycliques, qui contiennent un à trois hétéroatomes choisis parmi N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
R¹⁴ a la même signification que R¹³, mais est choisi indépendamment de celui-ci ;
R¹⁵ est choisi parmi
l'hydrogène, un groupe hydroxy, un groupe méthyle, un groupe benzyle, un groupe phényle,
le groupe constitué des hétérocycles de cinq et six membres aromatiques monocycliques, qui contiennent un à trois hétéroatomes du groupe N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
G2 est choisi parmi 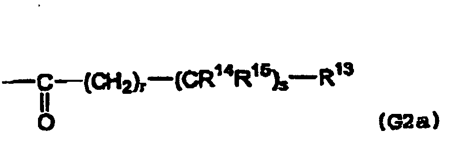 et 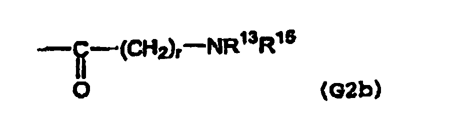 dans lesquels r, s et les substituants R¹³ à R¹⁵ ont la signification ci-dessus, ou le groupe
-NR¹³R¹⁵
représente un hétérocycle contenant de l'azote relié sur l'atome d'azote lequel hétérocycle contenant de l'azote est choisi parmi
le groupe constitué des hétérocycles de quatre à huit membres, monocycliques, saturés et insaturés, qui, en plus de l'atome d'azote essentiel, contiennent éventuellement un ou deux autres hétéroatomes choisis parmi N, S et O, et
le groupe constitué des hétérocycles bi- ou tricyclique condensés ou pontés, saturés et insaturés, avec 8 à 16 atomes de cycle, qui en plus de l'atome d'azote essentiel contiennent éventuellement un ou deux autres hétéroatomes choisis parmi N, S et O ;
G3 représente
-SO₂-(CH₂)ᵣ-R¹³,
dans lequel r et R¹³ ont les significations ci-dessus,
G4 représente 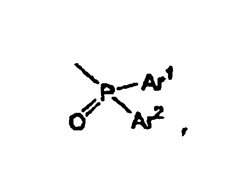 dans lequel
Ar¹ et Ar² sont choisis indépendamment l'un de l'autre parmi un groupe phényle, un groupe pyridyle et un groupe naphtyle ;
G5 représente
-COR¹⁶
dans lequel
R¹⁶ est choisi parmi
un groupe trifluorométhyle, un groupe alcoxy en C₁ à C₆, un groupe alcényloxy en C₃ à C₆, et un groupe benzyloxy,
où les systèmes cycliques aromatiques dans les substituants R¹, R², R⁴, R⁵, R⁶, R¹³, R¹⁴, R¹⁶, Ar¹ et Ar² et/ou dans le système cyclique -NR¹³R¹⁵ sont éventuellement substitués indépendamment les uns des autres par un à trois groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl (en C₁ à C₆)amino, et un groupe méthylène dioxyde dans le cas de deux résidus adjacents sur le noyau aromatique, et où
les résidus alkyle, alcényle et cycloalkyle dans les groupes G1, G2 et G3 sont éventuellement substitués par un ou deux des groupes identiques ou différents qui sont choisis parmi
un groupe hydroxy, un groupe carboxy, un groupe alcoxycarbonyle en C₂ à C₇, un groupe benzyloxycarbonyle, un groupe amino, un groupe monoalkyl(en C₁ à C₆)amino et un groupe dialkyl(en C₁ à C₆)amino.

17. Utilisation selon la revendication 15 ou 16, dans laquelle
R¹ est choisi parmi
l'hydrogène, un halogène, un groupe cyano, un groupe méthyle, un groupe éthyle, un groupe trifluorométhyle, un groupe hydroxy, un groupe alcoxy en C₁ à C₄, un groupe benzyloxy, un groupe alcanoyloxy en C₁ à C₅, un groupe méthylthio, un groupe éthylthio, un groupe méthoxycarbonyle, un groupe tert-butoxycarbonyle, un groupe aminocarbonyle, un groupe carboxy, un groupe phénoxy, et un groupe phénylthio,
R² est choisi parmi
l'hydrogène, un halogène, un groupe trifluorométhyle, et un groupe hydroxy,
R³ est choisi parmi
l'hydrogène et un halogène,
R⁴ est choisi parmi
l'hydrogène, un groupe alkyle en C₁ à C₃, un groupe allyle, un groupe hydroxy, et un groupe alcoxy en C₁ à C₃ ;
k est 0 ou 1,
A est choisi parmi
un groupe alkylène en C₁ à C₆, éventuellement substitué une fois ou deux fois par un groupe alkyle en C₁ à C₃, un groupe hydroxy ou le fluor ;
un groupe alkylène en C₂ à C₆, dans lequel une unité méthylène est remplacée de façon isostérique par O, S, NR⁹, CO, SO ou SO₂, dans lequel, à l'exception de CO, la substitution isostérique n'est pas adjacente au groupe amide,
un groupe alcénylène en C₂ à C₆, éventuellement substitué de une à trois fois par un groupe alkyle en C₁ à C₃, un groupe hydroxy et/ou le fluor ;
un groupe alcadiénylène en C₄ à C₆, éventuellement substitué par un groupe alkyle en C₁ à C₃ ou un ou deux atomes de fluor ; et
un groupe 1,3,5-hexatriénylène, éventuellement substitué par le fluor ;
D est choisi parmi
un groupe alkylène en C₂ à C₈, éventuellement substitué une fois ou deux fois par un groupe méthyle ou un groupe hydroxy ;
un groupe alcénylène en C₄ à C₈, éventuellement substitué une fois ou deux fois par un groupe méthyle ou un groupe hydroxy ;
un groupe alcynylène en C₄ à C₈, éventuellement substitué une fois ou deux fois par un groupe méthyle ou un groupe hydroxy ; et
le groupe constitué par un groupe alkylène en C₂ à C₈, un groupe alcénylène en C₄ à C₈ et un groupe alcynylène en C₄ à C₈, dans lequel une à trois unités méthylène sont remplacées de façon isostérique par O, S, NH, N(CH₃), N(COCH₃), N(SO₂CH₃), CO, SO ou SO₂,
E représente 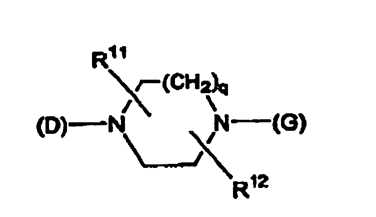 dans lequel
q est 1 ou 2 ;
R¹¹ est choisi parmi
l'hydrogène, un groupe alkyle en C₁ à C₃, un groupe hydroxyméthyle et un groupe carboxy, et
R¹² est choisi parmi
l'hydrogène et un groupe oxo adjacent à un atome d'azote
G est choisi parmi G1, G2, G3, G4 et G5, dans lesquels
G1 représente
-(CH₂)ᵣ-(CR¹⁴R¹⁵)ₛ-R¹³,
r est 0, 1 ou 2,
s est 0 ou 1 ;
R¹³ est choisi parmi
l'hydrogène, un groupe alkyle en C₁ à C₆, un groupe cycloalkyle en C₃ à C₈, un groupe benzyle, un groupe phényl,
le groupe constitué par un groupe benzocyclobutyle, un groupe indanyle, un groupe indényle, un groupe oxoindanyle, un groupe naphtyle, un groupe dihydronaphtyle, un groupe tétrahydronaphtyle, un groupe oxotétrahydronaphtyle, un groupe biphénylényle, un groupe fluorényle, un groupe oxofluorényle, un groupe anthryle, un groupe dihydroanthryle, un groupe oxodihydroanthryle, un groupe dioxodihydroanthryle, un groupe phénanthryle, un groupe dihydrophénanthryle, un groupe oxodihydrophénanthryle, un groupe dibenzocycloheptényle, un groupe oxodibenzocycloheptényle, un groupe dihydrodibenzocycloheptényle, un groupe oxodihydrodibenzocycloheptényle, un groupe dihydrodibenzocyclooctényle, un groupe tétrahydrodibenzocyclooctényle et un groupe oxotétrahydrodibenzocyclooctényle, liés directement ou sur un groupe méthylène ; et
le groupe constitué par un groupe furyle, un groupe thiényle, un groupe pyrrolyle, un groupe oxazolyle, un groupe isoxazolyle, un groupe thiazolyle, un groupe isothiazolyle, un groupe pyrazolyle, un groupe imidazolyle, un groupe oxadiazolyle, un groupe thiadiazolyle, un groupe triazolyle, un groupe pyridyle, un groupe pyrazinyle, un groupe pyridazinyle, un groupe pyrimidinyle, un groupe triazinyle, un groupe imidazothiazolyle, un groupe benzofuryle, un groupe dihydrobenzofuryle, un groupe benzothiényle, un groupe dihydrobenzothiényle, un groupe indolyle, un groupe indolinyle, un groupe isoindolinyle, un groupe oxoindolinyle, un groupe dioxoindolinyle, un groupe benzoxazolyle, un groupe oxobenzooxazolinyle, un groupe benzoisooxazolyle, un groupe oxobenzoisooxazolinyle, un groupe benzothiazolyle, un groupe oxobenzothiazolinyle, un groupe benzoisothiazolyle, un groupe oxobenzoisothiazolinyle, un groupe benzoimidazolyle, un groupe oxobenzoimidazolinyle, un groupe indazolyle, un groupe oxoindazolinyle, un groupe benzofurazanyle, un groupe benzothiadiazolyle, un groupe benzotriazolyle, un groupe oxazolopyridyle, un groupe oxodihydrooxazolopyridyle, un groupe thiazolopyridyle, un groupe oxodihydrothiazolopyridyle, un groupe isothiazolopyridyle, un groupe imidazopyridyle, un groupe oxodihydroimidazopyridyle, un groupe pyrazolopyridyle, un groupe oxodihydropyrazolopyridyle, un groupe thiénopyrimidinyle, un groupe chromanyle, un groupe chromanonyle, un groupe benzopyranyle, un groupe chromonyle, un groupe quinoloyle, un groupe isoquinoloyle, un groupe dihydroquinolyle, un groupe oxodihydroquinolinyle, un groupe tétrahydroquinolyle, un groupe oxotétrahydroquinolinyle, un groupe benzodioxanyle, un groupe quinoxalinyle, un groupe quinazolinyle, un groupe naphtypiridinyle, un groupe carbazolyle, un groupe tétrahydrocarbazolyle, un groupe oxotétrahydrocarbazolyle, un groupe pyridoindolyle, un groupe acridinyle, un groupe oxodihydroacridinyle, un groupe phénanthridinyle, un groupe dihydrophénanthridinyle, un groupe oxodihydrophénanthridinyle, un groupe dibenzoisoquinolinyle, un groupe dihydrodibenzoisoquinolinyle, un groupe oxodihydrodibenzoisoquinolinyle, un groupe phénothiazinyle, un groupe dihydrodibenzooxépinyle, un groupe oxodihydrodibenzooxépinyle, un groupe benzocycloheptathiényle, un groupe oxobenzocycloheptathiényle, un groupe dihydrothiénobenzothiépinyle, un groupe oxodihydrothiénobenzothiépinyle, un groupe dihydrodibenzothiépinyle, un groupe oxodihydrodibenzothiépinyle, un groupe octahydrodibenzothiépinyle, un groupe dibenzoazépinyle, un groupe dihydrodibenzoazépinyle, un groupe oxodihydrodibenzoazépinyle, un groupe octahydrodibenzoazépinyle, un groupe benzocycloheptapyridyle, un groupe oxobenzocycloheptapyridyle, un groupe pyridobenzoazépinyle, un groupe dihydropyridobenzoazépinyle, un groupe oxodihydropyridobenzoazépinyle, un groupe dihydropyridobenzodiazépinyle, un groupe dihydrodibenzooxazépinyle, un groupe dihydropyridobenzooxépinyle, un groupe dihydropyridobenzooxazépinyle, un groupe oxydihydropyridobenzooxazépinyle, un groupe dihydrodibenzothiazépinyle, un groupe oxodihydrodibenzothiazépinyle, un groupe dihydropyridobenzothiazépinyle et un groupe oxodihydropyridobenzothiazépinyle, liés directement ou sur un groupe méthylène ;
R¹⁴ a la même signification que R¹³ mais est choisi indépendamment de celui-ci ;
R¹⁵ est choisi parmi
un groupe hydroxy, un groupe méthyle, un groupe benzyle, un groupe phényle, un groupe indanyle, un groupe indényle, un groupe naphtyle, un groupe dihydronaphtyle, un groupe tétrahydronaphtyle, un groupe furyle, un groupe thiényle, un groupe pyrrolyle, un groupe oxazolyle, un groupe isoxazolyle, un groupe thiazolyle, un groupe isothiazolyle, un groupe pyrazolyle, un groupe imidazolyle, un groupe oxadiazolyle, un groupe thiadiazolyle, un groupe triazolyle, un groupe pyridyle, un groupe pyrazinyle, un groupe pyridazinyle, un groupe pyrimidinyle, un groupe triazinyle, un groupe benzofuryle, un groupe benzothiényle, un groupe indolyle, un groupe indolinyle, un groupe benzooxazolyle, un groupe benzothiazolyle, un groupe benzoimidazolyle, un groupe chromanyle, un groupe quinolyle et un groupe tétrahydroquinolyle, liés directement ou sur un groupe méthylène ;
G2 est choisi parmi 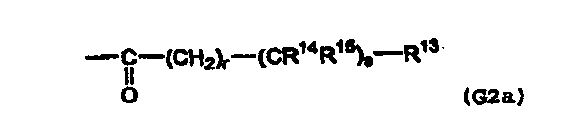 et 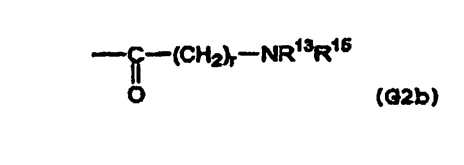 dans lesquelles r, s et les substituants R¹³ à R¹⁵ ont la signification ci-dessus, ou le groupe
-NR¹³R¹⁵
représente les groupes azétidine, pyrrolidine, pipéridine, (1H)-tétrahydropyridine, hexahydroazépine, (1H)-tétrahydroazépine, octahydroazocine, pyrazolidine, pipérazine, hexahydrodiazépine, morpholine, hexahydrooxazépine, thiomorpholine, thiomorpholin-1,1-dioxyde, 5-aza-bicyclo-[2.1.1]hexane, 2-aza-bicyclo[2.2.1]heptane, 7-aza-bicyclo-[2.2.1]heptane, 2,5-diaza-bicyclo[2.2.1]heptane, 2-aza-bicyclo[2.2.2]octane, 8-aza-bicyclo[3.2.1]octane, 2,5-diaza-bicyclo[2.2.2]octane, 9-aza-bicyclo[3.3.1]nonane, indoline, isoindoline, (1H)-dihydroquinoline, (1H)-tétrahydroquinoline, (2H)-tétrahydroisoquinoline, (1H)-tétrahydroquinoxaline, (4H)-dihydrobenzooxazine, (4H)-dihydrobenzothiazine, (1H)-tétrahydrobenzo[b]azépine, (1H)-tétrahydrobenzo[c]azépine, (1H)-tétrahydrobenzo[d]azépine, (5H)-tétrahydrobenzo[b]oxazépine, (5H)-tétrahydrobenzo[b]thiazépine, 1,2,3,4-tétrahydro-9H-pyrido[3,4-b]indole, (10H)-dihydroacridine, (10H)-dihydrophénanthridine, 1,2,3,4-tétrahydroacridanone, (10H)-phénoxazine, (10H)-phénothiazine, (5H)-dibenzoazépine, (5H)-dihydrodibenzoazépine, (5H)-octahydrodibenzoazépine, dihydrobenzo[d,e]isoquinoline, (5H)-dihydrodibenzodiazépine, (5H)-benzo[b]pyrido[f]azépine, (5H)-dihydrobenzo[b]pyrido[f]azépine, (11H)-dihydrodibenzo[b,e]oxazépine, (11H)-dihydrodibenzo[b,e]thiazépine, (10H)-dihydrodibenzo[b,f]oxazépine, (10H)-dihydrodibenzo[b,f]thiazépine, (5H)-tétrahydrodibenzoazocine, (11H)-dihydrobenzo[e]pyrido[b]-1,4-diazépin-6-one ou (11H)-dihydrobenzo[b]pyrido[e]-1,4-diazépin-5-one,
G3 représente
-SO₂-(CH₂)ᵣ-R¹³,
dans lequel r et R¹³ ont la signification ci-dessus,
G4 représente 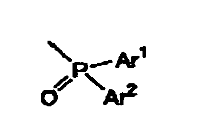 dans lequel
Ar¹ et Ar² sont choisis indépendamment l'un de l'autre parmi un groupe phényle, un groupe pyridyle et un groupe naphtyle ;
G5 représente
-COR¹⁶,
dans lequel
R¹⁶ est choisi parmi
un groupe trifluorométhyle, un groupe alcoxy en C₁ à C₆, un groupe alcényloxy en C₃ à C₆, et un groupe benzyloxy,
où les systèmes cycliques aromatiques dans les substituants sont éventuellement substitués indépendamment les uns des autres par un à trois des groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl (en C₁ à C₆)amino, et un groupe méthylène dioxyde dans le cas de deux résidus adjacents sur le noyau aromatique, et
où les résidus alkyle, alcényle et cycloalkyle dans les groupes G1, G2 et G3 sont éventuellement substitués par un ou deux des groupes identiques ou différents qui sont choisis parmi
un groupe hydroxy, un groupe carboxy, un groupe alcoxycarbonyle en C₂ à C₇, un groupe benzyloxycarbonyle, un groupe amino, un groupe monoalkyl(en C₁ à C₆)amino et un groupe dialkyl(en C₁ à C₆)amino.

18. Utilisation selon l'une quelconque des revendications 15 à 17, dans laquelle
R¹ est choisi parmi
l'hydrogène, le fluor, le chlore, le brome, un groupe méthyle, un groupe éthyle, un groupe trifluorométhyle, un groupe hydroxy, un groupe alcoxy en C₁ à C₄, un groupe méthylthio, un groupe éthylthio, un groupe carboxy et un groupe phénoxy ;
R² est choisi parmi l'hydrogène, le chlore et un groupe méthyle ;
R³ est l'hydrogène ;
R⁴ est choisi parmi
l'hydrogène, un groupe alkyle en C₁ à C₃ et un groupe hydroxy,
k est 0
A est choisi parmi
un groupe alkylène en C₂ à C₆, qui est éventuellement substitué une fois ou deux fois par un groupe hydroxy ou le fluor ;
un groupe alkylène en C₂ à C₆, dans lequel une unité méthylène est remplacée de façon isostérique par O, S ou CO, dans lequel, à l'exception de CO, la substitution isostérique n'est pas adjacente au groupe amide ;
un groupe alcénylène en C₂ à C₆ qui est éventuellement substitué par un groupe alkyle en C₁ à C₃ et/ou le fluor ; et
un groupe alcadiénylène en C₄ à C₆ ;
D est choisi parmi
un groupe alkylène en C₂ à C₈ qui est éventuellement substitué par un groupe méthyle ou un groupe hydroxy ;
un groupe alcénylène en C₄ à C₈, qui est éventuellement substitué par un groupe hydroxy ;
un groupe alcynylène en C₄ à C₈, qui est éventuellement substitué par un groupe hydroxy ; et
le groupe constitué par un groupe alkylène en C₂ à C₈, un groupe alcénylène en C₄ à C₈, et un groupe alcynylène en C₄ à C₈ dans lequel une unité méthylène est remplacée de façon isostérique par O, NH, N(CH₃), CO ou SO₂ ou un groupe éthylène est remplacé de façon isostérique par un groupe NH-CO et/ou CO-NH ou un groupe propylène est remplacé de façon isostérique par un groupe NH-CO-O et/ou O-CO-NH ;
E est choisi parmi une pipérazine et une hexahydro-1,4-diazépine, où le cycle est éventuellement substitué par un ou deux groupes méthylène et/ou par un groupe oxo adjacent à un atome d'azote ;
G est choisi parmi l'hydrogène, un groupe cycloalkyle en C₃ à C₈, un groupe méthoxycarbonyle, un groupe tert-butoxycarbonyle, un groupe benzyloxycarbonyle, un groupe trifluoroacétyle, un groupe diphénylphosphinoyle,
-(CH₂)ᵣ-(CR¹⁴R¹⁵)ₛ-R¹³, 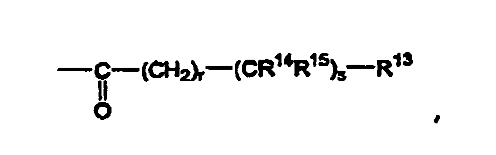 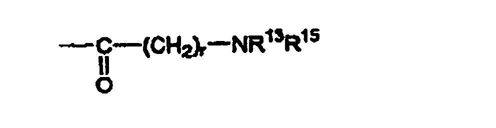
et
-SO₂-(CH₂)ᵣ-R¹³
où
r est 0 ou 1
s est 0 ou 1,
R¹³ est choisi parmi l'hydrogène, un groupe méthyle, un groupe benzyle, un groupe phényle,
le groupe constitué par un groupe indanyle, un groupe indényle, un groupe oxoindanyle, un groupe naphtyle, un groupe tétrahydronaphtyle, un groupe fluorényle, un groupe oxofluorényle, un groupe anthryle, un groupe dihydroanthryle, un groupe oxodihydroanthryle, un groupe dioxodihydroanthryle, un groupe phénanthryle, un groupe dihydrophénanthryle, un groupe oxydihydrophénanthryle, un groupe dibenzocycloheptényle, un groupe dihydrodibenzocycloheptényle, et un groupe oxodihydrodibenzocycloheptyle, liés directement ou sur un groupe méthylène, et
le groupe constitué par un groupe furyle, un groupe thiényle, un groupe oxazolyle, un groupe isooxazolyle, un groupe thiazolyle, un groupe imidazolyle, un groupe oxadiazolyle, un groupe pyridyle, un groupe pyrazinyle, un groupe pyrimidinyle, un groupe imidazothiazolyle, un groupe benzofuryle, un groupe benzothiényle, un groupe indolyle, un groupe indolinyle, un groupe oxoindolinyle, un groupe dioxoindolinyle, un groupe benzoxazolyle, un groupe oxobenzooxazolinyle, un groupe benzothiazolyle, un groupe oxobenzthiazolinyle, un groupe benzimidazolyle, un groupe oxobenzimidazolinyle, un groupe indazolyle, un groupe benzofurazanyle, un groupe benzotriazolyle, un groupe oxazolopyridyle, un groupe oxodihydrooxazolopyridyle, un groupe thiazolopyridyle, un groupe oxodihydrothiazolopyridyle, un groupe imidazopyridyle, un groupe oxodihydroimidazopyridyle, un groupe chromanyle, un groupe chromanonyle, un groupe benzopyranyle, un groupe chromonyle, un groupe quinolyle, un groupe isoquinolyle, un groupe oxodihydroquinolinyle, un groupe tétrahydroquinolyle, un groupe oxotétrahydroquinolinyle, un groupe benzodioxanyle, un groupe quinazolinyle, un groupe carbazolyle, un groupe acridinyle, un groupe dihydroacridinyle, un groupe oxodihydroacridinyle, un groupe dihydrophénanthridinyle, un groupe dihydrobenzoisoquinolinyle, un groupe phénothiazinyle, un groupe dihydrodibenzooxépinyle, un groupe benzocycloheptathiényle, un groupe dihydrothiénobenzothiépinyle, un groupe dihydrodibenzothiépinyle, un groupe oxodihydrodibenzothiépinyle, un groupe dihydrodibenzoazépinyle, un groupe oxodihydrodibenzoazépinyle, un groupe octahydrodibenzoazépinyle, un groupe benzocycloheptapyridyle, un groupe dihydropyridobenzodiazépinyle, et un groupe dihydrodibenzothiazépinyle, liés directement ou sur un groupe méthylène,
R¹⁴ est choisi parmi l'hydrogène, un groupe méthyle, un groupe benzyle, et un groupe phényle,
R¹⁵ est choisi parmi l'hydrogène, un groupe hydroxy, un groupe méthyle, un groupe benzyle, un groupe phényle ; et
le groupe constitué par un groupe naphtyle, un groupe tétrahydronaphtyle, un groupe furyle, un groupe thiényle, un groupe oxazolyle, un groupe thiazolyle, un groupe imidazolyle, un groupe pyridyle, un groupe benzofuryle, un groupe benzothiényle, un groupe indolyle, un groupe indolinyle, un groupe benzoxazolyle, un groupe benzothiazolyle, un groupe benzimidazolyle, un groupe chromanyle, un groupe quinolyle et un groupe tétrahydroquinolyle, liés directement ou sur un groupe méthylène ;
où le groupe
-NR¹³R¹⁵
représente un hétérocycle contenant de l'azote lié sur l'atome d'azote lequel hétérocycle contenant de l'azote est choisi parmi
un groupe pyrrolidine, un groupe pipéridine, un groupe hexahydroazépine, un groupe pipérazine, un groupe hexahydrodiazépine, un groupe morpholine, un groupe hexahydroazépine, un groupe thiomorpholine, un groupe 7-aza-bicyclo[2.2.1]heptane, un groupe 2,5-diaza-bicyclo[2.2.1]heptane, un groupe indoline, un groupe isoindoline, un groupe (1H)-dihydroquinoline, un groupe (1H)-tétrahydroquinoline, un groupe (2H)-tétrahydroisoquinoline, un groupe (4H)-dihydrobenzoxazine, un groupe (4H)-dihydrobenzothiazine, un groupe (1H)-tétrahydrobenzo[b]azépine, un groupe (1H)-tétrahydrobenzo[d]azépine, un groupe (5H)-tétrahydrobenzo[b]oxazépine, un groupe (5H)-tétrahydrobenzo[b]thiazépine, un groupe (10H)-dihydroacridine, un groupe 1,2,3,4-tétrahydroacridanone, un groupe (10H)-dihydrophénanthridine, un groupe (1H)-dihydrobenzo[d,e]isoquinoline, un groupe (10H)-phénothiazine, un groupe (5H)-dibenzo[b,f]azépine, un groupe (5H)-dihydrodibenzo[b,f]azépine, un groupe (5H)-dihydrodibenzo[c,e]azépine, un groupe (5H)-dihydrodibenzodiazépine, un groupe (11H)-dihydrodibenzo[b,e]oxazépine, un groupe (11H)-dihydrodibenzo[b,e]thiazépine, un groupe (5H)-dihydrobenzo[b]pyrido[3,2-f]azépine et un groupe (11H)-6-oxodihydrobenzo[e]pyrido[3,2-b][1,4]diazépine,
où les systèmes cycliques aromatiques dans les substituants sont éventuellement substitués, indépendamment les uns des autres, par un à trois des groupes identiques ou différents choisis parmi un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor, un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl(en C₁ à C₆)amino, et dans le cas de deux résidus adjacents sur le noyau aromatique, un groupe méthylènedioxy, et
où les résidus alkyle, alcényle et cycloalkyle dans le groupe G sont éventuellement substitué par un ou deux des groupes identiques ou différents qui sont choisis parmi un groupe hydroxy, un groupe carboxy, un groupe alcoxycarbonyle en C₂-C₇, un groupe benzyloxycarbonyle, un groupe amino, un groupe monoalkyl(en C₁ à C₆)amino et un groupe dialkyl(en C₁ à C₆)amino.

19. Utilisation selon l'une quelconque des revendications 15 à 18, dans laquelle
R¹ est choisi parmi
l'hydrogène, le fluor, un groupe méthyle, un groupe trifluorométhyle et un groupe éthylthio ;
R², R³ et R⁴ sont chacun un hydrogène ;
k est 0,
A est choisi parmi
le groupe constitué par l'éthylène, le propylène et le butylène, qui sont chacun éventuellement substitués par un groupe hydroxy ou un ou deux atome de fluor ;
OCH₂, SCH₂ ;
un groupe éthénylène, et un groupe 1,3-butadiénylène ;
D est choisi parmi
un groupe alkylène en C₂ à C₆ qui est éventuellement substitué par un groupe hydroxy ;
un groupe alcénylène en C₄ à C₆ ;
un groupe alcynylène en C₄ à C₆ ; et
le groupe constitué par un groupe alkylène en C₂ à C₆, un groupe alcénylène en C₄ à C₆ et un groupe alcynylène en C₄ à C₆, dans lequel une ou deux unités méthylène sont remplacée de façon isostérique par O, NH, CO ou SO₂ ;
E est une pipérazine ou une hexahydro-1,4-diazépine ;
G est choisi parmi
un groupe phényle, un groupe benzyle, un groupe phénéthyle, un groupe diphénylméthyle, un groupe naphtyle, un groupe tétrahydronaphtyle, un groupe naphtylméthyle, un groupe fluorényle, un groupe fluorénylméthyle, un groupe anthrylméthyle, un groupe dihydrodibenzocycloheptényle, un groupe furylméthyle, un groupe thiénylméthyle, un groupe thiazolylméthyle, un groupe pyridylméthyle, un groupe benzothiénylméthyle, un groupe quinolylméthyle, un groupe phénylthiénylméthyle, un groupe phénylpyridylméthyle, un groupe benzocycloheptapyridinyle, un groupe dihydrobenzocycloheptapyridinyle, un groupe dihydrodibenzooxépinyle, un groupe dihydrodibenzothiépinyle, un groupe dihydrodibenzoazépinyle, un groupe dihydrobenzopyridodiazépinyle, un groupe formyle, un groupe acétyle, un groupe pivaloyle, un groupe phénylacétyle, un groupe diphénylacétyle, un groupe diphénylpropionyle, un groupe naphtylacétyle, un groupe benzoyle, un groupe naphtoyle, un groupe oxofluorénylcarbonyle, un groupe oxodihydroanthrylcarbonyle, un groupe dioxodihydroanthrylcarbonyle, un groupe furoyle, un groupe pyridylacétyle, un groupe pyridylcarbonyle, un groupe chromonylcarbonyle, un groupe quinolylcarbonyle, un groupe phénylylaminocarbonyle, un groupe naphtylaminocarbonyle, un groupe tétrahydronaphtylaminocarbonyle, un groupe dibenzylaminocarbonyle, un groupe benzylphénylaminocarbonyle, un groupe diphénylaminocarbonyle, un groupe indolinyl-N-carbonyle, un groupe isoindolinyl-N-carbonyle, un groupe tétrahydroquinolinyl-N-carbonyle, un groupe carbazolyl-N-carbonyle, un groupe tétrahydrobenzoazépinyl-N-carbonyle, un groupe dihydrodibenzoazépinyl-N-carbonyle, un groupe dihydrobenzopyridoazépinyl-N-carbonyle, un groupe oxodihydrobenzopyridoazépinyl-N-carbonyle, un groupe méthanesulfonyle, un groupe toluènesulfonyle, un groupe naphtylsulfonyle, un groupe quinolinesulfonyle et un groupe diphénylphosphinoyle,
où les systèmes cycliques aromatiques sont éventuellement substitués indépendamment les uns des autres par un à trois des groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl(en C₁ à C₆)amino, et dans le cas de deux résidus adjacents sur le noyau aromatique, un groupe méthylènedioxy, et
où les résidus alkyle, alcényle et cycloalkyle dans le groupe G sont éventuellement substitués par un ou deux des groupes identiques ou différents qui sont choisis parmi
un groupe hydroxy, un groupe carboxy, un groupe alcoxycarbonyle en C₂ à C₇, un groupe benzyloxycarbonyle, un groupe amino, un groupe monoalkyl(en C₁ à C₆)amino et un groupe dialkyl(en C₁ à C₆)amino.

20. Utilisation selon l'une quelconque des revendications 15 à 19, dans laquelle le(s) composé(s) de formule (I) est/sont choisi(s) dans le groupe constitué par :
N-[4-(4-diphénylméthylpipérazin-1-yl)-3-hydroxybutyl]-3-pyridin-3-yl-acrylamide ;
N-[3-(4-diphénylméthylpipérazin-1-yl)-propoxy]-3-pyridin-3-yl-acrylamide ;
N-[4-(4-diphénylméthylpipérazin-1-yl)-4-oxo-butyl]-3-pyridin-3-yl-acrylamide ;
N-[3-(4-diphénylméthylpipérazin-1-sulfonyl)-propyl]-3-pyridin-3-yl-acrylamide ;
N-{2-[2-(4-diphénylméthylpipérazin-1-yl)-éthoxy]-éthyl}-3-pyridin-3-yl-acrylamide ;
N-(4-[bis-(4-fluorophényl)-méthyl]-pipérazin-1-1-yl}-but-2-in-yl)-3-yl-acrylamide ;
N-{4-[4-(4-carboxyphényl-phénylméthyl)-pipérazin-1-yl]-butyl}-3-pyridin-3-yl-acrylamide ;
N-[4-{4-[(4-aminophényl)-phénylméthyl]-pipérazin-1-yl}butyl)-3-pyridin-3-yl-acrylamide ;
N-{4-[4-(9H-fluorèn-9-yl)-pipérazin-1-yl]-butyl}-2-(pyridin-3-yloxy)-acétamide ;
N-{5-[4-(9H-fluorèn-9-yl)-pipérazin-1-yl]-pentyl}-3-pyridin-3-yl-acrylamide ;
N-{6-[4-(9H-fluorèn-9-yl)-pipérazin-1-yl]-hexyl}-3-pyridin-3-yl-acrylamide ;
3-pyridin-3-yl-N-{4-[4-(1,2,3,4-tétrahydronaphtalèn-1-yl)-pipérazin-1-yl]-butyl}-acrylamide ;
3-pyridin-3-yl-N-{4-[4-(5,6,7,8-tétrahydronaphtalèn-1-yl)-pipérazin-1-yl]-butyl}-acrylamide ;
N-{4-[4-(naphtalèn-1-yl)-pipérazin-1-yl]-butyl}-3-pyridin-3-yl-acrylamide ;
N-[4-(4-biphényl-2-yl-pipérazin-1-yl)-butyl]-3-pyridin-3-yl-propionamide ;
N-[5-(4-biphényl-2-yl-pipérazin-1-yl)-pentyl]-3-pyridin-3-yl-acrylamide ;
N-[6-(4-biphényl-2-yl-pipérazin-1-yl)-hexyl]-3-pyridin-3-yl-acrylamide ;
N-[4-(4-biphényl-2-yl-pipérazin-1-yl)-butyl]-2-(pyridin-3-yloxy)-acétamide ;
amide de l'acide N-[4-(4-biphényl-2-yl-pipérazin-1-yl)-butyl]-5-(pyridin-3-yl)-penta-2,4-diénoïque ;
N-{4-[4-(10,11-dihydro-5H-dibenzo[a,d]cycloheptèn-5-yl)-pipérazin-1-yl]-butyl}-3-pyridin-3-yl-propionamide ;
N-{5-[4-(10,11-dihydro-5H-dibenzo[a,d]cycloheptèn-5-yl)-pipérazin-1-yl]-pentyl}-3-pyridin-3-yl-acrylamide ;
N-{6-[4-(10,11-dihydro-5H-dibenzo[a,d]cycloheptèn-5-yl)-pipérazin-1-yl]-hexyl}-3-pyridin-3-yl-acrylamide ;
amide de l'acide N-{4-[4-(10,11-dihydro-5H-dibenzo[a,d]cycloheptèn-5-yl)-pipérazin-1-yl]-butyl}-5-(pyridin-3-yl)-penta-2,4-diénoïque ;
N-{4-[4-(6,11-dihydro-dibenzo[b,e]oxépin-11-yl)-pipérazin-1-yl]-butyl}-3-pyridin-3-yl-propionamide ;
N-{2-[4-(6,11-dihydrodibenzo[b,e]thiépin-11-yl)-pipérazin-1-yl]-éthyl}-3-pyridin-3-yl-acrylamide ;
N-[4-(4-diphénylacétyl-pipérazin-1-yl)-butyl]-3-pyridin-3-yl-acrylamide ;
N-[4-(4-benzoylpypérazin-1-yl)-butyl]-3-pyridin-3-yl-acrylamide ;
N-{4-[4-(2-aminobenzoyl)-pipérazin-1-yl]-butyl}-3-pyridin-3-yl-acrylamide ;
N-{4-[4-(4-carboxybenzoyl)-pipérazin-1-yl]-butyl}-3-pyridin-3-yl-acrylamide ;
N-{4-[4-(biphényl-2-carbonyl)-pipérazin-1-yl]-butyl}-3-pyridin-3-yl-acrylamide ;
N-{4-[4-(9-oxo-9H-fluorèn-4-carbonyl)-pipérazin-1-yl]-butyl}-3-pyridin-3-yl-acrylamide ;
N-{4-[4-(furan-2-carbonyl)-pipérazin-1-yl]-butyl}-3-pyridin-3-yl-acrylamide ;
N-{4-[4-(naphtalèn-1-yl-aminocarbonyl)-pipérazin-1-yl]-butyl}-3-pyridin-3-yl-propionamide ;
N-{4-[4-(diphénylaminocarbonyl)-pipérazin-1-yl]-butyl}-3-pyridin-3-yl-acrylamide ;
N-{4-[4-(naphtalèn-2-sulfonyl)-pipérazin-1-yl]-butyl}-3-pyridin-3-yl-acrylamide ;
N-[4-(4-diphénylphosphinoyl-pipérazin-1-yl)-butyl]-3-pyridin-3-yl-acrylamide ;
N-[4-(4-biphényl-2-yl-pipérazin-1-yl)-butyl}-3-pyridin-3-yl-acrylamide ;
N-{4-[4-(9H-fluorèn-9-yl)-pipérazin-1-yl]-butyl}-3-pyridin-3-yl-acrylamide ; et
N-{4-[4-(10,11-dihydro-5H-dibenzo[a,d]cyclohepten-5-yl)-pipérazin-1-yl]-butyl}-3-pyridin-3-yl-acrylamide.

21. Utilisation d'un ou plusieurs composés selon la formule (I) : 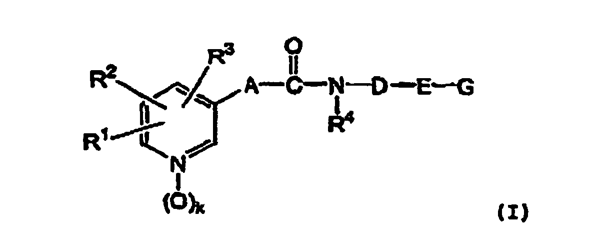 pour la fabrication d'une composition pharmaceutique pour la suppression de la réaction de rejet après une transplantation d'organe dans le corps humain ou animal, éventuellement en combinaison avec un autre agent cytostatique ou un agent immunosuppresseur et/ou d'autres compositions pharmaceutiques appropriées pour ces indications, dans laquelle :
R¹ est choisi parmi
l'hydrogène, un halogène, un groupe cyano, un groupe alkyle en C₁ à C₆, un groupe alcényle en C₃ à C₆, un groupe alcynyle en C₂ à C₆, un groupe trifluorométhyle, un groupe cycloalkyle en C₃ à C₈, un groupe hydroxyalkyle en C₁ à C₆, un groupe hydroxy, un groupe alcoxy en C₁ à C₆, un groupe alcényloxy en C₃ à C₆, un groupe alcynyloxy en C₃ à C₆, un groupe benzyloxy, un groupe alcanoyloxy en C₁ à C₇, un groupe alcoxycarbonyloxy en C₂ à C₇, un groupe alkylthio en C₁ à C₆, un groupe alcénylthio en C₃ à C₆, un groupe alcynylthio en C₃ à C₆, un groupe cycloalkyloxy en C₃ à C₈, un groupe cycloalkylthio en C₃ à C₈, un groupe alcoxycarbonyle en C₂ à C₇, un groupe aminocarbonyle, un groupe alkylaminocarbonyle en C₂ à C₇, un groupe dialkylaminocarbonyle en C₃ à C₁₃, un groupe carboxy, un groupe phényle, un groupe phénoxy, un groupe phénylthio, un groupe pyridyloxy, un groupe pyridylthio, et un groupe NR⁵R⁶, dans lequel
R⁵ et R⁶ sont choisis indépendamment l'un de l'autre parmi
l'hydrogène, un groupe alkyle en C₁ à C₆, un groupe alcényle en C₃ à C₆, un groupe alcynyle en C₃ à C₆, un groupe benzyle et un groupe phényle ;
R² est choisi parmi
l'hydrogène, un halogène, un groupe cyano, un groupe alkyle en C₁ à C₆, un groupe trifluorométhyle, un groupe hydroxy, un groupe alcoxy en C₁ à C₆, un groupe benzyloxy et un groupe alcanoyloxy en C₁ à C₇
ou
R¹ et R², s'ils sont adjacents, forment de façon facultative un pont choisi parmi -(CH₂)₄-, -(CH=CH)₂- et -CH₂O-CR⁷R⁸-O-, dans lequel
R⁷ et R⁸ sont choisis indépendamment l'un de l'autre parmi l'hydrogène et un groupe alkyle en C₁ à C₆ ;
R³ est choisi parmi
l'hydrogène, un halogène, un groupe alkyle en C₁ à C₆, un groupe trifluorométhyle et un groupe hydroxyalkyle en C₁ à C₆ ;
R⁴ est choisi parmi
l'hydrogène, un groupe alkyle en C₁ à C₆, un groupe alcényle en C₃ à C₆, un groupe alcynyle en C₃ à C₆, un groupe cycloalkyle en C₃ à C₆, un groupe hydroxy, un groupe alcoxy en C₁ à C₆ et un groupe benzyloxy ;
k est 0 ou 1,
A est choisi parmi
un groupe alkylène en C₁ à C₆, éventuellement substitué de une à trois fois par un groupe alkyle en C₁ à C₃, un groupe hydroxy, un groupe alcoxy en C₁ à C₃, le fluor, ou un groupe phényle,
un groupe alkylène en C₂ à C₆, dans lequel une unité méthylène est remplacée de façon isostérique par O, S, NR⁹, CO, SO ou SO₂, dans lequel, à l'exception de CO, la substitution isostérique n'est pas adjacente au groupe amide et R⁹ est choisi parmi l'hydrogène, un groupe alkyle en C₁ à C₆, un groupe alcényle en C₃ à C₆, un groupe alcynyle en C₃ à C₆, un groupe acyle en C₁ à C₆ et un groupe alcanesulfonyle en C₁ à C₆,
un groupe 1,2-cyclopropylène,
un groupe alcénylène en C₂ à C₆, éventuellement substitué de une à trois fois par un groupe alkyle en C₁ à C₃, un groupe hydroxy, un groupe alcoxy en C₁ à C₃, le fluor, un groupe cyano ou un groupe phényle,
un groupe alcadiénylène en C₄ à C₆, éventuellement substitué une fois ou deux fois par un groupe alkyle en C₁ à C₃, le fluor, un groupe cyano ou un groupe phényle ;
un groupe 1,3,5-hexatriénylène, éventuellement substitué par un groupe alkyle en C₁ à C₃, le fluor, un groupe cyano ou un groupe phényle, et
un groupe éthynylène
D est choisi parmi
un groupe alkylène en C₂ à C₁₀, éventuellement substitué une fois ou deux fois par un groupe alkyle en C₁ à C₆, un groupe hydroxy, ou un groupe alcoxy en C₁ à C₆ ;
un groupe alcénylène en C₄ à C₁₀, éventuellement substitué une fois ou deux fois par un groupe alkyle en C₁ à C₆, un groupe hydroxy, ou un groupe alcoxy en C₁ à C₆ ;
un groupe alcynylène en C₄ à C₁₀, éventuellement substitué une fois ou deux fois par un groupe alkyle en C₁ à C₆, un groupe hydroxy, ou un groupe alcoxy en C₁ à C₆ ; et
le groupe constitué par un groupe alkylène en C₂ à C₁₀, un groupe alcénylène en C₄ à C₁₀ et un groupe alcynylène en C₄ à C₁₀, dans lequel une à trois unités méthylène sont remplacées de façon isostérique par O, S, NR¹⁰, CO, SO ou SO₂, dans lequel
R¹⁰ a la même signification que R⁹, mais est choisi indépendamment de celui-ci;
E représente 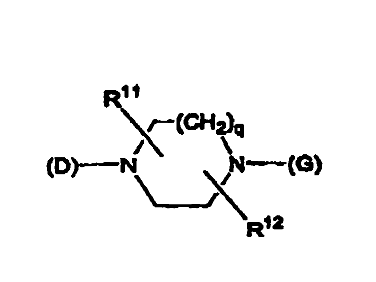 dans lequel
q est 1, 2 ou 3 ;
R¹¹ est choisi parmi
l'hydrogène, un groupe alkyle en C₁ à C₆, un groupe hydroxy, un groupe hydroxyméthyle, un groupe carboxy, et un groupe alcoxycarbonyle en C₂ à C₇, et
R¹² est choisi parmi
l'hydrogène, un groupe alkyle en C₁ à C₆ et un groupe oxo adjacent à un atome d'azote,
ou R¹¹ et R¹² forment ensemble un pont alkylène en C₁ à C₃ lors d'une formation d'un système à noyau bicyclique ;
G est choisi parmi G1, G2, G3, G4 et G5, dans lesquels
G1 représente
-(CH₂)ᵣ-(CR¹⁴R¹⁵)ₛ-R¹³,
r est 0, 1, 2 ou 3,
s est 0 ou 1
R¹³ est choisi parmi
l'hydrogène, un groupe alkyle en C₁ à C₆, un groupe alcényle en C₃ à C₆, un groupe alcynyle en C₃ à C₆, un groupe cycloalkyle en C₃ à C₈ ;
le groupe constitué des hétérocycles de quatre à huit membres, saturés et insaturés, qui contiennent éventuellement un ou deux hétéroatomes choisis parmi N, S et O ;
un groupe benzyle, un groupe phényle ;
le groupe constitué des hétérocycles de cinq à six membres aromatiques monocycliques, qui contiennent éventuellement un à trois hétéroatomes choisis parmi N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
R¹⁴ a la même signification que R¹³, mais est choisi indépendamment de celui-ci ;
R¹⁵ est choisi parmi
l'hydrogène, un groupe hydroxy, un groupe méthyle, un groupe benzyle, un groupe phényle,
le groupe constitué des hétérocycles de cinq et six membres aromatiques monocycliques, qui contiennent un à trois hétéroatomes du groupe N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
G2 est choisi parmi 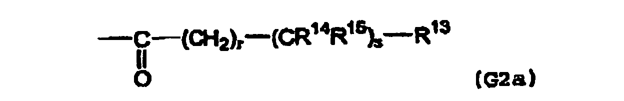 et 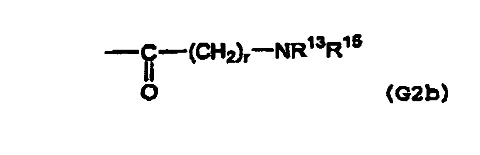 dans lesquels r, s et les substituants R¹³ à R¹⁵ ont la signification ci-dessus, ou le groupe
-NR¹³R¹⁵
représente un hétérocycle contenant de l'azote relié sur l'atome d'azote lequel hétérocycle contenant de l'azote est choisi parmi
le groupe constitué des hétérocycles de quatre à huit membres, monocycliques, saturés et insaturés, qui, en plus de l'atome d'azote essentiel, contiennent éventuellement un ou deux autres hétéroatomes choisis parmi N, S et O, et
le groupe constitué des hétérocycles bi- ou tricyclique condensés ou pontés, saturés et insaturés, avec 8 à 16 atomes de cycle, qui en plus de l'atome d'azote essentiel contiennent éventuellement un ou deux autres hétéroatomes choisis parmi N, S et O ;
G3 représente
-SO₂-(CH₂)ᵣ-R¹³,
dans lequel r et R¹³ ont les significations ci-dessus,
G4 représente 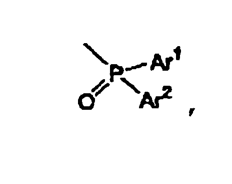 dans lequel
Ar¹ et Ar² sont choisis indépendamment l'un de l'autre parmi un groupe phényle, un groupe pyridyle et un groupe naphtyle ;
G5 représente
-COR¹⁶
dans lequel
R¹⁶ est choisi parmi
un groupe trifluorométhyle, un groupe alcoxy en C₁ à C₆, un groupe alcényloxy en C₃ à C₆, et un groupe benzyloxy,
où les systèmes cycliques aromatiques dans les substituants R¹, R², R⁴, R⁵, R⁶, R¹³, R¹⁴, R¹⁶, Ar¹ et Ar² et/ou dans le système cyclique -NR¹³R¹⁵ sont éventuellement substitués indépendamment les uns des autres par un à trois groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl (en C₁ à C₆)amino, et un groupe méthylène dioxyde pour deux résidus adjacents sur le noyau aromatique, et où
les résidus alkyle, alcényle et cycloalkyle dans les groupes G1, G2 et G3 sont éventuellement substitués par un ou deux des groupes identiques ou différents qui sont choisis parmi un groupe hydroxy, un groupe carboxy, un groupe alcoxycarbonyle en C₂ à C₇, un groupe benzyloxycarbonyle, un groupe amino, un groupe monoalkyl(en C₁ à C₆)amino et un groupe dialkyl (en C₁ à C₆)amino ;
leurs isomères cis et trans, leurs isomères E et Z, leurs énantiomères, leurs diastéréoisomères et d'autres isomères ainsi que leurs mélanges racémiques ou non racémiques et les isomères endo et exo correspondants dans le cas où le système cyclique E est bicyclique ;
leurs tautomères ; ainsi que
leurs sels d'addition d'acide comprenant leurs hydrates et produits de solvatation.

22. Utilisation selon la revendication 21, dans laquelle
dans laquelle
R¹ est choisi parmi
l'hydrogène, un halogène, un groupe cyano, un groupe alkyle en C₁ à C₆, un groupe trifluorométhyle, un groupe cycloalkyle en C₃ à C₈, un groupe hydroxyalkyle en C₁ à C₆, un groupe hydroxy, un groupe alcoxy en C₁ à C₄, un groupe benzyloxy, un groupe alkylthio en C₁ à C₄, un groupe alcanoyloxy en C₁ à C₅, un groupe alcoxycarbonyle en C₂ à C₅, un groupe aminocarbonyle, un groupe alkylaminocarbonyle en C₂ à C₅, un groupe dialkylaminocarbonyle en C₃ à C₉, un groupe carboxy, un groupe phényle, un groupe phénoxy, un groupe phénylthio, un groupe pyridyloxy, et un groupe NR⁵R⁶, dans lequel
R⁵ et R⁶ sont choisis indépendamment l'un de l'autre parmi l'hydrogène et un groupe alkyle en C₁ à C₆ ;
R² est choisi parmi
l'hydrogène, un halogène, un groupe cyano, un groupe alkyle en C₁ à C₆, un groupe trifluorométhyle, un groupe hydroxy, et un groupe alcoxy en C₁ à C₄ ;
R³ est choisi parmi
l'hydrogène, un halogène et un groupe alkyle en C₁ à C₆ ;
R⁴ est choisi parmi
l'hydrogène, un groupe alkyle en C₁ à C₆, un groupe alcényle en C₃ à C₆, un groupe cycloalkyle en C₃ à C₆, un groupe hydroxy, un groupe alcoxy en C₁ à C₆ et un groupe benzyloxy ;
k est ou 1,
A est choisi parmi
un groupe alkylène en C₁ à C₆, éventuellement substitué une fois à trois fois par un groupe alkyle en C₁ à C₃, un groupe hydroxy, le fluor, ou un groupe phényle,
un groupe alkylène en C₂ à C₆, dans lequel une unité méthylène est remplacée de façon isostérique par O, S, NR⁹, CO, SO ou SO₂, dans lequel, à l'exception de CO, la substitution isostérique n'est pas adjacente au groupe amide et le résidu R⁹ est choisi parmi l'hydrogène, un groupe alkyle en C₁ à C₆, un groupe acyle en C₁ à C₆ et un groupe méthanesulfonyle ;
un groupe 1,2-cyclopropylène,
un groupe alcénylène en C₂ à C₆, éventuellement substitué de une à trois fois par un groupe alkyle en C₁ à C₃, un groupe hydroxy, le fluor, un groupe cyano ou un groupe phényle,
un groupe alcadiénylène en C₄ à C₆, éventuellement substitué une fois ou deux fois par un groupe alkyle en C₁ à C₃, le fluor, un groupe cyano ou un groupe phényle ;
un groupe 1,3,5-hexatriénylène, éventuellement substitué par un groupe alkyle en C₁ à C₃, le fluor ou un groupe cyano, et
un groupe éthynylène
D est choisi parmi
un groupe alkylène en C₂ à C₁₀, éventuellement substitué une fois ou deux fois par un groupe alkyle en C₁ à C₃ ou un groupe hydroxy ;
un groupe alcénylène en C₄ à C₁₀, éventuellement substitué une fois ou deux fois par un groupe alkyle en C₁ à C₃ ou un groupe hydroxy ;
un groupe alcynylène en C₄ à C₁₀, éventuellement substitué une fois ou deux fois par un groupe alkyle en C₁ à C₃ ou un groupe hydroxy ; et
le groupe constitué par un groupe alkylène en C₂ à C₁₀, un groupe alcénylène en C₄ à C₁₀ et un groupe alcynylène en C₄ à C₁₀, dans lequel une à trois unités méthylène sont remplacées de façon isostérique par O, S, NR¹⁰, CO, SO ou SO₂, dans lequel
R¹⁰ a la même signification que R⁹, mais est choisi indépendamment de celui-ci;
E représente 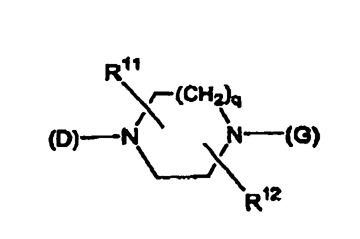 dans lequel
q est 1, 2 ou 3 ;
R¹¹ est choisi parmi
l'hydrogène, un groupe alkyle en C₁ à C₃, un groupe hydroxy, un groupe hydroxyméthyle, un groupe carboxy, et un groupe alcoxycarbonyle en C₂ à C₇, et
R¹² est choisi parmi l'hydrogène et un groupe oxo adjacent à un atome d'azote ou
R¹¹ et R¹², ensemble, forment un pont alkylène en C₁ à C₃ lors d'une formation d'un système à noyau bicyclique ;
G est choisi parmi G1, G2, G3, G4 et G5, dans lesquels
G1 représente
-(CH₂)ᵣ-(CR¹⁴R¹⁵)ₛ-R¹³,
r est 0, 1 ou 2,
s est 0 ou 1
R¹³ est choisi parmi
l'hydrogène, un groupe alkyle en C₁ à C₆, un groupe alcényle en C₃ à C₆, un groupe alcynyle en C₃ à C₆, un groupe cycloalkyle en C₃ à C₈ ; un groupe benzyle, un groupe phényle ;
le groupe constitué des hétérocycles de cinq à six membres aromatiques monocycliques, qui contiennent un à trois hétéroatomes choisis parmi N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
R¹⁴ a la même signification que R¹³, mais est choisi indépendamment de celui-ci ;
R¹⁵ est choisi parmi
l'hydrogène, un groupe hydroxy, un groupe méthyle, un groupe benzyle, un groupe phényle,
le groupe constitué des hétérocycles de cinq et six membres aromatiques monocycliques, qui contiennent un à trois hétéroatomes du groupe N, S et O et qui sont soit liés directement soit sur un groupe méthylène,
le groupe constitué des systèmes cycliques carbocycliques partiellement hydrogénés ou aromatiques bi- et tricycliques condensés avec 8 à 16 atomes de cycle, et au moins un cycle aromatique, où la liaison se produit soit sur un cycle aromatique soit sur un cycle hydrogéné et soit directement soit sur un groupe méthylène;
le groupe constitué des systèmes cycliques hétérocycliques partiellement hydrogénés ou aromatiques bi- et tricycliques condensés avec 8 à 16 atomes de cycle, et au moins un cycle aromatique, où un à trois atomes de cycle sont choisis parmi N, S et O et la liaison se produit soit sur un cycle aromatique soit sur un cycle hydrogéné et soit directement soit sur un groupe méthylène;
G2 est choisi parmi 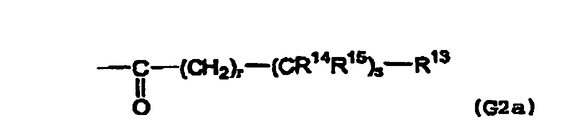 et 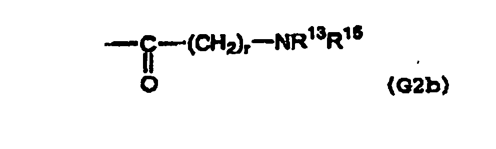 dans lesquels r, s et les substituants R¹³ à R¹⁵ ont la signification ci-dessus, ou le groupe
-NR¹³R¹⁵
représente un hétérocycle contenant de l'azote relié sur l'atome d'azote lequel hétérocycle contenant de l'azote est choisi parmi
le groupe constitué des hétérocycles de quatre à huit membres, monocycliques, saturés et insaturés, qui, en plus de l'atome d'azote essentiel, contiennent éventuellement un ou deux autres hétéroatomes choisis parmi N, S et O, et
le groupe constitué des hétérocycles bi- ou tricyclique condensés ou pontés, saturés et insaturés, avec 8 à 16 atomes de cycle, qui en plus de l'atome d'azote essentiel contiennent éventuellement un ou deux autres hétéroatomes choisis parmi N, S et O ;
G3 représente
-SO₂-(CH₂)ᵣ-R¹³,
dans lequel r et R¹³ ont les significations ci-dessus,
G4 représente 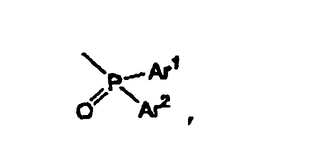 dans lequel
Ar¹ et Ar² sont choisis indépendamment l'un de l'autre parmi un groupe phényle, un groupe pyridyle et un groupe naphtyle ;
G5 représente
-COR¹⁶
dans lequel
R¹⁶ est choisi parmi
un groupe trifluorométhyle, un groupe alcoxy en C₁ à C₆, un groupe alcényloxy en C₃ à C₆, et un groupe benzyloxy,
où les systèmes cycliques aromatiques dans les substituants R¹, R², R⁴, R⁵, R⁶, R¹³, R¹⁴, R¹⁶, Ar¹ et Ar² et/ou dans le système cyclique -NR¹³R¹⁵ sont éventuellement substitués indépendamment les uns des autres par un à trois groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl (en C₁ à C₆) amino, et un groupe méthylène dioxyde dans le cas de deux résidus adjacents sur le noyau aromatique, et où
les résidus alkyle, alcényle et cycloalkyle dans les groupes G1, G2 et G3 sont éventuellement substitués par un ou deux des groupes identiques ou différents qui sont choisis parmi
un groupe hydroxy, un groupe carboxy, un groupe alcoxycarbonyle en C₂ à C₇, un groupe benzyloxycarbonyle, un groupe amino, un groupe monoalkyl(en C₁ à C₆)amino et un groupe dialkyl(en C₁ à C₆)amino.

23. Utilisation selon la revendication 21 ou 22, dans laquelle
R¹ est choisi parmi
l'hydrogène, un halogène, un groupe cyano, un groupe méthyle, un groupe éthyle, un groupe trifluorométhyle, un groupe hydroxy, un groupe alcoxy en C₁ à C₄, un groupe benzyloxy, un groupe alcanoyloxy en C₁ à C₅, un groupe méthylthio, un groupe éthylthio, un groupe méthoxycarbonyle, un groupe tert-butoxycarbonyle, un groupe aminocarbonyle, un groupe carboxy, un groupe phénoxy, et un groupe phénylthio,
R² est choisi parmi
l'hydrogène, un halogène, un groupe trifluorométhyle, et un groupe hydroxy,
R³ est choisi parmi
l'hydrogène et un halogène,
R⁴ est choisi parmi
l'hydrogène, un groupe alkyle en C₁ à C₃, un groupe allyle, un groupe hydroxy, et un groupe alcoxy en C₁ à C₃ ;
k est 0 ou 1,
A est choisi parmi
un groupe alkylène en C₁ à C₆, éventuellement substitué une fois ou deux fois par un groupe alkyle en C₁ à C₃, un groupe hydroxy ou le fluor ;
un groupe alkylène en C₂ à C₆, dans lequel une unité méthylène est remplacée de façon isostérique par O, S, NR⁹, CO, SO ou SO₂, dans lequel, à l'exception de CO, la substitution isostérique n'est pas adjacente au groupe amide,
un groupe alcénylène en C₂ à C₆, éventuellement substitué de une à trois fois par un groupe alkyle en C₁ à C₃, un groupe hydroxy et/ou le fluor ;
un groupe alcadiénylène en C₄ à C₆, éventuellement substitué par un groupe alkyle en C₁ à C₃ ou un ou deux atomes de fluor ; et
un groupe 1,3,5-hexatriénylène, éventuellement substitué par le fluor ;
D est choisi parmi
un groupe alkylène en C₂ à C₈, éventuellement substitué une fois ou deux fois par un groupe méthyle ou un groupe hydroxy ;
un groupe alcénylène en C₄ à C₈, éventuellement substitué une fois ou deux fois par un groupe méthyle ou un groupe hydroxy ;
un groupe alcynylène en C₄ à C₈, éventuellement substitué une fois ou deux fois par un groupe méthyle ou un groupe hydroxy ; et
le groupe constitué par un groupe alkylène en C₂ à C₈, un groupe alcénylène en C₄ à C₈ et un groupe alcynylène en C₄ à C₈, dans lequel une à trois unités méthylène sont remplacées de façon isostérique par O, S, NH, N(CH₃), N(COCH₃), N(SO₂CH₃), CO, SO ou SO₂,
E représente 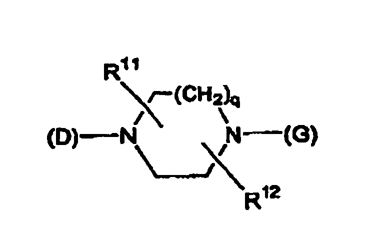 dans lequel
q est 1 ou 2 ;
R¹¹ est choisi parmi
l'hydrogène, un groupe alkyle en C₁ à C₃, un groupe hydroxyméthyle et un groupe carboxy, et
R¹² est choisi parmi
l'hydrogène et un groupe oxo adjacent à un atome d'azote
G est choisi parmi G1, G2, G3, G4 et G5, dans lesquels
G1 représente
-(CH₂)ᵣ-(CR¹⁴R¹⁵)ₛ-R¹³,
r est 0, 1 ou 2,
s est 0 ou 1 ;
R¹³ est choisi parmi
l'hydrogène, un groupe alkyle en C₁ à C₆, un groupe cycloalkyle en C₃ à C₈, un groupe benzyle, un groupe phényl,
le groupe constitué par un groupe benzocyclobutyle, un groupe indanyle, un groupe indényle, un groupe oxoindanyle, un groupe naphtyle, un groupe dihydronaphtyle, un groupe tétrahydronaphtyle, un groupe oxotétrahydronaphtyle, un groupe biphénylényle, un groupe fluorényle, un groupe oxofluorényle, un groupe anthryle, un groupe dihydroanthryle, un groupe oxodihydroanthryle, un groupe dioxodihydroanthryle, un groupe phénanthryle, un groupe dihydrophénanthryle, un groupe oxodihydrophénanthryle, un groupe dibenzocycloheptényle, un groupe oxodibenzocycloheptényle, un groupe dihydrodibenzocycloheptényle, un groupe oxodihydrodibenzocycloheptényle, un groupe dihydrodibenzocyclooctényle, un groupe tétrahydrodibenzocyclooctényle et un groupe oxotétrahydrodibenzocyclooctényle, liés directement ou sur un groupe méthylène ; et
le groupe constitué par un groupe furyle, un groupe thiényle, un groupe pyrrolyle, un groupe oxazolyle, un groupe isoxazolyle, un groupe thiazolyle, un groupe isothiazolyle, un groupe pyrazolyle, un groupe imidazolyle, un groupe oxadiazolyle, un groupe thiadiazolyle, un groupe triazolyle, un groupe pyridyle, un groupe pyrazinyle, un groupe pyridazinyle, un groupe pyrimidinyle, un groupe triazinyle, un groupe imidazothiazolyle, un groupe benzofuryle, un groupe dihydrobenzofuryle, un groupe benzothiényle, un groupe dihydrobenzothiényle, un groupe indolyle, un groupe indolinyle, un groupe isoindolinyle, un groupe oxoindolinyle, un groupe dioxoindolinyle, un groupe benzoxazolyle, un groupe oxobenzooxazolinyle, un groupe benzoisooxazolyle, un groupe oxobenzoisooxazolinyle, un groupe benzothiazolyle, un groupe oxobenzothiazolinyle, un groupe benzoisothiazolyle, un groupe oxobenzoisothiazolinyle, un groupe benzoimidazolyle, un groupe oxobenzoimidazolinyle, un groupe indazolyle, un groupe oxoindazolinyle, un groupe benzofurazanyle, un groupe benzothiadiazolyle, un groupe benzotriazolyle, un groupe oxazolopyridyle, un groupe oxodihydrooxazolopyridyle, un groupe thiazolopyridyle, un groupe oxodihydrothiazolopyridyle, un groupe isothiazolopyridyle, un groupe imidazopyridyle, un groupe oxodihydroimidazopyridyle, un groupe pyrazolopyridyle, un groupe oxodihydropyrazolopyridyle, un groupe thiénopyrimidinyle, un groupe chromanyle, un groupe chromanonyle, un groupe benzopyranyle, un groupe chromonyle, un groupe quinoloyle, un groupe isoquinoloyle, un groupe dihydroquinolyle, un groupe oxodihydroquinolinyle, un groupe tétrahydroquinolyle, un groupe oxotétrahydroquinolinyle, un groupe benzodioxanyle, un groupe quinoxalinyle, un groupe quinazolinyle, un groupe naphtypiridinyle, un groupe carbazolyle, un groupe tétrahydrocarbazolyle, un groupe oxotétrahydrocarbazolyle, un groupe pyridoindolyle, un groupe acridinyle, un groupe oxodihydroacridinyle, un groupe phénanthridinyle, un groupe dihydrophénanthridinyle, un groupe oxodihydrophénanthridinyle, un groupe dibenzoisoquinolinyle, un groupe dihydrodibenzoisoquinolinyle, un groupe oxodihydrodibenzoisoquinolinyle, un groupe phénothiazinyle, un groupe dihydrodibenzooxépinyle, un groupe oxodihydrodibenzooxépinyle, un groupe benzocycloheptathiényle, un groupe oxobenzocycloheptathiényle, un groupe dihydrothiénobenzothiépinyle, un groupe oxodihydrothiénobenzothiépinyle, un groupe dihydrodibenzothiépinyle, un groupe oxodihydrodibenzothiépinyle, un groupe octahydrodibenzothiépinyle, un groupe dibenzoazépinyle, un groupe dihydrodibenzoazépinyle, un groupe oxodihydrodibenzoazépinyle, un groupe octahydrodibenzoazépinyle, un groupe benzocycloheptapyridyle, un groupe oxobenzocycloheptapyridyle, un groupe pyridobenzoazépinyle, un groupe dihydropyridobenzoazépinyle, un groupe oxodihydropyridobenzoazépinyle, un groupe dihydropyridobenzodiazépinyle, un groupe dihydrodibenzooxazépinyle, un groupe dihydropyridobenzooxépinyle, un groupe dihydropyridobenzooxazépinyle, un groupe oxydihydropyridobenzooxazépinyle, un groupe dihydrodibenzothiazépinyle, un groupe oxodihydrodibenzothiazépinyle, un groupe dihydropyridobenzothiazépinyle et un groupe oxodihydropyridobenzothiazépinyle, liés directement ou sur un groupe méthylène ;
R¹⁴ a la même signification que R¹³ mais est choisi indépendamment de celui-ci ;
R¹⁵ est choisi parmi
un groupe hydroxy, un groupe méthyle, un groupe benzyle, un groupe phényle, un groupe indanyle, un groupe indényle, un groupe naphtyle, un groupe dihydronaphtyle, un groupe tétrahydronaphtyle, un groupe furyle, un groupe thiényle, un groupe pyrrolyle, un groupe oxazolyle, un groupe isoxazolyle, un groupe thiazolyle, un groupe isothiazolyle, un groupe pyrazolyle, un groupe imidazolyle, un groupe oxadiazolyle, un groupe thiadiazolyle, un groupe triazolyle, un groupe pyridyle, un groupe pyrazinyle, un groupe pyridazinyle, un groupe pyrimidinyle, un groupe triazinyle, un groupe benzofuryle, un groupe benzothiényle, un groupe indolyle, un groupe indolinyle, un groupe benzooxazolyle, un groupe benzothiazolyle, un groupe benzoimidazolyle, un groupe chromanyle, un groupe quinolyle et un groupe tétrahydroquinolyle, liés directement ou sur un groupe méthylène ;
G2 est choisi parmi 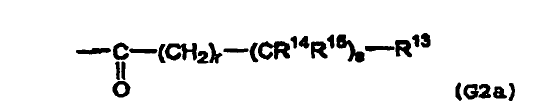 et 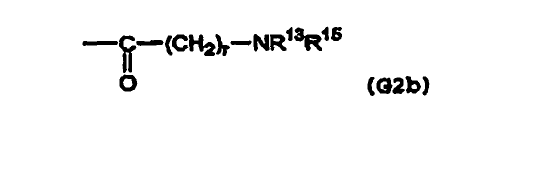 dans lesquelles r, s et les substituants R¹³ à R¹⁵ ont la signification ci-dessus, ou le groupe
-NR¹³R¹⁵
représente les groupes azétidine, pyrrolidine, pipéridine, (1H)-tétrahydropyridine, hexahydroazépine, (1H)-tétrahydroazépine, octahydroazocine, pyrazolidine, pipérazine, hexahydrodiazépine, morpholine, hexahydrooxazépine, thiomorpholine, thiomorpholin-1,1-dioxyde, 5-aza-bicyclo-[2.1.1]hexane, 2-aza-bicyclo[2.2.1]heptane, 7-aza-bicyclo-[2.2.1]heptane, 2,5-diaza-bicyclo[2.2.1]heptane, 2-aza-bicyclo[2.2.2]octane, 8-aza-bicyclo[3.2.1]octane, 2,5-diaza-bicyclo[2.2.2]octane, 9-aza-bicyclo[3.3.1]nonane, indoline, isoindoline, (1H)-dihydroquinoline, (1H)-tétrahydroquinoline, (2H)-tétrahydroisoquinoline, (1H)-tétrahydroquinoxaline, (4H)-dihydrobenzooxazine, (4H)-dihydrobenzothiazine, (1H)-tétrahydrobenzo[b]azépine, (1H)-tétrahydrobenzo[c]azépine, (1H)-tétrahydrobenzo[d]azépine, (5H)-tétrahydrobenzo[b]oxazépine, (5H)-tétrahydrobenzo[b]thiazépine, 1,2,3,4-tétrahydro-9H-pyrido[3,4-b]indole, (10H)-dihydroacridine, (10H)-dihydrophénanthridine, 1,2,3,4-tétrahydroacridanone, (10H)-phénoxazine, (10H)-phénothiazine, (5H)-dibenzoazépine, (5H)-dihydrodibenzoazépine, (5H)-octahydrodibenzoazépine, dihydrobenzo[d,e]isoquinoline, (5H)-dihydrodibenzodiazépine, (5H)-benzo[b]pyrido[f]azépine, (5H)-dihydrobenzo[b]pyrido[f]azépine, (11H)-dihydrodibenzo[b,e]oxazépine, (11H)-dihydrodibenzo[b,e]thiazépine, (10H)-dihydrodibenzo[b,f]oxazépine, (10H)-dihydrodibenzo[b,f]thiazépine, (5H)-tétrahydrodibenzoazocine, (11H)-dihydrobenzo[e]pyrido[b]-1,4-diazépin-6-one ou (11H)-dihydrobenzo[b]pyrido[e]-1,4-diazépin-5-one,
G3 représente
-SO₂-(CH₂)ᵣ-R¹³,
dans lequel r et R¹³ ont la signification ci-dessus,
G4 représente 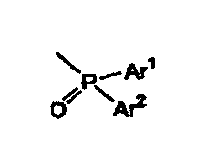 dans lequel
Ar¹ et Ar² sont choisis indépendamment l'un de l'autre parmi un groupe phényle, un groupe pyridyle et un groupe naphtyle ;
G5 représente
-COR¹⁶,
dans lequel
R¹⁶ est choisi parmi
un groupe trifluorométhyle, un groupe alcoxy en C₁ à C₆, un groupe alcényloxy en C₃ à C₆, et un groupe benzyloxy,
où les systèmes cycliques aromatiques dans les substituants sont éventuellement substitués indépendamment les uns des autres par un à trois des groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl (en C₁ à C₆)amino, et un groupe méthylène dioxyde dans le cas de deux résidus adjacents sur le noyau aromatique, et
où les résidus alkyle, alcényle et cycloalkyle dans les groupes G1, G2 et G3 sont éventuellement substitués par un ou deux des groupes identiques ou différents qui sont choisis parmi
un groupe hydroxy, un groupe carboxy, un groupe alcoxycarbonyle en C₂ à C₇, un groupe benzyloxycarbonyle, un groupe amino, un groupe monoalkyl (en C₁ à C₆)amino et un groupe dialkyl(en C₁ à C₆)amino.

24. Utilisation selon l'une quelconque des revendications 21 à 23, dans laquelle
R¹ est choisi parmi
l'hydrogène, le fluor, le chlore, le brome, un groupe méthyle, un groupe éthyle, un groupe trifluorométhyle, un groupe hydroxy, un groupe alcoxy en C₁ à C₄, un groupe méthylthio, un groupe éthylthio, un groupe carboxy et un groupe phénoxy ;
R² est choisi parmi l'hydrogène, le chlore et un groupe méthyle ;
R³ est l'hydrogène ;
R⁴ est choisi parmi
l'hydrogène, un groupe alkyle en C₁ à C₃ et un groupe hydroxy,
k est 0
A est choisi parmi
un groupe alkylène en C₂ à C₆, qui est éventuellement substitué une fois ou deux fois par un groupe hydroxy ou le fluor ;
un groupe alkylène en C₂ à C₆, dans lequel une unité méthylène est remplacée de façon isostérique par O, S ou CO, dans lequel, à l'exception de CO, la substitution isostérique n'est pas adjacente au groupe amide ;
un groupe alcénylène en C₂ à C₆ qui est éventuellement substitué par un groupe alkyle en C₁ à C₃ et/ou le fluor ; et
un groupe alcadiénylène en C₄ à C₆ ;
D est choisi parmi
un groupe alkylène en C₂ à C₈ qui est éventuellement substitué par un groupe méthyle ou un groupe hydroxy ;
un groupe alcénylène en C₄ à C₈, qui est éventuellement substitué par un groupe hydroxy ;
un groupe alcynylène en C₄ à C₈, qui est éventuellement substitué par un groupe hydroxy ; et
le groupe constitué par un groupe alkylène en C₂ à C₈, un groupe alcénylène en C₄ à C₈, et un groupe alcynylène en C₄ à C₈ dans lequel une unité méthylène est remplacée de façon isostérique par O, NH, N(CH₃), CO ou SO₂ ou un groupe éthylène est remplacé de façon isostérique par un groupe NH-CO et/ou CO-NH ou un groupe propylène est remplacé de façon isostérique par un groupe NH-CO-O et/ou O-CO-NH ;
E est choisi parmi une pipérazine et une hexahydro-1,4-diazépine, où le cycle est éventuellement substitué par un ou deux groupes méthylène et/ou par un groupe oxo adjacent à un atome d'azote ;
G est choisi parmi l'hydrogène, un groupe cycloalkyle en C₃ à C₈, un groupe méthoxycarbonyle, un groupe tert-butoxycarbonyle, un groupe benzyloxycarbonyle, un groupe trifluoroacétyle, un groupe diphénylphosphinoyle,
-(CH₂)ᵣ-(CR¹⁴R¹⁵)ₛ-R¹³, 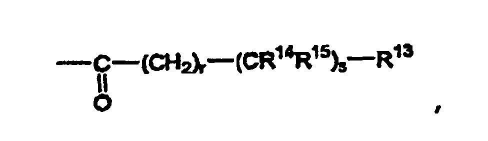 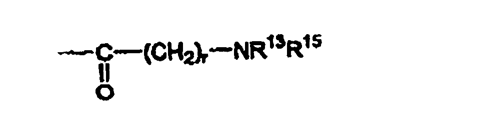
et
-SO₂- (CH₂)ᵣ-R¹³
où
r est 0 ou 1
s est 0 ou 1,
R¹³ est choisi parmi l'hydrogène, un groupe méthyle, un groupe benzyle, un groupe phényle,
le groupe constitué par un groupe indanyle, un groupe indényle, un groupe oxoindanyle, un groupe naphtyle, un groupe tétrahydronaphtyle, un groupe fluorényle, un groupe oxofluorényle, un groupe anthryle, un groupe dihydroanthryle, un groupe oxodihydroanthryle, un groupe dioxodihydroanthryle, un groupe phénanthryle, un groupe dihydrophénanthryle, un groupe oxydihydrophénanthryle, un groupe dibenzocycloheptényle, un groupe dihydrodibenzocycloheptényle, et un groupe oxodihydrodibenzocycloheptyle, liés directement ou sur un groupe méthylène, et
le groupe constitué par un groupe furyle, un groupe thiényle, un groupe oxazolyle, un groupe isooxazolyle, un groupe thiazolyle, un groupe imidazolyle, un groupe oxadiazolyle, un groupe pyridyle, un groupe pyrazinyle, un groupe pyrimidinyle, un groupe imidazothiazolyle, un groupe benzofuryle, un groupe benzothiényle, un groupe indolyle, un groupe indolinyle, un groupe oxoindolinyle, un groupe dioxoindolinyle, un groupe benzoxazolyle, un groupe oxobenzooxazolinyle, un groupe benzothiazolyle, un groupe oxobenzthiazolinyle, un groupe benzimidazolyle, un groupe oxobenzimidazolinyle, un groupe indazolyle, un groupe benzofurazanyle, un groupe benzotriazolyle, un groupe oxazolopyridyle, un groupe oxodihydrooxazolopyridyle, un groupe thiazolopyridyle, un groupe oxodihydrothiazolopyridyle, un groupe imidazopyridyle, un groupe oxodihydroimidazopyridyle, un groupe chromanyle, un groupe chromanonyle, un groupe benzopyranyle, un groupe chromonyle, un groupe quinolyle, un groupe isoquinolyle, un groupe oxodihydroquinolinyle, un groupe tétrahydroquinolyle, un groupe oxotétrahydroquinolinyle, un groupe benzodioxanyle, un groupe quinazolinyle, un groupe carbazolyle, un groupe acridinyle, un groupe dihydroacridinyle, un groupe oxodihydroacridinyle, un groupe dihydrophénanthridinyle, un groupe dihydrobenzoisoquinolinyle, un groupe phénothiazinyle, un groupe dihydrodibenzooxépinyle, un groupe benzocycloheptathiényle, un groupe dihydrothiénobenzothiépinyle, un groupe dihydrodibenzothiépinyle, un groupe oxodihydrodibenzothiépinyle, un groupe dihydrodibenzoazépinyle, un groupe oxodihydrodibenzoazépinyle, un groupe octahydrodibenzoazépinyle, un groupe benzocycloheptapyridyle, un groupe dihydropyridobenzodiazépinyle, et un groupe dihydrodibenzothiazépinyle, liés directement ou sur un groupe méthylène,
R¹⁴ est choisi parmi l'hydrogène, un groupe méthyle, un groupe benzyle, et un groupe phényle,
R¹⁵ est choisi parmi l'hydrogène, un groupe hydroxy, un groupe méthyle, un groupe benzyle, un groupe phényle ; et
le groupe constitué par un groupe naphtyle, un groupe tétrahydronaphtyle, un groupe furyle, un groupe thiényle, un groupe oxazolyle, un groupe thiazolyle, un groupe imidazolyle, un groupe pyridyle, un groupe benzofuryle, un groupe benzothiényle, un groupe indolyle, un groupe indolinyle, un groupe benzoxazolyle, un groupe benzothiazolyle, un groupe benzimidazolyle, un groupe chromanyle, un groupe quinolyle et un groupe tétrahydroquinolyle, liés directement ou sur un groupe méthylène ;
où le groupe
-NR¹³R¹⁵
représente un hétérocycle contenant de l'azote lié sur l'atome d'azote lequel hétérocycle contenant de l'azote est choisi parmi
un groupe pyrrolidine, un groupe pipéridine, un groupe hexahydroazépine, un groupe pipérazine, un groupe hexahydrodiazépine, un groupe morpholine, un groupe hexahydroazépine, un groupe thiomorpholine, un groupe 7-aza-bicyclo[2.2.1]heptane, un groupe 2,5-diaza-bicyclo[2.2.1]heptane, un groupe indoline, un groupe isoindoline, un groupe (1H)-dihydroquinoline, un groupe (1H)-tétrahydroquinoline, un groupe (2H)-tétrahydroisoquinoline, un groupe (4H)-dihydrobenzoxazine, un groupe (4H)-dihydrobenzothiazine, un groupe (1H)-tétrahydrobenzo[b]azépine, un groupe (1H)-tétrahydrobenzo[d]azépine, un groupe (5H)-tétrahydrobenzo[b]oxazépine, un groupe (5H)-tétrahydrobenzo[b]thiazépine, un groupe (10H)-dihydroacridine, un groupe 1,2,3,4-tétrahydroacridanone, un groupe (10H)-dihydrophénanthridine, un groupe (1H)-dihydrobenzo[d,e]isoquinoline, un groupe (10H)-phénothiazine, un groupe (5H)-dibenzo[b,f]azépine, un groupe (5H)-dihydrodibenzo[b,f]azépine, un groupe (5H)-dihydrodibenzo[c,e]azépine, un groupe (5H)-dihydrodibenzodiazépine, un groupe (11H)-dihydrodibenzo[b,e]oxazépine, un groupe (11H)-dihydrodibenzo[b,e]thiazépine, un groupe (5H)-dihydrobenzo[b]pyrido[3,2-f]azépine et un groupe (11H)-6-oxodihydrobenzo[e]pyrido[3,2-b][1,4]diazépine,
où les systèmes cycliques aromatiques dans les substituants sont éventuellement substitués, indépendamment les uns des autres, par un à trois des groupes identiques ou différents choisis parmi un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor, un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl(en C₁ à C₆)amino, et dans le cas de deux résidus adjacents sur le noyau aromatique, un groupe méthylènedioxy, et
où les résidus alkyle, alcényle et cycloalkyle dans le groupe G sont éventuellement substitué par un ou deux des groupes identiques ou différents qui sont choisis parmi un groupe hydroxy, un groupe carboxy, un groupe alcoxycarbonyle en C₂-C₇, un groupe benzyloxycarbonyle, un groupe amino, un groupe monoalkyl(en C₁ à C₆)amino et un groupe dialkyl(en C₁ à C₆)amino.

25. Utilisation selon l'une quelconque des revendications 21 à 24, dans laquelle
R¹ est choisi parmi
l'hydrogène, le fluor, un groupe méthyle, un groupe trifluorométhyle et un groupe éthylthio ;
R², R³ et R⁴ sont chacun un hydrogène ;
k est 0,
A est choisi parmi
le groupe constitué par l'éthylène, le propylène et le butylène, qui sont chacun éventuellement substitués par un groupe hydroxy ou un ou deux atome de fluor ;
OCH₂, SCH₂ ;
un groupe éthénylène, et un groupe 1,3-butadiénylène ;
D est choisi parmi
un groupe alkylène en C₂ à C₆ qui est éventuellement substitué par un groupe hydroxy ;
un groupe alcénylène en C₄ à C₆ ;
un groupe alcynylène en C₄ à C₆ ; et
le groupe constitué par un groupe alkylène en C₂ à C₆, un groupe alcénylène en C₄ à C₆ et un groupe alcynylène en C₄ à C₆, dans lequel une ou deux unités méthylène sont remplacée de façon isostérique par O, NH, CO ou SO₂ ;
E est une pipérazine ou une hexahydro-1,4-diazépine ;
G est choisi parmi
un groupe phényle, un groupe benzyle, un groupe phénéthyle, un groupe diphénylméthyle, un groupe naphtyle, un groupe tétrahydronaphtyle, un groupe naphtylméthyle, un groupe fluorényle, un groupe fluorénylméthyle, un groupe anthrylméthyle, un groupe dihydrodibenzocycloheptényle, un groupe furylméthyle, un groupe thiénylméthyle, un groupe thiazolylméthyle, un groupe pyridylméthyle, un groupe benzothiénylméthyle, un groupe quinolylméthyle, un groupe phénylthiénylméthyle, un groupe phénylpyridylméthyle, un groupe benzocycloheptapyridinyle, un groupe dihydrobenzocycloheptapyridinyle, un groupe dihydrodibenzooxépinyle, un groupe dihydrodibenzothiépinyle, un groupe dihydrodibenzoazépinyle, un groupe dihydrobenzopyridodiazépinyle, un groupe formyle, un groupe acétyle, un groupe pivaloyle, un groupe phénylacétyle, un groupe diphénylacétyle, un groupe diphénylpropionyle, un groupe naphtylacétyle, un groupe benzoyle, un groupe naphtoyle, un groupe oxofluorénylcarbonyle, un groupe oxodihydroanthrylcarbonyle, un groupe dioxodihydroanthrylcarbonyle, un groupe furoyle, un groupe pyridylacétyle, un groupe pyridylcarbonyle, un groupe chromonylcarbonyle, un groupe quinolylcarbonyle, un groupe phénylylaminocarbonyle, un groupe naphtylaminocarbonyle, un groupe tétrahydronaphtylaminocarbonyle, un groupe dibenzylaminocarbonyle, un groupe benzylphénylaminocarbonyle, un groupe diphénylaminocarbonyle, un groupe indolinyl-N-carbonyle, un groupe isoindolinyl-N-carbonyle, un groupe tétrahydroquinolinyl-N-carbonyle, un groupe carbazolyl-N-carbonyle, un groupe tétrahydrobenzoazépinyl-N-carbonyle, un groupe dihydrodibenzoazépinyl-N-carbonyle, un groupe dihydrobenzopyridoazépinyl-N-carbonyle, un groupe oxodihydrobenzopyridoazépinyl-N-carbonyle, un groupe méthanesulfonyle, un groupe toluènesulfonyle, un groupe naphtylsulfonyle, un groupe quinolinesulfonyle et un groupe diphénylphosphinoyle,
où les systèmes cycliques aromatiques sont éventuellement substitués indépendamment les uns des autres par un à trois des groupes identiques ou différents choisis parmi
un halogène, un groupe cyano, un groupe alkyle en C₁ à C₆, un groupe trifluorométhyle, un groupe cycloalkyle en C₃ à C₈, un groupe phényle, un groupe benzyle, un groupe hydroxy, un groupe hydroxyalkyle en C₁ à C₆, un groupe alcoxy en C₁ à C₆, un groupe alcoxy en C₁ à C₆ qui est entièrement ou partiellement substitué par le fluor ; un groupe benzyloxy, un groupe phénoxy, un groupe mercapto, un groupe alkylthio en C₁ à C₆, un groupe carboxy, un groupe carboxyalkyle en C₂ à C₇, un groupe carboxyalcényle en C₂ à C₇, un groupe alcoxycarbonyle en C₂ à C₇, un groupe benzyloxycarbonyle, un groupe nitro, un groupe amino, un groupe monoalkyl(en C₁ à C₆)amino, un groupe dialkyl(en C₁ à C₆)amino, et dans le cas de deux résidus adjacents sur le noyau aromatique, un groupe méthylènedioxy, et
où les résidus alkyle, alcényle et cycloalkyle dans le groupe G sont éventuellement substitués par un ou deux des groupes identiques ou différents qui sont choisis parmi
un groupe hydroxy, un groupe carboxy, un groupe alcoxycarbonyle en C₂ à C₇, un groupe benzyloxycarbonyle, un groupe amino, un groupe monoalkyl(en C₁ à C₆)amino et un groupe dialkyl(en C₁ à C₆)amino.

26. Utilisation selon l'une quelconque des revendications 21 à 25, dans laquelle le(s) composé(s) de formule (I) est/sont choisi(s) dans le groupe constitué par :
N-[4-(4-diphénylméthylpipérazin-1-yl)-3-hydroxybutyl]-3-pyridin-3-yl-acrylamide ;
N-[3-(4-diphénylméthylpipérazin-1-yl)-propoxy]-3-pyridin-3-yl-acrylamide ;
N-[4-(4-diphénylméthylpipérazin-1-yl)-4-oxo-butyl]-3-pyridin-3-yl-acrylamide ;
N-[3-(4-diphénylméthylpipérazin-1-sulfonyl)-propyl]-3-pyridin-3-yl-acrylamide ;
N-{2-[2-(4-diphénylméthylpipérazin-1-yl)-éthoxy]-éthyl}-3-pyridin-3-yl-acrylamide ;
N-(4-[bis-(4-fluorophényl)-méthyl]-pipérazin-1-1-yl}-but-2-in-yl)-3-yl-acrylamide ;
N-{4-[4-(4-carboxyphényl-phénylméthyl)-pipérazin-1-yl]-butyl}-3-pyridin-3-yl-acrylamide ;
N-[4-{4-[(4-aminophényl)-phénylméthyl]-pipérazin-1-yl}butyl)-3-pyridin-3-yl-acrylamide ;
N-{4-[4-(9H-fluorèn-9-yl)-pipérazin-1-yl]-butyl}-2-(pyridin-3-yloxy)-acétamide ;
N-{5-[4-(9H-fluorèn-9-yl)-pipérazin-1-yl]-pentyl}-3-pyridin-3-yl-acrylamide ;
N-{6-[4-(9H-fluorèn-9-yl)-pipérazin-1-yl]-hexyl}-3-pyridin-3-yl-acrylamide ;
3-pyridin-3-yl-N-{4-[4-(1,2,3,4-tétrahydronaphtalèn-1-yl)-pipérazin-1-yl]-butyl}-acrylamide ;
3-pyridin-3-yl-N-{4-[4-(5,6,7,8-tétrahydronaphtalèn-1-yl)-pipérazin-1-yl]-butyl}-acrylamide ;
N-{4-[4-(naphtalèn-1-yl)-pipérazin-1-yl]-butyl}-3-pyridin-3-yl-acrylamide ;
N-[4-(4-biphényl-2-yl-pipérazin-1-yl)-butyl]-3-pyridin-3-yl-propionamide ;
N-[5-(4-biphényl-2-yl-pipérazin-1-yl)-pentyl]-3-pyridin-3-yl-acrylamide ;
N-[6-(4-biphényl-2-yl-pipérazin-1-yl)-hexyl]-3-pyridin-3-yl-acrylamide ;
N-[4-(4-biphényl-2-yl-pipérazin-1-yl)-butyl]-2-(pyridin-3-yloxy)-acétamide ;
amide de l'acide N-[4-(4-biphényl-2-yl-pipérazin-1-yl)-butyl]-5-(pyridin-3-yl)-penta-2,4-diénoïque ;
N-{4-[4-(10,11-dihydro-5H-dibenzo[a,d]cycloheptèn-5-yl)-pipérazin-1-yl]-butyl}-3-pyridin-3-yl-propionamide ;
N-{5-[4-(10,11-dihydro-5H-dibenzo[a,d]cycloheptèn-5-yl)-pipérazin-1-yl]-pentyl}-3-pyridin-3-yl-acrylamide ;
N-{6-[4-(10,11-dihydro-5H-dibenzo[a,d]cycloheptèn-5-yl)-pipérazin-1-yl]-hexyl}-3-pyridin-3-yl-acrylamide ;
amide de l'acide N-{4-[4-(10,11-dihydro-5H-dibenzo[a,d]cycloheptèn-5-yl)-pipérazin-1-yl]-butyl}-5-(pyridin-3-yl)-penta-2,4-diénoïque ;
N-{4-[4-(6,11-dihydro-dibenzo[b,e]oxépin-11-yl)-pipérazin-1-yl]-butyl}-3-pyridin-3-yl-propionamide ;
N-{2-[4-(6,11-dihydrodibenzo[b,e]thiépin-11-yl)-pipérazin-1-yl]-éthyl}-3-pyridin-3-yl-acrylamide ;
N-[4-(4-diphénylacétyl-pipérazin-1-yl)-butyl]-3-pyridin-3-yl-acrylamide ;
N-[4-(4-benzoylpypérazin-1-yl)-butyl]-3-pyridin-3-yl-acrylamide ;
N-{4-[4-(2-aminobenzoyl)-pipérazin-1-yl]-butyl}-3-pyridin-3-yl-acrylamide ;
N-{4-[4-(4-carboxybenzoyl)-pipérazin-1-yl]-butyl}-3-pyridin-3-yl-acrylamide ;
N-{4-[4-(biphényl-2-carbonyl)-pipérazin-1-yl]-butyl}-3-pyridin-3-yl-acrylamide ;
N-{4-[4-(9-oxo-9H-fluorèn-4-carbonyl)-pipérazin-1-yl]-butyl}-3-pyridin-3-yl-acrylamide ;
N-{4-[4-(furan-2-carbonyl)-pipérazin-1-yl]-butyl}-3-pyridin-3-yl-acrylamide ;
N-{4-[4-(naphtalèn-1-yl-aminocarbonyl)-pipérazin-1-yl]-butyl}-3-pyridin-3-yl-propionamide ;
N-{4-[4-(diphénylaminocarbonyl)-pipérazin-1-yl]-butyl}-3-pyridin-3-yl-acrylamide ;
N-{4-[4-(naphtalèn-2-sulfonyl)-pipérazin-1-yl]-butyl}-3-pyridin-3-yl-acrylamide ;
N-[4-(4-diphénylphosphinoyl-pipérazin-1-yl)-butyl]-3-pyridin-3-yl-acrylamide ;
N-[4-(4-biphényl-2-yl-pipérazin-1-yl)-butyl}-3-pyridin-3-yl-acrylamide ;
N-{4-[4-(9H-fluorèn-9-yl)-pipérazin-1-yl]-butyl}-3-pyridin-3-yl-acrylamide ; et
N-{4-[4-(10,11-dihydro-5H-dibenzo[a,d]cyclohepten-5-yl)-pipérazin-1-yl]-butyl}-3-pyridin-3-yl-acrylamide.

27. Utilisation d'un ou plusieurs composés selon l'une quelconque des revendications 1 à 7 pour la fabrication d'une composition pharmaceutique pour l'immunodépression dans le corps humain ou animal, de façon facultative en combinaison avec un autre agent cytostatique ou un agent immunosuppresseur et/ou d'autres compositions pharmaceutiques appropriées pour cette indication.
